# EUROPEAN PATENT APPLICATION

(11) **EP 4 644 548 A1**
(43) Date of publication of application: **05.11.2025**
(21) Application number: 23912220.3
(22) Date of filing: 27.12.2023
(51) Int. Cl.: C12N 15/13, C07K 1/02, C07K 16/00, C12N 1/15, C12N 1/19, C12N 1/21, C12N 5/10, C12P 21/08

(54) **POLYPEPTIDE WITH CONTROLLED ASSOCIATION**

(30) Priority: 27.12.2022 JP 2022210580
(71) Applicant: CHUGAI SEIYAKU KABUSHIKI KAISHA, Tokyo 115-8543 (JP)
(72) Inventor: KOGA, Hikaru, Yokohama-shi, Kanagawa 244-8602 (JP); SHIMIZU, Shun, Yokohama-shi, Kanagawa 244-8602 (JP)
(74) Representative: Vossius & Partner Patentanwälte Rechtsanwälte mbB
(86) International application number: PCT/JP2023/046857
(87) International publication number: WO 2024/143442

(57) **Abstract**

The present disclosure relates to polypeptides for which association between polypeptides is controlled, and in one embodiment, polypeptides for which association between Fc regions is controlled, methods for producing polypeptides for which association between Fc regions (in one embodiment, CH3 regions) is controlled, methods for controlling association between Fc regions, compositions comprising such an Fc region, compositions comprising a nucleic acid encoding such a polypeptide, and the like.

## Description

### [Technical Field]

The present disclosure relates to polypeptides for which association between polypeptides is controlled, for example, polypeptides for which association between heavy chains or between Fc regions is controlled, methods for producing polypeptides for which association between Fc regions (in one embodiment, CH3 regions) is controlled, methods for controlling association between heavy chains or between Fc regions, compositions comprising a polypeptide having an Fc region for which association is controlled, and compositions containing a nucleic acid encoding such a polypeptide, and the like.

### [Background Art]

Methods for coexpressing multiple antibodies in a single cell are important for efficient production of pharmaceutical mixtures or generation of pharmaceutical mixtures in living organisms using mRNAs, DNA plasmids, gene therapy, or such. However, when antibody A that is composed of HA (heavy chain) and LA (light chain) and antibody B that is composed of HB (heavy chain) and LB (light chain) are coexpressed, eight antibody species in addition to the desired antibodies A and B are formed in a random manner. This leads to production of a heterogeneous pharmaceutical mixture or heterogeneous efficacy. Therefore, there was a need for a technique to suppress the expression of the antibodies other than antibodies A and B and allow antibodies A and B to be expressed efficiently.

As methods for solving this problem, a method that utilizes differences of the specificity of heavy chain association between isotypes such as IgG and IgA, and a method that utilizes the difference of the specificity of heavy chain association between the subclasses IgG1 and IgG3, have been reported (PTL 1). However, since antibodies that are generally used for therapeutic purposes are IgG, there is a demand for a technique that is applicable to all IgG subclasses and enables even antibodies of the same IgG subclass to be coexpressed. To address this problem, there have been reports of methods for promoting the association of heavy chains of the same type, in which amino acid modifications are introduced into the heavy chain CH3 regions to suppress the expression of IgG in which HA and HB are associated (PTLs 2-4). These are all methods for controlling the association of heavy chains of the same type using the attractive and repulsive forces of electric charges, but have actually been tested for limited modifications and their combinations. Thus, they have not been able to achieve efficient coexpression of three or more antibodies. Moreover, these prior studies were intended for simultaneous production of multiple antibodies, not for expression of antibodies in living organisms. In the case of expressing therapeutic antibodies in living organisms, their not associating with endogenous IgG would lead to reduced side effects. However, there has been no report of a technique that achieves coexpression of multiple antibodies that do not associate with endogenous IgG.

### [Citation List]

### [Patent Literature]

[PTL 1] WO 2004/009618
[PTL 2] WO 2013/157953
[PTL 3] WO 2014/015804
[PTL 4] WO 2017/205014

### [Summary of Invention]

### [Technical Problem]

The present invention was achieved in view of the above circumstances. An objective of the present invention is to provide polypeptides for which association between polypeptides is controlled, methods for producing polypeptides for which association is controlled, methods for controlling the association of polypeptides, nucleic acids encoding polypeptides for which association is controlled, compositions containing such a nucleic acid, and the like.

A further objective of the present invention is, in one embodiment, to provide polypeptides for which association between Fc regions is controlled, methods for producing polypeptides for which association between Fc regions is controlled, expression of polypeptides for which association between Fc regions is controlled in living organisms, and methods for controlling association between Fc regions. Further, an objective of the present disclosure is to provide, in one embodiment, antibodies for which association in the interface between CH3 regions is controlled, and methods for coexpressing two or more antibodies in an efficient manner.

### [Solutions to Problem]

The present inventors selected CH3, a constant region of the heavy chain, as an inter-Fc region to be subjected to control of association, and conducted dedicated studies on control of the association between Fc regions of the same type. As a result, the inventors discovered that multimers of Fc regions of the same type can be efficiently formed by suppressing association between Fc regions of different types using steric hindrance caused by modification of amino acid residues in the CH3 interface into bulky and small amino acids at the same time or using artificially introduced disulfide linkages, or by introducing novel combinations of modifications utilizing the attractive and repulsive forces of electric charges.

Thus, with the findings of the present inventors, it is possible to control the association between heavy chains of the same type. Moreover, the present disclosure is not only applicable to the control of association between heavy chains of the same type, but is also able to control association between any Fc-containing polypeptides.

The present inventors further confirmed that antibodies for which association between Fc regions is controlled according to the present disclosure retain the functions of Fc.

As stated above, the present inventors successfully developed polypeptides for which association between Fc regions is controlled, thereby completing the present invention.

The present disclosure relates to polypeptides for which association between polypeptides is controlled, methods for producing polypeptides for which association is controlled, methods for controlling association of polypeptides, nucleic acids encoding polypeptides for which association is controlled, compositions containing such a nucleic acid, and such.

In one embodiment, the present disclosure relates to polypeptides for which association between Fc regions is controlled, methods for producing polypeptides for which association between Fc regions is controlled, expression of polypeptides for which association between Fc regions is controlled in living organisms, and methods for controlling association between Fc regions, and more specifically relates to the following:
[1] A nucleic acid encoding a first polypeptide,
   wherein the first polypeptide comprises an Fc region into which a modification has been introduced, and
   wherein, because of the modification introduced into the Fc region, the first polypeptide associates more readily with a first polypeptide having the modification via the Fc region than with a first polypeptide comprising an Fc region into which the modification has not been introduced.
   [1-1] The aforementioned nucleic acid, wherein the first polypeptide associates less readily with a polypeptide into which the modification has not been introduced than with a polypeptide having the modification.
   [1-1-1] The aforementioned nucleic acid, wherein the first polypeptide less readily forms a heterodimer with another polypeptide into which the modification has not been introduced.
   [1-1-2] The aforementioned nucleic acid, which less readily forms a heterodimer than a homodimer.
   [1-1-3] The aforementioned nucleic acid, wherein the first polypeptide does not associate, or associates less readily, with endogenous IgG or an antibody fragment containing an Fc region thereof.
   [1-2] The aforementioned nucleic acid, wherein the first polypeptides having the modification associate with each other.
   [1-2-1] The aforementioned nucleic acid, wherein the first polypeptide having the modification forms a homomer.
   [1-2-2] The aforementioned nucleic acid, which more readily forms a homodimer than a heterodimer.
   [1-2-3] The aforementioned nucleic acid, wherein the first polypeptide exhibits a stronger homodimerization-promoting ability than a control (e.g., wild-type IgG).
   [1-2-4] The aforementioned nucleic acid, wherein a homodimer of a polypeptide other than the first polypeptide (e.g., a second polypeptide) is further formed.
   [1-2-5] The aforementioned nucleic acid, wherein a homodimer(s) of one or two or more types of polypeptides are formed.
   [1-2-6] The aforementioned nucleic acid, wherein the modifications in the two or more types of polypeptides are different from each other.
   [1-2-7] The aforementioned nucleic acid, wherein the combination of the modifications different from each other is a combination shown in Table 7.
   [1-3] The aforementioned nucleic acid, wherein the first polypeptide having the modification has an increased ability to form an associated multimer with a polypeptide having the modification as compared to a polypeptide into which the modification has not been introduced.
   [1-4] The aforementioned nucleic acid, wherein the nucleic acid is an RNA, a DNA, or a vector or plasmid carrying the nucleic acid.
   [1-5] The aforementioned nucleic acid, wherein the first polypeptide is a polypeptide for which association is controlled.
   [1-5-1] The aforementioned nucleic acid, wherein the association is association between Fc regions.
   [1-6] The aforementioned nucleic acid, wherein the first polypeptide has CH3.
   [1-6-1] The aforementioned nucleic acid, wherein the association is association in an interface between CH3 regions.
   [1-6-2] The aforementioned nucleic acid, wherein the modification is a modification in CH3.
[2] The aforementioned nucleic acid, wherein the first polypeptide associates more readily with a first polypeptide having the modification via the Fc region than with a first polypeptide comprising an Fc region into which the modification has not been introduced due to at least one of the following actions resulting from the introduced modification: (1) steric complementarity (also referred to as "steric hindrance"), (2) disulfide linkage (also referred to as "disulfide bond"), and (3) electrostatic charge (also referred to as "charge").
   [2-1] The aforementioned nucleic acid, wherein the steric complementarity results from the presence of a knob and a hole in the Fc region.
   [2-1-1] The aforementioned nucleic acid, wherein the modification is a modification to introduce a knob and a hole into the Fc region.
   [2-1-2] The aforementioned nucleic acid, wherein the knob and the hole in the Fc region induce the formation of a homodimer of the first polypeptide.
   [2-1-3] The aforementioned nucleic acid, wherein the knob and the hole are all introduced into the first polypeptide.
   [2-1-4] The aforementioned nucleic acid, which associates more readily with a polypeptide having the modification than a polypeptide into which the modification has not been introduced due to at least the combinations of the actions of (1) steric complementarity and (3) electrostatic charge.
   [2-2] The aforementioned nucleic acid, wherein the disulfide linkage is formed by substituting one or more amino acids in the Fc region with cysteine (C).
   [2-2-1] The aforementioned nucleic acid, wherein the modification is a modification to introduce one or more cysteines into the Fc region.
   [2-2-2] The aforementioned nucleic acid, wherein the modification is substitution of one or more amino acids in the Fc region with cysteine.
   [2-2-3] The aforementioned nucleic acid, wherein the disulfide linkage is a linkage between the cysteines modified (introduced) in the Fc region.
   [2-2-4] The aforementioned nucleic acid, which associates more readily with a polypeptide having the modification than a polypeptide into which the modification has not been introduced due to at least the combinations of the actions of (2) disulfide linkage and (3) electrostatic charge.
   [2-3] The aforementioned nucleic acid, wherein the action of the electrostatic charge results from one or more charged amino acids modified (introduced) in the Fc region.
   [2-3-1] The aforementioned nucleic acid, wherein the introduced charged amino acid(s) is a positively charged amino acid and/or a negatively charged amino acid.
   [2-3-2] The aforementioned nucleic acid, wherein at least two amino acids in the Fc region are substituted with a positively charged amino acid and a negatively charged amino acid.
   [2-3-3] The aforementioned nucleic acid, wherein the positively charged amino acid is selected from lysine (K), arginine (R), and histidine (H), and/or the negatively charged amino acid is selected from aspartic acid (D) and glutamic acid (E).
   [2-4] The aforementioned nucleic acid, wherein the action is (a combination of) more than one action (two or three actions) selected from (1) steric complementarity, (2) disulfide linkage, and (3) electrostatic charge.
[3] A composition comprising:
   the nucleic acid of [1]; and
   a nucleic acid encoding a second polypeptide,
   wherein the second polypeptide comprises an Fc region into which a modification has been introduced, and
   wherein, because of the modification introduced into the Fc region, the second polypeptide associates more readily with a second polypeptide having the modification via the Fc region than with a second polypeptide comprising an Fc region into which the modification has not been introduced.
   [3-1] The aforementioned composition, wherein the modification in the second polypeptide is different from the modification in the first polypeptide.
   [3-2] The aforementioned composition, wherein the action in the second polypeptide is different from the action in the first polypeptide.
   [3-3] The aforementioned composition, wherein the amino acid sequence of the second polypeptide prior to modification differs from the amino acid sequence of the first polypeptide prior to modification.
   [3-4] The aforementioned composition, which further comprises a nucleic acid encoding a polypeptide different from the first or second polypeptide (in one embodiment, a third and/or fourth polypeptide).
[4] A composition comprising:
   the nucleic acid of [1];
   a nucleic acid encoding a second polypeptide; and
   a nucleic acid encoding a third polypeptide,
   wherein the second polypeptide associates more readily with the third polypeptide than with the second polypeptide.
[5] A composition comprising:
   the nucleic acid of [2]; and
   a nucleic acid encoding a second polypeptide, wherein the second polypeptide does not have the modification the first polypeptide has.
[6] The aforementioned composition, wherein the modification introduced into the first polypeptide is different from the modification introduced into the second polypeptide.
[7] The aforementioned composition, wherein the second polypeptide associates more readily with a second polypeptide having the modification via the Fc region than with a second polypeptide comprising an Fc region into which the modification has not been introduced due to at least one of the following actions resulting from the introduced modification: (1) steric complementarity, (2) disulfide linkage, and (3) electrostatic charge.
   [7-1] The aforementioned composition, wherein the modification in the second polypeptide is different from the modification in the first polypeptide.
   [7-2] The aforementioned composition, wherein the action in the second polypeptide is different from the action in the first polypeptide.
[8] The aforementioned nucleic acid or composition, wherein the modification introduced into the Fc region is at least one of the modifications set forth in Table 2.
[9] The aforementioned nucleic acid or composition, wherein the modification introduced into the Fc region is at least one of the modifications set forth in Table 4.
[10] The aforementioned nucleic acid or composition, wherein the modification introduced into the first polypeptide and the modification introduced into the second polypeptide are at least one of the combinations of modifications set forth in Table 6.
[11] The aforementioned nucleic acid or composition, wherein the polypeptide is an antibody.
[12] The aforementioned nucleic acid or composition, wherein the Fc region is an Fc region of IgG.
[13] The aforementioned nucleic acid or composition, wherein the polypeptide into which the modification has been introduced maintains a function of the polypeptide prior to modification.
[14] The aforementioned nucleic acid or composition, wherein the Fc region into which the modification has been introduced maintains a function of the Fc of IgG.
[15] The aforementioned nucleic acid or composition, wherein the Fc region is derived from any one of IgG1, 2, 3, and 4.
[16] A host cell into which the aforementioned nucleic acid or composition has been introduced.
[17] A polypeptide expressed from the aforementioned nucleic acid.
[18] A composition comprising a nucleic acid encoding a polypeptide,
   wherein the polypeptide comprises an Fc region into which a modification has been introduced, wherein the modification is at least one of the modifications set forth in Table 2.
[19] A polypeptide which comprises an Fc region into which a modification has been introduced, wherein the polypeptide associates more readily with a polypeptide having the modification via the Fc region than with a polypeptide comprising an Fc region into which the modification has not been introduced due to at least one of the following actions resulting from the introduced modification: (1) steric complementarity, (2) disulfide linkage, and (3) electrostatic charge.
   [19-1] The aforementioned polypeptide, wherein the modification is a modification in CH3.
[20] A method for obtaining a polypeptide for which association is controlled, comprising the step of obtaining a nucleic acid encoding the polypeptide; and
   the step of expressing the nucleic acid,
   wherein the polypeptide comprises an Fc region, and
   wherein, because of the modification introduced into the Fc region, the polypeptide associates more readily with a polypeptide having the modification via the Fc region than with a polypeptide comprising an Fc region into which the modification has not been introduced.
   [20-1] The aforementioned method, wherein the expression is *in vivo, ex vivo,* or *in vitro* expression.
[21] A method for controlling association of a homomer of a polypeptide, comprising:
   the step of obtaining a nucleic acid encoding the polypeptide; and
   the step of expressing the nucleic acid,
   wherein the polypeptide comprises an Fc region, and
   wherein, because of the modification introduced into the Fc region, the polypeptide associates more readily with a polypeptide having the modification via the Fc region than with a polypeptide comprising an Fc region into which the modification has not been introduced.
[22] A method for promoting the expression of a homomer of a polypeptide, comprising
   the step of obtaining a nucleic acid encoding the polypeptide; and
   the step of expressing the nucleic acid,
   wherein the polypeptide comprises an Fc region, and
   wherein, because of the modification introduced into the Fc region, the polypeptide associates more readily with a polypeptide having the modification via the Fc region than with a polypeptide comprising an Fc region into which the modification has not been introduced.

Furthermore, the present disclosure relates to the following. The technical features set forth in [1]-[22] above are also incorporated into the following inventions as appropriate.
[101] A polypeptide for which association is controlled, wherein the polypeptide comprises an Fc region into which a modification has been introduced, wherein, because of the modification introduced into the Fc region, the polypeptide associates more readily with a polypeptide having the modification via the Fc region than with a polypeptide into which the modification has not been introduced.
[101-1] The aforementioned polypeptide, wherein the polypeptide associates less readily with a polypeptide into which the modification has not been introduced than with a polypeptide having the modification.
[101-1-1] The aforementioned polypeptide, wherein the polypeptide less readily forms a heterodimer with another polypeptide that does not have the modification.
[101-1-2] The aforementioned polypeptide, which less readily forms a heterodimer than a homodimer.
[101-1-3] The aforementioned polypeptide, wherein the polypeptide does not associate, or associates less readily, with endogenous IgG or an antibody fragment containing an Fc region thereof.
[101-2] The aforementioned polypeptide, wherein the polypeptides having the modification associate with each other.
[101-2-1] The aforementioned polypeptide, wherein the polypeptide having the modification forms a homomer.
[101-2-2] The aforementioned polypeptide, which more readily forms a homodimer than a heterodimer.
[101-2-3] The aforementioned polypeptide, which exhibits a stronger homodimerization-promoting ability than a control (e.g., wild-type IgG).
[101-3] The aforementioned polypeptide, wherein the polypeptide having the modification has an increased ability to form an associated multimer with a polypeptide having the modification as compared to a polypeptide that does not have the modification.
[101-3-1] The aforementioned polypeptide, wherein the association is association between Fc regions.
[101-4] The aforementioned polypeptide, wherein the polypeptide has CH3.
[101-4-1] The aforementioned polypeptide, wherein the association is association in an interface between CH3 regions.
[101-4-2] The aforementioned polypeptide, wherein the modification is a modification in CH3.
[102] The aforementioned polypeptide, wherein the polypeptide associates more readily with a polypeptide having the modification via the Fc region than with a polypeptide into which the modification has not been introduced due to at least one of the following actions resulting from the introduced modification: (1) steric complementarity, (2) disulfide linkage, and (3) electrostatic charge.
[102-1] The aforementioned polypeptide, wherein the steric complementarity results from the presence of a knob and a hole in the Fc region.
[102-1-1] The aforementioned polypeptide, wherein the modification is a modification to introduce a knob and a hole into the Fc region.
[102-1-2] The aforementioned polypeptide, wherein the knob and the hole in the Fc region induce the formation of a homodimer of the first polypeptide.
[102-1-3] The aforementioned polypeptide, wherein the knob and the hole are all introduced into the first polypeptide.
[102-2] The aforementioned polypeptide, wherein the disulfide linkage is formed by substituting one or more amino acids in the Fc region with cysteine (C).
[102-2-1] The aforementioned polypeptide, wherein the modification is a modification to introduce one or more cysteines into the Fc region.
[102-2-2] The aforementioned polypeptide, wherein the modification is substitution of one or more amino acids in the Fc region with cysteine.
[102-2-3] The aforementioned polypeptide, wherein the disulfide linkage is a linkage between the cysteines modified (introduced) in the Fc region.
[102-2-4] The aforementioned polypeptide, which associates more readily with a polypeptide having the modification than a polypeptide into which the modification has not been introduced due to at least the combinations of the actions of (2) disulfide linkage and (3) electrostatic charge.
[102-3] The aforementioned polypeptide, wherein the action of the electrostatic charge results from one or more charged amino acids modified (introduced) in the Fc region.
[102-3-1] The aforementioned polypeptide, wherein the introduced charged amino acid(s) is a positively charged amino acid and/or a negatively charged amino acid.
[102-3-2] The aforementioned polypeptide, wherein at least two amino acids in the Fc region are substituted with a positively charged amino acid and a negatively charged amino acid.
[102-3-3] The aforementioned polypeptide, wherein the positively charged amino acid is selected from lysine (K), arginine (R), and histidine (H), and/or the negatively charged amino acid is selected from aspartic acid (D) and glutamic acid (E).
[102-4] The aforementioned polypeptide, wherein the action is (a combination of) more than one action (two or three actions) selected from (1) steric complementarity, (2) disulfide linkage, and (3) electrostatic charge.
[103] The aforementioned polypeptide, wherein the modification introduced into the Fc region is at least one of the modifications set forth in Table 2.
[104] The aforementioned polypeptide, wherein the modification introduced into the Fc region is at least one of the modifications set forth in Table 4.
[105] The aforementioned polypeptide, wherein the steric complementarity comprises knob-into-hole.
[106] The aforementioned polypeptide, wherein the polypeptide is an antibody.
[107] The aforementioned polypeptide, wherein the Fc region is an Fc region of IgG.
[108] The aforementioned polypeptide, wherein the Fc region is derived from any one of IgG1, 2, 3, and 4.
[201] A method for producing a polypeptide for which association between Fc regions is controlled, comprising:
   (a) obtaining a nucleic acid encoding a polypeptide into which a modification has been introduced in the Fc region, wherein the polypeptide associates more readily with a polypeptide having the modification than with a polypeptide into which the modification has not been introduced due to at least one of the following actions: (1) steric complementarity, (2) disulfide linkage, and (3) electrostatic charge;
   (b) introducing the nucleic acid into a host cell and culturing the host cell to express the nucleic acid; and
   (c) recovering the polypeptide from the culture of the host cell.
[201-1] The aforementioned method, which further comprises introducing a modification into the Fc region such that the action of (1) steric complementarity, (2) disulfide linkage, or (3) electrostatic charge occurs in the polypeptide.
[202-2] The aforementioned method, wherein the modification introduced into the Fc region is at least one of the modifications set forth in Table 2 or Table 4.
[202] A method for producing a polypeptide for which association between Fc regions is controlled, comprising:
   (a) modifying a nucleic acid encoding a polypeptide comprising an Fc region such that the polypeptide associates more readily with a polypeptide having the modification than with a polypeptide into which the modification has not been introduced due to at least one of the following actions: (1) steric complementarity, (2) disulfide linkage, and (3) electrostatic charge;
   (b) introducing the modified nucleic acid into a host cell and culturing the host cell to express the nucleic acid; and
   (c) recovering the polypeptide from the culture of the host cell.
[203] A method for controlling association between polypeptides comprising an Fc region, comprising modifying the polypeptide such that the polypeptide associates more readily with a polypeptide having the modification than with a polypeptide into which the modification has not been introduced due to at least one of the following actions: (1) steric complementarity, (2) disulfide linkage, and (3) electrostatic charge.
[204] A method for generating in a living organism a polypeptide for which association between polypeptides comprising an Fc region is controlled, comprising:
   (a) preparing a nucleic acid encoding a polypeptide comprising an Fc region such that the polypeptide comprising an Fc region is modified into a polypeptide that associates more readily with a polypeptide having the modification than with a polypeptide into which the modification has not been introduced due to at least one of the following actions: (1) steric complementarity, (2) disulfide linkage, and (3) electrostatic charge; and
   (b) introducing the nucleic acid of step (a) into a living organism.
[204-1] The aforementioned method, which comprises introducing a modification into the Fc region such that the action of (1) steric complementarity, (2) disulfide linkage, or (3) electrostatic charge occurs in the polypeptide.
[204-2] The aforementioned method, wherein the nucleic acid is included in a vesicle.
[204-3] The aforementioned method, which comprises contacting the nucleic acid with a vesicle.
[204-4] The aforementioned method, wherein the vesicle is a lipid nanoparticle (LNP), a virus, an extracellular vesicle (EV), or a liposome.
[204-5] The aforementioned method, wherein the nucleic acid is an RNA, a DNA, or a vector or plasmid carrying the nucleic acid.
[204-6] The aforementioned method, wherein the RNA is a mRNA, a genomic RNA, or a circular RNA.
[204-7] The aforementioned method, wherein the polypeptide comprising an Fc region is an antibody.
[204-8] The aforementioned method, wherein the polypeptide comprising an Fc region is a single-domain antibody.
[204-9] The aforementioned method, wherein the polypeptide comprising an Fc region is IgG.
[204-10] The aforementioned method, wherein the polypeptide comprising an Fc region does not associate with endogenous IgG.
[204-11] The aforementioned method, wherein the polypeptide comprising an Fc region retains a function of the Fc of wild-type IgG.
[204-12] The aforementioned method, which comprises administering the nucleic acid to a subject.
[204-13] The aforementioned method, wherein the subject is a human.
[204-14] The aforementioned method, wherein the polypeptide comprising an Fc region is a humanized antibody or a human antibody.
[205] A method for expressing in a cell a polypeptide for which association between polypeptides comprising an Fc region is controlled, comprising:
   (a) preparing a nucleic acid encoding a polypeptide comprising an Fc region such that the polypeptide comprising an Fc region is modified into a polypeptide that associates more readily with a polypeptide having the modification than with a polypeptide into which the modification has not been introduced due to at least one of the following actions: (1) steric complementarity, (2) disulfide linkage, and (3) electrostatic charge; and
   (b) introducing the nucleic acid of step (a) into a cell.
[205-1] The aforementioned method, which comprises introducing a modification into the Fc region such that the action of (1) steric complementarity, (2) disulfide linkage, or (3) electrostatic charge occurs in the polypeptide.
[205-2] The aforementioned method, wherein the nucleic acid is an RNA, a DNA, or a vector or plasmid carrying the nucleic acid.
[205-3] The aforementioned method, wherein the RNA is a mRNA, a genomic RNA, or a circular RNA.
[205-4] The aforementioned method, wherein the polypeptide comprising an Fc region is an antibody.
[205-5] The aforementioned method, wherein the polypeptide comprising an Fc region is a single-domain antibody.
[205-6] The aforementioned method, wherein the polypeptide comprising an Fc region is IgG.
[205-7] The aforementioned method, wherein the cell is a human cell.
[205-8] The aforementioned method, which is an *in vivo, ex vivo,* or *in vitro* method.
[206] A method for expressing in a cell two or more types of polypeptides comprising an Fc region, wherein association between polypeptides of the same type is controlled:
   (a) preparing a nucleic acid encoding a polypeptide comprising an Fc region such that a first polypeptide comprising an Fc region is modified into a polypeptide that associates more readily with a polypeptide having the modification than with a polypeptide into which the modification has not been introduced due to at least one of the following actions: (1) steric complementarity, (2) disulfide linkage, and (3) electrostatic charge; and
   (b) preparing a nucleic acid encoding a polypeptide comprising an Fc region such that a second polypeptide comprising an Fc region is modified into a polypeptide that associates more readily with a polypeptide having the modification than with a polypeptide into which the modification has not been introduced due to at least one of the following actions: (1) steric complementarity, (2) disulfide linkage, and (3) electrostatic charge; wherein the modification in the second polypeptide is different from the modification in the first polypeptide.
[206-1] The aforementioned method, wherein two or more types of polypeptides comprising an Fc region are expressed, and polypeptides of the same type are allowed to form a homomer.
[206-2] The aforementioned method, wherein the modifications in the two or more types of polypeptides to be expressed are different from each other.
[206-3] The aforementioned method, wherein the combination of the modifications different from each other is a combination shown in Table 7.
[207] A method for controlling association of two or more types of polypeptides comprising an Fc region, wherein association between polypeptides of the same type is controlled, wherein the two or more types of polypeptides are modified such that they associate more readily with a polypeptide having the modification than with a polypeptide into which the modification has not been introduced due to at least one of the following actions: (1) steric complementarity, (2) disulfide linkage, and (3) electrostatic charge, and further wherein the modifications in the two or more types of polypeptides are different from each other.
[207-1] The aforementioned method, wherein the combination of the modifications different from each other is a combination shown in Table 7.
[208] A method for producing a pharmaceutical composition comprising a vesicle including a nucleic acid encoding a polypeptide for which association between Fc regions is controlled, comprising:
   (a) obtaining a nucleic acid encoding a polypeptide into which a modification has been introduced in the Fc region, wherein the polypeptide associates more readily with a polypeptide having the modification than with a polypeptide into which the modification has not been introduced due to at least one of the following actions: (1) steric complementarity, (2) disulfide linkage, and (3) electrostatic charge; and
   (b) contacting the nucleic acid of step (a) with a vesicle.
[208-1] The aforementioned method, wherein the vesicle is a lipid nanoparticle (LNP), a virus, an extracellular vesicle (EV), or a liposome.
[208-2] The aforementioned method, wherein a pharmaceutically acceptable carrier is further contacted.
[208-3] The aforementioned method, wherein the nucleic acid is an RNA, a DNA, or a vector or plasmid carrying the nucleic acid.
[301] A pharmaceutical composition comprising the aforementioned nucleic acid or the aforementioned polypeptide, and a pharmaceutically acceptable carrier.
[301-1] A pharmaceutical composition comprising a nucleic acid encoding a polypeptide comprising an Fc region for which association is controlled.
[301-2] The aforementioned pharmaceutical composition, which comprises the nucleic acid included (encapsulated) in a vesicle.
[301-3] A pharmaceutical composition comprising a nucleic acid encoding a polypeptide comprising an Fc region for which association is controlled, and a vesicle.
[301-4] The aforementioned pharmaceutical composition, wherein the vesicle is a lipid nanoparticle (LNP), a virus, an extracellular vesicle (EV), or a liposome.
[301-5] The aforementioned pharmaceutical composition, wherein the nucleic acid is an RNA, a DNA, or a vector or plasmid carrying the nucleic acid.
[301-6] The aforementioned pharmaceutical composition, which is for generating in a living organism a polypeptide for which association between Fc regions is controlled.
[301-7] The aforementioned pharmaceutical composition, which is for expressing in a cell a polypeptide for which association between Fc regions is controlled.

In one embodiment, the present disclosure relates to the following:
[Embodiment 1]
   A nucleic acid encoding a first polypeptide,
   wherein the first polypeptide comprises an Fc region into which a modification has been introduced,
   wherein, because of the modification introduced into the Fc region, the first polypeptide associates more readily with a first polypeptide having the modification via the Fc region than with a first polypeptide comprising an Fc region into which the modification has not been introduced.
[Embodiment 2]
   The nucleic acid of Embodiment 1, wherein the first polypeptide associates more readily with a first polypeptide having the modification via the Fc region than with a first polypeptide comprising an Fc region into which the modification has not been introduced due to at least one of the following actions resulting from the introduced modification: (1) steric complementarity, (2) disulfide linkage, and (3) electrostatic charge.
[Embodiment 3]
   A composition comprising:
   the nucleic acid of present disclosure 1; and
   a nucleic acid encoding a second polypeptide,
   wherein the second polypeptide comprises an Fc region into which a modification has been introduced,
   wherein, because of the modification introduced into the Fc region, the second polypeptide associates more readily with a second polypeptide having the modification via the Fc region than with a second polypeptide comprising an Fc region into which the modification has not been introduced.
[Embodiment 4]
   A composition comprising:
   the nucleic acid of Embodiment 1;
   a nucleic acid encoding a second polypeptide; and
   a nucleic acid encoding a third polypeptide,
   wherein the second polypeptide associates more readily with the third polypeptide than with the second polypeptide.
[Embodiment 5]
   A composition comprising:
   the nucleic acid of Embodiment 2; and
   a nucleic acid encoding a second polypeptide,
   wherein the second polypeptide does not have the modification the first polypeptide has.
[Embodiment 6]
   The composition of Embodiment 3, wherein the modification introduced into the first polypeptide is different from the modification introduced into the second polypeptide.
[Embodiment 7]
   The composition of Embodiment 3, wherein the second polypeptide associates more readily with a second polypeptide having the modification via the Fc region than with a second polypeptide comprising an Fc region into which the modification has not been introduced due to at least one of the following actions resulting from the introduced modification: (1) steric complementarity, (2) disulfide linkage, and (3) electrostatic charge.
[Embodiment 8]
   The nucleic acid of Embodiment 1, wherein the modification introduced into the Fc region is at least one of the modifications set forth in Table 2.
[Embodiment 9]
   The nucleic acid of Embodiment 1, wherein the modification introduced into the Fc region is at least one of the modifications set forth in Table 4.
[Embodiment 10]
   The nucleic acid of Embodiment 6, wherein the modification introduced into the first polypeptide and the modification introduced into the second polypeptide are at least one of the combinations of modifications set forth in Table 6.
[Embodiment 11]
   The nucleic acid of Embodiment 1, wherein the polypeptide is an antibody.
[Embodiment 12]
   The nucleic acid of Embodiment 1, wherein the Fc region is an Fc region of IgG.
[Embodiment 13]
   The nucleic acid of Embodiment 1, wherein the polypeptide into which the modification has been introduced retains a function of the polypeptide prior to modification.
[Embodiment 14]
   The nucleic acid of Embodiment 12, wherein the Fc region into which the modification has been introduced retains a function of an Fc of IgG.
[Embodiment 15]
   The nucleic acid of Embodiment 1, wherein the Fc region is derived from any one of IgG1, 2, 3, and 4.
[Embodiment 16]
   A host cell into which the nucleic acid of any one of Embodiments 1, 2, and 8-14 or the composition of any one of Embodiments 3-7 has been introduced.
[Embodiment 17]
   A polypeptide expressed from the nucleic acid of Embodiment 1.
[Embodiment 18]
   A composition comprising a nucleic acid encoding a polypeptide, wherein the polypeptide comprises an Fc region into which a modification has been introduced, wherein the modification is at least one of the modifications set forth in Table 2.
[Embodiment 19]
   A polypeptide which comprises an Fc region into which a modification has been introduced, wherein the polypeptide associates more readily with a polypeptide having the modification via the Fc region than with a polypeptide comprising an Fc region into which the modification has not been introduced due to at least one of the following actions resulting from the introduced modification: (1) steric complementarity, (2) disulfide linkage, and (3) electrostatic charge.
[Embodiment 20]
   A method for obtaining a polypeptide for which association is controlled, comprising:
   obtaining a nucleic acid encoding the polypeptide; and
   expressing the nucleic acid,
   wherein the polypeptide comprises an Fc region, and
   wherein, because of the modification introduced into the Fc region, the polypeptide associates more readily with a polypeptide having the modification via the Fc region than with a polypeptide comprising an Fc region into which the modification has not been introduced.
[Embodiment 21]
   A method for controlling association of a homomer of a polypeptide, comprising:
   obtaining a nucleic acid encoding the polypeptide; and
   expressing the nucleic acid,
   wherein the polypeptide comprises an Fc region, and
   wherein, because of the modification introduced into the Fc region, the polypeptide associates more readily with a polypeptide having the modification via the Fc region than with a polypeptide comprising an Fc region into which the modification has not been introduced.
[Embodiment 22]
   A method for promoting expression of a homomer of a polypeptide, comprising:
   obtaining a nucleic acid encoding the polypeptide; and
   expressing the nucleic acid,
   wherein the polypeptide comprises an Fc region, and
   wherein, because of the modification introduced into the Fc region, the polypeptide associates more readily with a polypeptide having the modification via the Fc region than with a polypeptide comprising an Fc region into which the modification has not been introduced.
[Embodiment 23]
   The composition of Embodiment 4, wherein the second polypeptide comprises an Fc region into which a modification has been introduced,
   wherein, because of the modification introduced into the Fc region, the second polypeptide associates more readily with the third polypeptide via the Fc region than with the second polypeptide.
[Embodiment 24]
   The composition of Embodiment 1, wherein the Fc region into which the modification has been introduced comprises an amino acid modification at one combination or two or more combinations of positions selected from the combinations of positions shown in (a) to (d) below according to EU numbering:
   (a) positions 394 and 405;
   (b) positions 366, 368, and 407;
   (c) positions 347, 360, 399, 405 and 409; and
   (d) positions 356, 392, 399, and 439.
[Embodiment 25]
   The composition of Embodiment 24, wherein the Fc region into which the modification has been introduced comprises at least one amino acid selected from the group consisting of:
   (a) W, F, or Y at position 394, and
      A, S, T, C, G, or V at position 405;
   (b) W, Y, or F at position 366,
      A, S, T, C, V, or G at position 368, and
      V, L, I, M, A, S, T, C, N, or Q at position 407;
   (c) R, K, Y, or H at position 347,
      E or D at position 360,
      V, L, I, M, S, T, C, H, A, N, Q, or G at position 399,
      T, A, V, S, C, N, D, or G at position 405, and
      W, F, Y, or H at position 409; and
   (d) K or R at position 356,
      D or E at position 392,
      K or R at position 399, and
      E or D at position 439,
   according to EU numbering.
[Embodiment 26]
   The composition of Embodiment 24, wherein the Fc region into which the modification has been introduced comprises at least one amino acid selected from the group consisting of:
   (a) W or F at position 394, and
      A, S, T, or G at position 405;
   (b) W, Y, or F at position 366,
      A, T, C, V, or G at position 368, and
      V, L, I, M, A, or C at position 407;
   (c) R, K, Y, or H at position 347
      E or D at position 360
      V, L, I, M, S, T, C, H, A, N, or G at position 399,
      T, A, or V at position 405, and
      W, F, or Y at position 409; and
   (d) K at position 356,
      D at position 392,
      K at position 399, and
      E at position 439,
   according to EU numbering.
[Embodiment 27]
   The nucleic acid of Embodiment 1, wherein the modification introduced into the Fc region is at least one of the modifications set forth in Table 15.
[Embodiment 28]
   The nucleic acid of Embodiment 1, wherein the modification introduced into the Fc region is at least one of the modifications set forth in Table 17.

In one embodiment, the present disclosure relates to the following:
[Embodiment 29]
   The composition of Embodiment 1, wherein the Fc region into which the modification has been introduced comprises amino acid modifications at one combination or two or more combinations of positions selected from the combinations of positions shown in (a) to (d) below according to EU numbering:
   (a) positions 345, 347, 360, 366, 399, 407, and 409;
   (b) positions 356, 399, 409, and 439;
   (c) positions 356, 392, 399, and 409; and
   (d) positions 392, 399, and 409.
[Embodiment 30]
   The composition of Embodiment 29, wherein the Fc region into which the modification has been introduced comprises at least one amino acid selected from the group consisting of:
   (a) R or E at position 345,
      R or K at position 347,
      D or E at position 360,
      V at position 366,
      M, Q, N, H, I, F, Y, T, S, V, or L at position 399,
      A at position 407, and
      V, Q, N, H, L, I, F, Y, T, or S at position 409;
   (b) K, R, or H at position 356,
      K, R, or H at position 399,
      E or D at position 409, and
      E or D at position 439,
   (c) K, R, or H at position 356,
      D or E at position 392,
      K, R, or H at position 399, and
      D or E at position 409; and
   (d) D or E at position 392,
      K or R at position 399, and
      D or E at position 409,
   according to EU numbering.

### [Brief Description of Drawings]

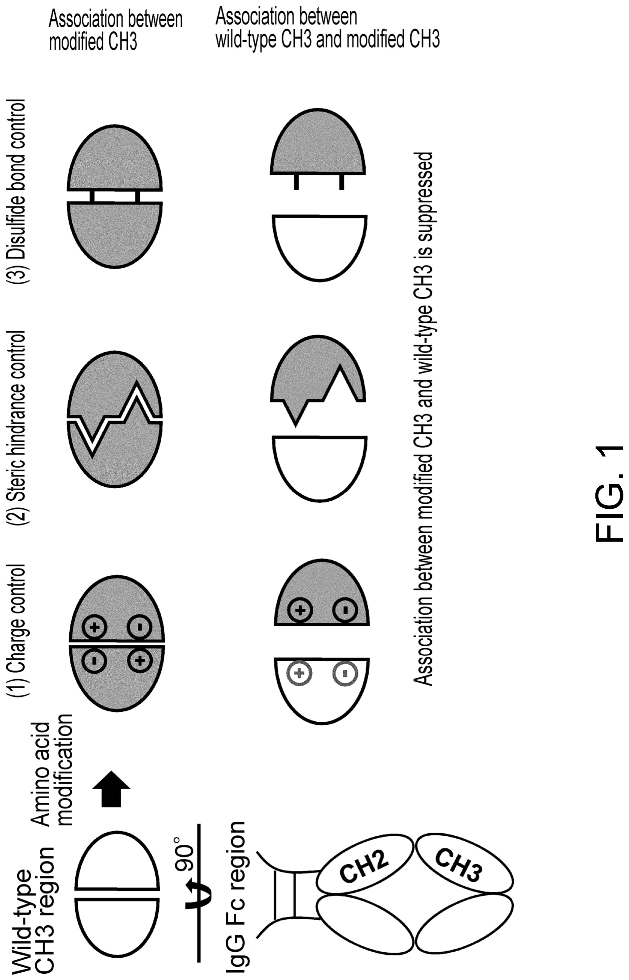

Fig. 1 shows the concept of the control of CH3 interface association which promotes homodimer formation of heavy chains. (1) Amino acid modifications with charges that cause modified CH3 regions to have an attractive force with each other but a repulsive force with wild-type CH3. (2) Amino acid modifications for producing modified CH3 with a protuberance and a cavity, where modified CH3 regions can bind with each other but cannot associate with wild-type CH3 due to steric hindrance. (3) Amino acid modifications that allow multiple artificial disulfide bonds to be formed between modified CH3 regions. Association with wild-type CH3 is suppressed.
Fig. 2 shows a method of screening for heavy chain homodimerization-promoting modifications by SEC analysis.
Fig. 3 (Fig. 3-1 to Fig. 3-47) shows the evaluation of heavy chain association of antibodies by CIEX analysis. In Fig. 3-1, one antibody without CH3 modification is analyzed.
In Fig. 3-2, one antibody with CH3 modification is analyzed.
In Fig. 3-3, one antibody without CH3 modification is analyzed.

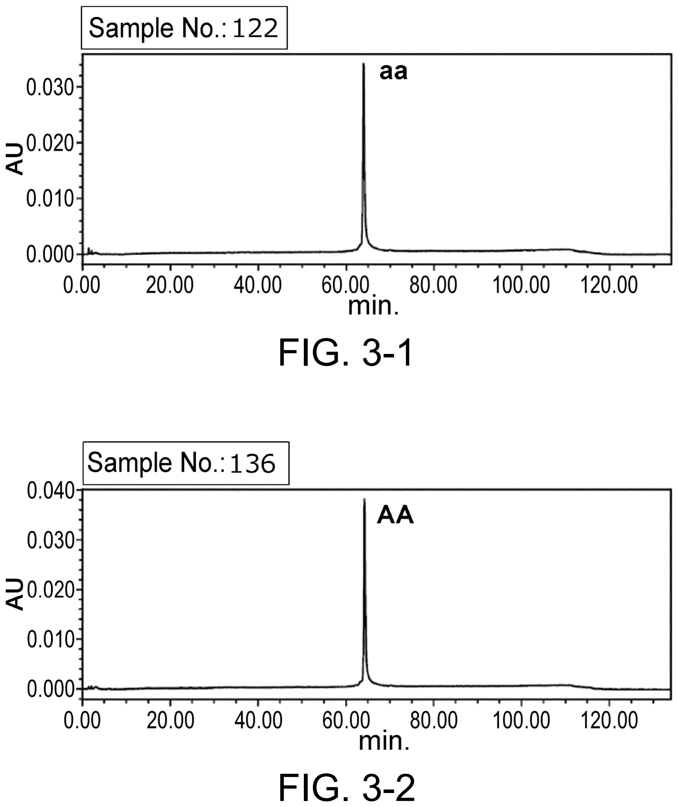

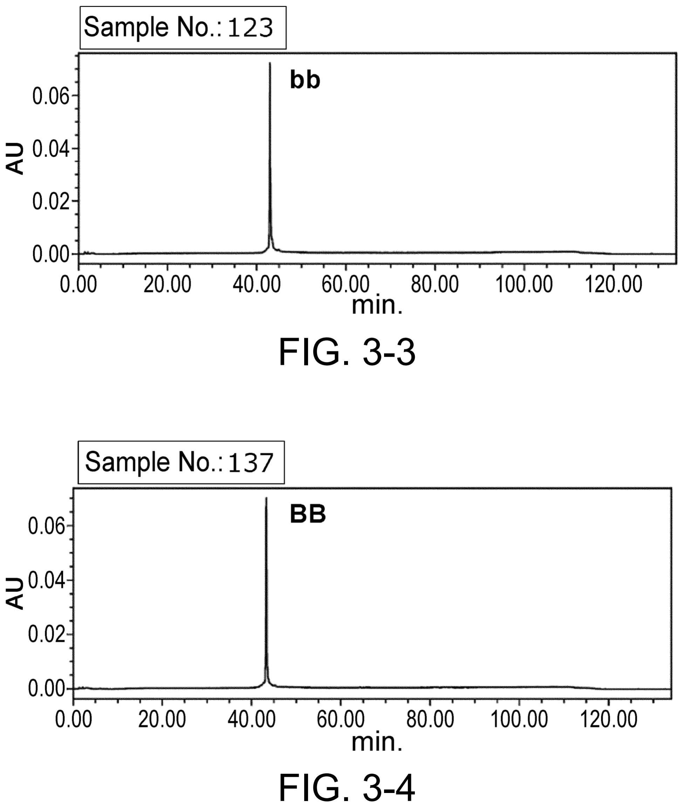

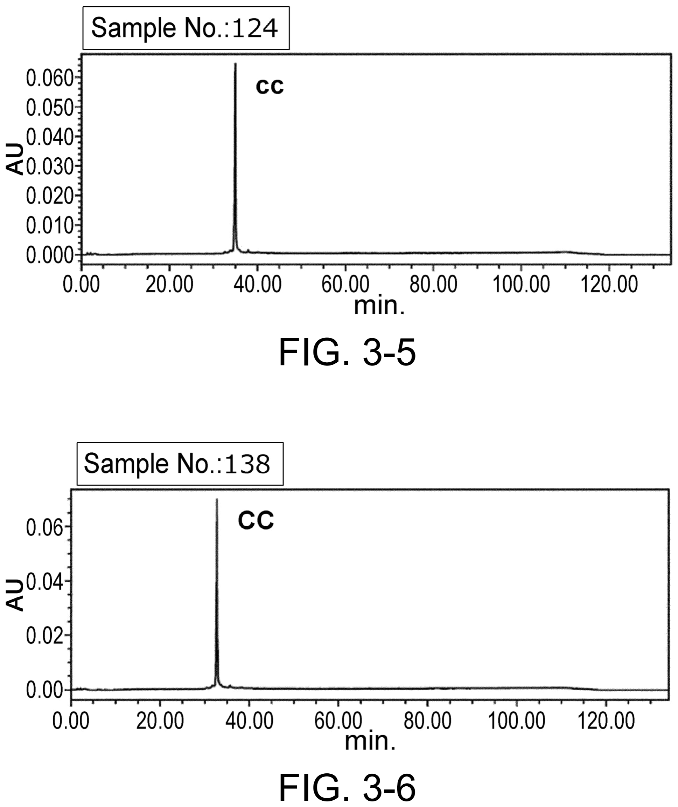

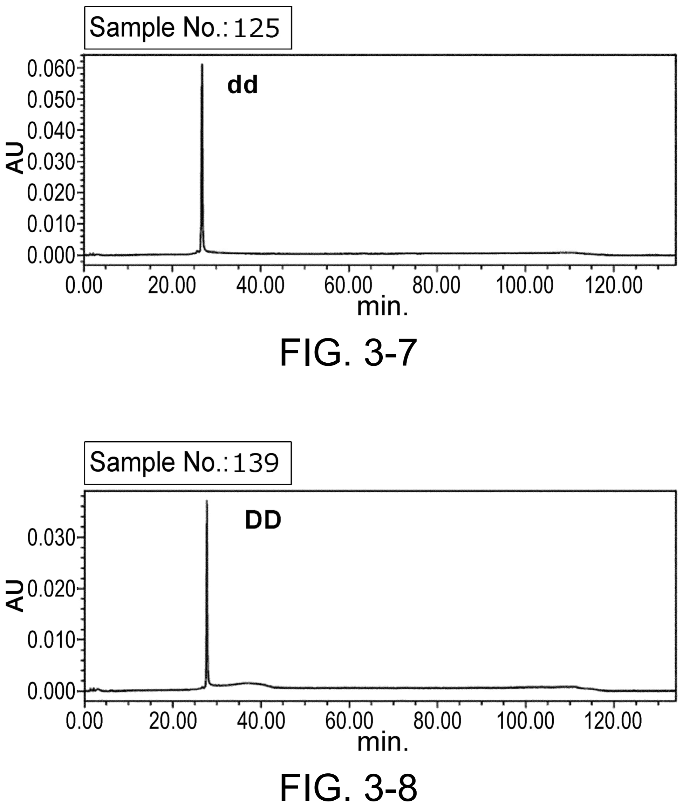

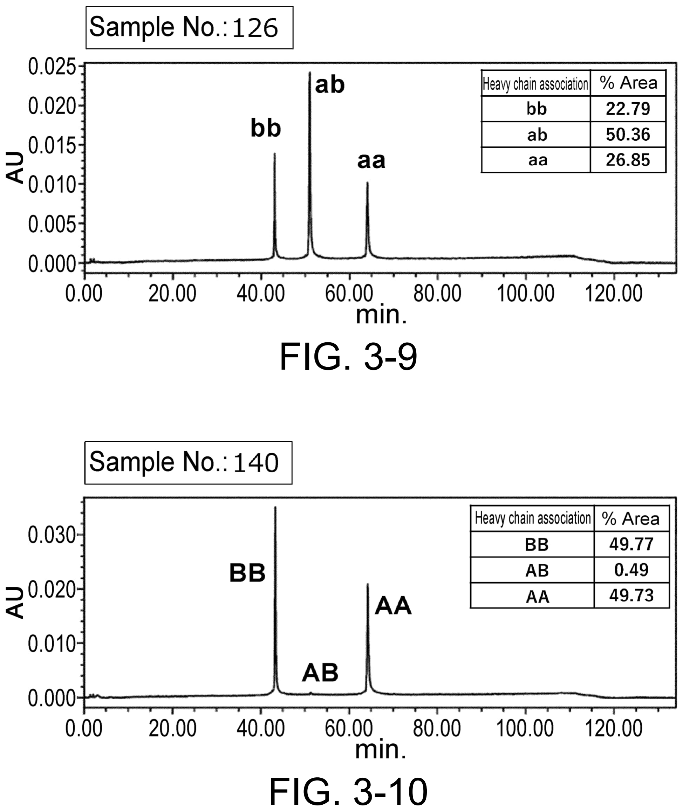

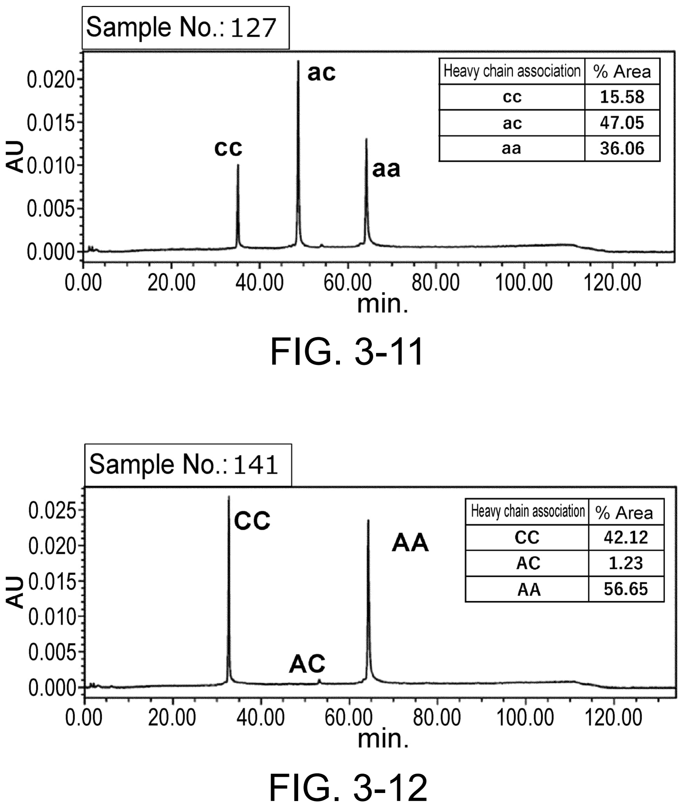

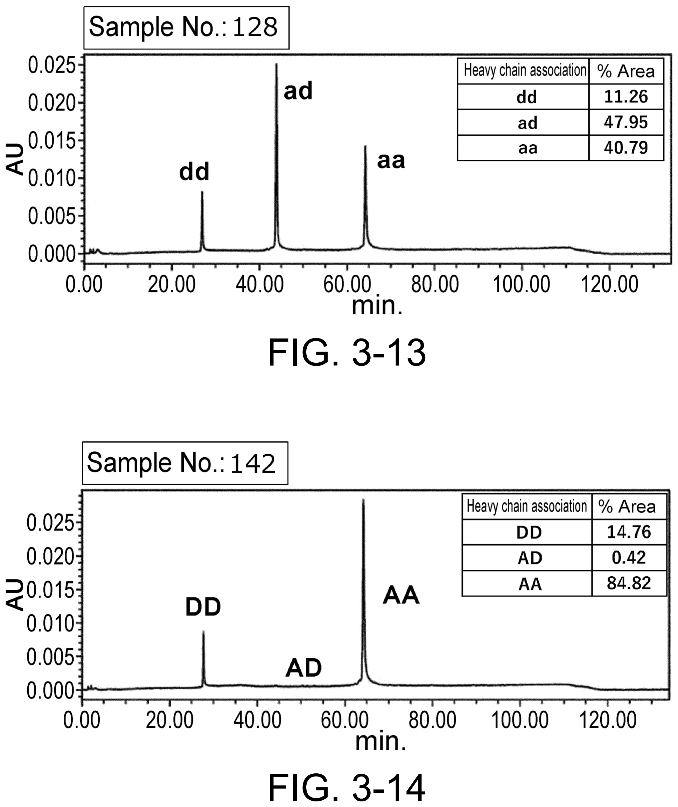

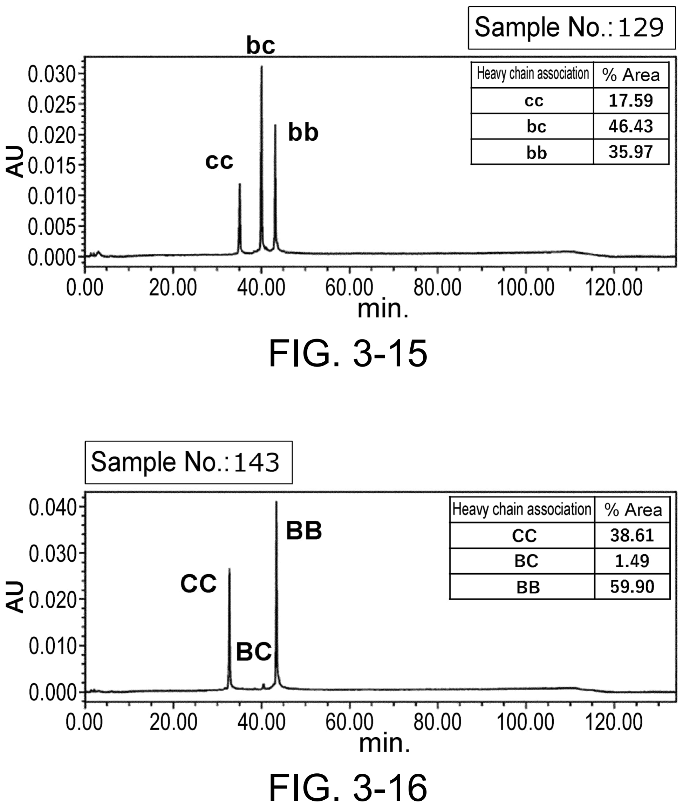

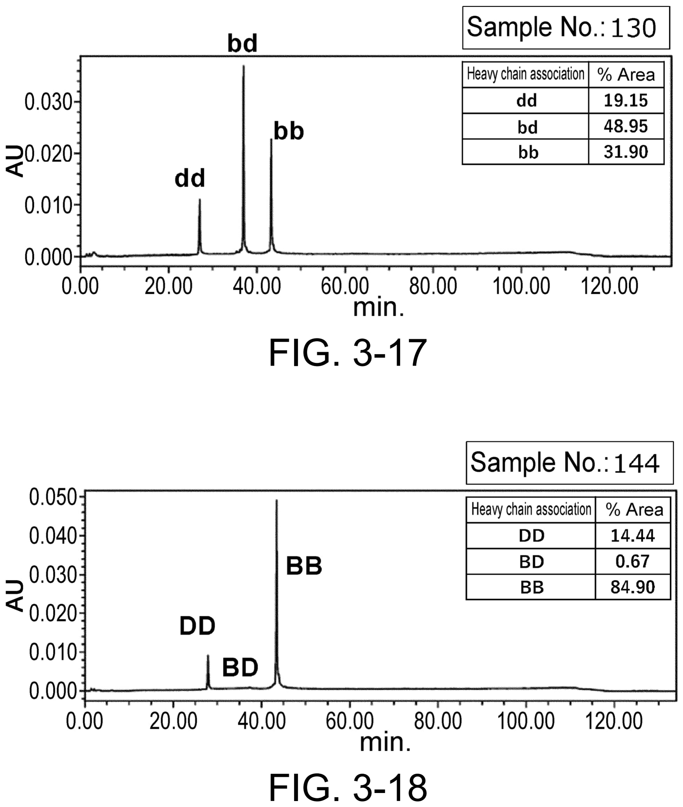

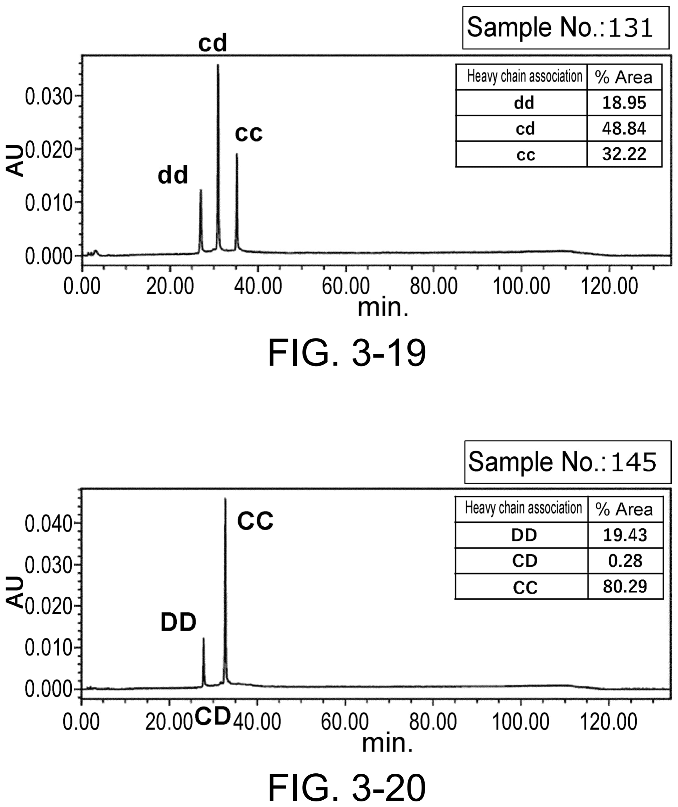

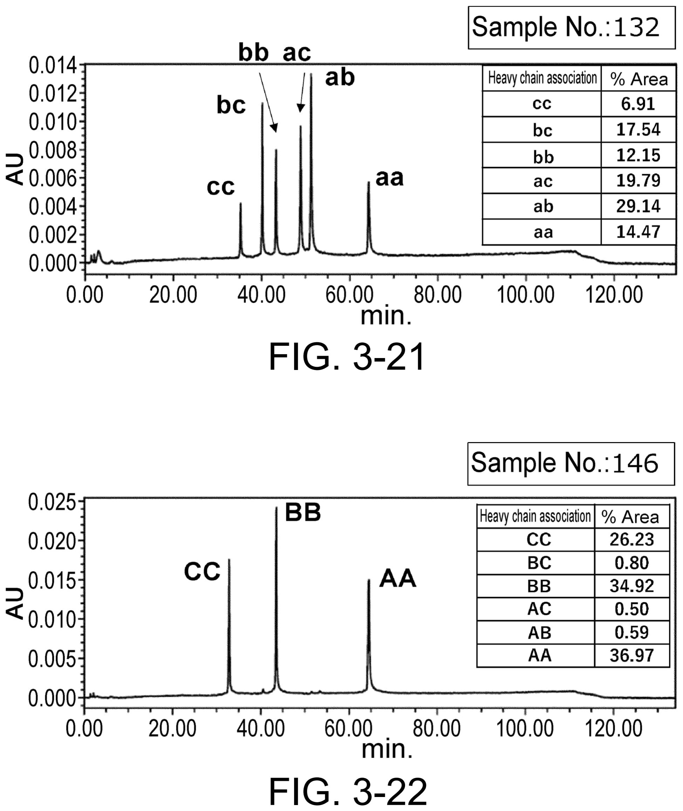

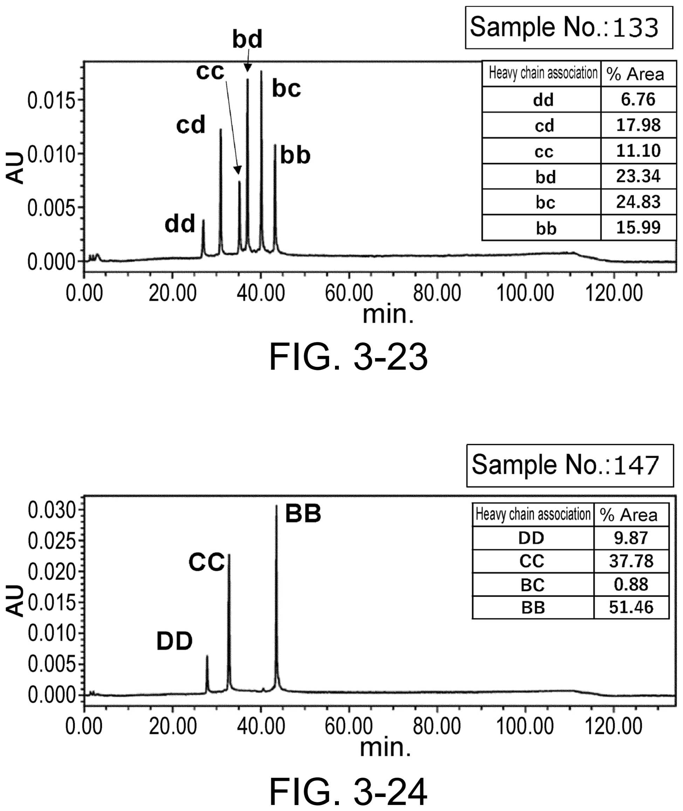

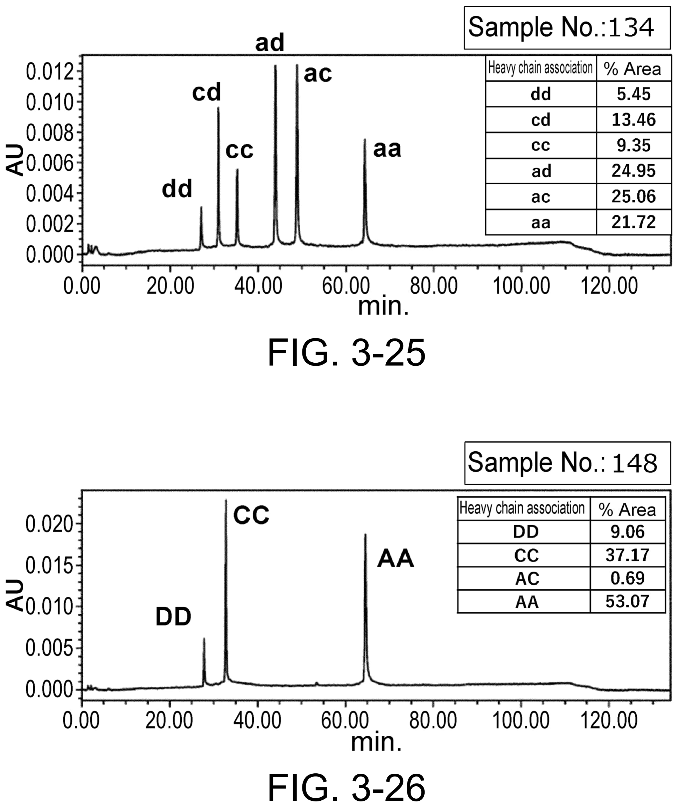

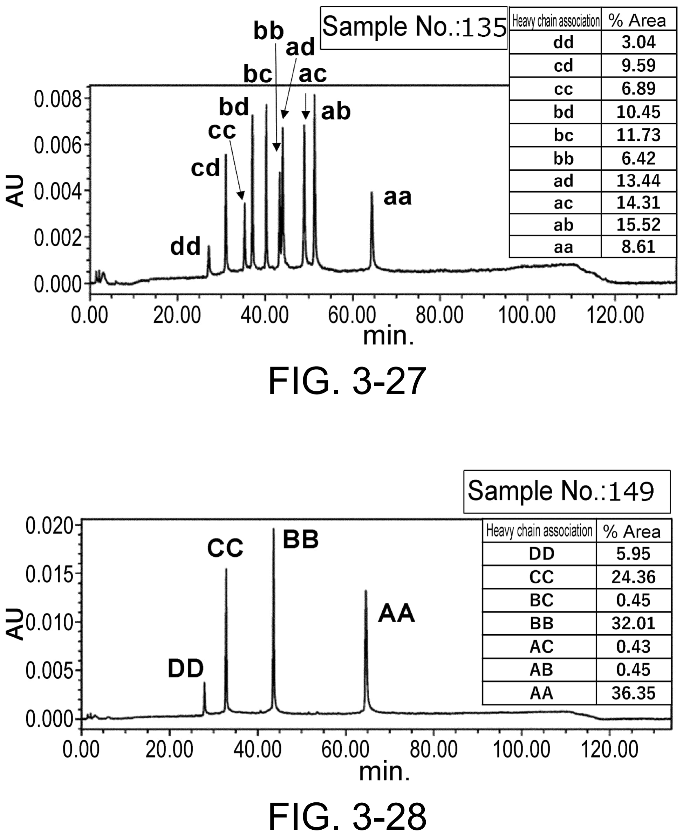

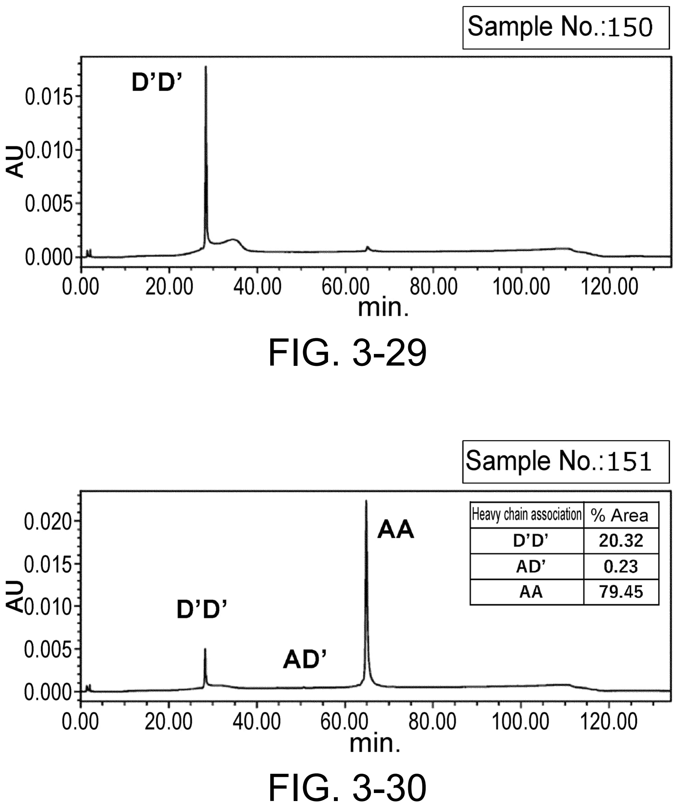

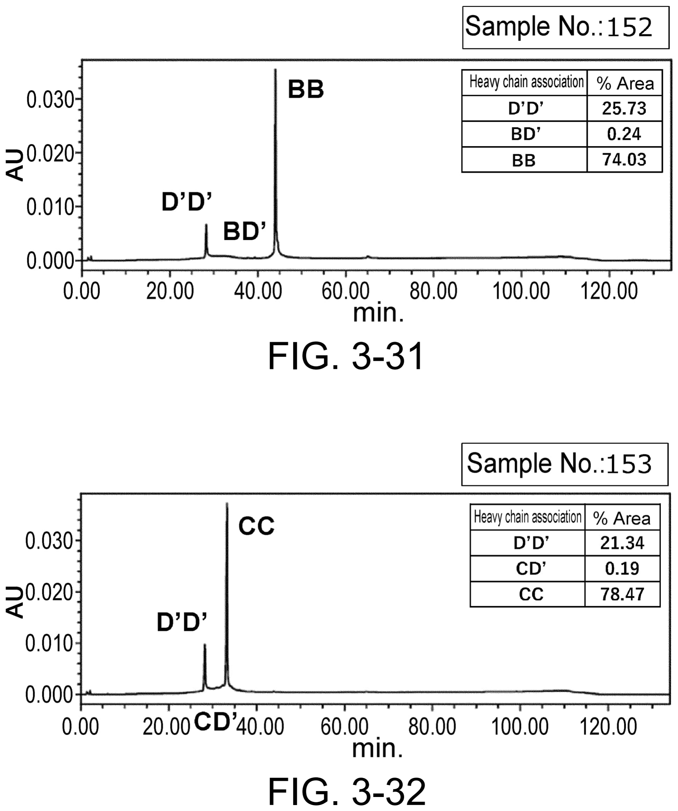

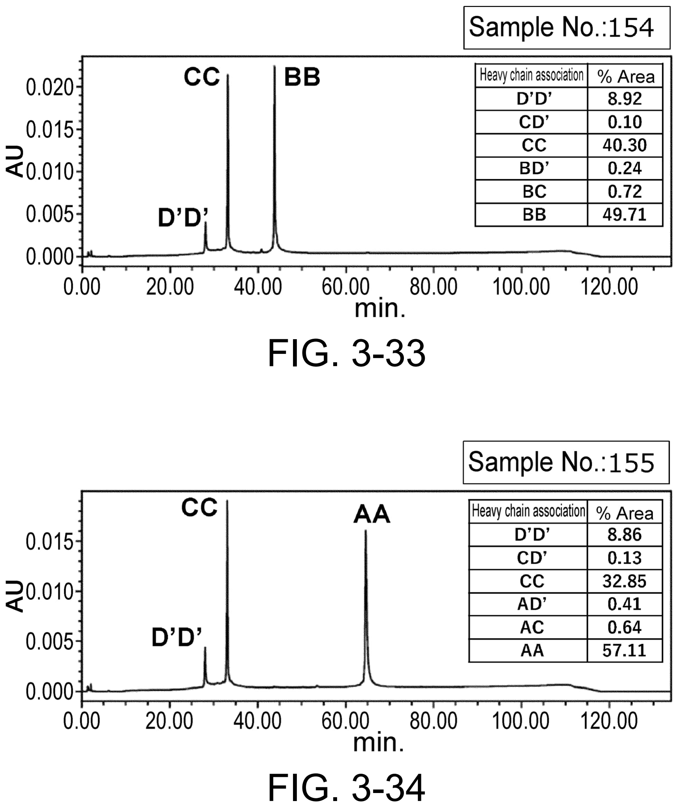

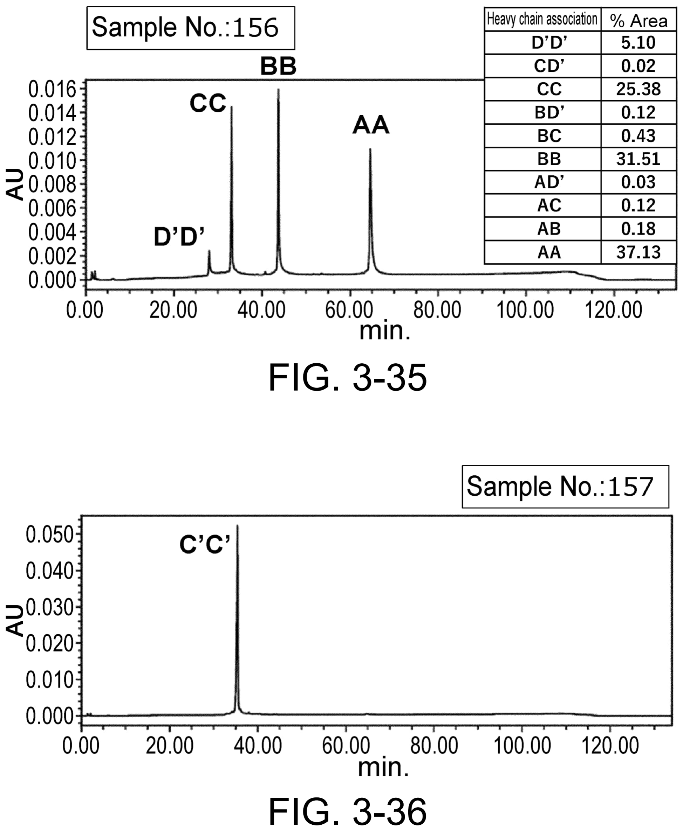

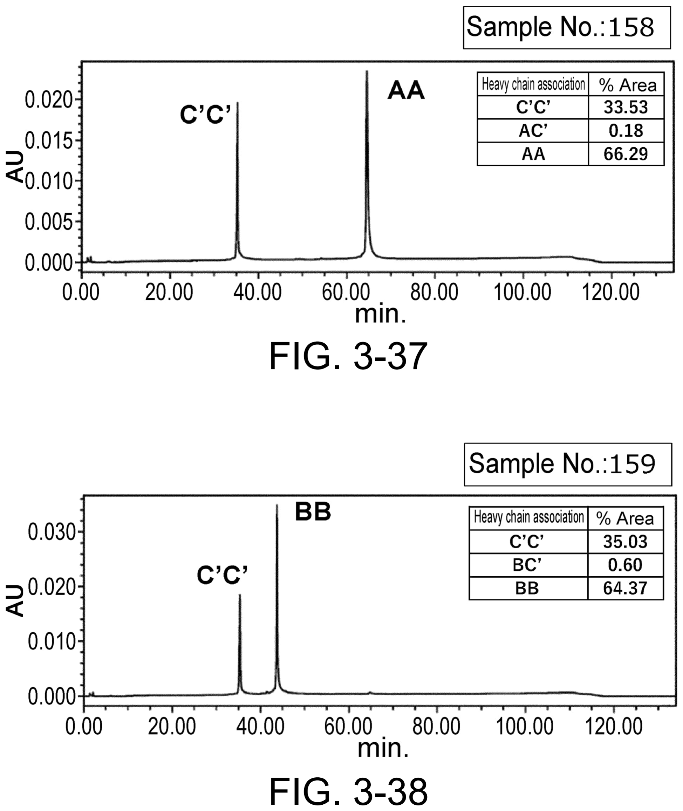

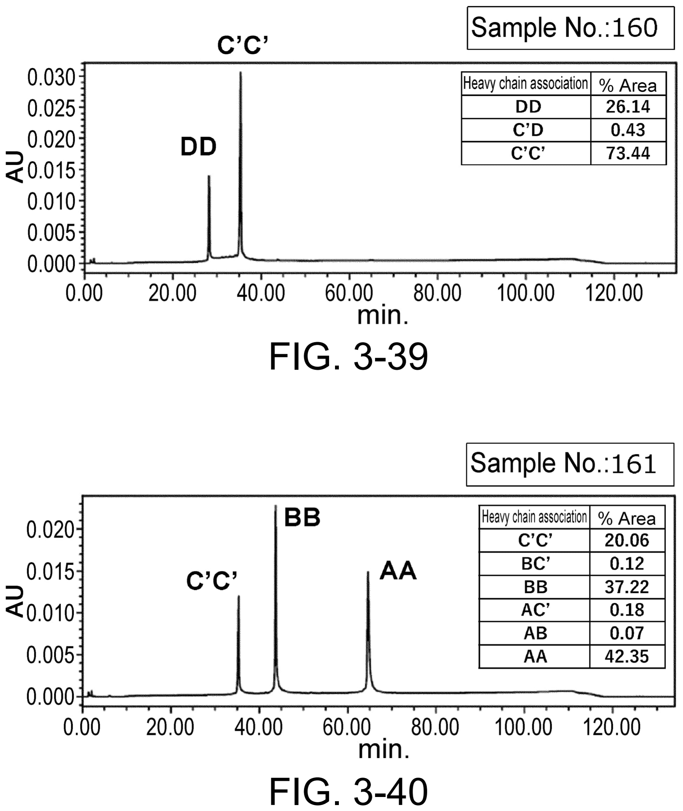

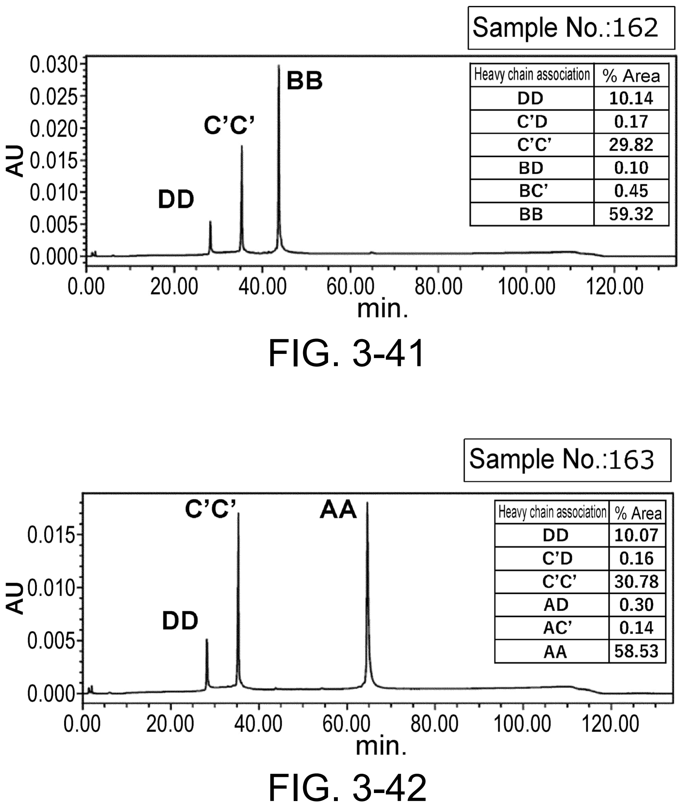

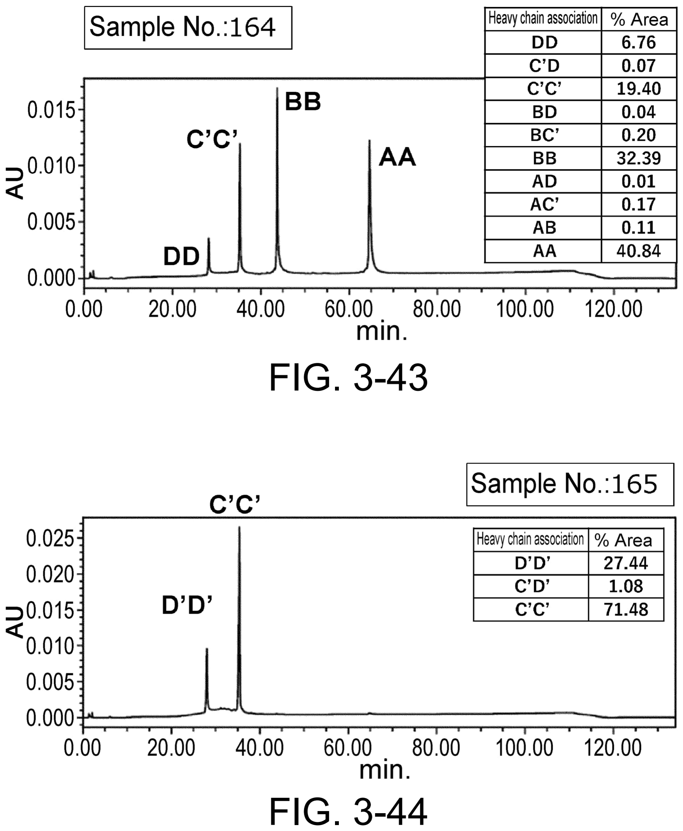

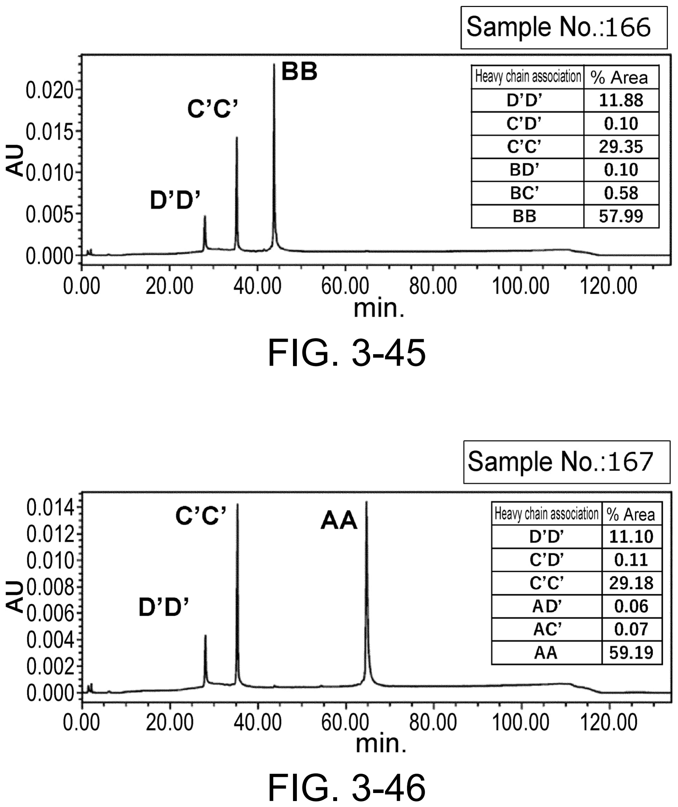

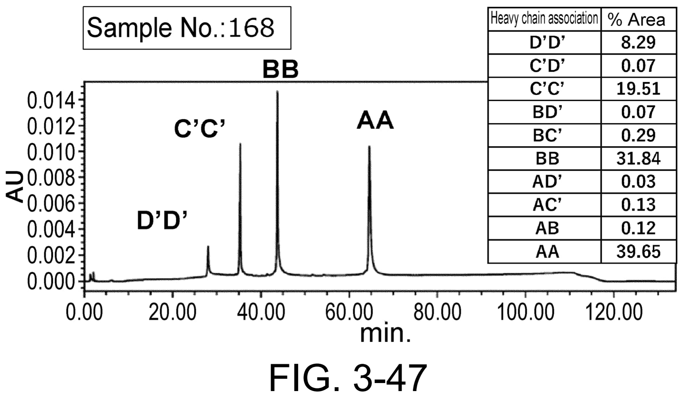

In Fig. 3-4, one antibody with CH3 modification is analyzed.
In Fig. 3-5, one antibody without CH3 modification is analyzed.
In Fig. 3-6, one antibody with CH3 modification is analyzed.
In Fig. 3-7, one antibody without CH3 modification is analyzed.
In Fig. 3-8, one antibody with CH3 modification is analyzed.
Fig. 3-9 shows the result of analysis in coexpression of two antibodies without CH3 modification. Each peak position was identified and assigned based on the elution position observed when expressing each one antibody.
Fig. 3-10 shows the result of analysis in coexpression of two antibodies with CH3 modification. Each peak position was identified and assigned based on the eluted position observed when expressing each one antibody.
Fig. 3-11 shows the result of analysis in coexpression of two antibodies without CH3 modification. Each peak position was identified and assigned based on the eluted position observed when expressing each one antibody.
Fig. 3-12 shows the result of analysis in coexpression of two antibodies with CH3 modification. Each peak position was identified and assigned based on the elution position observed when expressing each one antibody.
Fig. 3-13 shows the result of analysis in coexpression of two antibodies without CH3 modification. Each peak position was identified and assigned based on the elution position observed when expressing each one antibody.
Fig. 3-14 shows the result of analysis in coexpression of two antibodies with CH3 modification. Each peak position was identified and assigned based on the elution position observed when expressing each one antibody.
Fig. 3-15 shows the result of analysis in coexpression of two antibodies without CH3 modification. Each peak position was identified and assigned based on the elution position observed when expressing each one antibody.
Fig. 3-16 shows the result of analysis in coexpression of two antibodies with CH3 modification. Each peak position was identified and assigned based on the elution position observed when expressing each one antibody.
Fig. 3-17 shows the result of analysis in coexpression of two antibodies without CH3 modification. Each peak position was identified and assigned based on the elution position observed when expressing each one antibody.
Fig. 3-18 shows the result of analysis in coexpression of two antibodies with CH3 modification. Each peak position was identified and assigned based on the elution position observed when expressing each one antibody.
Fig. 3-19 shows the result of analysis in coexpression of two antibodies without CH3 modification. Each peak position was identified and assigned based on the elution position observed when expressing each one antibody.
Fig. 3-20 shows the result of analysis in coexpression of two antibodies with CH3 modification. Each peak position was identified and assigned based on the elution position observed when expressing each one antibody.
Fig. 3-21 shows the result of analysis in coexpression of three antibodies without CH3 modification. Each peak was assigned with reference to the peak positions observed when expressing one antibody or coexpressing two antibodies.
Fig. 3-22 shows the result of analysis in coexpression of three antibodies with CH3 modification. Each peak was assigned with reference to the peak positions observed when expressing one antibody or coexpressing two antibodies.
Fig. 3-23 shows the result of analysis in coexpression of three antibodies without CH3 modification. Each peak was assigned with reference to the peak positions observed when expressing one antibody or coexpressing two antibodies.
Fig. 3-24 shows the result of analysis in coexpression of three antibodies with CH3 modification. Each peak was assigned with reference to the peak positions observed when expressing one antibody or coexpressing two antibodies.
Fig. 3-25 shows the result of analysis in coexpression of three antibodies without CH3 modification. Each peak was assigned with reference to the peak positions observed when expressing one antibody or coexpressing two antibodies.
Fig. 3-26 shows the result of analysis in coexpression of three antibodies with CH3 modification. Each peak was assigned with reference to the peak positions observed when expressing one antibody or coexpressing two antibodies.
Fig. 3-27 shows the result of analysis in coexpression of four antibodies without CH3 modification. Each peak was assigned with reference to the peak positions observed when expressing one antibody or coexpressing two antibodies.
Fig. 3-28 shows the result of analysis in coexpression of four antibodies with CH3 modification. Each peak was assigned with reference to the peak positions observed when expressing one antibody or coexpressing two antibodies.
In Fig. 3-29, one antibody with CH3 modification is analyzed.
Fig. 3-30 shows the result of analysis in coexpression of two antibodies with CH3 modification. Each peak position was identified and assigned based on the elution position observed when expressing each one antibody.
Fig. 3-31 shows the result of analysis in coexpression of two antibodies with CH3 modification. Each peak position was identified and assigned based on the elution position observed when expressing each one antibody.
Fig. 3-32 shows the result of analysis in coexpression of two antibodies with CH3 modification. Each peak position was identified and assigned based on the elution position observed when expressing each one antibody.
Fig. 3-33 shows the result of analysis in coexpression of three antibodies with CH3 modification. Each peak was assigned with reference to the peak positions observed when expressing one antibody or coexpressing two antibodies.
Fig. 3-34 shows the result of analysis in coexpression of three antibodies with CH3 modification. Each peak was assigned with reference to the peak positions observed when expressing one antibody or coexpressing two antibodies.
Fig. 3-35 shows the result of analysis in coexpression of four antibodies with CH3 modification. Each peak was assigned with reference to the peak positions observed when expressing one antibody or coexpressing two antibodies.
In Fig. 3-36, one antibody with CH3 modification is analyzed.
Fig. 3-37 shows the result of analysis in coexpression of two antibodies with CH3 modification. Each peak position was identified and assigned based on the elution position observed when expressing each one antibody.
Fig. 3-38 shows the result of analysis in coexpression of two antibodies with CH3 modification. Each peak position was identified and assigned based on the elution position observed when expressing each one antibody.
Fig. 3-39 shows the result of analysis in coexpression of two antibodies with CH3 modification. Each peak position was identified and assigned based on the elution position observed when expressing each one antibody.
Fig. 3-40 shows the result of analysis in coexpression of three antibodies with CH3 modification. Each peak was assigned with reference to the peak positions observed when expressing one antibody or coexpressing two antibodies.
Fig. 3-41 shows the result of analysis in coexpression of three antibodies with CH3 modification. Each peak was assigned with reference to the peak positions observed when expressing one antibody or coexpressing two antibodies.
Fig. 3-42 shows the result of analysis in coexpression of three antibodies with CH3 modification. Each peak was assigned with reference to the peak positions observed when expressing one antibody or coexpressing two antibodies.
Fig. 3-43 shows the result of analysis in coexpression of four antibodies with CH3 modification. Each peak was assigned with reference to the peak positions observed when expressing one antibody or coexpressing two antibodies.
Fig. 3-44 shows the result of analysis in coexpression of two antibodies with CH3 modification. Each peak position was identified and assigned based on the elution position observed when expressing each one antibody.
Fig. 3-45 shows the result of analysis in coexpression of three antibodies with CH3 modification. Each peak was assigned with reference to the peak positions observed when expressing one antibody or coexpressing two antibodies.
Fig. 3-46 shows the result of analysis in coexpression of three antibodies with CH3 modification. Each peak was assigned with reference to the peak positions observed when expressing one antibody or coexpressing two antibodies.
Fig. 3-47 shows the result of analysis in coexpression of four antibodies with CH3 modification. Each peak was assigned with reference to the peak positions observed when expressing one antibody or coexpressing two antibodies.

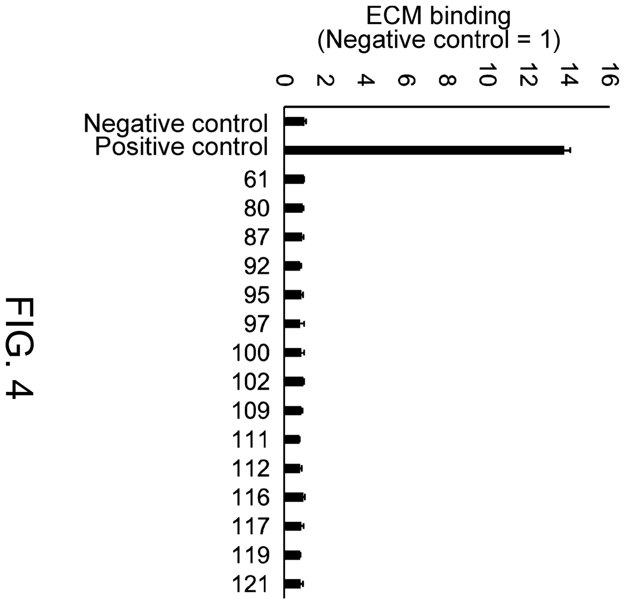

Fig. 4 shows the evaluation of ECM binding of CH3-modified antibodies. Non-specific binding of each antibody into which modifications for promoting heavy chain homodimerization were introduced was evaluated.

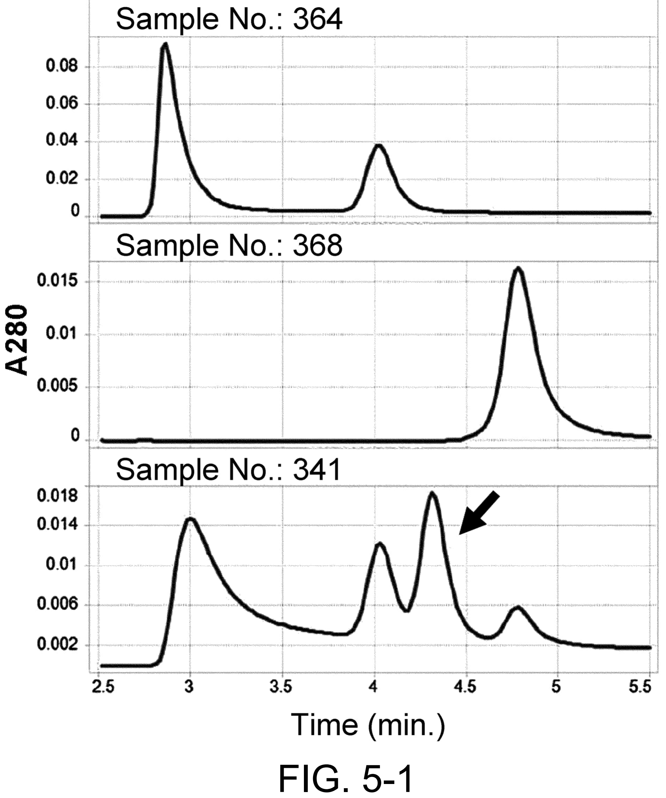

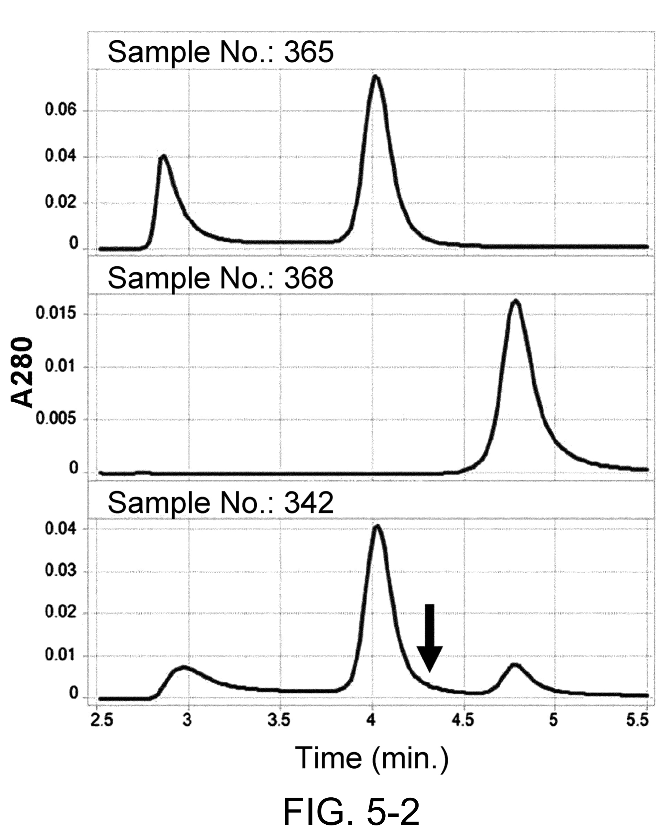

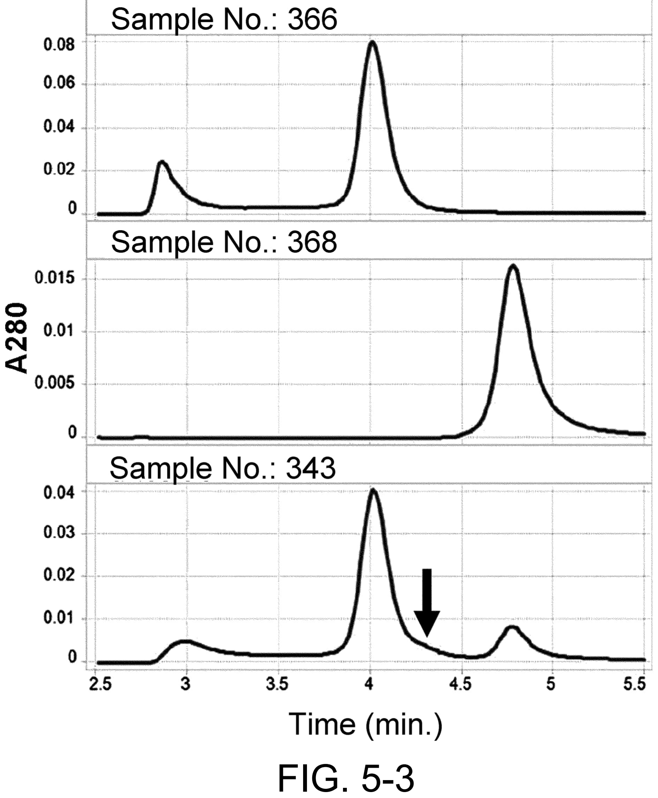

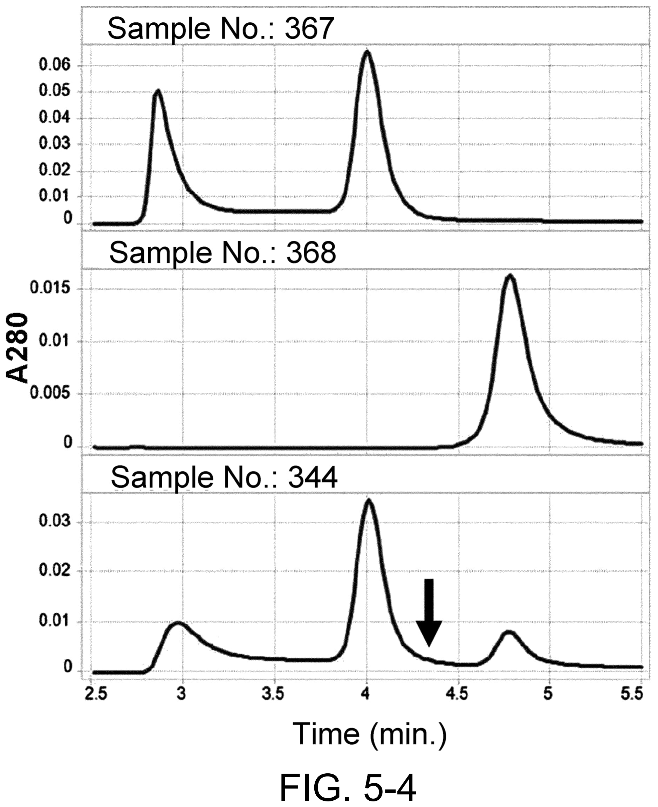

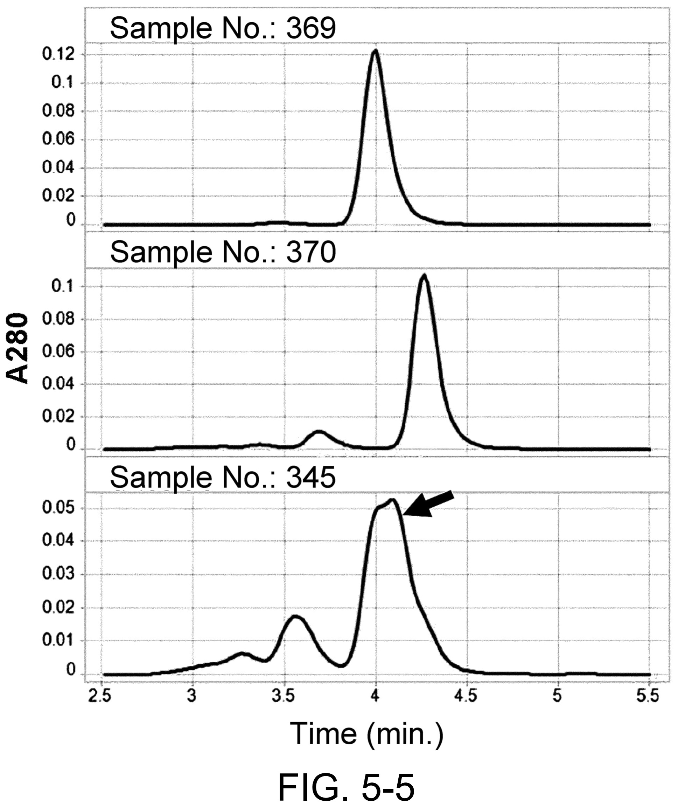

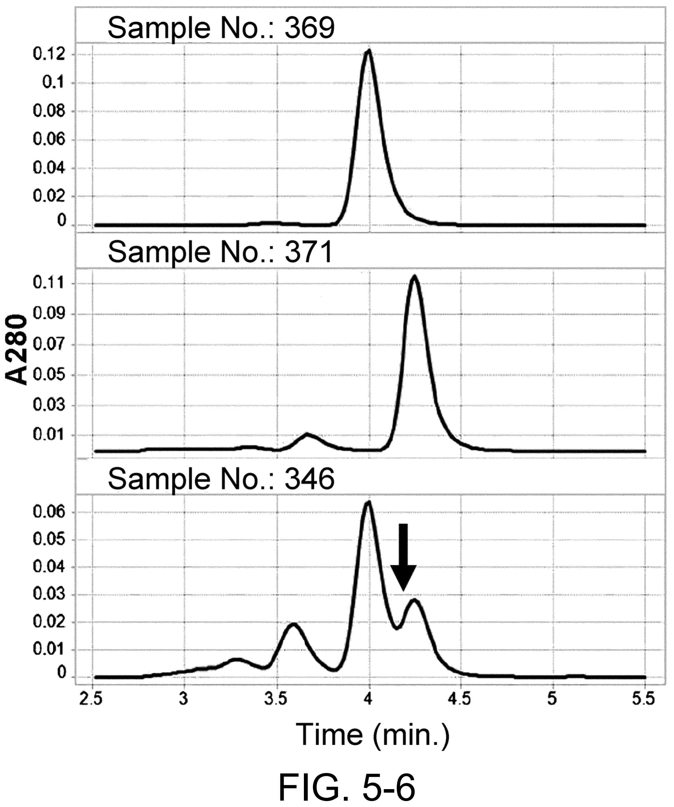

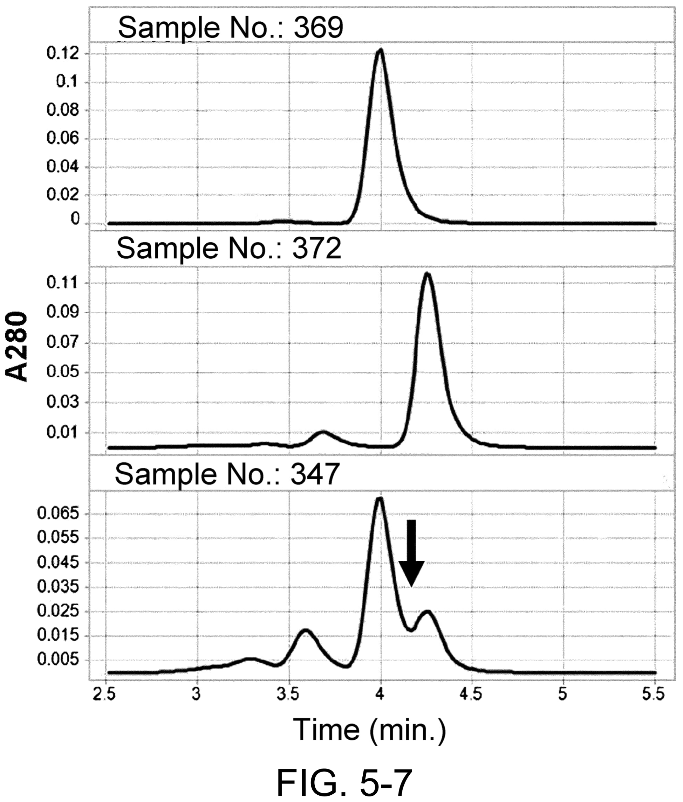

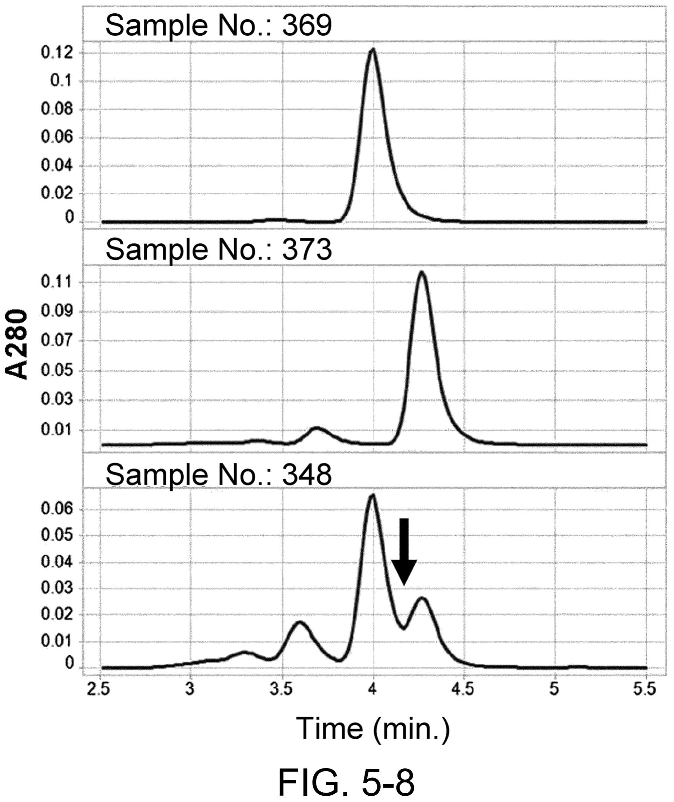

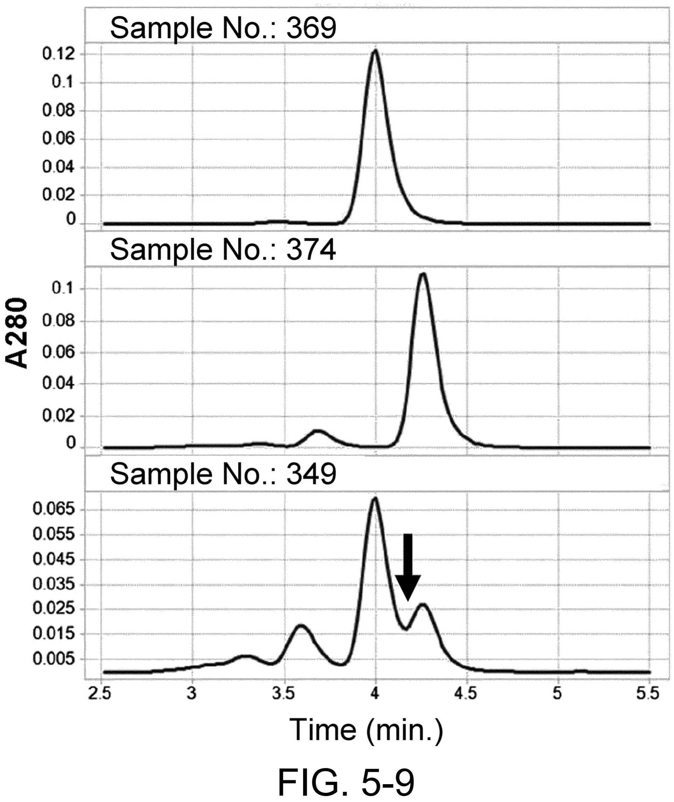

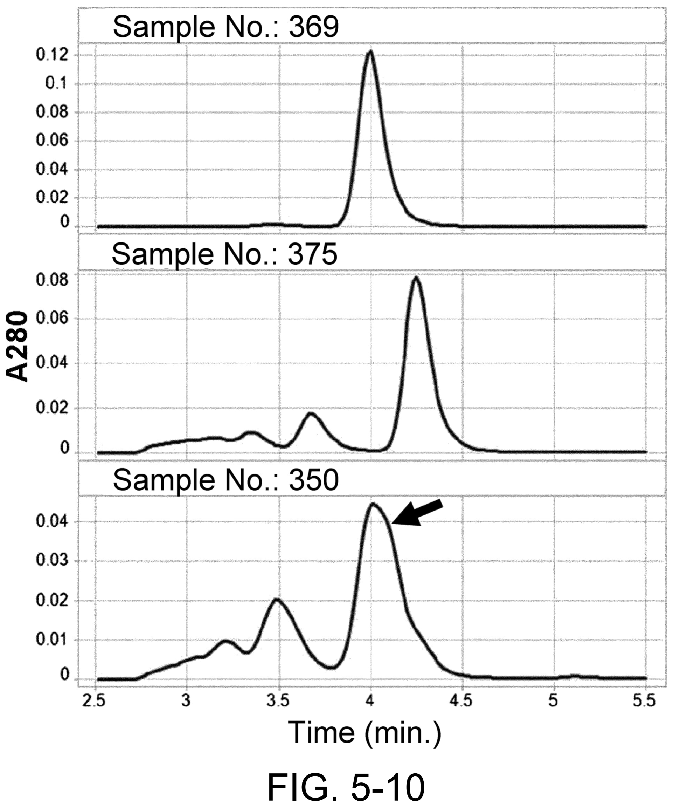

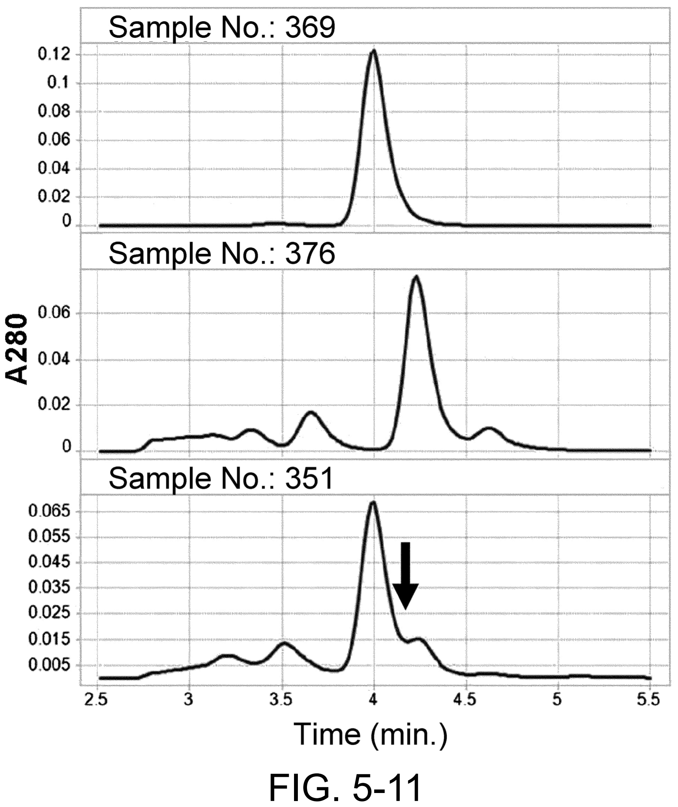

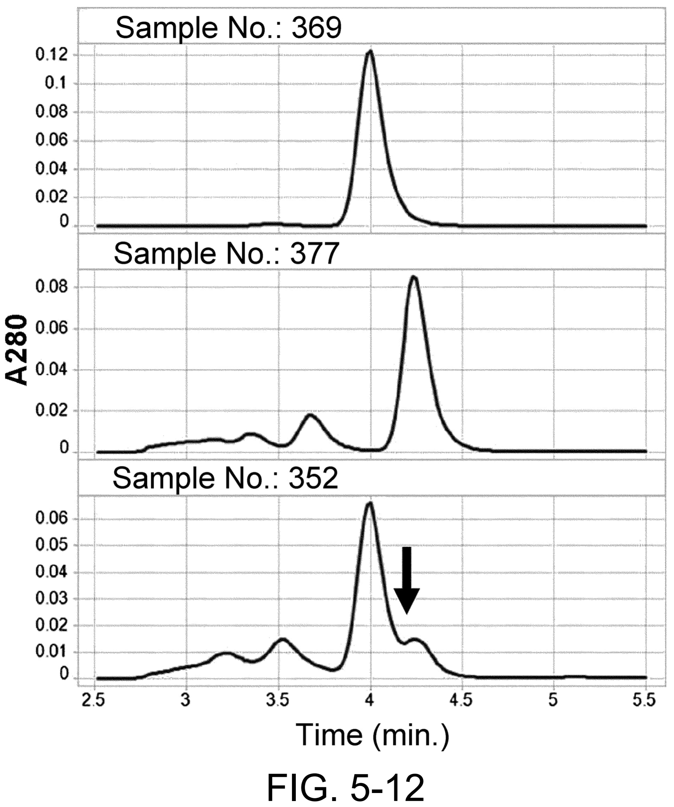

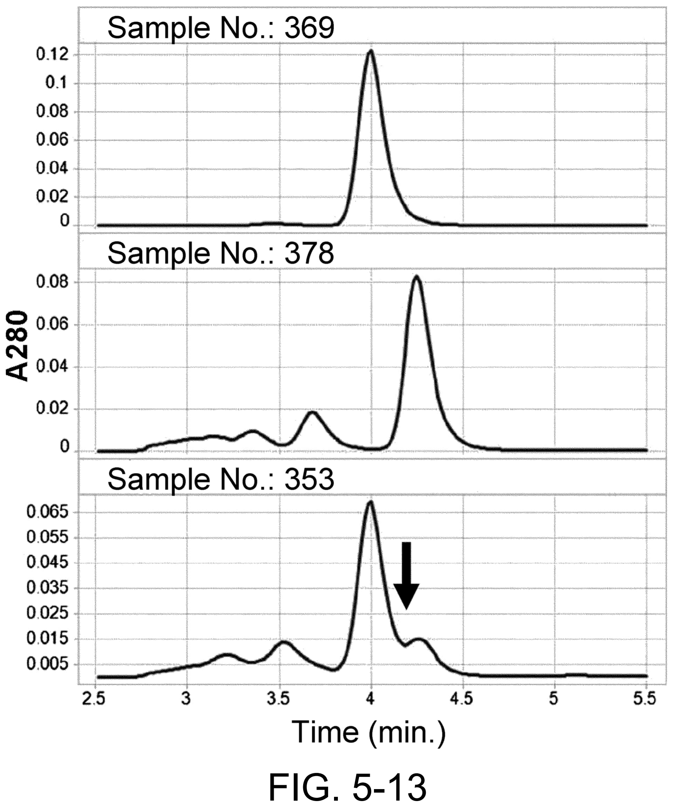

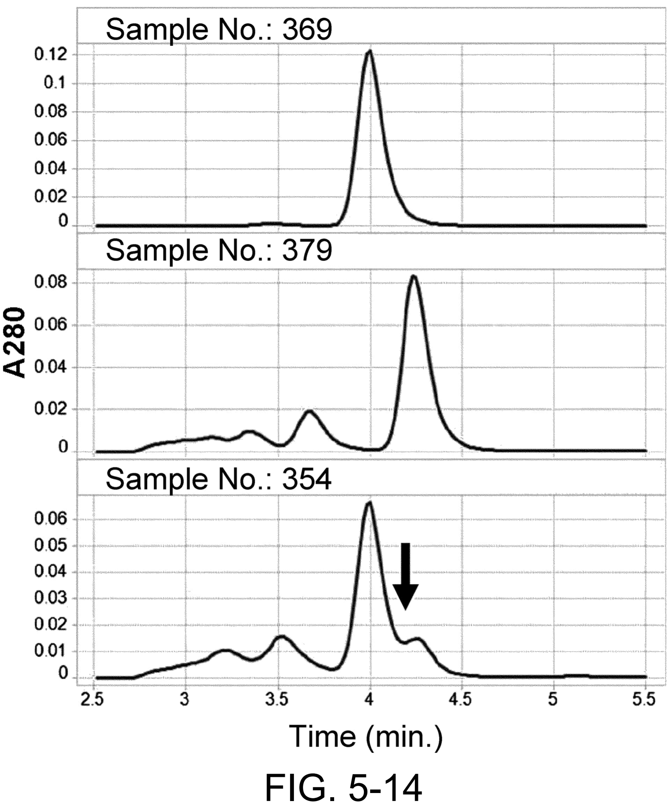

Fig. 5 (Fig. 5-1 to Fig. 5-14) shows the evaluation of heavy chain association of two antibodies by SEC analysis. Since there were multimeric components resulting from amino acid modification or antibody format, comparison was performed focusing on the heavy chain heteromultimer component indicated by the arrow in the figure. The elution position of the heteromultimer component in the chromatogram was identified by comparison with the elution position of each heavy chain homodimer reference sample. The analysis results of the reference samples are also shown in the figure. In Fig. 5-1, the elution positions of reference antibodies without CH3 modification, and the ability of heavy chain homodimerization under coexpression of full-length heavy chains and Fc fragments, are analyzed.
In Fig. 5-2, the elution positions of reference antibodies with CH3 modification, and the ability of heavy chain homodimerization under coexpression of full-length heavy chains and Fc fragments, are analyzed.
In Fig. 5-3, the elution positions of reference antibodies with CH3 modification, and the ability of heavy chain homodimerization under coexpression of full-length heavy chains and Fc fragments, are analyzed.
In Fig. 5-4, the elution positions of reference antibodies with CH3 modification, and the ability of heavy chain homodimerization under coexpression of full-length heavy chains and Fc fragments, are analyzed.
In Fig. 5-5, the elution positions of reference antibodies without CH3 modification, and the ability of heavy chain homodimerization under coexpression of full-length heavy chains and Fc fragments, are analyzed.
In Fig. 5-6, the elution positions of reference antibodies with CH3 modification, and the ability of heavy chain homodimerization under coexpression of full-length heavy chains and Fc fragments, are analyzed.
In Fig. 5-7, the elution positions of reference antibodies with CH3 modification, and the ability of heavy chain homodimerization under coexpression of full-length heavy chains and Fc fragments, are analyzed.
In Fig. 5-8, the elution positions of reference antibodies with CH3 modification, and the ability of heavy chain homodimerization under coexpression of full-length heavy chains and Fc fragments, are analyzed.
In Fig. 5-9, the elution positions of reference antibodies with CH3 modification, and the ability of heavy chain homodimerization under coexpression of full-length heavy chains and Fc fragments, are analyzed.
In Fig. 5-10, the elution positions of reference antibodies without CH3 modification, and the ability of heavy chain homodimerization under coexpression of full-length heavy chains and Fc fragments, are analyzed.
In Fig. 5-11, the elution positions of reference antibodies with CH3 modification, and the ability of heavy chain homodimerization under coexpression of full-length heavy chains and Fc fragments, are analyzed.
In Fig. 5-12, the elution positions of reference antibodies with CH3 modification, and the ability of heavy chain homodimerization under coexpression of full-length heavy chains and Fc fragments, are analyzed.
In Fig. 5-13, the elution positions of reference antibodies with CH3 modification, and the ability of heavy chain homodimerization under coexpression of full-length heavy chains and Fc fragments, are analyzed.
In Fig. 5-14, the elution positions of reference antibodies with CH3 modification, and the ability of heavy chain homodimerization under coexpression of full-length heavy chains and Fc fragments, are analyzed.

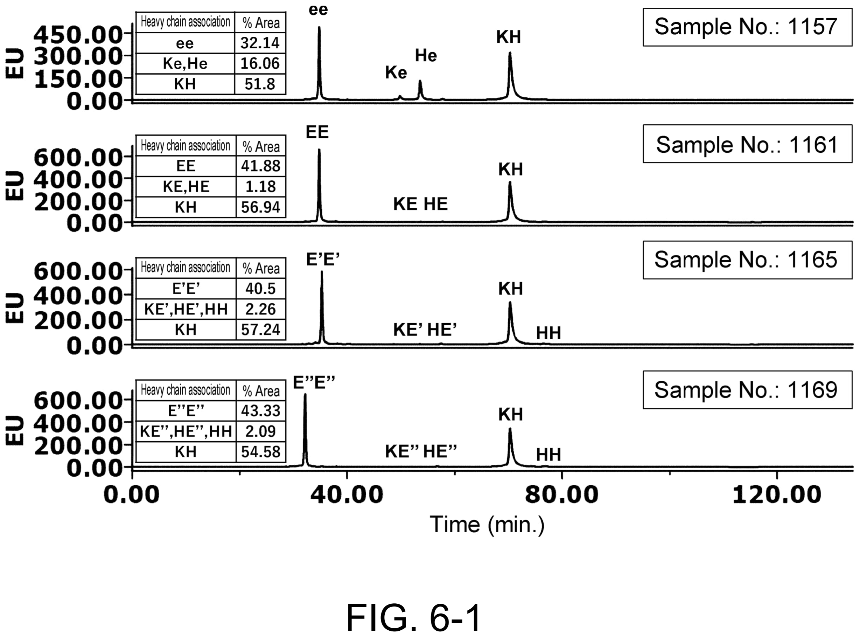

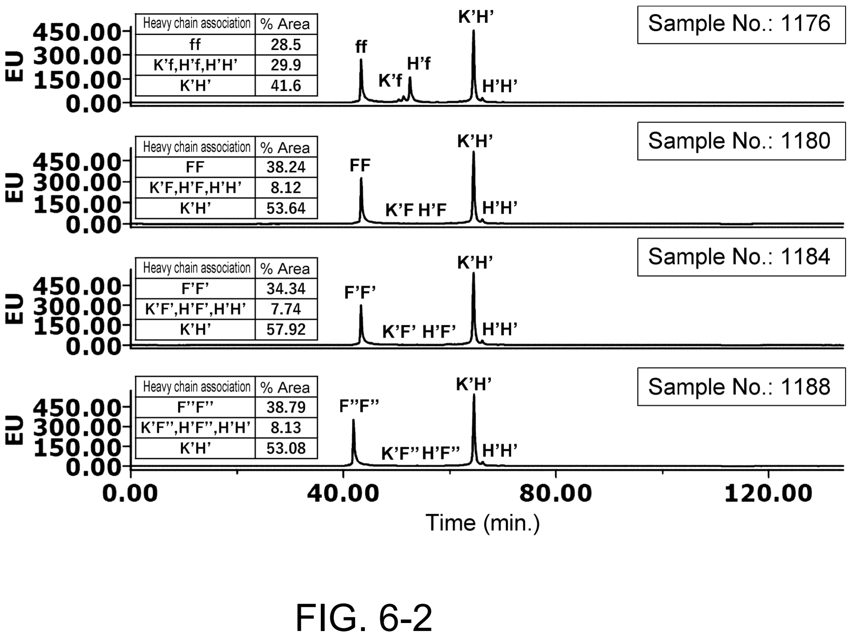

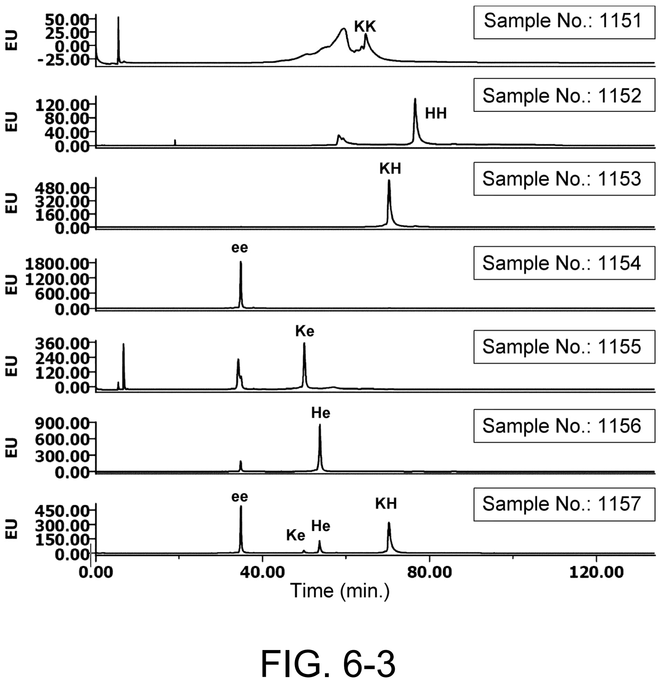

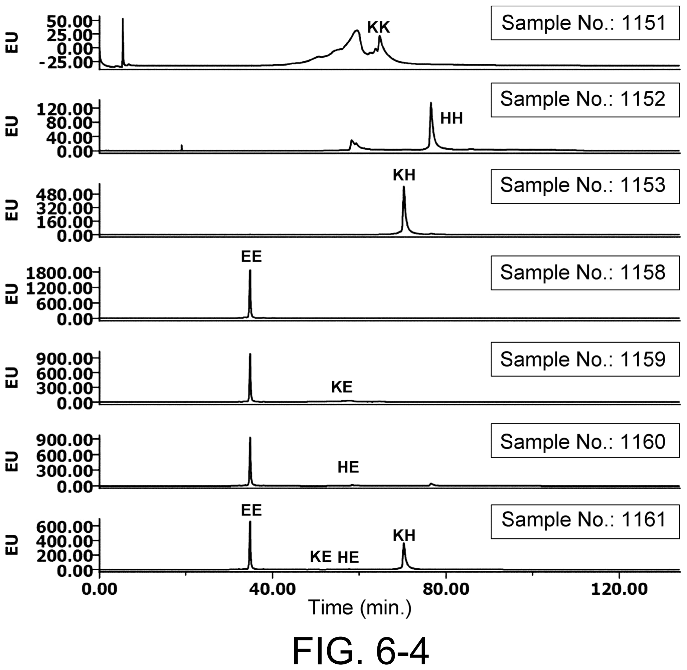

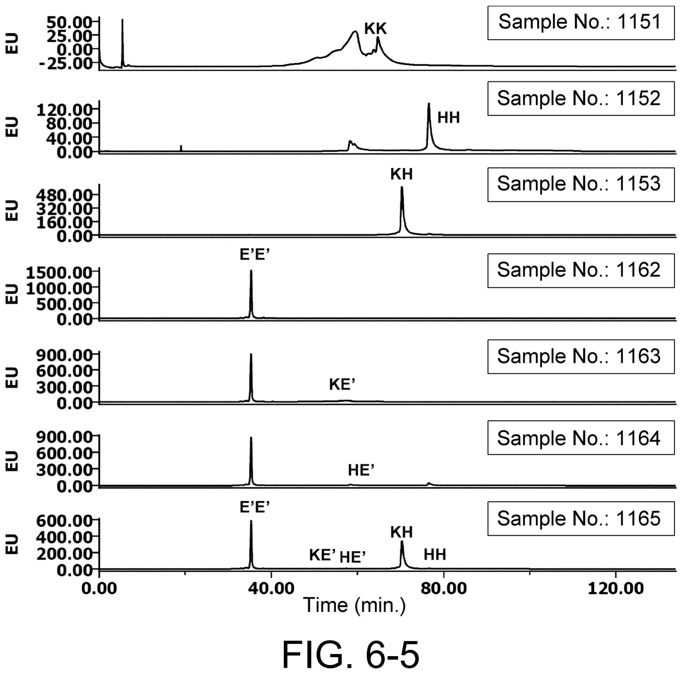

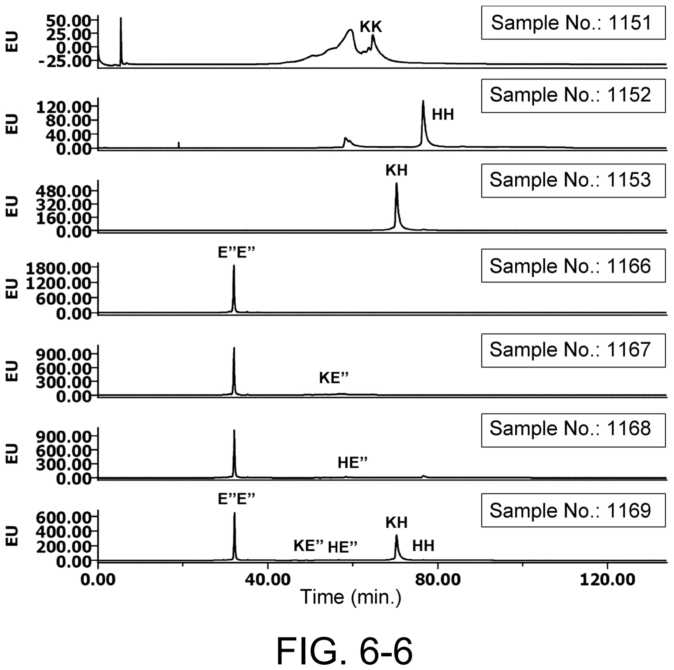

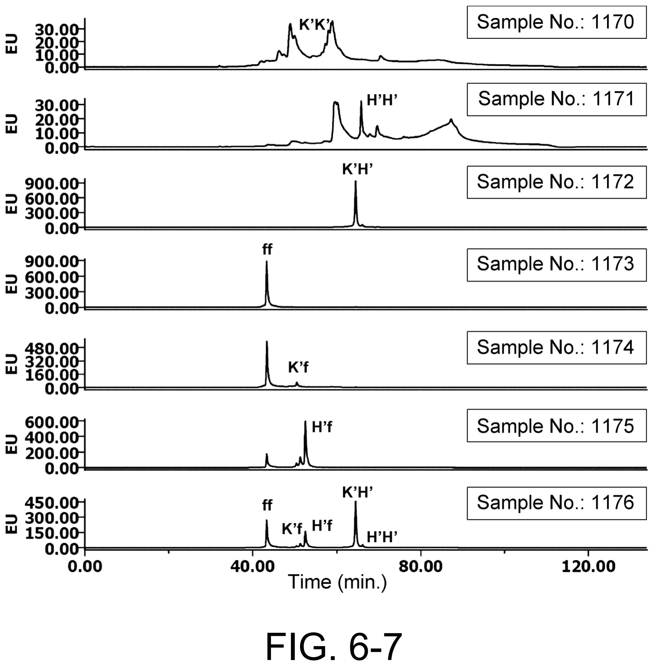

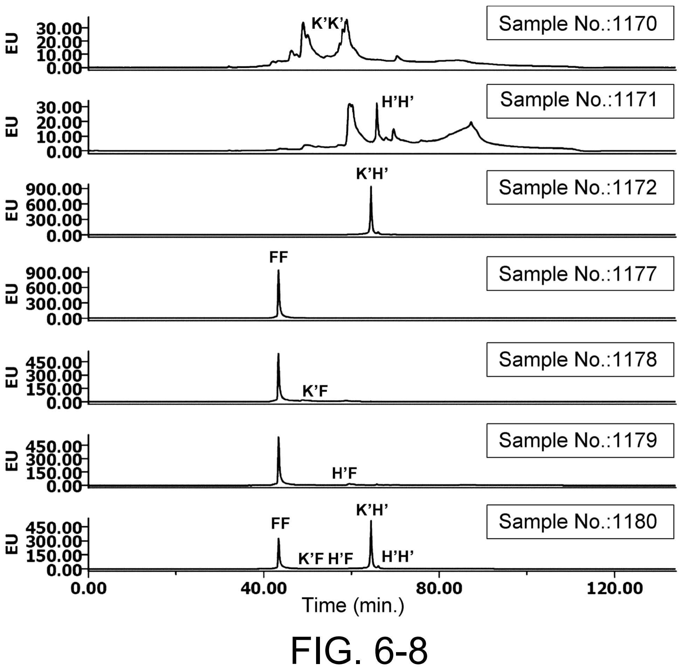

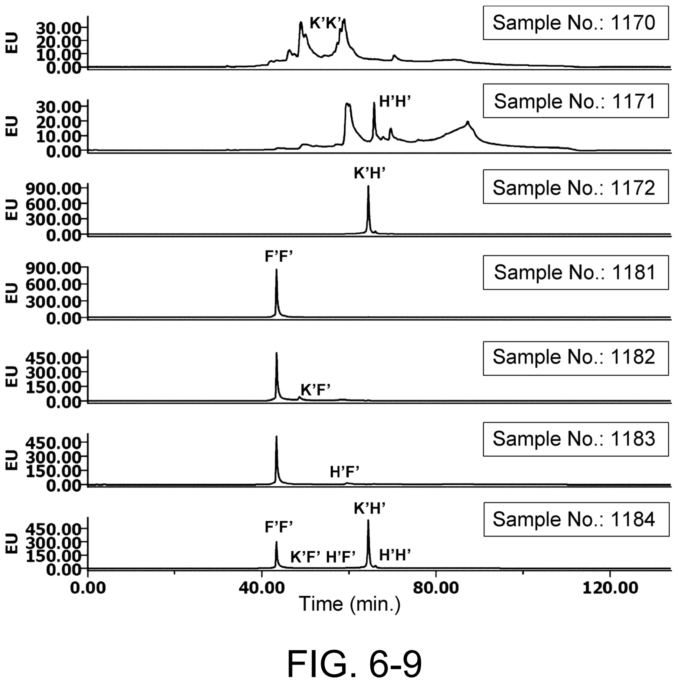

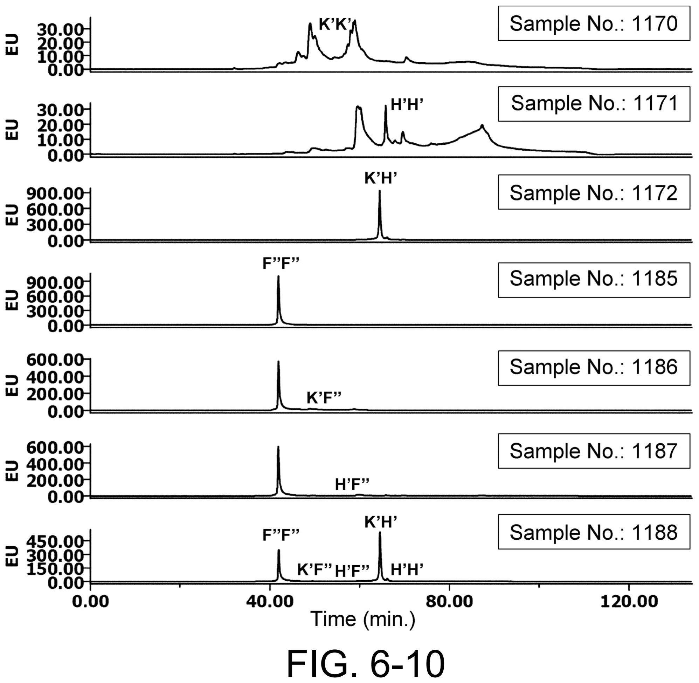

Fig. 6 (Fig. 6-1 to Fig. 6-10) shows the evaluation of the independent heavy chain heteromeric association ability and heavy chain homomeric association ability of antibodies by CIEX analysis. Fig. 6-1 shows the result of analysis when coexpressing one homodimeric antibody and one heterodimeric antibody for which heteromeric association was promoted by knobs-into-holes modifications. In each of the cases where the homodimer had no CH3 modification (Sample No. 1157) and had CH3 modifications (Samples Nos. 1161, 1165, and 1169), the area ratios of homodimer, heterodimer, and unintended heavy chain multimer were calculated. In all cases, the pair containing modifications for promoting homodimerization suppressed unintended heavy chain association as compared to the case of no CH3 modification.
Fig. 6-2 shows the result of analysis when coexpressing one homodimeric antibody and one heterodimeric antibody for which heteromeric association was promoted by knobs-into-holes modifications including disulfide bonds. In each of the cases where the homodimer had no CH3 modification (Sample No. 1176) and had CH3 modifications (Samples Nos. 1180, 1184, and 1188), the area ratios of homodimer, heterodimer, and unintended heavy chain multimer were calculated. In all cases, the pair containing modifications for promoting homodimerization suppressed unintended heavy chain association as compared to the case of no CH3 modification.
Fig. 6-3 shows the result of peak assignment in the CIEX analysis of Samples No. 1157. The plasmids used for expression of Sample No. 1157 were expressed in various combinations and each reference antibody thus produced was analyzed. The peaks for the heavy chain homodimer and heavy chain heterodimer of interest and other unintended peaks were assigned. The assigned heavy chain association is indicated at each peak in the figure.
Fig. 6-4 shows the result of peak assignment in the CIEX analysis of Samples No. 1161. The plasmids used for expression of Sample No. 1161 were expressed in various combinations and each reference antibody thus produced was analyzed. The peaks for the heavy chain homodimer and heavy chain heterodimer of interest and other unintended peaks were assigned. The assigned heavy chain association is indicated at each peak in the figure.
Fig. 6-5 shows the result of peak assignment in the CIEX analysis of Samples No. 1165. The plasmids used for expression of Sample No. 1165 were expressed in various combinations and each reference antibody thus produced was analyzed. The peaks for the heavy chain homodimer and heavy chain heterodimer of interest and other unintended peaks were assigned. The assigned heavy chain association is indicated at each peak in the figure.
Fig. 6-6 shows the result of peak assignment in the CIEX analysis of Samples No. 1169. The plasmids used for expression of Sample No. 1169 were expressed in various combinations and each reference antibody thus produced was analyzed. The peaks for the heavy chain homodimer and heavy chain heterodimer of interest and other unintended peaks were assigned. The assigned heavy chain association is indicated at each peak in the figure.
Fig. 6-7 shows the result of peak assignment in the CIEX analysis of Samples No. 1176. The plasmids used for expression of Sample No. 1176 were expressed in various combinations and each reference antibody thus produced was analyzed. The peaks for the heavy chain homodimer and heavy chain heterodimer of interest and other unintended peaks were assigned. The assigned heavy chain association is indicated at each peak in the figure.
Fig. 6-8 shows the result of peak assignment in the CIEX analysis of Samples No. 1180. The plasmids used for expression of Sample No. 1180 were expressed in various combinations and each reference antibody thus produced was analyzed. The peaks for the heavy chain homodimer and heavy chain heterodimer of interest and other unintended peaks were assigned. The assigned heavy chain association is indicated at each peak in the figure.
Fig. 6-9 shows the result of peak assignment in the CIEX analysis of Samples No. 1184. The plasmids used for expression of Sample No. 1184 were expressed in various combinations and each reference antibody thus produced was analyzed. The peaks for the heavy chain homodimer and heavy chain heterodimer of interest and other unintended peaks were assigned. The assigned heavy chain association is indicated at each peak in the figure.
Fig. 6-10 shows the result of peak assignment in the CIEX analysis of Samples No. 1188. The plasmids used for expression of Sample No. 1188 were expressed in various combinations and each reference antibody thus produced was analyzed. The peaks for the heavy chain homodimer and heavy chain heterodimer of interest and other unintended peaks were assigned. The assigned heavy chain association is indicated at each peak in the figure.

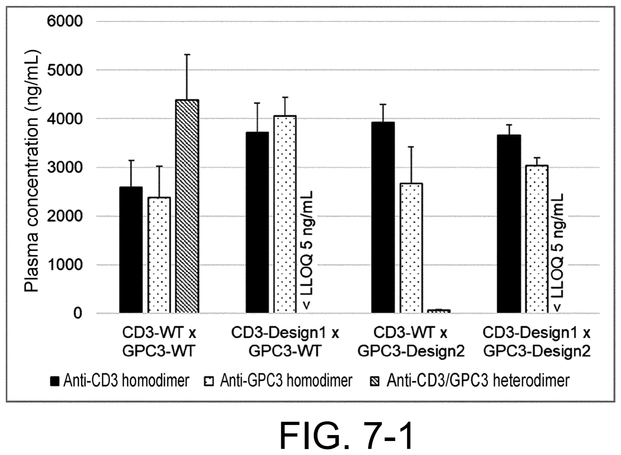

Fig. 7-1 shows the plasma concentration of the anti-CD3 homodimer, anti-GPC3 homodimer, and anti-CD3/GPC3 heterodimer on day 3 after administration.

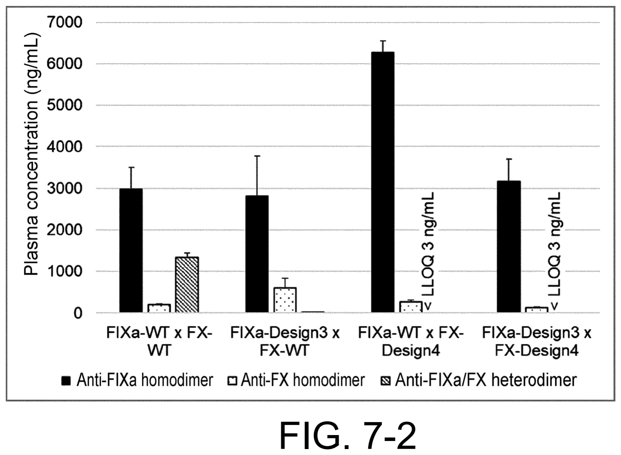

Fig. 7-2 shows the plasma concentration of the anti-FIXa homodimer, anti-FX homodimer, and anti-FIXa/FX heterodimer on day 3 after administration.

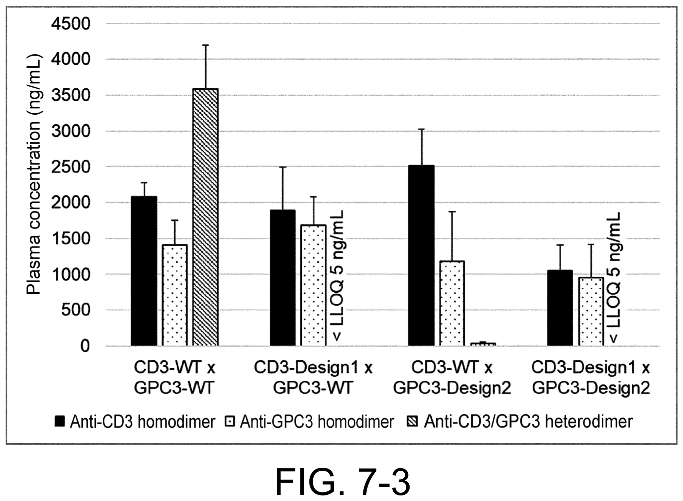

Fig. 7-3 shows the plasma concentration of the anti-CD3 homodimer, anti-GPC3 homodimer, and anti-CD3/GPC3 heterodimer on day 7 after administration.

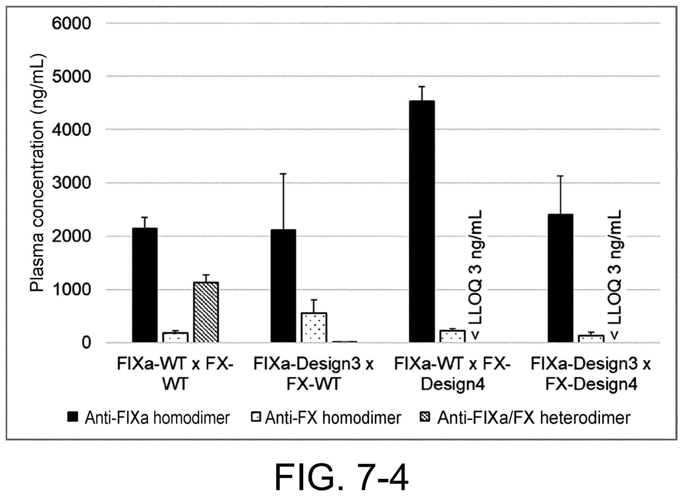

Fig. 7-4 shows the plasma concentration of the anti-FIXa homodimer, anti-FX homodimer, and anti-FIXa/FX heterodimer on day 7 after administration.

### [Description of Embodiments]

The techniques and procedures described or referenced herein are generally well understood and commonly employed using conventional methodology by those skilled in the art, such as, for example, the widely utilized methodologies described in Sambrook et al., Molecular Cloning: A Laboratory Manual 3d edition (2001) Cold Spring Harbor Laboratory Press, Cold Spring Harbor, N.Y.; Current Protocols in Molecular Biology (F.M. Ausubel, et al. eds., (2003)); the series Methods in Enzymology (Academic Press, Inc.): PCR 2: A Practical Approach (M.J. MacPherson, B.D. Hames and G.R. Taylor eds. (1995)), Harlow and Lane, eds. (1988) Antibodies, A Laboratory Manual, and Animal Cell Culture (R.I. Freshney, ed. (1987)); Oligonucleotide Synthesis (M.J. Gait, ed., 1984); Methods in Molecular Biology, Humana Press; Cell Biology: A Laboratory Notebook (J.E. Cellis, ed., 1998) Academic Press; Animal Cell Culture (R.I. Freshney), ed., 1987); Introduction to Cell and Tissue Culture (J. P. Mather and P.E. Roberts, 1998) Plenum Press; Cell and Tissue Culture: Laboratory Procedures (A. Doyle, J.B. Griffiths, and D.G. Newell, eds., 1993-8) J. Wiley and Sons; Handbook of Experimental Immunology (D.M. Weir and C.C. Blackwell, eds.); Gene Transfer Vectors for Mammalian Cells (J.M. Miller and M.P. Calos, eds., 1987); PCR: The Polymerase Chain Reaction, (Mullis et al., eds., 1994); Current Protocols in Immunology (J.E. Coligan et al., eds., 1991); Short Protocols in Molecular Biology (Wiley and Sons, 1999); Immunobiology (C.A. Janeway and P. Travers, 1997); Antibodies (P. Finch, 1997); Antibodies: A Practical Approach (D. Catty., ed., IRL Press, 1988-1989); Monoclonal Antibodies: A Practical Approach (P. Shepherd and C. Dean, eds., Oxford University Press, 2000); Using Antibodies: A Laboratory Manual (E. Harlow and D. Lane (Cold Spring Harbor Laboratory Press, 1999); The Antibodies (M. Zanetti and J. D. Capra, eds., Harwood Academic Publishers, 1995); and Cancer: Principles and Practice of Oncology (V.T. DeVita et al., eds., J.B. Lippincott Company, 1993).

The definitions and detailed description below are provided to facilitate understanding of the present disclosure illustrated herein.

### Definitions

### Amino acids

Herein, amino acids are described by one- or three-letter codes or both, for example, Ala/A, Leu/L, Arg/R, Lys/K, Asn/N, Met/M, Asp/D, Phe/F, Cys/C, Pro/P, Gln/Q, Ser/S, Glu/E, Thr/T, Gly/G, Trp/W, His/H, Tyr/Y, Ile/I, or Val/V.

### Modification of amino acids

For amino acid modification (herein also referred to as "amino acid substitution" or "amino acid mutation") in the amino acid sequence of a polypeptide, known methods such as site-directed mutagenesis methods (Kunkel et al. (Proc. Natl. Acad. Sci. USA (1985) 82, 488-492)) and overlap extension PCR may be appropriately employed. Furthermore, several known methods may also be employed as amino acid alteration methods for substitution to non-natural amino acids (Annu Rev. Biophys. Biomol. Struct. (2006) 35, 225-249; and Proc. Natl. Acad. Sci. U.S.A. (2003) 100 (11), 6353-6357). For example, it is possible to use a cell-free translation system (Clover Direct (Protein Express)) containing a tRNA which has a non-natural amino acid bound to a complementary amber suppressor tRNA of one of the stop codons, the UAG codon (amber codon).

In the present specification, the meaning of the term "and/or" when describing the site of amino acid modification includes every combination where "and" and "or" are suitably combined. Specifically, for example, "the amino acids at positions x, y, and/or z are substituted" includes the following variation of amino acid alterations: amino acid(s) at (a) position x, (b) position y, (c) position z, (d) positions x and y, (e) positions x and z, (f) positions y and z, and (g) positions x, y, and z.

Furthermore, herein, as an expression showing modification of amino acids, an expression that shows before and after a number indicating a specific position, one-letter or three-letter codes for amino acids before and after modification, respectively, may be used appropriately. For example, the modification E345K used when substituting an amino acid contained in an antibody variable region indicates substitution of Glu (E) at position 345 (according to EU numbering) with Lys (K). That is, the number shows the amino acid position according to EU numbering, the one-letter or three-letter amino-acid code written before the number shows the amino acid before substitution, and the one-letter or three-letter amino-acid code written after the number shows the amino acid after substitution.

### Polypeptides

As used herein, term "polypeptide" refers to a molecule composed of monomers (amino acids) linearly linked by amide bonds (also known as peptide bonds). The term "polypeptide" does not refer to a product with a specific length. The term "polypeptide" is also intended to refer to the products of post-expression modifications of the polypeptide, including without limitation glycosylation, acetylation, phosphorylation, amidation, derivatization by known protecting/blocking groups, proteolytic cleavage, or modification by non-naturally occurring amino acids. A polypeptide may be derived from a natural biological source or produced by recombinant technology, but is not necessarily translated from a designated nucleic acid sequence. It may be generated in any manner, including by chemical synthesis. A polypeptide as described herein may be, in one embodiment, of a size of 10 or more, 20 or more, 25 or more, 50 or more, 75 or more, 100 or more, 200 or more, 500 or more, or 1,000 or more amino acids.

A polypeptide of the present disclosure is, in one embodiment, produced in a living organism. For example, a polypeptide of the present disclosure can be produced in a living organism by introducing a nucleic acid encoding the polypeptide of the present disclosure into the living organism. In one embodiment, a polypeptide of the present disclosure is a polypeptide expressed in a living organism.

### Recombinant methods and compositions

Polypeptides which are antibodies or antigen-binding molecules may be produced using recombinant methods and compositions, e.g., as described in U.S. Patent No. 4,816,567. In one embodiment, isolated nucleic acid encoding a polypeptide as described herein is provided. In one embodiment, such nucleic acid may encode a polypeptide having a heavy chain Fc region. In a further embodiment, one or more vectors (e.g., expression vectors) comprising such nucleic acid are provided. In a further embodiment, a host cell comprising such nucleic acid is provided. In one of such embodiments, the host cell comprises (e.g., has been transformed with) a vector comprising a nucleic acid encoding an amino acid sequence comprising a heavy chain Fc region. In one embodiment, the host cell is eukaryotic, e.g. a Chinese Hamster Ovary (CHO) cell or lymphoid cell (e.g., Y0, NS0, Sp2/0 cell). In one embodiment, a method for producing the polypeptide for which association is controlled according to the present disclosure is provided, wherein the method comprises culturing a host cell comprising a nucleic acid encoding the polypeptide, as provided above, under conditions suitable for expression of the polypeptide, and optionally recovering the polypeptide from the host cell (or host cell culture medium).

For recombinant production of a polypeptide described herein (in one embodiment, an antibody), a nucleic acid encoding the polypeptide is isolated and inserted into one or more vectors for further cloning and/or expression in a host cell. Such nucleic acids may be readily isolated and sequenced using conventional procedures, e.g., by using oligonucleotide probes that are capable of binding specifically to a gene encoding the heavy chain Fc region.

Suitable host cells for cloning or expression of vectors encoding polypeptides (in one embodiment, antibodies) include prokaryotic or eukaryotic cells described herein. For example, polypeptides (in one embodiment, antibodies) may be produced in bacteria, in particular when glycosylation and Fc effector function are not needed. For expression of antibody fragments and polypeptides in bacteria, see, e.g., U.S. Patent Nos. 5,648,237, 5,789,199, and 5,840,523. (See also Charlton, Methods in Molecular Biology, Vol. 248 (B.K.C. Lo, ed., Humana Press, Totowa, NJ, 2003), pp. 245-254, describing expression of polypeptide (antibody) fragments in E. coli.) After expression, the polypeptide may be isolated from the bacterial cell paste in a soluble fraction and can be further purified.

In addition to prokaryotes, eukaryotic microbes such as filamentous fungi or yeast are suitable cloning or expression hosts for polypeptide (antibody)-encoding vectors, including fungi and yeast strains whose glycosylation pathways have been "humanized," resulting in the production of a polypeptide (an antibody in one embodiment) with a partially or fully human glycosylation pattern. (See Gerngross, Nat. Biotech. 22:1409-1414 (2004), and Li et al., Nat. Biotech. 24:210-215 (2006).)

Suitable host cells for the expression of glycosylated antibody are also derived from multicellular organisms (invertebrates and vertebrates). Examples of invertebrate cells include plant and insect cells. Numerous baculoviral strains have been identified which may be used in conjunction with insect cells, particularly for transfection of *Spodoptera frugiperda* cells.

Plant cell cultures can also be utilized as hosts. Reference can be made to, e.g., US Patent Nos. 5,959,177, 6,040,498, 6,420,548, 7,125,978, and 6,417,429 (describing PLANTIBODIES^{™} technology for producing polypeptides (antibodies in one embodiment) in transgenic plants).

Vertebrate cells may also be used as hosts. For example, mammalian cell lines that are adapted to grow in suspension may be useful. Other examples of useful mammalian host cell lines are monkey kidney CV1 line transformed by SV40 (COS-7); human embryonic kidney line (293 or 293 cells as described, e.g., in Graham et al., J. Gen Virol. 36:59 (1977)); baby hamster kidney cells (BHK); mouse sertoli cells (TM4 cells as described, e.g., in Mather, Biol. Reprod. 23:243-251 (1980)); monkey kidney cells (CV1); African green monkey kidney cells (VERO-76); human cervical carcinoma cells (HELA); canine kidney cells (MDCK); buffalo rat liver cells (BRL 3A); human lung cells (W138); human liver cells (Hep G2); mouse mammary tumor (MMT 060562); TRI cells, as described, e.g., in Mather et al., Annals N.Y. Acad. Sci. 383:44-68 (1982); MRC 5 cells; and FS4 cells. Other useful mammalian host cell lines include Chinese hamster ovary (CHO) cells, including DHFR⁻ CHO cells (Urlaub et al., Proc. Natl. Acad. Sci. USA 77:4216 (1980)); and myeloma cell lines such as Y0, NS0 and Sp2/0. For a review of certain mammalian host cell lines suitable for polypeptide (antibody) production, reference can be made to, e.g., Yazaki and Wu, Methods in Molecular Biology, Vol. 248 (B.K.C. Lo, ed., Humana Press, Totowa, NJ), pp. 255-268 (2003).

Recombinant production of a polypeptide (in one embodiment, an antibody) described herein could be done with methods similar to those described above, by using a host cell that comprises (e.g., has been transformed with) one or more vectors comprising a nucleic acid that encodes an amino acid sequence comprising the whole polypeptide (antibody) or part of the polypeptide (antibody).

The polypeptides of the present disclosure (in one embodiment, antibodies) include polypeptides that have undergone posttranslational modification. Examples of the polypeptides thereof of the present disclosure undergoing posttranslational modification include, in the case of the polypeptides being antibodies, antibodies which have undergone pyroglutamylation at the N terminal of the heavy chain variable region and/or deletion of lysine at the C terminal of the heavy chain. It is known in the field that such posttranslational modification due to pyroglutamylation at the N terminal and deletion of lysine at the C terminal does not have any influence on the activity of the antibody (Analytical Biochemistry, 2006, Vol. 348, p. 24-39).

As used herein, the terms "first", "second", and such with respect to polypeptides etc., are used for convenience of distinguishing. Use of these terms is not intended to confer a specific order or orientation unless explicitly so stated.

A polypeptide may be a TCR (T cell receptor) or part of a TCR.

### Expression in cells in living organisms

In one embodiment, a polypeptide of the present disclosure may be expressed in cells in living organisms.

In one embodiment, expression of a polypeptide of the present disclosure in cells is achieved by introducing a nucleic acid encoding the polypeptide of the present disclosure into target cells. Any standard method of introducing nucleic acids into a cell may be used. Such methods include, for example, microinjection, ballistic injection, electroporation, calcium phosphate precipitation, liposomes, and transfection with retroviral, adenoviral, adeno-associated viral and vaccinia vectors carrying the nucleic acid of interest.

In one embodiment, a nucleic acid encoding a polypeptide of the present disclosure may be introduced into cells of a subject (in one embodiment, a human) by *in vivo* and *ex vivo* methods. In one example of *in vivo* delivery, a nucleic acid is injected directly into the subject e.g., at the site where treatment is required. In a further example of *in vivo* delivery, a nucleic acid is introduced into a cell using transfection with a viral vector (such as adenovirus, Herpes simplex I virus, or adeno-associated virus) and a lipid-based system (useful lipids for lipid-mediated transfer of the gene are DOTMA, DOPE and DC-Chol, for example) (For review of certain gene marking and gene therapy protocols, see Anderson et al., Science 256:808-813 (1992), and WO 93/25673 and the references cited therein.). In an example of *ex vivo* treatment, a subject's cells are removed, a nucleic acid is introduced into those isolated cells, and the modified cells are administered to the subject either directly or, for example, encapsulated within porous membranes which are implanted into the subject (see, e.g., U.S. Patent Nos. 4,892,538 and 5,283,187). A commonly used vector for *ex vivo* delivery of a nucleic acid is, in one embodiment, a retroviral vector.

### Intrabody

In one embodiment, a polypeptide of the present disclosure can be expressed intracellularly as an intrabody (intracellularly expressed antibody).

The term "intrabody," as used herein, refers to an antibody or antigen-binding portion thereof that is expressed intracellularly and that is capable of selectively binding to a target molecule, as described, e.g., in Marasco, Gene Therapy 4: 11-15 (1997); Kontermann, Methods 34: 163-170 (2004); U.S. Patent Nos. 6,004,940 and 6,329,173; U.S. Patent Application Publication No. 2003/0104402, and PCT Publication No. WO2003/077945 (see also, for example, WO96/007321 published March 14, 1996, concerning the use of gene therapy to generate intracellular antibodies).

In one embodiment, intracellular expression of an intrabody may be effected by introducing a nucleic acid encoding the antibody or antigen-binding portion thereof into a target cell. One or more nucleic acids encoding all or a portion of an antibody including a polypeptide of the present disclosure can be delivered to a target cell, such that one or more intrabodies are expressed.

In one embodiment, a polypeptide of the present disclosure for which association between polypeptides is controlled can be used for intracellular production of intrabodies.

### Single-domain antibody

In one embodiment, a polypeptide of the present disclosure may be a polypeptide having a single-domain antibody.

As used herein, the term "single-domain antibody" is not limited by its structure as long as the domain can exert antigen binding activity by itself. It is known that a general antibody, for example, an IgG antibody, exhibits antigen binding activity in a state where a variable region is formed by the pairing of VH and VL, whereas the own domain structure of the single-domain antibody can exert antigen binding activity by itself without pairing with another domain. Usually, the single-domain antibody has a relatively low molecular weight and exists in the form of a monomer.

Examples of the single-domain antibody include, but are not limited to, antigen-binding molecules congenitally lacking a light chain, such as VHH of an animal of the family Camelidae and shark VNAR, and antibody fragments containing the whole or a portion of an antibody VH domain or the whole or a portion of an antibody VL domain. Examples of the single-domain antibody which is an antibody fragment containing the whole or a portion of an antibody VH or VL domain include, but are not limited to, artificially prepared single-domain antibodies originating from human antibody VH or human antibody VL as described in U.S. Patent No. 6,248,516 B1, etc. In some embodiments of the present disclosure, one single-domain antibody has three CDRs (CDR1, CDR2 and CDR3).

In one embodiment, single-domain antibodies of the present disclosure include scFv-Fc or VHH-Fc.

### Variable region

In one embodiment, a polypeptide of the present disclosure may be a polypeptide having a variable region.

The term "variable region" or "variable domain" refers to the domain of an antibody heavy or light chain that is involved in binding the antibody to antigen. The variable domains of the heavy chain and light chain (VH and VL, respectively) of a naturally-occurring antibody generally have similar structures, with each domain comprising four conserved framework regions (FRs) and three hypervariable regions (HVRs). (See, e.g., Kindt et al. Kuby Immunology, 6th ed., W.H. Freeman and Co., page 91 (2007).) A single VH or VL domain is capable of conferring antigen-binding specificity. Furthermore, antibodies that bind a particular antigen may be isolated using a VH or VL domain from an antibody that binds the antigen to screen a library of complementary VL or VH domains, respectively. See, e.g., Portolano et al., J. Immunol. 150:880-887 (1993); Clarkson et al., Nature 352:624-628 (1991).

### HVR or CDR

In one embodiment, a polypeptide of the present disclosure is an antibody, and may have HVRs or CDRs.

The term "hypervariable region" or "HVR" as used herein refers to each of the regions of an antibody variable domain which are hypervariable in sequence ("complementarity determining regions" or "CDRs") and/or form structurally defined loops ("hypervariable loops") and/or contain the antigen-contacting residues ("antigen contacts"). Hypervariable regions (HVRs) are also referred to as "complementarity determining regions" (CDRs), and these terms are used herein interchangeably in reference to portions of the variable region that form the antigen binding regions. Generally, antibodies comprise six HVRs: three in the VH (H1, H2, H3), and three in the VL (L1, L2, L3). Exemplary HVRs herein include:
(a) hypervariable loops occurring at amino acid residues 26-32 (L1), 50-52 (L2), 91-96 (L3), 26-32 (H1), 53-55 (H2), and 96-101 (H3) (Chothia and Lesk, J. Mol. Biol. 196:901-917 (1987));
(b) CDRs occurring at amino acid residues 24-34 (L1), 50-56 (L2), 89-97 (L3), 31-35b (H1), 50-65 (H2), and 95-102 (H3) (Kabat et al., Sequences of Proteins of Immunological Interest, 5th Ed. Public Health Service, National Institutes of Health, Bethesda, MD (1991));
(c) antigen contacts occurring at amino acid residues 27c-36 (L1), 46-55 (L2), 89-96 (L3), 30-35b (H1), 47-58 (H2), and 93-101 (H3) (MacCallum et al. J. Mol. Biol. 262: 732-745 (1996)); and
(d) combinations of (a), (b), and/or (c), including HVR amino acid residues 46-56 (L2), 47-56 (L2), 48-56 (L2), 49-56 (L2), 26-35 (H1), 26-35b (H1), 49-65 (H2), 93-102 (H3), and 94-102 (H3).

Unless otherwise indicated, HVR residues and other residues in the variable domain (e.g., FR residues) are numbered herein according to Kabat et al., supra.

HVR-H1, HVR-H2, HVR-H3, HVR-L1, HVR-L2, and HVR-L3 are also mentioned as "H-CDR1", "H-CDR2", "H-CDR3", "L-CDR1", "L-CDR2", and "L-CDR3", respectively.

### Framework

In one embodiment, a polypeptide of the present disclosure is an antibody, and may have a variable region containing a framework.

In one embodiment, a polypeptide of the present disclosure may have a variable region containing a framework.

"Framework" or "FR" refers to variable domain residues other than hypervariable region (HVR) residues. The FR of a variable domain generally consists of four FR domains: FR1, FR2, FR3, and FR4. Accordingly, the HVR and FR sequences generally appear in the following sequence in VH (or VL): FR1-H1(L1)-FR2-H2(L2)-FR3-H3(L3)-FR4.

### Antibody

In one embodiment, a polypeptide of the present disclosure is an antibody. The term "antibody" herein is used in the broadest sense and encompasses various antibody structures, including but not limited to monoclonal antibodies, polyclonal antibodies, monospecific antibodies, and antibody fragments so long as they exhibit the desired antigen-binding activity.

### Class of antibody

In one embodiment, a polypeptide of the present disclosure is an antibody, which is not limited to a particular class of antibody.

The "class" of an antibody refers to the type of constant domain or constant region possessed by its heavy chain. There are five major classes of antibodies: IgA, IgD, IgE, IgG, and IgM, and several of these may be further divided into subclasses (isotypes), e.g., IgG1, IgG2, IgG3, IgG4, IgA1, and IgA2. The heavy chain constant domains that correspond to the different classes of immunoglobulins are called alpha, delta, epsilon, gamma, and mu, respectively.

### EU numbering

Unless otherwise indicated, amino acid residues in the light chain constant region are numbered herein according to Kabat et al., and numbering of amino acid residues in the heavy chain constant region is according to the EU numbering system, also called the EU index, as described in Kabat et al., Sequences of Proteins of Immunological Interest, 5th Ed. Public Health Service, National Institutes of Health, Bethesda, MD, 1991.

### Chimeric antibody

In one embodiment, a polypeptide of the present disclosure may be a chimeric antibody.

The term "chimeric" antibody refers to an antibody in which a portion of the heavy and/or light chain is derived from a particular source or species, while the remainder of the heavy and/or light chain is derived from a different source or species. Similarly, the term "chimeric antibody variable domain" refers to an antibody variable region in which a portion of the heavy and/or light chain variable region is derived from a particular source or species, while the remainder of the heavy and/or light chain variable region is derived from a different source or species.

### Humanized antibody

In one embodiment, a polypeptide of the present disclosure may be a humanized antibody.

A "humanized" antibody refers to a chimeric antibody comprising amino acid residues from non-human HVRs and amino acid residues from human FRs. In certain embodiments, a humanized antibody will comprise substantially all of at least one, and typically two, variable domains, in which all or substantially all of the HVRs (e.g., CDRs) correspond to those of a non-human antibody, and all or substantially all of the FRs correspond to those of a human antibody. A humanized antibody optionally may comprise at least a portion of an antibody constant region derived from a human antibody.

### Human antibody

In one embodiment, a polypeptide of the present disclosure may be a human antibody.

A "human antibody" is one which possesses an amino acid sequence which corresponds to that of an antibody produced by a human or a human cell or derived from a non-human source that utilizes human antibody repertoires or other human antibody-encoding sequences.

### Nucleic acid (polynucleotide)

In one embodiment, the present disclosure relates to nucleic acids encoding a polypeptide of the present disclosure.

In one embodiment, a nucleic acid is RNA, DNA, or a vector or plasmid carrying the nucleic acid.

"Nucleic acid" or "polynucleotide" as used interchangeably herein, refers to polymers of nucleotides of any length, and include DNA and RNA. The nucleotides can be deoxyribonucleotides, ribonucleotides, modified nucleotides or bases, and/or their analogs, or any substrate that can be incorporated into a polymer by DNA or RNA polymerase or by a synthetic reaction. A polynucleotide may comprise modified nucleotides, such as methylated nucleotides and their analogs. A sequence of nucleotides may be interrupted by non-nucleotide components. A polynucleotide may comprise modification(s) made after synthesis, such as conjugation to a label.

In one embodiment, RNAs of the present disclosure include, for example, mRNAs, genomic RNAs, and circular RNAs.

A polynucleotide of the present disclosure may comprise a 5'-cap structure. The "5'-cap structure" is, for example, a 5'-modified nucleotide, particularly a guanine nucleotide, positioned at the 5'-terminus of RNA, e.g., mRNA. A 5'-cap structure may be linked via a 5'-5'-triphosphate linkage. Examples of the 5'-cap structure are cap0 (methylation of the first nucleobase, e.g., m7GpppN), cap1 (additional methylation of the ribose in the nucleotide adjacent to m7GpppN), cap2 (additional methylation of the ribose of the second nucleotide downstream of m7GpppN), cap3 (additional methylation of the ribose of the third nucleotide downstream of m7GpppN), cap4 (additional methylation of the ribose of the fourth nucleotide downstream of m7GpppN), ARCA (anti-reverse cap analog), modified ARCA (e.g., phosphorothioate-modified ARCA), inosine, N1-methyl-guanosine, 2'-fluoro-guanosine, 7-deaza-guanosine, 8-oxo-guanosine, 2-amino-guanosine, LNA-guanosine, and 2-azido-guanosine.

A polynucleotide of the present disclosure may comprise at least one poly(A) sequence and/or at least one poly(C) sequence and/or at least one histone step-loop sequence/structure.

A polynucleotide of the present disclosure may comprise a 5'-untranslated region (UTR) and/or a 3'-untranslated region (UTR). In one embodiment, an untranslated region may include a structure that promotes translation, such as an IRES (internal ribosome entry site). In one embodiment, a nucleic acid encoding a polypeptide of the present disclosure is RNA (e.g., mRNA). In one embodiment, the RNA comprises one or more of the following elements: a coding region (coding sequence; CDS), 5'UTR, 3'UTR, Cap, and poly-A tail. In one embodiment, the coding region comprises a start codon and a stop codon. In an RNA of the present disclosure, in one embodiment, one or more or all of the uridines may be substituted with pseudouridines (N1-methyl pseudouridines).

In one embodiment, a nucleic acid of the present disclosure is DNA. In one embodiment, the DNA is configured such that the aforementioned RNA of the present disclosure is transcribed. In one embodiment, a DNA of the present disclosure is a vector DNA or a plasmid DNA. In one embodiment, the vector or plasmid comprises one or more of the following elements: an ORF (a region encoding a polypeptide of the present disclosure), a promoter, Kozak, a marker gene (in one embodiment, a drug selection gene, a fluorescent marker gene, a reporter gene, or such).

A polynucleotide of the present disclosure may be formulated with a transport agent. The transport agent is, for example, a lipidoid, liposome, lipoplex, lipid nanoparticle, polymeric compound, peptide, protein, cell, nanoparticle mimic, nanotube, or conjugate. A transport agent may contain a lipid. The lipid may be selected from the group consisting of cholesterol, fecosterol, sitosterol, ergosterol, campesterol, stigmasterol, brassicasterol, tomatidine, ursolic acid, alpha-tocopherol, and mixtures thereof.

### Vector

In one embodiment, the present disclosure relates to a vector carrying a nucleic acid of the present disclosure.

The term "vector," as used herein, refers to a nucleic acid molecule capable of propagating another nucleic acid to which it is linked. The term includes the vector as a self-replicating nucleic acid structure as well as the vector incorporated into the genome of a host cell into which it has been introduced. Certain vectors are capable of directing the expression of nucleic acids to which they are operatively linked. Such vectors are referred to herein as "expression vectors." Vectors could be introduced into host cells using virus or electroporation. However, introduction of vectors is not limited to *in vitro* method. For example, vectors could also be introduced into a subject using *in vivo* method directly.

### Host cell

In one embodiment, the present disclosure relates to host cells into which a nucleic acid of the present disclosure has been introduced. In one embodiment, a host cell is a cell expressing a polypeptide of the present disclosure. The cell may be a eukaryotic cell or a prokaryotic cell.

The terms "host cell," "host cell line," and "host cell culture" are used interchangeably and refer to, for example, cells into which exogenous nucleic acid has been introduced, including the progeny of such cells. Host cells include "transformants" and "transformed cells," which include the primary transformed cell and progeny derived therefrom without regard to the number of passages. Progeny may not be completely identical in nucleic acid content to a parent cell, but may contain mutations.

Further, in one embodiment, the present disclosure relates to polypeptides expressed from the aforementioned host cell.

### Fc region

In the present disclosure, the term "Fc region" or "Fc domain" is used interchangeably, and refers to a region comprising a fragment consisting of a hinge or a portion thereof and CH2 and CH3 domains in an antibody molecule. The Fc region of IgG class means, but is not limited to, a region from, for example, cysteine 226 (EU numbering) to the C terminus or proline 230 (EU numbering) to the C terminus. The Fc region can be preferably obtained by the partial digestion of, for example, an IgG1, IgG2, IgG3, or IgG4 monoclonal antibody with a proteolytic enzyme such as pepsin followed by the re-elution of a fraction adsorbed on a protein A column or a protein G column. Such a proteolytic enzyme is not particularly limited as long as the enzyme is capable of digesting a whole antibody to restrictively form Fab or F(ab')₂ under appropriately set reaction conditions (e.g., pH) of the enzyme. Examples thereof can include pepsin and papain.

In one embodiment, an Fc region derived from, for example, naturally occurring IgG can be used as an "Fc region" prior to modification. In this context, the naturally occurring IgG means a polypeptide that contains an amino acid sequence identical to that of IgG found in nature and belongs to a class of an antibody substantially encoded by an immunoglobulin gamma gene. The naturally occurring human IgG means, for example, naturally occurring human IgG1, naturally occurring human IgG2, naturally occurring human IgG3, or naturally occurring human IgG4. The naturally occurring IgG also includes variants or the like spontaneously derived therefrom. A plurality of allotype sequences based on gene polymorphism are described as the constant regions of human IgG1, human IgG2, human IgG3, and human IgG4 antibodies in Sequences of proteins of immunological interest, NIH Publication No. 91-3242, any of which can be used in the present disclosure. Particularly, the sequence of human IgG1 may have DEL or EEM as an amino acid sequence of EU numbering positions 356 to 358.

In one embodiment described herein, the Fc region is an IgG Fc region. In one embodiment, the Fc region is an Fc region of IgG1, IgG2, IgG3, or IgG4. Furthermore, in one embodiment, the Fc region is a human IgG1 Fc region.

In one embodiment, a polypeptide having an Fc region of the present disclosure shows a binding affinity for human FcRn or FcγR that is comparable to that of a naturally-occurring (wild-type) human IgG1 Fc region.

In one embodiment, a polypeptide having an Fc region of the present disclosure shows a binding affinity for human FcRn or FcγR that is similar to that of the polypeptide prior to modification.

In one embodiment, an Fc region (Fc domain) exhibits a binding affinity for an Fc receptor that is similar to that of a naturally-occurring IgG1 Fc region. In one embodiment, an Fc region (or a polypeptide having the Fc region) exhibits 80% or higher, preferably 90% or higher, more preferably 95% or higher, and most preferably 98% or higher binding affinity for an Fc receptor, as compared to a naturally-occurring IgG1 Fc region (or a polypeptide comprising a naturally-occurring IgG1 Fc region).

In one embodiment, an Fc region (or a polypeptide having the Fc region) binds to an Fc receptor. In one embodiment, the Fc receptor is an Fcγ receptor. In one embodiment, the Fc receptor is a human Fc receptor. In one embodiment, the Fc receptor is an activating Fc receptor. In a specific embodiment, the Fc receptor is an activating human Fcγ receptor, more specifically human FcγRIIIa, FcγRI, or FcγRIIa.

In one embodiment, the Fc region (Fc domain) of a polypeptide of the present disclosure comprises a modification that does not reduce the binding affinity of the Fc region for an Fc receptor. In one embodiment, a modification in a polypeptide of the present disclosure does not reduce the binding affinity of the Fc region for human FcRn. In embodiment, such modifications include those shown in Table 8.

Modified Fc regions (mutant Fc domains) can be prepared by amino acid deletion, substitution, insertion or modification using genetic or chemical methods well known in the art. Genetic methods may include site-specific mutagenesis of the encoding DNA sequence, PCR, gene synthesis, and the like. The correct nucleotide changes can be verified for example by sequencing.

Binding to Fc receptors can be easily determined e.g. by ELISA, or by Surface Plasmon Resonance (SPR) using standard instrumentation such as a BIAcore instrument (GE Healthcare), and Fc receptors and such may be obtained by recombinant expression.

### Fc receptor

The term "Fc receptor" or "FcR" refers to a receptor that binds to the Fc region of an antibody. In some embodiments, an FcR is a naturally-occurring human FcR. In some embodiments, an FcR is one which binds an IgG antibody (a gamma receptor) and includes receptors of the FcγRI, FcγRII, and FcγRIII subclasses, including allelic variants and alternatively spliced forms of those receptors. FcyRII receptors include FcγRIIA (an "activating receptor") and FcγRIIB (an "inhibiting receptor"), which have similar amino acid sequences that differ primarily in the cytoplasmic domains thereof. Activating receptor FcγRIIA contains an immunoreceptor tyrosine-based activation motif (ITAM) in its cytoplasmic domain. Inhibiting receptor FcγRIIB contains an immunoreceptor tyrosine-based inhibition motif (ITIM) in its cytoplasmic domain. (see, e.g., Daeron, Annu. Rev. Immunol. 15:203-234 (1997)). FcRs are reviewed, for example, in Ravetch and Kinet, Annu. Rev. Immunol 9:457-92 (1991); Capel et al., Immunomethods 4:25-34 (1994); and de Haas et al., J. Lab. Clin. Med. 126:330-41 (1995). Other FcRs, including those to be identified in the future, are encompassed by the term "Fc receptor" herein.

The term "Fc receptor" or "FcR" also includes the neonatal receptor, FcRn, which is responsible for the transfer of maternal IgGs to the fetus (Guyer et al., J. Immunol. 117:587 (1976) and Kim et al., J. Immunol. 24:249 (1994)) and regulation of homeostasis of immunoglobulins. Methods of measuring binding to FcRn are known (see, e.g., Ghetie and Ward., Immunol. Today 18(12):592-598 (1997); Ghetie et al., Nature Biotechnology, 15(7):637-640 (1997); Hinton et al., J. Biol. Chem. 279(8):6213-6216 (2004); WO 2004/92219 (Hinton et al.).

### Fcγ receptor

Fcy receptor refers to a receptor capable of binding to the Fc domain of monoclonal IgG1, IgG2, IgG3, or IgG4 antibodies, and includes all members belonging to the family of proteins substantially encoded by an Fcy receptor gene. In human, the family includes FcyRI (CD64) including isoforms FcγRIa, FcγRIb and FcγRIc; FcγRII (CD32) including isoforms FcyRIIa (including allotype H131 and R131), FcyRIIb (including FcγRIIb-1 and FcyRIIb-2), and FcyRIIc; and FcγRIII (CD16) including isoform FcγRIIIa (including allotype V158 and F158) and FcγRIIIb (including allotype FcγRIIIb-NA1 and FcγRIIIb-NA2); as well as all unidentified human Fcγ receptors, Fcγ receptor isoforms, and allotypes thereof. However, Fcγ receptor is not limited to these examples. Without being limited thereto, Fcγ receptor includes those derived from humans, mice, rats, rabbits, and monkeys. Fcy receptor may be derived from any organisms. Mouse Fcy receptor includes, without being limited to, FcγRI (CD64), FcγRII (CD32), FcγRIII (CD16), and FcγRIII-2 (CD16-2), as well as all unidentified mouse Fcγ receptors, Fcγ receptor isoforms, and allotypes thereof. Such preferred Fcγ receptors include, for example, human FcyRI (CD64), FcγRIIA (CD32), FcγRIIB (CD32), FcγRIIIA (CD16), and/or FcyRIIIB (CD16). The polynucleotide sequence and amino acid sequence of FcγRI are shown in RefSeq accession number NM_000566.3 and RefSeq accession number NP_000557.1, respectively; the polynucleotide sequence and amino acid sequence of FcγRIIA are shown in RefSeq accession number BC020823.1 and RefSeq accession number AAH20823.1, respectively; the polynucleotide sequence and amino acid sequence of FcγRIIB are shown in RefSeq accession number BC146678.1 and RefSeq accession number AAI46679.1, respectively; the polynucleotide sequence and amino acid sequence of FcγRIIIA are shown in RefSeq accession number BC033678.1 and RefSeq accession number AAH33678.1, respectively; and the polynucleotide sequence and amino acid sequence of FcγRIIIB are shown in RefSeq accession number BC128562.1 and RefSeq accession number AAI28563.1, respectively. Whether an Fcγ receptor has binding activity to the Fc domain of a monoclonal IgG1, IgG2, IgG3, or IgG4 antibody can be assessed by ALPHA screen (Amplified Luminescent Proximity Homogeneous Assay), surface plasmon resonance (SPR)-based BIACORE method, and others (Proc. Natl. Acad. Sci. USA (2006) 103(11), 4005-4010), in addition to the above-described FACS and ELISA formats.

### Fcγ receptor-binding activity

The binding activity of Fc region (Fc domain) to any of the Fcy receptors FcγRI, FcyRIIA, FcyRIIB, FcyRIIIA, and/or FcγRIIIB can be assessed by using the above-described FACS and ELISA formats as well as ALPHA screen (Amplified Luminescent Proximity Homogeneous Assay) and surface plasmon resonance (SPR)-based BIACORE method (Proc. Natl. Acad. Sci. USA (2006) 103(11), 4005-4010).

ALPHA screen is performed by the ALPHA technology based on the principle described below using two types of beads: donor and acceptor beads. A luminescent signal is detected only when molecules linked to the donor beads interact biologically with molecules linked to the acceptor beads and when the two beads are located in close proximity. Excited by laser beam, the photosensitizer in a donor bead converts oxygen around the bead into excited singlet oxygen. When the singlet oxygen diffuses around the donor beads and reaches the acceptor beads located in close proximity, a chemiluminescent reaction within the acceptor beads is induced. This reaction ultimately results in light emission. If molecules linked to the donor beads do not interact with molecules linked to the acceptor beads, the singlet oxygen produced by donor beads do not reach the acceptor beads and chemiluminescent reaction does not occur.

For example, a biotin-labeled antigen-binding molecule or antibody is immobilized to the donor beads and glutathione S-transferase (GST)-tagged Fcγ receptor is immobilized to the acceptor beads. In the absence of a polypeptide comprising a competitive mutant Fc domain, Fcy receptor interacts with a polypeptide comprising a wild-type Fc region, inducing a signal of 520 to 620 nm as a result. The polypeptide having a non-tagged mutant Fc region competes with the polypeptide comprising a wild-type Fc region for the interaction with Fcy receptor. The relative binding affinity can be determined by quantifying the reduction of fluorescence as a result of competition. Methods for biotinylating polypeptides such as antibodies using Sulfo-NHS-biotin or the like are known. Appropriate methods for adding the GST tag to an Fcy receptor include methods that involve fusing polypeptides encoding Fcγ receptor and GST in-frame, expressing the fused gene using cells introduced with a vector carrying the gene, and then purifying using a glutathione column. The induced signal can be preferably analyzed, for example, by fitting to a one-site competition model based on nonlinear regression analysis using software such as GRAPHPAD PRISM (GraphPad; San Diego).

One of the substances for observing their interaction is immobilized as a ligand onto the gold thin layer of a sensor chip. When light is shed on the rear surface of the sensor chip so that total reflection occurs at the interface between the gold thin layer and glass, the intensity of reflected light is partially reduced at a certain site (SPR signal). The other substance for observing their interaction is injected as an analyte onto the surface of the sensor chip. The mass of immobilized ligand molecule increases when the analyte binds to the ligand. This alters the refraction index of solvent on the surface of the sensor chip. The change in refraction index causes a positional shift of SPR signal (conversely, the dissociation shifts the signal back to the original position). In the Biacore system, the amount of shift described above (i.e., the change of mass on the sensor chip surface) is plotted on the vertical axis, and thus the change of mass over time is shown as measured data (sensorgram). Kinetic parameters (association rate constant (ka) and dissociation rate constant (kd)) are determined from the curve of sensorgram, and affinity (KD) is determined from the ratio between these two constants. Inhibition assay is preferably used in the BIACORE methods. Examples of such inhibition assay are described in Proc. Natl. Acad. Sci. USA (2006) 103(11), 4005-4010.

### Maintenance/retention of Fc functions and physicochemical properties

In one embodiment, a polypeptide having a modified Fc region of the present disclosure retains a function of Fc.

In one embodiment, a polypeptide having a modified Fc region of the present disclosure maintains a physicochemical property of a polypeptide having a wild-type Fc region (in one embodiment, a wild-type IgG antibody, e.g., wild-type IgG1). In one embodiment, the physicochemical properties include the monomer ratio, thermal stability (e.g., thermal transition midpoint (Tm) value), antibody yield, etc., of the polypeptide. In one embodiment, the monomer ratio, Tm value, and polypeptide (antibody) yield of a polypeptide having a modified Fc region of the present disclosure (in one embodiment, an antibody) are not changed, not decreased, or not greatly decreased, as compared to the polypeptide (antibody) before modification.

In one embodiment, "the monomer ratio of a polypeptide is not greatly decreased" means that the monomer ratio is not decreased by 50% or more, preferably 30% or more, more preferably 10% or more, or yet more preferably 5% or more, as compared to a polypeptide having a wild-type Fc region.

The monomer ratio of a polypeptide is measured by the following method: It is evaluated by size exclusion chromatography (SEC) using ACQUITY UPLC H-Class (Waters), in which 50 mM phosphate buffer containing 300 mM sodium chloride, pH7.0 (Isekyu) is used as a running buffer, and TSKgel SuperSW3000 custom-made column (4.6 mm x 15 cm, 4µm, Gel Lot 89R)(TOSOH) is used as an analysis column. Chromatograms are recorded at a wavelength of UV 215 nm. Samples are diluted at 0.1 mg/mL, and 10 µL is injected. The column temperature is set to 30°C, and measurement is performed at a flow rate of 0.35 mL/min for 10 minutes. Data is analyzed using Empower3 (Waters). The peak area ratios (%) of the monomer, associated multimers, and degradation products of the antibody as estimated from an antibody reference sample (e.g., Tocilizumab) are calculated, and the monomer ratio is determined.

In one embodiment, "the Tm of a polypeptide is not greatly decreased" means that the Tm is not decreased by 30°C or more, preferably 20°C or more, more preferably 10°C or more, even more preferably 5°C or more, or yet more preferably 2°C or more, as compared to a polypeptide having a wild-type Fc region.

The Tm of a polypeptide (antibody Fc region) is evaluated by differential scanning fluorimetry (DSF). It has already been reported that Tm determined by this method shows good correlation with Tm determined by differential scanning calorimetry, which is a widely-known method for evaluating the thermal stability of antibodies (Journal of Pharmaceutical Science 2010;4: 1707-1720). A 5000x concentrate of SYPRO orange protein gel stain (Invitrogen) was diluted 50 times with 0.5 M PBS (Sigma), and 18 µL of Fc-containing polypeptide solution diluted to 0.1 mg/mL was mixed with 2 µL of this detection dye. The 20-µL mixture was dispensed into a tube for measurement, and its temperature was raised from 30°C to 99°C at a rate of 240°C/hr using Rotor-Gene Q (QIAGEN). Fluorescent changes with increasing temperature were observed at 470 nm (excitation wavelength) / 555 nm (fluorescence wavelength). Obtained data was used to determine the temperature at which fluorescent transition was seen using Rotor-Gene Q Series Software (QIAGEN), and the lowest value (Tm1) was determined as the Tm value derived from the Fc region. However, if the Tm value derived from the polypeptide domain fused to the Fc region is comparable to or lower than the Tm value of the Fc, the Tm1 value cannot be said to be the Tm derived from the Fc. In this case, it is necessary to assign the Tm derived from the Fc region (CH3, CH2) separately by preparing and comparing the Fc domain alone, or enzymatically cleaving the polypeptide domain fused to the Fc region before measurement.

In one embodiment, "the yield of a polypeptide (antibody) is not greatly decreased" means that the yield of the polypeptide (antibody) is not decreased by 95% or more, preferably 80% or more, more preferably 50% or more, even more preferably 30% or more, or yet more preferably 10% or more, as compared to a polypeptide having a wild-type Fc region.

The yield of a polypeptide (antibody) can be determined by the following method: One mL of 2E+6 cells/mL of Expi293-F cells (Thermo Fisher Scientific) are transfected with a total of 1 µg of plasmids encoding the full-length heavy and light chains of an antibody at a mass ratio of 1:1 or 1:2, and allowed to express them transiently. For transfection, ExpiFectamine293 transfection kit (Thermo Fisher Scientific) is used. After 4 days of transfection, the culture supernatant was collected and purified using MonoSpin ProA (GL science) or MonoSpin ProG (GL science). The amount of the antibody thus purified (mg) is determined as the yield of the polypeptide (antibody). A polypeptide having a wild-type Fc region and a polypeptide having amino acid modifications in the Fc region can be expressed and purified by the same method and their yields can compared to calculate the expression decrease ratio.

In one embodiment, the expression level of a polypeptide (in one embodiment, an antibody) having a modified Fc region of the present disclosure is comparable to that of a wild-type polypeptide (e.g., IgG1). The ratio of monomer formation is also high, and the Tm of the Fc region is also comparable.

In one embodiment, a polypeptide having a modified Fc region of the present disclosure maintains a Fc function of a polypeptide having a wild-type Fc region (in one embodiment, a wild-type IgG antibody, e.g., wild-type IgG1). In one embodiment, the Fc functions include the ability to bind to FcRn or FcyR. In one embodiment, the FcRn-binding ability and/or FcγR-binding ability of a polypeptide (in one embodiment, an antibody) having a modified Fc region of the present disclosure is not changed, not decreased, or not greatly decreased due to modification in the CH3 interface, as compared to the polypeptide (antibody) prior to modification.

In one embodiment, "the FcRn-binding ability of a polypeptide is not greatly decreased" means that the KD value for FcRn does not become 50 times or more greater, preferably 10 times or more greater, more preferably 2 times or more greater, or even more preferably 1.1 times or more greater than that of a polypeptide having a wild-type Fc region.

The KD value for FcRn can be determined by the following method: Binding to human neonatal Fc receptor (FcRn) is evaluated using Biacore T200 (Cytiva). Evaluation is performed at 25°C using 50 mM phosphate buffer, 150 mM NaCl, 0.05 w/v%-P20, pH6.0, as a running buffer. rProtein L (BioVision) is immobilized onto Series S CM4 (Cytiva) as a ligand-capturing molecule. However, in the case of an antibody molecule that does not bind to Protein L, other capturing molecules such as Protein A and Protein G may be used. An antibody solution prepared with the running buffer is allowed to interact with this CM4 sensor chip to capture about 400 RU of the antibody. The human FcRn protein used in this measurement is prepared by the method described in WO2010107110. Human FcRn is diluted to 0, 250, 500, 1000, 2000, and 4000 nM with the running buffer and allowed to bind to the captured antibody. The chip is regenerated using 10 mM Glycine-HCl (pH 1.5) and repeatedly used to capture antibodies for measurement. The KD (M) of each antibody for FcRn is calculated using Biacore T200 Evaluation Software 3.2.1 with a steady-state model. By calculating the ratio of the KD value of a polypeptide having amino acid modification in the Fc region to that of a polypeptide having a wild-type Fc region, the binding ability to human FcRn can be compared.

In one embodiment, "the FcγR-binding ability of a polypeptide is not greatly decreased" means that the level of binding to FcγR per amount of captured antibody does not become 1/10 or lower, preferably 1/5 or lower, more preferably 1/2 or lower, or even more preferably 2/3 or lower due to modification in the CH3 interface as compared to a polypeptide having a wild-type Fc region.

The level of binding to FcγRs per amount of captured antibody can be measured by the following method: The binding activity of the produced Fc-modified antibodies to each human Fcy receptor is evaluated using Biacore T200 (Cytiva). Evaluation was performed at 25°C using 50 mM phosphate buffer, 150 mM NaCl, 0.05 w/v%-P20, pH7.4, as a running buffer. rProtein L (BioVision) was immobilized onto Series S CM4 (Cytiva) as a ligand-capturing molecule. An antibody solution prepared with the running buffer was allowed to interact with this CM4 sensor chip to capture about 500 RU of the antibody in the case of measurement for human FcyRIa, and 2000 RU in the case of measurement for the other human FcγRs. The human FcγR proteins used in this measurement are prepared by the method described in WO2022220275. Human FcγR was diluted with the running buffer to 8 nM in the case of FcγRIa or 1000 nM in the case of the other FcγRs, and allowed to bind to the captured antibody. The chip is regenerated using 10 mM Glycine-HCl (pH 1.5) and repeatedly used to capture antibodies for measurement. The binding activity of each antibody to each FcγR was evaluated by calculating the level of FcγR-binding (RU) per unit amount of antibody using Biacore T200 Evaluation Software version 3.2.1. By calculating the ratio of the level of FcγR binding (RU) per unit amount of antibody of a polypeptide having amino acid modification in the Fc region to that of a polypeptide having a wild-type Fc region, the ability of binding to the human FcγR can be compared.

In one embodiment, a polypeptide (in one embodiment, an antibody) having a modified Fc region of the present disclosure has both an FcRn-binding ability and an FcγR-binding ability comparable to those of a wild-type polypeptide (e.g., IgG1).

### Affinity

In one embodiment, polypeptides of the present disclosure have affinity between the polypeptides (e.g., between CH3).

In one embodiment, a polypeptide of the present disclosure binds to an FcRn or an FcγR. This binding can be evaluated from the affinity of the polypeptide of the present disclosure for FcRn or FcγR.

"Affinity" refers to the strength of noncovalent interactions between a single binding site of a molecule (e.g., a polypeptide or antibody) and its binding partner (e.g., FcRn). The affinity of a molecule X for its partner Y can generally be represented by the dissociation constant (KD), which is the ratio of dissociation and association rate constants (koff and kon, respectively). Affinity can be measured by well-established methods known in the art, including those described herein. A particular method for measuring affinity is Surface Plasmon Resonance (SPR).

In one embodiment, KD is measured by a radiolabeled antigen binding assay (RIA). In one embodiment, an RIA is performed with the Fab version of an antibody of interest and its antigen. For example, solution binding affinity of a polypeptide for an assay target is measured by equilibrating the polypeptide with a minimal concentration of (¹²⁵I)-labeled antigen in the presence of a titration series of unlabeled antigen, then capturing the bound assay target with an anti-polypeptide antibody-coated plate (see, e.g., Chen et al., J. Mol. Biol. 293:865-881(1999)).

According to another embodiment, Kd is measured using a BIACORE^{®} surface plasmon resonance assay, such as an assay using BIACORE^{®}-2000 or BIACORE^{®}-3000 (BIAcore, Inc., Piscataway, NJ).

### Control of association

In one embodiment, the control of association of the present disclosure is control of association between polypeptides of the same type. In one embodiment, it is control of association between heavy chains of the same type or between CH3 domains of the same type.

In one embodiment, the association between a polypeptide having a modified Fc region and a polypeptide having a wild-type Fc region is controlled.

In one embodiment, the association between modified CH3 and wild-type CH3 is controlled.

In one embodiment, the control of association of the present disclosure includes control of association between any polypeptides, for example, any polypeptides comprising an Fc region.

The "control" of association, in one embodiment, refers to the promotion of association between polypeptides into which a modification of the present disclosure has been introduced, or the promotion or elevation of formation of a homodimer of a polypeptide of the present disclosure. (The formation of a homodimer mentioned here refers to formation of a dimer molecule by association of two identical polypeptides. In the case of antibody, it particularly refers to a molecule comprising a component in which any two identical polypeptides comprising an Fc region are associated.) In one embodiment, the "control" of association is suppression or inhibition of formation of a heterodimer of a polypeptide of the present disclosure. The formation of a heterodimer mentioned here refers to a dimer molecule formed by association of two different polypeptides. In the case of antibody, it particularly refers to a molecule comprising a component in which any two different polypeptides comprising an Fc region are associated. Alternatively, it may be referred to as a heteromultimer as a component in which a plurality of different polypeptides are associated.

### Polypeptides into which modifications have been introduced

In one embodiment, the present disclosure relates to a polypeptide comprising an Fc region into which a modification has been introduced (also referred to as "a polypeptide of the present disclosure").

In one embodiment, a polypeptide of the present disclosure is an antibody, and may have a variable region or a variable domain.

In one embodiment, the present disclosure relates to a polypeptide having a heavy chain Fc region or heavy chain CH3 into which a modification has been introduced.

In one embodiment, a polypeptide of the present disclosure has a heavy chain Fc region. In one embodiment, a polypeptide comprises, e.g., a CH3 region.

In one embodiment, a polypeptide of the present disclosure is an antibody heavy chain. A polypeptide of the present disclosure may have an antigen-binding domain, a heavy chain variable region, and/or a light chain variable region.

In one embodiment, a polypeptide comprising an Fc region into which a modification has been introduced according to the present disclosure associates more readily with a polypeptide having the modification than with a polypeptide not having the modification.

In one embodiment, a polypeptide comprising an Fc region into which a modification has been introduced according to the present disclosure associates less readily with a polypeptide not having the modification than with a polypeptide having the modification.

In one embodiment, a polypeptide comprising an Fc region into which a modification has been introduced according to the present disclosure less readily forms a heterodimer with another polypeptide not having the modification. In one embodiment, a polypeptide of the present disclosure less readily forms a heterodimer than a homodimer. In one embodiment, a polypeptide of the present disclosure more readily forms a homodimer than a heterodimer.

In one embodiment, a polypeptide comprising an Fc region into which a modification has been introduced according to the present disclosure has an increased ability to form an associated multimer with a polypeptide having the modification as compared to a polypeptide not having the modification.

In one embodiment, a polypeptide comprising an Fc region into which a modification has been introduced according to the present disclosure associates with a polypeptide having the modification.

In one embodiment, a polypeptide of the present disclosure forms a homomer (homodimer, homodimerized molecule).

In one embodiment, a polypeptide of the present disclosure shows a stronger homodimerization-promoting ability than a control (e.g., the polypeptide before modification, wild-type IgG, or such).

In one embodiment, a polypeptide of the present disclosure is a polypeptide for which association between polypeptides is controlled. In one embodiment, the association is association between Fc regions or between CH3 domains.

In one embodiment, a polypeptide of the present disclosure does not associate, or associates less readily, with endogenous IgG or antibody fragments containing an Fc region thereof.

### Modifications to be introduced

In one embodiment, because of one of the following actions due to an introduced modification, a polypeptide of the present disclosure associates more readily with a polypeptide having the modification than with a polypeptide not having the modification: (1) steric complementarity (also referred to as "steric hindrance"), (2) disulfide linkage (also referred to as "disulfide bond"), and (3) electrostatic charge (also referred to as "charge").

In one embodiment, a modification introduced into a polypeptide of the present disclosure causes (1) steric complementarity (also referred to as "steric hindrance"), (2) disulfide linkage (also referred to as "disulfide bond"), or (3) electrostatic charge (also referred to as "charge").

### Steric complementarity (steric hindrance control)

In one embodiment, a modification that causes steric complementarity (steric hindrance) according to the present disclosure includes modifying an amino acid residue(s) present in a region where association between polypeptides occurs (e.g., CH3 interface) to a bulky amino acid and/or a small amino acid. In one embodiment, modifications to a bulky amino acid and a small amino acid are carried out concurrently. In one embodiment, such a modification includes replacing an original small amino acid or non-bulky amino acid prior to modification with a bulky amino acid, or replacing an original bulky amino acid or non-bulky amino acid with a small amino acid.

In one embodiment, "bulky amino acids" include those with a larger molecular weight than the amino acid prior to modification. Bulky amino acids include, for example, tyrosine (Y), tryptophan (W), arginine (R), histidine (H), phenylalanine (F), leucine (L), valine (V), isoleucine (I), methionine (M), serine (S), threonine (T), cysteine (C), asparagine (N), glutamine (Q), lysine (K), aspartic acid (D), and glutamic acid (E).

In one embodiment, "small amino acids" include those with a smaller molecular weight than the amino acid prior to modification. Smaller amino acids include, for example, alanine (A), threonine (T), leucine (L), valine (V), asparagine (N), serine (S), isoleucine (I), methionine (M), glycine (G), cysteine (C), glutamine (Q), lysine (K), arginine (R), histidine (H), aspartic acid (D), and glutamic acid (E).

In one embodiment, a modification introduced into a polypeptide of the present disclosure causes steric complementarity (steric hindrance). In one embodiment, steric complementarity results from introducing knob-into-hole into a polypeptide of the present disclosure.

In one embodiment, said modification is a so-called "knob-into-hole" modification, comprising a protuberance ("knob") modification and a cavity ("hole") modification in an Fc region. The knob-into-hole technology is described e.g. in US 5,731,168 ; US 7,695,936 ; Ridgway et al., Prot Eng 9, 617-621 (1996) and Carter, J Immunol Meth 248, 7-15 (2001). Protuberances (knobs) are constructed, for example, by replacing small amino acid side chains from the interface of a polypeptide of the present disclosure with larger side chains (e.g. tyrosine or tryptophan). Compensatory cavities (holes) of identical or similar size to the protuberances are created in the interface of the polypeptide, for example, by replacing large amino acid side chains with smaller ones (e.g. alanine or threonine).

In one embodiment, steric complementarity results from the presence of a knob and a hole in an Fc region. In one embodiment, a modification introduced into a polypeptide of the present disclosure is introduction of a knob and a hole into the Fc region. The knob and hole in the Fc region induces or promotes the homodimer formation of the polypeptide of the present disclosure.

In one embodiment, both a knob and a hole are introduced into the same molecule of a polypeptide of the present disclosure.

In one embodiment, in the CH3 domain of the Fc region of a polypeptide, an amino acid residue is replaced with an amino acid residue with a larger side-chain volume (knob construction), and another amino acid position in the same polypeptide is replaced with an amino acid residue with a smaller side-chain volume to create a cavity (hole construction).

The protuberance and cavity can be made by altering the nucleic acid encoding the polypeptides, e.g. by site-specific mutagenesis, or by peptide synthesis.

In one embodiment, amino acid positions to be modified for steric complementarity (steric hindrance control) include, for example, the following positions on the Fc region:
positions 347, 349, 350, 351, 354, 357, 364, 366, 368, 370, 392, 394, 399, 405, 407, and 409 (EU numbering).

In one embodiment, amino acid modifications for steric complementarity (steric hindrance control) include, for example, the following modifications (amino acid substitutions): Q347E, T350V, L351Y, S354Y, Y349T, E357N, S364H, T366W, T366Y, T366V, T366L, L368A, K370E, K392L, T394W, T394F, D399V, D399M, F405A, F405L, F405T, Y407V, Y407T, Y407A, K409R, K409W, and K409V.

In one embodiment, one or more of the above-mentioned modifications are combined.

In one embodiment, combinations of amino acid modifications for steric complementarity (steric hindrance control) include, for example, the following: T394W/F405A ("/" means that the preceding and following modifications are all included; the same applies hereinafter), T366W/L368A/Y407V, T366Y/Y407T, T366W/Y407A, T366Y/T394W/F405A/Y407T, E357N/K370E/D399V/F405T/K409W, Y349T/S364H/T394F/F405A, T394F/F405A, Q347E/S354Y/T366Y/Y407T, T350V/L351Y/T366L/T394W/F405A, K392L/T394W/F405A/Y407V, and T350V/L351Y/T366L/K392L/T394W/F405A/Y407V.

### Disulfide linkage (disulfide bond control)

In one embodiment, a modification introduced into a polypeptide of the present disclosure causes a disulfide linkage (disulfide bond) between modified polypeptides.

Modifications to introduce a disulfide linkage in a polypeptide of the present disclosure include, for example, substituting an amino acid other than cysteine with cysteine.

In one embodiment, the modification is a modification to introduce one or more cysteines into an Fc region. In one embodiment, the modification is substitution of one or more amino acids in an Fc region with cysteine.

In one embodiment, a disulfide linkage is formed between modified polypeptides, thereby stabilizing the homodimer.

In one embodiment, at least one amino acid substitution with a cysteine residue is introduced into a polypeptide comprising an Fc region, and a disulfide linkage is formed between the introduced cysteine and an existing cysteine.

In one embodiment, at least two amino acid substitutions with a cysteine residue are introduced into a polypeptide comprising an Fc region, and a disulfide linkage is formed between the introduced cysteine residues.

In one embodiment, a disulfide linkage is formed between polypeptides of the present disclosure.

In one embodiment, a disulfide linkage is formed between a modified (introduced) cysteine in one of polypeptides forming a homodimer and a cysteine at the corresponding same position in the other polypeptide.

In one embodiment, a disulfide linkage is formed between a modified (introduced) cysteine in one of polypeptides forming a homodimer and a cysteine at a different position in the other polypeptide.

In one embodiment, positions for amino acid modification to (substitution with) cysteine for disulfide linkage (disulfide bond control) include, for example, the following positions on the Fc region:
349, 351, 354, 356, 357, 392, 394, 397, and 399 (EU numbering).

In one embodiment, modifications (substitutions with cysteine) at one or more of the above-mentioned positions are combined.

In one embodiment, combinations of amino acid modifications to cysteine for disulfide linkage (disulfide bond control) include, for example, the following:
K392C/D399C, Y349C/E356C, Y349C/E357C, L351C/S354C, and T394C/V397C.

In one embodiment, these modifications are combined.

In one embodiment, combined modifications (substitutions with cysteine) include, for example, the following:
Y349C/E357C/K392C/D399C, Y349C/L351C/S354C/E357C, Y349C/E357C/T394C/V397C, Y349C/E357C/K392C/T394C/V397C/D399C, K392C/T394C/V397C/D399C, and L351C/S354C/K392C/D399C.

### Electrostatic charge (charge control)

In one embodiment, a modification introduced into a polypeptide of the present disclosure is introduction of a charged amino acid. In one embodiment, such modifications include substituting an amino acid residue in a polypeptide of the present disclosure with a charged amino acid residue.

In one embodiment, the action of electrostatic charge results from one or more charged amino acids modified (introduced) in an Fc region.

In one embodiment, "charged amino acid residues" (charged amino acids) are selected, for example, from amino acid residues included in either one of the following groups:
(a) glutamic acid (E) and aspartic acid (D); and
(b) lysine (K), arginine (R), and histidine (H).

In one embodiment, a modification introduced into a polypeptide of the present disclosure is introduction of two or more oppositely charged amino acids.

The phrase "oppositely charged" means, for example, that when at least one of the two or more amino acid residues is selected from the amino acid residues included in either one of groups (a) and (b) mentioned above, the remaining amino acid residues are selected from the amino acid residues included in the other group.

In one embodiment, a modification in a polypeptide of the present disclosure introduces a positively charged amino acid and/or a negatively charged amino acid.

In general, lysine (K), arginine (R), and histidine (H) are known as amino acids with a positive charge (positively charged amino acids). Glutamic acid (E) and aspartic acid (D) are known as amino acids with a negative charge (negatively charged amino acids).

In one embodiment, the above-mentioned positively charged amino acid is selected from lysine (K), arginine (R), and histidine (H), and/or the above-mentioned negatively charged amino acid is selected from aspartic acid (D) and glutamic acid (E).

In one embodiment, amino acid positions to be modified for electrostatic charge (charge control) include, for example, the following positions on the Fc region: positions 345, 347, 351, 356, 357, 360, 366, 370, 392, 399, 409, and 439 (EU numbering).

In one embodiment, modifications at one or more of the above-mentioned positions are combined.

In one embodiment, positions to be modified to a positively charged amino acid include, for example, the following:
positions 345, 347, 351, 356, 357, 366, and 399.

In one embodiment, positions to be modified to a negatively charged amino acid include, for example, the following:
positions 351, 360, 370, 392, 409, and 439.

In one embodiment, combinations of amino acid positions to be modified to a positively charged amino acid or a negatively charged amino acid include, for example, the following:
modification of position 345 to a positively charged amino acid and modification of position 360 to a negatively charged amino acid;
modification of position 347 to a positively charged amino acid and modification of position 360 to a negatively charged amino acid;
modification of position 357 to a positively charged amino acid and modification of position 370 to a negatively charged amino acid;
modification of position 399 to a positively charged amino acid and modification of position 409 to a negatively charged amino acid;
modification of position 399 to a positively charged amino acid and modification of position 392 to a negatively charged amino acid;
modification of position 366 to a positively charged amino acid and modification of position 351 to a negatively charged amino acid;
modification of position 351 to a positively charged amino acid and modification of position 366 to a negatively charged amino acid; and
modification of position 356 to a positively charged amino acid and modification of position 439 to a negatively charged amino acid.

In one embodiment, one or more of the above-mentioned modifications are combined. In one embodiment, the above-mentioned modifications may be combined with a further modification to a positively or negatively charged amino acid alone.

Combined modifications include, for example, the following:
"modification of position 357 to a positively charged amino acid and modification of position 370 to a negatively charged amino acid" and "modification of position 399 to a positively charged amino acid and modification of position 409 to a negatively charged amino acid";
"modification of position 356 to a positively charged amino acid and modification of position 439 to a negatively charged amino acid" and "modification of position 399 to a positively charged amino acid and modification of position 409 to a negatively charged amino acid";
"modification of position 356 to a positively charged amino acid and modification of position 439 to a negatively charged amino acid" and "modification of position 357 to a positively charged amino acid and modification of position 370 to a negatively charged amino acid";
"modification of position 356 to a positively charged amino acid and modification of position 439 to a negatively charged amino acid" and "modification of position 399 to a positively charged amino acid and modification of position 392 to a negatively charged amino acid";
"modification of position 357 to a positively charged amino acid and modification of position 370 to a negatively charged amino acid" and "modification of position 399 to a positively charged amino acid and modification of position 392 to a negatively charged amino acid";
"modification of position 399 to a positively charged amino acid and modification of position 409 to a negatively charged amino acid" and "modification of position 392 to a negatively charged amino acid";
"modification of position 399 to a positively charged amino acid and modification of position 409 to a negatively charged amino acid", "modification of position 392 to a negatively charged amino acid", and "modification of position 356 to a positively charged amino acid"; and
"modification of position 356 to a positively charged amino acid and modification of position 439 to a negatively charged amino acid", "modification of position 357 to a positively charged amino acid and modification of position 370 to a negatively charged amino acid", and "modification of position 399 to a positively charged amino acid and modification of position 409 to a negatively charged amino acid".

In one embodiment, amino acid modifications for electrostatic charge (charge control) include, for example, the following modifications:
E345K/K360E, E345R/K360E, Q347R/K360D, Q347R/K360E, E357K/K370E, D399K/K409E, D399K/K409D, D399R/K409E, D399R/K409D, K392E/D399K, K392D/D399K,
K392E/D399R, K392D/D399R, L351D/T366K, L351E/T366K, L351K/T366D, L351K/T366E, and E356K/K439E.
In one embodiment, two or more of these modification can be combined. Examples include the following:
   E357K/K370E/D399K/K409E, E356K/D399K/K409E/K439E, E356K/E357K/K370E/K439E, E356K/K392D/D399K/K439E, E357K/K370E/K392D/D399K, K392D/D399K/K409D, E356K/K392D/D399K/K409D, and E356K/E357K/K370E/D399K/K409E/K439E.

### Combinations of introduced modifications

In one embodiment, modifications introduced into a polypeptide of the present disclosure may be a combination of two or more different types of modifications.

In one embodiment, the combination of two or more different types of modifications is a combination selected from the aforementioned (1) steric complementarity, (2) disulfide linkage, and (3) electrostatic charge.

In one embodiment, the combination is a combination of (1) steric complementarity and (2) disulfide linkage, a combination of (1) steric complementarity and (3) electrostatic charge, or a combination of (2) disulfide linkage and (3) electrostatic charge.

In one embodiment, modifications introduced into a polypeptide of the present disclosure include those to introduce all of (1) steric complementarity, (2) disulfide linkage, and (3) electrostatic charge.

In the case of combining (1) steric complementarity and (3) electrostatic charge, in one embodiment, examples of modifications include, but are not limited to, the following: Q347R/K360E/D399V/F405T/K409W, E345R/Q347R/K360D/T366V/D399M/Y407A/K409V, and E345R/K360E/D399M/Y407A/K409V.

In the case of combining (2) disulfide linkage and (3) electrostatic charge, in one embodiment, examples of modifications include, but are not limited to, the following: Y349C/E357C/K392D/D399K, E357K/K370E/T394C/V397C, Y349C/E357C/K370E, E357K/K370E/K392C/D399C, and K392D/T394C/V397C/D399K.

In one embodiment, different modifications can be introduced into each of a plurality of polypeptides of the present disclosure to control the association of each polypeptide (in one embodiment, promote the homodimer formation of each polypeptide).

In one embodiment, a modification in a polypeptide of the present disclosure promotes the homodimer formation of the polypeptide. Amino acid modifications exhibiting strong homodimer formation-promoting ability include, for example, the following, but are not limited thereto (categories of modification effect shown in parentheses):
T394W/F405A (steric hindrance)
T366W/L368A/Y407V (steric hindrance)
Q347R/K360E/D399V/F405T/K409W (steric hindrance + charge)
T394F/F405A (steric hindrance)
E356K/D399K/K409E/K439E (charge)
Q347E/S354Y/T366Y/Y407T (steric hindrance)
K392L/T394W/F405A/Y407V (steric hindrance)
E356K/K392D/D399K/K439E (charge)
K392D/D399K/K409D (charge)
Y349C/E357C/K392D/D399K (charge + disulfide bond)
E345R/Q347R/K360D/T366V/D399M/Y407A/K409V (steric hindrance + charge)
E356K/K392D/D399K/K409D (charge)
E345R/K360E/D399M/Y407A/K409V (steric hindrance + charge)
T350V/L351Y/T366L/K392L/T394W/F405A/Y407V (steric hindrance).

Additionally, in one embodiment, such modifications include those shown in Tables 16-18. In one embodiment, the amino acid modifications disclosed herein can also be combined. Examples include the combinations of amino acid modifications shown in Tables 19-21.

In one embodiment, modifications introduced into a plurality of polypeptides of the present disclosure are selected from the above-mentioned (1) steric complementarity, (2) disulfide linkage, and (3) electrostatic charge. In one embodiment, when a modification for (1) steric complementarity, (2) disulfide linkage, or (3) electrostatic charge is introduced into a first polypeptide, a modification different from the one in the first polypeptide is introduced into a second polypeptide.

In one embodiment, combinations of modifications introduced into the first and second polypeptides include the combinations in Table 5 that show an amount of heterodimer of less than 52%.

In one embodiment, those combinations of modifications can be used when two antibodies are coexpressed. As shown in Example 6, pairs of modifications in such a relation as to suppress heterodimers in experimental results are expected not to cause heteromeric association no matter how many of them are combined. Therefore, such pairs can be used for not only coexpressing two antibodies but also coexpressing three, four, five or more antibodies.

### Modification of amino acid residues

A "modification" of an amino acid residue in the present disclosure specifically refers to substitution of the original amino acid residue with another amino acid residue, deletion of the original amino acid residue, addition of a new amino acid residue, and such. In one embodiment, the modification refers to substitution of the original amino acid residue with another amino acid residue.

Modifications introduced into a polypeptide of the present disclosure are not limited to only the modifications for the above-mentioned (1) steric complementarity, (2) disulfide linkage, and (3) electrostatic charge, but, in one embodiment, may further include modifications other than these modifications.

In one embodiment, when a polypeptide of the present disclosure is an antibody, the constant regions of the antibody, in particular the heavy chain constant region, may be modified as necessary in order to improve antibody functions and/or stability. Examples of modifications to improve antibody functions include modifications to enhance or attenuate the binding of the antibody with an Fcy receptor (FcyR), modifications to enhance or attenuate the binding of the antibody with an FcRn, and modifications to enhance or attenuate the cytotoxic activity of the antibody (e.g., ADCC activity, CDC activity, and such). Moreover, in one embodiment, modifications to improve the heterogeneity of the antibody and/or modifications to improve the immunogenicity and/or pharmacokinetics may be included. Moreover, in one embodiment, modifications to promote the hexamerization of the antibody and modifications to glycosylation sequences may be included.

For the heterogeneity of the heavy-chain C-terminal sequence of IgG antibody, deletion of the C-terminal lysine residue, and amidation of the C-terminal carboxyl group due to deletion of both of the C-terminal two amino acids, glycine and lysine, have been reported (Anal Biochem. 2007 Jan 1;360(1):75-83). Accordingly, when a polypeptide of the present disclosure is an antibody, in one embodiment, IgG with the C-terminal lysine or C-terminal lysine and glycine deleted can be used to reduce the C-terminal heterogeneity of the heavy chain Fc region.

### Interface

In one embodiment, the above-mentioned modifications are introduced into a region of the polypeptide that forms an interface. In one embodiment, the above-mentioned modifications are introduced into a region of the polypeptide comprising an Fc region that forms an interface in the Fc region. In one embodiment, the modifications are introduced into a region that forms the interface of CH3.

An "interface" in the present disclosure usually refers to a surface at which association (interaction) occurs. An amino acid residue(s) forming an interface usually refers to one or more amino acid residues included in a polypeptide region subjected to that association, and, in one embodiment, refers to amino acid residue(s) that come close and are involved in interaction when association occurs.

In the present disclosure, the amino acid residues in a polypeptide subjected to modification are not limited to those in the Fc region or CH3 region. In one embodiment, amino acid residues are modified in a region that forms an interface between polypeptides. Those skilled in the art can identify the amino acid residues that form an interface by homology modeling and such using commercially available software. Then, amino acid residues of these positions can be subjected to modification so as to control the association.

In one embodiment, the amino acid residues to be modified are those that come close to each other when association occurs between polypeptide regions forming an interface.

### Antibody formats subjected to modification

In one embodiment, an antibody format to be subjected to a modification of the present disclosure is an IgG format. For example, wild-type IgG, which has no modification in Fc, or IgG containing a modification in Fc, is used. In one embodiment, various antibody formats such as a VHH or scFv linked with an Fc, and antibody formats into which an amino acid modification for changing an Fc function has been introduced can be used. In one embodiment, IgG formats having a deglycosylated Fc region and/or containing modifications for enhancing the binding to an FcRn, suppressing the binding to an FcyR, and/or promoting hexamer formation may be used. In one embodiment, Ig formats containing an Fc that is linked with a polypeptide containing a Fab, VHH, or scFv, or with a polypeptide other than Fab, VHH, or scFv, can also be used. In one embodiment, IgG formats containing an amino acid modification that does not affect the Fc interface can be used.

### Coexpression of homodimer and heteromultimer

In one embodiment, an amino acid modification for promoting homodimer formation of the present disclosure can be used concurrently with an amino acid modification for promoting heterodimer formation (e.g., a knob-into-hole modification).

In one embodiment, the present disclosure relates to a method for coexpressing a homodimer and a heteromultimer.

In one embodiment, the method comprises:
the step of obtaining a nucleic acid encoding a first polypeptide;
the step of obtaining a nucleic acid encoding a second polypeptide;
the step of obtaining a nucleic acid encoding a third polypeptide; and/or
the step of expressing said nucleic acids.

Here, in one embodiment, the first polypeptide has an Fc region into which a modification has been introduced. Because of the modification introduced into the Fc region, the first polypeptide associates more readily with a first polypeptide having that modification via the Fc region than with a first polypeptide comprising an Fc region into which the modification has not been introduced.

Here, in one embodiment, the second polypeptide associates more readily with the third polypeptide than with the second polypeptide.

Here, in one embodiment, the second polypeptide has an Fc region into which a modification has been introduced. Because of the modification introduced into the Fc region, the second polypeptide associates more readily with the third polypeptide via the Fc region than with the second polypeptide.

In one embodiment, amino acid modifications for promoting heterodimer formation include the modifications shown in Example 13 described later.

### Combinations of modifications exhibiting strong suppression of heteromeric association

In one embodiment, the above-mentioned Fc region into which a modification has been introduced includes Fc regions into which a modification exhibiting strong suppression of heteromeric association (e.g., a combination of modifications) has been introduced. Combinations of modifications that exhibit strong suppression of heteromeric association include, for example, the amino acid modifications shown in Tables 19-21.

In one embodiment, the above-mentioned Fc region into which a modification has been introduced contains amino acid modifications at one combination or two or more combinations of positions selected from the combinations of positions shown in (a) to (d) below according to EU numbering:
(a) positions 394 and 405;
(b) positions 366, 368, and 407;
(c) positions 347, 360, 399, 405 and 409; and
(d) positions 356, 392, 399 and 439.

In one embodiment, the above-mentioned Fc region into which a modification has been introduced contains at least one amino acid selected from the group consisting of:
(a) W, F, or Y at position 394, and
   A, S, T, C, G, or V at position 405;
(b) W, Y, or F at position 366,
   A, S, T, C, V, or G at position 368, and
   V, L, I, M, A, S, T, C, N, or Q at position 407;
(c) R, K, Y, or H at position 347,
   E or D at position 360,
   V, L, I, M, S, T, C, H, A, N, Q, or G at position 399,
   T, A, V, S, C, N, D, or G at position 405, and
   W, F, Y, or H at position 409; and
(d) K or R at position 356,
   D or E at position 392,
   K or R at position 399, and
   E or D at position 439
according to EU numbering.

In one embodiment, the above-mentioned Fc region into which a modification has been introduced contains at least one amino acid selected from the group consisting of:
(a) W or F at position 394, and
   A, S, T, or G at position 405;
(b) W, Y, or F at position 366,
   A, T, C, V, or G at position 368, and
   V, L, I, M, A, or C at position 407;
(c) R, K, Y, or H at position 347,
   E or D at position 360,
   V, L, I, M, S, T, C, H, A, N, or G at position 399,
   T, A, or V at position 405, and
   W or F at position 409; and
(d) K at position 356,
   D at position 392,
   K at position 399, and
   E at position 439,
according to EU numbering.

In one embodiment, the above-mentioned Fc region into which a modification has been introduced contains amino acid modifications at one combination or two or more combinations of positions selected from the combinations of positions shown in (a) to (d) below according to EU numbering:
(a) positions 345, 347, 360, 366, 399, 407, and 409;
(b) positions 356, 399, 409, and 439;
(c) positions 356, 392, 399, and 409; and
(d) positions 392, 399, and 409.

In one embodiment, the above-mentioned Fc region into which a modification has been introduced contains at least one amino acid selected from the group consisting of:
(a) R or E at position 345,
   R or K at position 347,
   D or E at position 360,
   V at position 366,
   M, Q, N, H, I, F, Y, T, S, V, or L at position 399,
   A at position 407, and
   V, Q, N, H, L, I, F, Y, T, or S at position 409;
(b) K, R, or H at position 356,
   K, R, or H at position 399,
   E or D at position 409, and
   E or D at position 439,
(c) K, R, or H at position 356,
   D or E at position 392,
   K, R, or H at position 399,
   D or E at position 409; and
(d) D or E at position 392,
   K or R at position 399, and
   D or E at position 409,
according to EU numbering.

### Purification of polypeptides

Polypeptides (in one embodiment, antibodies) prepared as described herein may be purified by art-known techniques such as high performance liquid chromatography, ion exchange chromatography, gel electrophoresis, affinity chromatography, size exclusion chromatography, and the like. The actual conditions used to purify a particular protein will depend, in part, on factors such as net charge, hydrophobicity, hydrophilicity etc., and will be apparent to those having skill in the art. For affinity chromatography purification an antibody, ligand, receptor or antigen can be used to which the polypeptide binds. For example, for affinity chromatography purification of polypeptides (e.g., antibodies) of the present disclosure, a matrix with protein A or protein G may be used. Sequential Protein A or G affinity chromatography and size exclusion chromatography can be used to isolate a polypeptide. The purity of a polypeptide of the present disclosure can be determined by any of a variety of well-known analytical methods including gel electrophoresis, high pressure liquid chromatography, and the like.

### Compositions

In one embodiment, the present disclosure relates to a composition comprising a nucleic acid encoding a polypeptide of the present disclosure.

In one embodiment, the composition comprises a nucleic acid encoding a first polypeptide, wherein the first polypeptide has an Fc region into which a modification has been introduced, and wherein, because of the modification introduced into the Fc region, the first polypeptide associates more readily with a polypeptide having the modification than with a polypeptide not having the modification.

In one embodiment, the composition of the present disclosure comprises a nucleic acid encoding a second polypeptide, wherein the second polypeptide has an Fc region into which a modification has been introduced, and wherein, because of the modification introduced into the Fc region, the second polypeptide associates more readily with a polypeptide having the modification than with a polypeptide not having the modification.

In one embodiment, the composition of the present disclosure may further comprise a nucleic acid(s) encoding multiple different polypeptides different from the above-mentioned first or second polypeptide (in one embodiment, a third and/or a fourth polypeptide).

In one embodiment, a composition of the present disclosure comprises:
a nucleic acid encoding a first polypeptide;
a nucleic acid encoding a second polypeptide; and
a nucleic acid encoding a third polypeptide.

Here,
in one embodiment, the first polypeptide has an Fc region into which a modification has been introduced, and because of the modification introduced into the Fc region, the first polypeptide associates more readily with a first polypeptide having the modification via the Fc region than with a first polypeptide comprising an Fc region into which the modification has not been introduced.

In one embodiment, the second polypeptide associates more readily with the third polypeptide than with the second polypeptide.

In one embodiment, the second polypeptide has an Fc region into which a modification has been introduced, and because of the modification introduced into the Fc region, the second polypeptide associates more readily with the third polypeptide via the Fc region than with the second polypeptide.

### Pharmaceutical compositions

In one embodiment, the present disclosure provides a pharmaceutical composition comprising a nucleic acid or polypeptide (in one embodiment, an antibody) of the disclosure. In one embodiment, the present disclosure relates to a composition (pharmaceutical composition) comprising a nucleic acid or polypeptide of the disclosure and a pharmaceutically acceptable carrier.

In one embodiment, a pharmaceutical formulation of the present disclosure is prepared by mixing a polypeptide (in one embodiment, an antibody) having the desired degree of purity with one or more optional pharmaceutically acceptable carriers (Remington's Pharmaceutical Sciences 16th edition, Osol, A. Ed. (1980)), in the form of lyophilized formulations or aqueous solutions. Pharmaceutically acceptable carriers are generally nontoxic to recipients at the dosages and concentrations employed, and include, but are not limited to: buffers such as phosphate, citrate, and other organic acids; antioxidants including ascorbic acid and methionine; preservatives (such as octadecyldimethylbenzyl ammonium chloride; hexamethonium chloride; benzalkonium chloride; benzethonium chloride; phenol, butyl or benzyl alcohol; alkyl parabens such as methyl or propyl paraben; catechol; resorcinol; cyclohexanol; 3-pentanol; and m-cresol); low molecular weight (less than about 10 residues) polypeptides; proteins, such as serum albumin, gelatin, or immunoglobulins; hydrophilic polymers such as polyvinylpyrrolidone; amino acids such as glycine, glutamine, asparagine, histidine, arginine, or lysine; monosaccharides, disaccharides, and other carbohydrates including glucose, mannose, or dextrins; chelating agents such as EDTA; sugars such as sucrose, mannitol, trehalose or sorbitol; salt-forming counter-ions such as sodium; metal complexes (e.g. Zn-protein complexes); and/or non-ionic surfactants such as polyethylene glycol (PEG).

A polypeptide (in one embodiment, an antibody), a pharmaceutical composition, or such of the present disclosure is, for example, either orally or parenterally administered to a subject (in one embodiment, a patient). For example, parenteral administration is preferred. Specifically, such administration methods include injection, nasal administration, transpulmonary administration, and percutaneous administration. Injections include, for example, intravenous injections, intramuscular injections, intraperitoneal injections, and subcutaneous injections. For example, pharmaceutical compositions of the present disclosure can be administered locally or systemically by injection. Furthermore, appropriate administration methods can be selected according to the subject's (patient's) age and symptoms. The administered dose can be selected, for example, from the range of 0.0001 mg to 1,000 mg per kg of body weight for each administration. Alternatively, the dose can be selected, for example, from the range of 0.001 mg/body to 100,000 mg/body per patient. However, the dose of a pharmaceutical composition of the present disclosure is not limited to these doses.

In one embodiment, a pharmaceutical composition of the present disclosure comprises a nucleic acid of the disclosure. In one embodiment, the pharmaceutical composition comprises a nucleic acid of the disclosure included (encapsulated) in a vesicle. Such vesicles include, for example, lipid nanoparticles (LNPs), viruses, extracellular vesicles (EVs), and liposomes.

### Direct expression in subjects

If necessary, a vector comprising a nucleic acid molecule encoding a polypeptide (in one embodiment, an antibody) of the present disclosure may be introduced into a subject to express the polypeptide of the present disclosure directly within the subject. Subjects include, for example, a human, a non-human animal, an *ex vivo* cell, and an *in vitro* cell. An example of the vector that can be used is adenovirus, but is not limited thereto. It is also possible to administer a nucleic acid molecule encoding a polypeptide (in one embodiment, an antibody) of the present disclosure directly into a subject, to administer the nucleic acid molecule included (encapsulated) in a vesicle, to transfer a nucleic acid molecule encoding a polypeptide of the present disclosure via electroporation to a subject, or to administer cells comprising a nucleic acid molecule encoding a polypeptide of the present disclosure to be expressed into a subject to express the polypeptide (in one embodiment, antibody) of the present disclosure in the subject.

In one embodiment, the present disclosure relates to expression of a polypeptide for which association between Fc regions is controlled, in a living organism. Polypeptides of the present disclosure are, in one embodiment, antibodies that do not associate with wild-type IgG, thus making it possible to provide therapeutic drugs based on homogeneous, safe, multiple antibodies when antibody expression in living organisms is intended. In one embodiment, in the case of expressing therapeutic antibodies in living organisms, their not associating with endogenous IgG will lead to reduced side effects.

In one embodiment, a messenger RNA (mRNA) encoding a polypeptide of the present disclosure can be introduced into a subject (a mammal, a human, a non-human animal, or such) to promote homodimer formation of the polypeptide of the present disclosure.

In one embodiment, the introduction into a subject can be performed using mRNA-encapsulated lipid nanoparticles (mRNA-LNPs).

### Method for obtaining polypeptides for which association is controlled

In one embodiment, the present disclosure relates to a method for obtaining a polypeptide for which association between polypeptides is controlled.

In one embodiment, the method comprises:
the step of obtaining a nucleic acid encoding the polypeptide; and
the step of expressing the nucleic acid.

In one embodiment, the above-mentioned polypeptide has an Fc region, and because of the modification introduced into the Fc region, the polypeptide associates more readily with a polypeptide having the modification than with a polypeptide not having the modification.

In one embodiment, the above-mentioned expression is *in vivo, ex vivo,* or *in vitro* expression.

Herein, the term "step" is used interchangeably with "process" and "phase".

### Method for production and method for controlling association

In one embodiment, the present disclosure relates to a method for producing a polypeptide for which association is controlled. In one embodiment, the production method is a method for producing a polypeptide for which association of the Fc region is controlled.

In one embodiment, the production method comprises the following steps:
(a) obtaining a nucleic acid encoding a polypeptide into which a modification has been introduced in the Fc region;
(b) introducing the nucleic acid into a host cell and culturing the host cell to express the nucleic acid; and
(c) recovering the polypeptide from the culture of the host cell.

In one embodiment, the above-mentioned polypeptide associates more readily with a polypeptide having the modification than with a polypeptide not having the modification due to at least one of the following actions: (1) steric complementarity, (2) disulfide linkage, and (3) electrostatic charge.

In one embodiment, the above-mentioned production method further comprises the step of introducing a modification into the Fc region such that the action of (1) steric complementarity, (2) disulfide linkage, and/or (3) electrostatic charge occurs in the above-mentioned polypeptide.

In one embodiment, the production method comprises the following steps:
(a) modifying a nucleic acid encoding a polypeptide comprising an Fc region such that the polypeptide associates more readily with a polypeptide having the modification than with a polypeptide not having the modification due to at least one of the following actions: (1) steric complementarity, (2) disulfide linkage, and (3) electrostatic charge;
(b) introducing the modified nucleic acid into a host cell and culturing the host cell to express the nucleic acid; and
(c) recovering the polypeptide from the culture of the host cell.

In one embodiment, the present disclosure relates to a method for controlling association between polypeptides.

In one embodiment, the method is a method for controlling association between polypeptides comprising an Fc region. In one embodiment, the method is a method for controlling association between CH3 domains.

In one embodiment, the method comprises modifying a polypeptide such that it associates more readily with a polypeptide having the modification than with a polypeptide not having the modification due to at least one of the following actions: (1) steric complementarity, (2) disulfide linkage, and (3) electrostatic charge.

In one embodiment, a method for controlling the association of the homomer of a polypeptide comprises:
the step of obtaining a nucleic acid encoding the polypeptide; and
the step of expressing the nucleic acid,
wherein the polypeptide has an Fc region, and
wherein, because of the modification introduced into the Fc region, the polypeptide associates more readily with a polypeptide having the modification via the Fc region than with a polypeptide comprising an Fc region into which the modification has not been introduced.

In one embodiment, the present disclosure relates to a method for promoting the expression of a homomer of a polypeptide.

In one embodiment, the method comprises the step of obtaining a nucleic acid encoding
the polypeptide; and
the step of expressing the nucleic acid,
wherein the polypeptide comprises an Fc region, and
wherein, because of the modification introduced into the Fc region, the polypeptide associates more readily with a polypeptide having the modification via the Fc region than with a polypeptide comprising an Fc region into which the modification has not been introduced.

All prior art documents cited herein are incorporated herein by reference.

The Examples provided below are one embodiment of the present invention, and the present invention is not limited to these embodiments. In the Sequence Listing, SEQ ID NOs: 1-60, 78-101, and 104-114 are full-length heavy chains, SEQ ID NO: 61 is a light chain, and SEQ ID NOs: 62-77 and 102-103 are Fc fragments. (For SEQ ID NOs: 115-620, see Table 14 and subsequent tables.)

The value "0" in the tables presented in the Examples is construed as having the same significant figures as the other values in the same column. For example, when the other values in the same column have significant figures to one decimal place, "0" is construed as "0.0".

### [Examples]

### [Example 1] Search for amino acid modifications for promoting homodimer formation of heavy chains

To express multiple antibodies simultaneously in cells, first, a search was performed for amino acid modifications positioned in the CH3 interface that are disadvantageous for heterodimer formation between the heavy chains of two antibodies in terms of electrostatic force, disulfide linkage formation, or steric hindrance, but are advantageous for homodimer formation. The concept is shown in Figure 1. A number of amino acid modifications for the purpose of promoting heterodimerization of the heavy chains of antibodies A and B have so far been reported (listed below). Accordingly, it was considered that similar residues might also be able to be used as residues for promoting homodimerization.
- WO2006106905 - PROCESS FOR PRODUCTION OF POLYPEPTIDE BY REGULATION OF ASSEMBLY
- An efficient route to human bispecific IgG (Nat Biotechnol. 1998 Jul;16(7):677-81. doi: 10.1038/nbt0798-677.)
- Efficient generation of stable bispecific IgG1 by controlled Fab-arm exchange (Proc Natl Acad Sci U S A. 2013 Mar 26;110(13):5145-50. doi: 10.1073/pnas.1220145110.)
- Immunoglobulin Fc Heterodimer Platform Technology: From Design to Applications in Therapeutic Antibodies and Proteins (Front Immunol. 2016 Oct 6;7:394. doi: 10.3389/fimmu.2016.00394. eCollection 2016.)

However, since the modification for promoting the homodimer formation of heavy chain needs to be achieved between heavy chains of one type, the degree of freedom of available amino acid modifications is lower than when promoting heterodimer formation using two different heavy chains. In particular, the promotion of disulfide formation between identical heavy chains and the controlling method based on physical hindrance via protuberances and cavities are expected to be difficult because they require more stringent orientation than electrostatic interactions, which can take effect in a relatively long distance of about 5-10Å, and therefore the modifications used for heterodimers may not be compatible. In fact, only limited combinations of charged amino acid modifications have so far been reported to promote homodimer formation (WO2013157953A1, WO2014015804A1, WO2017205014, J Biol Chem. 2017 Oct 27; 292(43): 17885-17896). Under these circumstances, the present inventors carried out a large-scale study on combinations of amino acid modifications on the CH3 interface to search for amino acid modifications that promote homodimer formation. A list of the studied amino acid modifications and the intended effects of modification is shown in Table 1. The amino acid numbers in Table 1 are in accordance with EU numbering (Sequences of proteins of immunological interest, NIH Publication No.91-3242). The subsequent amino acid numbers are also in accordance with EU numbering. In screening, the heavy chain used was a human IgG1 sequence.

Plasmids expressing a full-length heavy chain with these amino acid modifications and an Fc fragment below the hinge region containing no modification were prepared by a method known to those skilled in the art. When these two plasmids and a plasmid encoding a light chain are expressed simultaneously in mammalian cells, the homodimer of the full-length IgG heavy chain, the homodimer of the Fc fragment, and the heterodimer in which the two are associated, will be produced. The ratio of expression of these three can be analyzed by size exclusion chromatography (SEC) because they have different molecular weights. To confirm whether the formation of heavy chain homodimer was promoted, each peak area ratio was calculated and compared with unmodified (WT) human IgG1. If the formation of heavy chain homodimer is promoted, the area ratio of the heterodimer is expected to decrease as compared to WT-IgG1. The combinations of the genes of each antibody used in screening and their SEQ ID NOs are shown in Table 1.

The antibodies listed in Table 1 were transiently expressed in mammalian cells by a method known to those skilled in the art using the produced genes, and then purified by a method known to those skilled in the art. Specifically, the antibodies of Table 1 were transiently expressed by transfecting 1 mL of 2E+6 cells/mL Expi293F (Thermo Fisher Scientific) with the plasmids encoding the full-length heavy chain, the light chain, and the Fc fragment at a mass ratio of 1:2:1. After 4 days of transfection, the supernatant was recovered and purified using MonoSpin ProA (GL science).

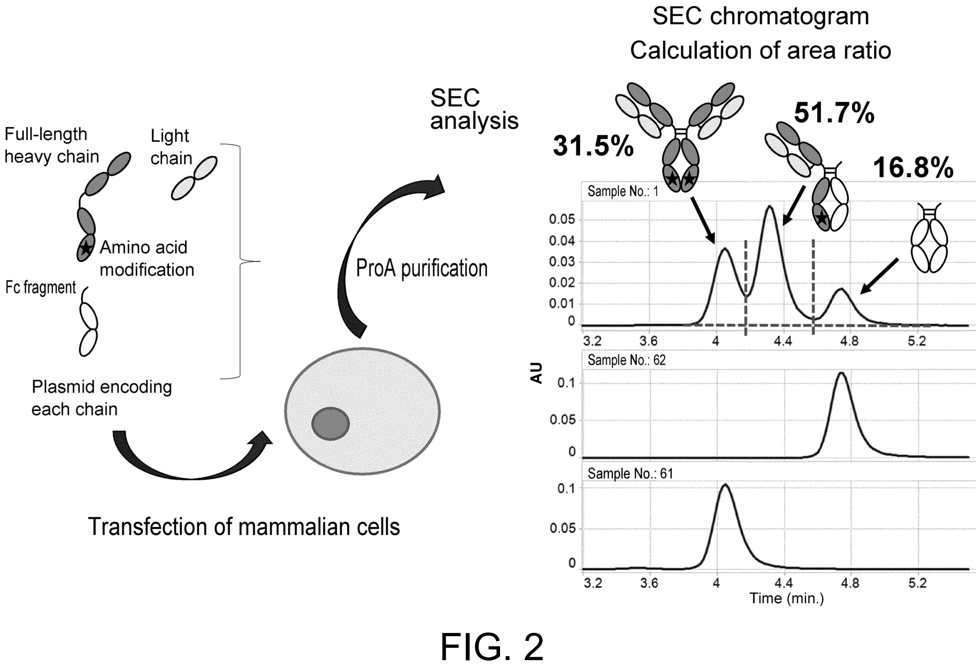

The purified antibodies were assessed by size exclusion chromatography (SEC) analysis using ACQUITY UPLC H-Class (Waters). Fifty mM phosphate buffer containing 300 mM sodium chloride, pH7.0 (Isekyu) was used as a running buffer, and TSKgel SuperSW3000 custom-made column (4.6 mm x 15 cm, 4µm, Gel Lot 89R)(TOSOH) was used as an analysis column. Chromatograms were recorded at a wavelength of UV 215 nm. Data was analyzed using Empower3 (Waters). For analysis of the antibodies of Table 1, the area ratios of the three components, i.e., the homodimer component of the full-length heavy chain, the heterodimer component with the Fc fragment, and the homodimer component of the Fc fragment, were calculated separately and presented in percent as shown in the representative example of Fig. 2. In Fig. 2, the reference samples prepared in Sample Nos. 61 and 62 (Table 2) are shown as well, thereby confirming that the three components can be properly separated in SEC analysis. Analysis results are shown in Table 1. In this analysis, the ratio of the heteromultimer for WT-IgG1 was 51.7%, suggesting that amino acid modifications exhibiting this ratio or lower will promote homodimerization of heavy chains.

**(Table 1) Modifications and amino acid sequences used in screening and SEC analysis results**

| Sample No. | Category of modification effect | Modifications that promote formation of heavy chain homodimers | Full-length heavy chain | Light chain | Fc fragment | Homo full-length heavy chain (%) | Heteromultimer (%) | Homo Fc fragment (%) |
|---|---|---|---|---|---|---|---|---|
| | | | SEQ ID NO | SEQ ID NO | SEQ ID NO | | | |
| 1 | WT-IgG1 | - | 1 | 61 | 62 | 31.5 | 51.7 | 16.8 |
| 2 | Charge | E345K/K360E | 2 | 61 | 62 | 34.3 | 50.3 | 15.4 |
| 3 | Charge | E345R/K360E | 3 | 61 | 62 | 31.0 | 51.8 | 17.1 |
| 4 | Charge | E357K/K370E | 4 | 61 | 62 | 42.5 | 40.6 | 17.0 |
| 5 | Charge | D399K/K409E | 5 | 61 | 62 | 56.4 | 12.1 | 31.5 |
| 6 | Charge | D399K/K409D | 6 | 61 | 62 | 53.6 | 18.6 | 27.8 |
| 7 | Charge | D399R/K409E | 7 | 61 | 62 | 57.0 | 13.2 | 29.8 |
| 8 | Charge | D399R/K409D | 8 | 61 | 62 | 57.1 | 14.4 | 28.5 |
| 9 | Charge | K392E/D399K | 9 | 61 | 62 | 40.0 | 36.1 | 23.9 |
| 10 | Charge | K392D/D399K | 10 | 61 | 62 | 54.2 | 20.3 | 25.6 |
| 11 | Charge | K392E/D399R | 11 | 61 | 62 | 40.4 | 37.7 | 21.8 |
| 12 | Charge | K392D/D399R | 12 | 61 | 62 | 47.7 | 27.9 | 24.4 |
| 13 | Disulfide bond | K392C/D399C | 13 | 61 | 62 | 35.8 | 41.5 | 22.8 |
| 14 | Disulfide bond | Y349C/S354C | 14 | 61 | 62 | 27.6 | 52.9 | 19.5 |
| 15 | Disulfide bond | Y349C/E356C | 15 | 61 | 62 | 34.2 | 48.1 | 17.7 |
| 16 | Disulfide bond | Y349C/E357C | 16 | 61 | 62 | 36.2 | 41.5 | 22.3 |
| 17 | Disulfide bond | L351C/S354C | 17 | 61 | 62 | 27.4 | 49.4 | 23.2 |
| 18 | Disulfide bond | T394C/V397C | 18 | 61 | 62 | 33.1 | 46.8 | 20.1 |
| 19 | Steric hindrance | T394W/F405A | 19 | 61 | 62 | 70.7 | 0.0 | 29.3 |
| 20 | Charge | L351D/T366K | 20 | 61 | 62 | 66.8 | 1.0 | 32.2 |
| 21 | Charge | L351E/T366K | 21 | 61 | 62 | 50.8 | 12.1 | 37.1 |
| 22 | Charge | L351K/T366D | 22 | 61 | 62 | 55.0 | 16.2 | 28.8 |
| 23 | Charge | L351K/T366E | 23 | 61 | 62 | 39.6 | 38.0 | 22.4 |
| 24 | Steric hindrance + Charge | F405L/K409R | 24 | 61 | 62 | 8.0 | 83.0 | 9.0 |
| 25 | Charge | E356K/K439E | 25 | 61 | 62 | 53.0 | 16.0 | 31.0 |
| 26 | Steric hindrance | T366W/L368A/Y407V | 26 | 61 | 62 | 64.0 | 0.0 | 36.0 |
| 27 | Steric hindrance | T366Y/Y407T | 27 | 61 | 62 | 65.5 | 3.3 | 31.2 |
| 28 | Steric hindrance | T366W/Y407A | 28 | 61 | 62 | 55.4 | 7.7 | 37.0 |
| 29 | Steric hindrance | T366Y/T394W/F405A/Y407T | 29 | 61 | 62 | 58.4 | 6.7 | 34.9 |
| 30 | Steric hindrance | E357N/K370E/D399V/F405T/K409W | 30 | 61 | 62 | 62.5 | 3.9 | 33.6 |
| 31 | Steric hindrance + Charge | Q347R/K360E/D399V/F405T/K409W | 31 | 61 | 62 | 73.6 | 0.0 | 26.4 |
| 32 | Steric hindrance | Y349T/S364H/T394F/F405A | 32 | 61 | 62 | 30.7 | 26.5 | 42.8 |
| 33 | Steric hindrance | Y349T/S364H | 33 | 61 | 62 | 24.1 | 65.2 | 10.7 |
| 34 | Steric hindrance | T394F/F405A | 34 | 61 | 62 | 72.6 | 0.0 | 27.4 |
| 35 | Charge | E357K/K370E/D399K/K409E | 35 | 61 | 62 | 27.0 | 45.0 | 28.0 |
| 36 | Charge | E356K/D399K/K409E/K439E | 36 | 61 | 62 | 64.0 | 0.0 | 36.0 |
| 37 | Charge | E356K/E357K/K370E/K439E | 37 | 61 | 62 | 55.8 | 10.4 | 33.8 |
| 38 | Charge | E356K/E357K/K370E/D399K/K409E/K439E | 38 | 61 | 62 | 58.5 | 1.3 | 40.2 |
| 39 | Steric hindrance | Q347E/S354Y/T366Y/Y407T | 39 | 61 | 62 | 61.6 | 0.0 | 38.4 |
| 40 | Steric hindrance | T350V/L351Y/T366L/T394W/F405A | 40 | 61 | 62 | 54.2 | 8.6 | 37.3 |
| 41 | Steric hindrance | K392L/T394W/F405A/Y407V | 41 | 61 | 62 | 65.9 | 0.0 | 34.1 |
| 42 | Disulfide bond | Y349C/E357C/K392C/D399C | 42 | 61 | 62 | 22.8 | 7.2 | 70.1 |
| 43 | Disulfide bond | Y349C/L351C/S354C/E357C | 43 | 61 | 62 | 40.2 | 22.7 | 37.1 |
| 44 | Disulfide bond | Y349C/E357C/T394C/V397C | 44 | 61 | 62 | 20.0 | 23.1 | 56.9 |
| 45 | Disulfide bond | Y349C/E357C/K392C/T394C/V397C/D399C | 45 | 61 | 62 | 24.3 | 0.0 | 75.7 |
| 46 | Disulfide bond | K392C/T394C/V397C/D399C | 46 | 61 | 62 | 24.0 | 30.2 | 45.8 |
| 47 | Disulfide bond | L351C/S354C/K392C/D399C | 47 | 61 | 62 | 21.1 | 39.3 | 39.7 |
| 48 | Charge | E356K/K392D/D399K/K439E | 48 | 61 | 62 | 68.6 | 0.0 | 31.4 |
| 49 | Charge | E357K/K370E/K392D/D399K | 49 | 61 | 62 | 59.6 | 8.3 | 32.1 |
| 50 | Charge | K392D/D399K/K409D | 50 | 61 | 62 | 65.5 | 0.0 | 34.5 |
| 51 | Charge + Disulfide bond | Y349C/E357C/K392D/D399K | 51 | 61 | 62 | 36.0 | 0.0 | 64.0 |
| 52 | Charge + Disulfide bond | E357K/K370E/T394C/V397C | 52 | 61 | 62 | 38.8 | 28.4 | 32.8 |
| 53 | Charge + Disulfide bond | Y349C/E357C/K370E | 53 | 61 | 62 | 39.8 | 31.3 | 28.9 |
| 54 | Charge + Disulfide bond | E357K/K370E/K392C/D399C | 54 | 61 | 62 | 52.4 | 20.6 | 27.0 |
| 55 | Steric hindrance + Charge | E345R/Q347R/K360D/T366V/D399M/Y407A/K409V | 55 | 61 | 62 | 67.9 | 0.0 | 32.1 |
| 56 | Charge | E356K/K392D/D399K/K409D | 56 | 61 | 62 | 62.9 | 0.0 | 37.1 |
| 57 | Steric hindrance + Charge | E345R/Q347R/K360D/T366V | 57 | 61 | 62 | 11.5 | 70.8 | 17.7 |
| 58 | Steric hindrance + Charge | E345R/K360E/D399M/Y407A/K409V | 58 | 61 | 62 | 63.1 | 0.0 | 36.9 |
| 59 | Charge + Disulfide bond | K392D/T394C/V397C/D399K | 59 | 61 | 62 | 39.9 | 19.9 | 40.3 |
| 60 | Steric hindrance | T350V/L351Y/T366L/K392L/T394W/F405A/Y407V | 60 | 61 | 62 | 61.9 | 0.0 | 38.1 |

### [Example 2] Expression and purification of antibodies

Next, in order to examine the effect of these amino modifications in the CH3 interface on the physicochemical properties of antibody, the antibodies of Table 2 were prepared. The antibodies were transiently expressed in mammalian cells by the method described in Example 1 using genes produced by a method known to those skilled in the art, and then purified. The plasmids encoding the full length heavy chain and light chain were used for transfection at a mass ratio of 1:1 or 1:2.

**(Table 2) Modifications and SEQ ID NOs of antibodies used in analysis**

| Sample No. | Category of modification effect | Modifications that promote formation of heavy chain homodimers | Full-length heavy chain | Light chain |
|---|---|---|---|---|
| | | | SEQ ID NO | SEQ ID NO |
| 61 | WT-IgG1 | - | 1 | 61 |
| 62 | WT-Fc fragment | - | 62 | - |
| 63 | Charge | E345K/K360E | 2 | 61 |
| 64 | Charge | E345R/K360E | 3 | 61 |
| 65 | Charge | E357K/K370E | 4 | 61 |
| 66 | Charge | D399K/K409E | 5 | 61 |
| 67 | Charge | D399K/K409D | 6 | 61 |
| 68 | Charge | D399R/K409E | 7 | 61 |
| 69 | Charge | D399R/K409D | 8 | 61 |
| 70 | Charge | K392E/D399K | 9 | 61 |
| 71 | Charge | K392D/D399K | 10 | 61 |
| 72 | Charge | K392E/D399R | 11 | 61 |
| 73 | Charge | K392D/D399R | 12 | 61 |
| 74 | Disulfide bond | K392C/D399C | 13 | 61 |
| 75 | Disulfide bond | Y349C/S354C | 14 | 61 |
| 76 | Disulfide bond | Y349C/E356C | 15 | 61 |
| 77 | Disulfide bond | Y349C/E357C | 16 | 61 |
| 78 | Disulfide bond | L351C/S354C | 17 | 61 |
| 79 | Disulfide bond | T394C/V397C | 18 | 61 |
| 80 | Steric hindrance | T394W/F405A | 19 | 61 |
| 81 | Charge | L351D/T366K | 20 | 61 |
| 82 | Charge | L351E/T366K | 21 | 61 |
| 83 | Charge | L351K/T366D | 22 | 61 |
| 84 | Charge | L351K/T366E | 23 | 61 |
| 85 | Steric hindrance + Charge | F405L/K409R | 24 | 61 |
| 86 | Charge | E356K/K439E | 25 | 61 |
| 87 | Steric hindrance | T366W/L368A/Y407V | 26 | 61 |
| 88 | Steric hindrance | T366Y/Y407T | 27 | 61 |
| 89 | Steric hindrance | T366W/Y407A | 28 | 61 |
| 90 | Steric hindrance | T366Y/T394W/F405A/Y407T | 29 | 61 |
| 91 | Steric hindrance | E357N/K370E/D399V/F405T/K409W | 30 | 61 |
| 92 | Steric hindrance + Charge | Q347R/K360E/D399V/F405T/K409W | 31 | 61 |
| 93 | Steric hindrance | Y349T/S364H/T394F/F405A | 32 | 61 |
| 94 | Steric hindrance | Y349T/S364H | 33 | 61 |
| 95 | Steric hindrance | T394F/F405A | 34 | 61 |
| 96 | Charge | E357K/K370E/D399K/K409E | 35 | 61 |
| 97 | Charge | E356K/D399K/K409E/K439E | 36 | 61 |
| 98 | Charge | E356K/E357K/K370E/K439E | 37 | 61 |
| 99 | Charge | E356K/E357K/K370E/D399K/K409E/K439E | 38 | 61 |
| 100 | Steric hindrance | Q347E/S354Y/T366Y/Y407T | 39 | 61 |
| 101 | Steric hindrance | T350V/L351Y/T366L/T394W/F405A | 40 | 61 |
| 102 | Steric hindrance | K392L/T394W/F405A/Y407V | 41 | 61 |
| 103 | Disulfide bond | Y349C/E357C/K392C/D399C | 42 | 61 |
| 104 | Disulfide bond | Y349C/L351C/S354C/E357C | 43 | 61 |
| 105 | Disulfide bond | Y349C/E357C/T394C/V397C | 44 | 61 |
| 106 | Disulfide bond | Y349C/E357C/K392C/T394C/V397C/D399C | 45 | 61 |
| 107 | Disulfide bond | K392C/T394C/V397C/D399C | 46 | 61 |
| 108 | Disulfide bond | L351C/S354C/K392C/D399C | 47 | 61 |
| 109 | Charge | E356K/K392D/D399K/K439E | 48 | 61 |
| 110 | Charge | E357K/K370E/K392D/D399K | 49 | 61 |
| 111 | Charge | K392D/D399K/K409D | 50 | 61 |
| 112 | Charge + Disulfide bond | Y349C/E357C/K392D/D399K | 51 | 61 |
| 113 | Charge + Disulfide bond | E357K/K370E/T394C/V397C | 52 | 61 |
| 114 | Charge + Disulfide bond | Y349C/E357C/K370E | 53 | 61 |
| 115 | Charge + Disulfide bond | E357K/K370E/K392C/D399C | 54 | 61 |
| 116 | Steric hindrance + Charge | E345R/Q347R/K360D/T366V/D399M/Y407A/K4 09V | 55 | 61 |
| 117 | Charge | E356K/K392D/D399K/K409D | 56 | 61 |
| 118 | Steric hindrance + Charge | E345R/Q347R/K360D/T366V | 57 | 61 |
| 119 | Steric hindrance + Charge | E345R/K360E/D399M/Y407A/K409V | 58 | 61 |
| 120 | Charge + Disulfide bond | K392D/T394C/V397C/D399K | 59 | 61 |
| 121 | Steric hindrance | T350V/L351Y/T366L/K392L/T394W/F405A/Y40 7V | 60 | 61 |

### [Example 3] Evaluation of physicochemical properties of antibodies with amino acid modifications introduced in the CH3 interface

The effects of CH3 interface modifications on the expression level, monomer content (%), and thermal denaturation midpoint temperature (Tm) of antibody were compared. The yields of the antibodies prepared in Example 2 are shown in Table 3.

In addition, the monomer content in the purified antibodies was evaluated by the SEC analysis method described in Example 1. The results of the SEC analysis were analyzed to calculate the monomer content (%), where the components eluted on the higher molecular weight side than the monomer were collectively regarded as associated multimers, and the components eluted on the lower molecular weight side than the monomer were collectively regarded as degradation products. The analysis results are shown in Table 3.

The Tm of the antibodies subjected to amino acid modification was evaluated by differential scanning fluorimetry (DSF). It has already been reported that Tm determined by this method shows good correlation with Tm determined by differential scanning calorimetry, which is a widely-known method for evaluating the thermal stability of antibodies (Journal of Pharmaceutical Science 2010;4: 1707-1720).

A 5000x concentrate of SYPRO orange protein gel stain (Invitrogen) was diluted with PBS (Sigma), and an antibody solution was mixed with this detection dye. The 20-µL mixture was dispensed into a tube for measurement, and its temperature was raised from 30°C to 99°C at a rate of 240°C/hr using Rotor-Gene Q (QIAGEN). Fluorescent changes with increasing temperature were observed at 470 nm (excitation wavelength) / 555 nm (fluorescence wavelength).

Obtained data was used to determine the temperature at which fluorescent transition was seen using Rotor-Gene Q Series Software (QIAGEN), and this value was determined as the Tm value. For the antibody used in this test, the Tm of Fab is about 95°C, which is markedly higher than the Tm of CH2 and CH3. Thus, in this test, the Tm value observed at the lowest temperature was regarded as a change in Tm resulting from Fc modification, and used for comparison. The results are shown in Table 3.

**(Table 3) Analysis results of antibodies with amino acid modifications**

| Sample No. | Full-length heavy chain | Light chain | Modifications that promote formation of heavy chain homodimers | SEC monom er ratio (%) | Tm (°C) | Yield (mg) |
|---|---|---|---|---|---|---|
| | SEQ ID NO | SEQ ID NO | | | | |
| 61 | 1 | 61 | - | 98.32 | 69 | 0.25 |
| 63 | 2 | 61 | E345K/K360E | 98.76 | 69 | 0.28 |
| 64 | 3 | 61 | E345R/K360E | 98.97 | 69.6 | 0.29 |
| 65 | 4 | 61 | E357K/K370E | 97.81 | 69.6 | 0.21 |
| 66 | 5 | 61 | D399K/K409E | 97.4 | 65.4 | 0.3 |
| 67 | 6 | 61 | D399K/K409D | 97.78 | 63 | 0.29 |
| 68 | 7 | 61 | D399R/K409E | 97.75 | 66 | 0.25 |
| 69 | 8 | 61 | D399R/K409D | 97.77 | 64.8 | 0.28 |
| 70 | 9 | 61 | K392E/D399K | 97.06 | 67.2 | 0.27 |
| 71 | 10 | 61 | K392D/D399K | 98.67 | 68.4 | 0.26 |
| 72 | 11 | 61 | K392E/D399R | 97.05 | 67.8 | 0.24 |
| 73 | 12 | 61 | K392D/D399R | 98.9 | 68.4 | 0.31 |
| 74 | 13 | 61 | K392C/D399C | 98.62 | 68.4 | 0.3 |
| 75 | 14 | 61 | Y349C/S354C | 97.98 | 69 | 0.26 |
| 76 | 15 | 61 | Y349C/E356C | 98.31 | 67.8 | 0.25 |
| 77 | 16 | 61 | Y349C/E357C | 98 | 69 | 0.25 |
| 78 | 17 | 61 | L351C/S354C | 92.42 | 68.4 | 0.26 |
| 79 | 18 | 61 | T394C/V397C | 84.93 | 68.4 | 0.15 |
| 80 | 19 | 61 | T394W/F405A | 97.65 | 68.4 | 0.22 |
| 81 | 20 | 61 | L351D/T366K | 93.99 | 63.6 | 0.23 |
| 82 | 21 | 61 | L351E/T366K | 76.86 | 58.8 | 0.31 |
| 83 | 22 | 61 | L351K/T366D | 97.02 | 67.2 | 0.25 |
| 84 | 23 | 61 | L351K/T366E | 86.6 | 60.6 | 0.31 |
| 85 | 24 | 61 | F405L/K409R | 95.26 | 66 | 0.27 |
| 86 | 25 | 61 | E356K/K439E | 98.74 | 69 | 0.29 |
| 87 | 26 | 61 | T366W/L368A/Y407V | 98.67 | 69 | 0.33 |
| 88 | 27 | 61 | T366Y/Y407T | 92.45 | 66.6 | 0.28 |
| 89 | 28 | 61 | T366W/Y407A | 90.2 | 69.6 | 0.25 |
| 90 | 29 | 61 | T366Y/T394W/F405A/Y407T | 85.25 | 61.2 | 0.23 |
| 91 | 30 | 61 | E357N/K370E/D399V/F405T/K409W | 91.82 | 63.6 | 0.27 |
| 92 | 31 | 61 | Q347R/K360E/D399V/F405T/K409W | 98.32 | 69 | 0.22 |
| 93 | 32 | 61 | Y349T/S364H/T394F/F405A | 68.34 | 53.4 | 0.15 |
| 94 | 33 | 61 | Y349T/S364H | 94.25 | 67.8 | 0.19 |
| 95 | 34 | 61 | T394F/F405A | 96.44 | 67.8 | 0.21 |
| 96 | 35 | 61 | E357K/K370E/D399K/K409E | 82.35 | 58.8 | 0.36 |
| 97 | 36 | 61 | E356K/D399K/K409E/K439E | 99.14 | 66 | 0.32 |
| 98 | 37 | 61 | E356K/E357K/K370E/K439E | 98.23 | 69 | 0.24 |
| 99 | 38 | 61 | E356K/E357K/K370E/D399K/K409E/K439E | 95.35 | 59.4 | 0.32 |
| 100 | 39 | 61 | Q347E/S354Y/T366Y/Y407T | 97.75 | 67.2 | 0.27 |
| 101 | 40 | 61 | T350V/L351Y/T366L/T394W/F405A | 85.08 | 63.6 | 0.32 |
| 102 | 41 | 61 | K392L/T394W/F405A/Y407V | 98.98 | 68.4 | 0.24 |
| 103 | 42 | 61 | Y349C/E357C/K392C/D399C | 54.85 | 54.6 | 0.07 |
| 104 | 43 | 61 | Y349C/L351C/S354C/E357C | 90.99 | 69 | 0.21 |
| 105 | 44 | 61 | Y349C/E357C/T394C/V397C | 90.51 | 68.4 | 0.06 |
| 106 | 45 | 61 | Y349C/E357C/K392C/T394C/V397C/D399C | 83.28 | 53.4 | 0.04 |
| 107 | 46 | 61 | K392C/T394C/V397C/D399C | 70.35 | 57.6 | 0.15 |
| 108 | 47 | 61 | L351C/S354C/K392C/D399C | 63.43 | 58.2 | 0.16 |
| 109 | 48 | 61 | E356K/K392D/D399K/K439E | 98.67 | 69.6 | 0.24 |
| 110 | 49 | 61 | E357K/K370E/K392D/D399K | 96.25 | 64.8 | 0.25 |
| 111 | 50 | 61 | K392D/D399K/K409D | 98.87 | 66 | 0.26 |
| 112 | 51 | 61 | Y349C/E357C/K392D/D399K | 97.2 | 68.4 | 0.05 |
| 113 | 52 | 61 | E357K/K370E/T394C/V397C | 98.22 | 67.2 | 0.22 |
| 114 | 53 | 61 | Y349C/E357C/K370E | 97.44 | 69.6 | 0.28 |
| 115 | 54 | 61 | E357K/K370E/K392C/D399C | 98.58 | 67.8 | 0.21 |
| 116 | 55 | 61 | E345R/Q347R/K360D/T366V/D399M/Y407 A/K409V | 98.36 | 52.8 | 0.27 |
| 117 | 56 | 61 | E356K/K392D/D399K/K409D | 99.36 | 62.4 | 0.26 |
| 118 | 57 | 61 | E345R/Q347R/K360D/T366V | 90.8 | 66.6 | 0.26 |
| 119 | 58 | 61 | E345R/K360E/D399M/Y407A/K409V | 96.93 | 69.6 | 0.27 |
| 120 | 59 | 61 | K392D/T394C/V397C/D399K | 93.48 | 64.8 | 0.2 |
| 121 | 60 | 61 | T350V/L351Y/T366L/K392L/T394W/F405A/ Y407V | 97.23 | 69.6 | 0.24 |

### [Example 4] Selection and discussion of modifications for promoting homodimerization of heavy chains

In the results of SEC analysis performed after expression of the full-length heavy chain, light chain, and Fc fragment as shown in Table 1, all modification pairs that exhibited a heterodimer formation ratio lower than that for WT-IgG1 (51.7%) can be said to provide a certain level of promotion of heavy-chain homodimer formation. As originally expected, the previously reported modifications for promoting the heteromeric association of heavy chains did not necessarily result in promotion of the homodimer formation of heavy chains. In particular, Sample Nos. 24, 33, and 57 resulted in considerable promotion of heavy chain heterodimerization, yielding no desired effect. It was also difficult for them to maintain the good physicochemical properties of antibody. Some modifications were found to greatly reduce the monomer ratio after SEC analysis, lower the Tm value, or reduce the antibody yield. This experimental fact suggests that modification pairs for promoting heavy-chain heterodimerization are not necessarily the same as those for promoting heavy-chain homodimerization, and it is important to discover pairs of amino acid modifications that promote homodimerization between heavy chains of one type and maintain good physicochemical properties. The present inventors evaluated the various antibodies shown in Table 1, and thereby discovered amino acid modifications that particularly potently suppress the heterodimerization of heavy chains and promote homodimerization of heavy chains. These selected antibodies are shown in Table 4. Compared to WT-IgG1, all these antibodies showed a comparable antibody expression level, a high monomer formation ratio, and a comparable Tm of the Fc region (however, a decrease was observed only in the antibody yield of Sample No. 112 compared to WT-IgG1).

**(Table 4) Amino acid modifications that exhibited strong heavy-chain homodimerization-promoting ability**

| Sample No. | Category of modification effect | Modifications that promote formation of heavy chain homodimers |
|---|---|---|
| 80 | Steric hindrance | T394W/F405A |
| 87 | Steric hindrance | T366W/L368A/Y407V |
| 92 | Steric hindrance + Charge | Q347R/K360E/D399V/F405T/K409W |
| 95 | Steric hindrance | T394F/F405A |
| 97 | Charge | E356K/D399K/K409E/K439E |
| 100 | Steric hindrance | Q347E/S354Y/T366Y/Y407T |
| 102 | Steric hindrance | K392L/T394W/F405A/Y407V |
| 109 | Charge | E356K/K392D/D399K/K439E |
| 111 | Charge | K392D/D399K/K409D |
| 112 | Charge + Disulfide bond | Y349C/E357C/K392D/D399K |
| 116 | Steric hindrance + Charge | E345R/Q347R/K360D/T366V/D399M/Y407A/K409V |
| 117 | Charge | E356K/K392D/D399K/K409D |
| 119 | Steric hindrance + Charge | E345R/K360E/D399M/Y407A/K409V |
| 121 | Steric hindrance | T350V/L351Y/T366L/K392L/T394W/F405A/Y407V |

### [Example 5] Identification of amino acid modification pairs that allow coexpression of multiple antibodies

Next, in order to identify amino acid modification pairs that allow coexpression of multiple antibodies, genes were prepared such that the amino acid modifications shown in Table 4 were also introduced into the Fc fragment of IgG1. These exhaustive combinations were examined to find modification pairs that promote heavy-chain homodimer formation between modified Fc regions. For expression, the light chain of SEQ ID NO: 61 was used. Antibodies were expressed by the same method as described in Example 1. The homodimerization-promoting ability of each modification pair was determined based on whether the heterodimer level after SEC analysis was reduced as compared to a pair of identical modifications. (For example, when a pair of WT-IgG is expressed, the heterodimer level is 52%. Thus, a combination that shows a heterodimer level of less than 52% can be determined to be a pair having homodimerization-promoting ability. The SEQ ID NOs and combinations thereof used in the experiment, and their heterodimer formation ratios are shown in Table 5. Pairs of modifications in such a relation as to suppress heterodimerization in the experimental result are expected not to cause heteromeric association no matter how many of them are combined. Therefore, they can be used not only for coexpression of two antibodies but also for coexpression of three, four, five, six, seven, eight, nine, or more antibodies.

More specifically, amino acid modification pairs that showed a heteromultimer level of less than 10% in Table 5 can be used in combination. Table 6 shows effective amino acid modification pairs composed of these combinations (<10% Combinations 1-3512).

Furthermore, amino acid modification pairs composed only of pairs having stronger heavy-chain homomeric association-promoting ability (amino acid modification pairs that showed a heteromultimer level of 0% in Table 5) (0% Combinations 1-744) are shown in Table 7. In Tables 5, 6, and 7, the non-introduction of modifications is denoted as WT.

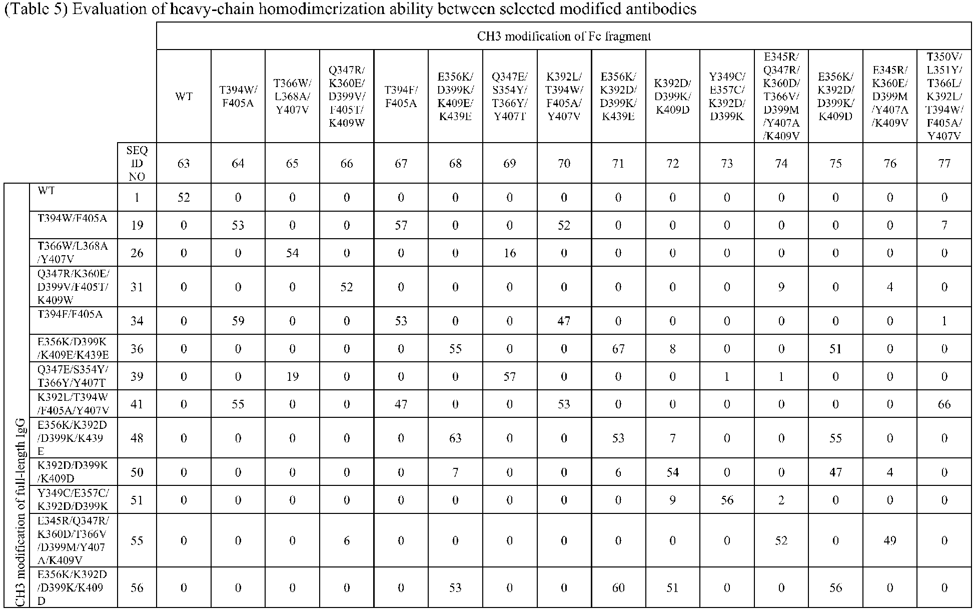

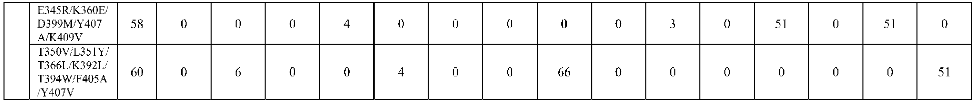

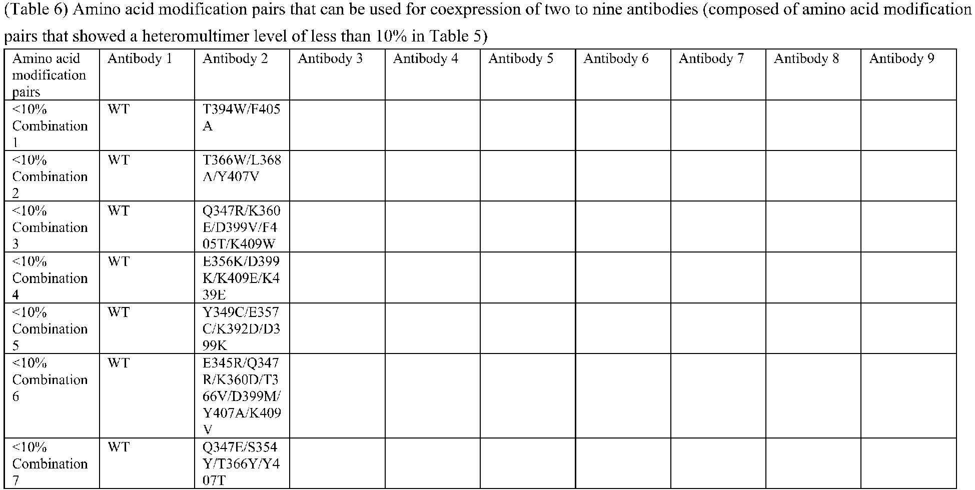

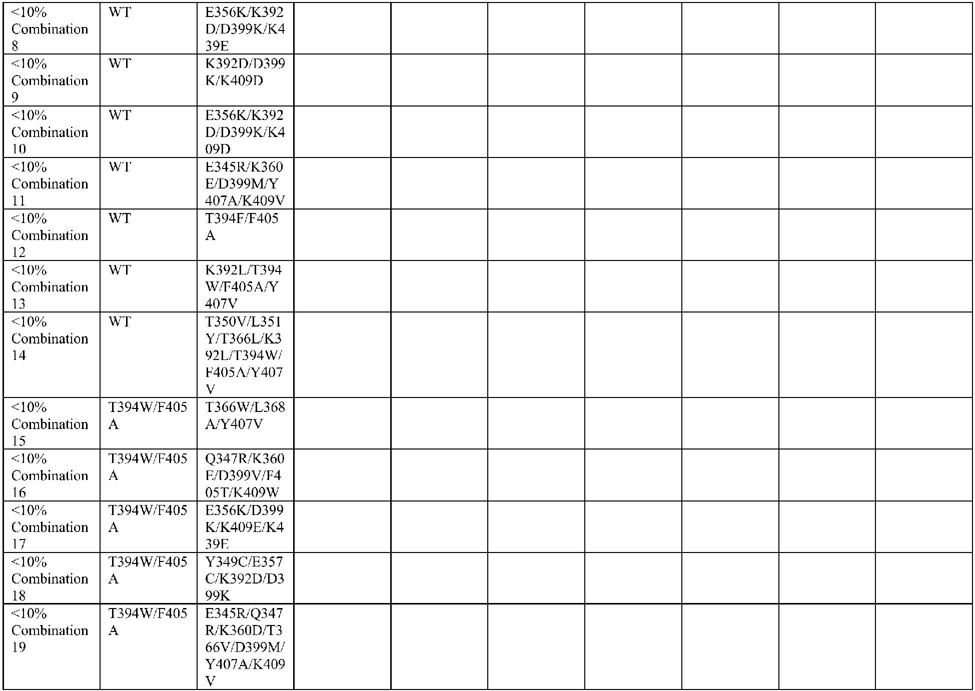

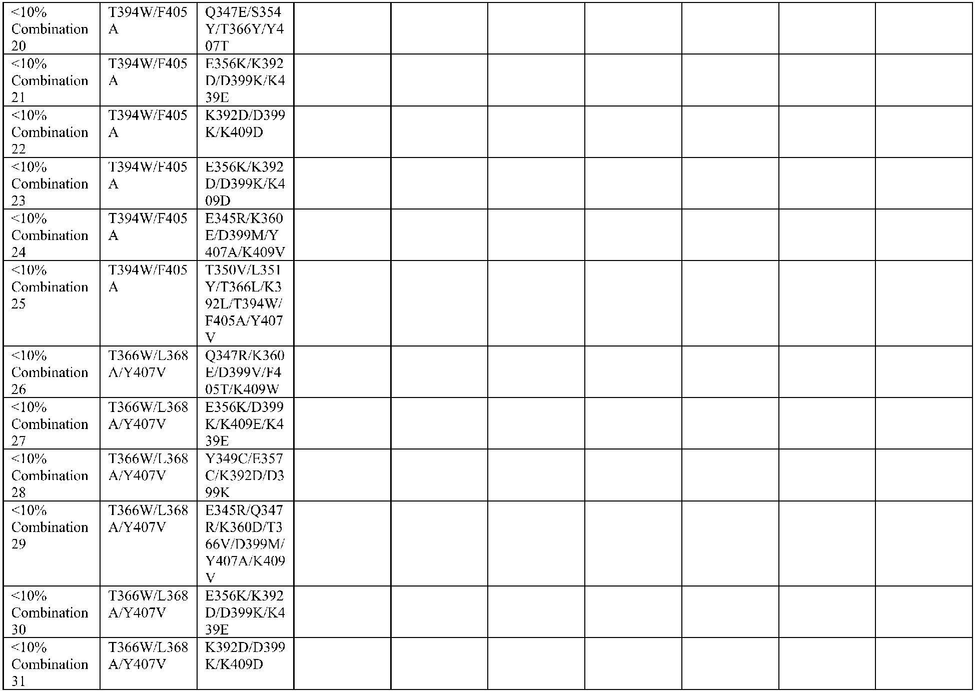

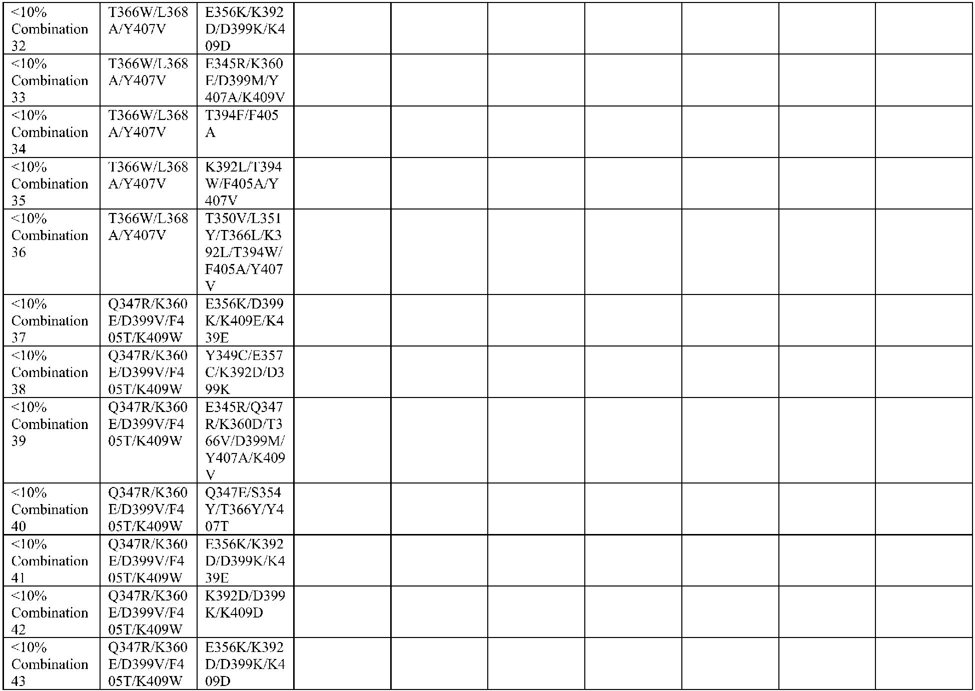

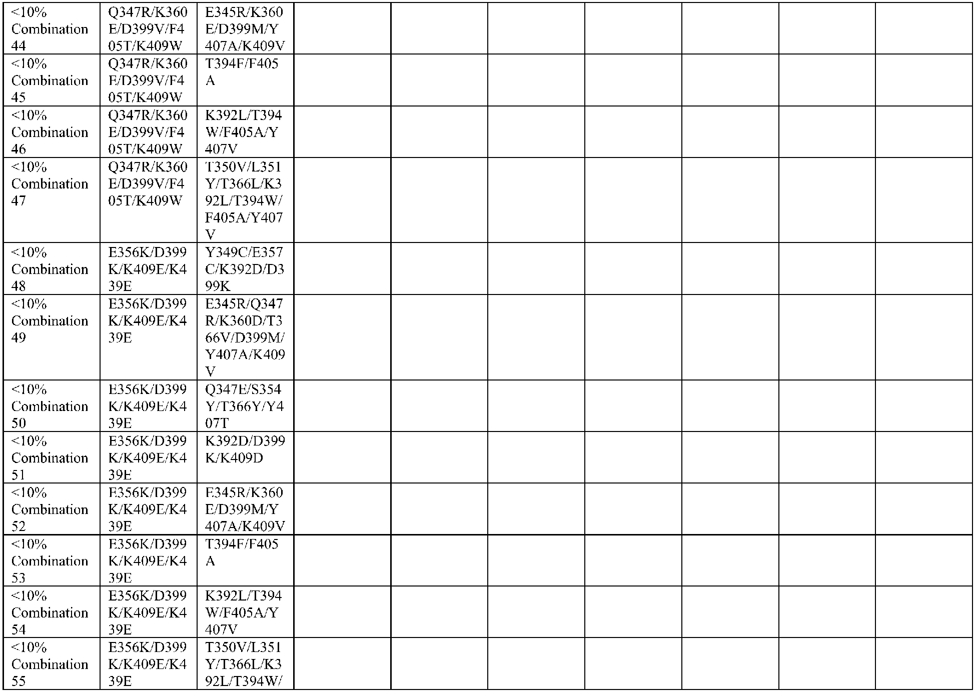

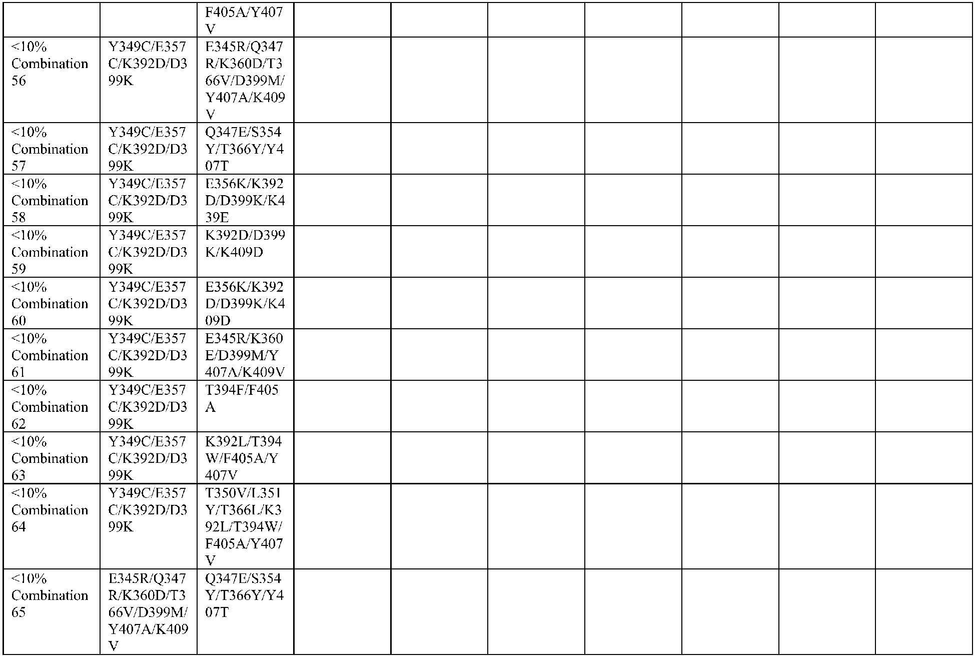

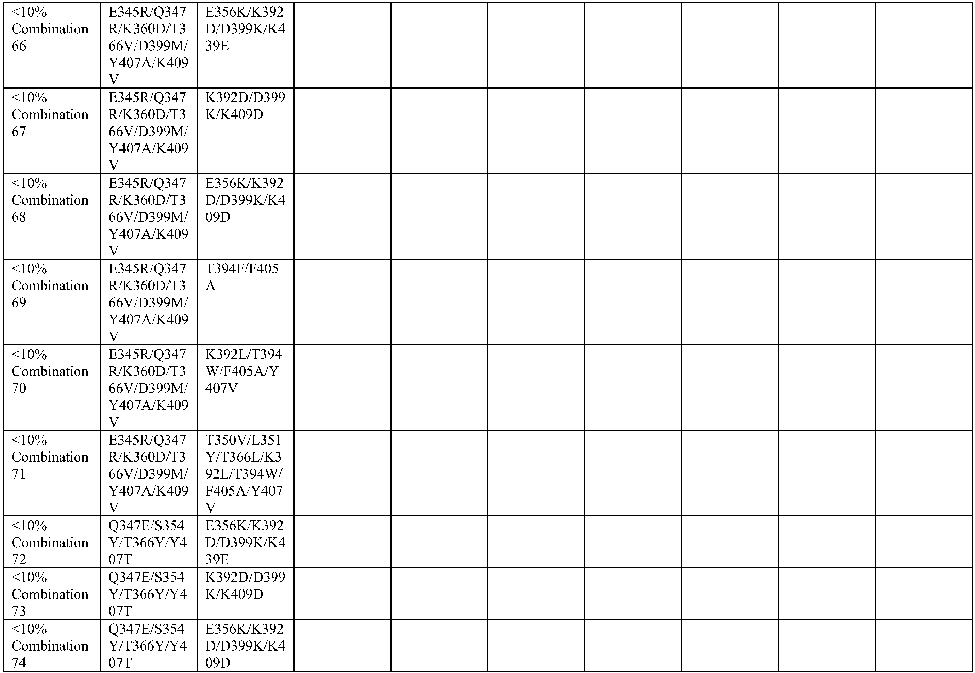

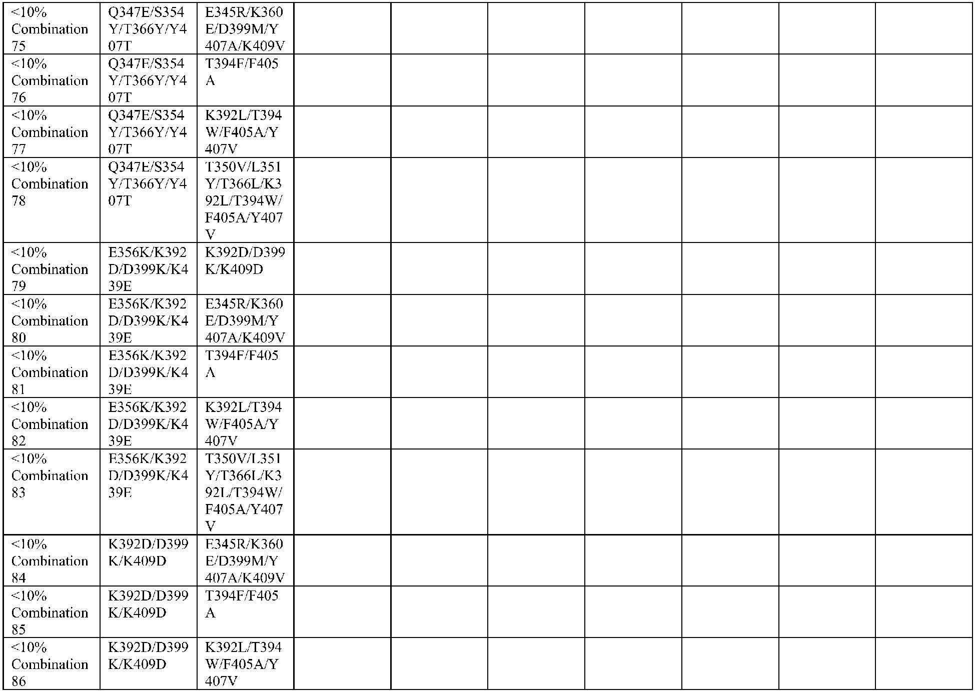

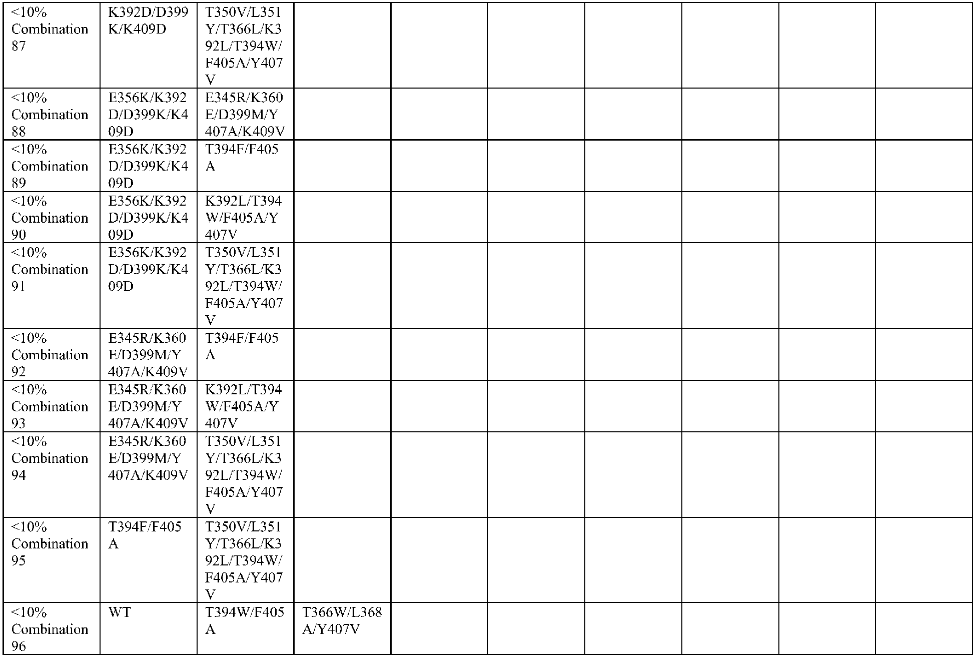

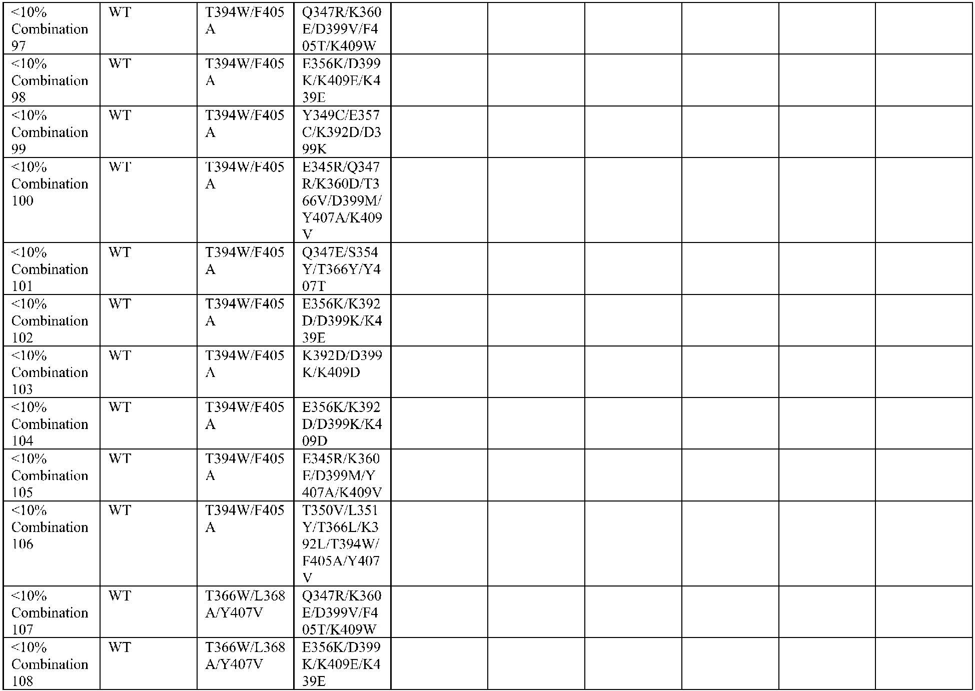

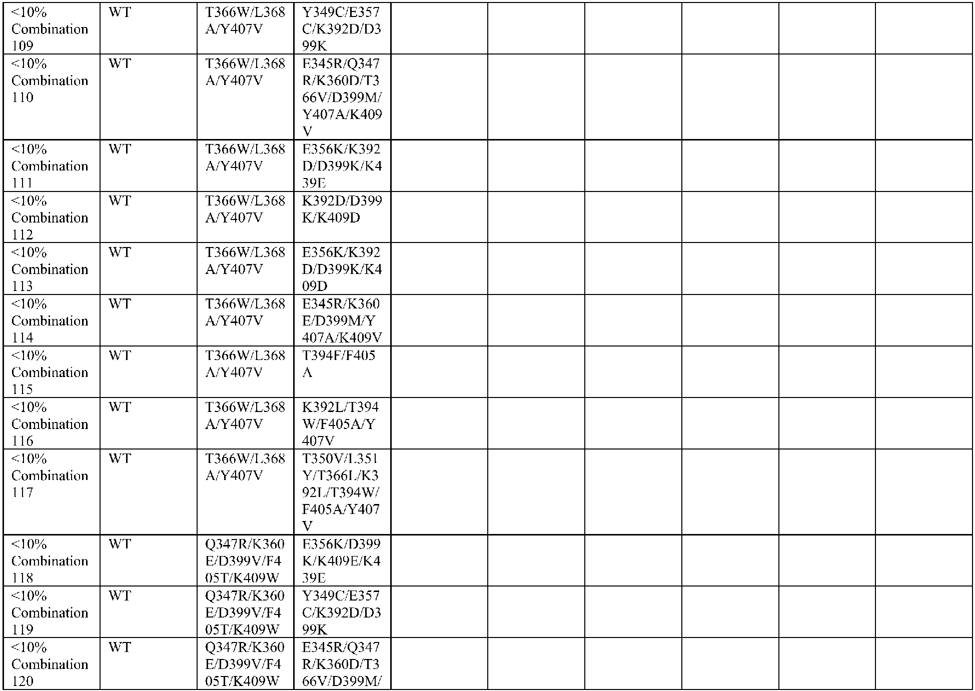

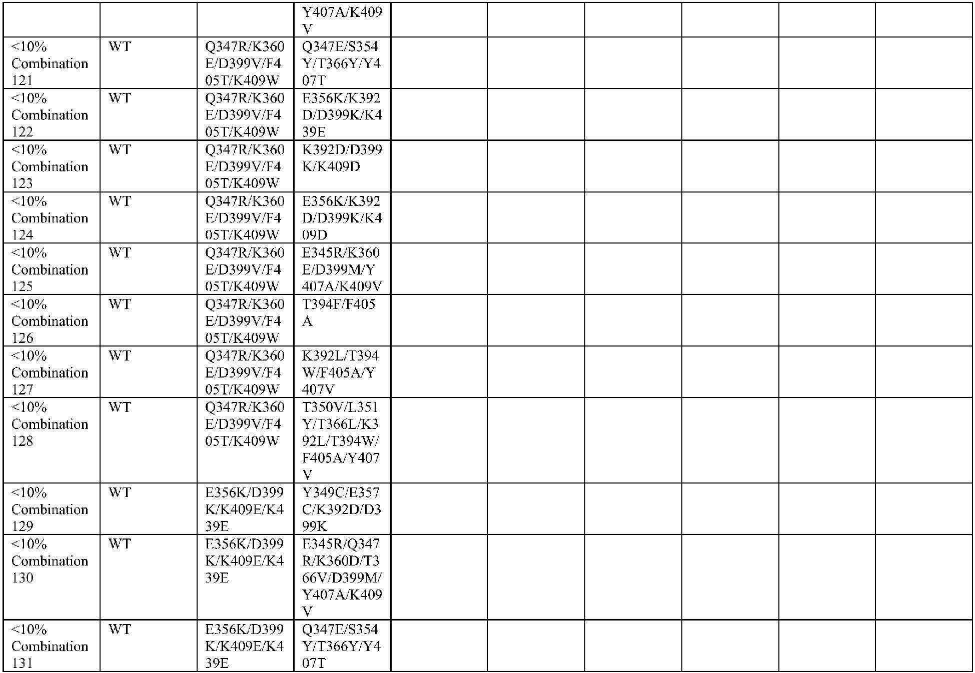

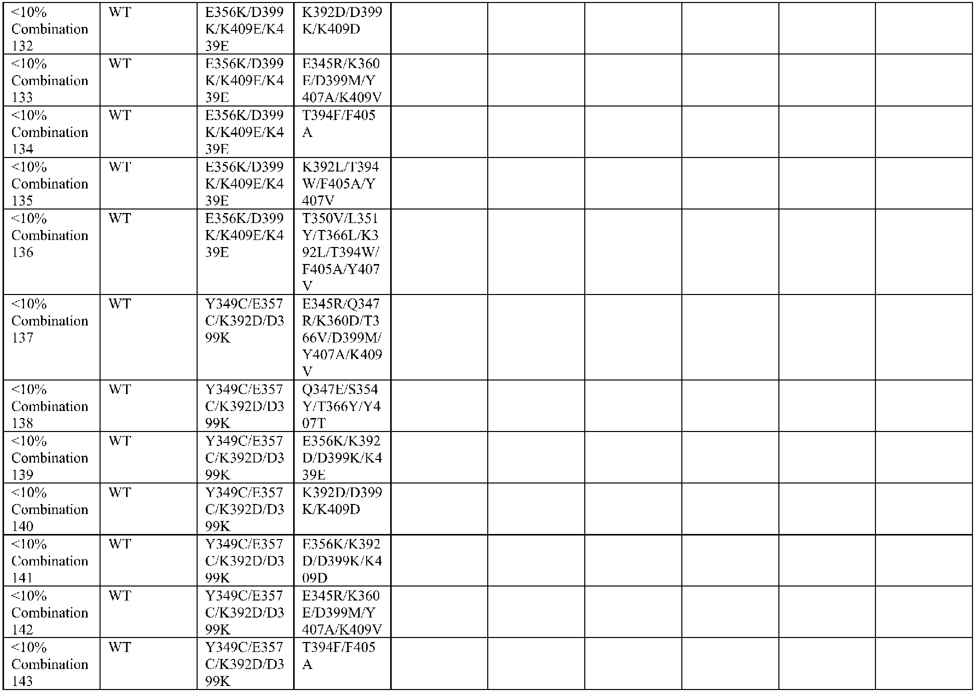

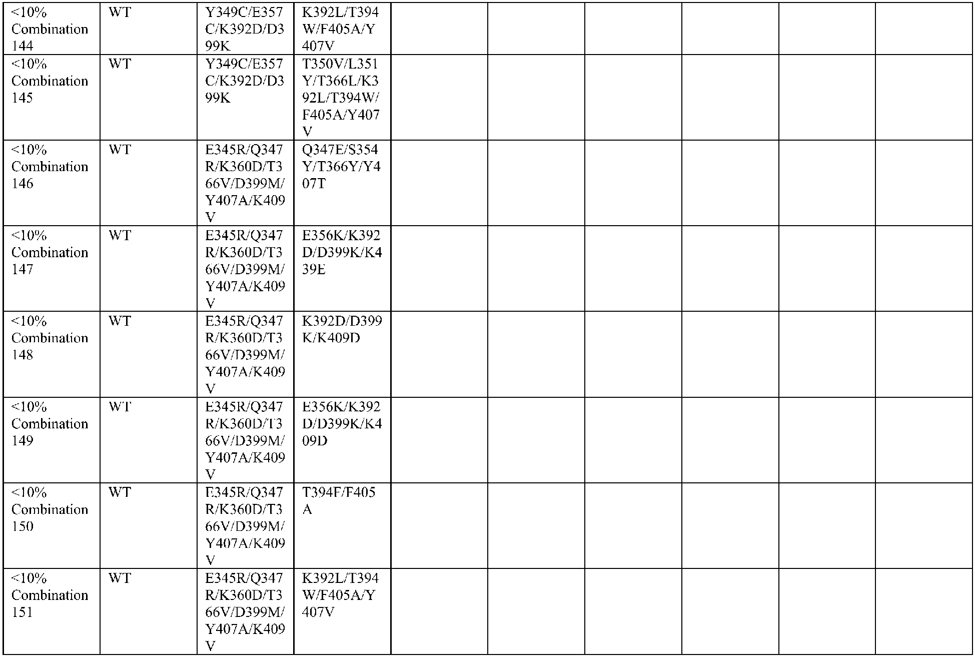

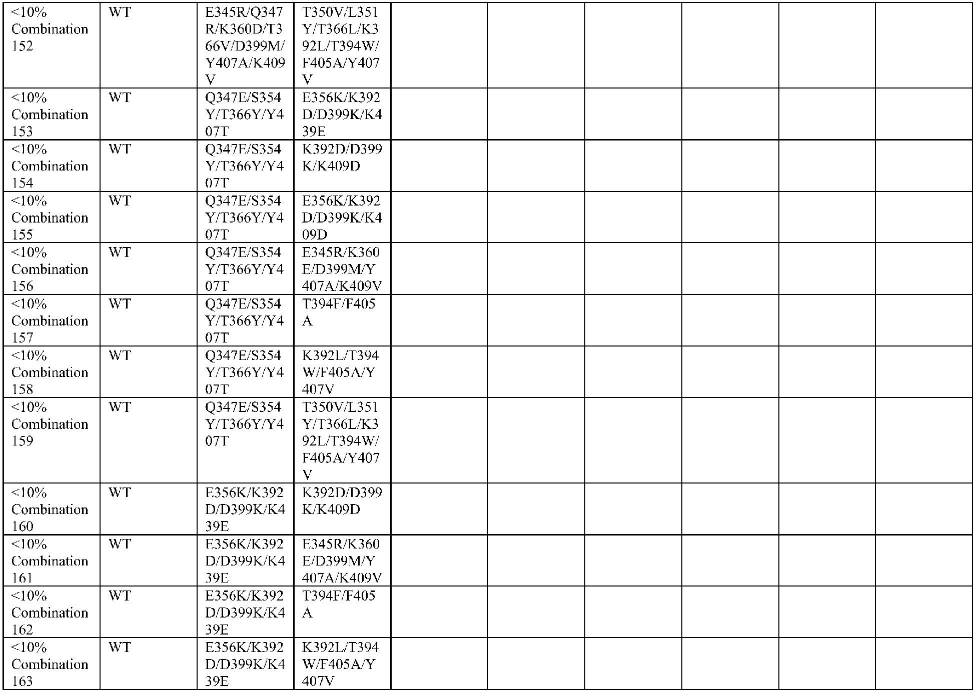

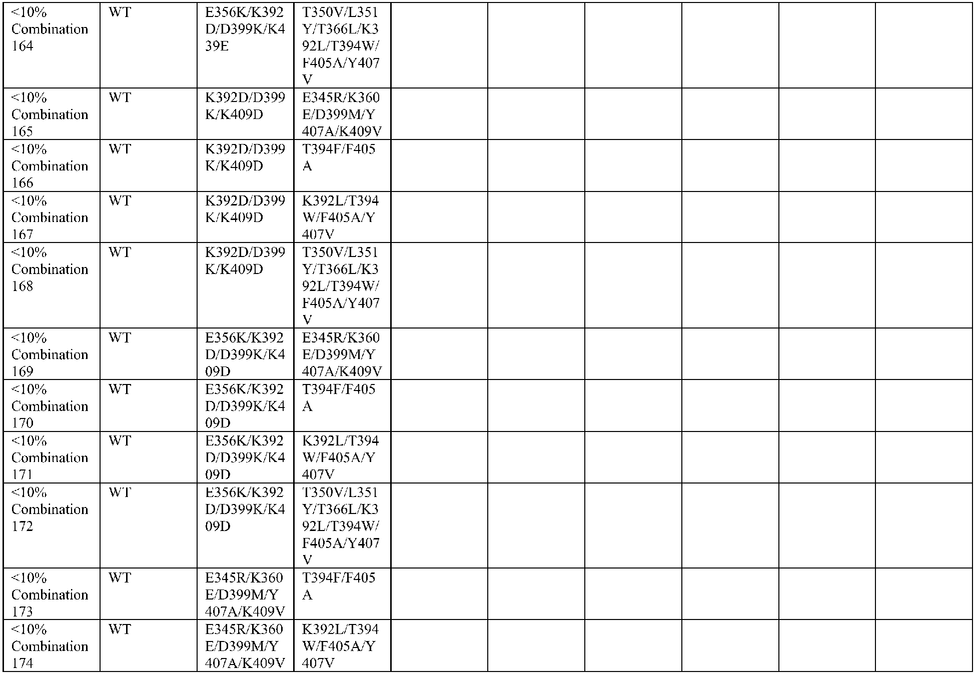

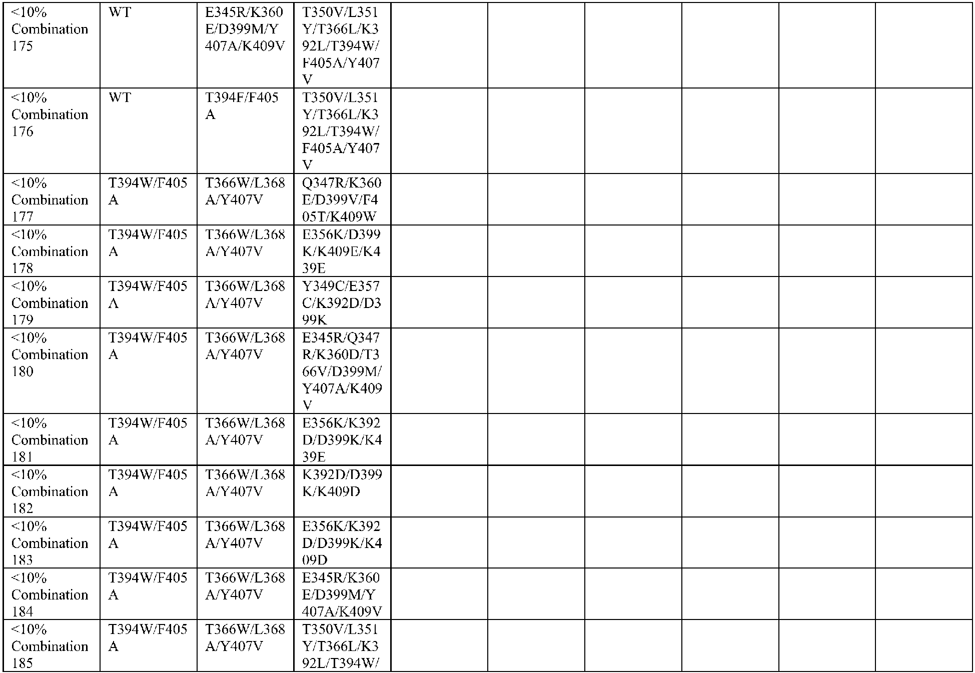

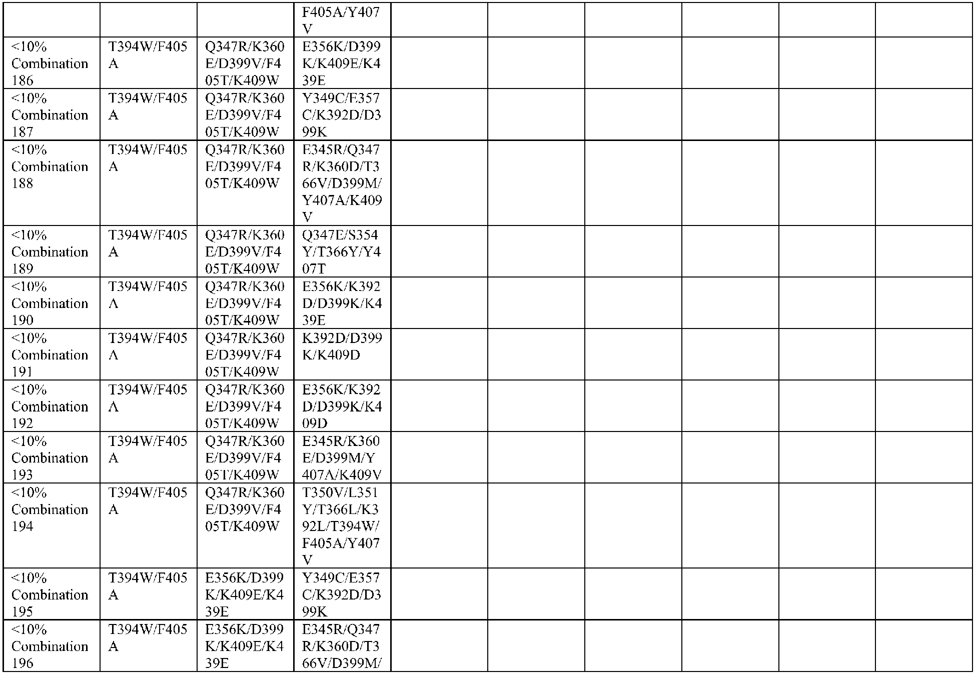

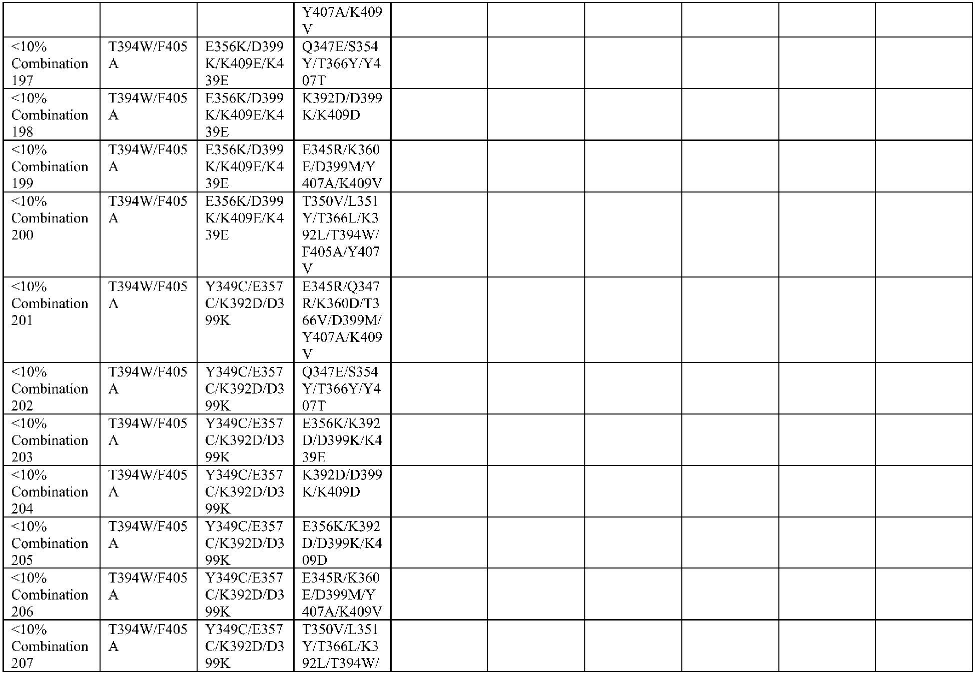

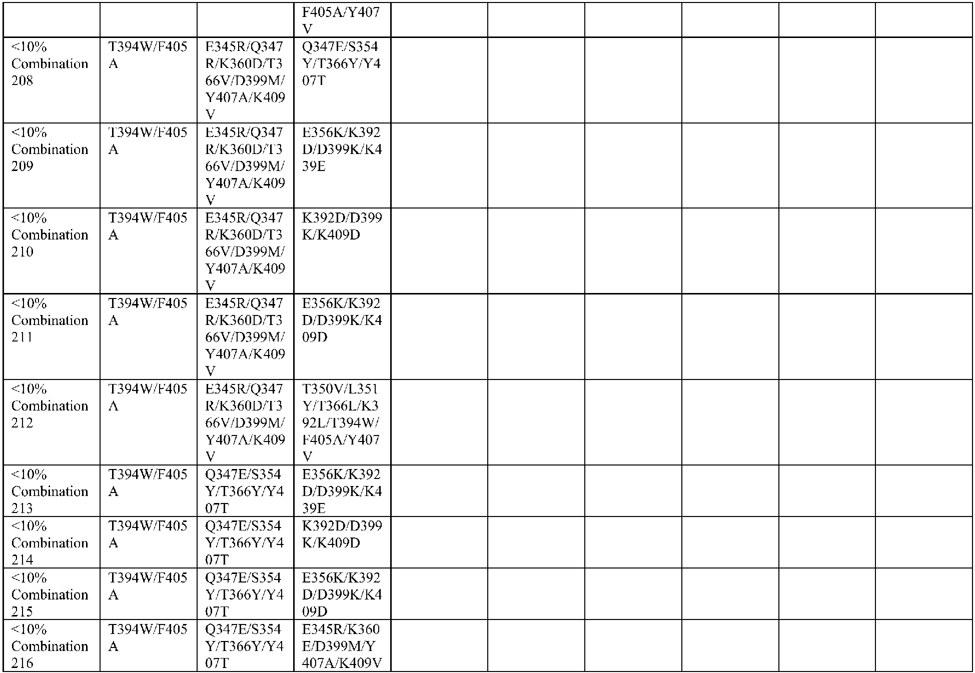

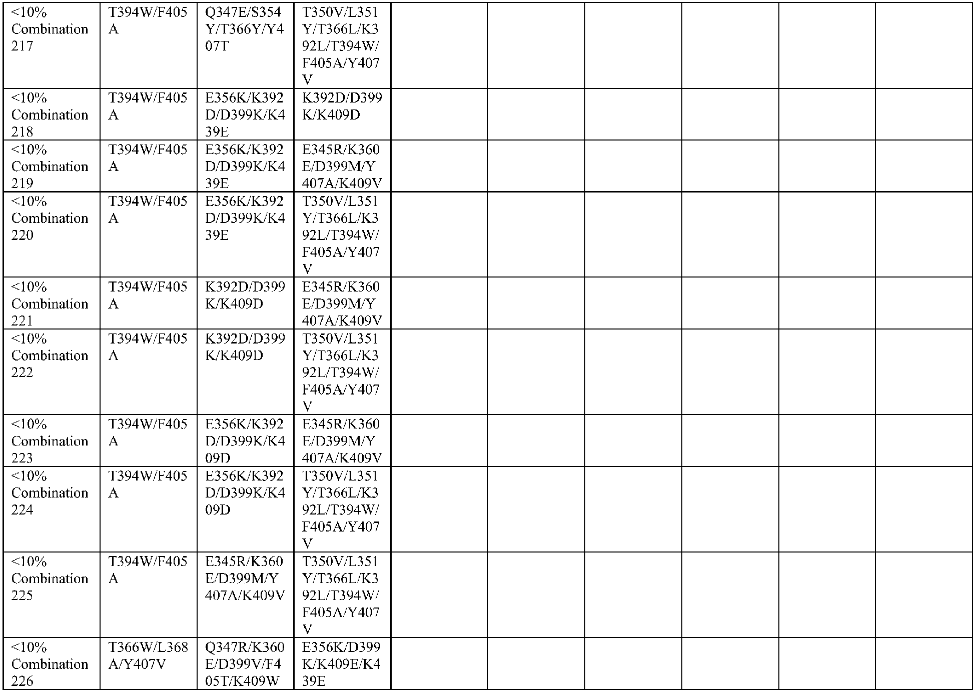

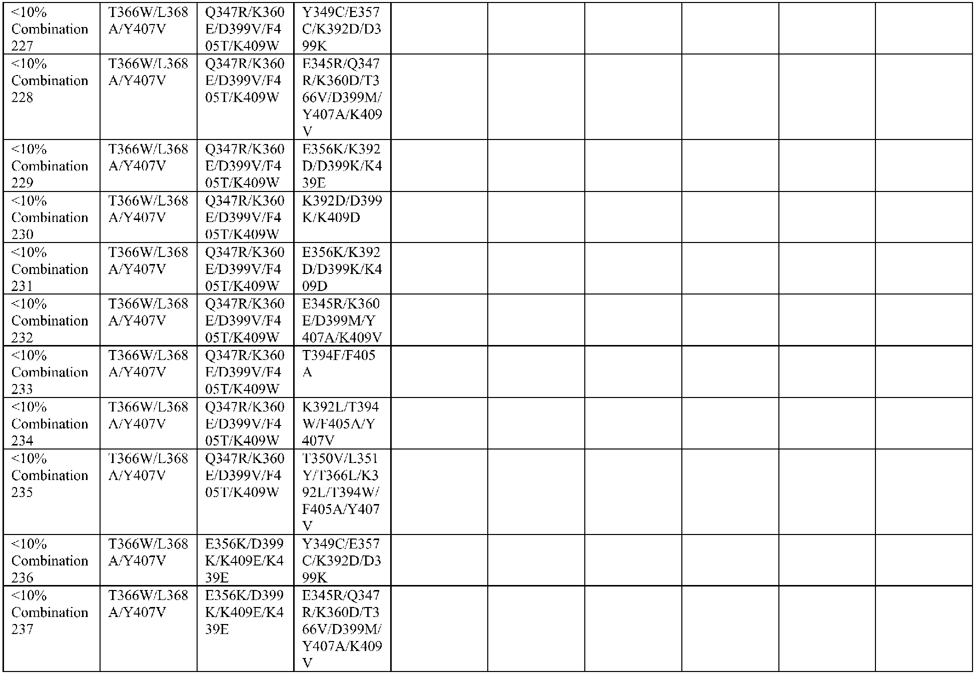

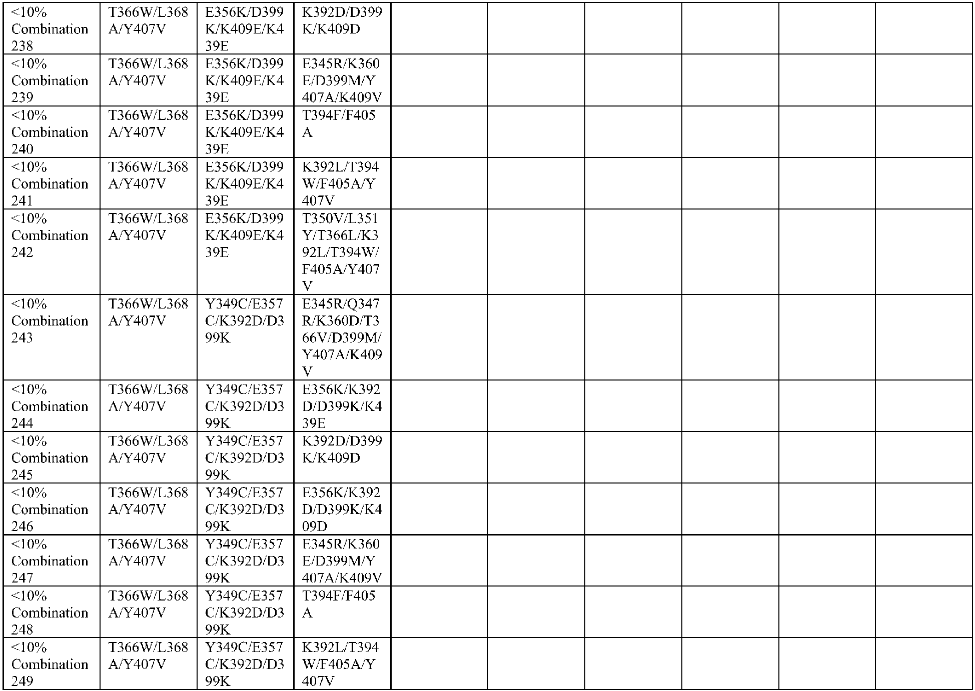

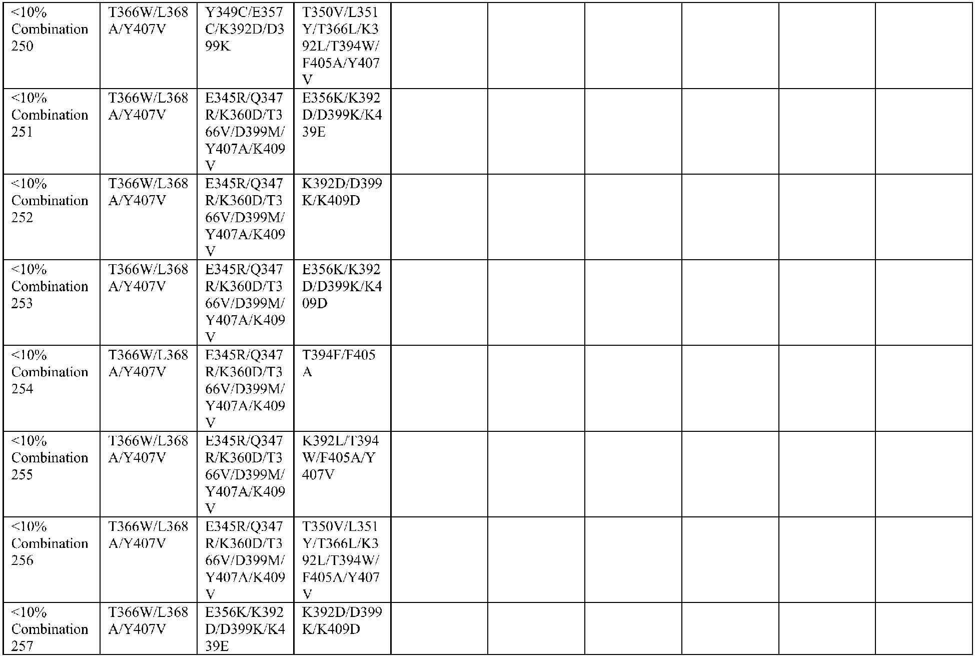

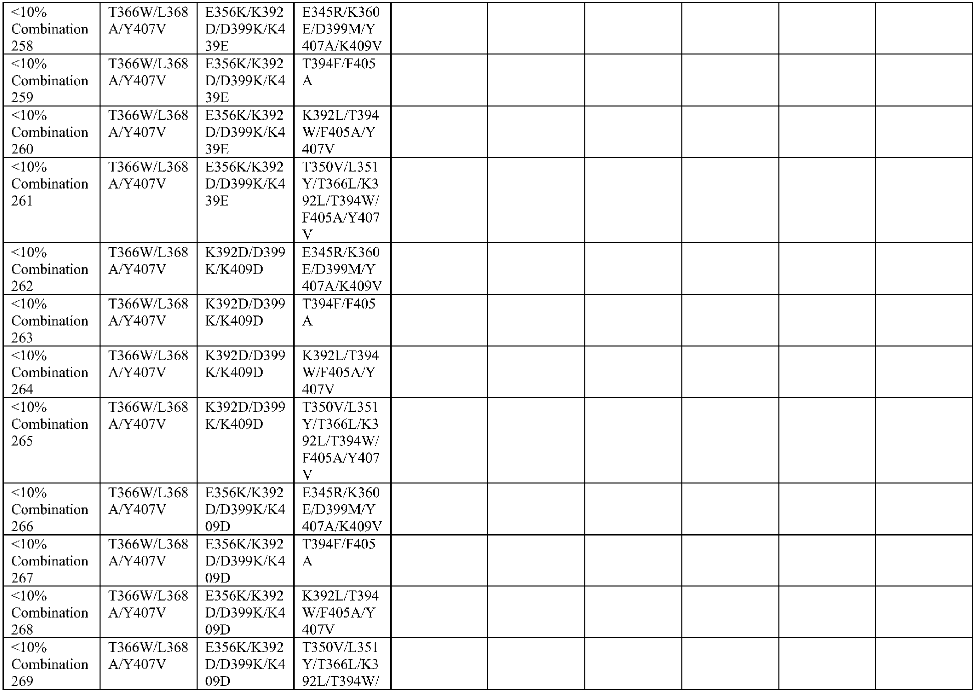

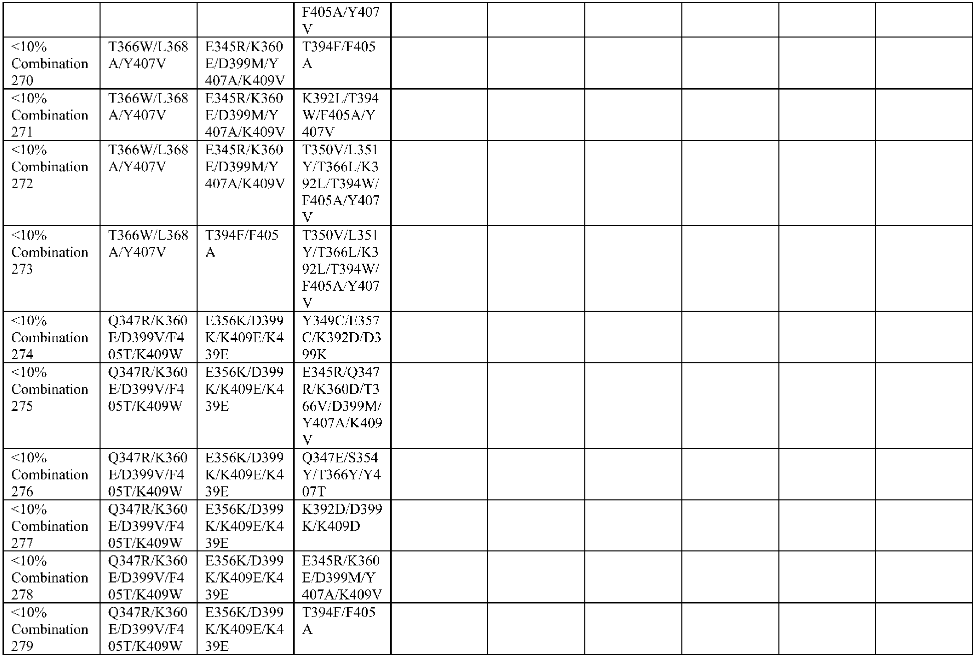

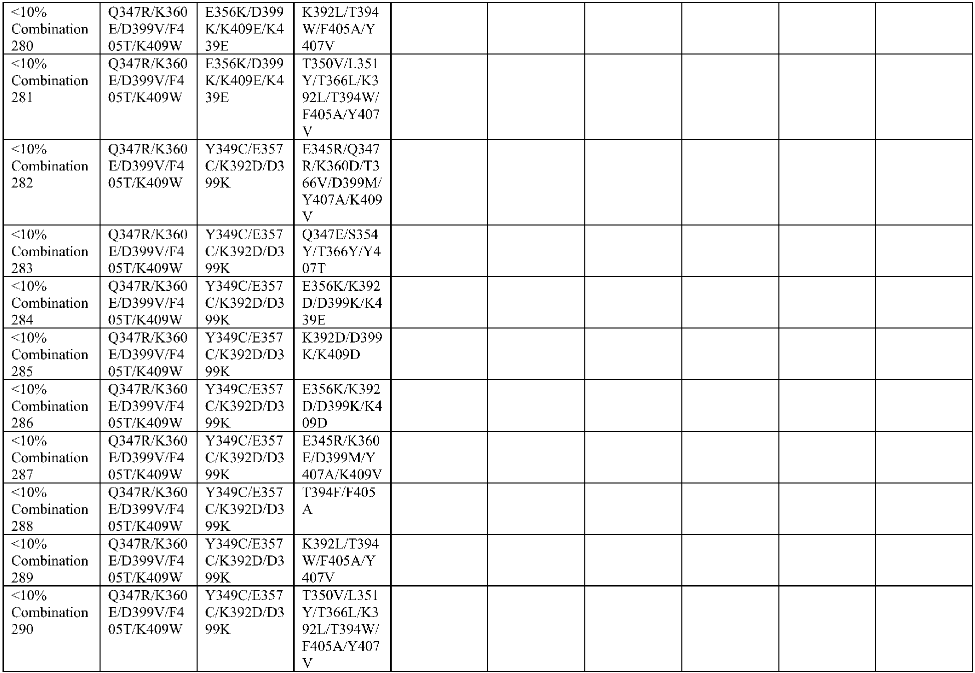

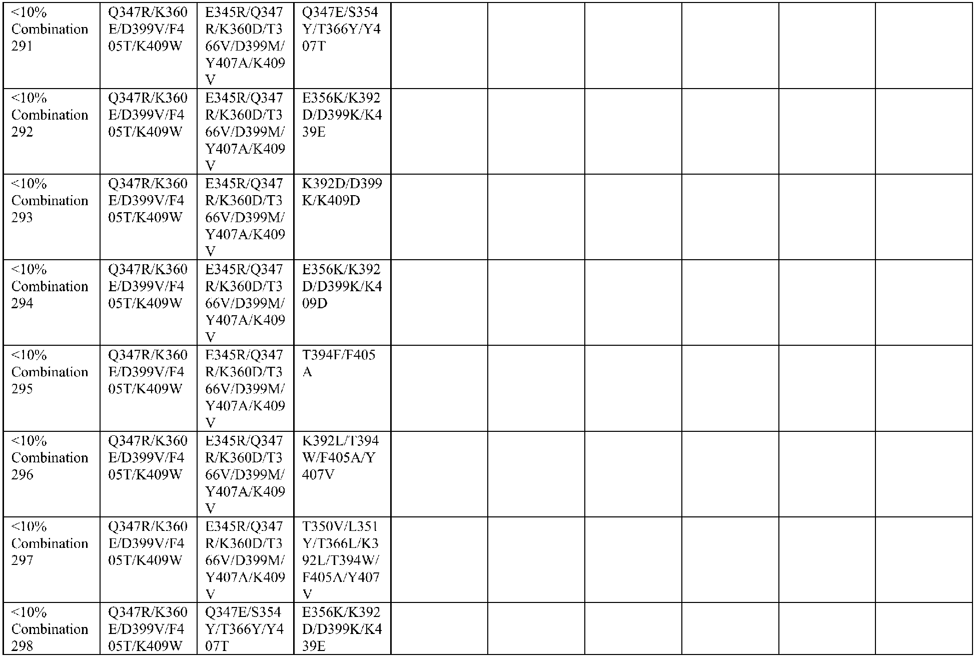

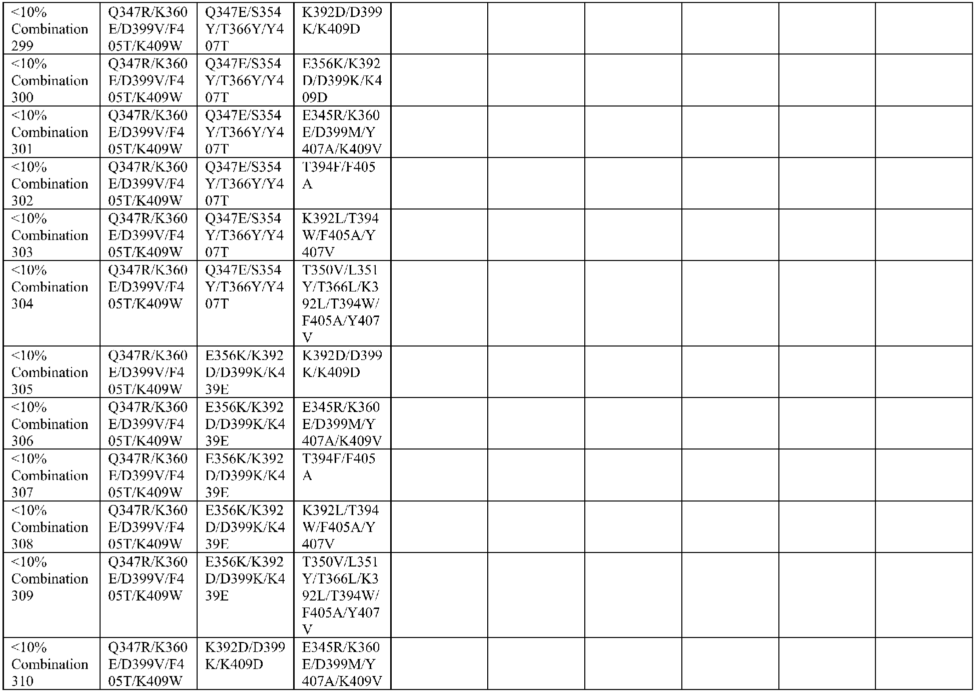

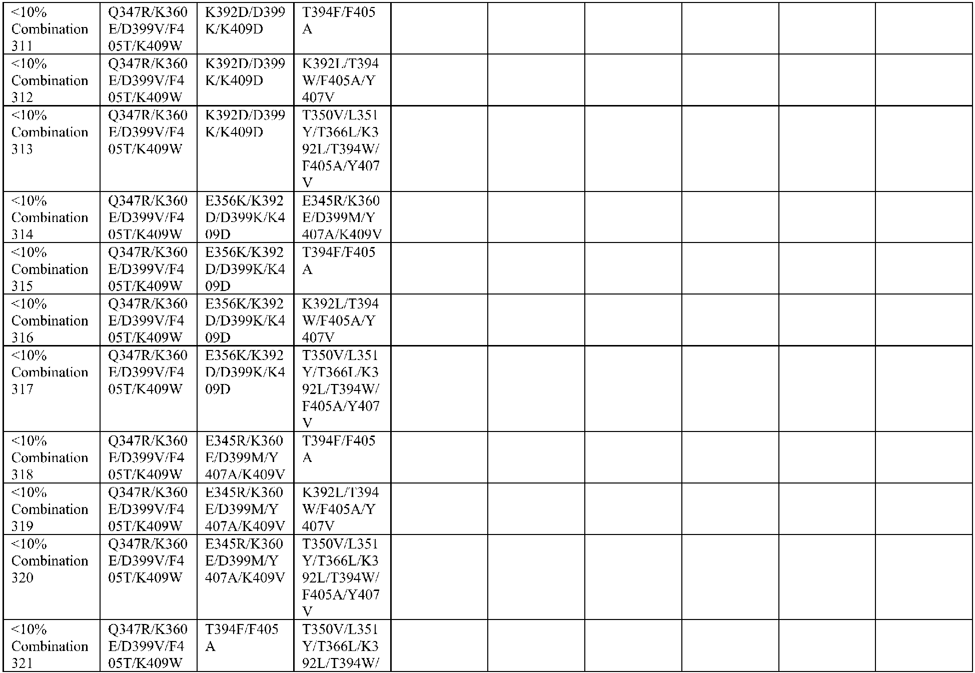

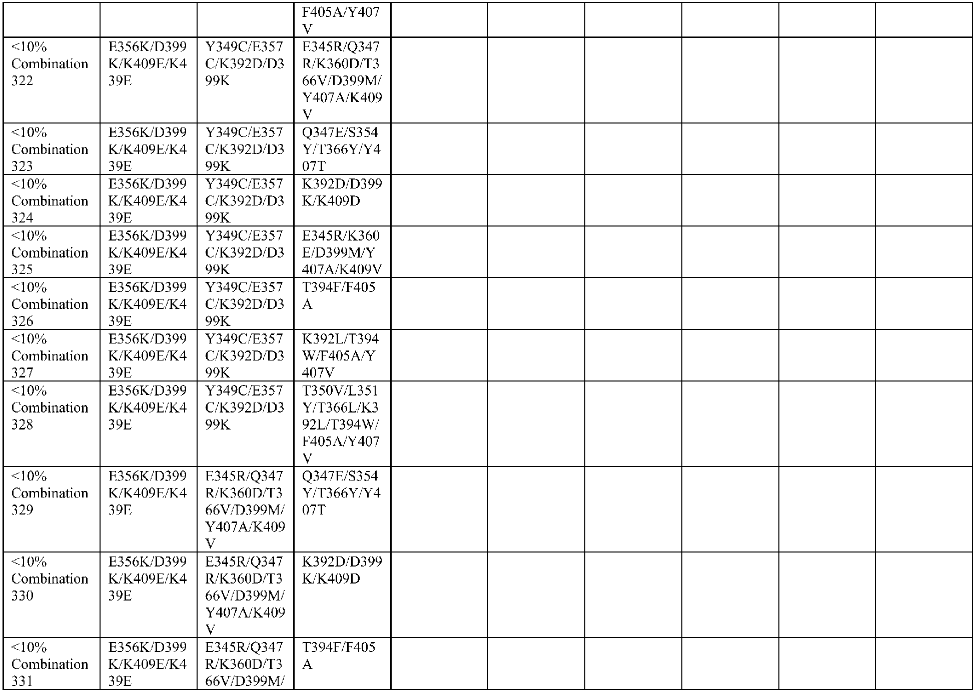

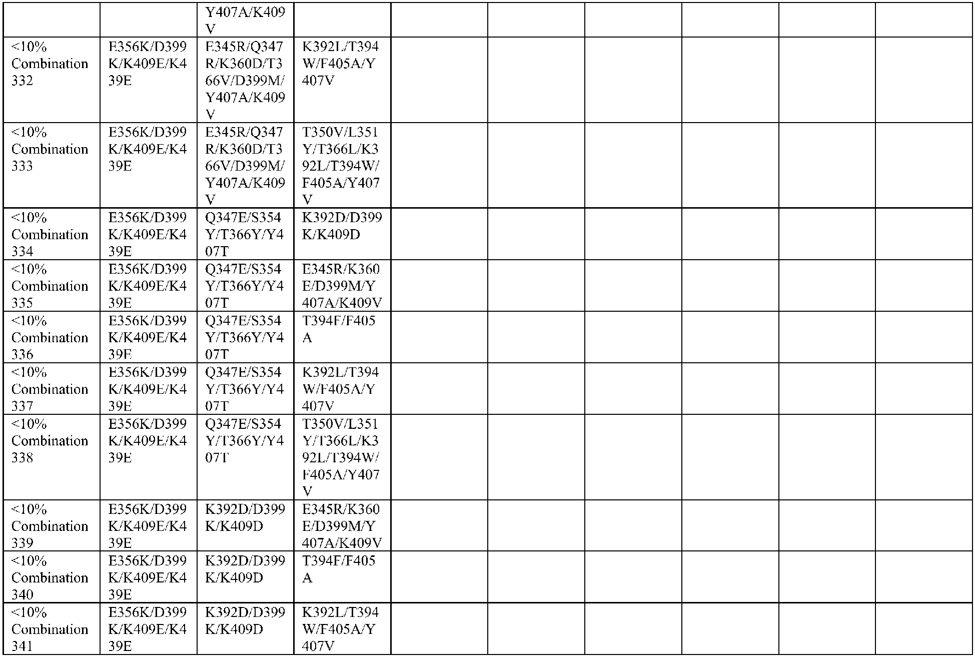

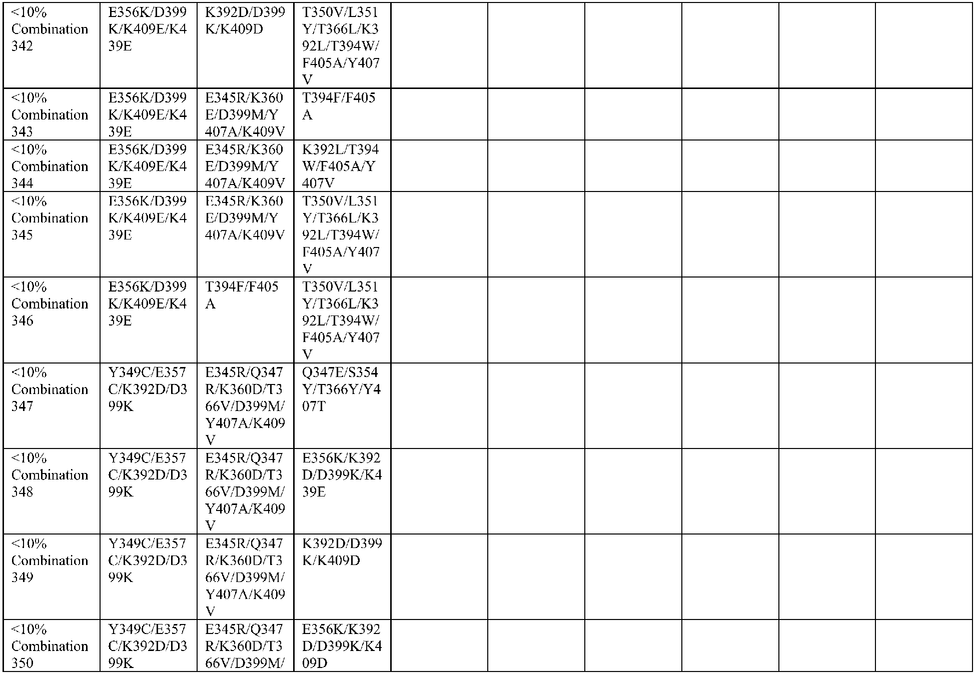

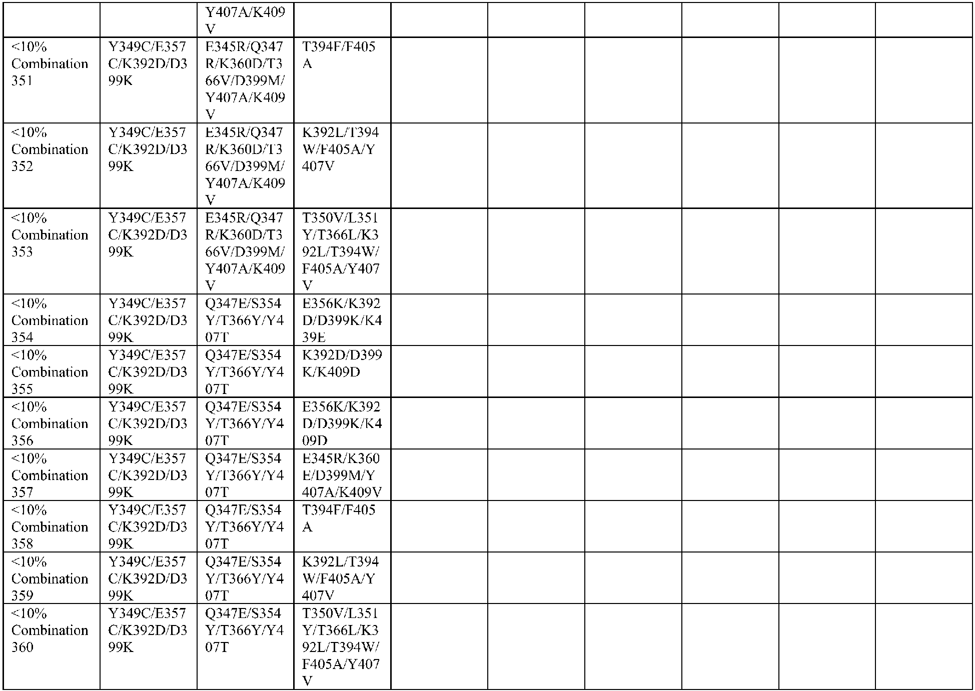

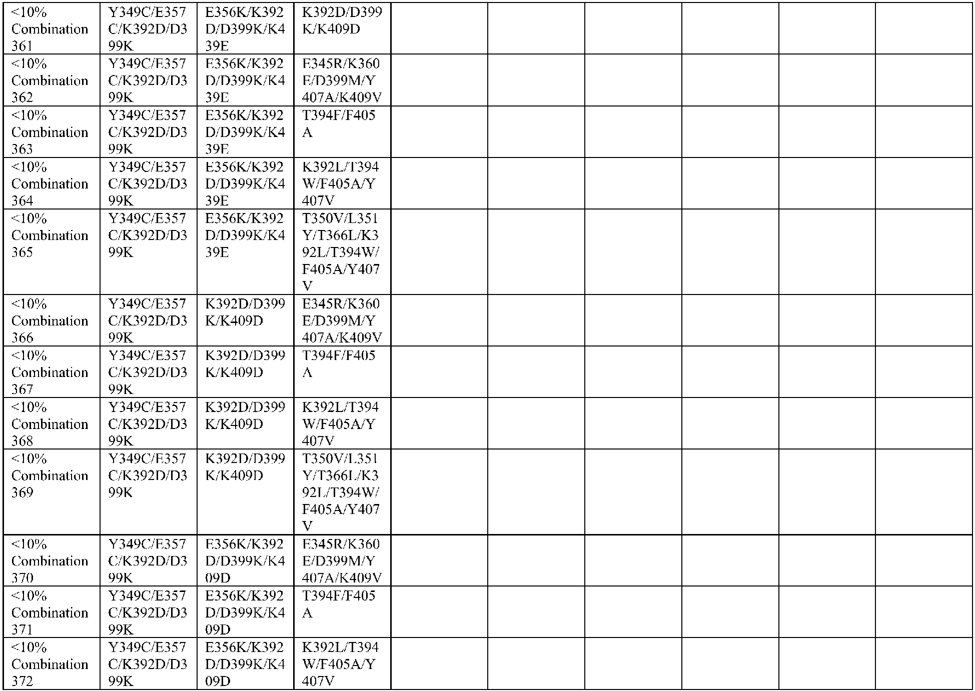

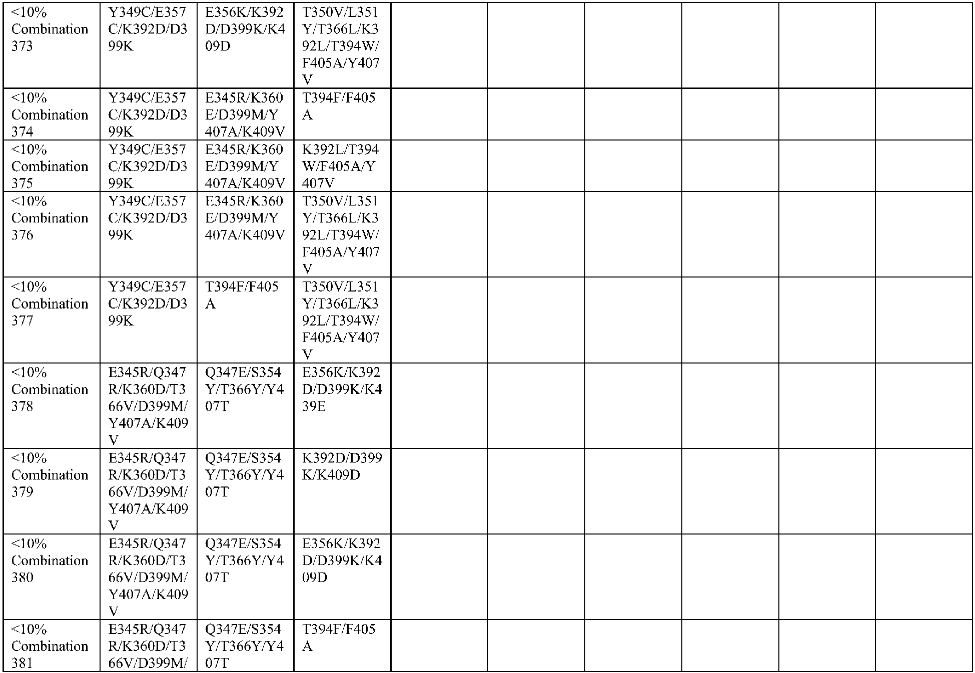

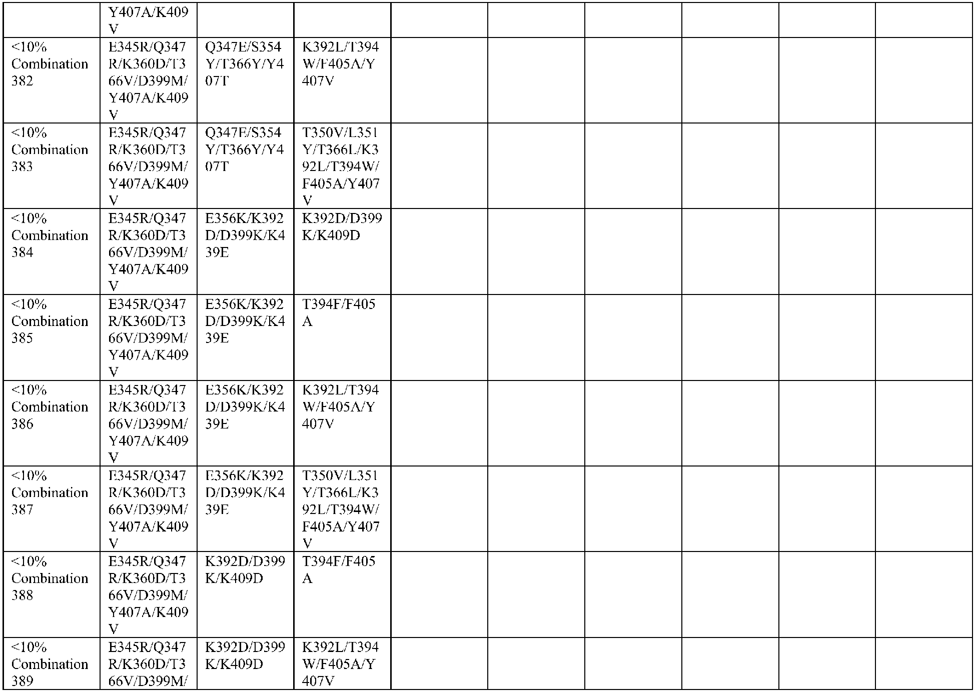

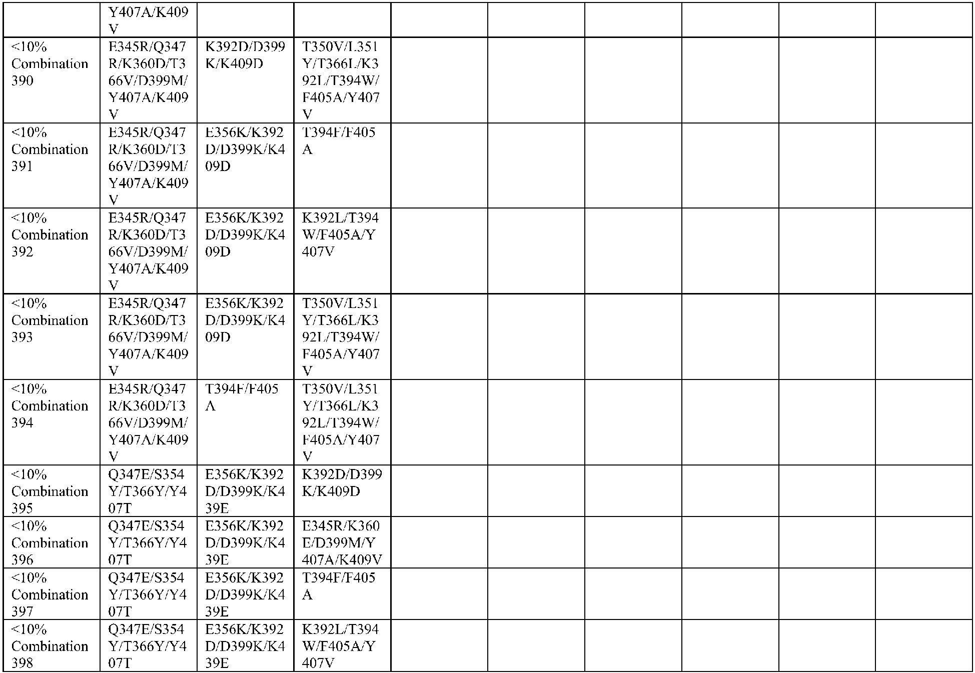

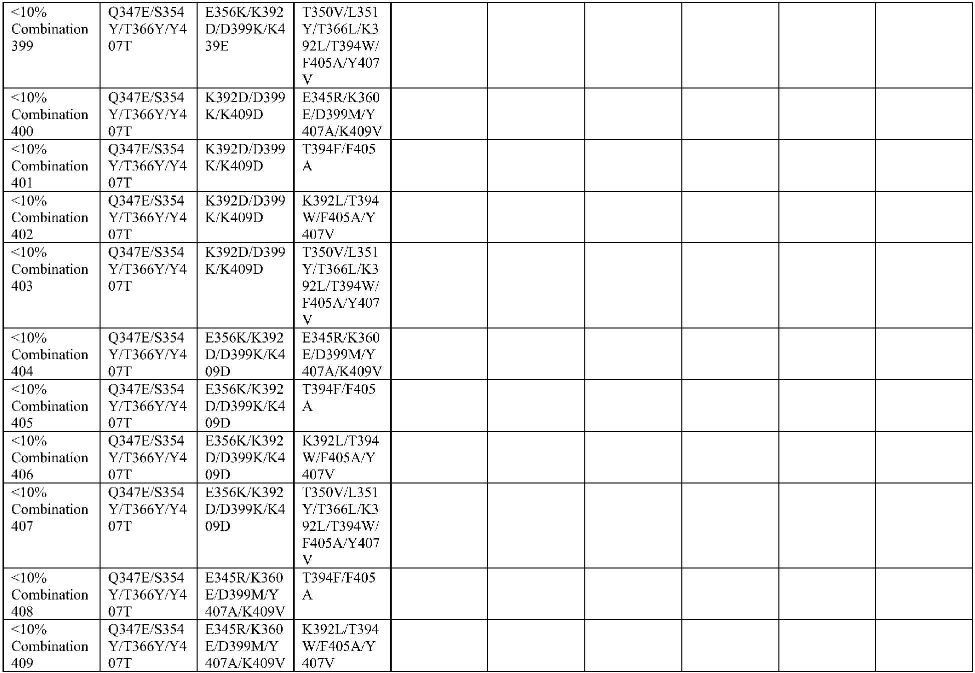

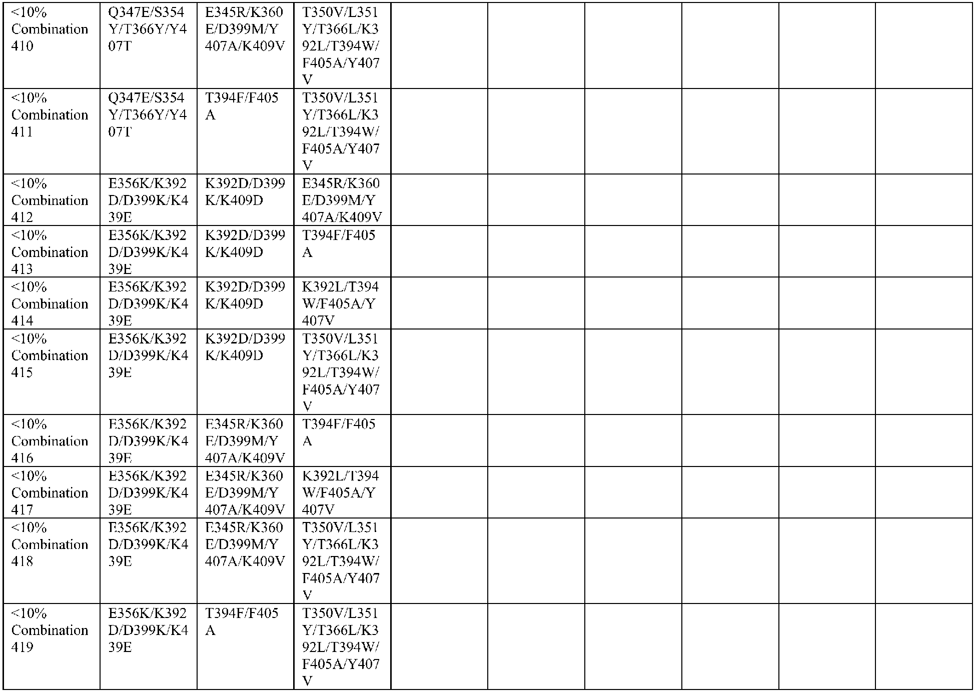

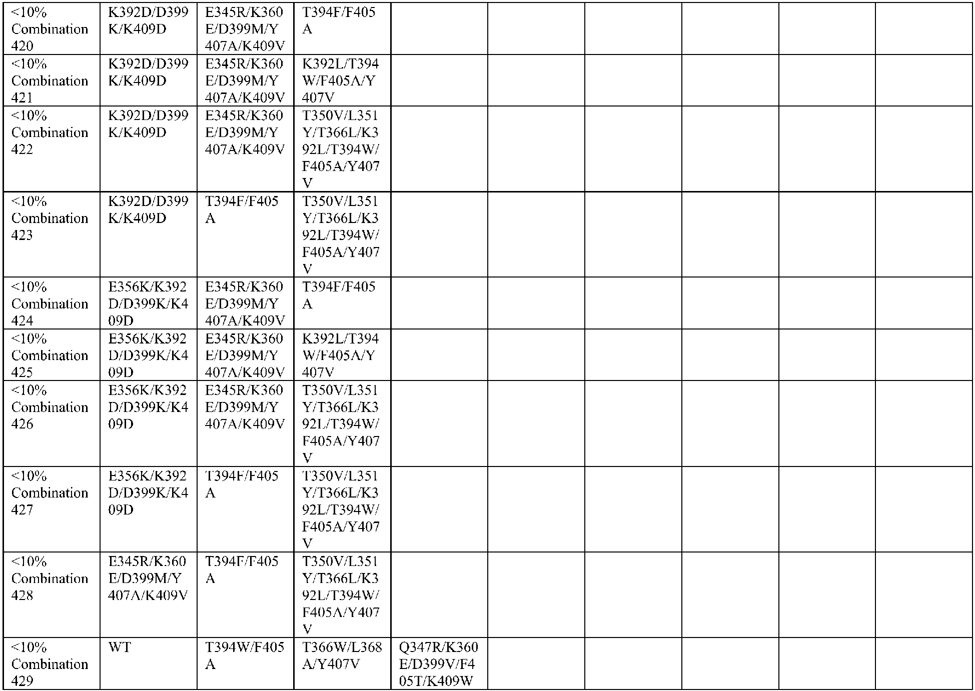

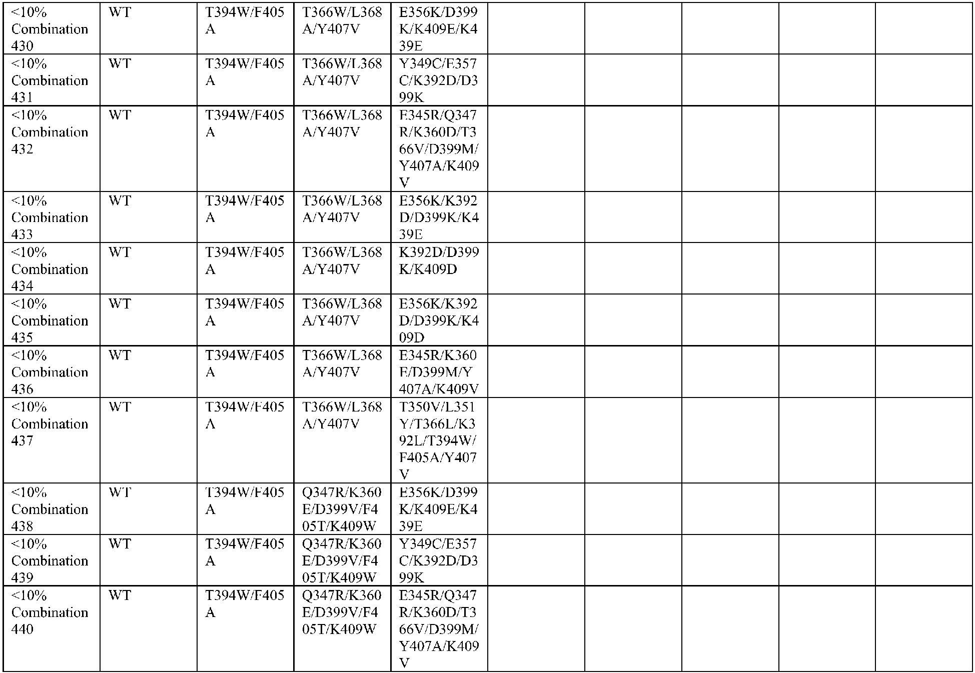

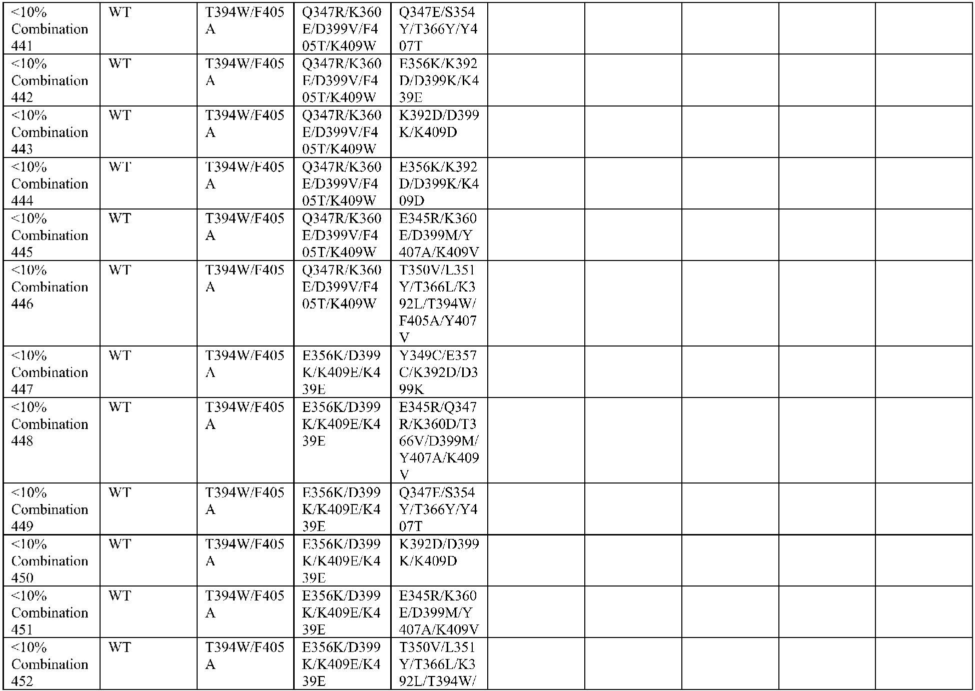

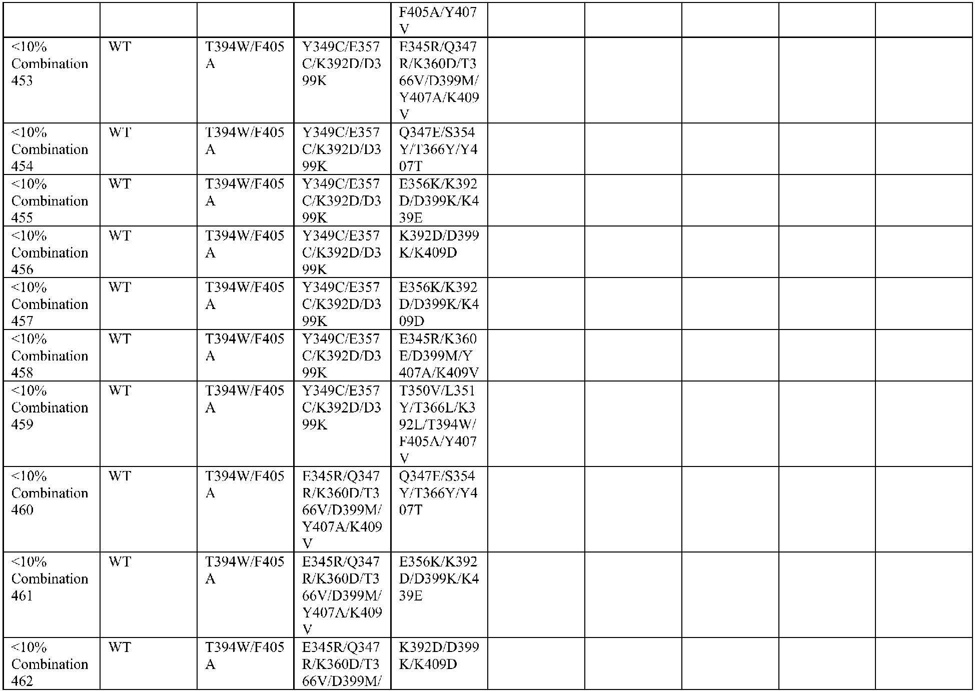

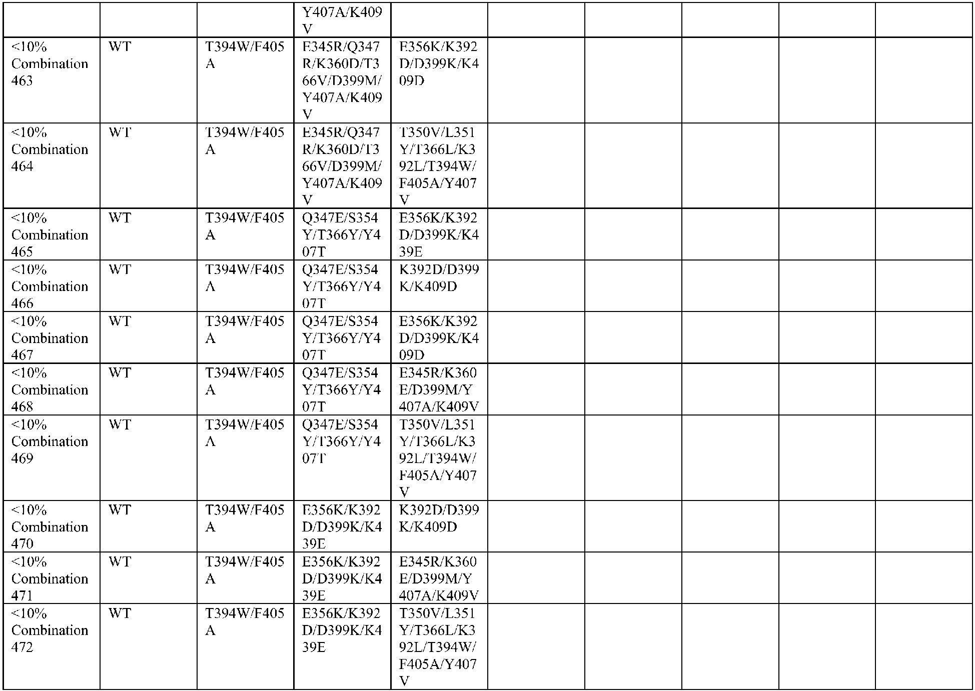

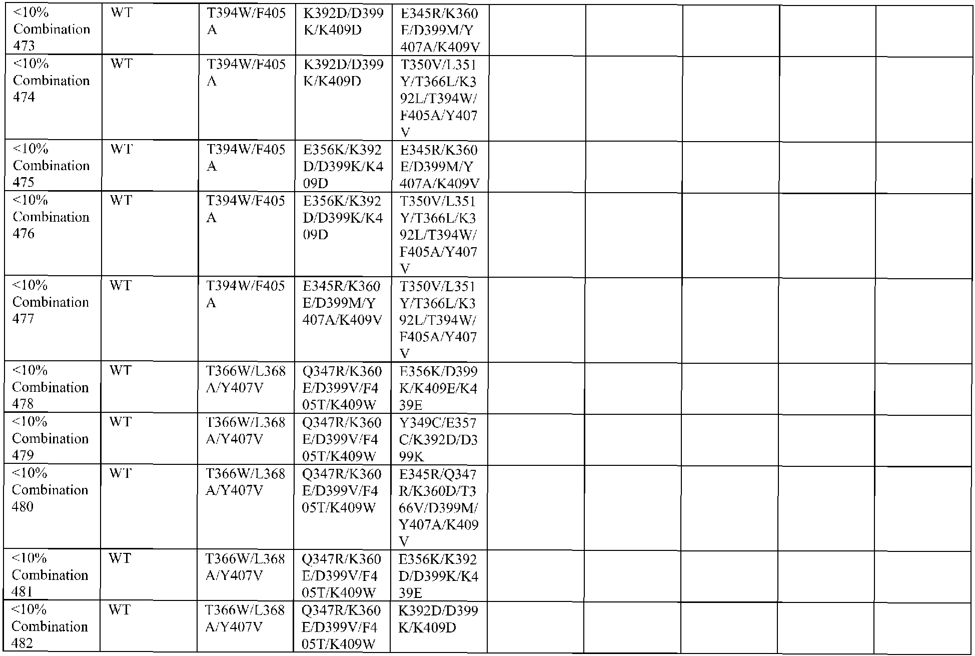

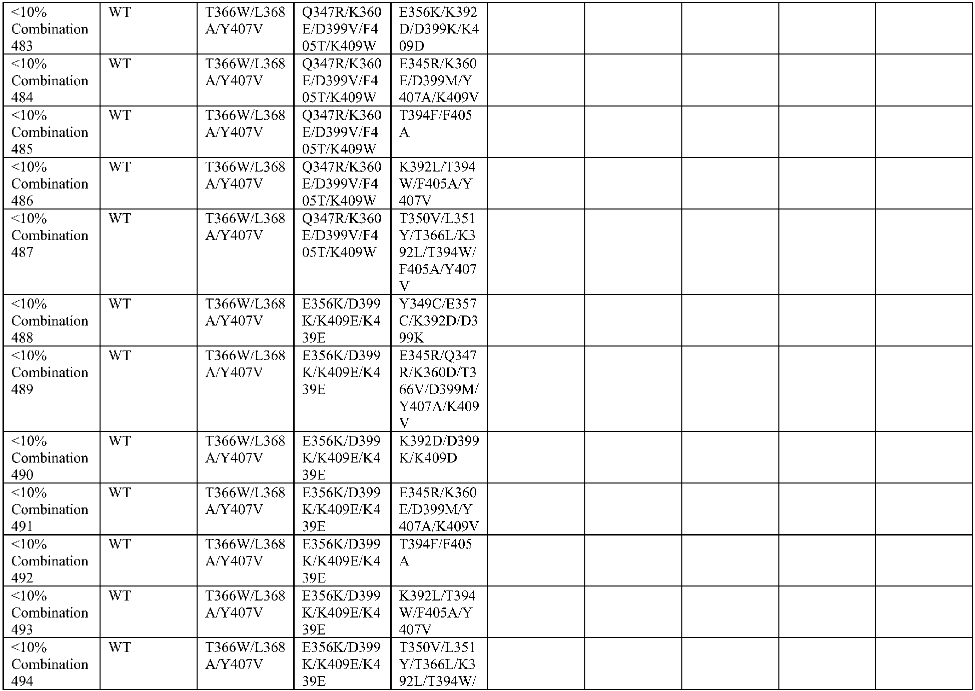

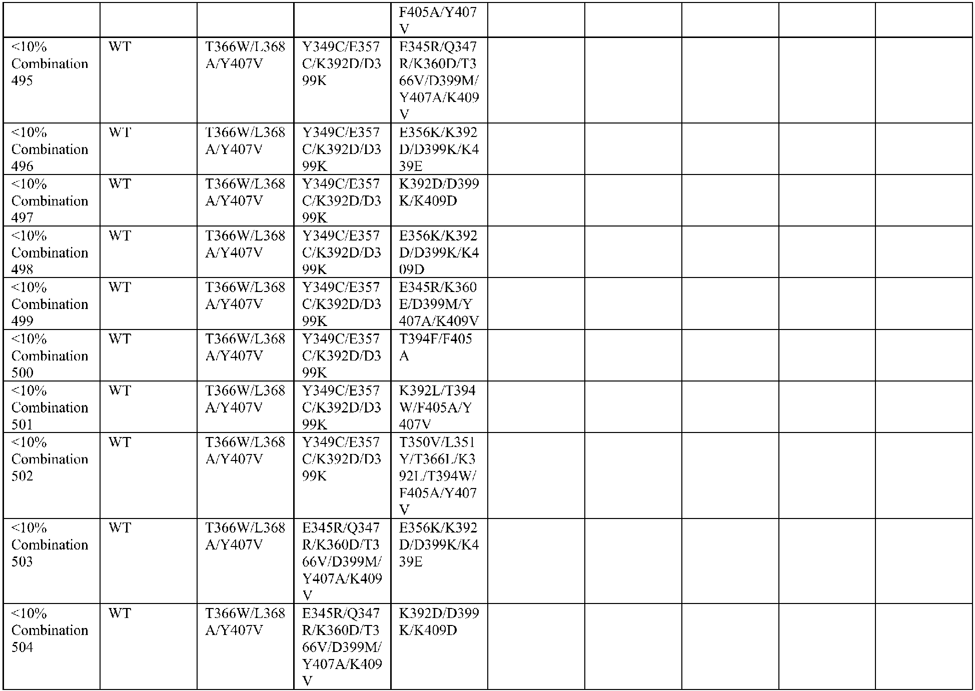

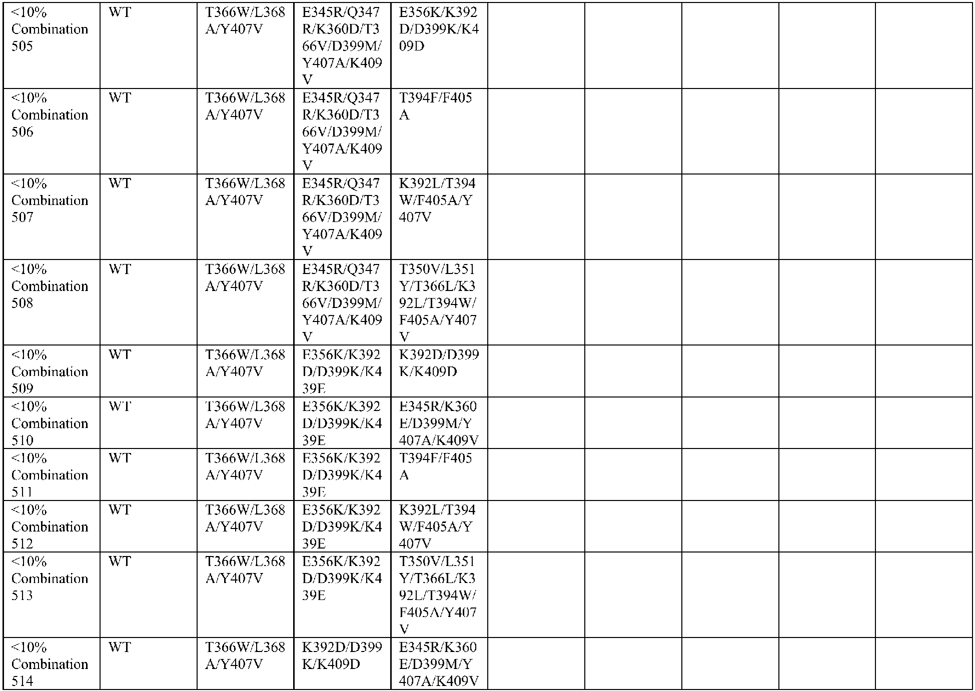

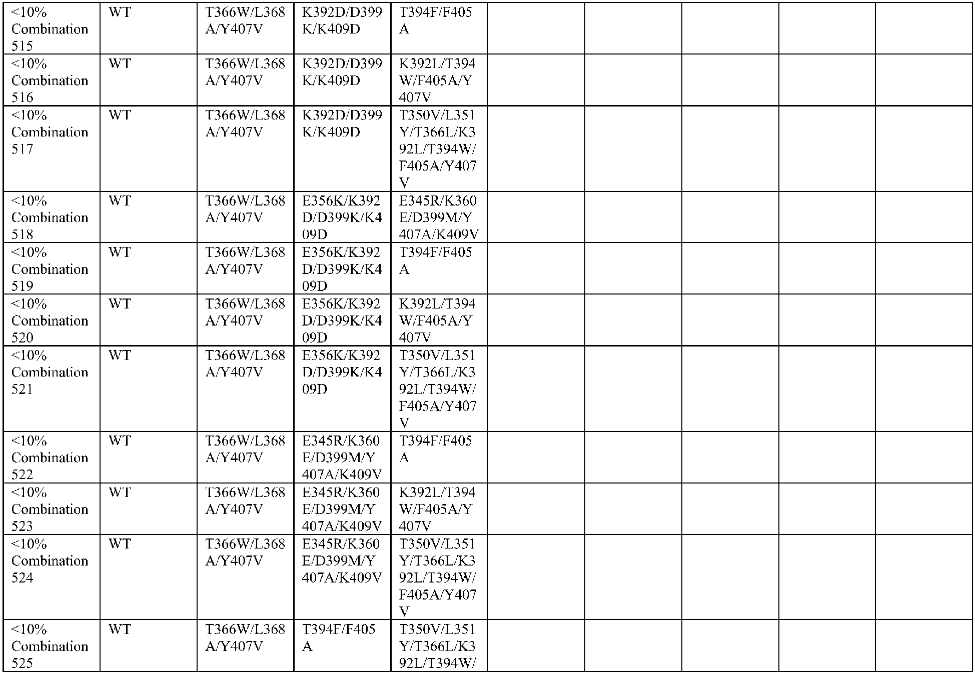

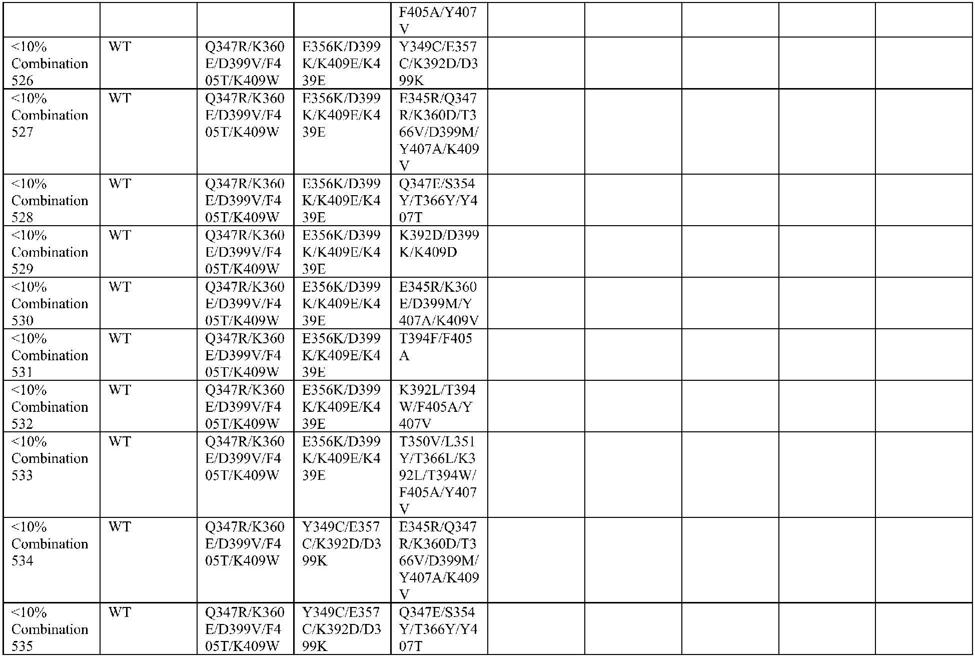

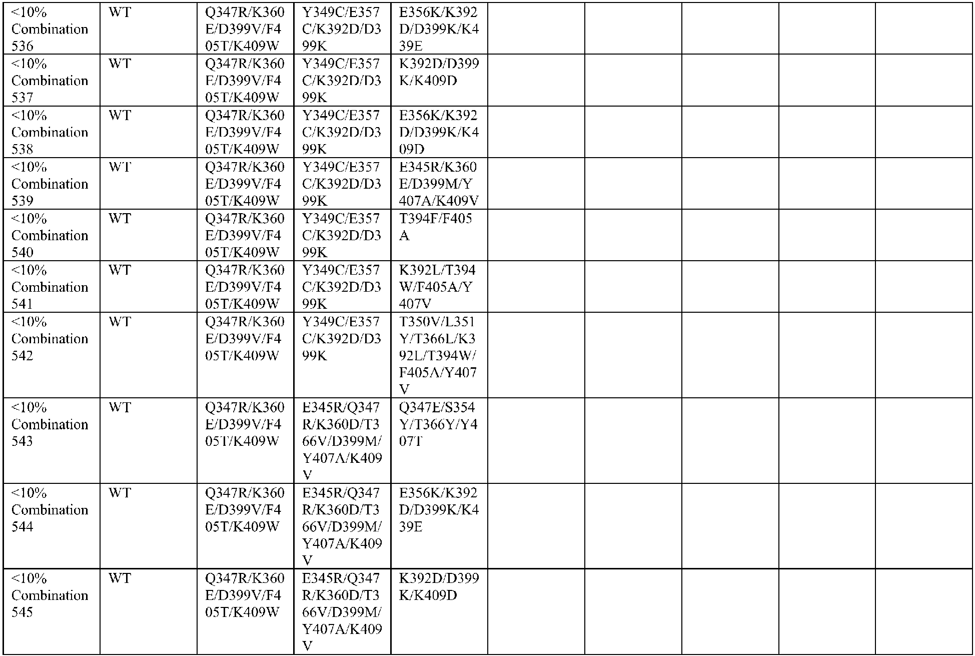

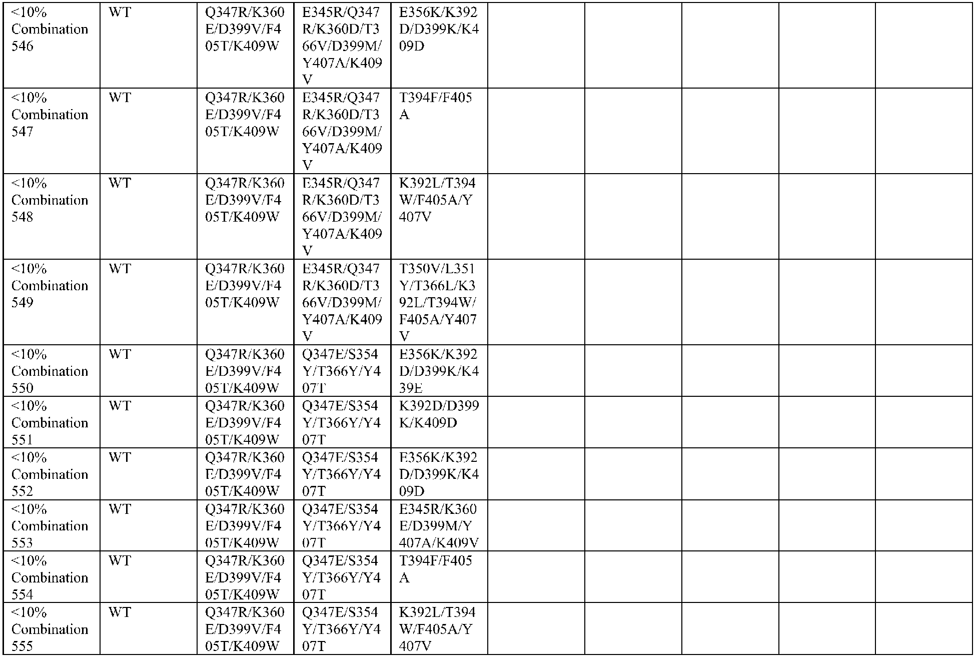

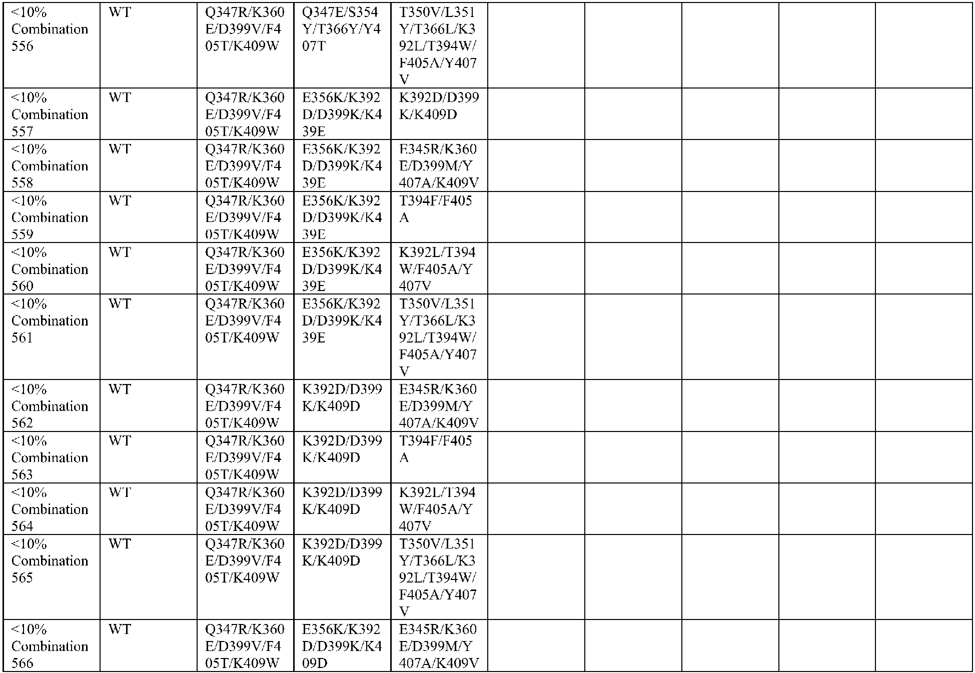

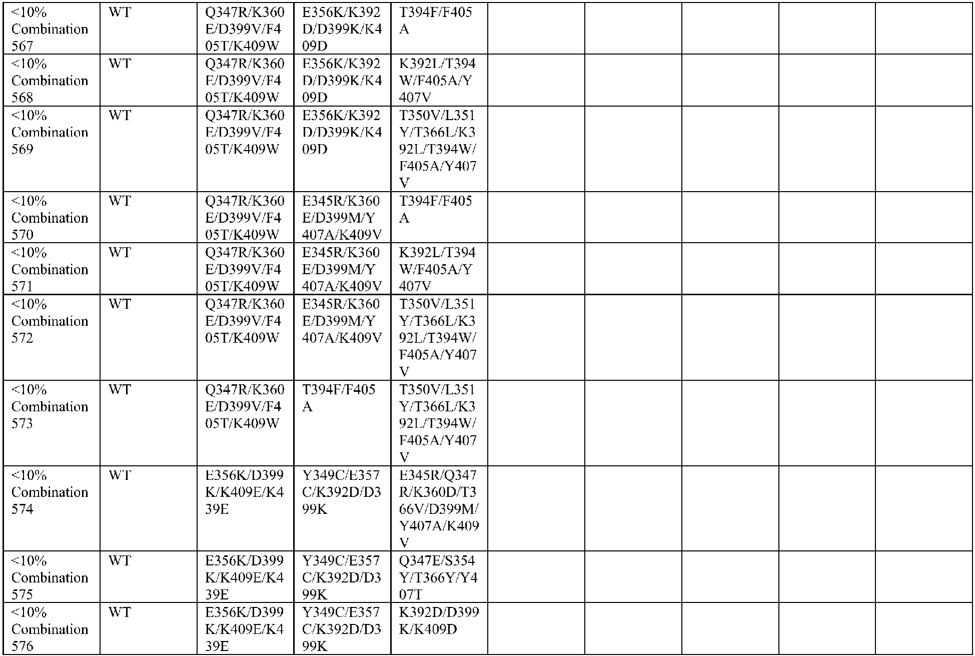

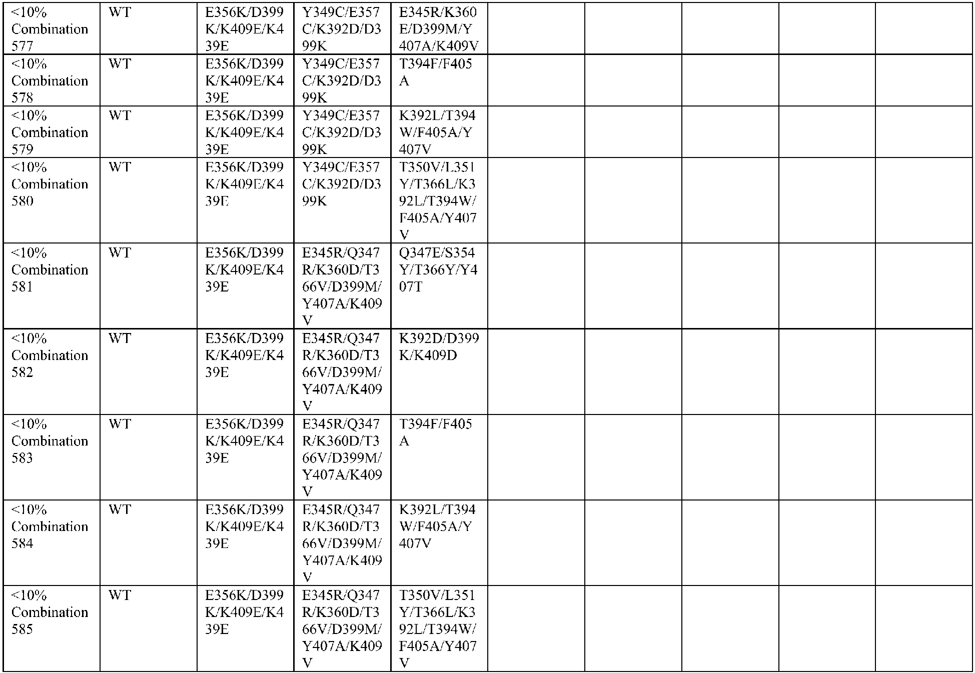

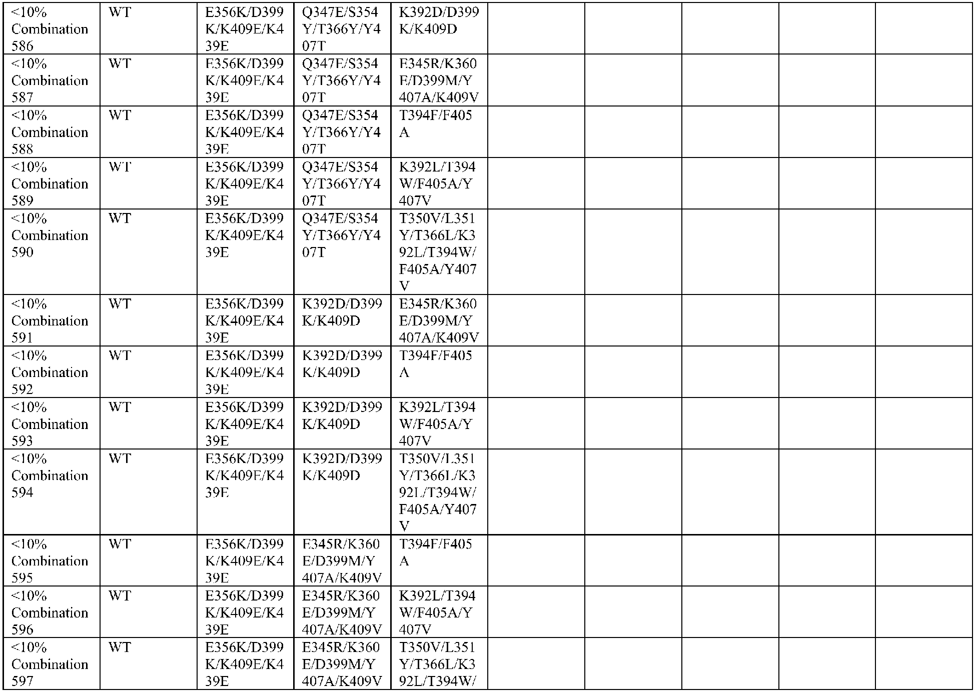

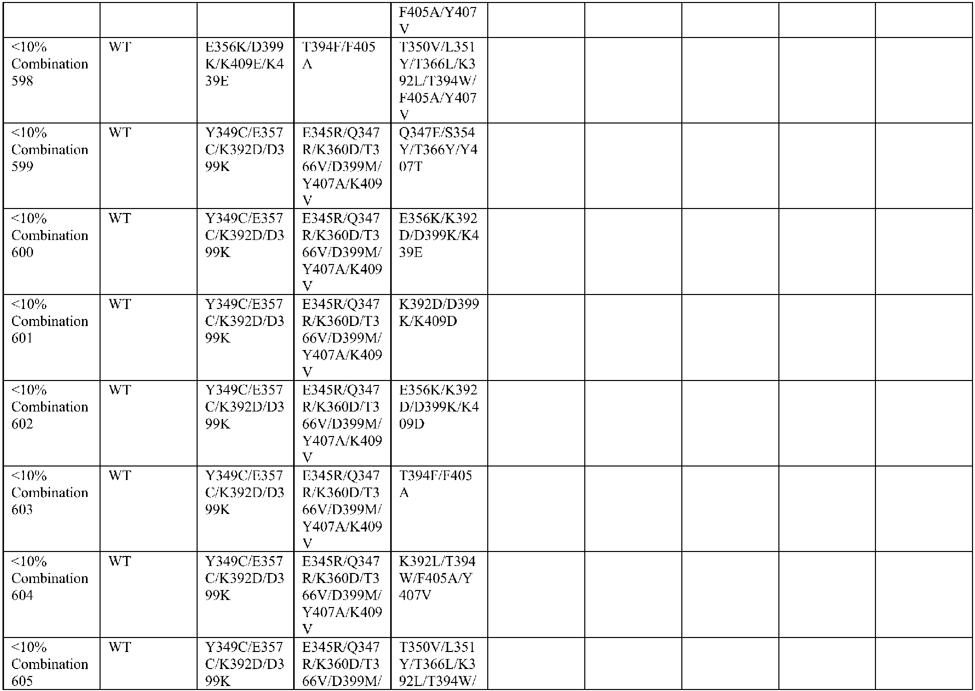

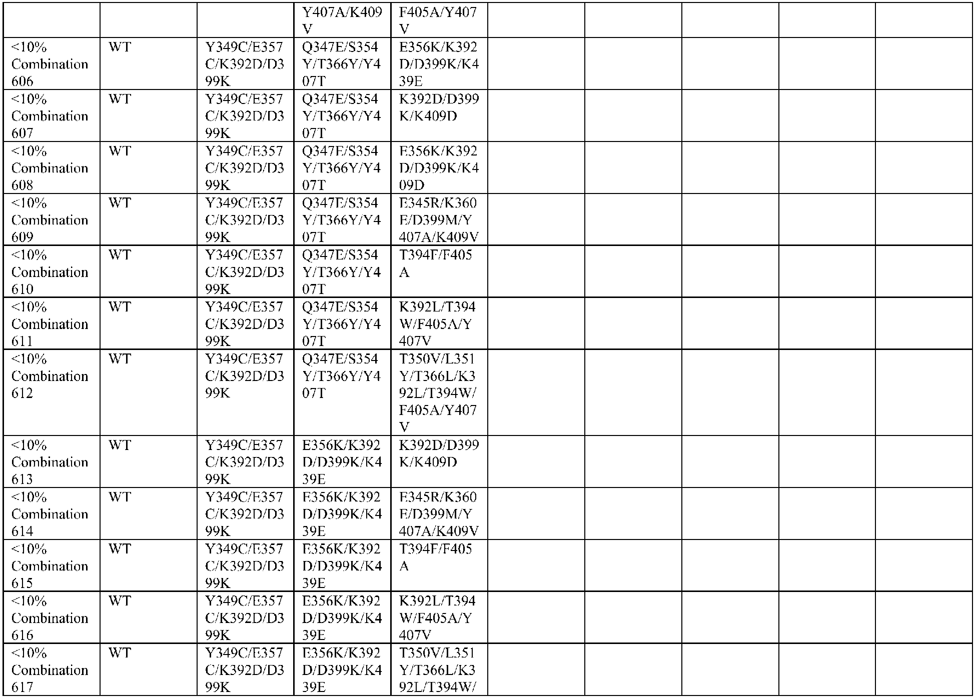

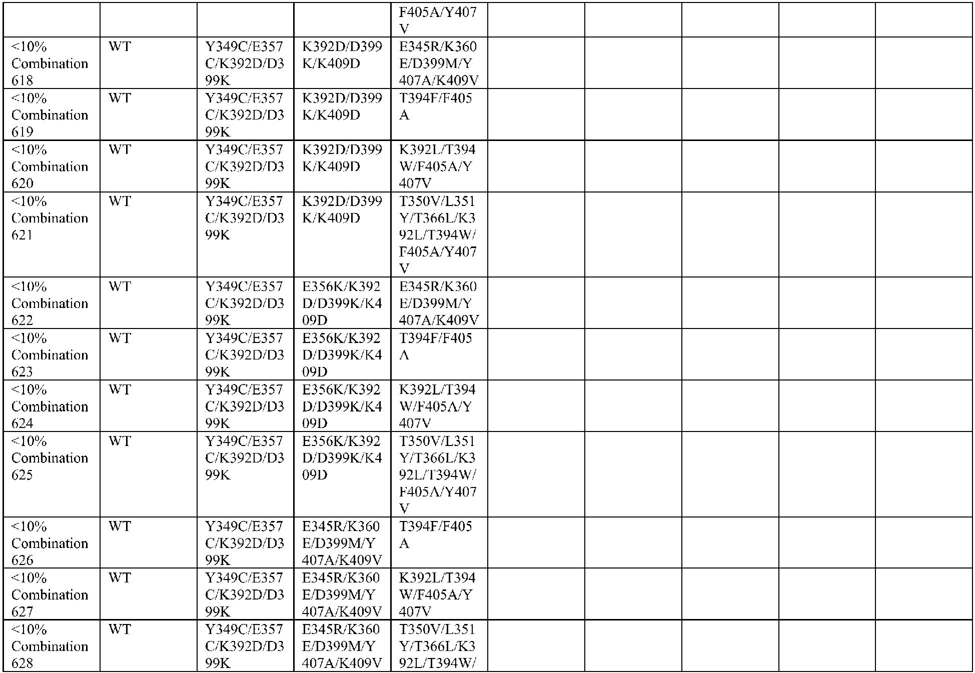

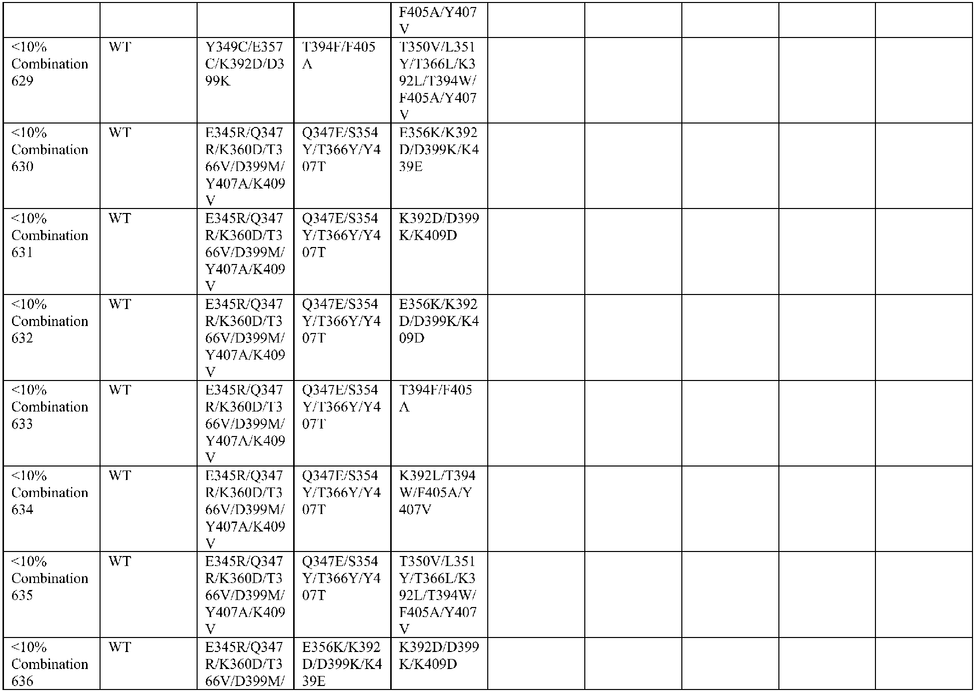

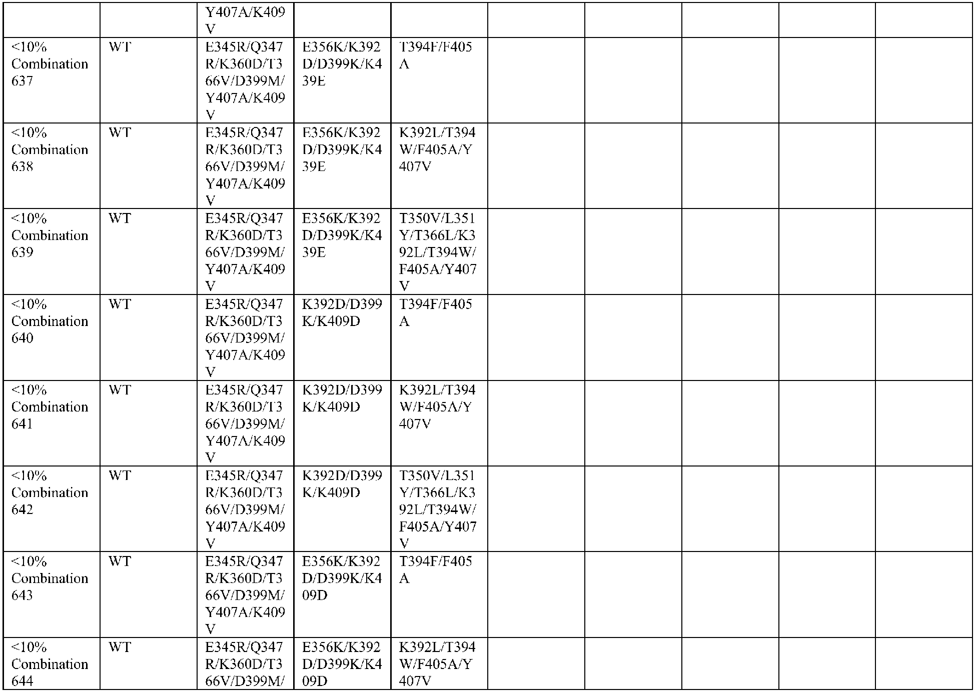

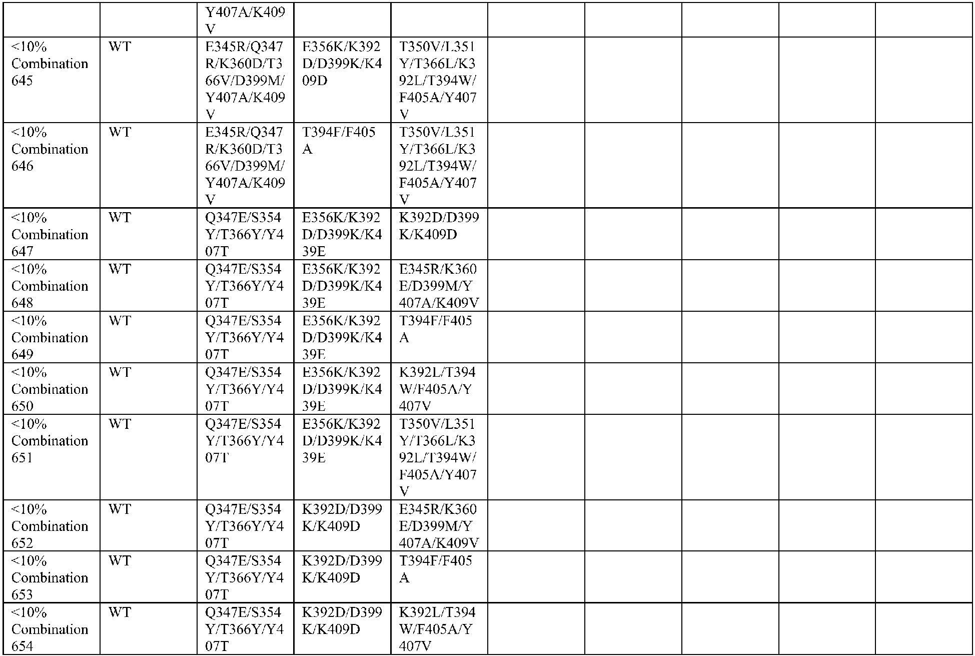

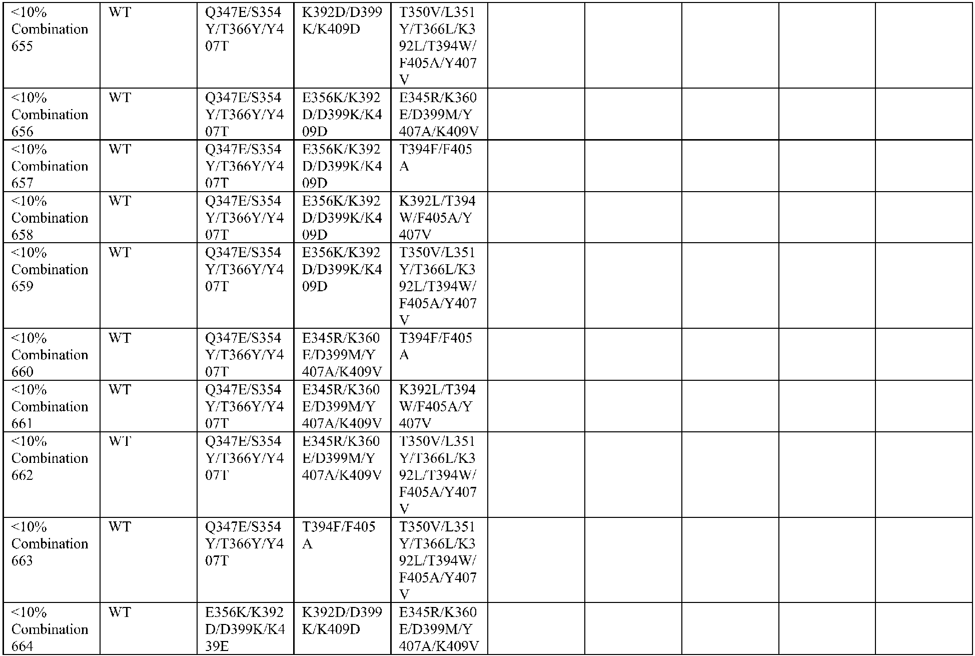

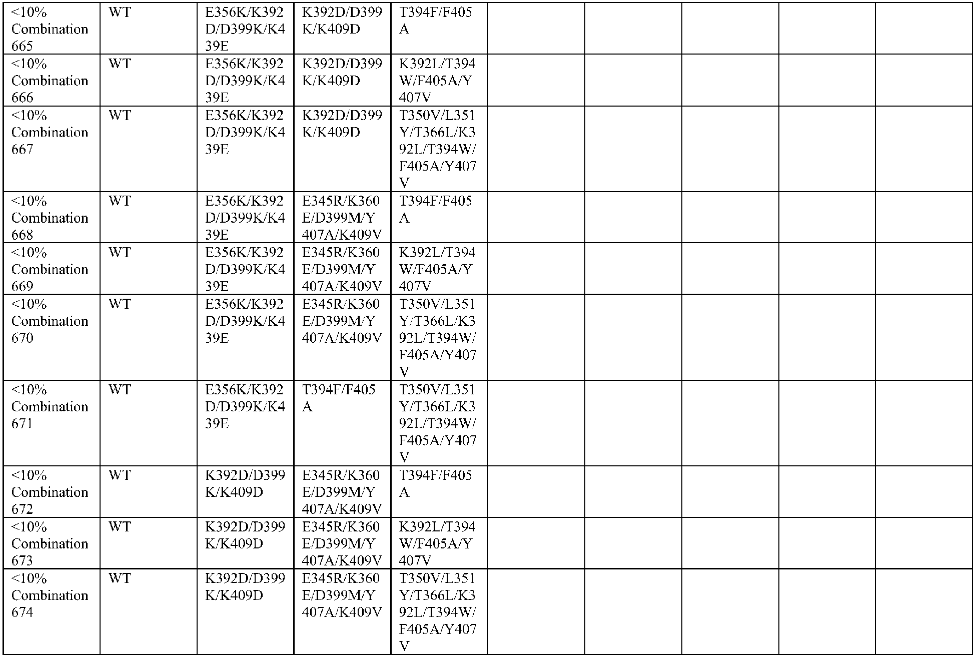

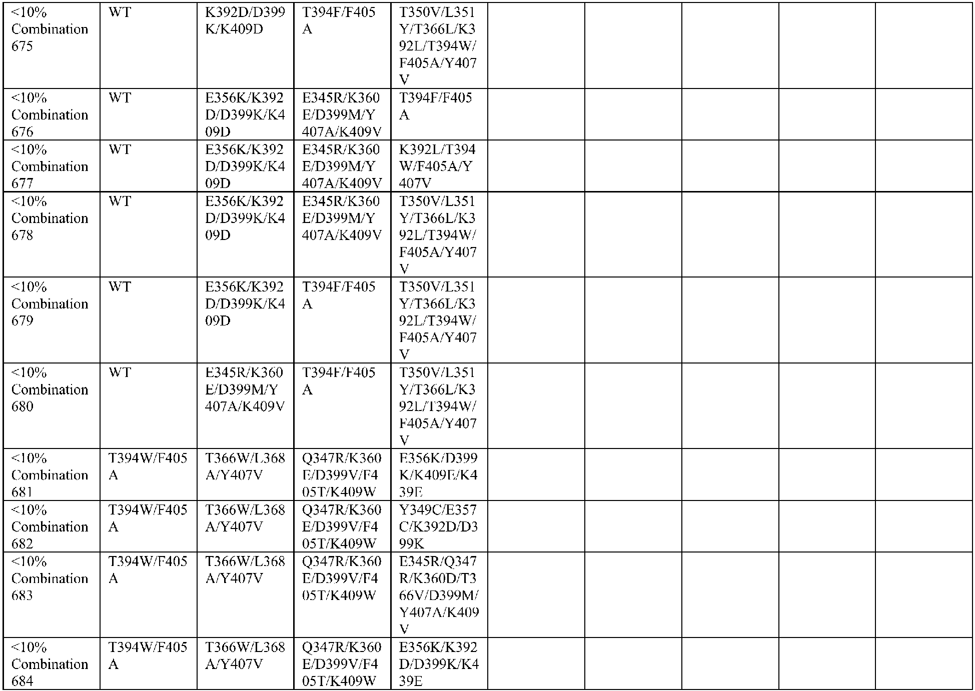

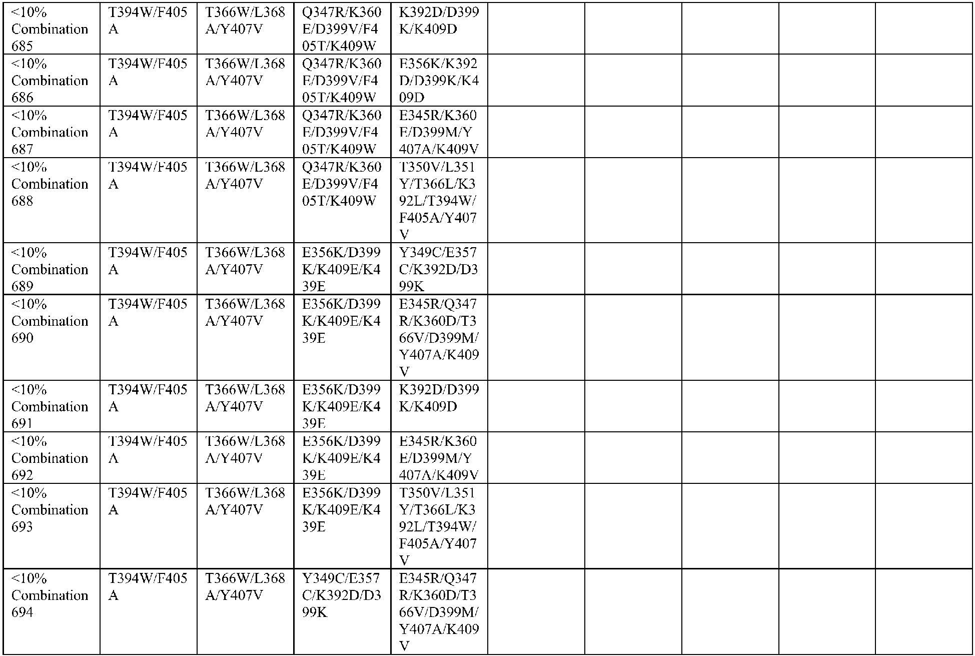

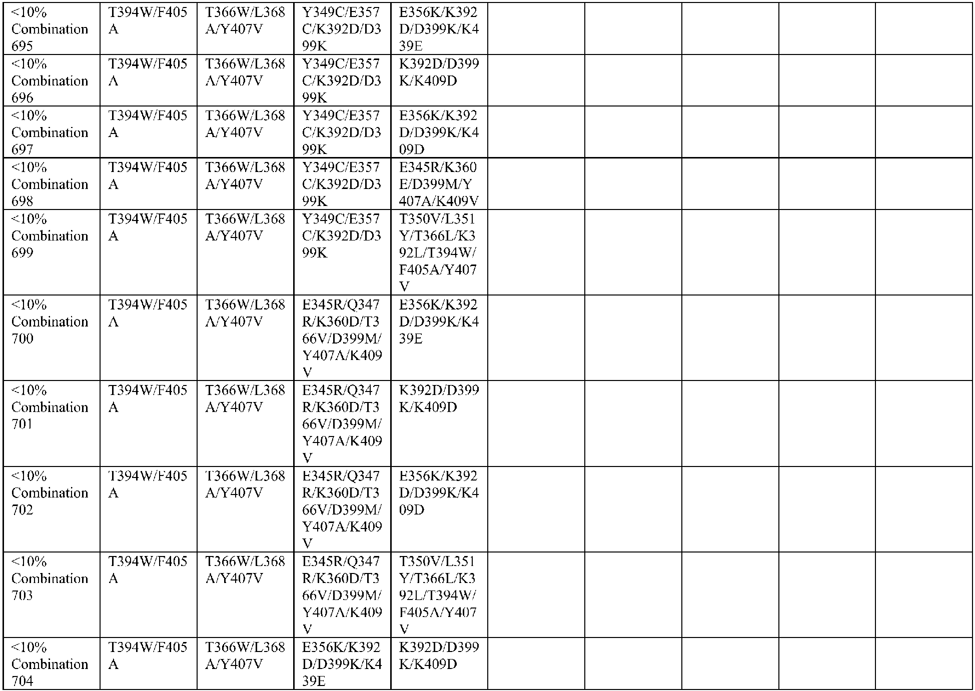

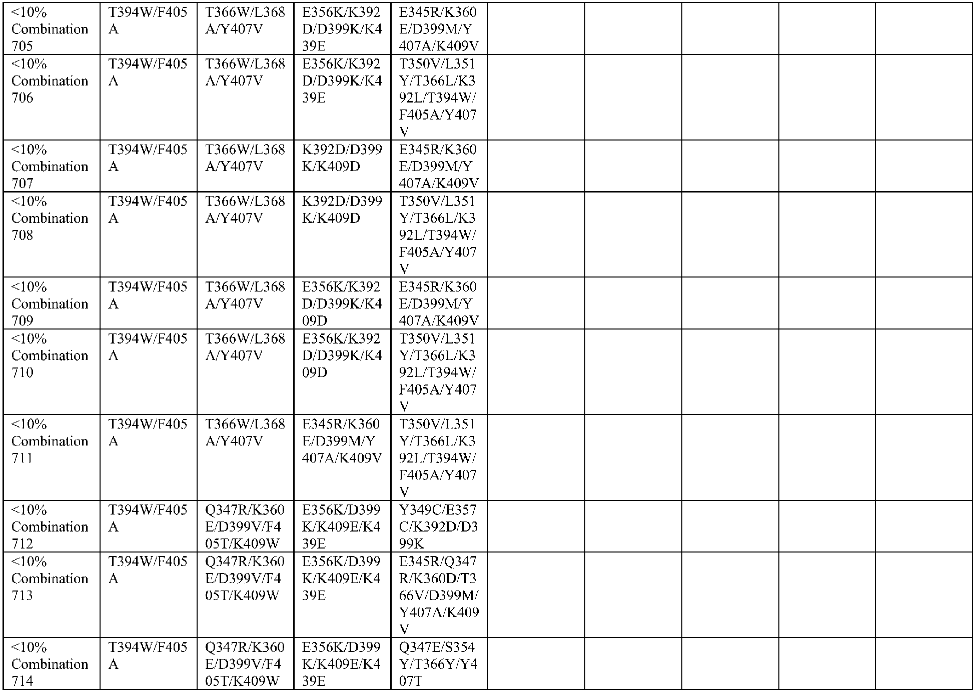

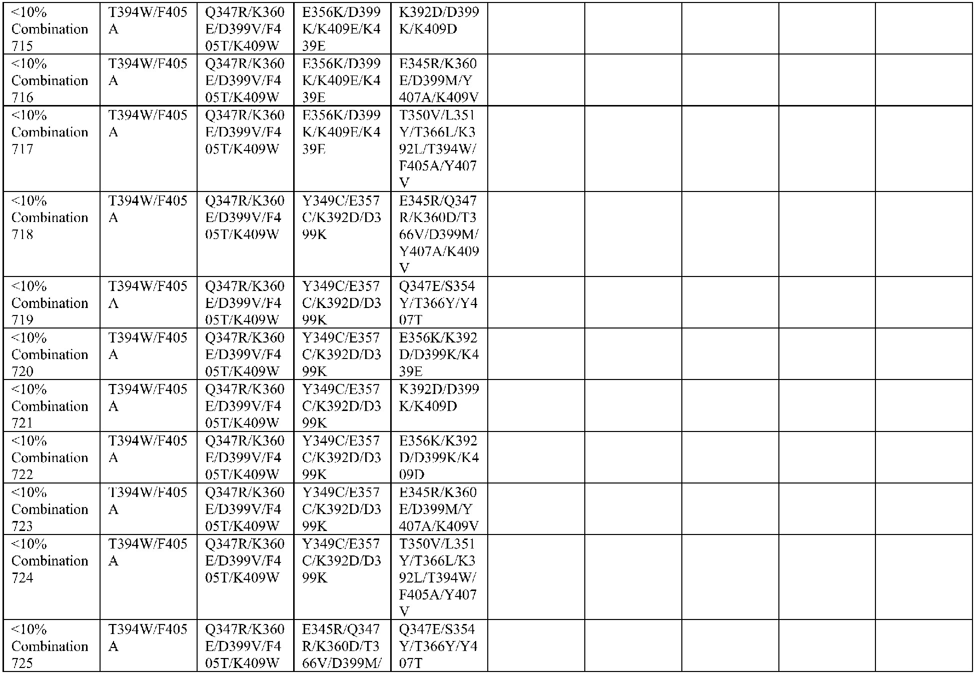

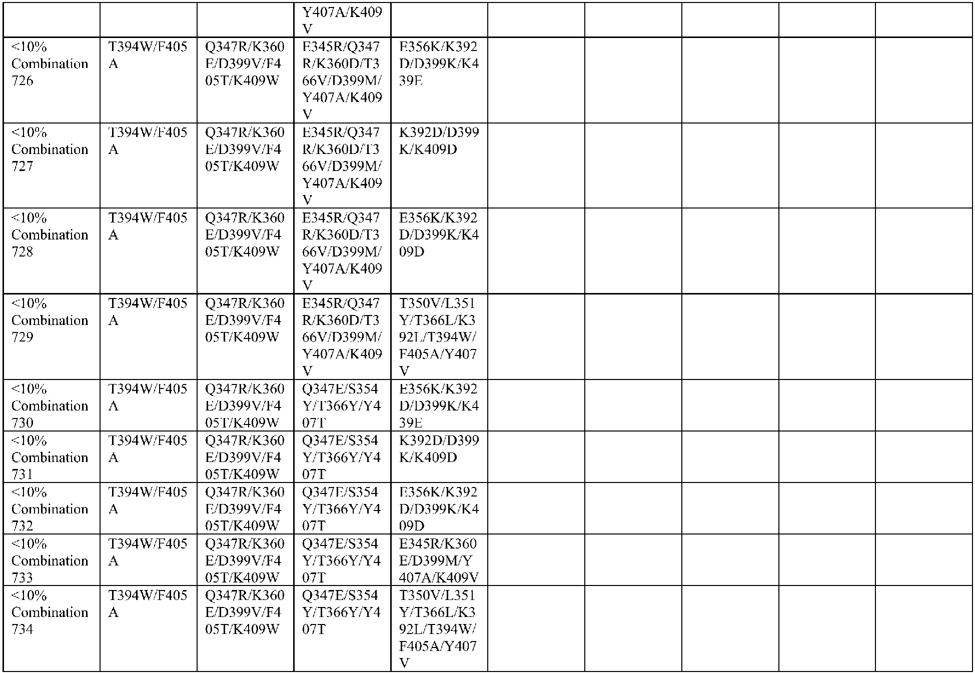

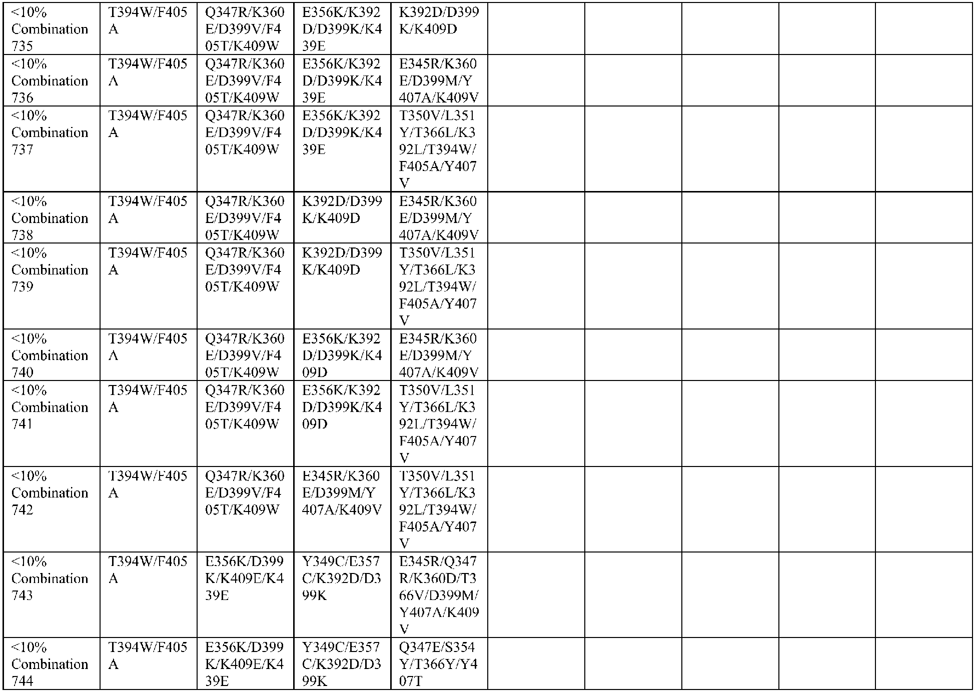

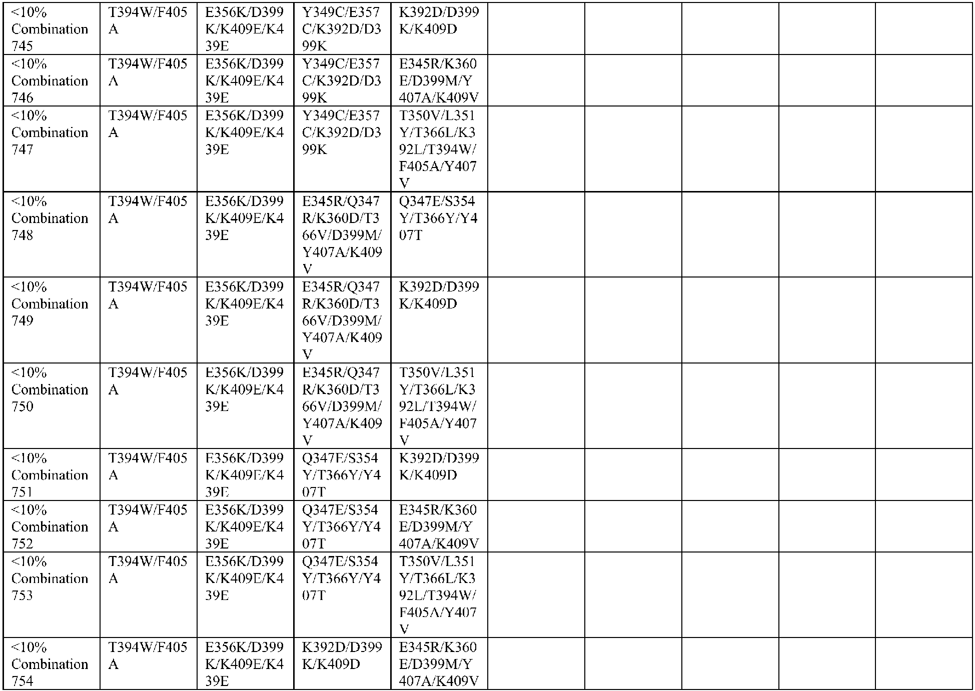

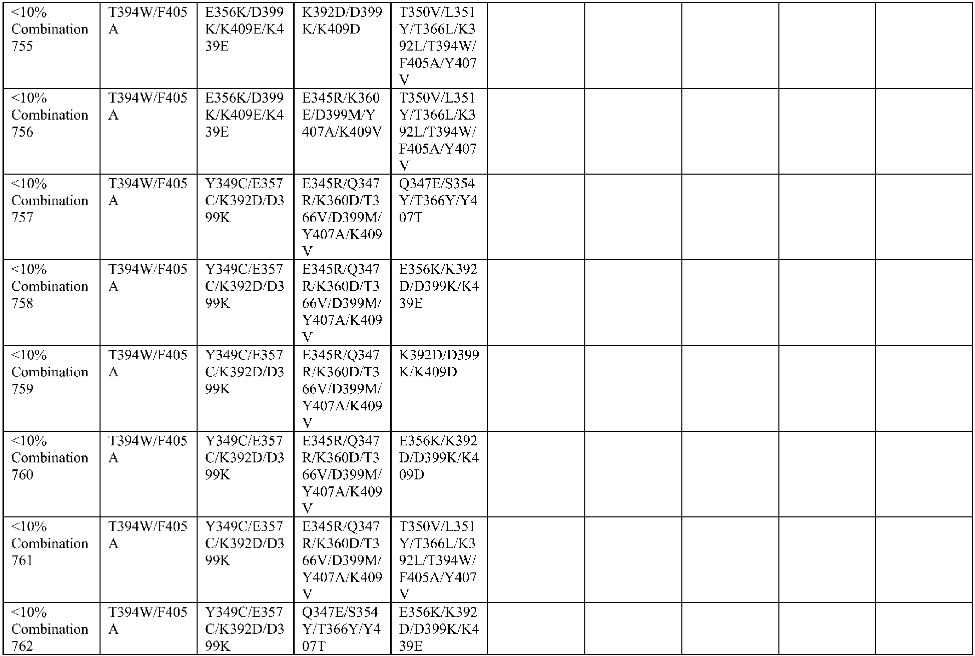

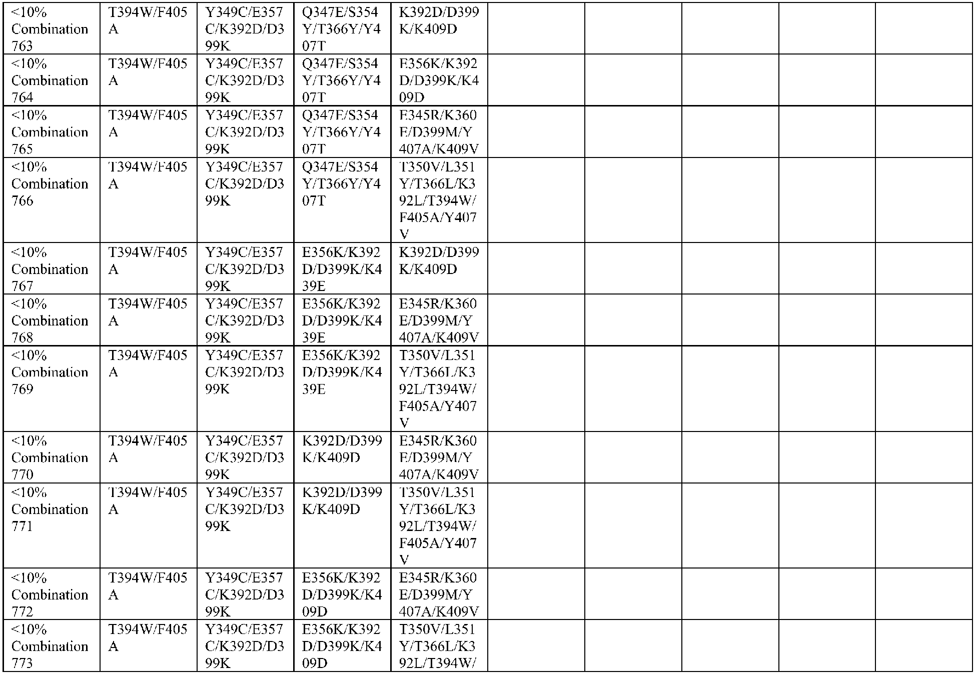

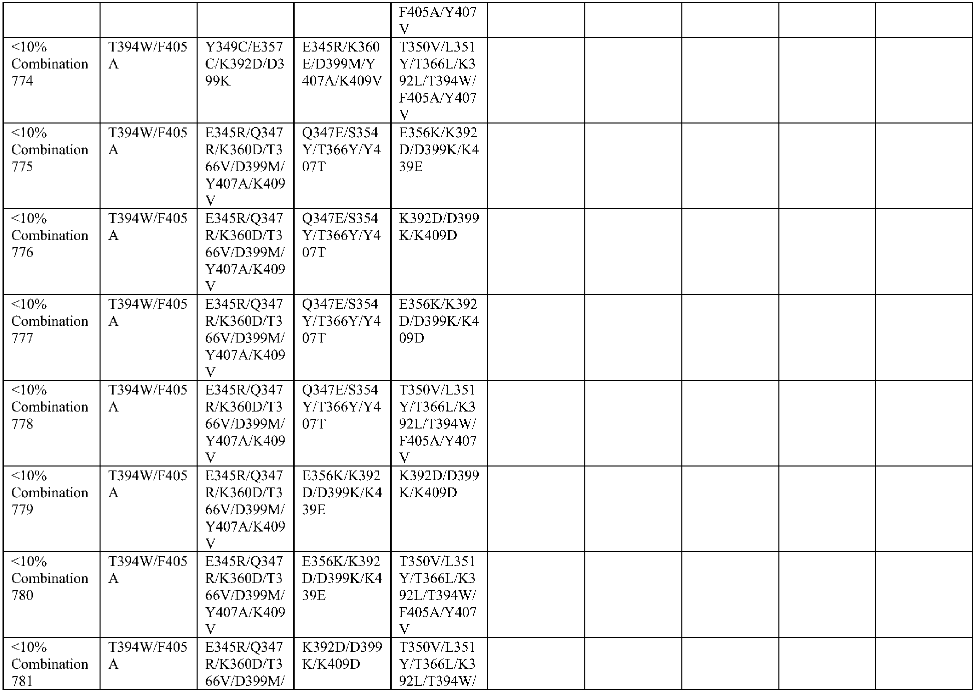

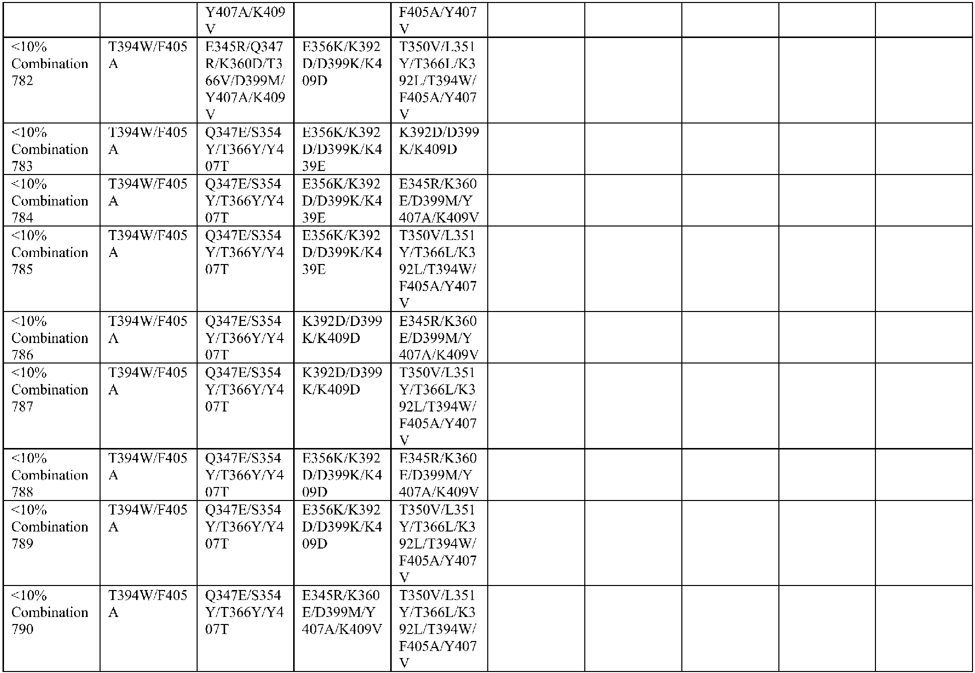

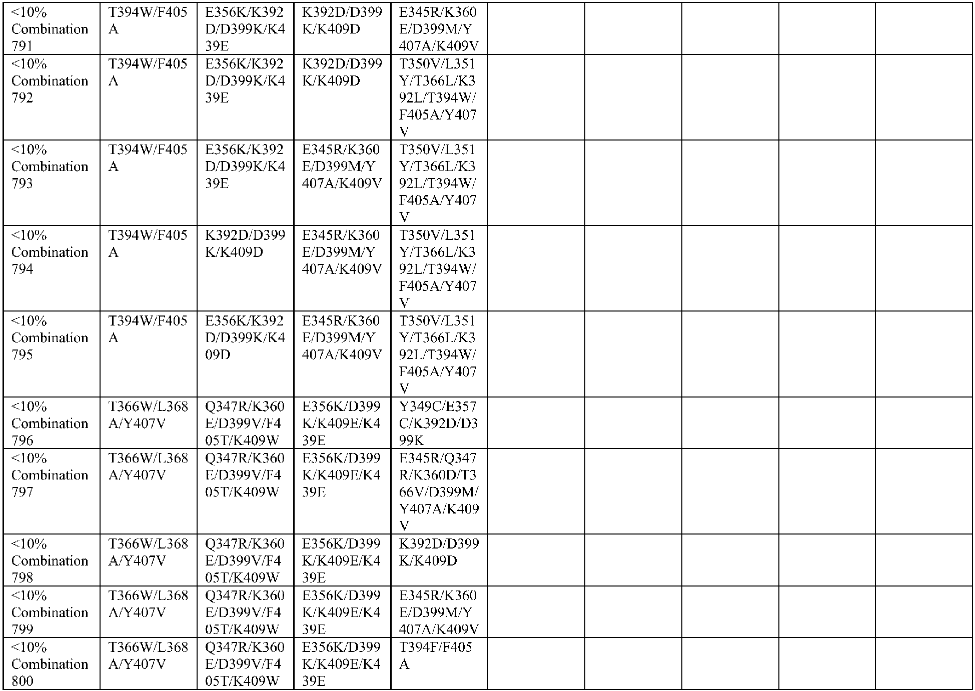

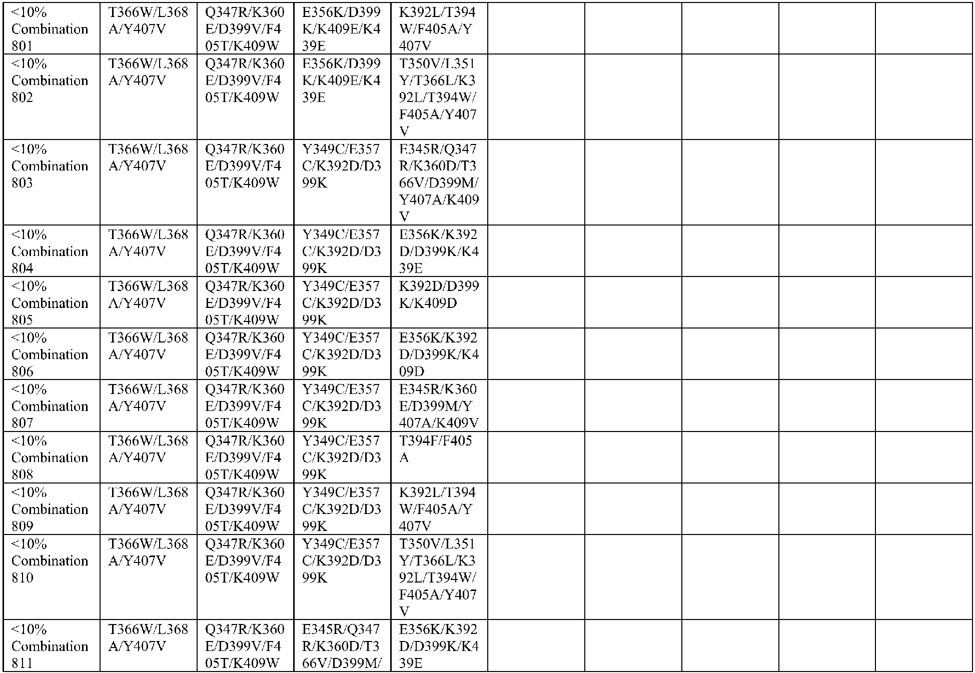

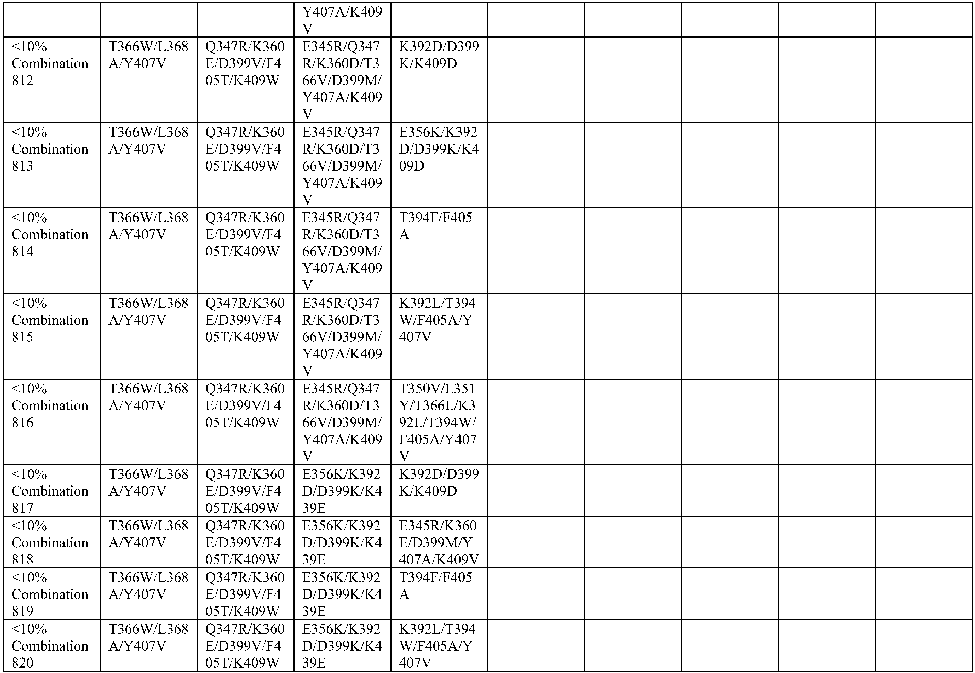

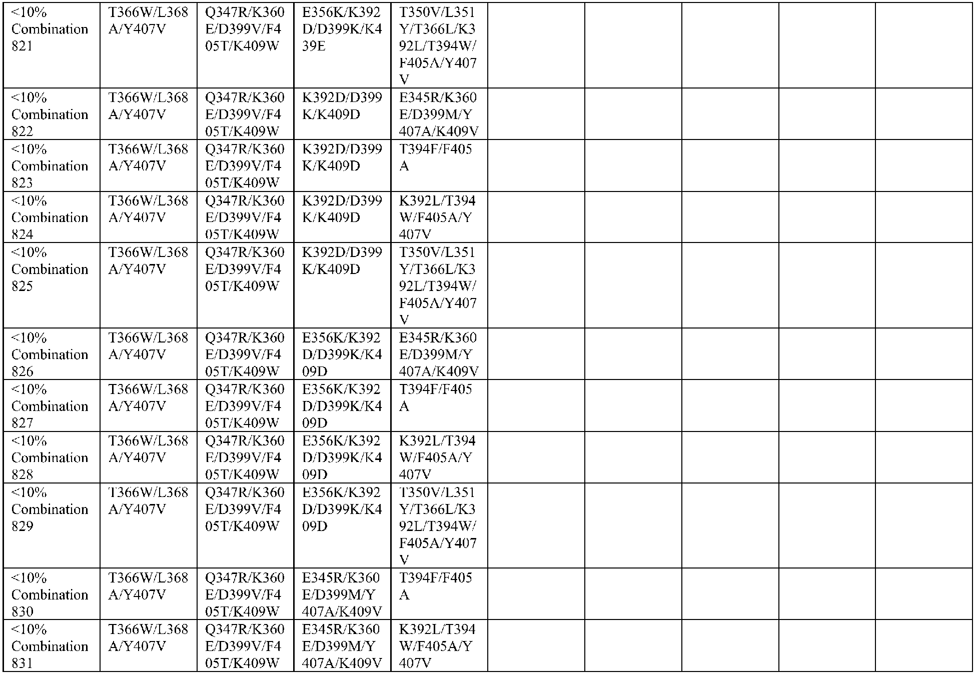

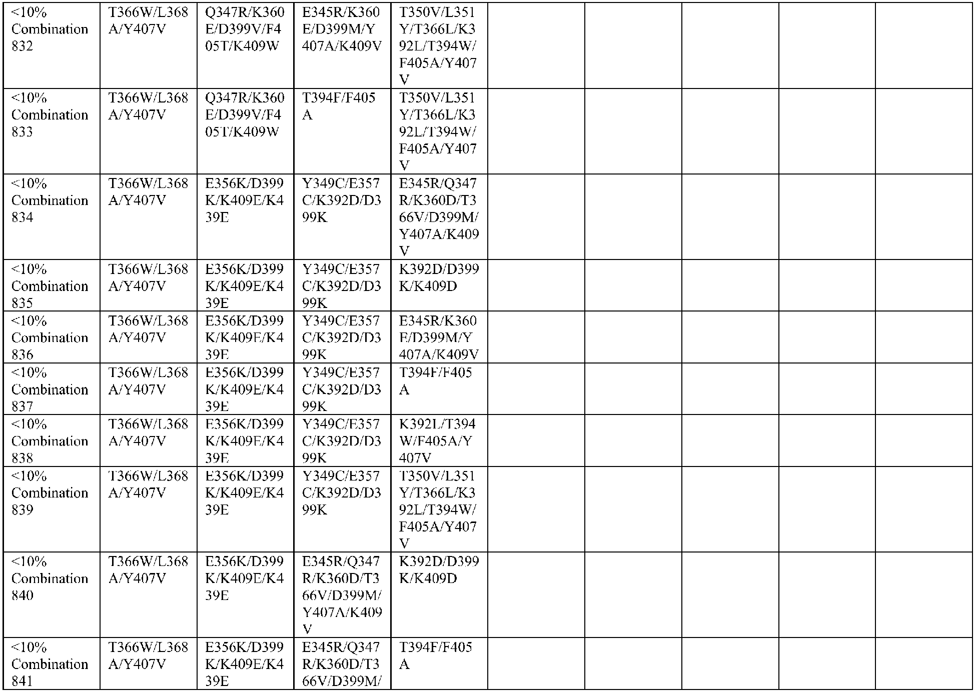

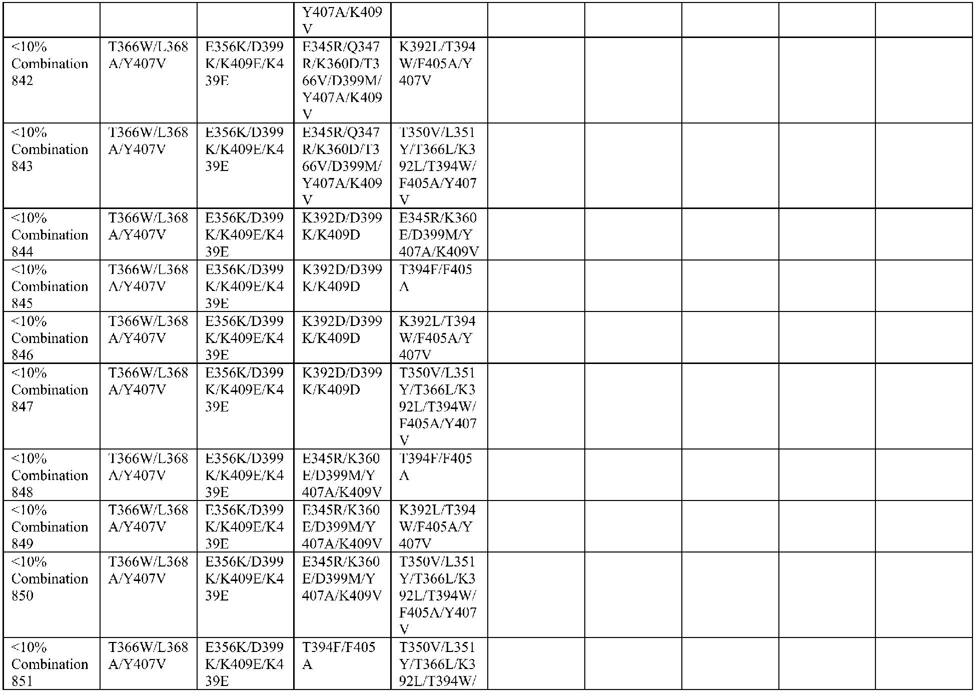

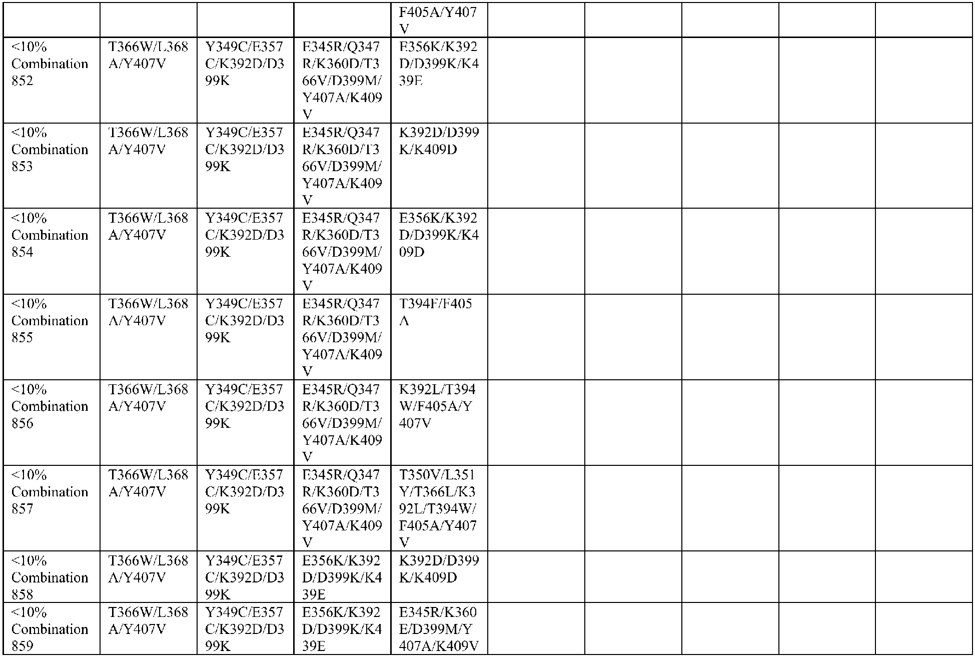

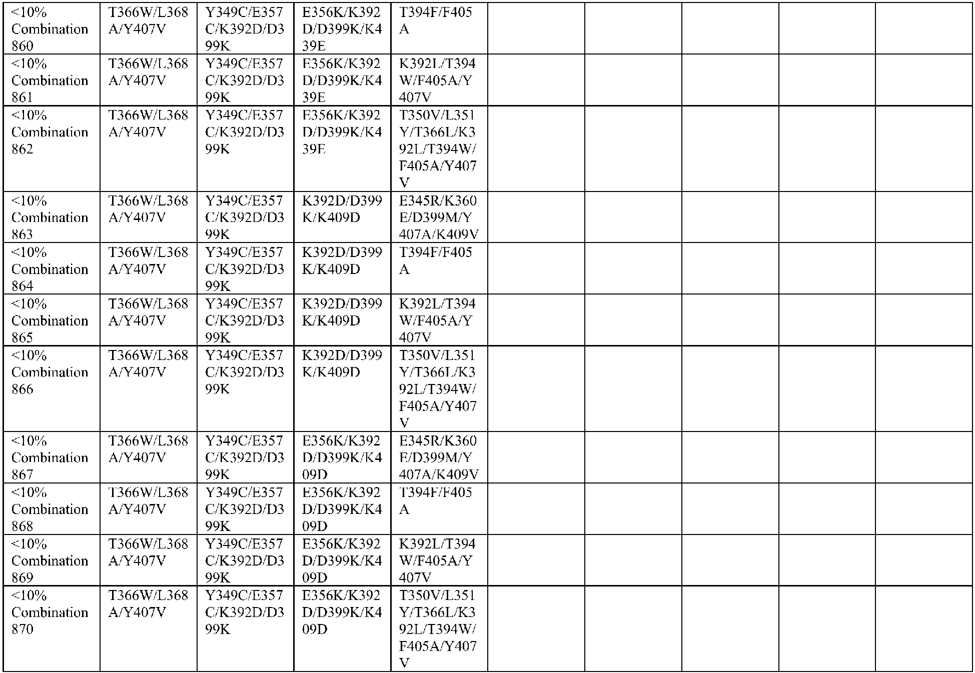

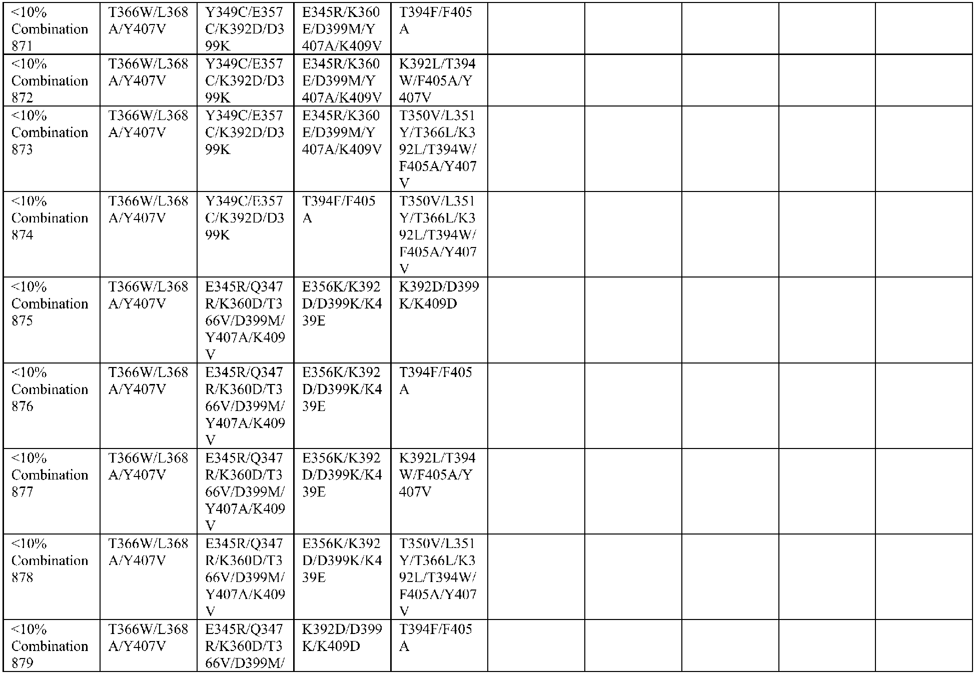

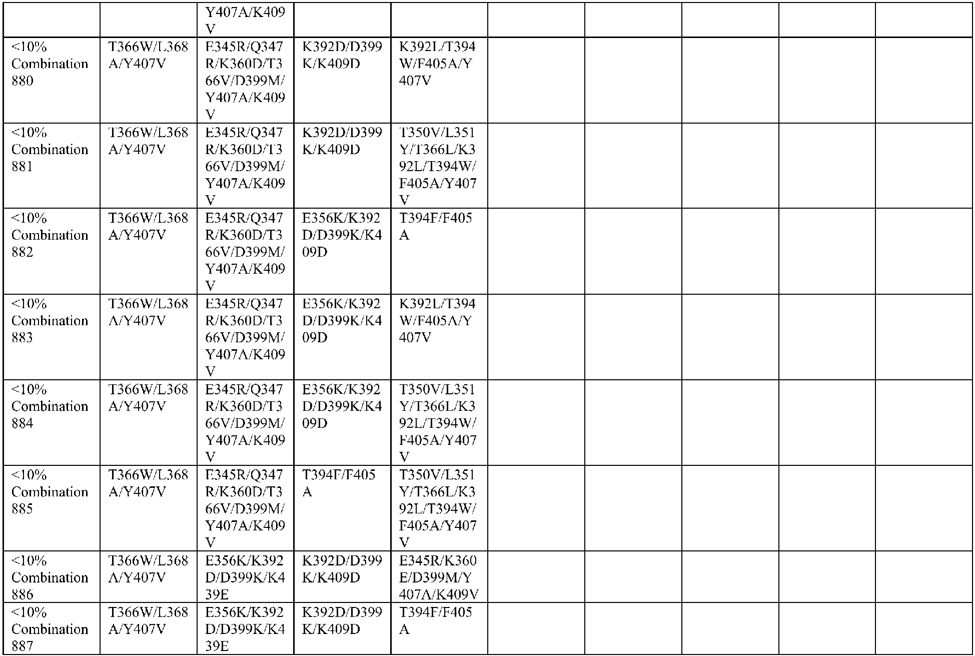

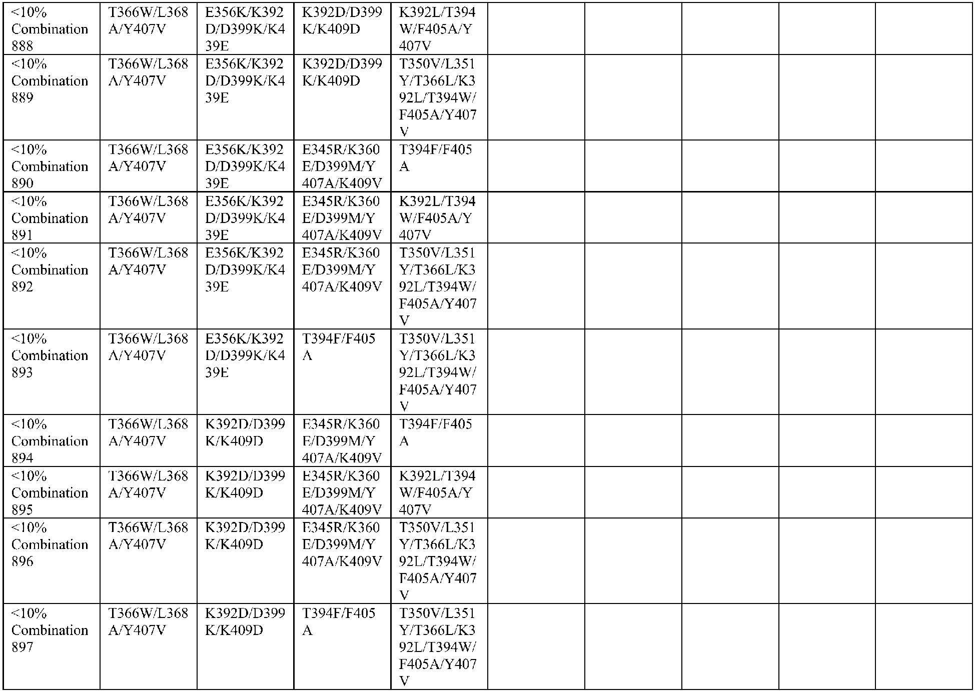

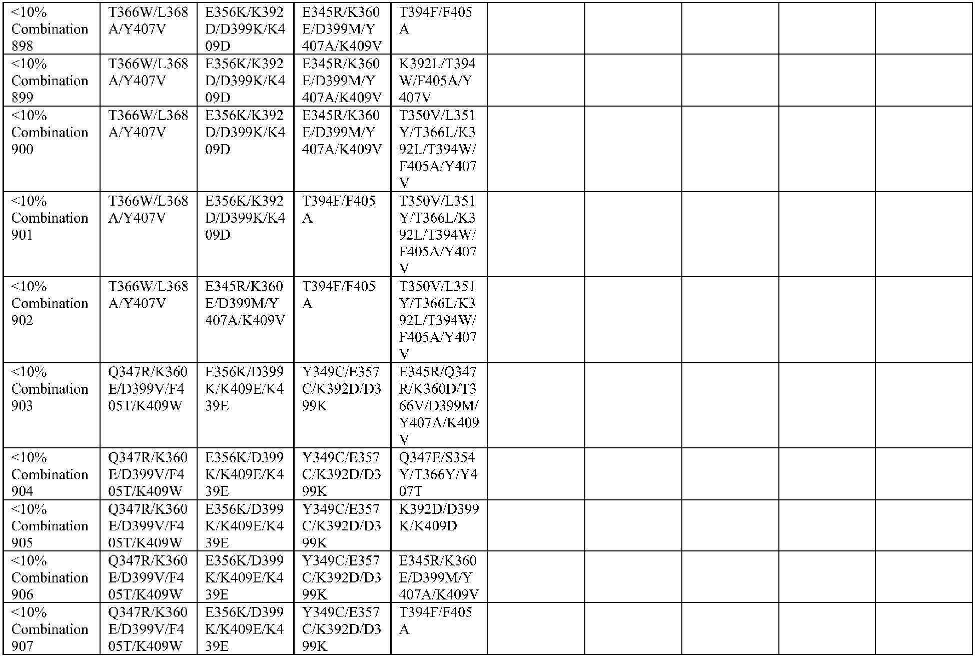

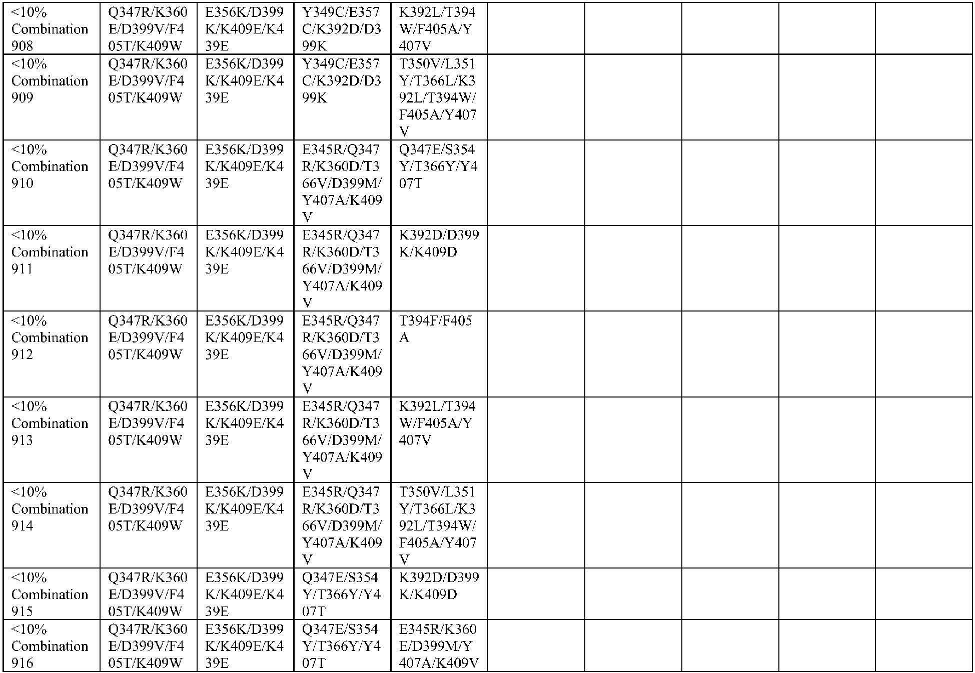

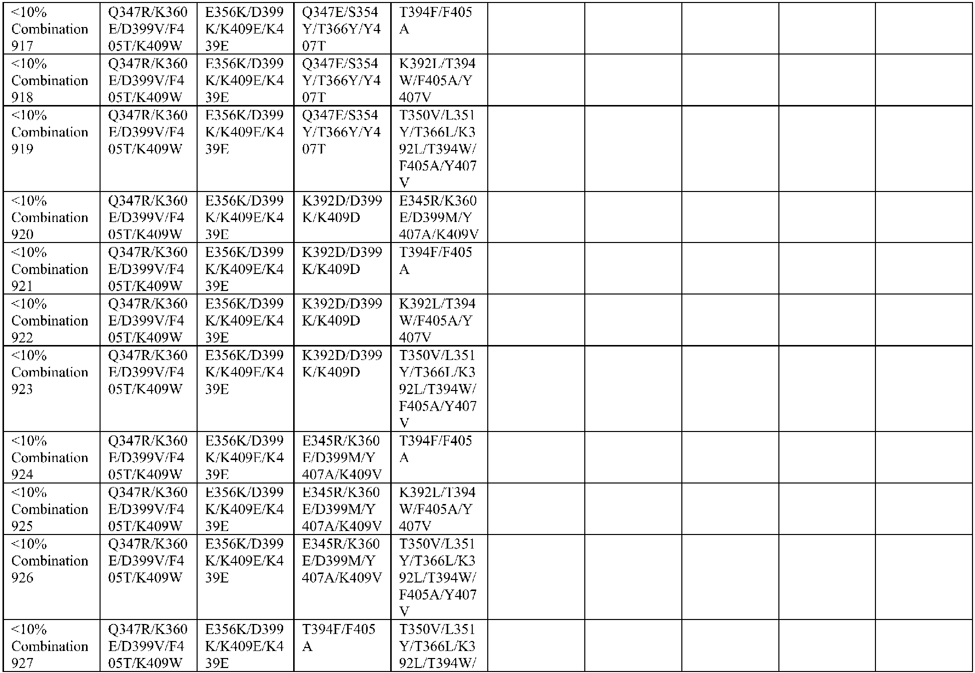

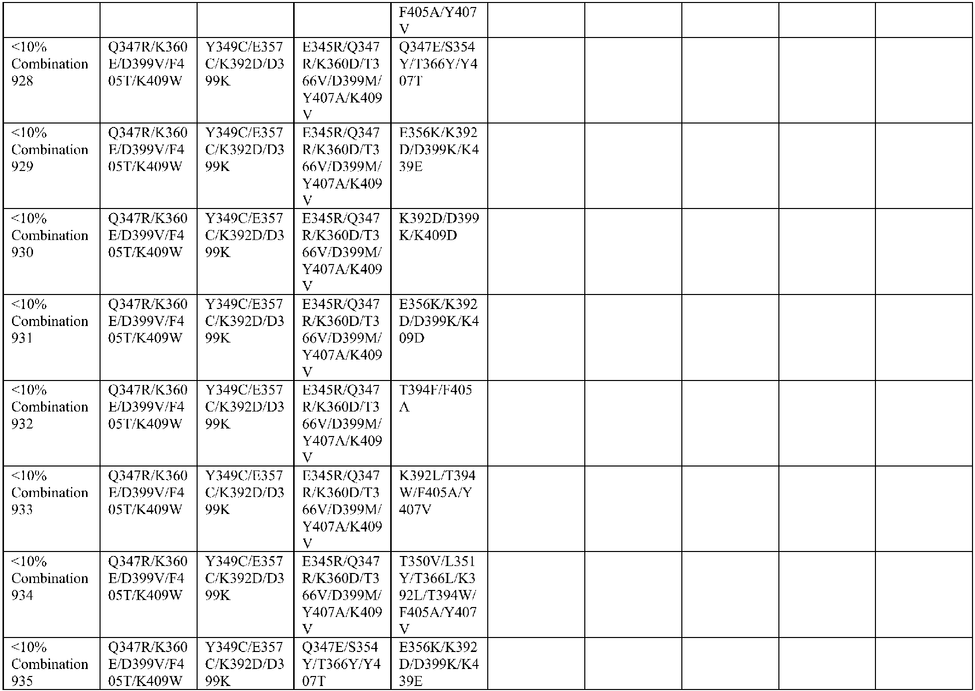

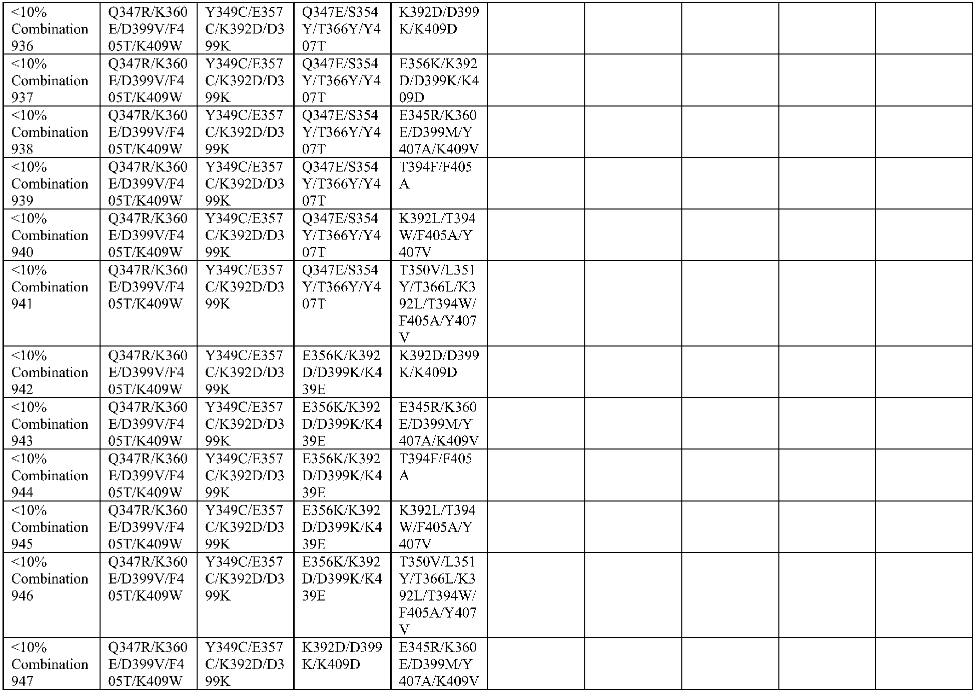

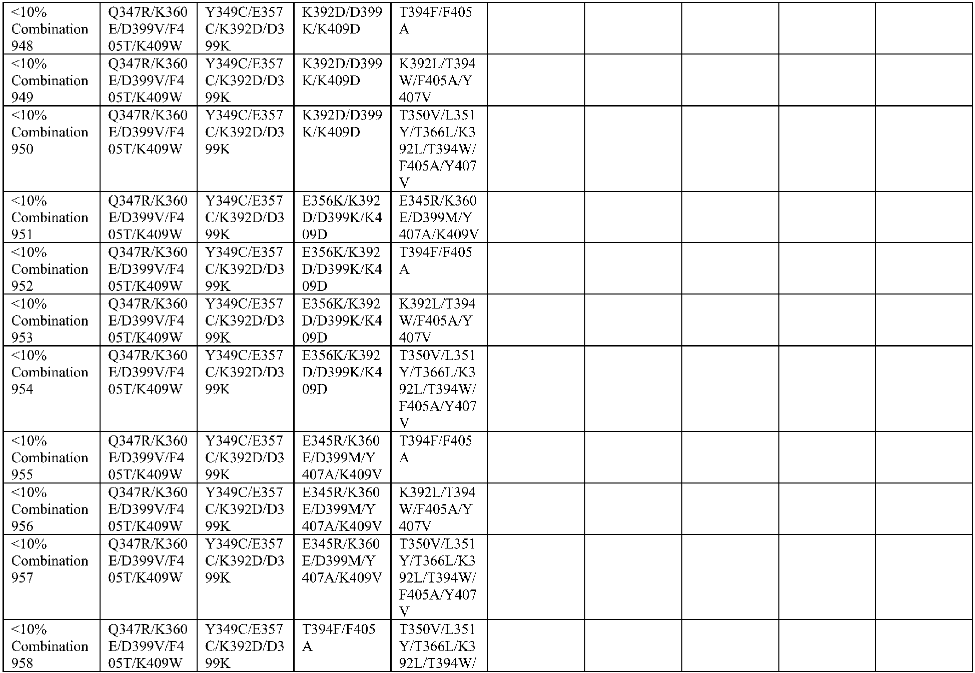

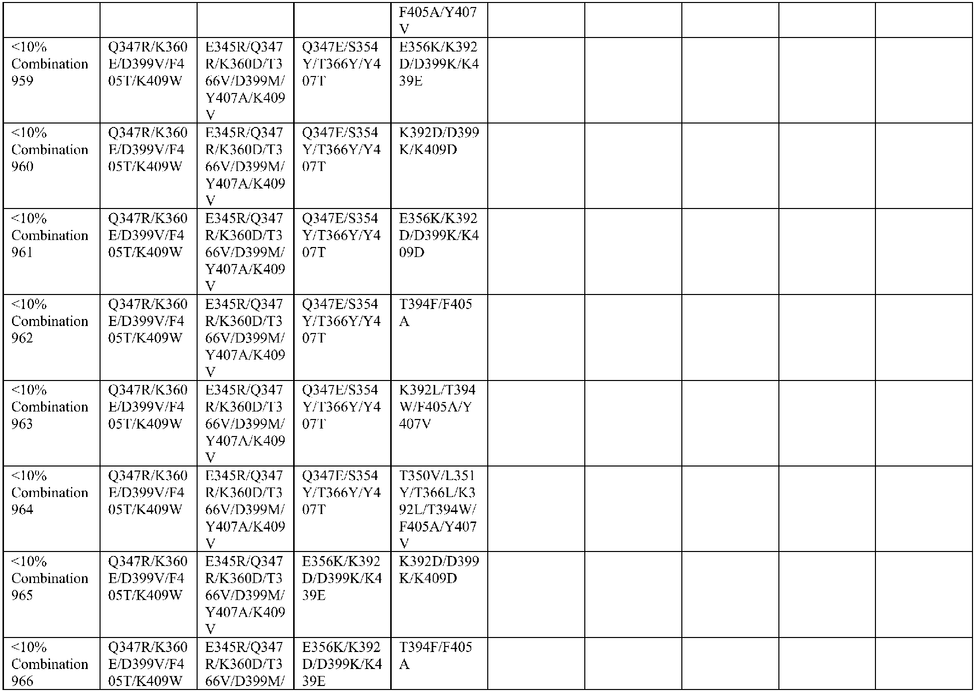

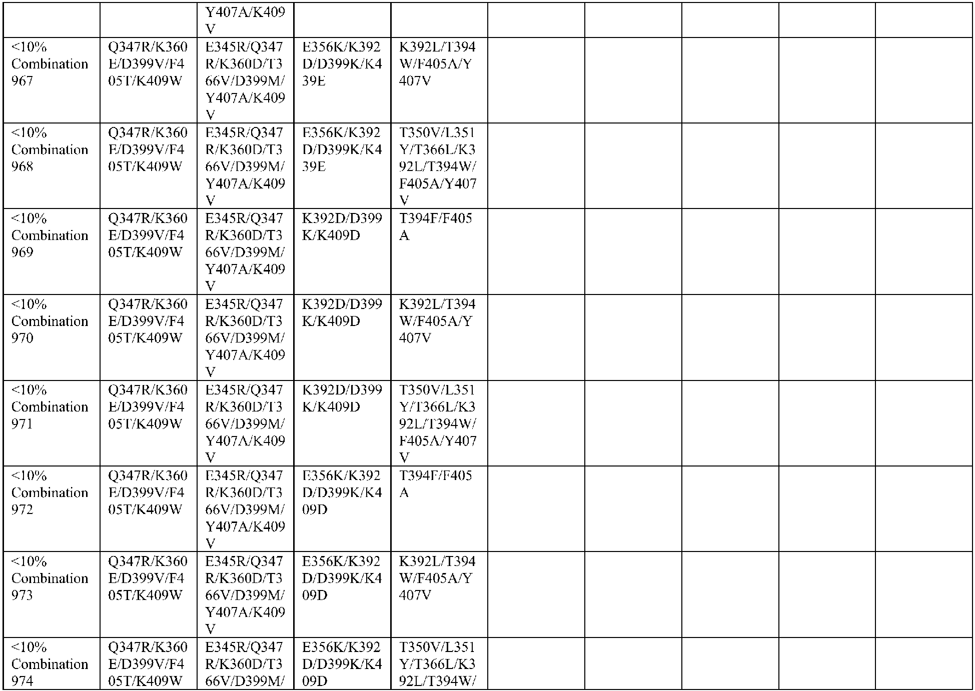

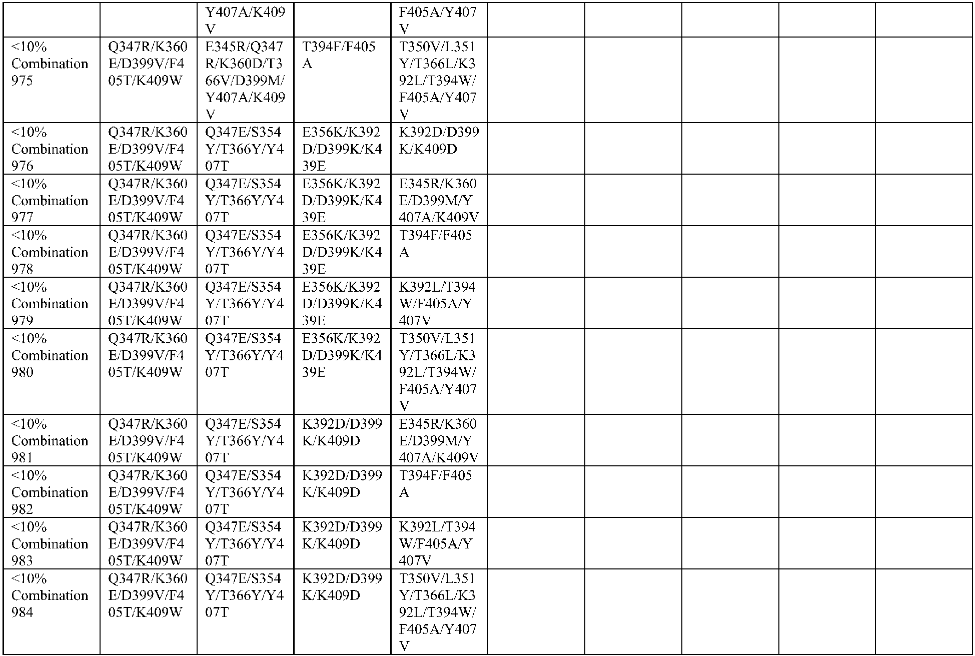

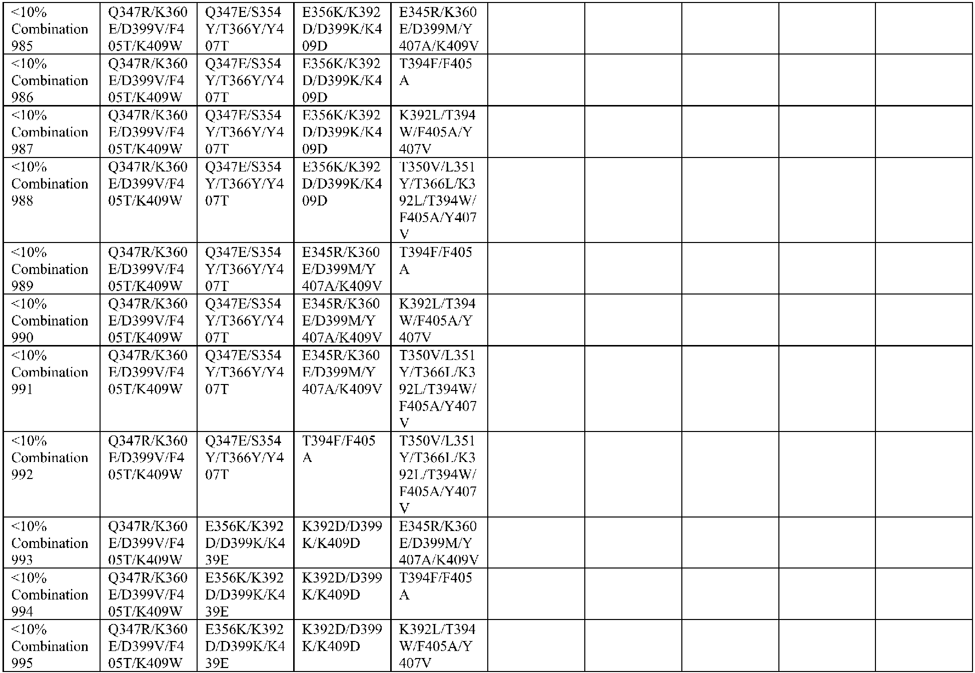

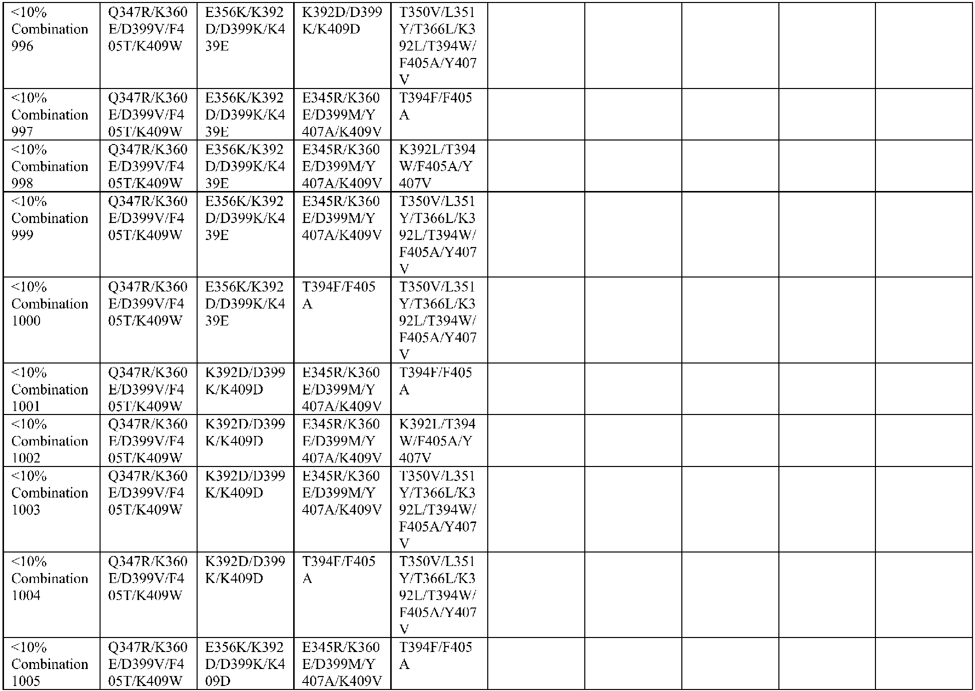

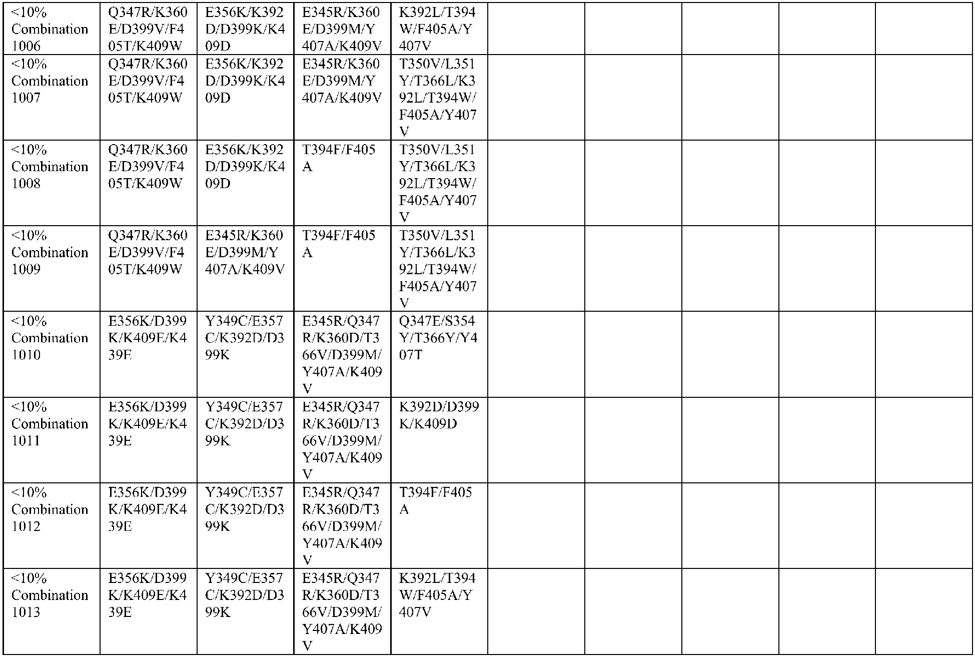

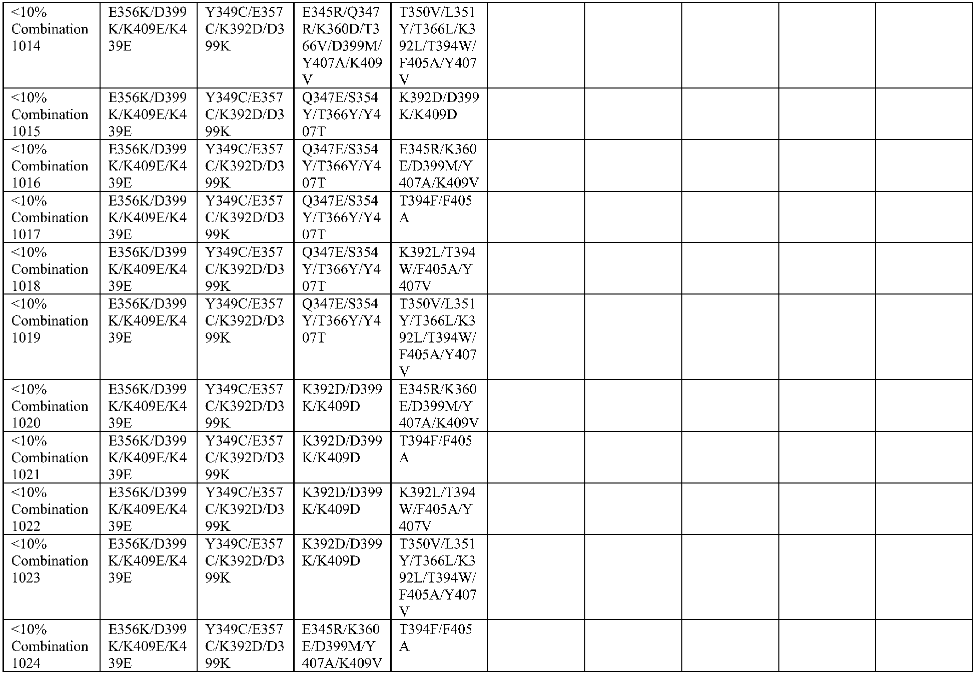

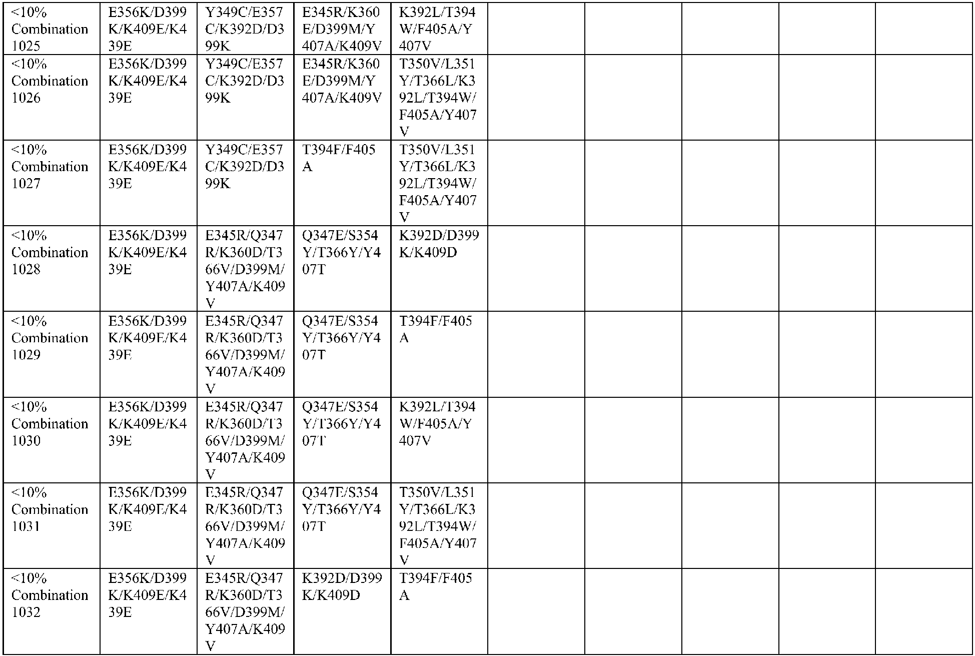

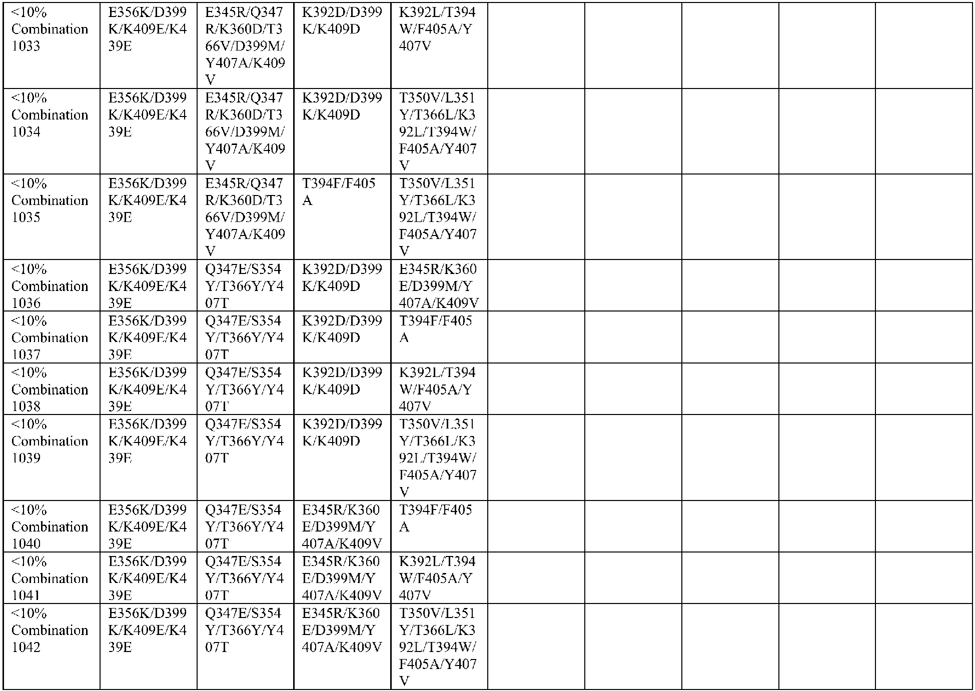

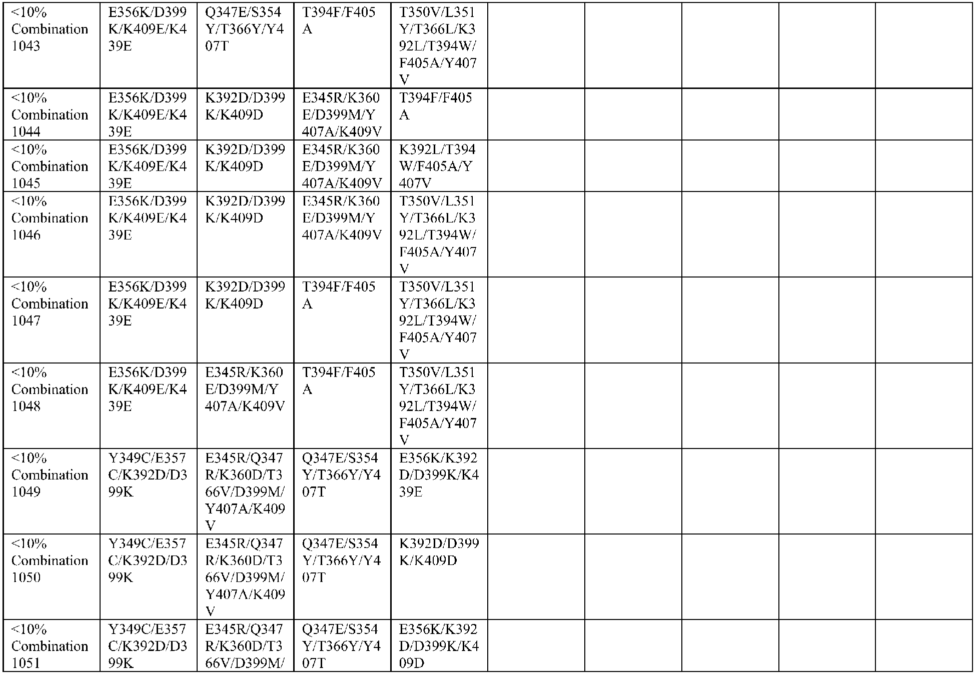

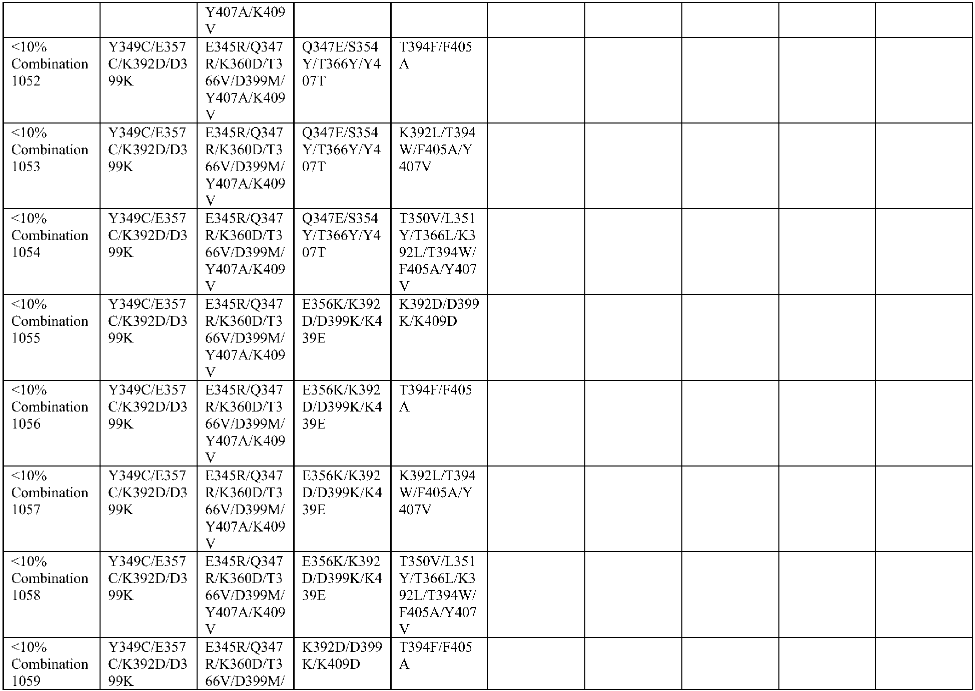

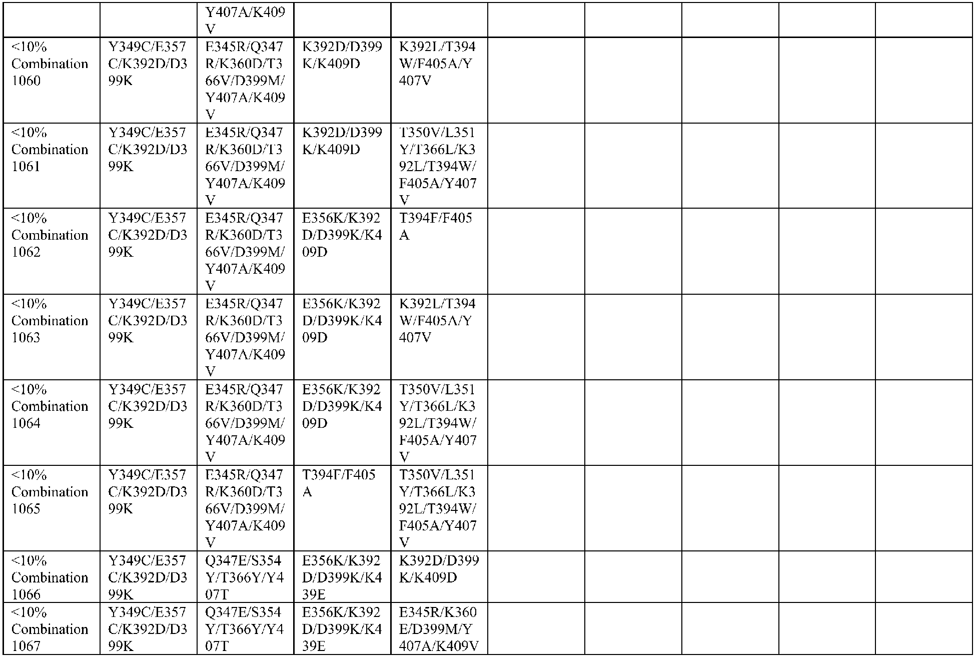

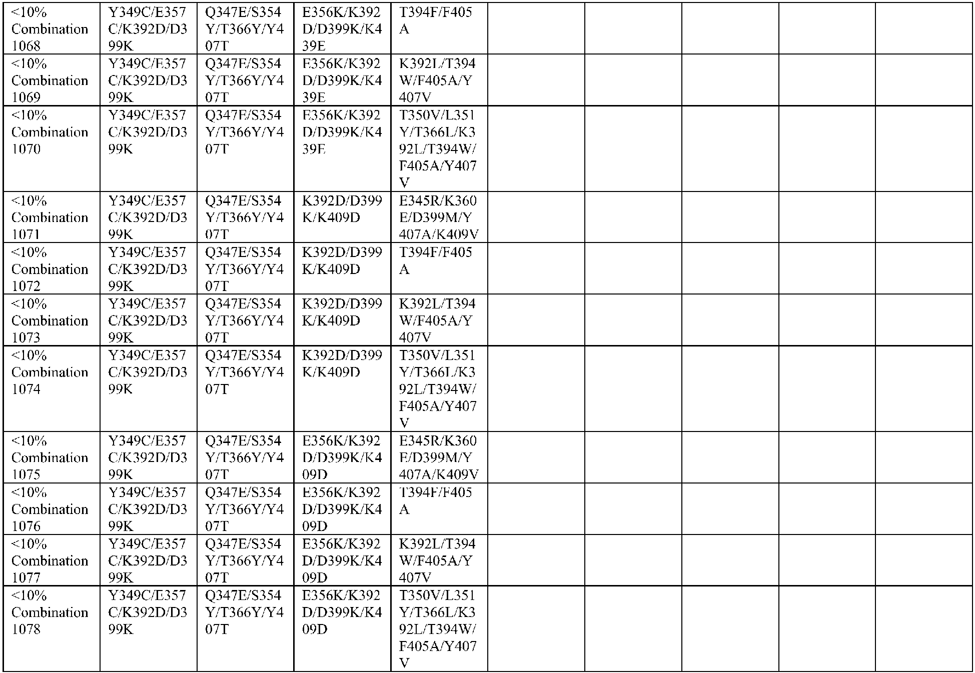

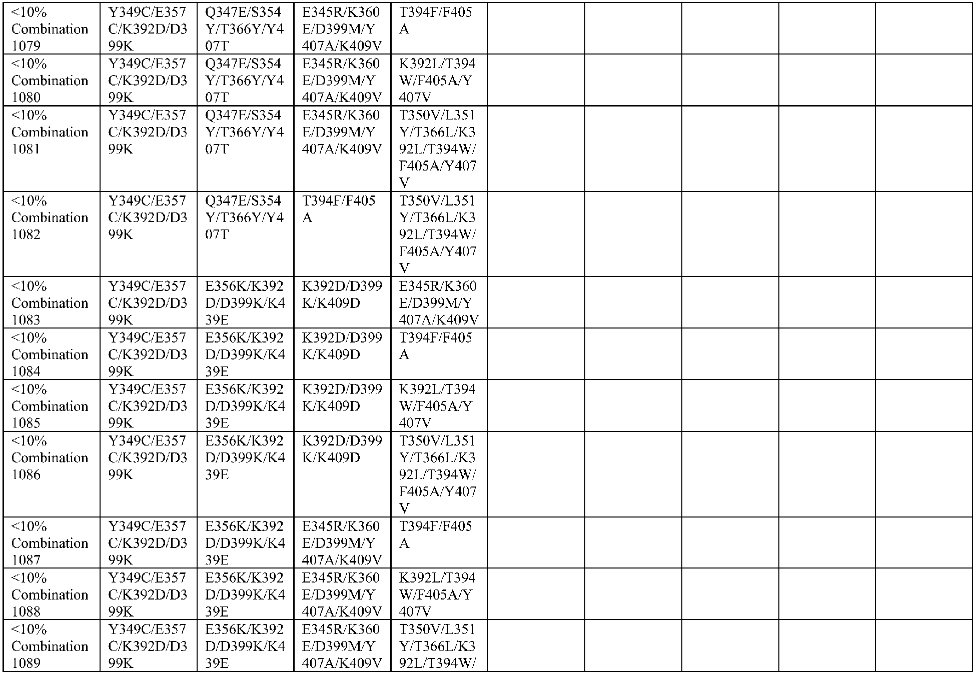

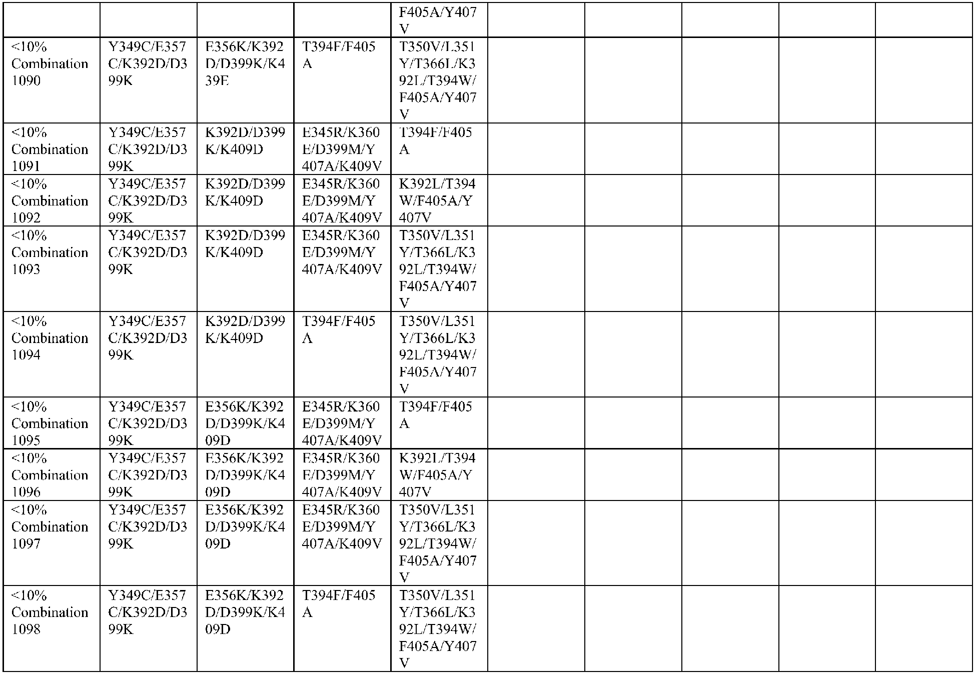

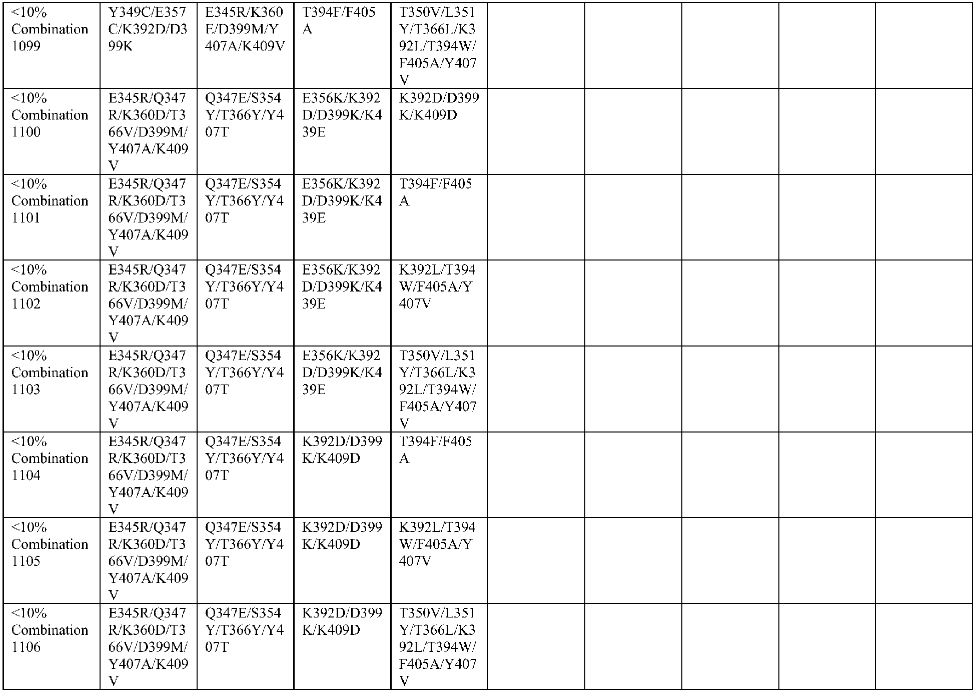

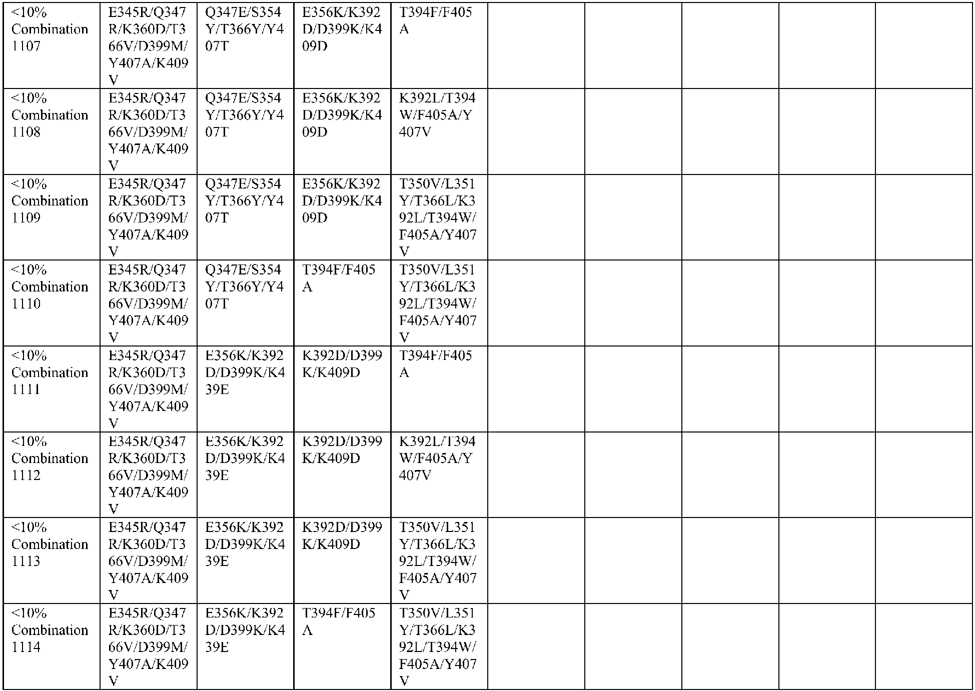

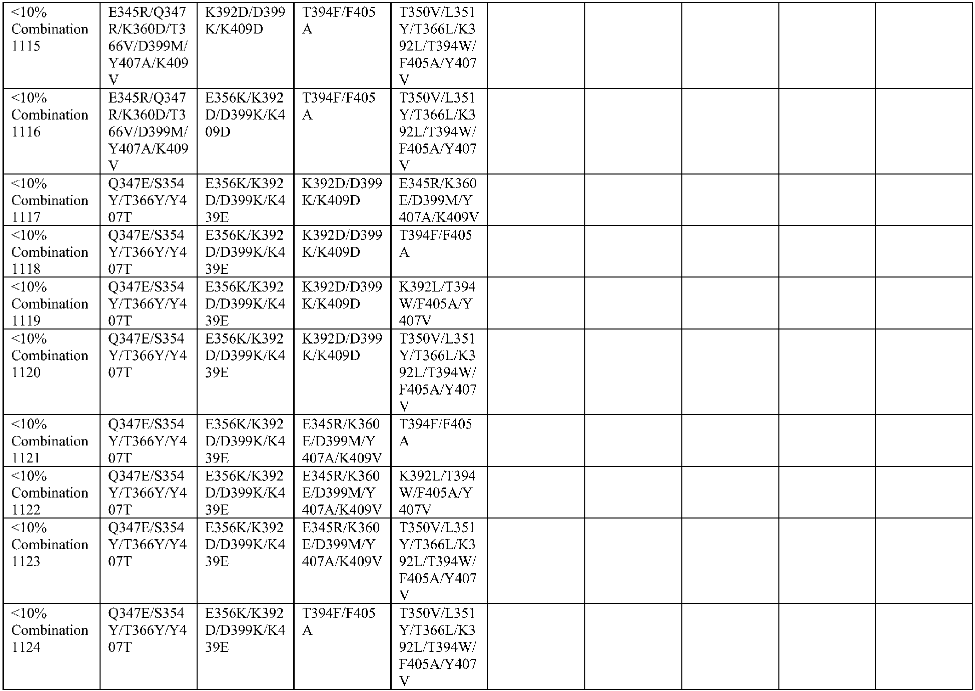

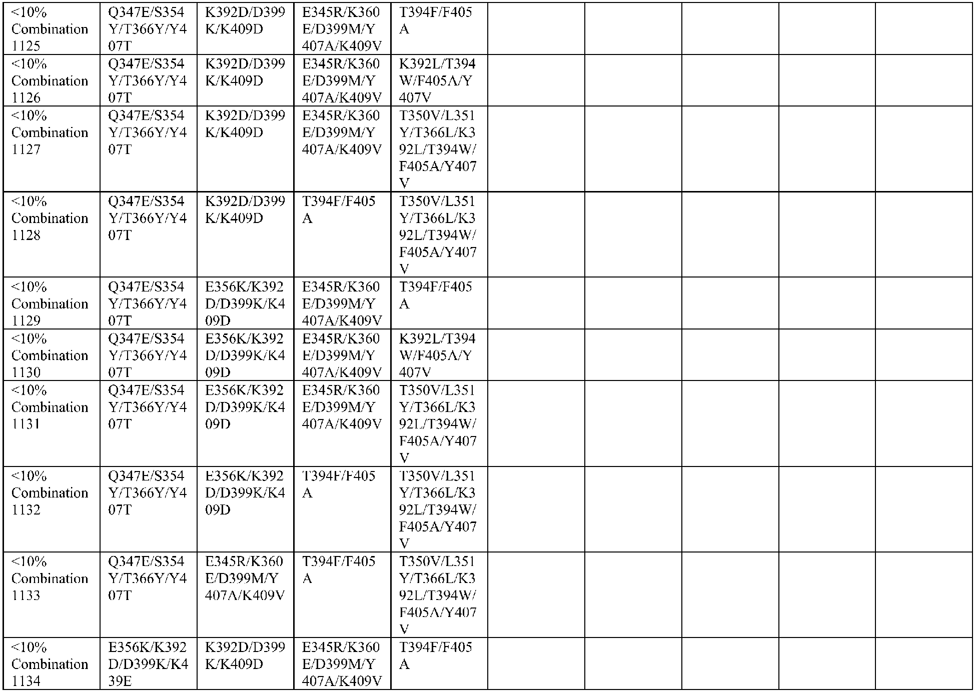

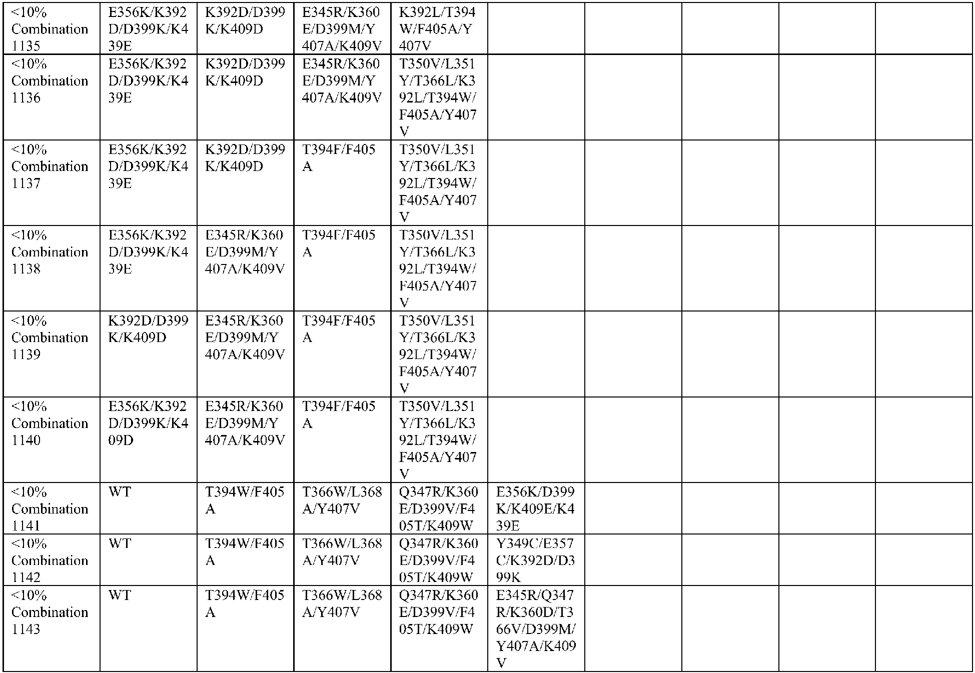

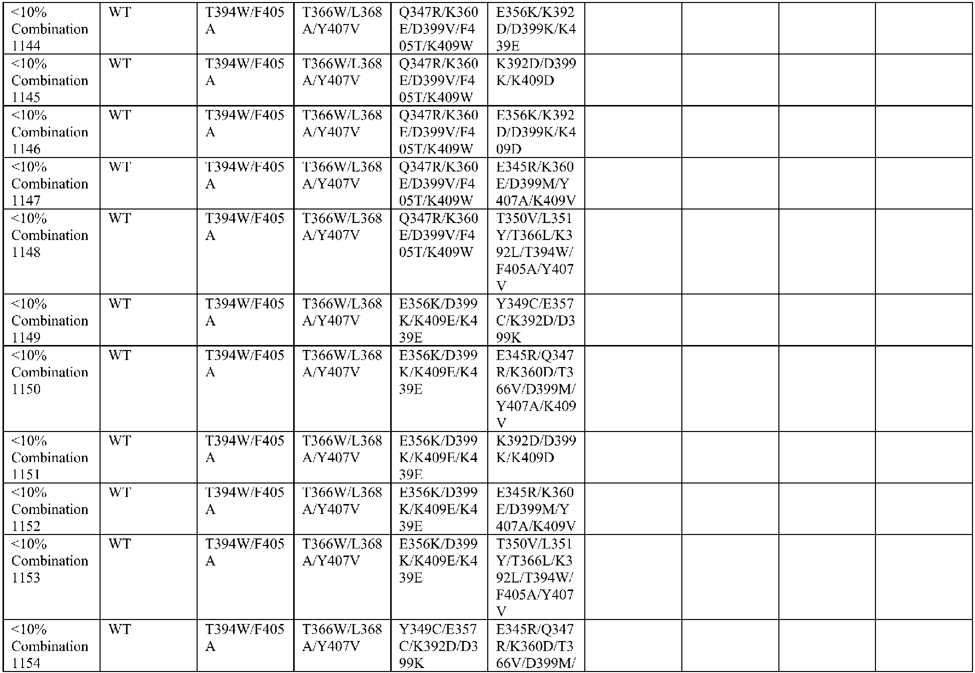

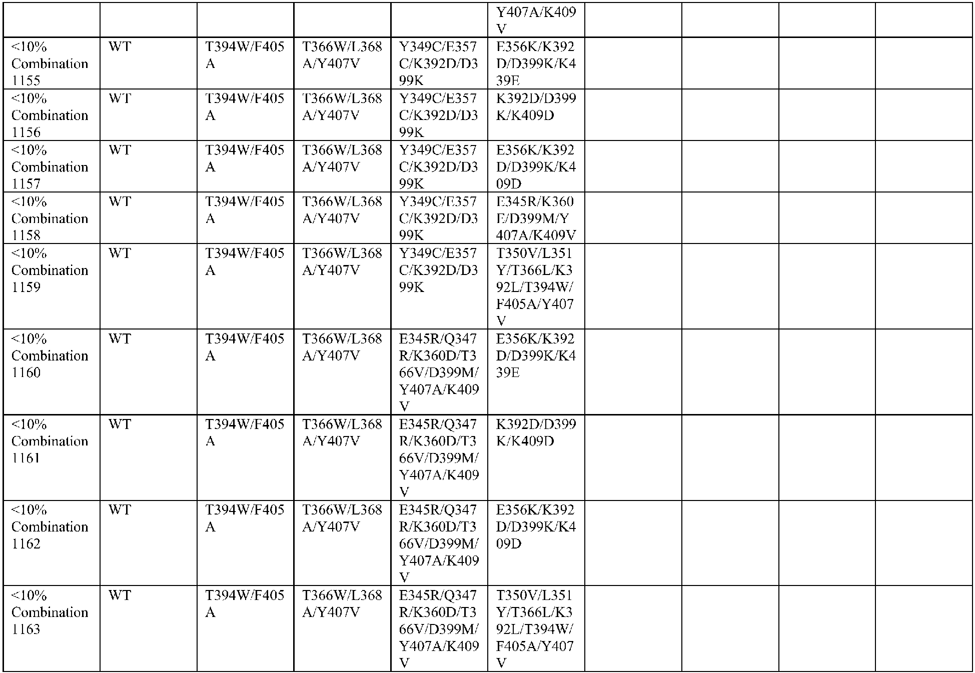

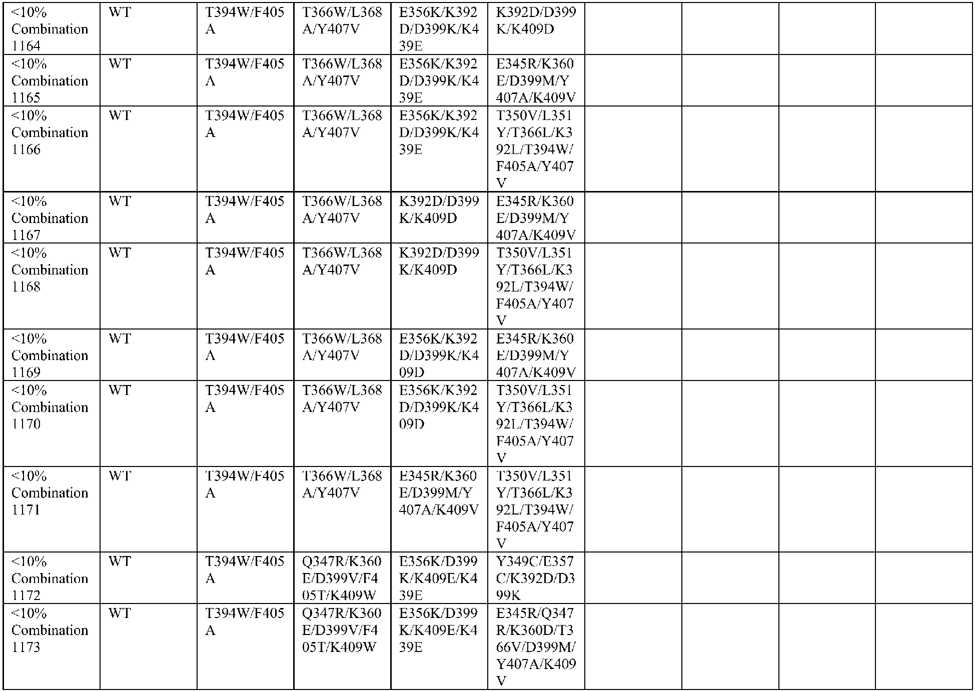

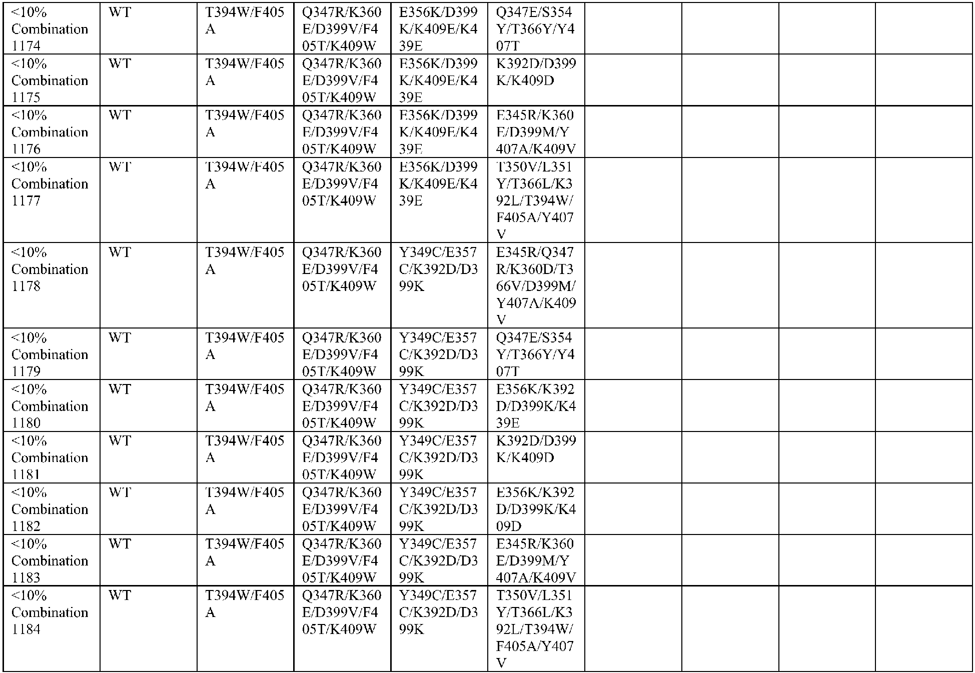

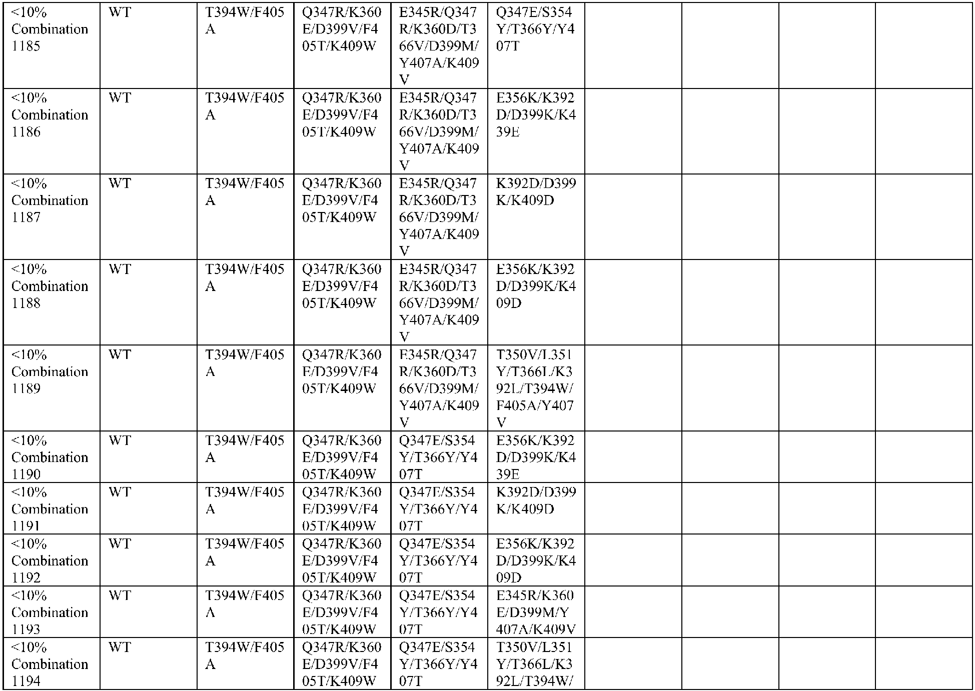

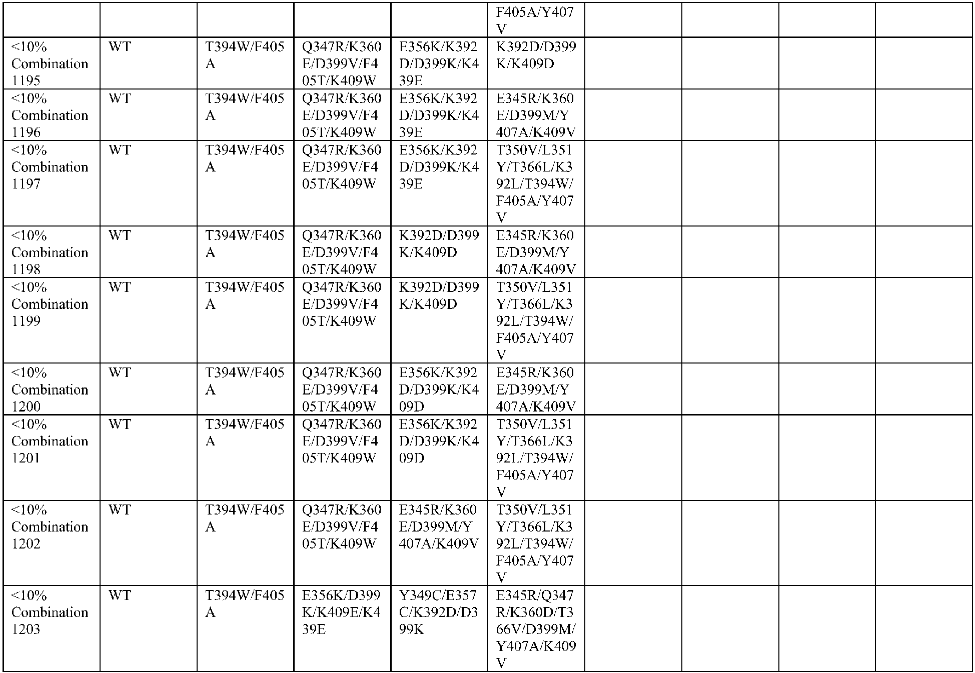

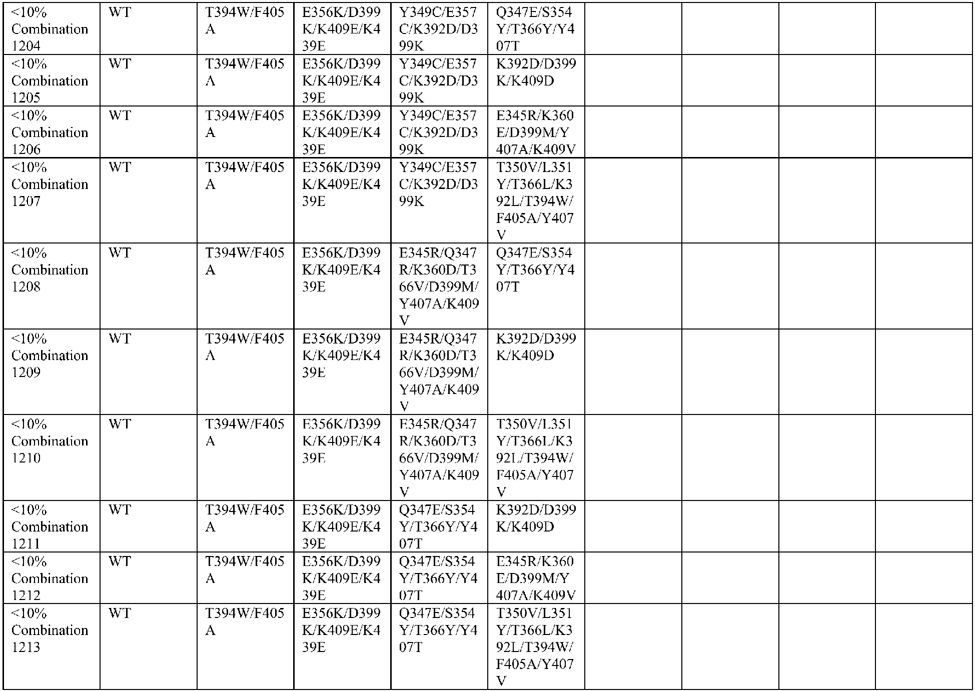

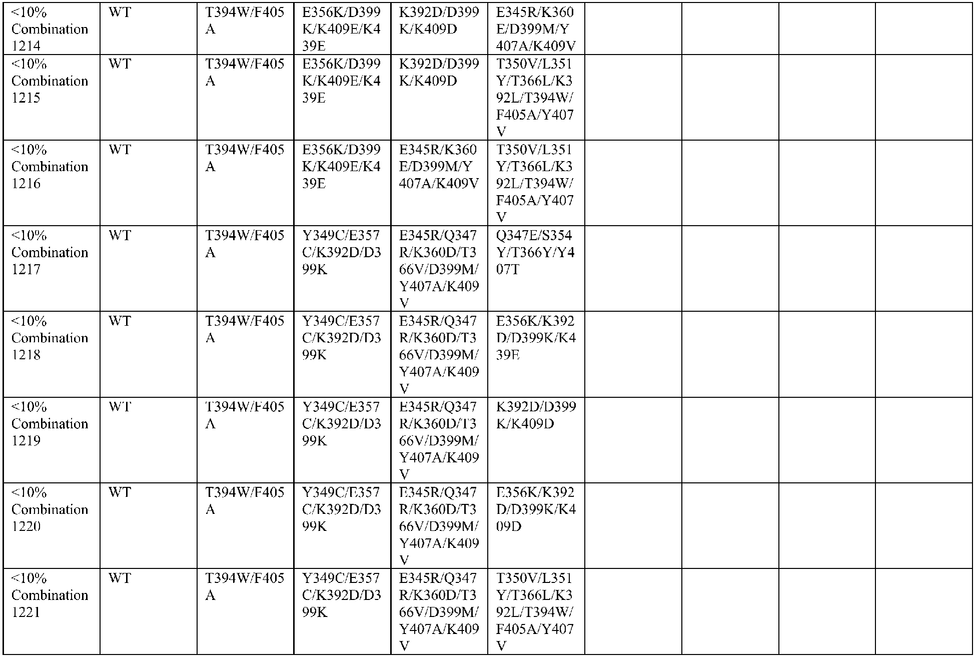

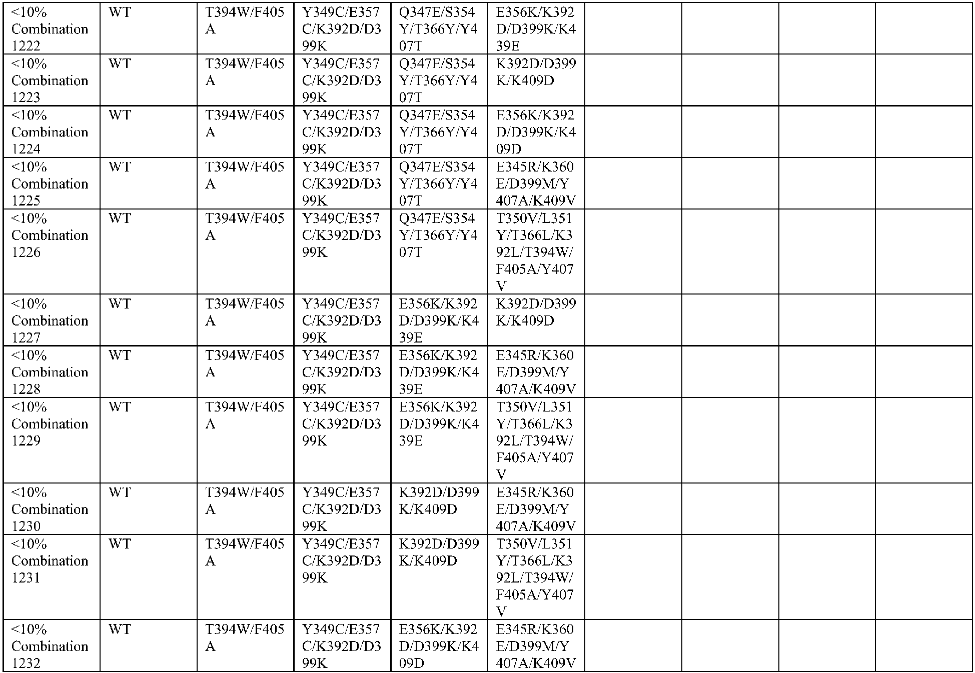

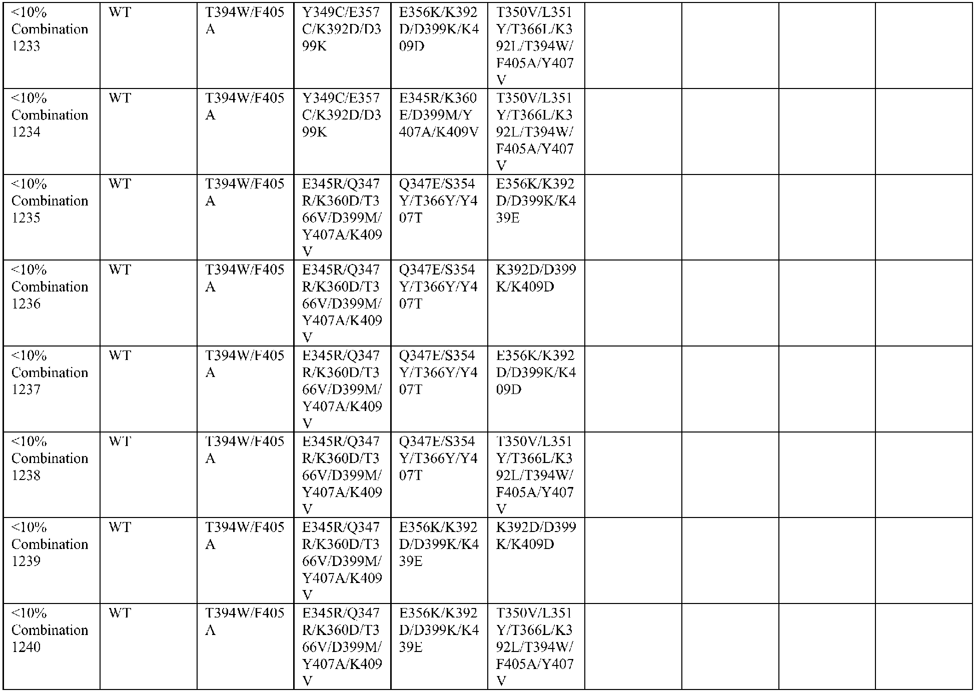

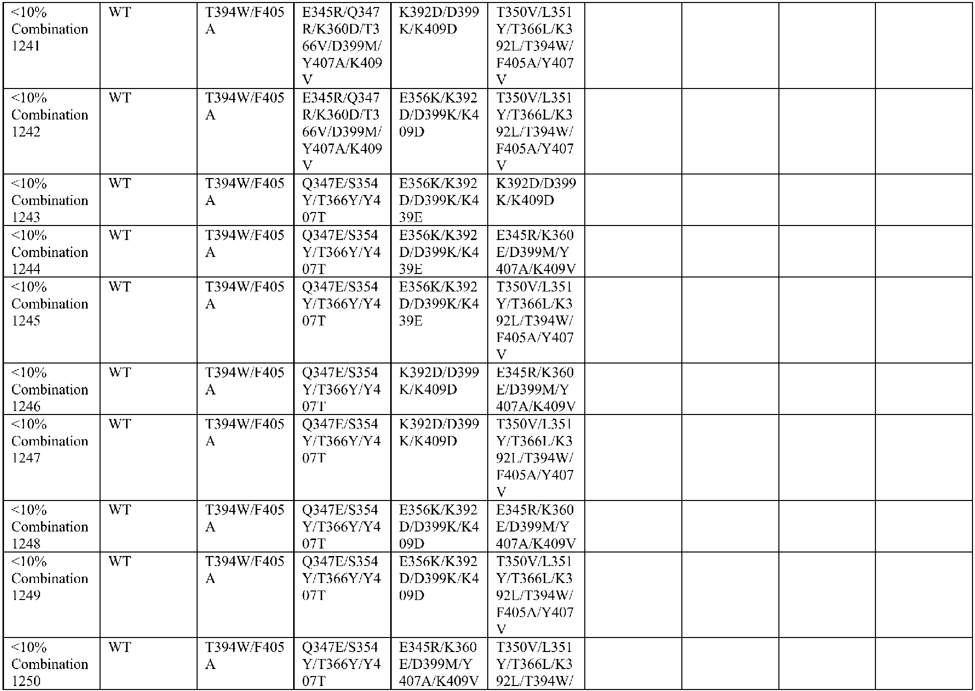

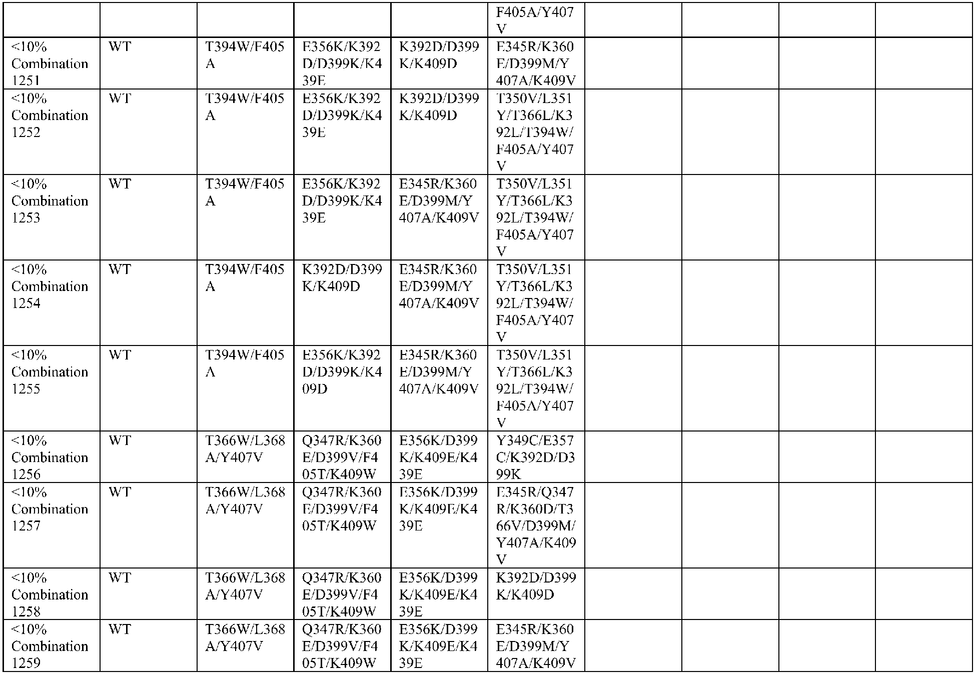

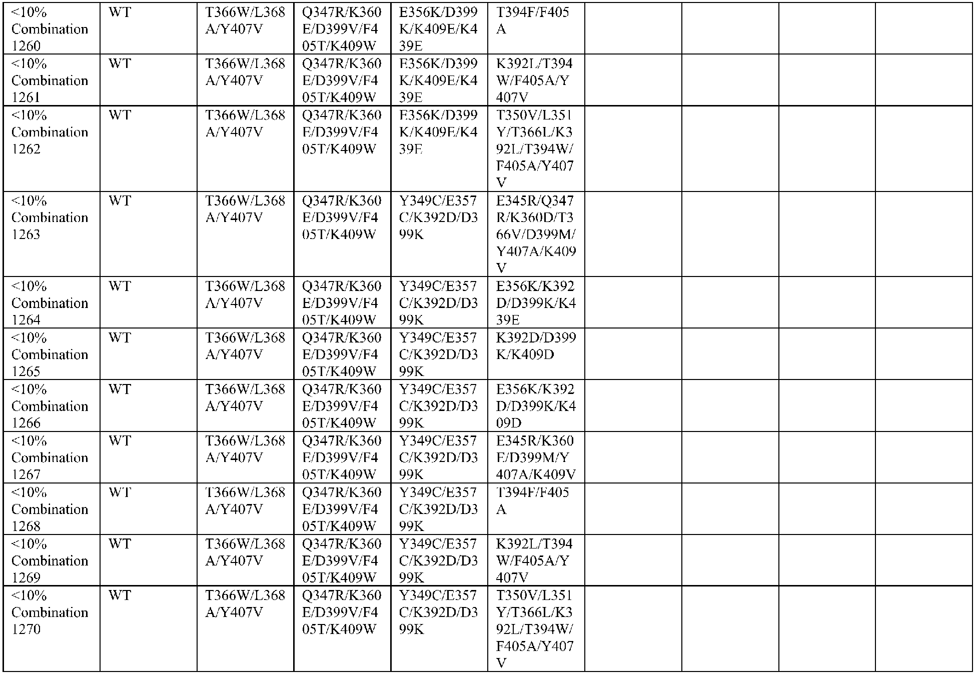

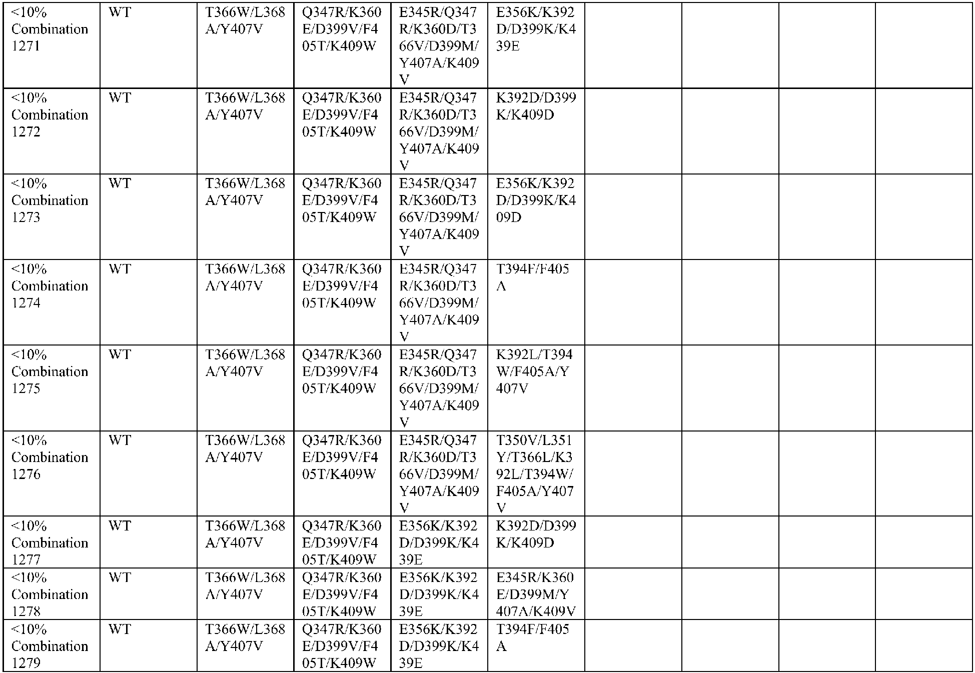

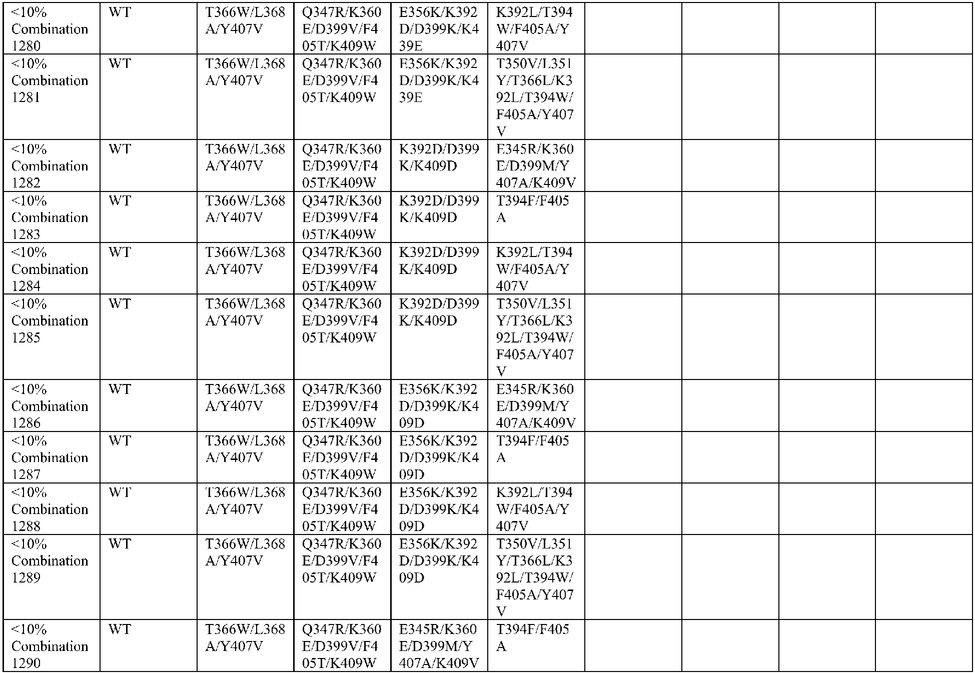

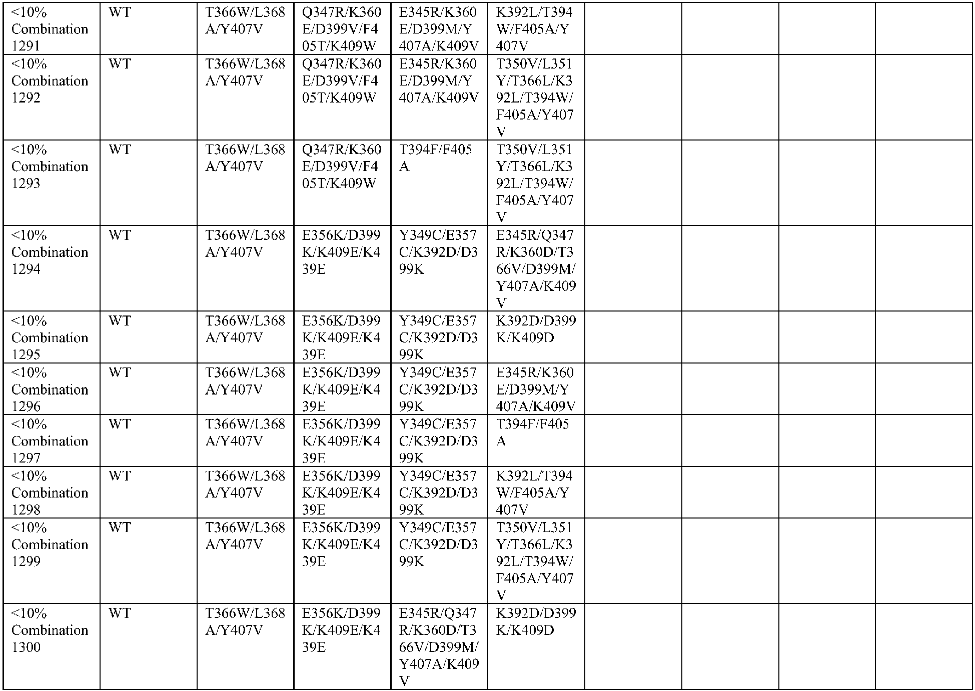

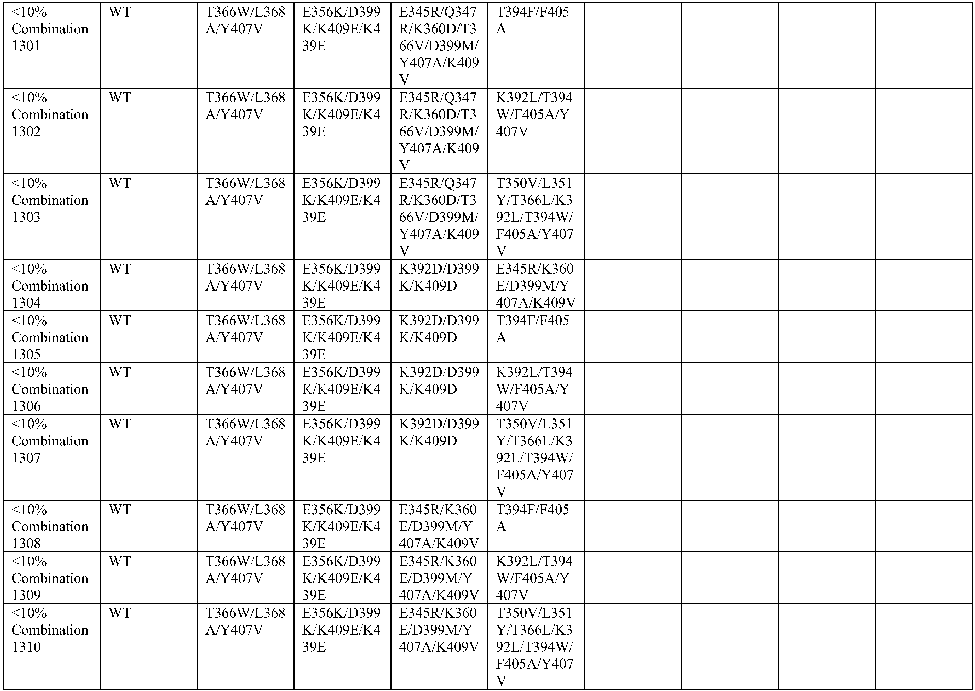

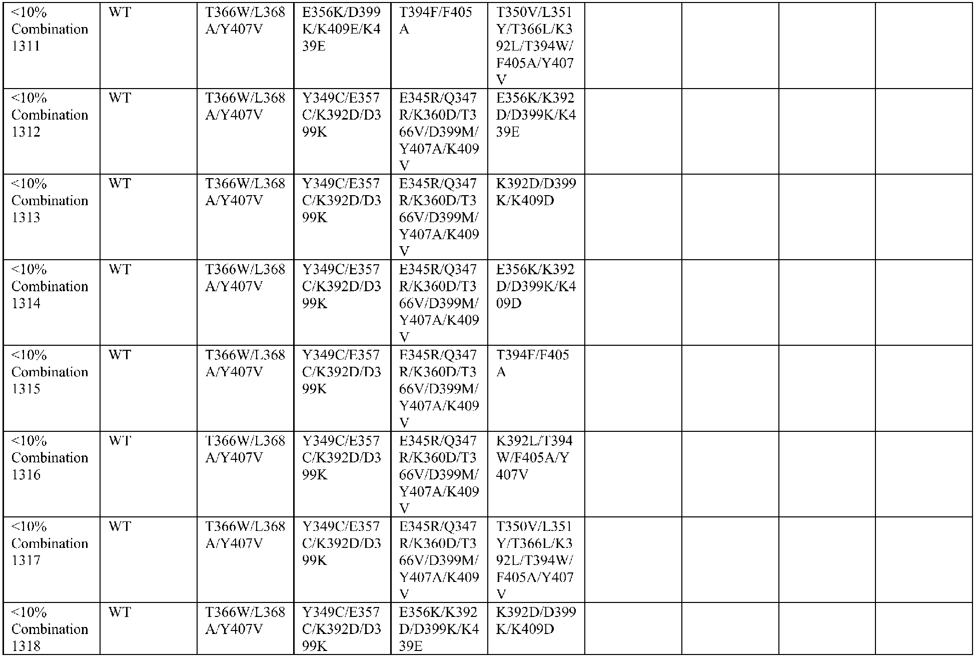

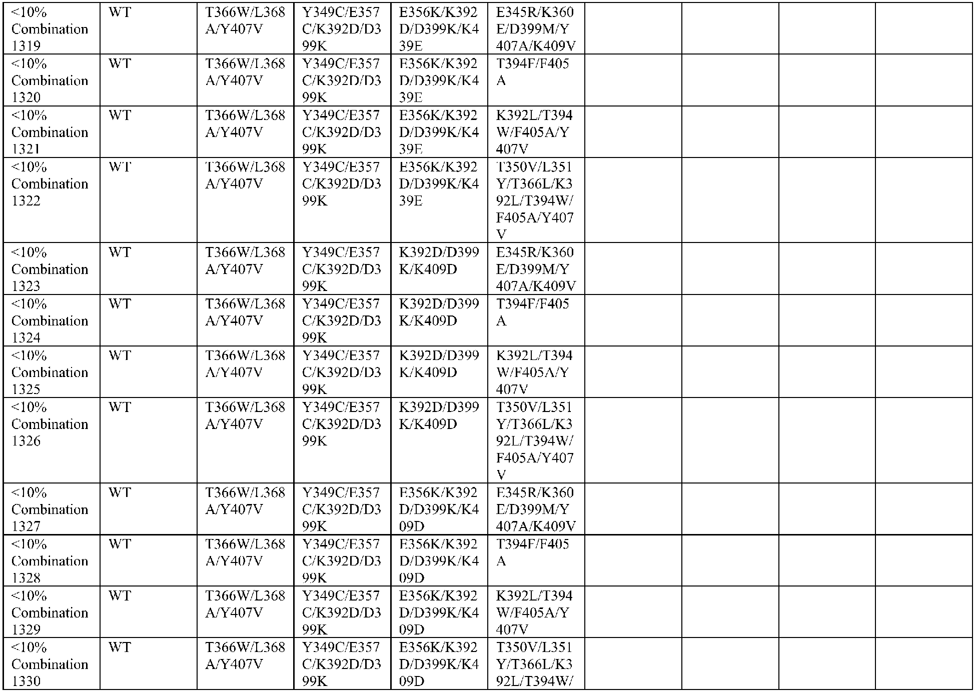

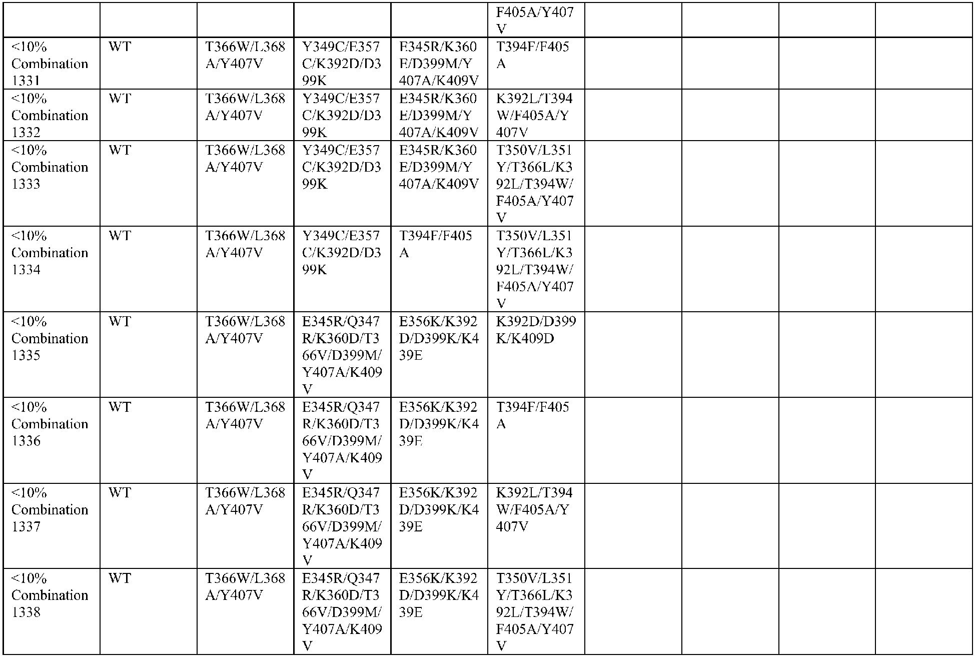

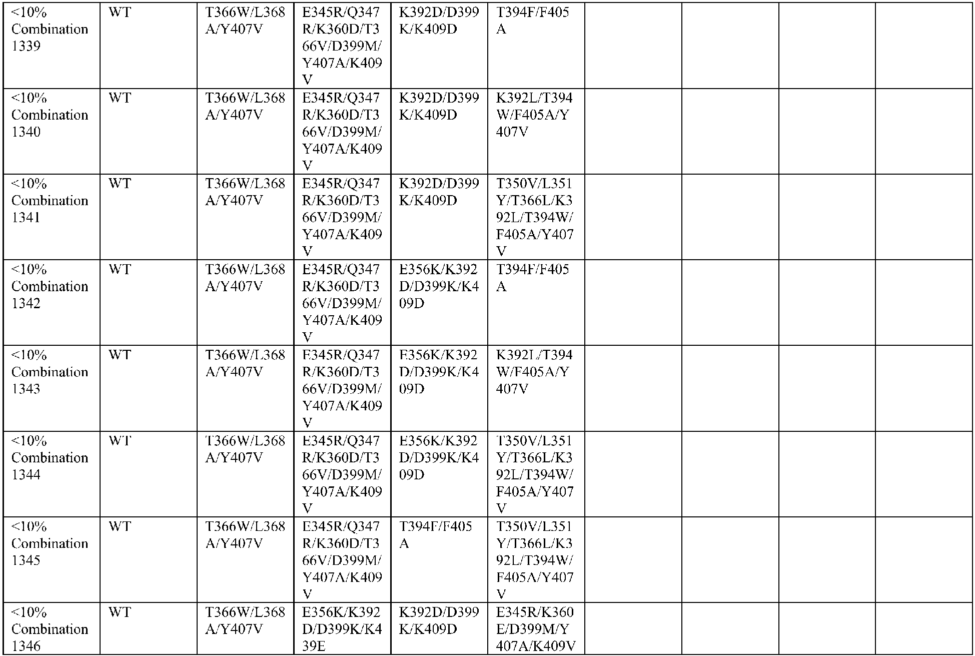

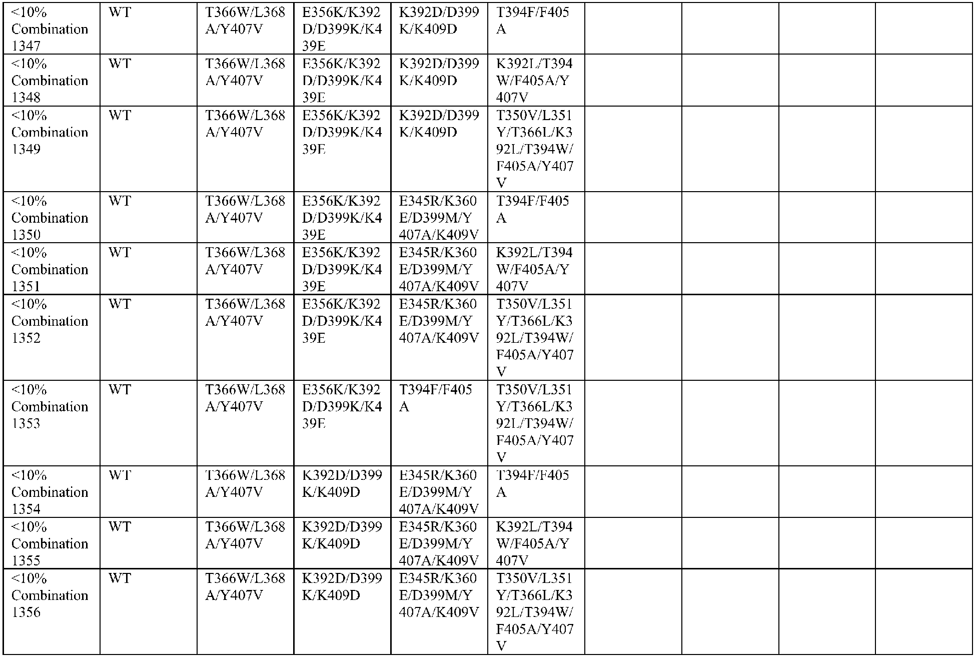

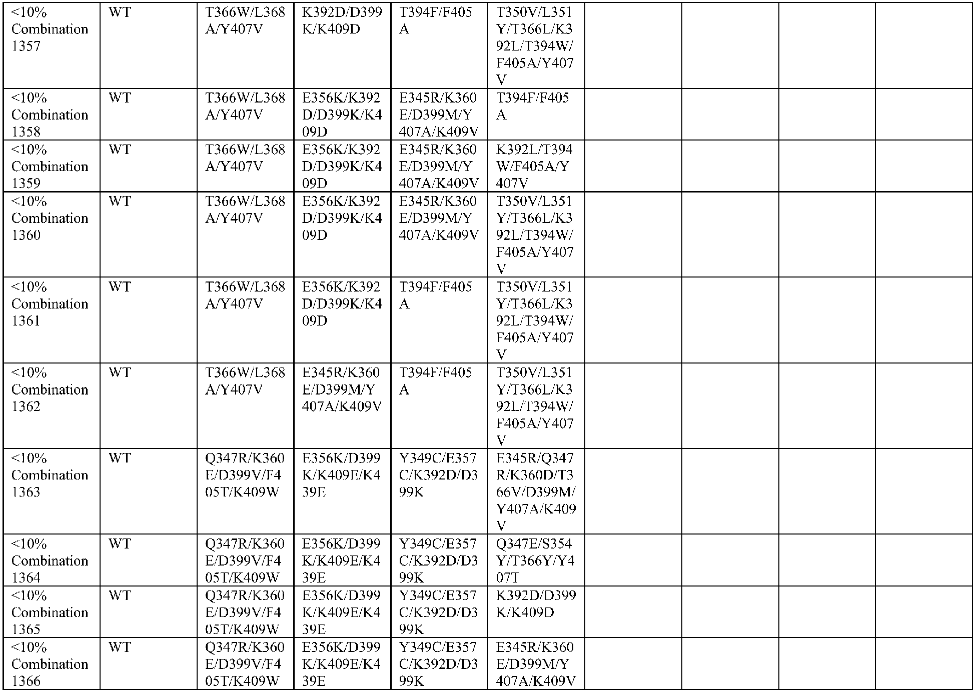

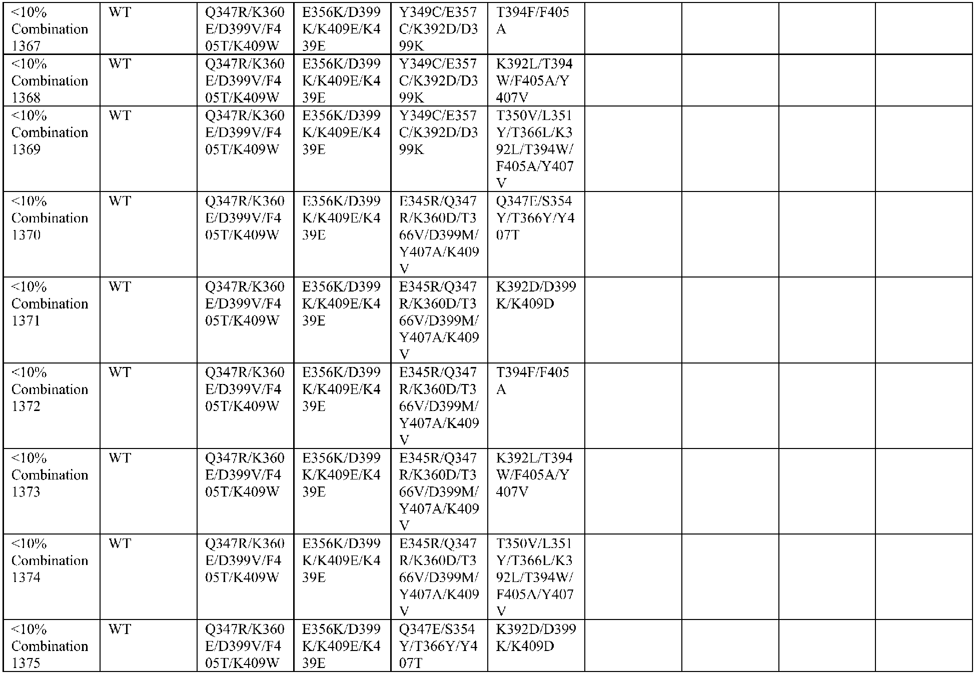

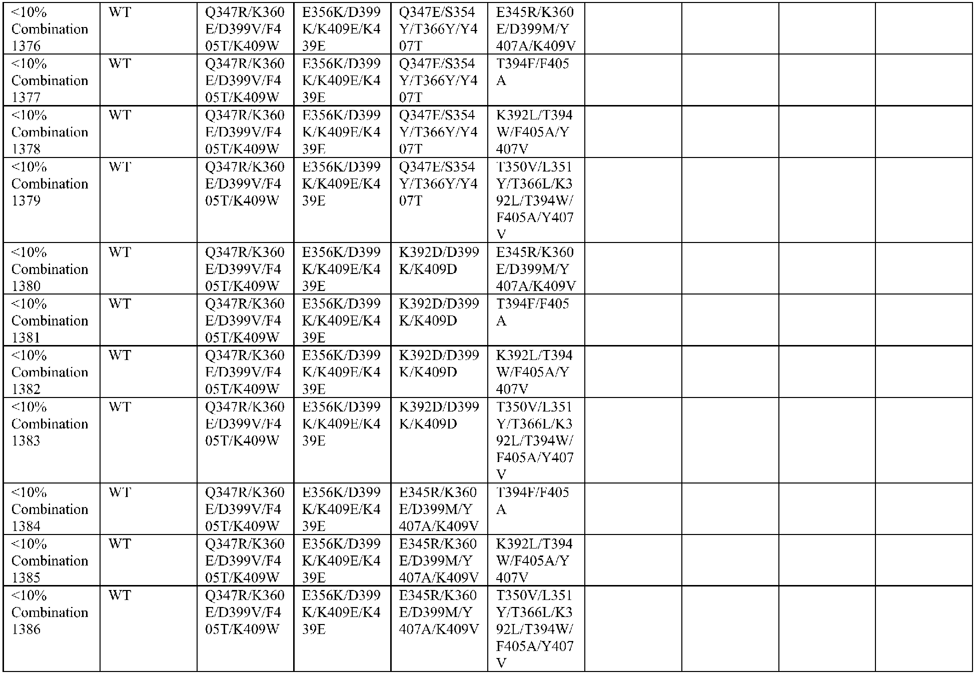

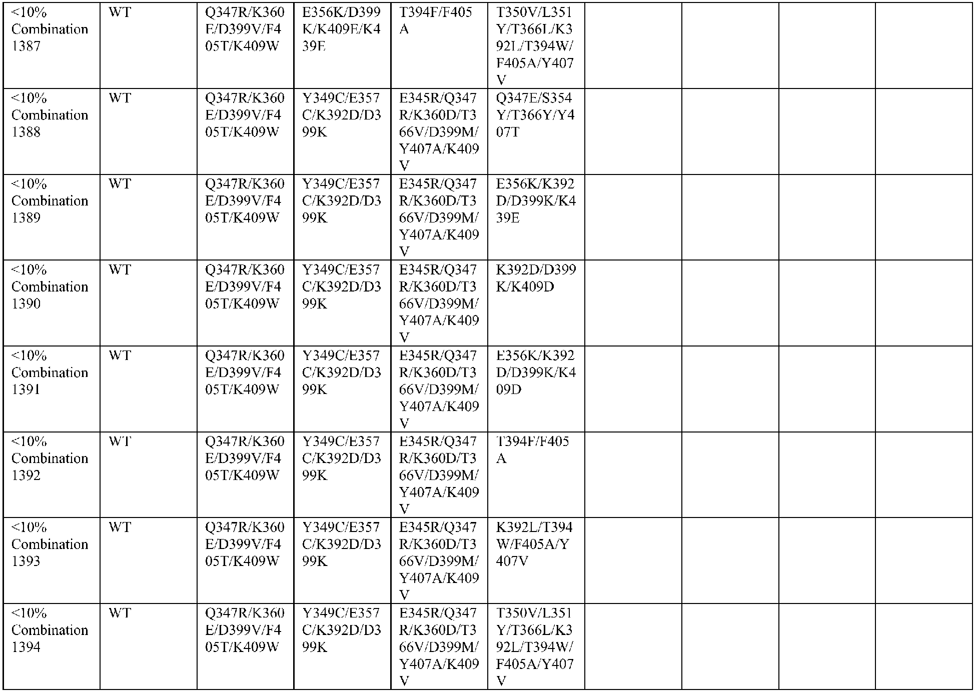

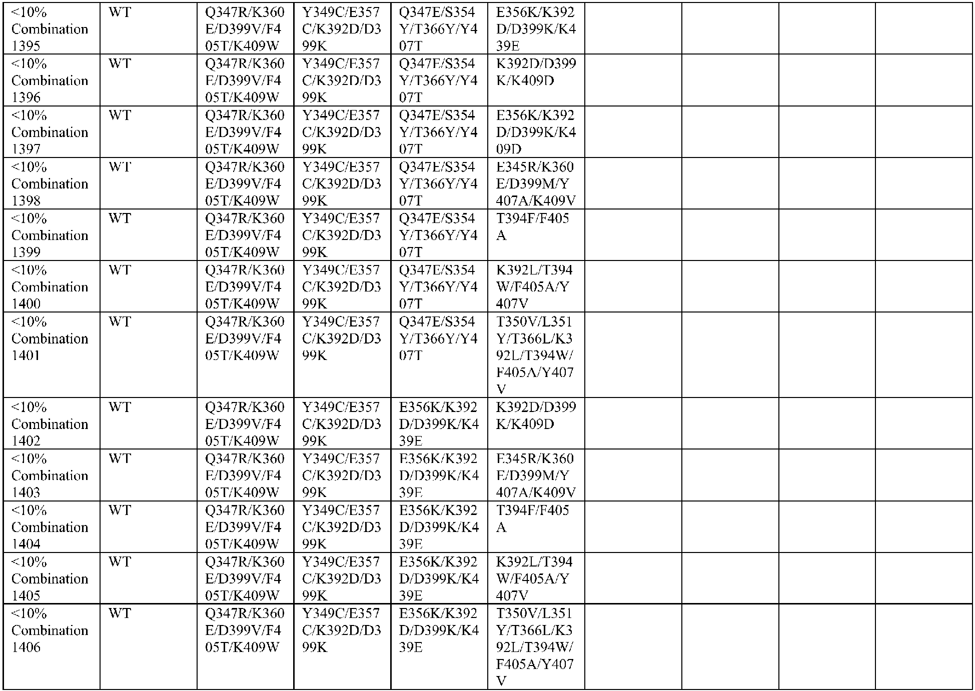

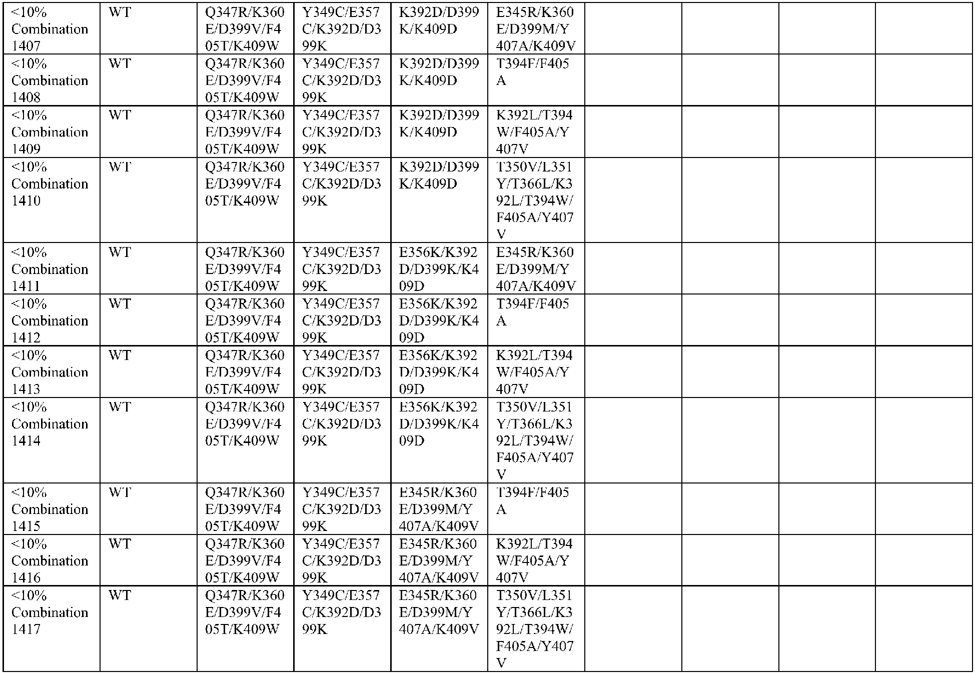

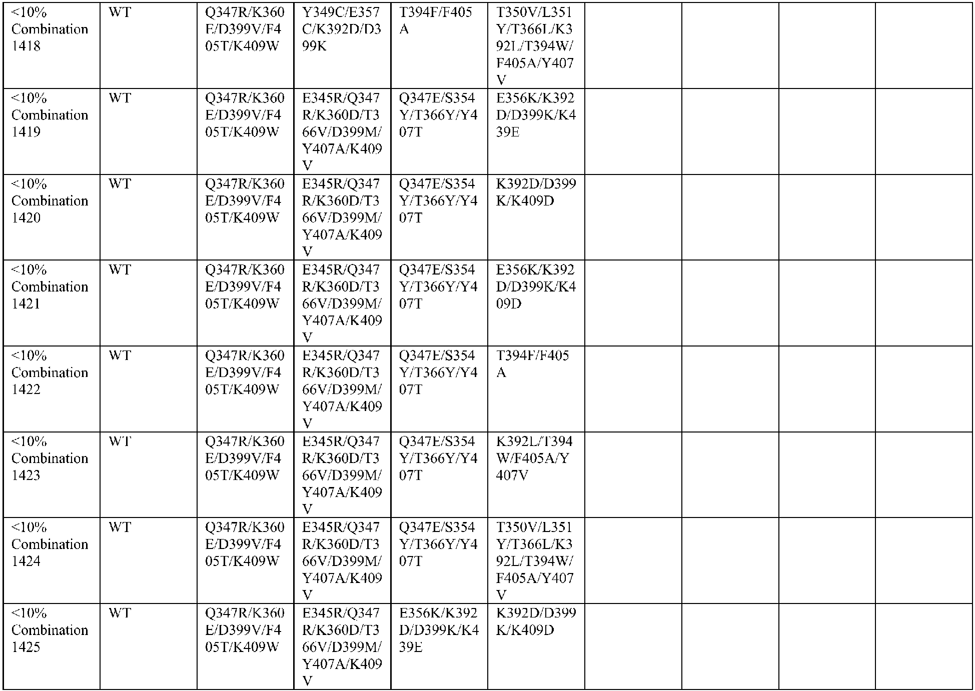

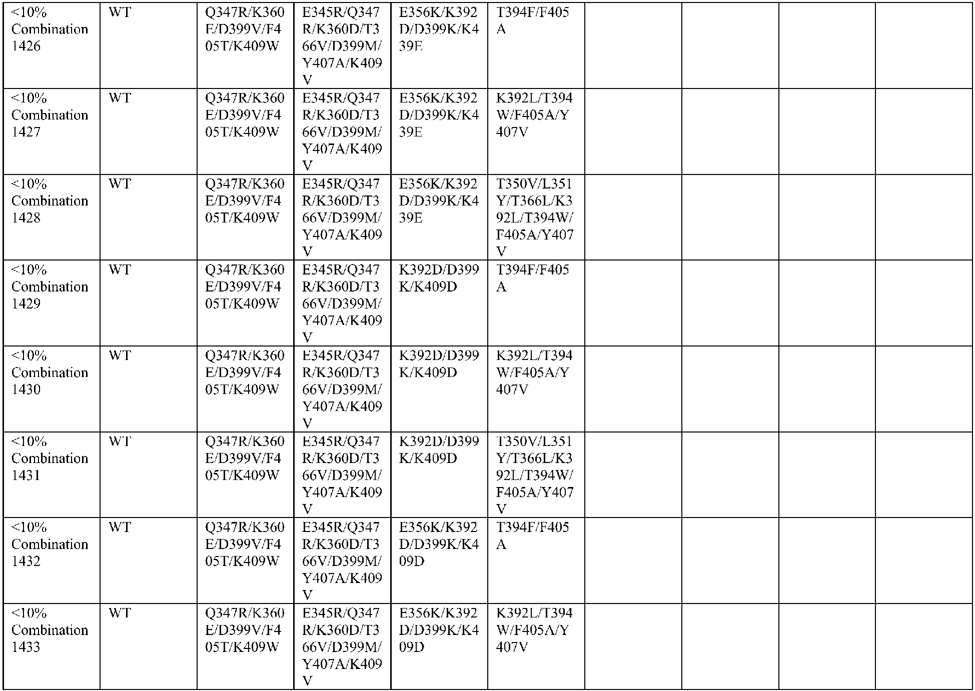

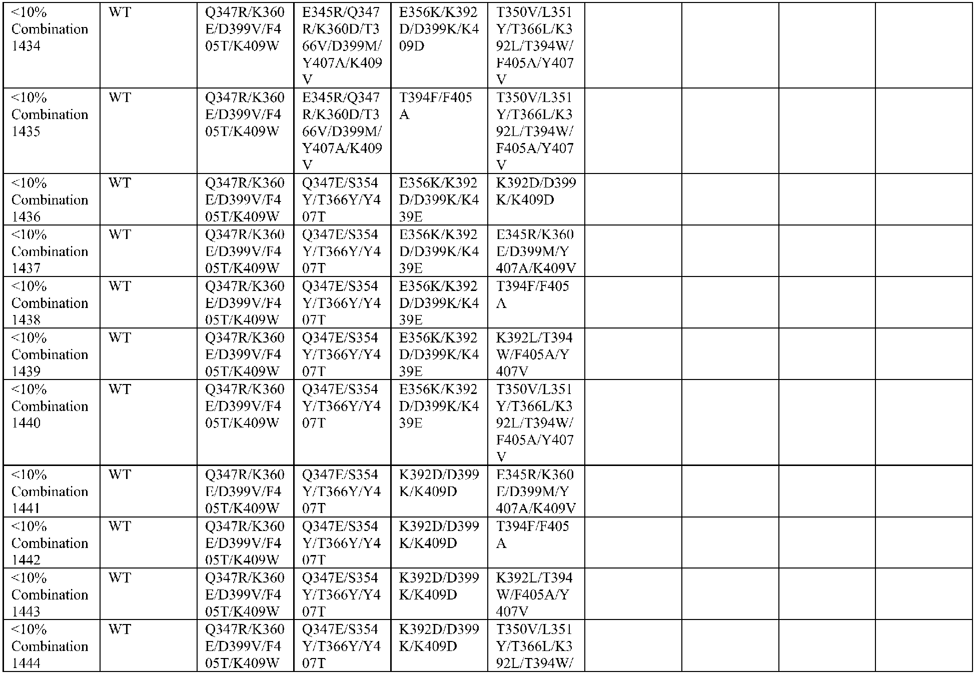

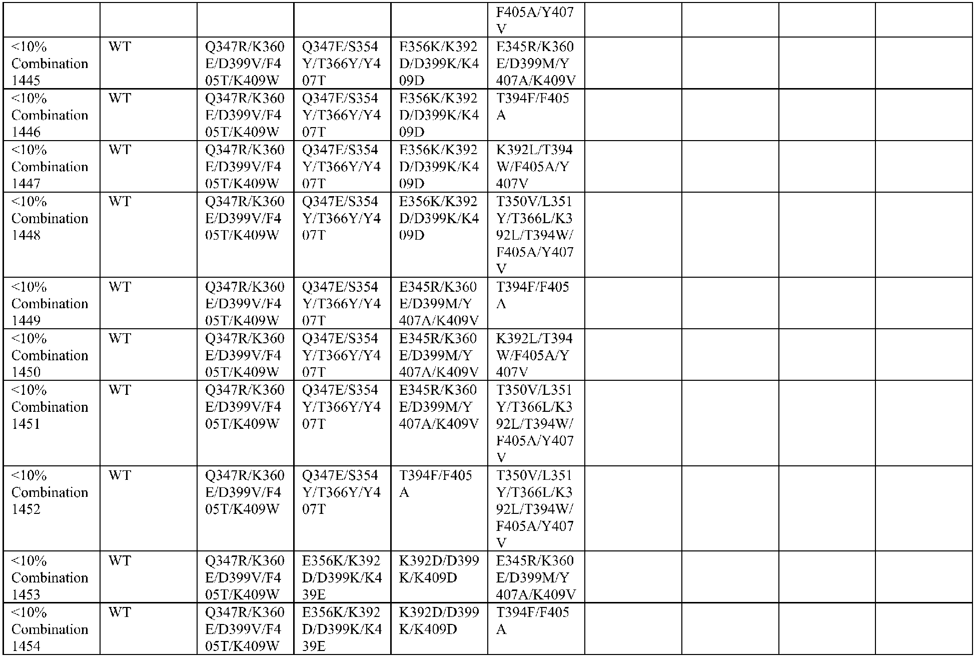

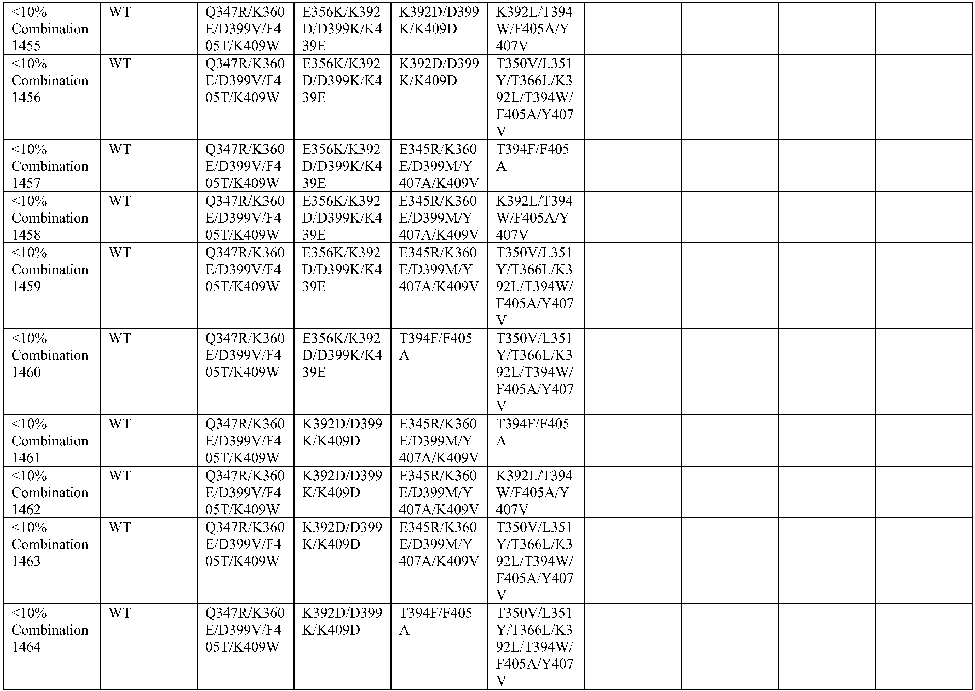

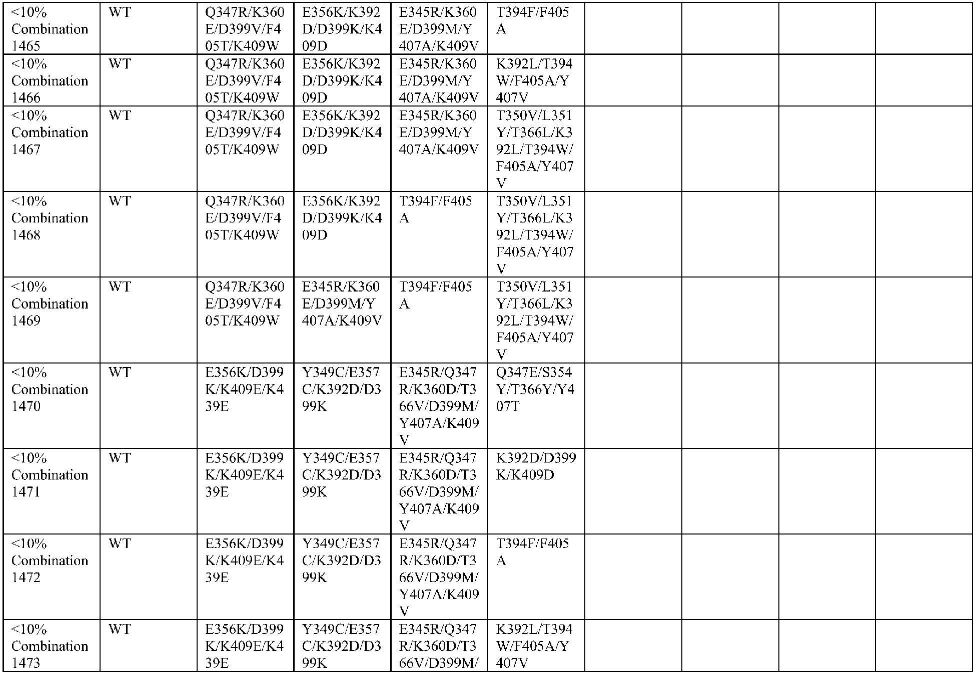

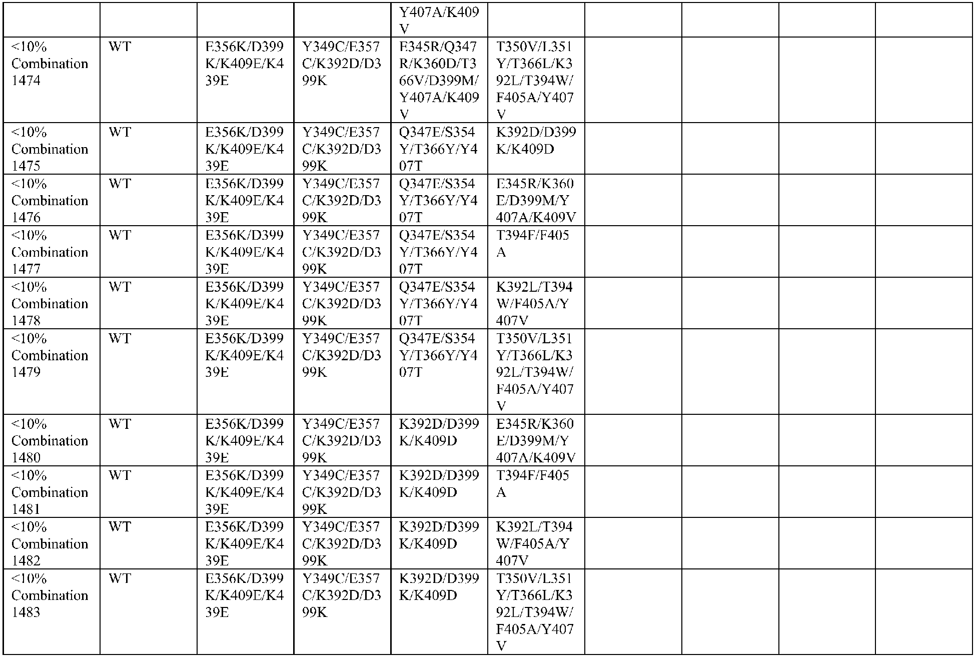

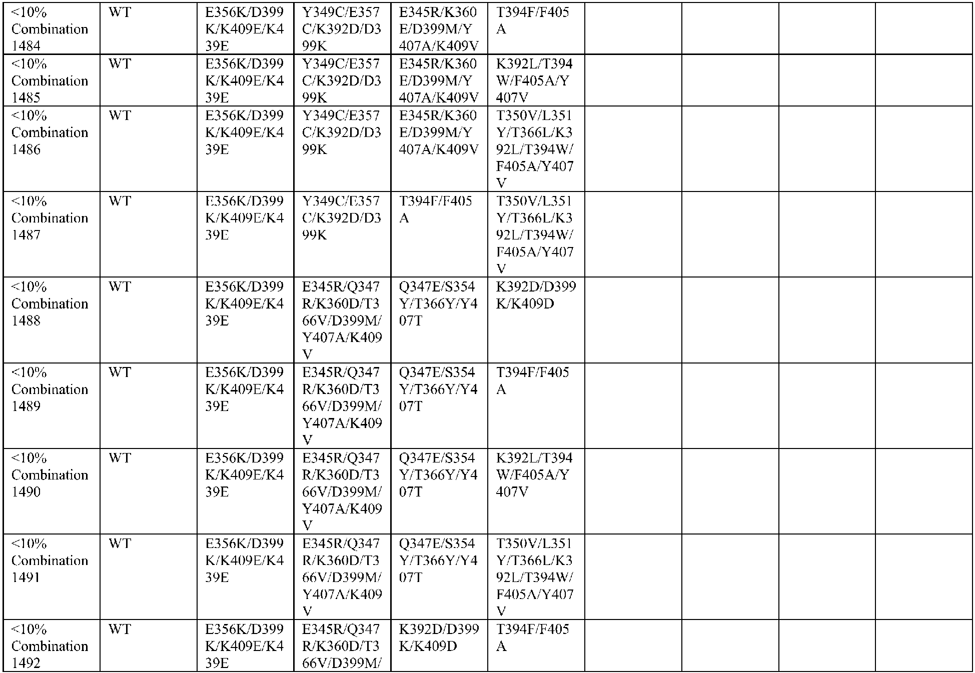

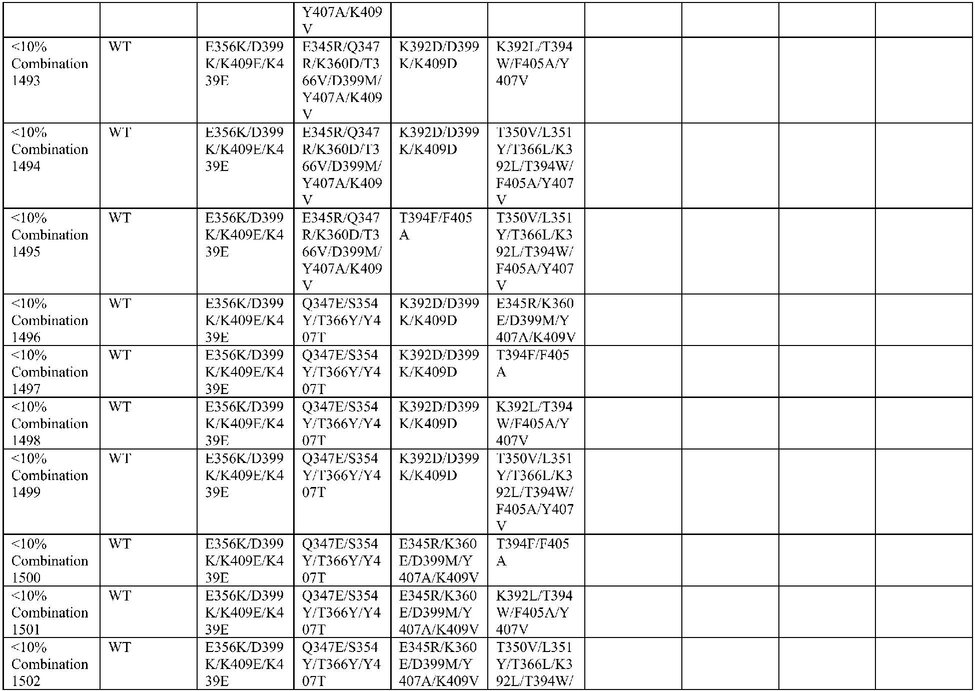

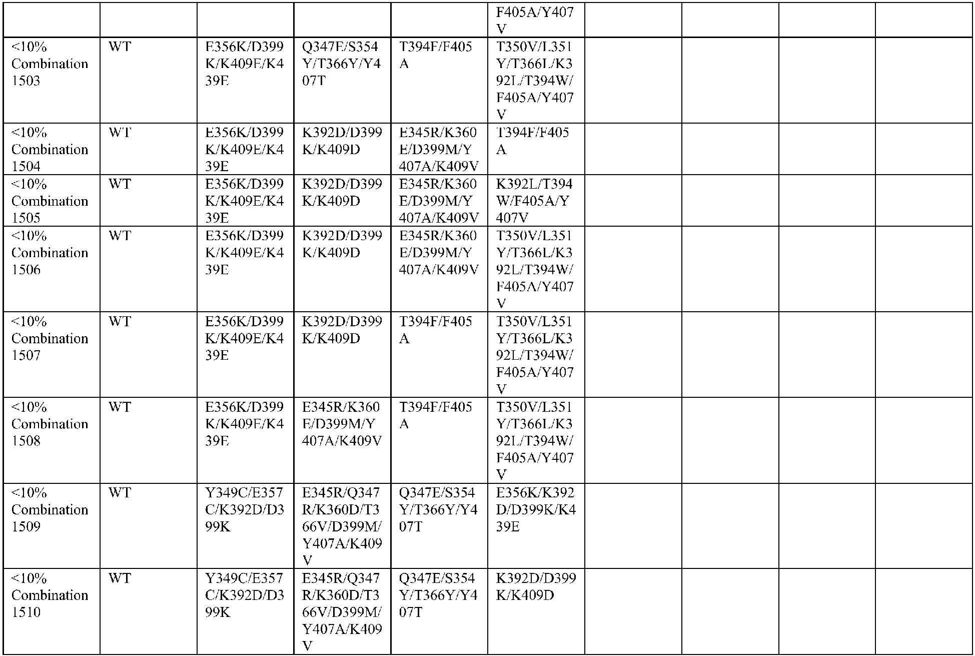

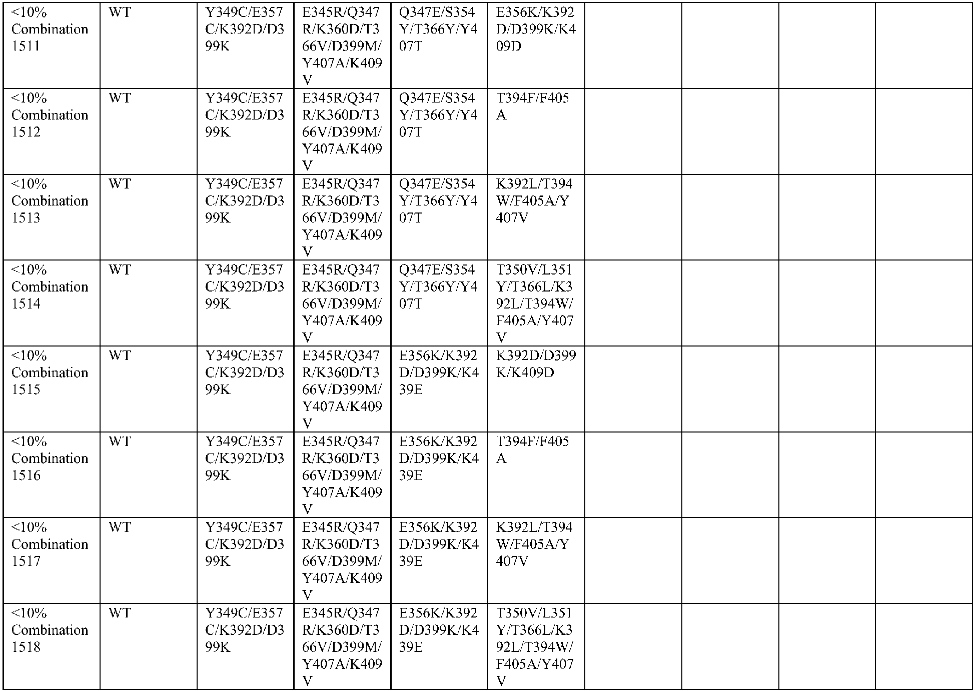

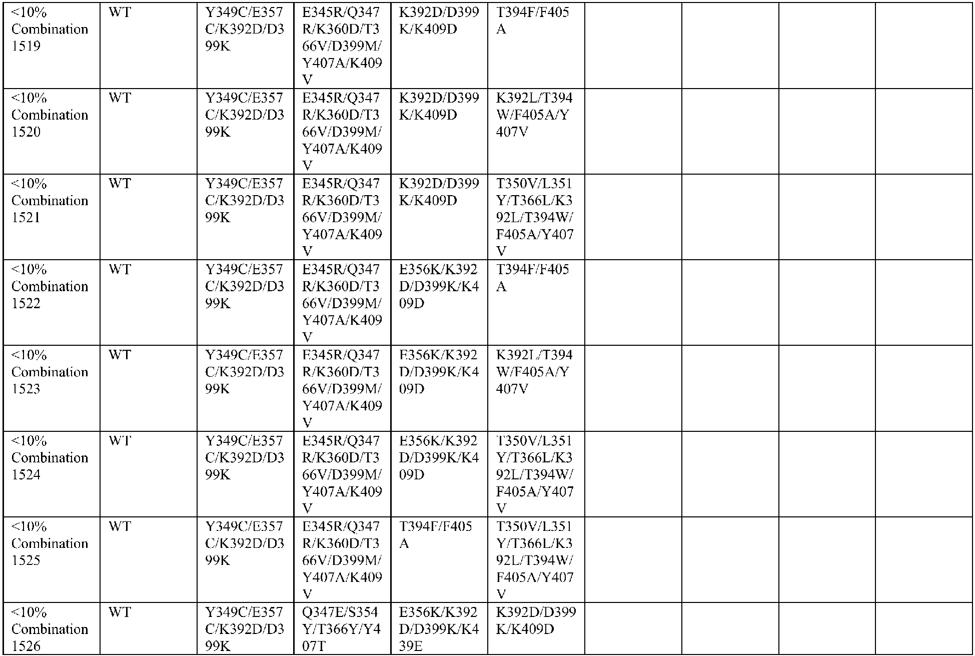

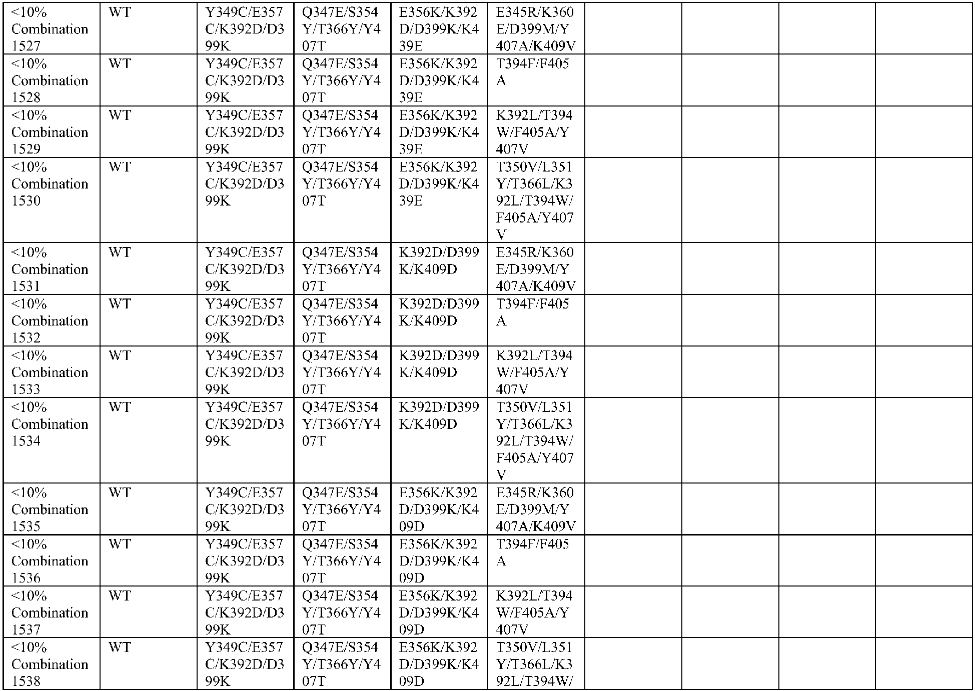

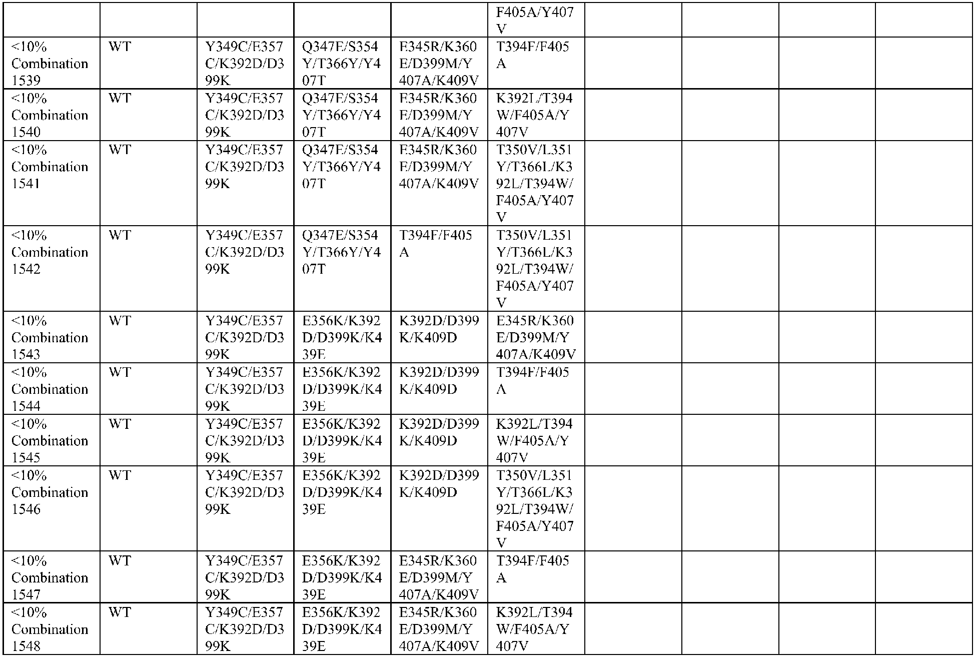

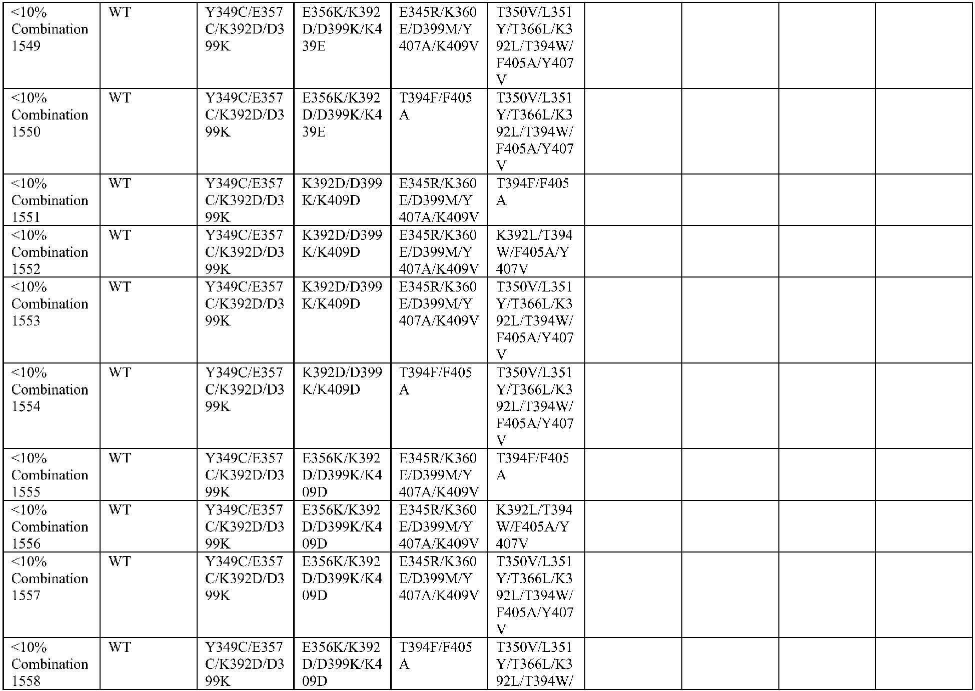

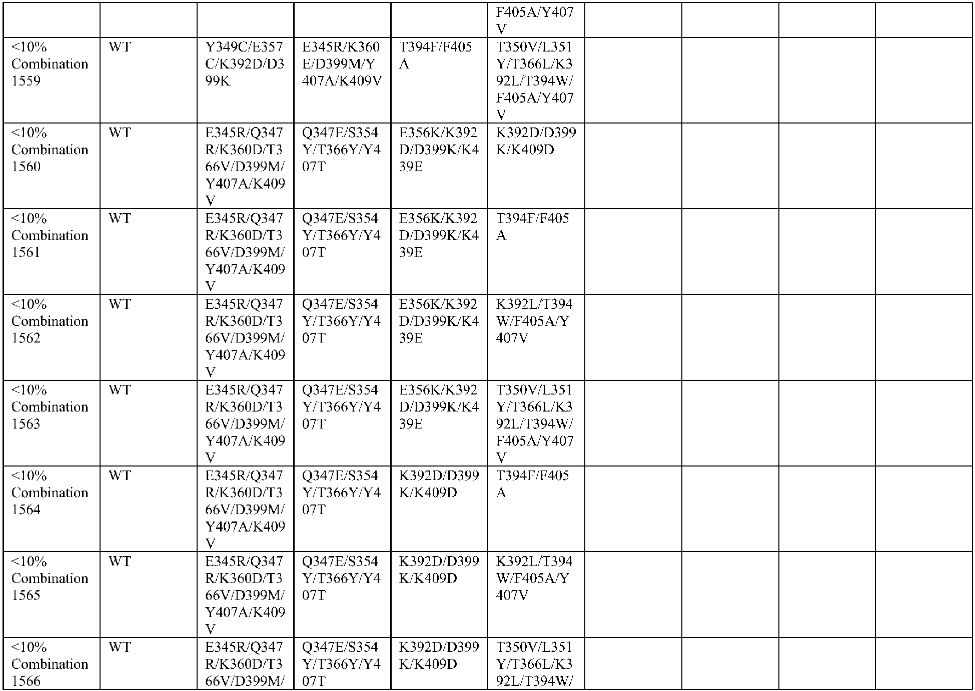

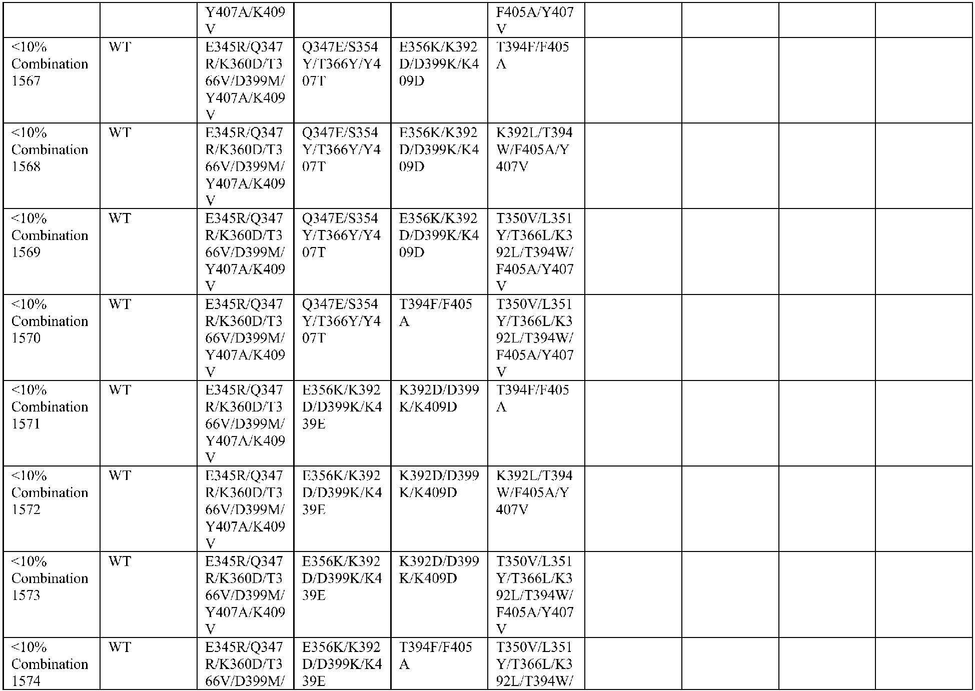

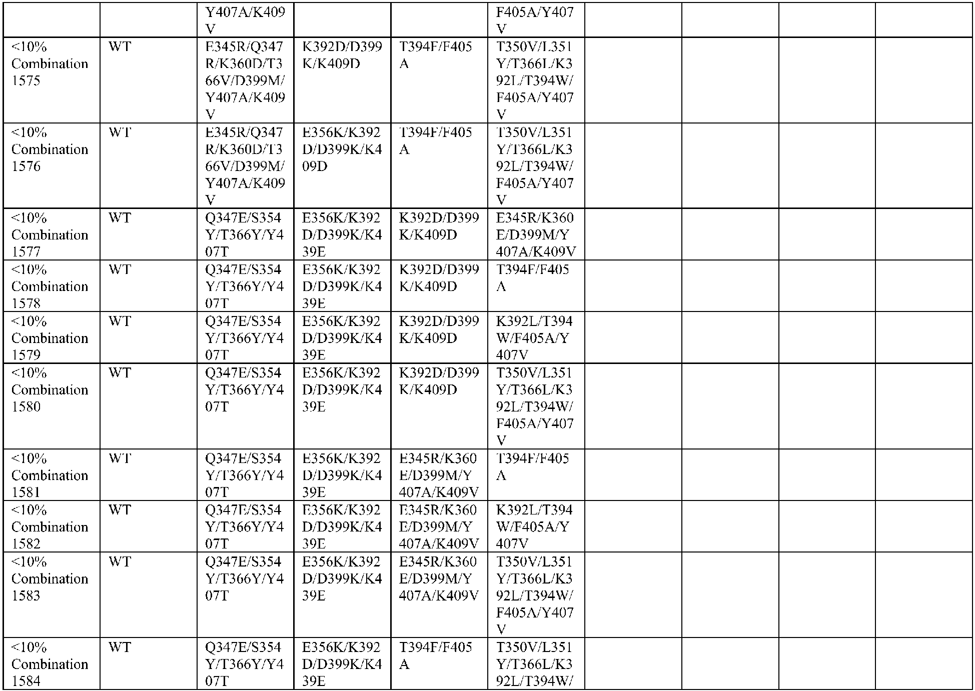

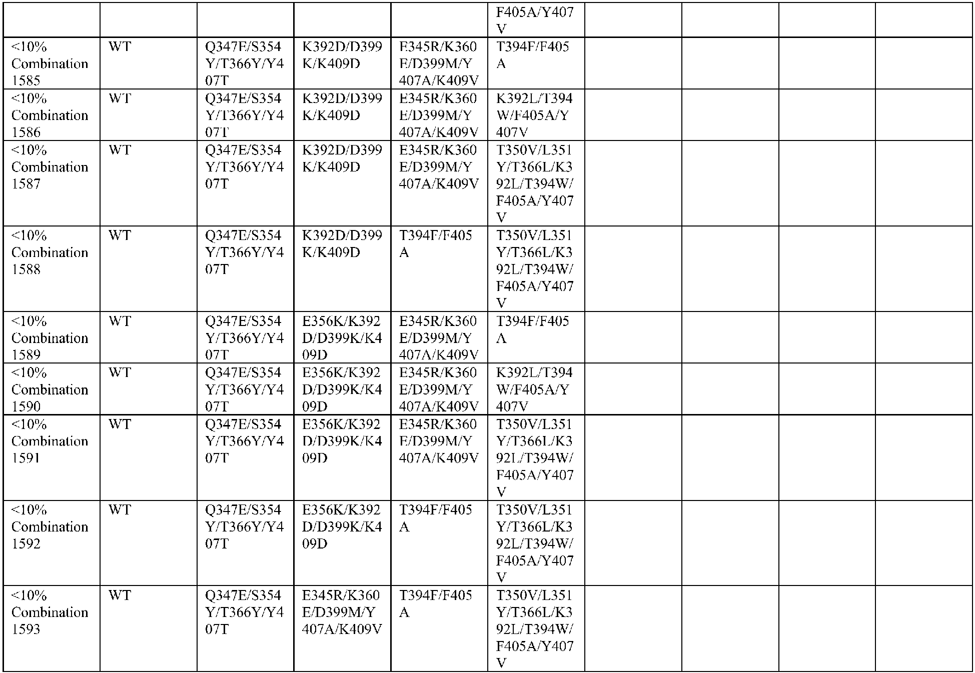

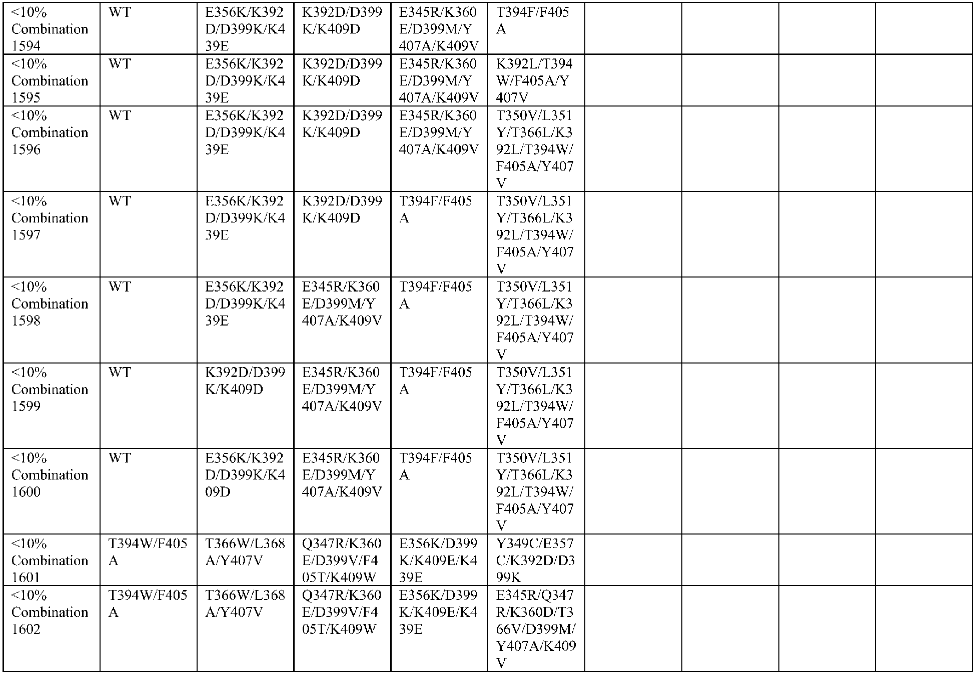

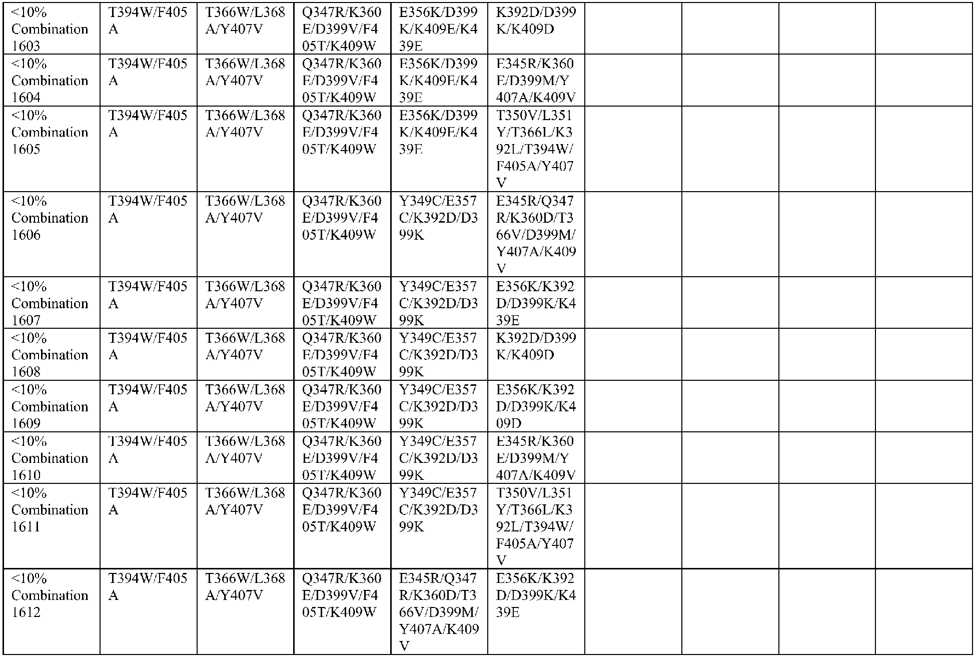

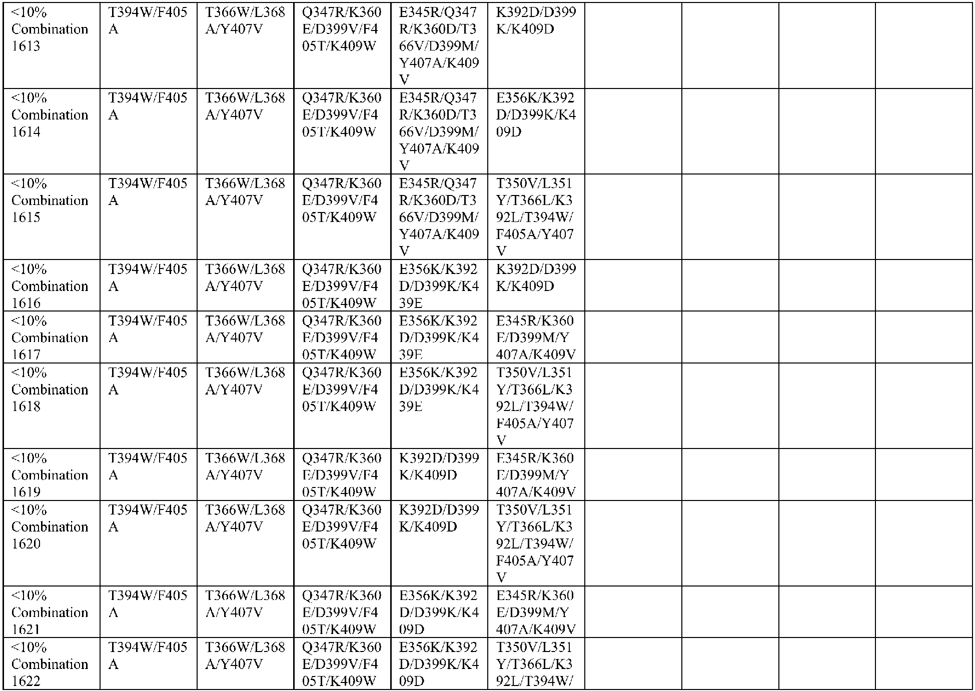

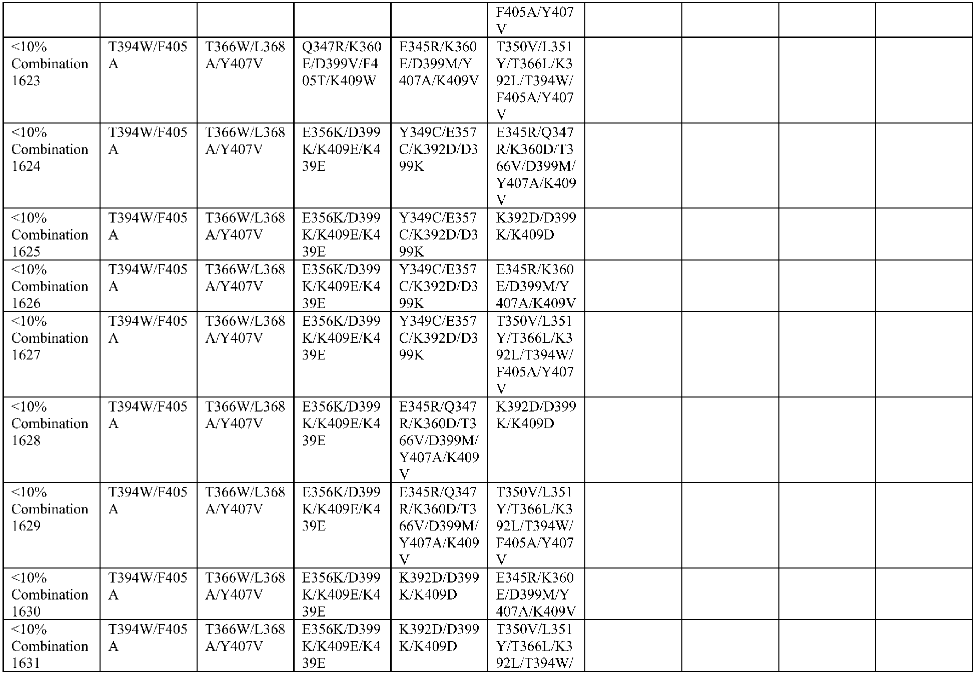

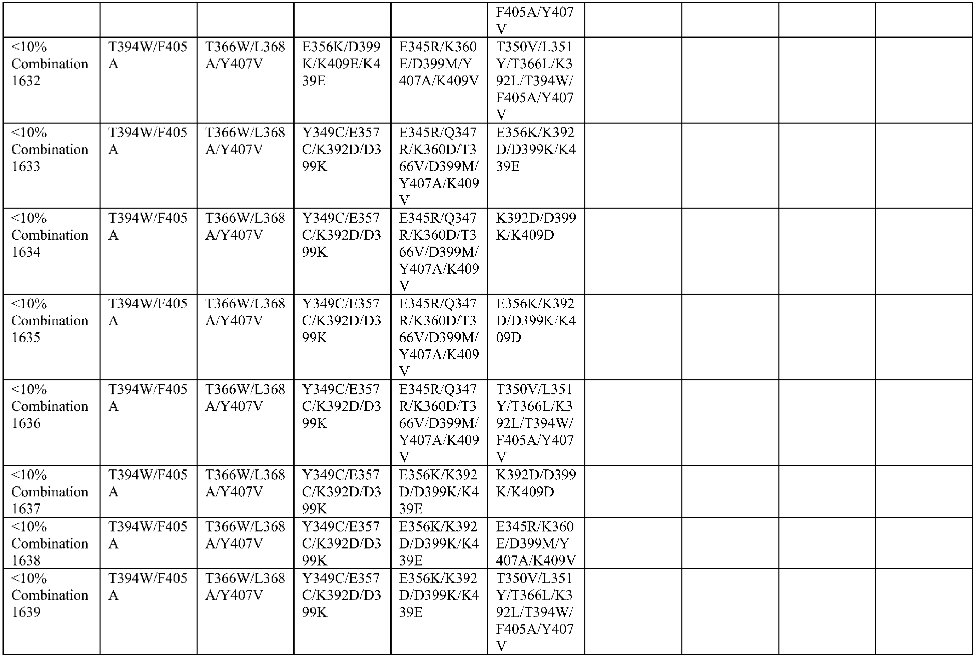

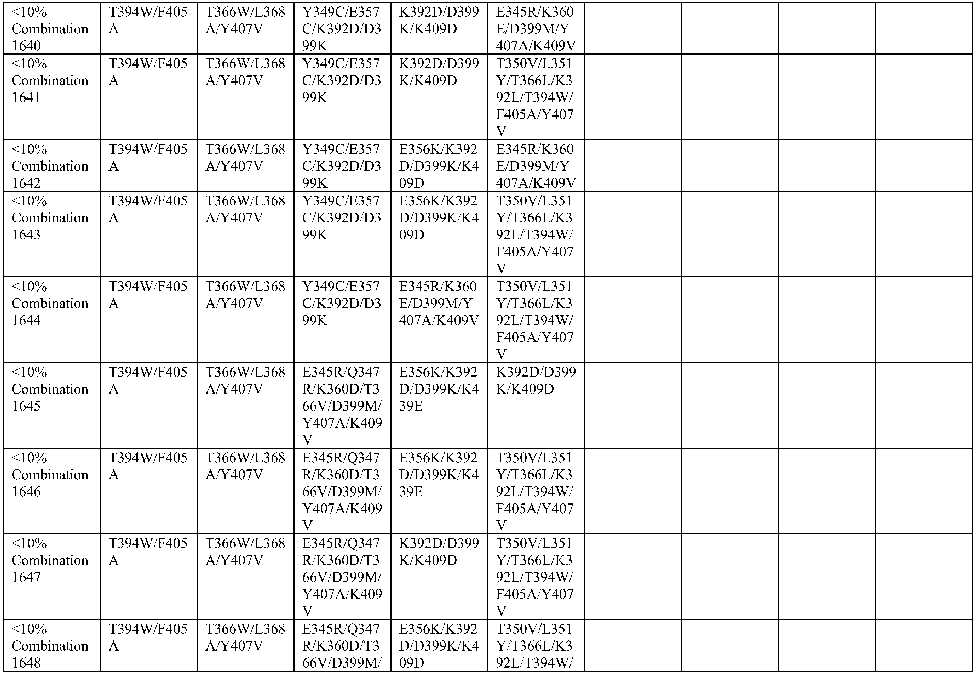

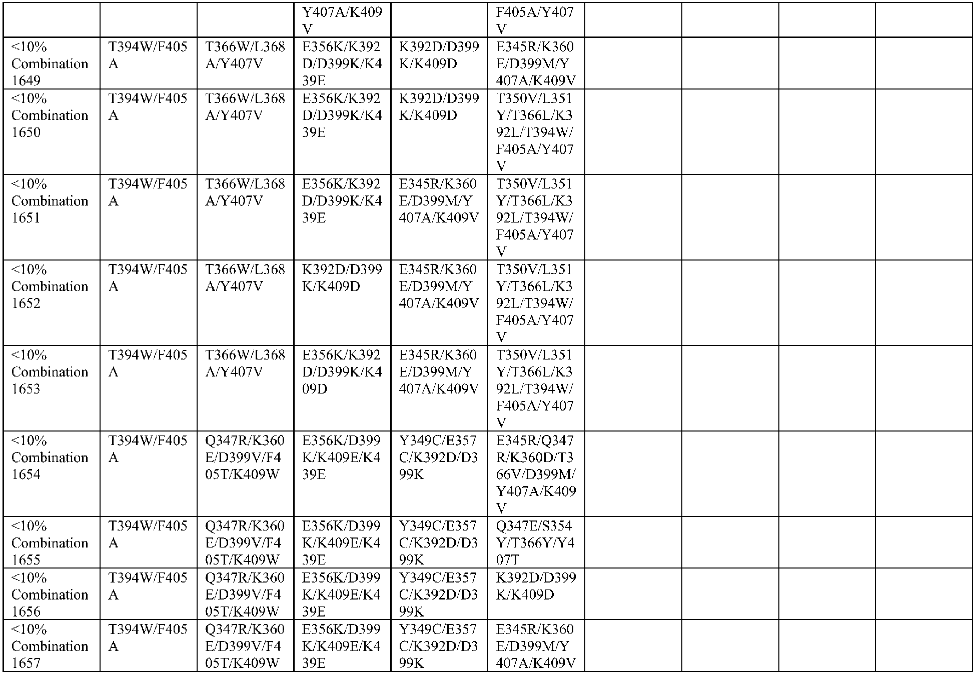

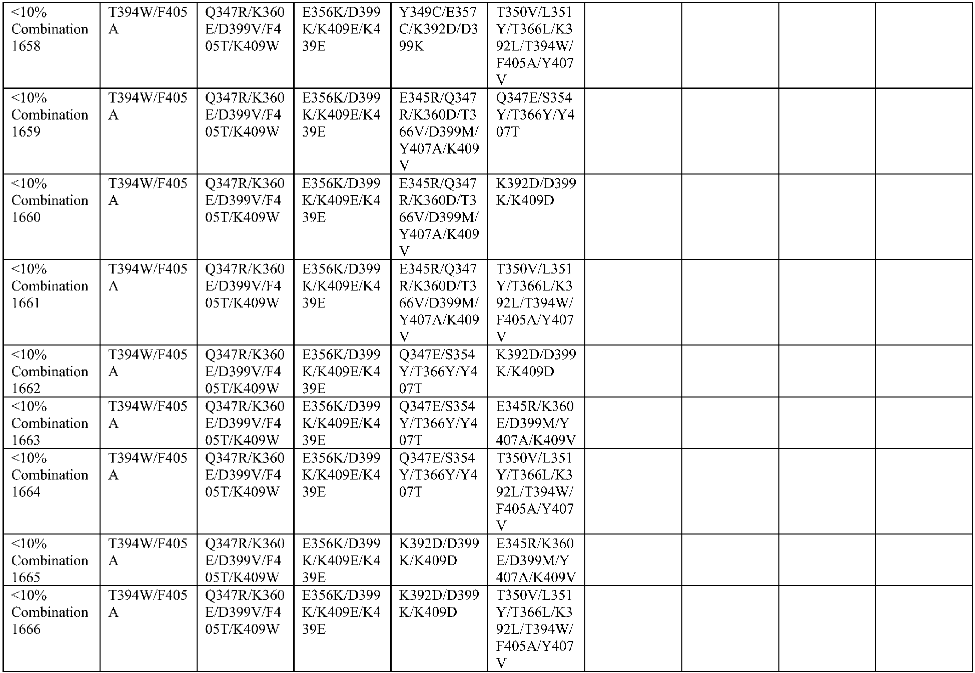

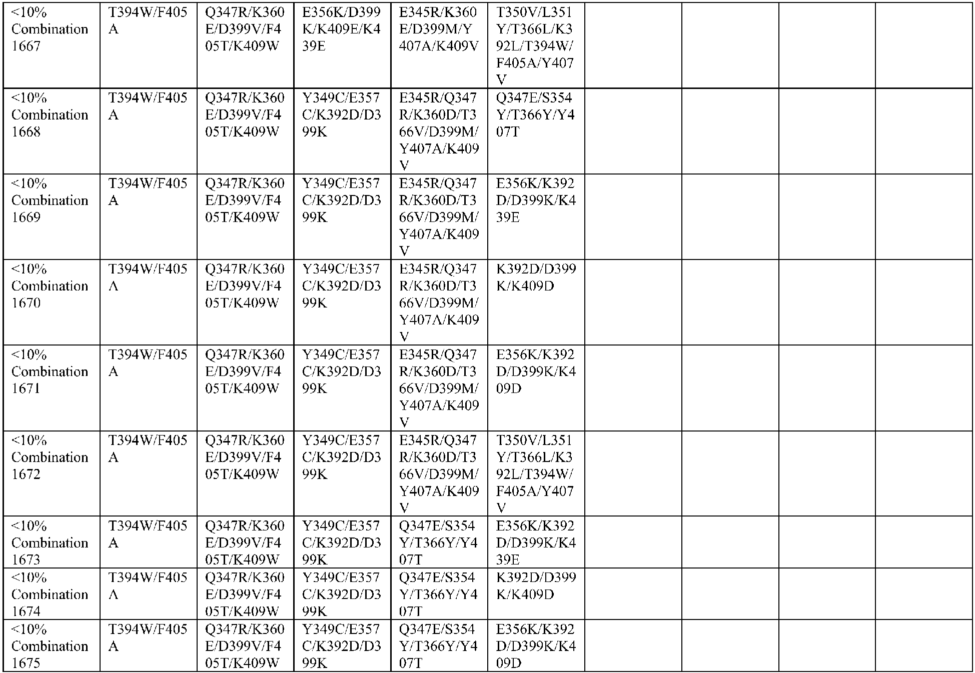

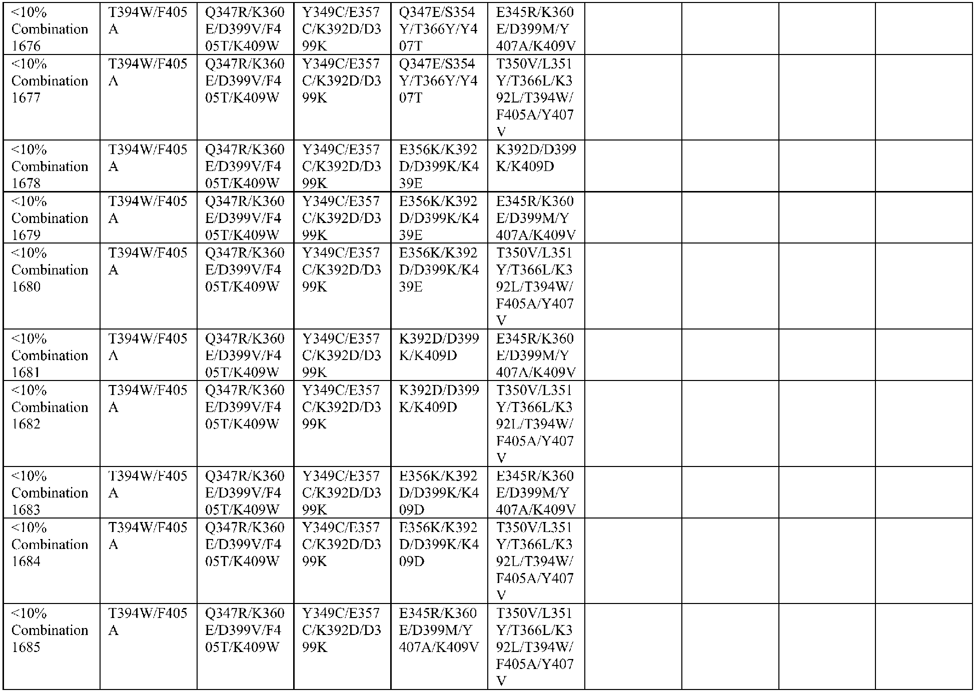

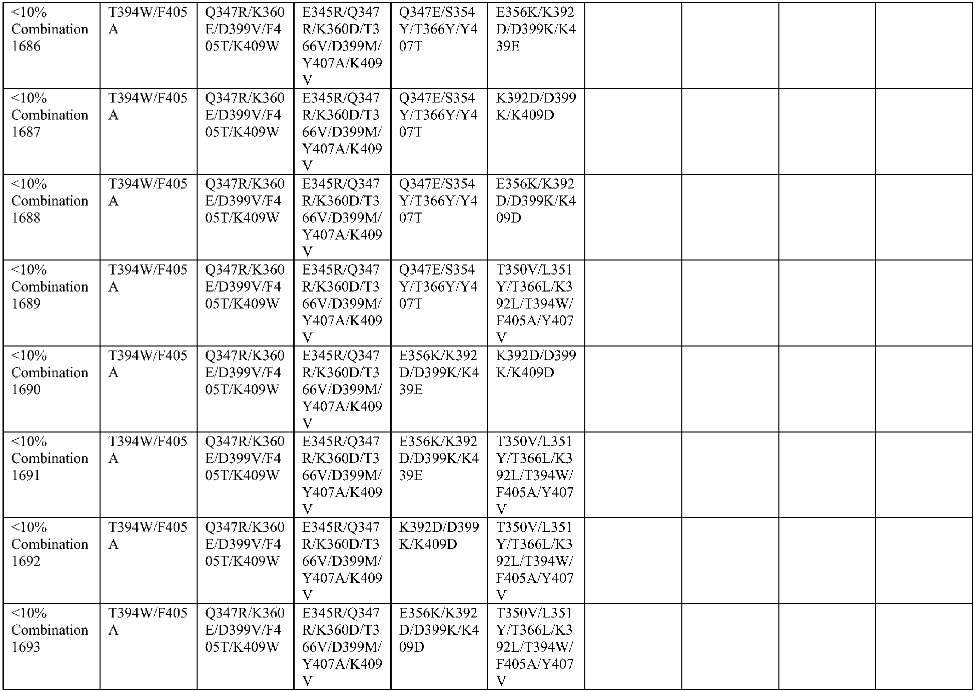

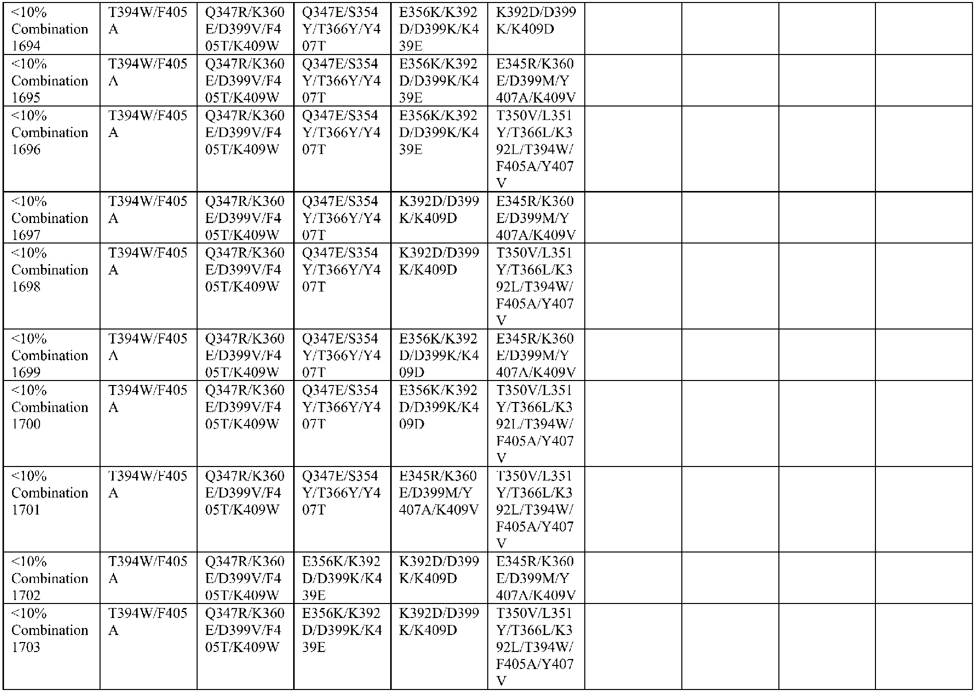

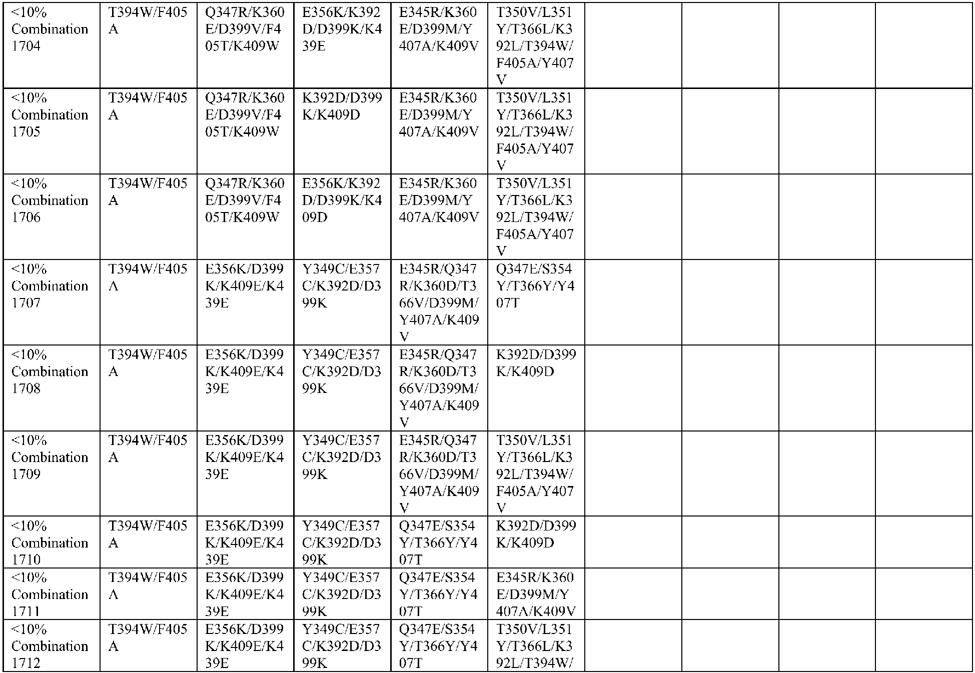

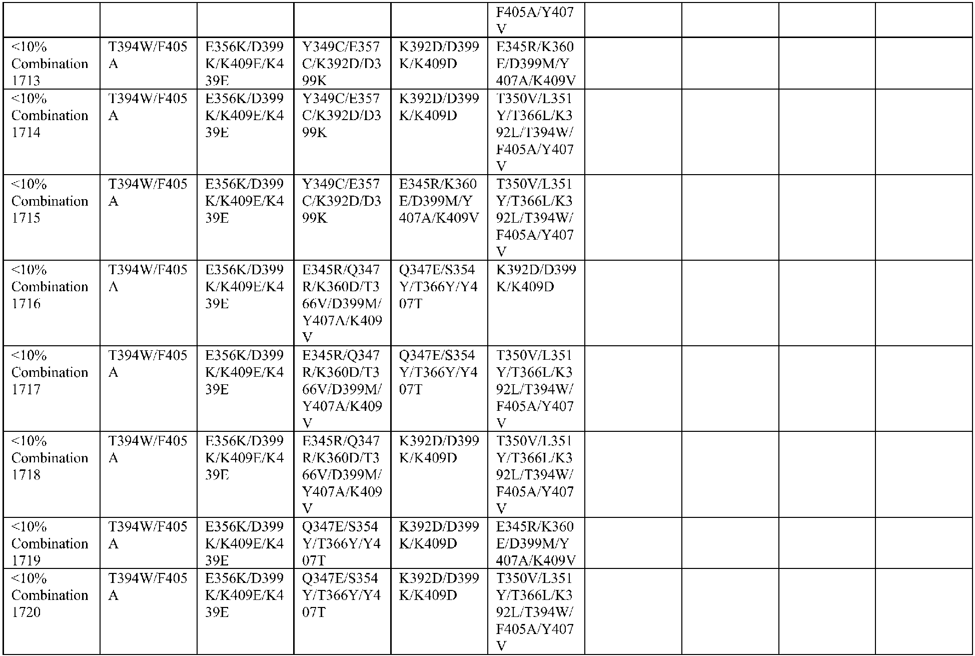

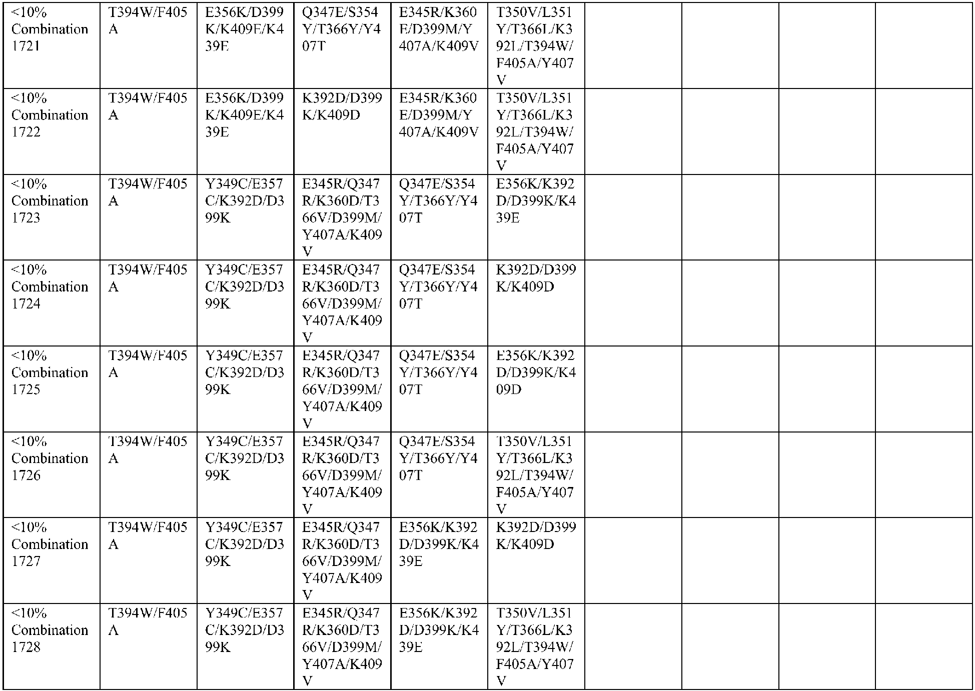

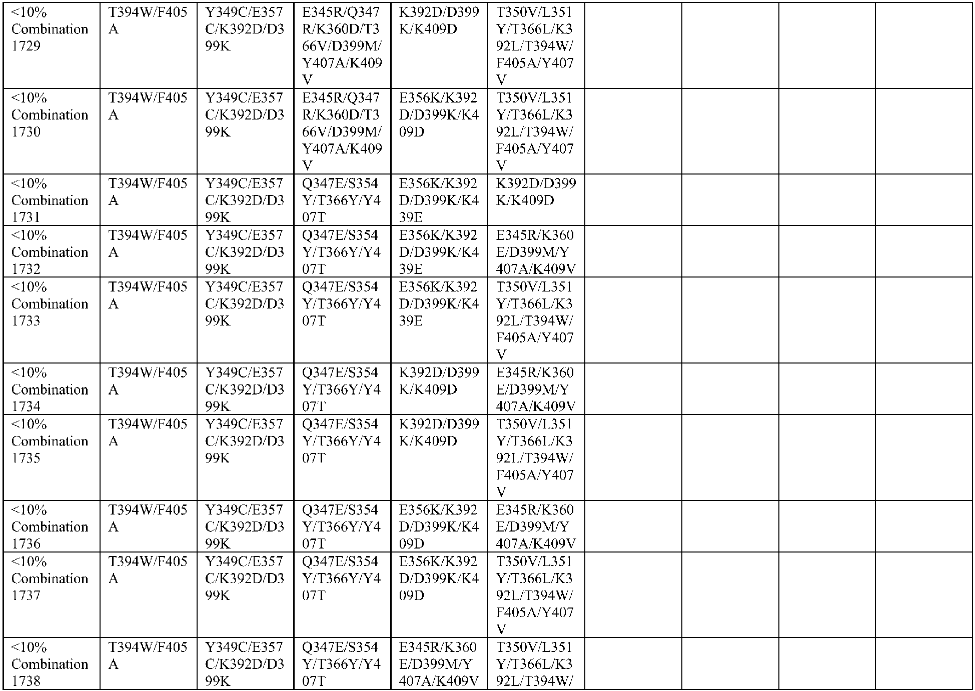

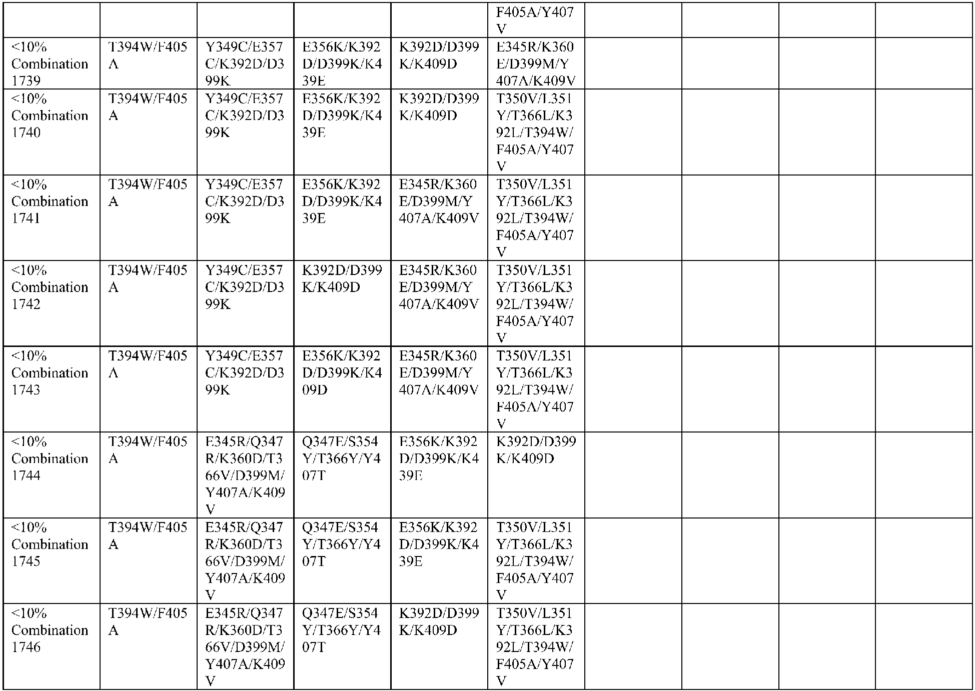

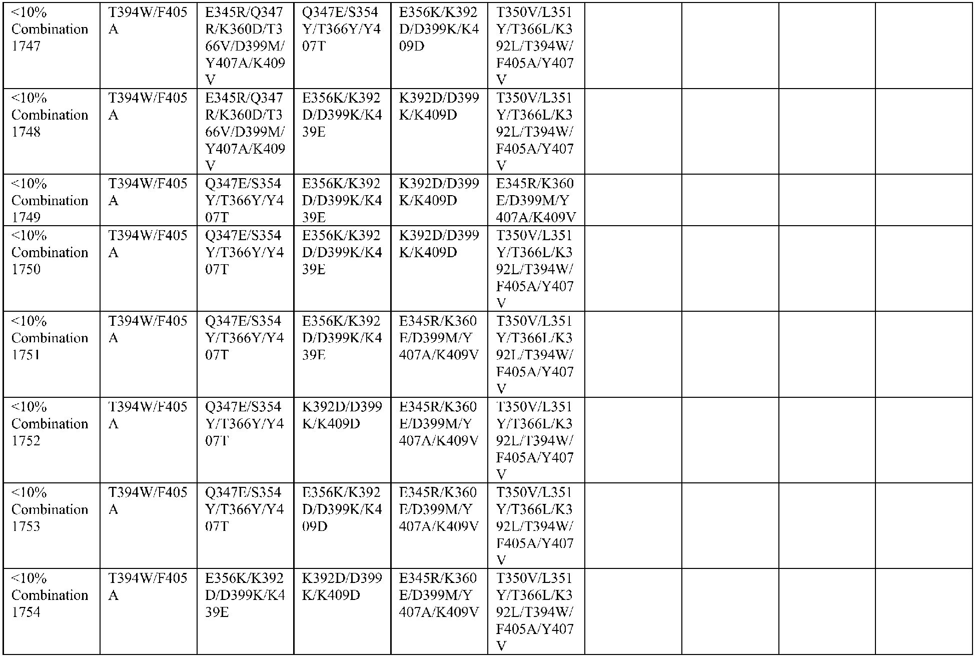

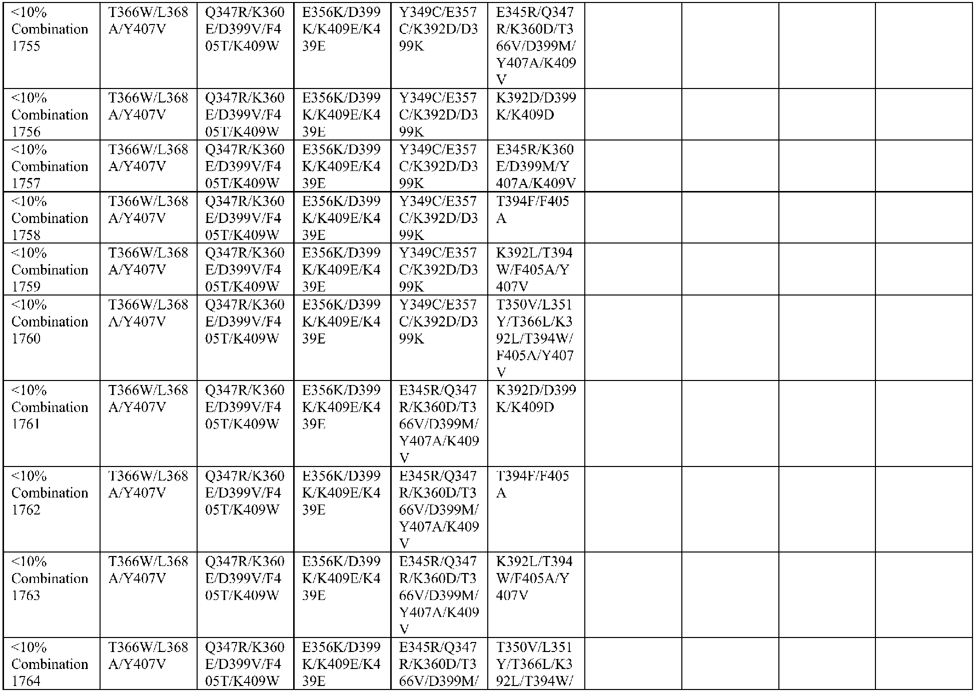

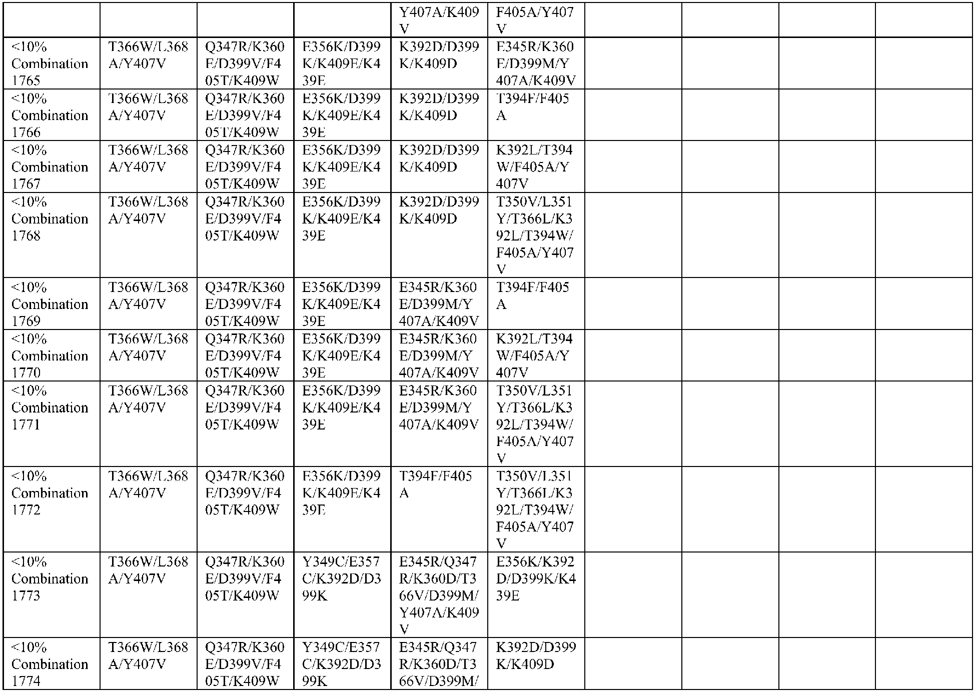

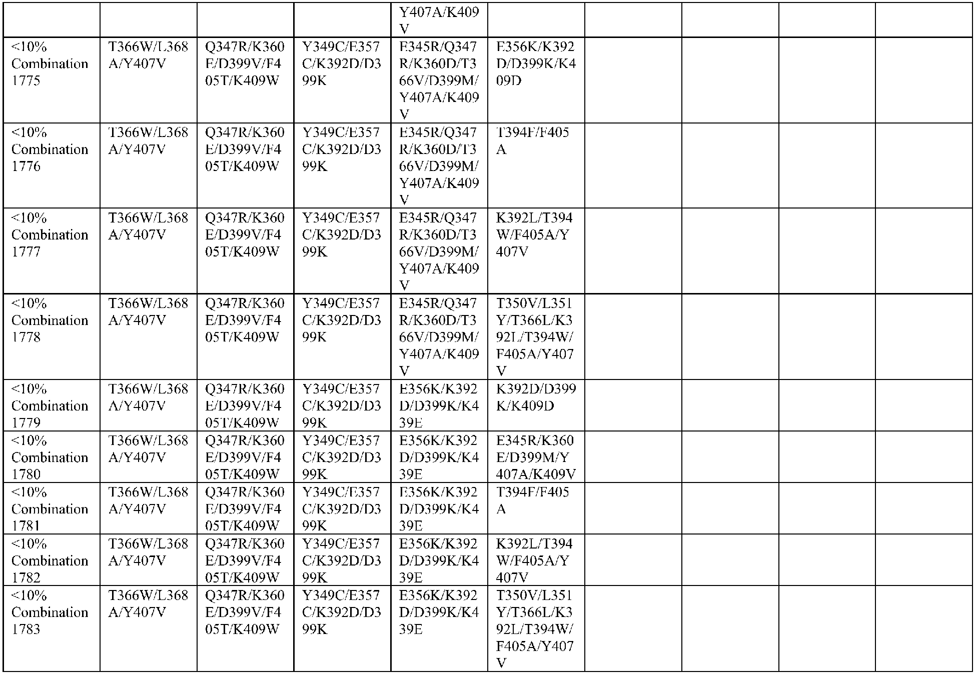

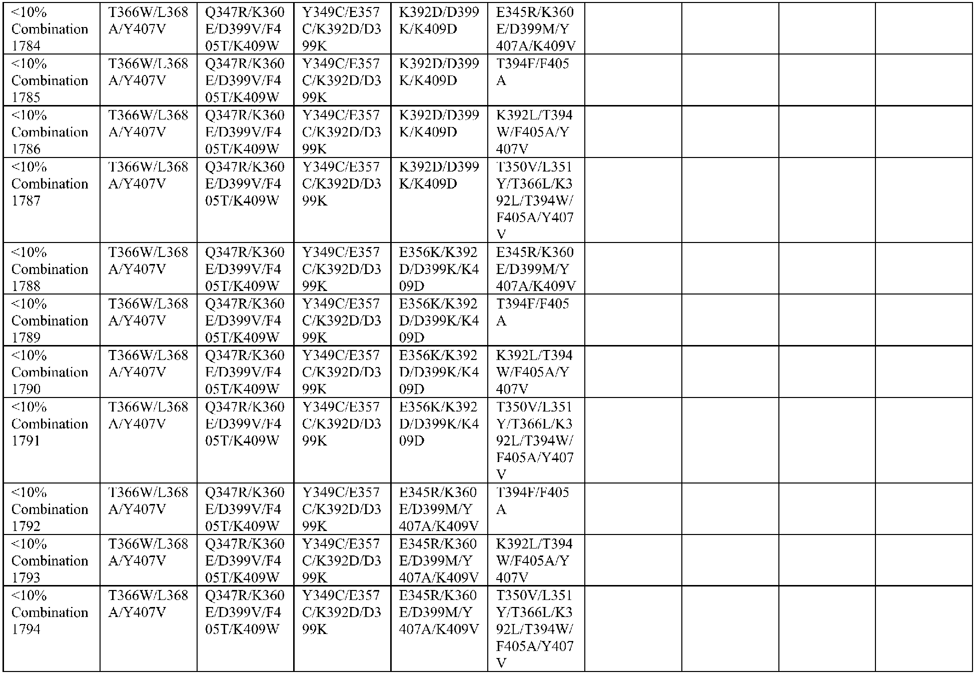

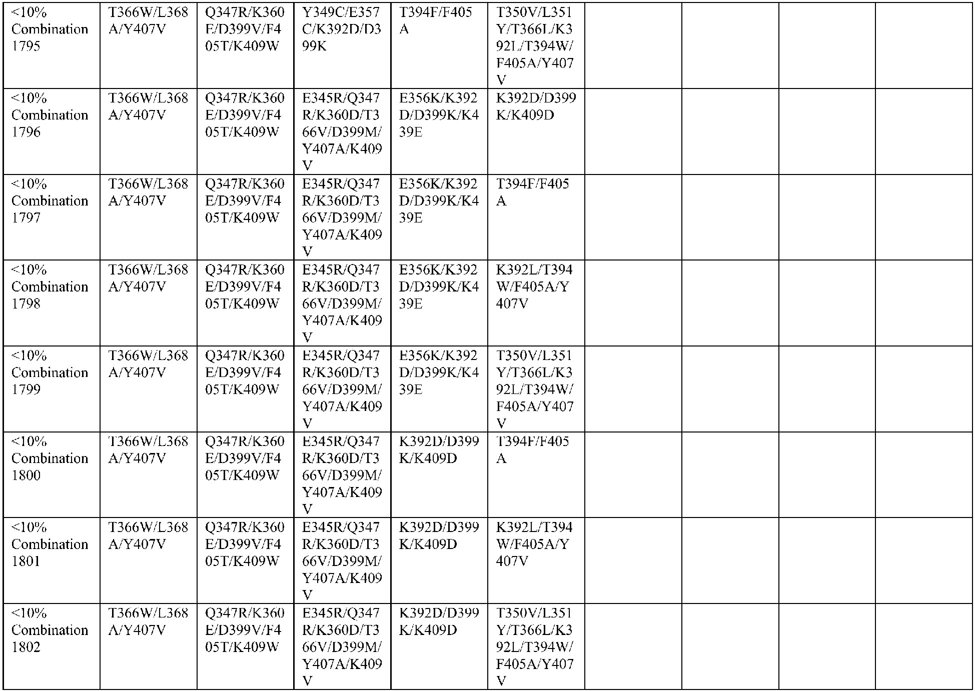

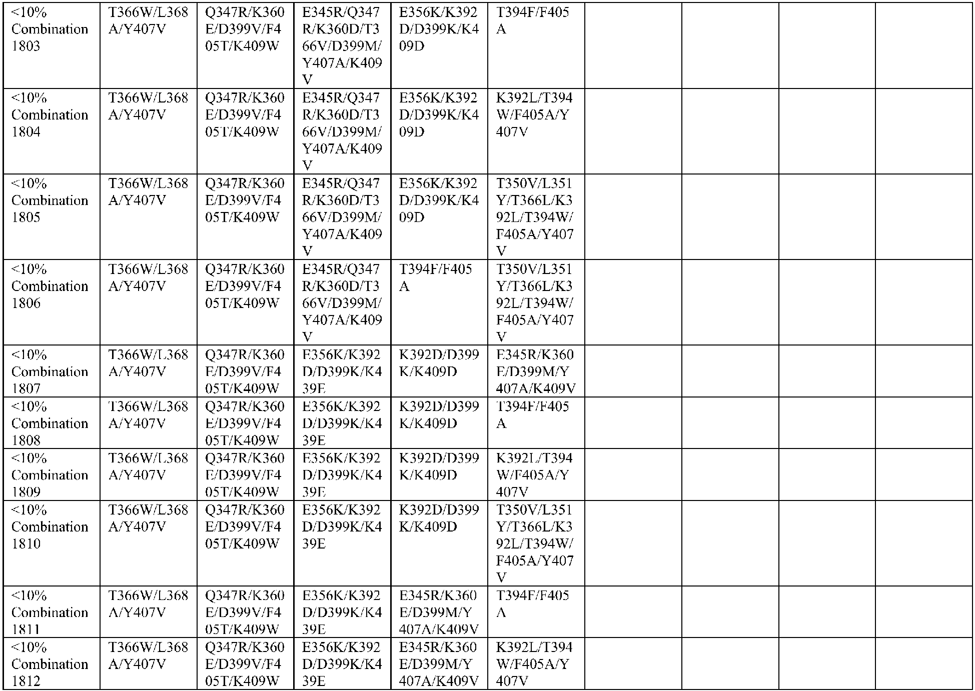

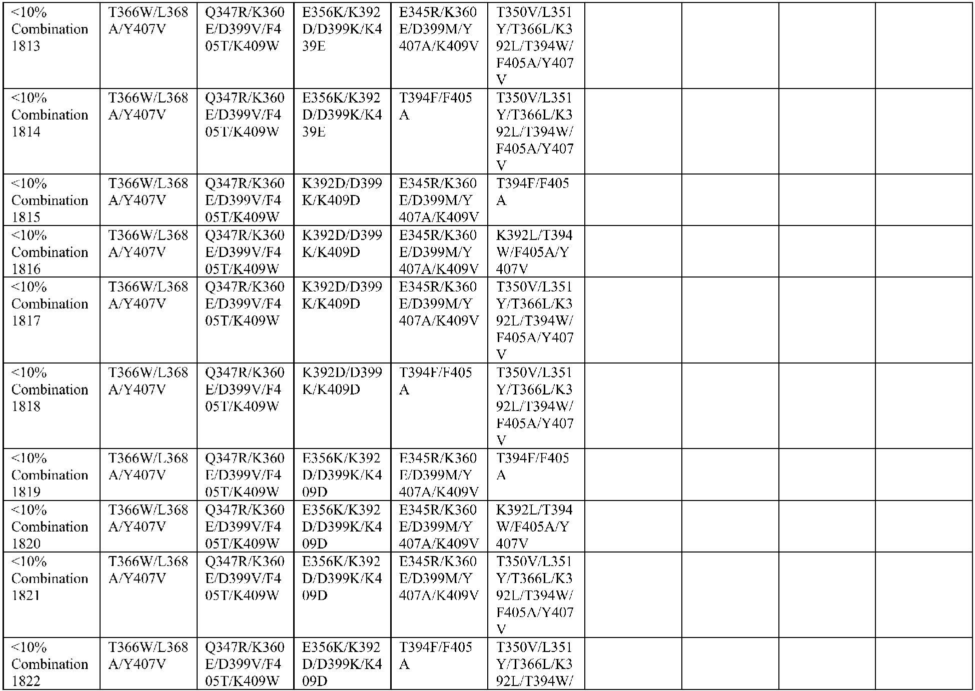

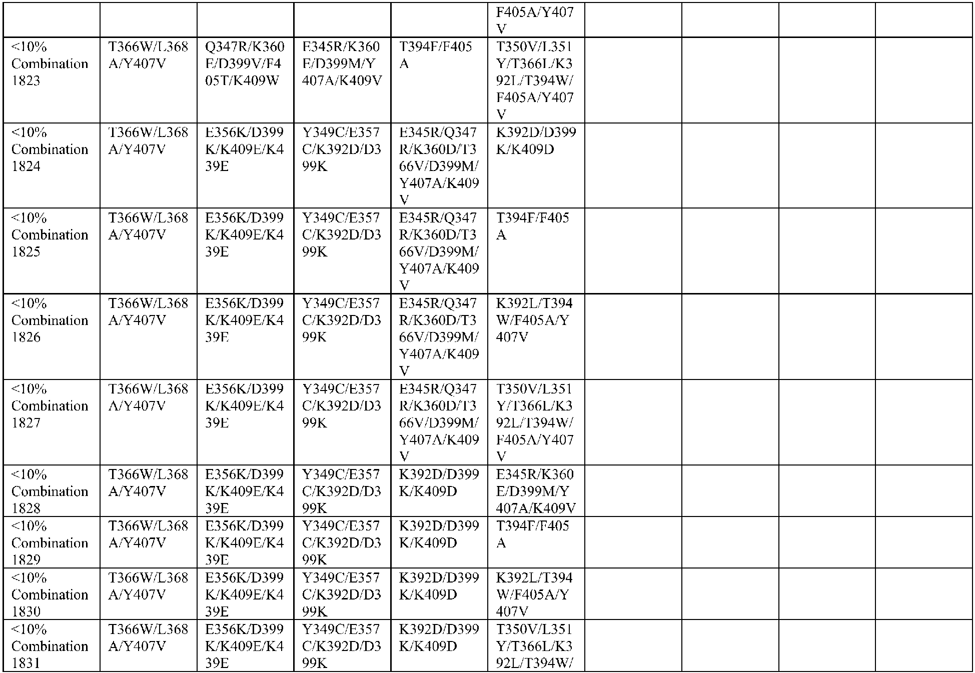

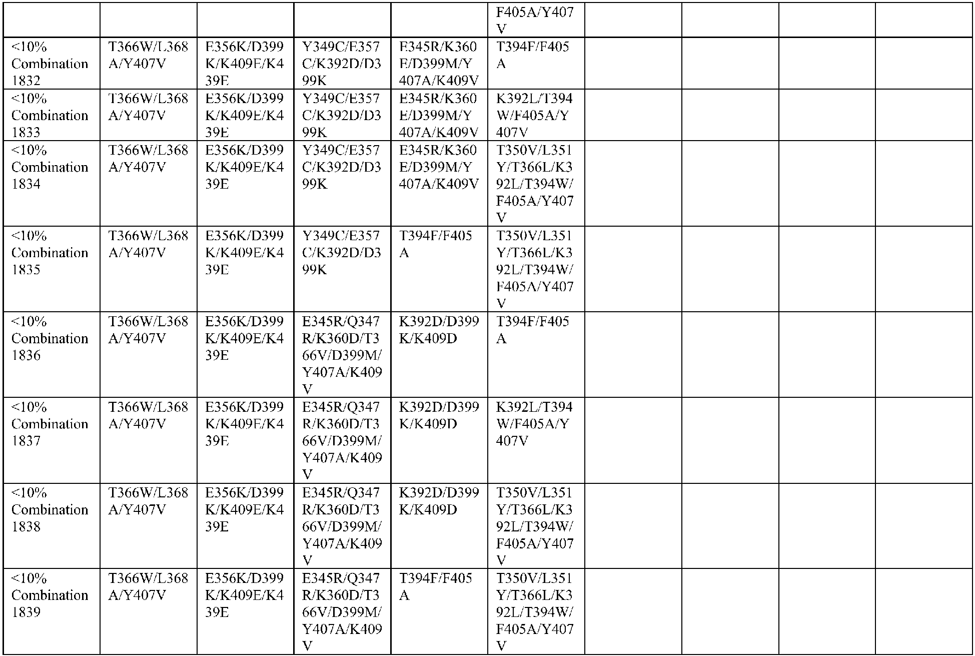

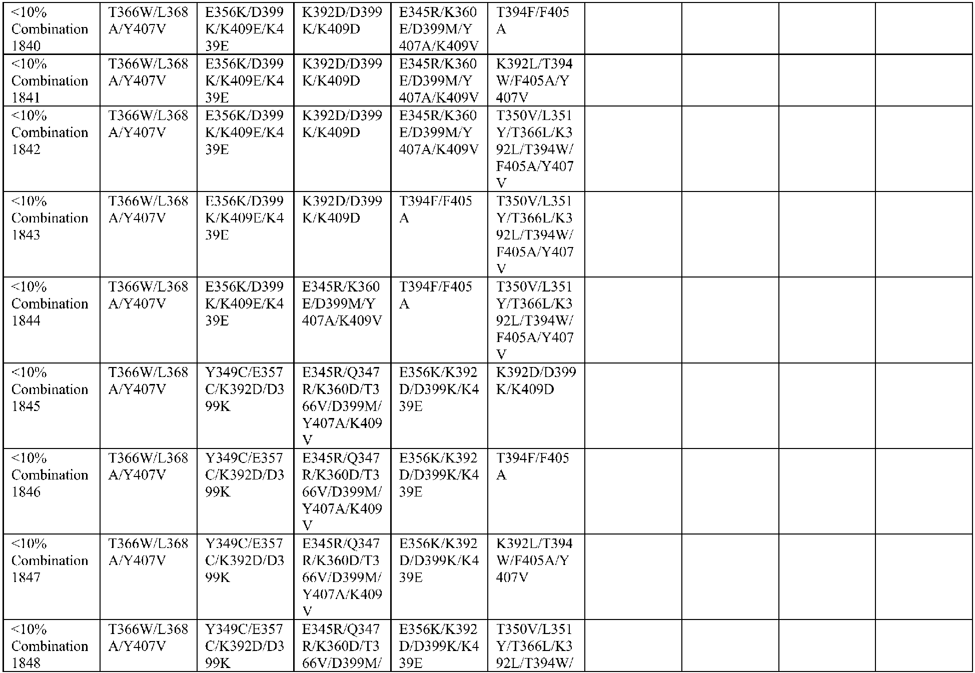

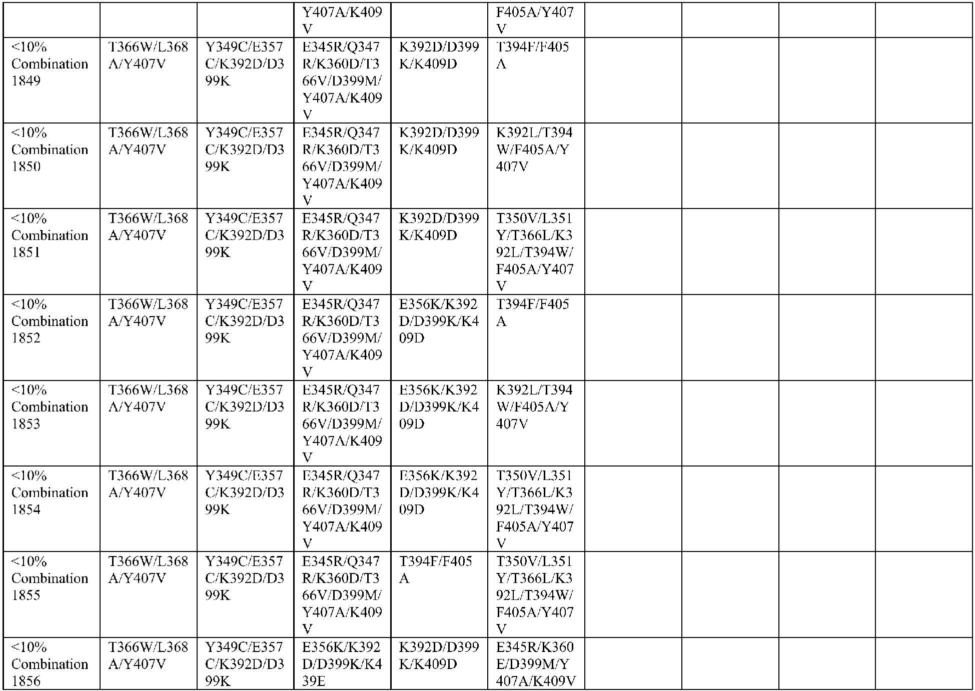

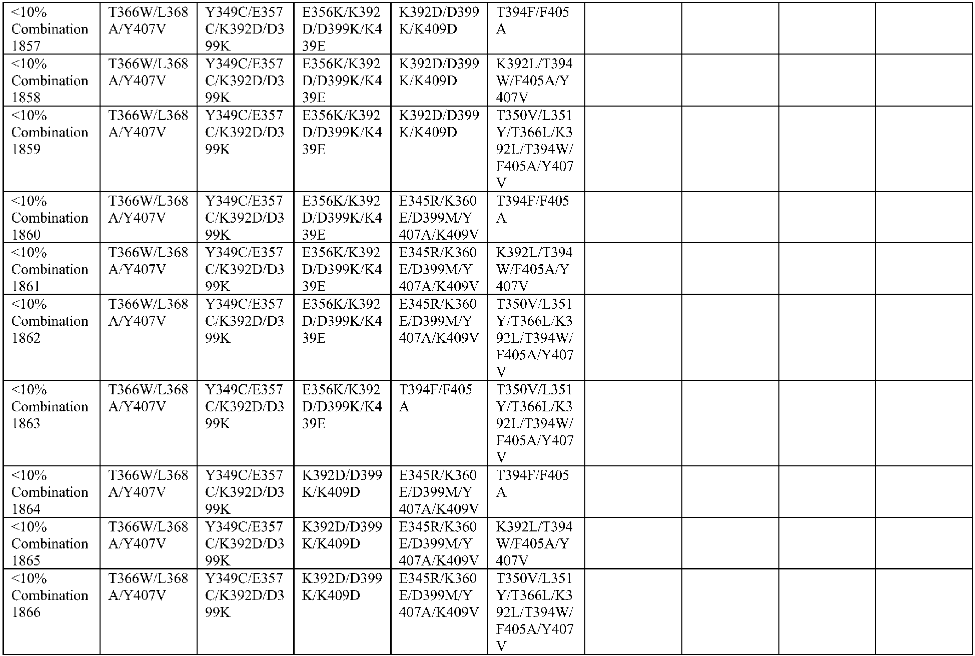

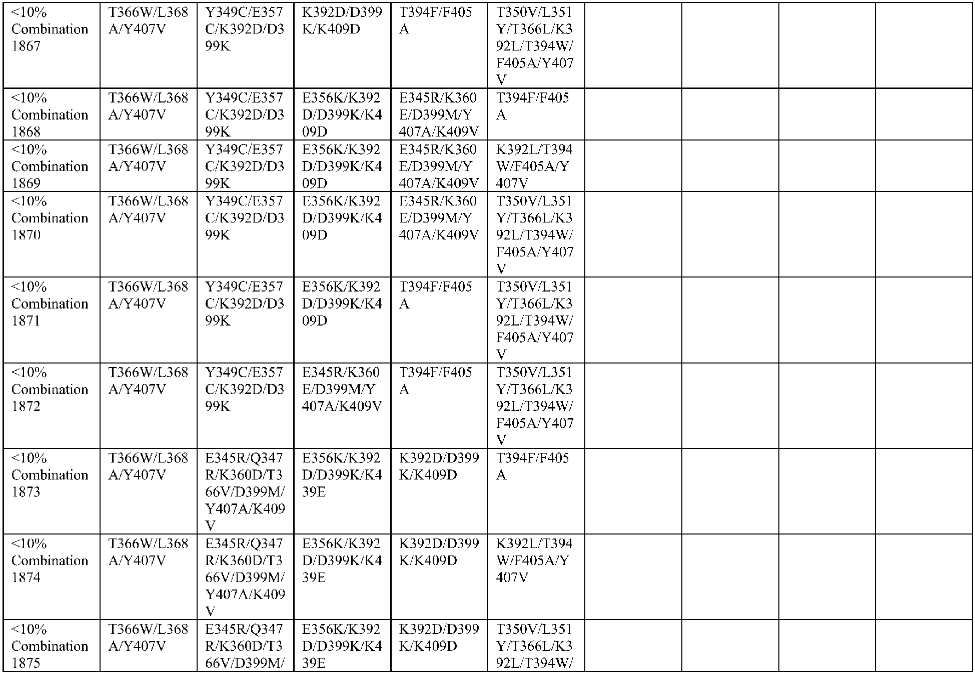

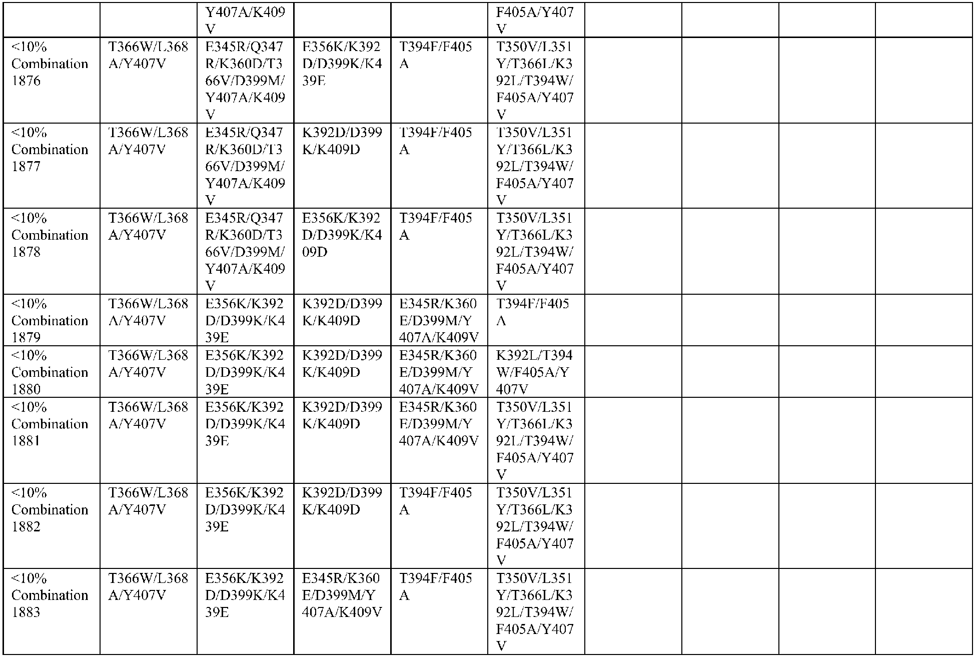

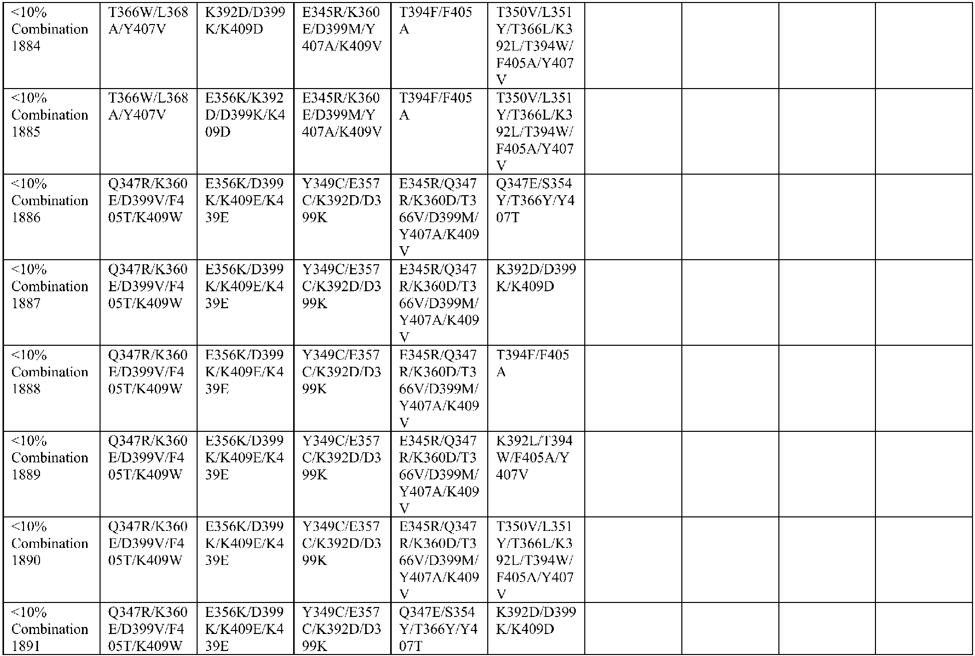

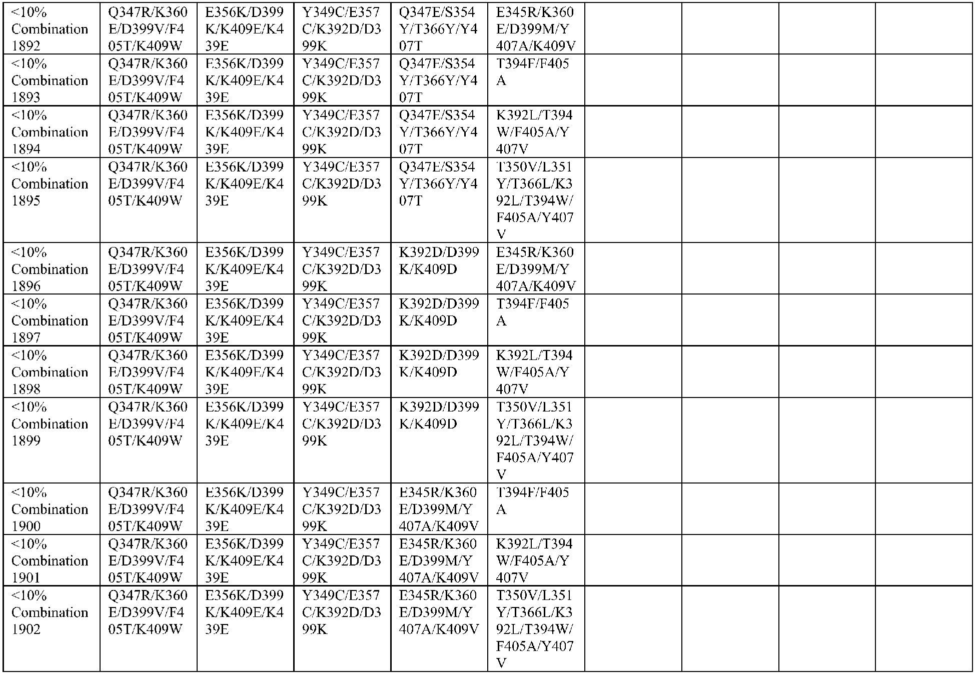

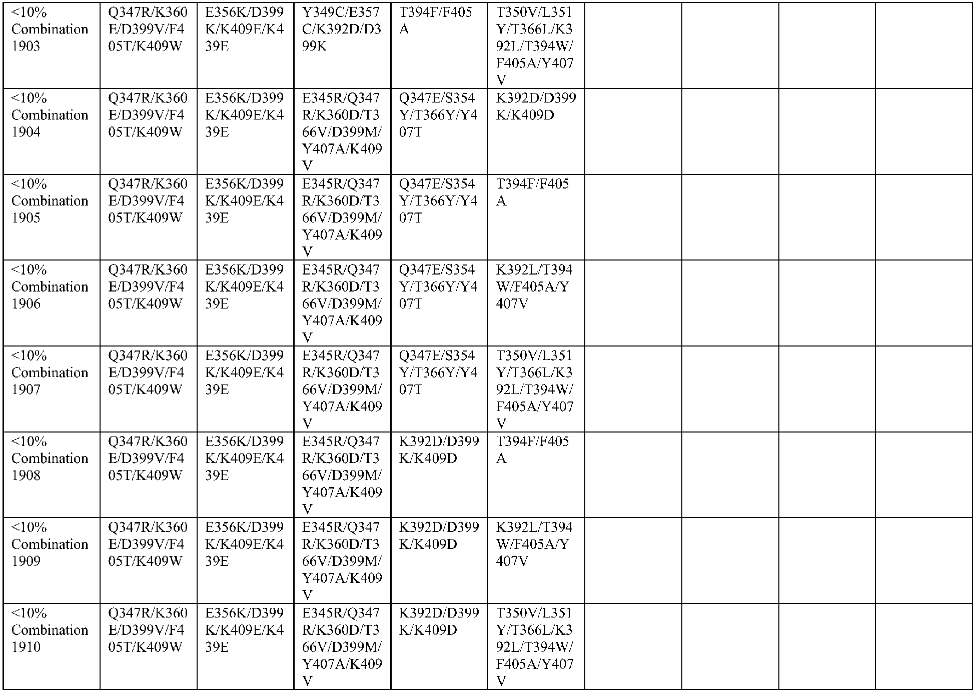

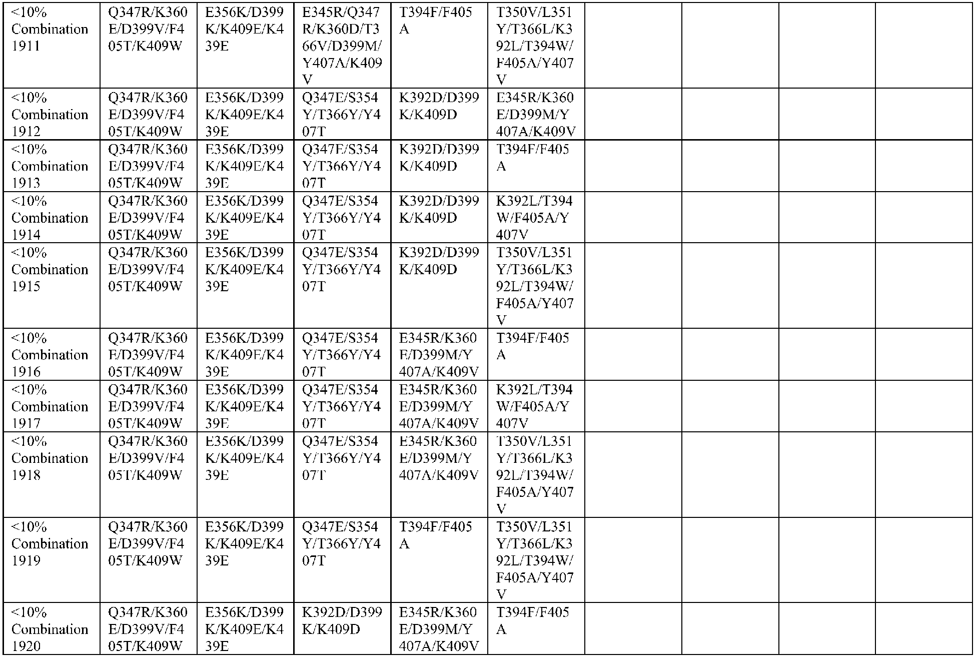

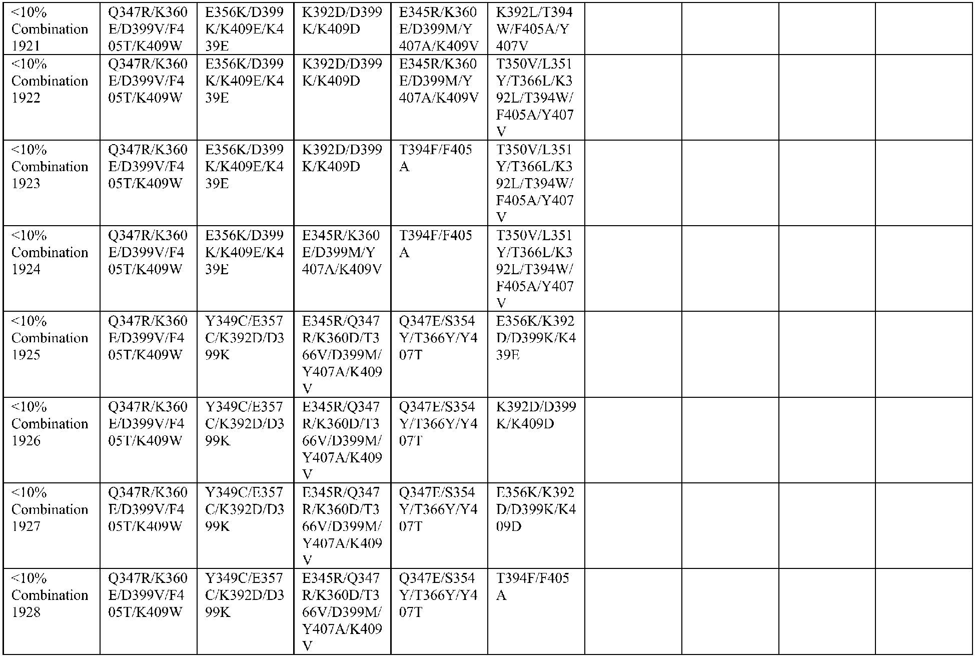

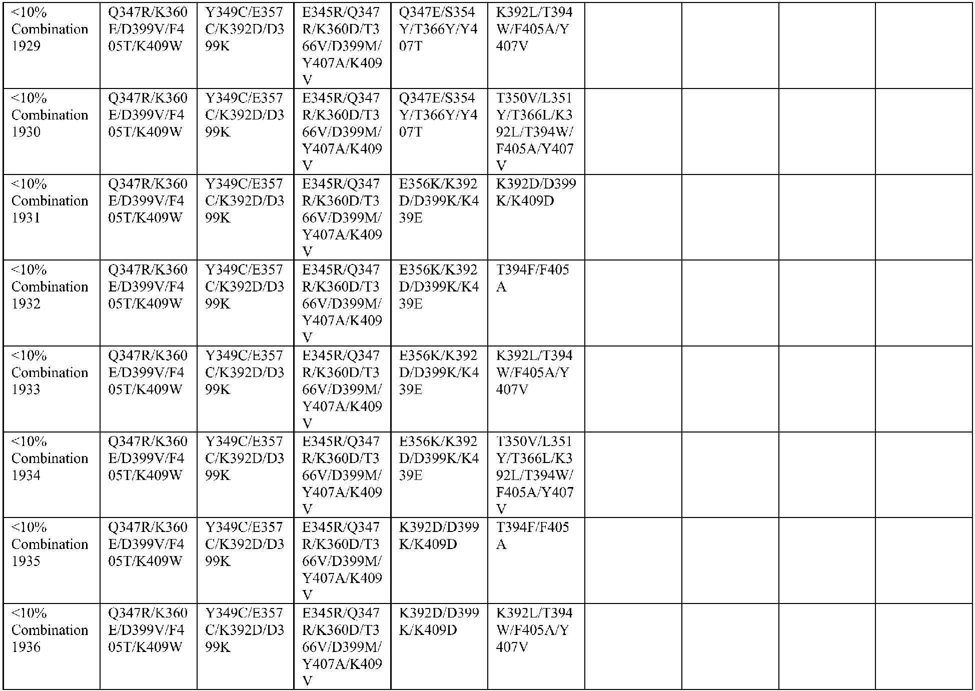

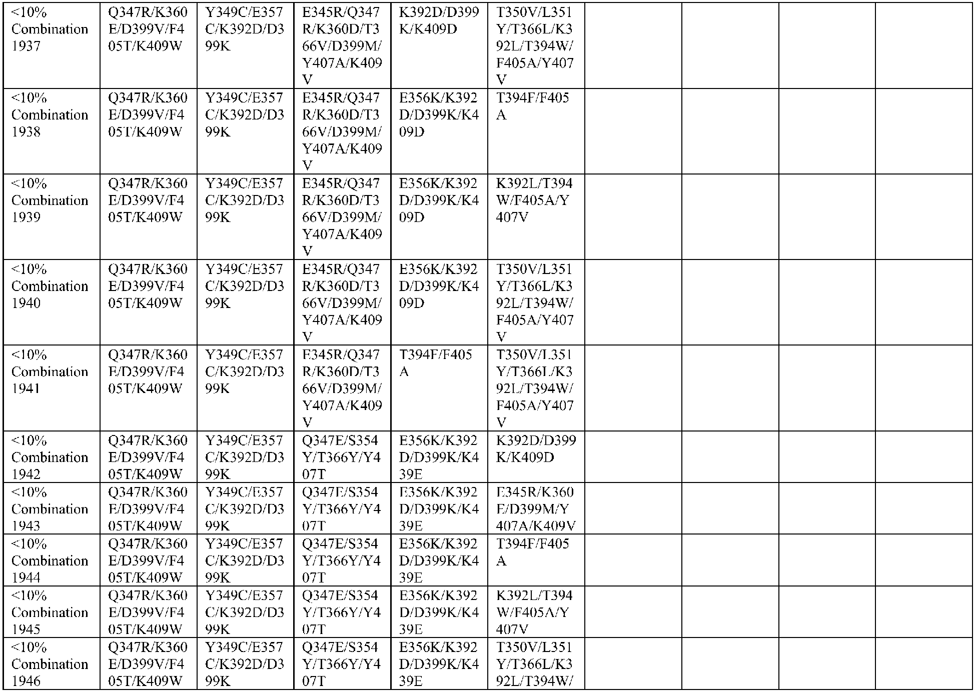

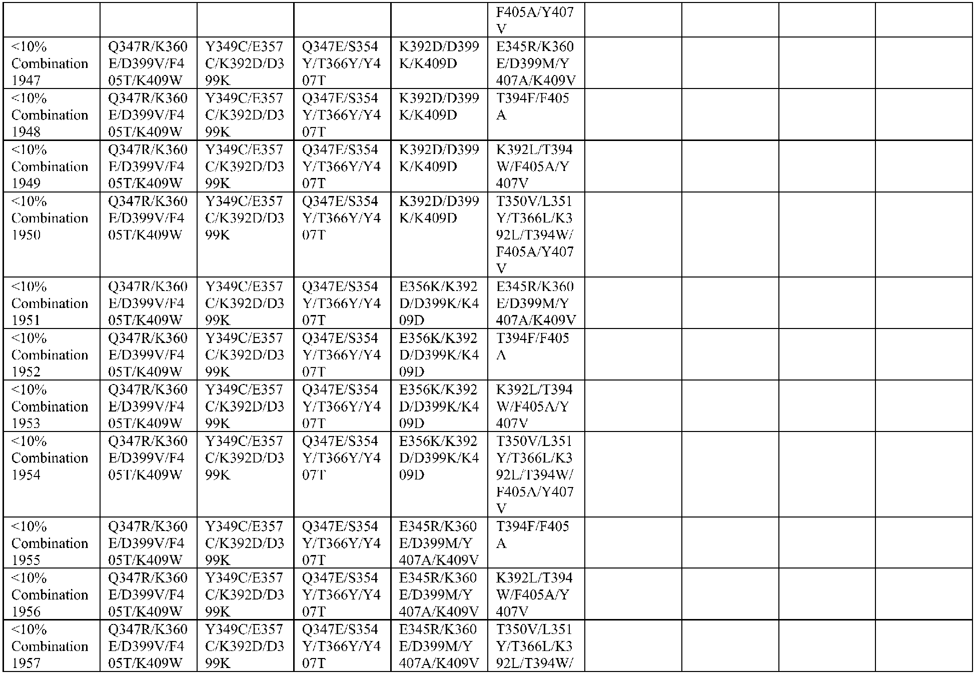

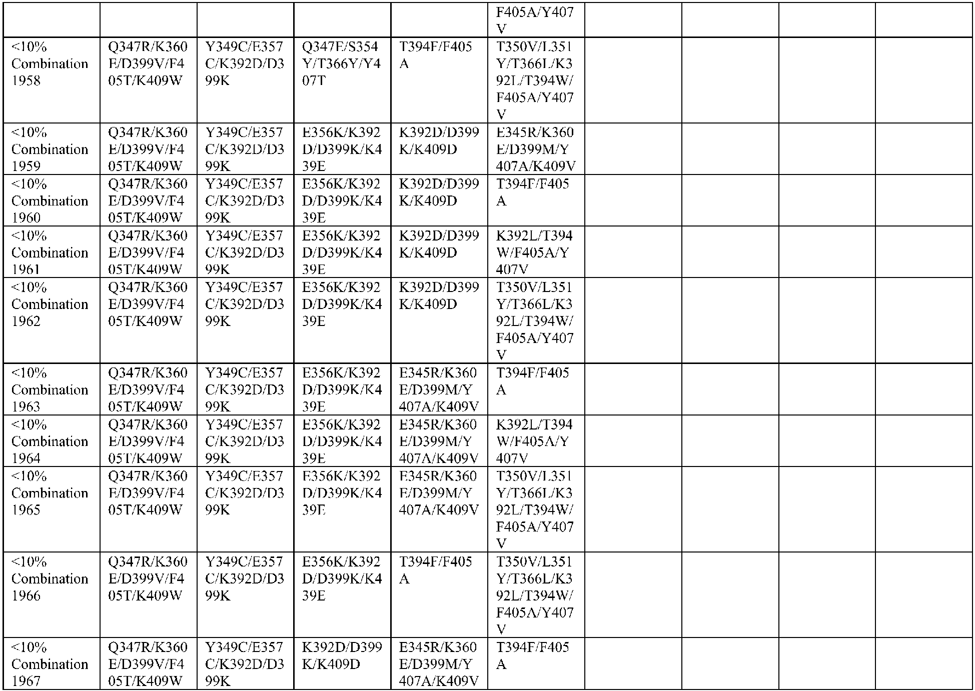

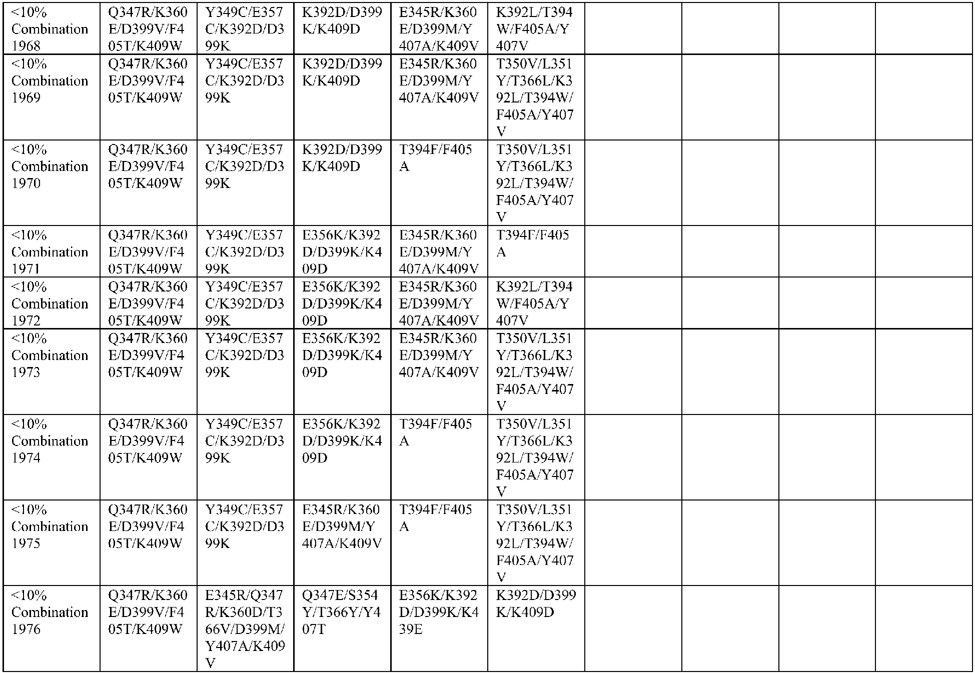

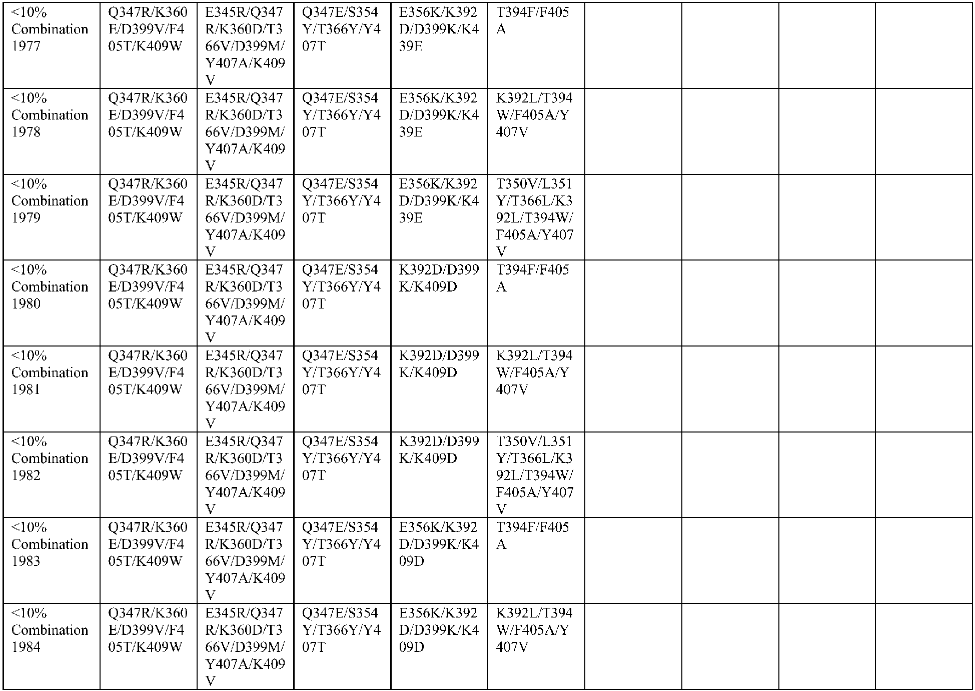

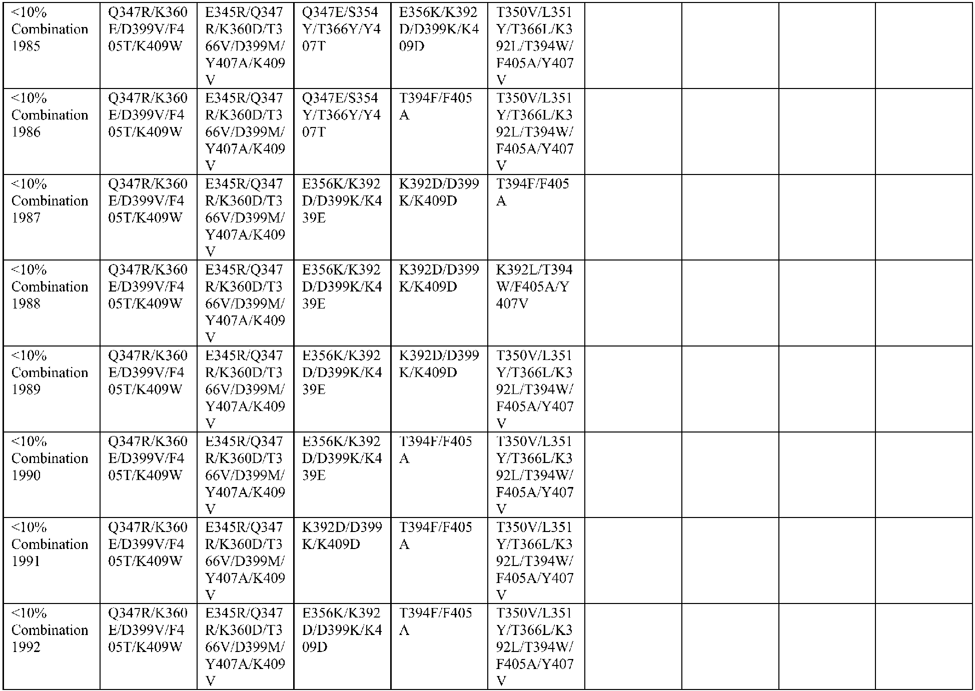

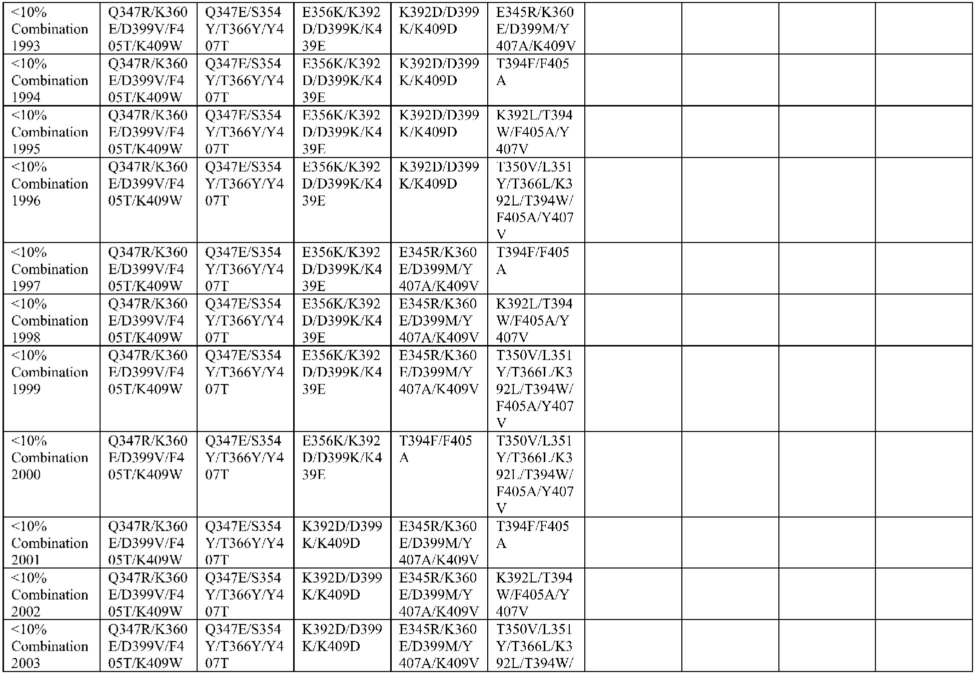

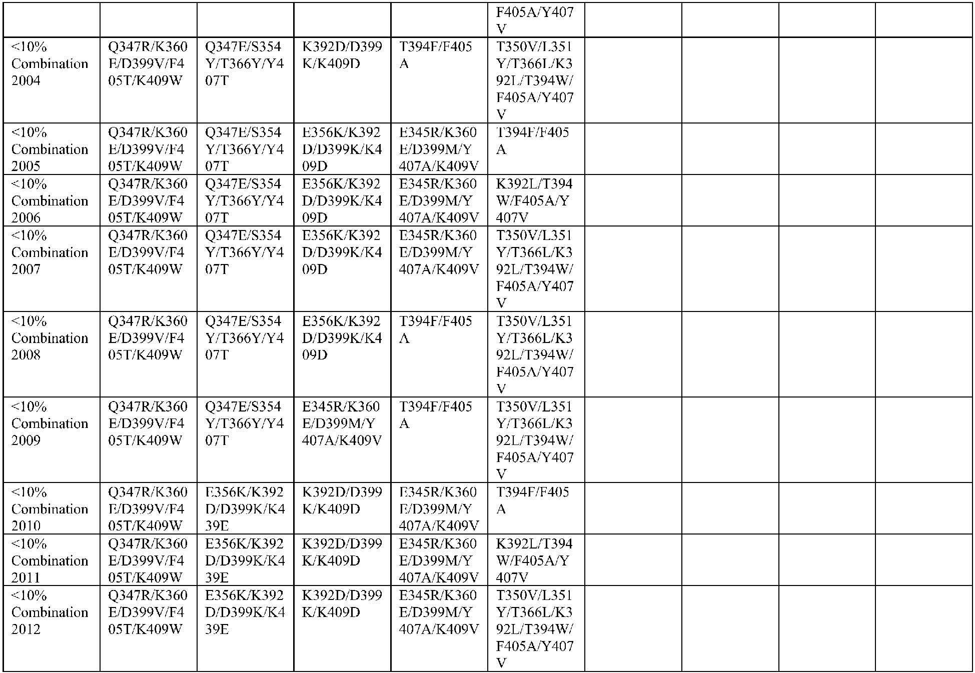

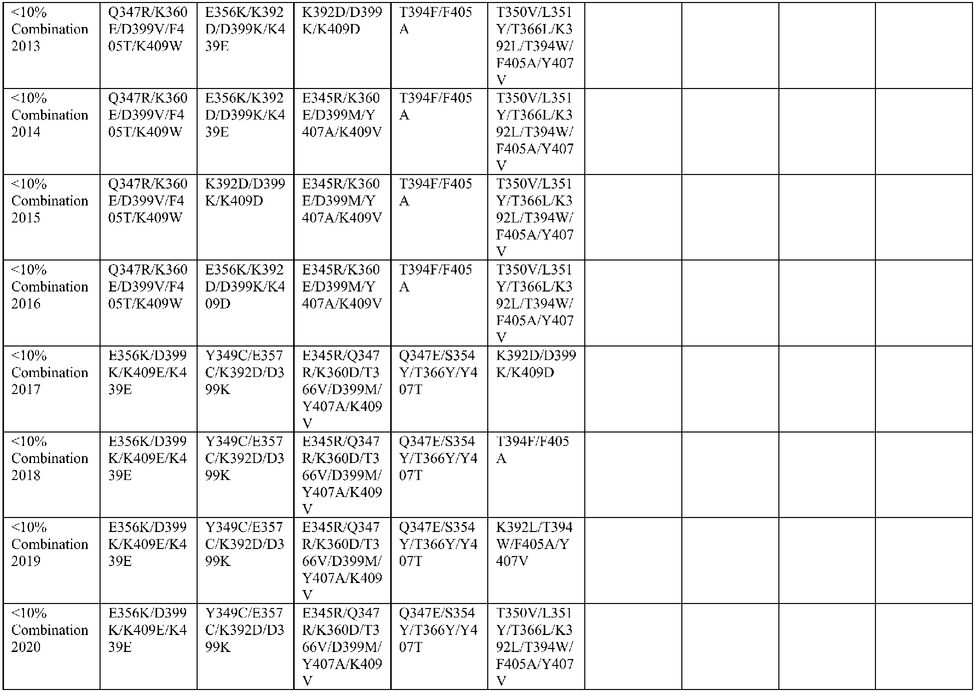

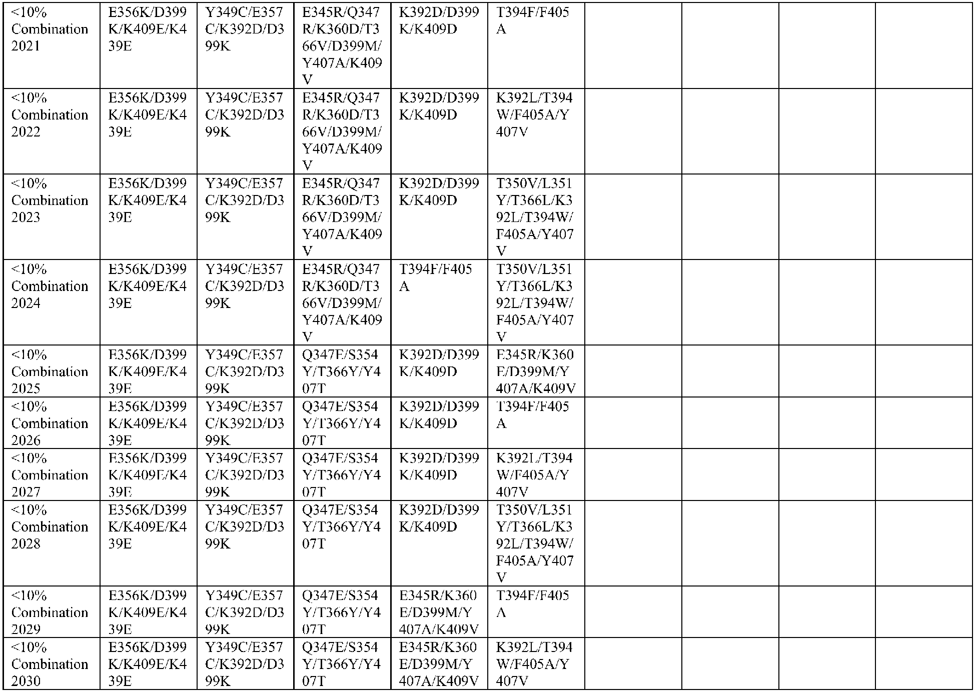

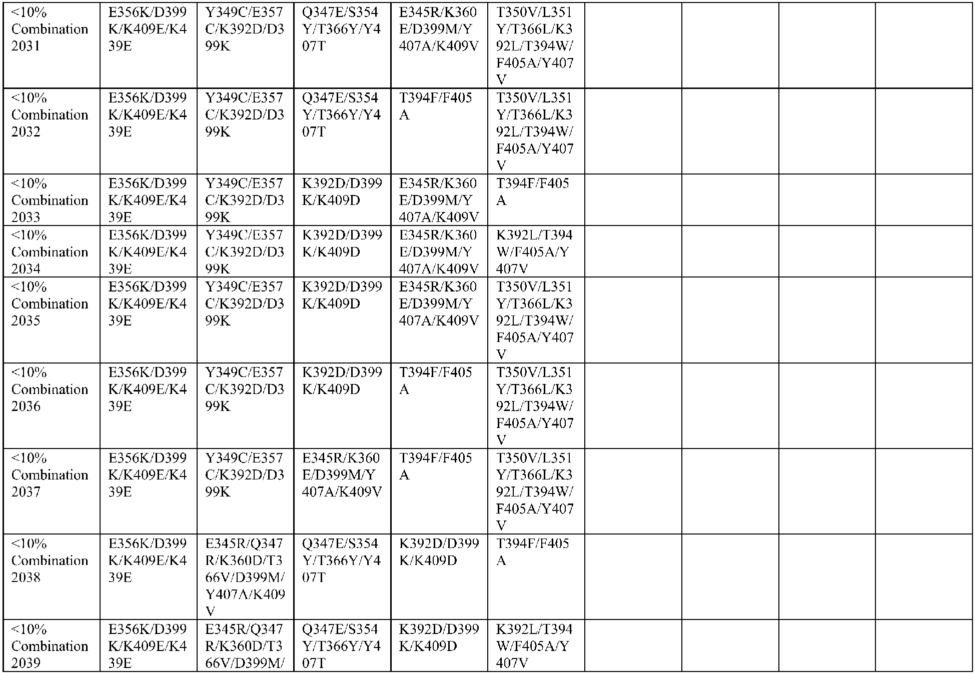

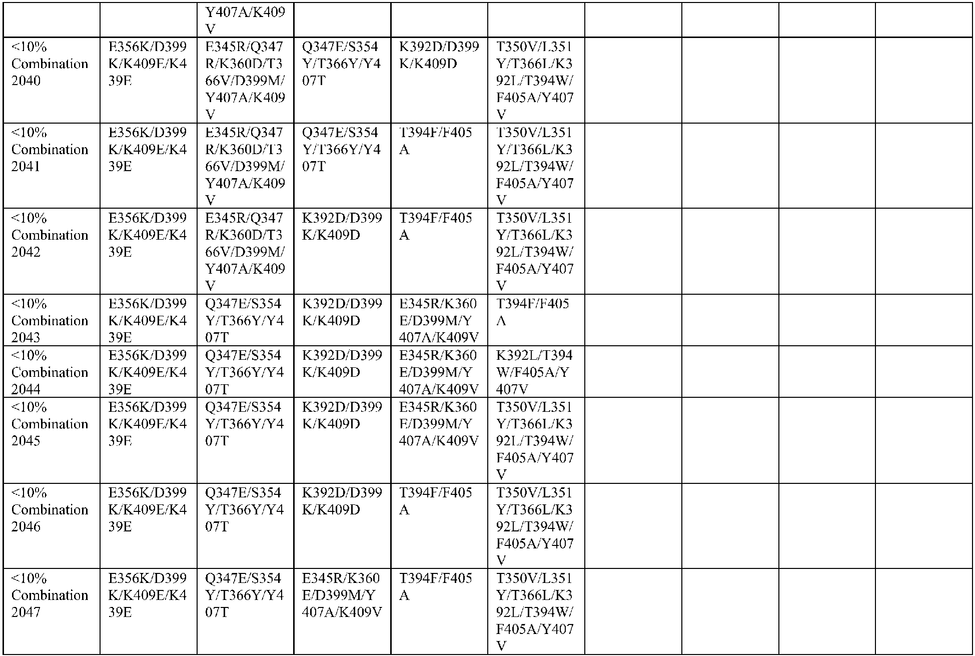

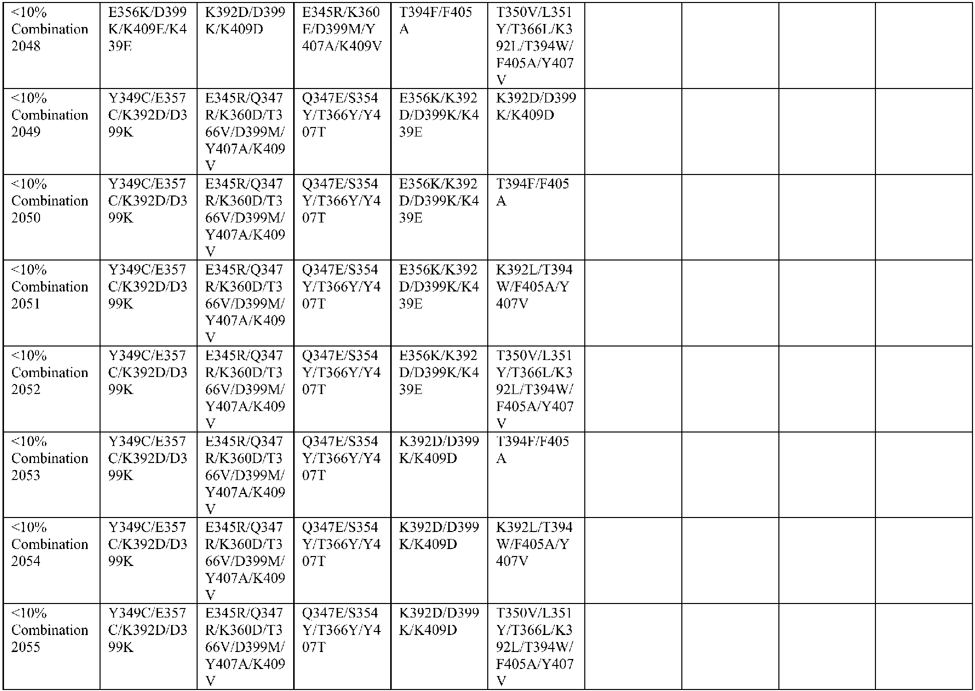

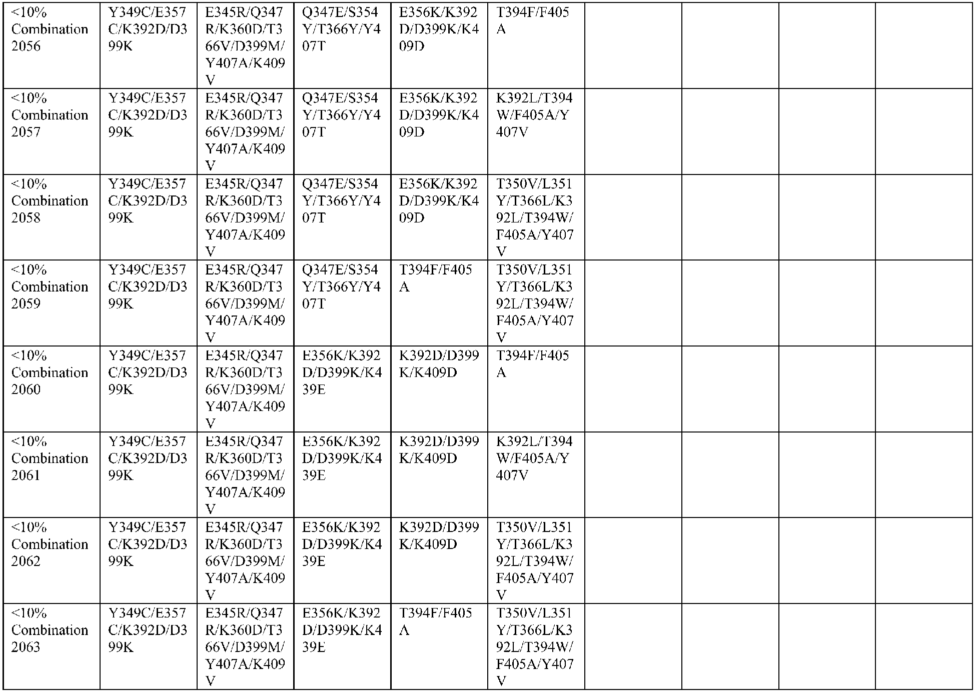

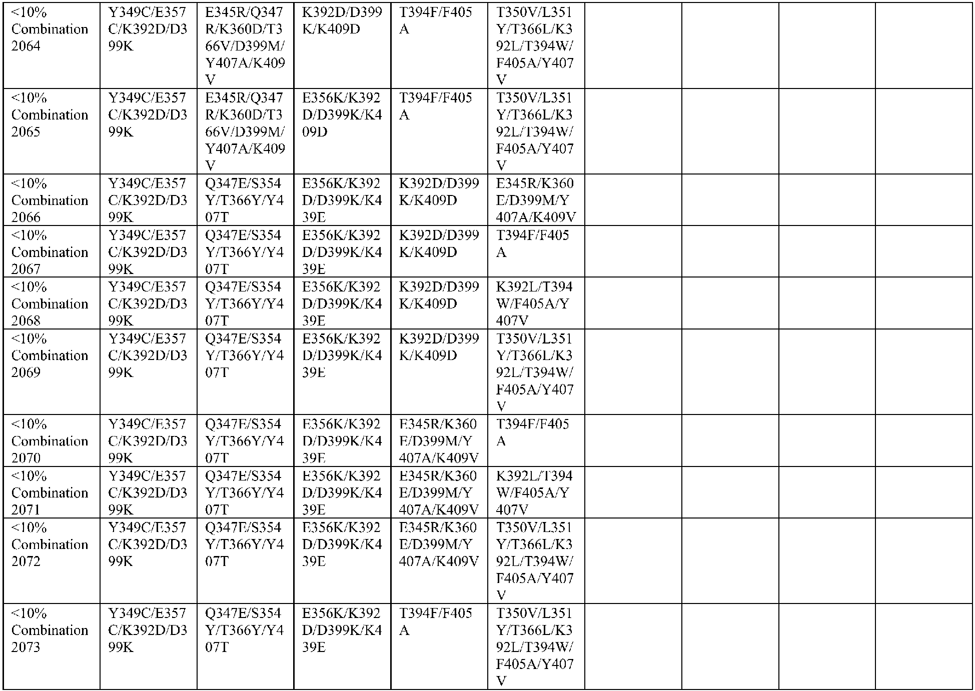

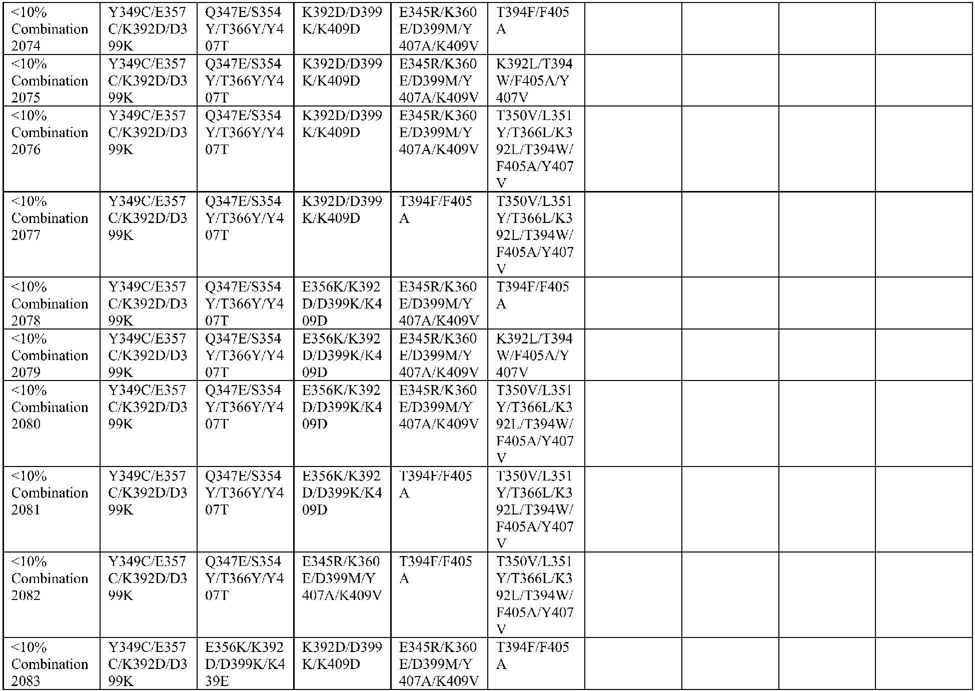

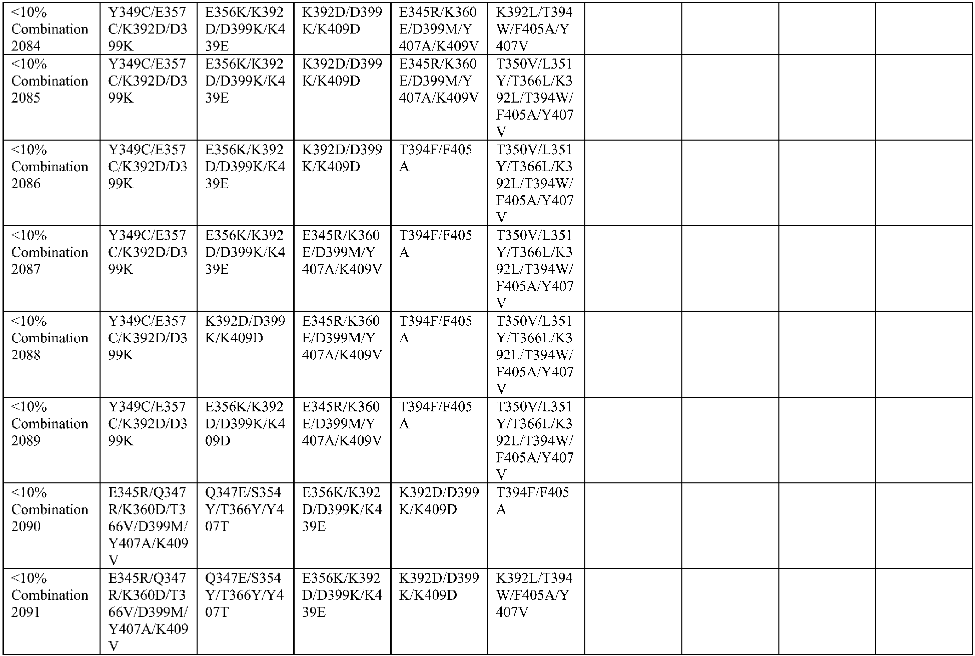

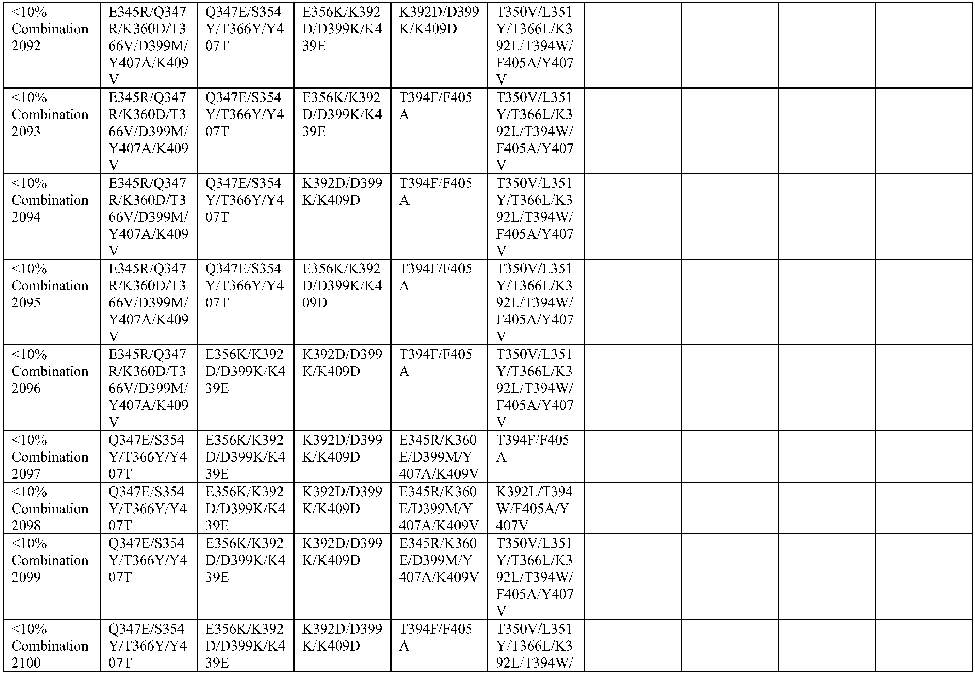

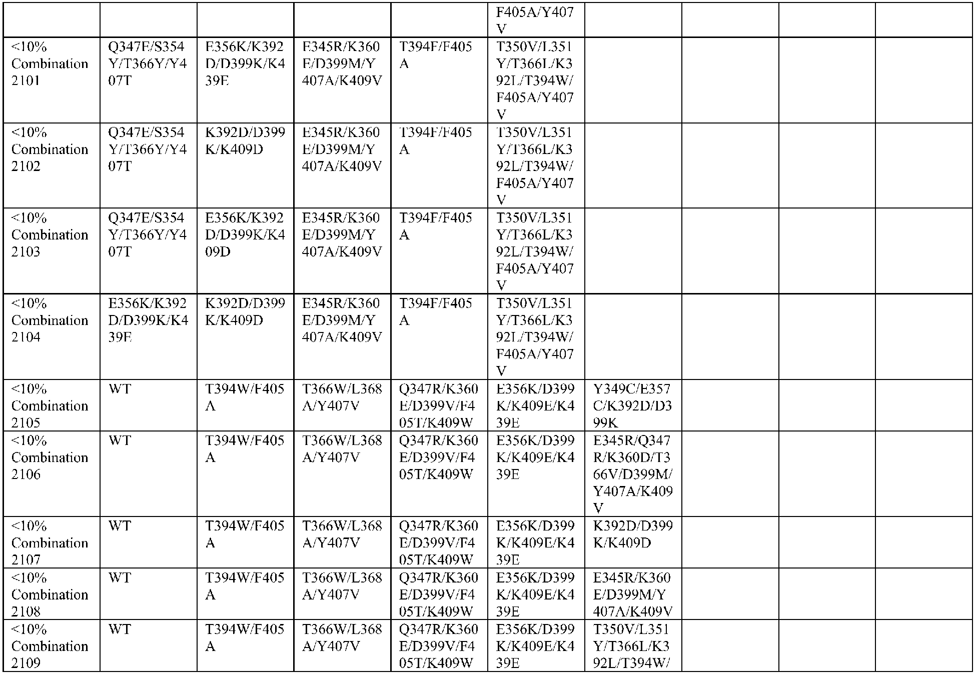

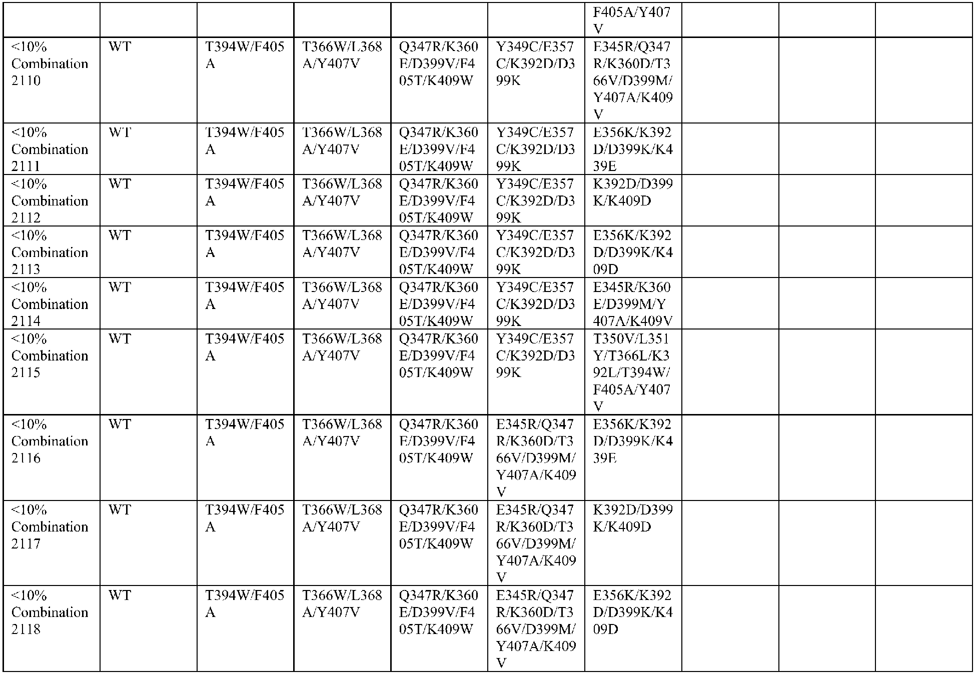

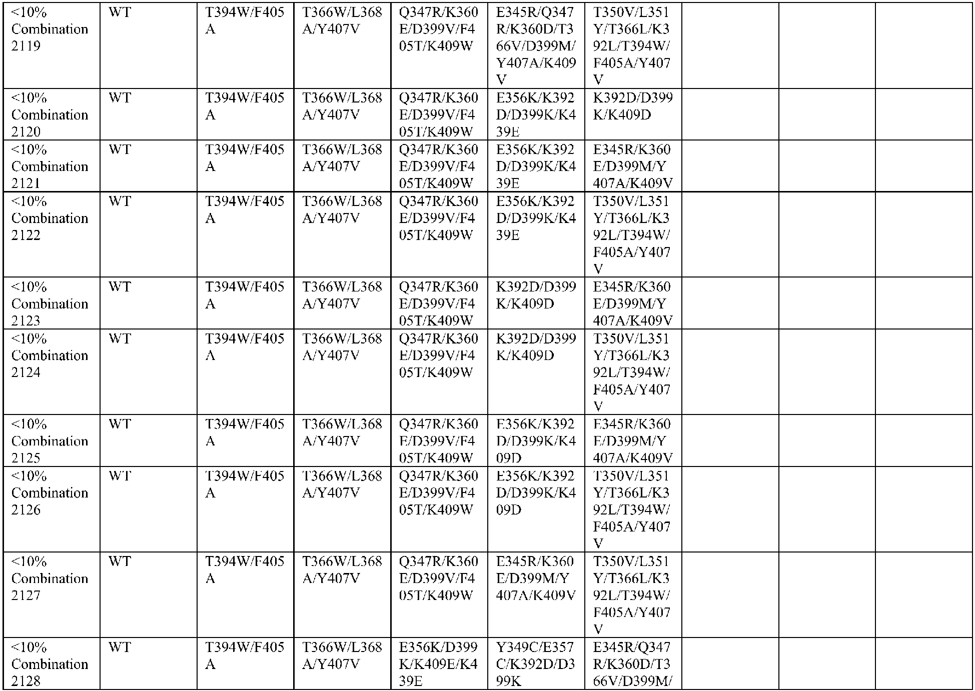

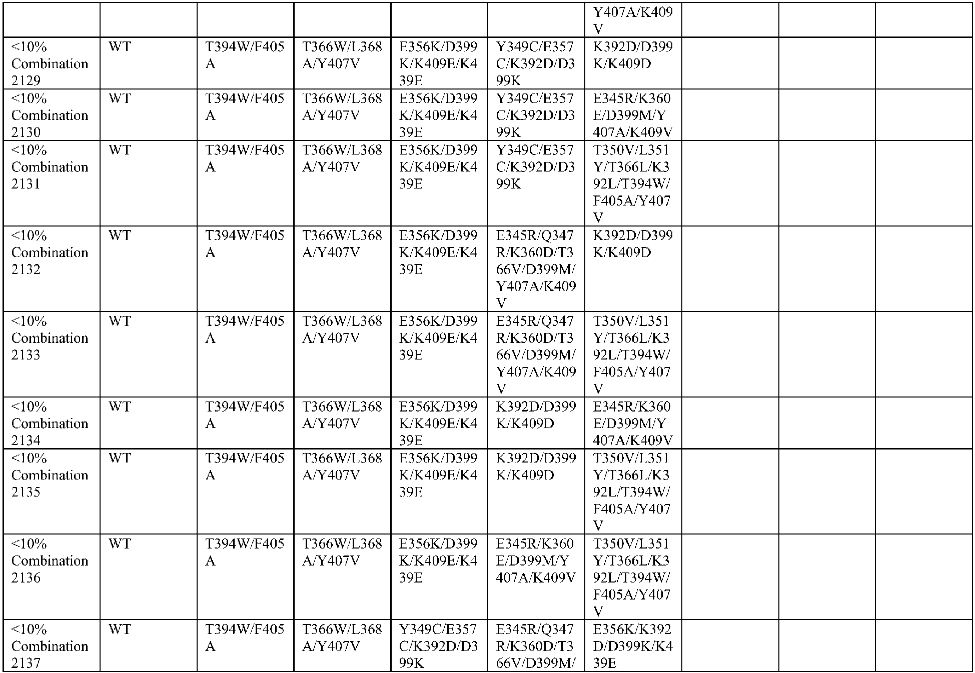

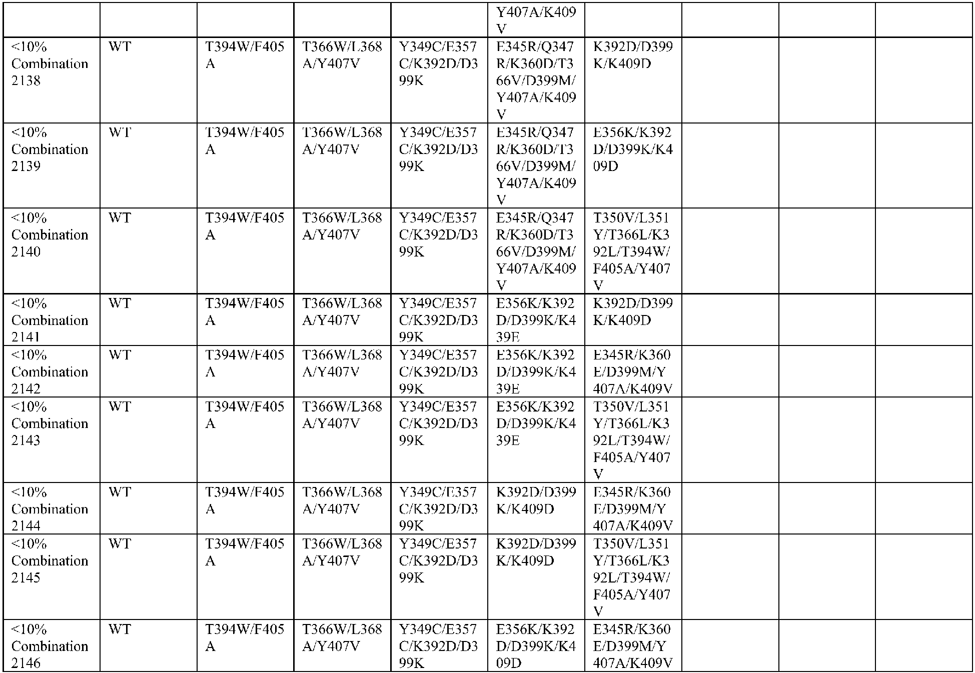

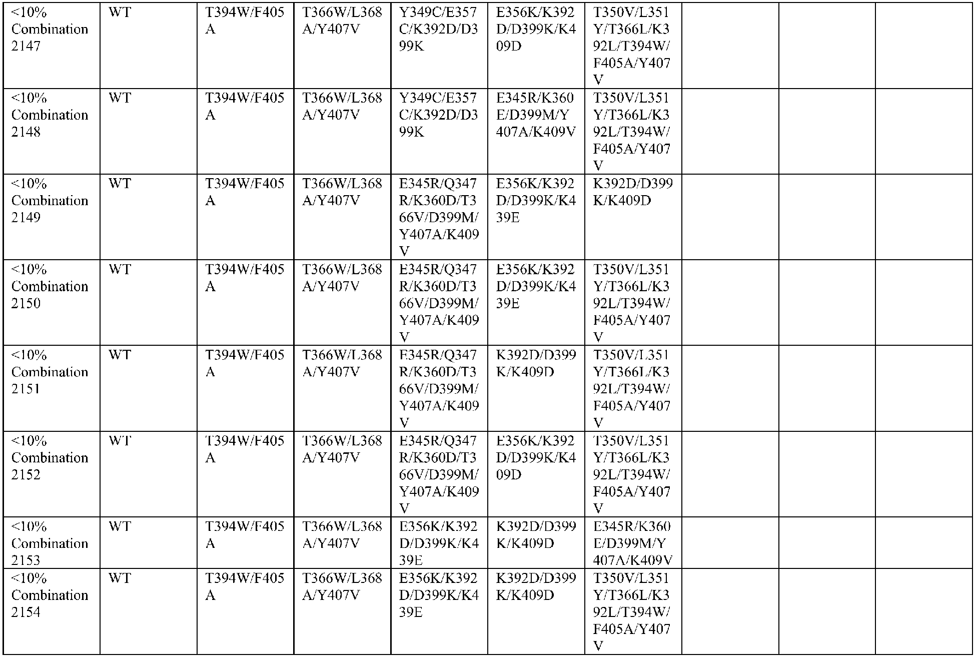

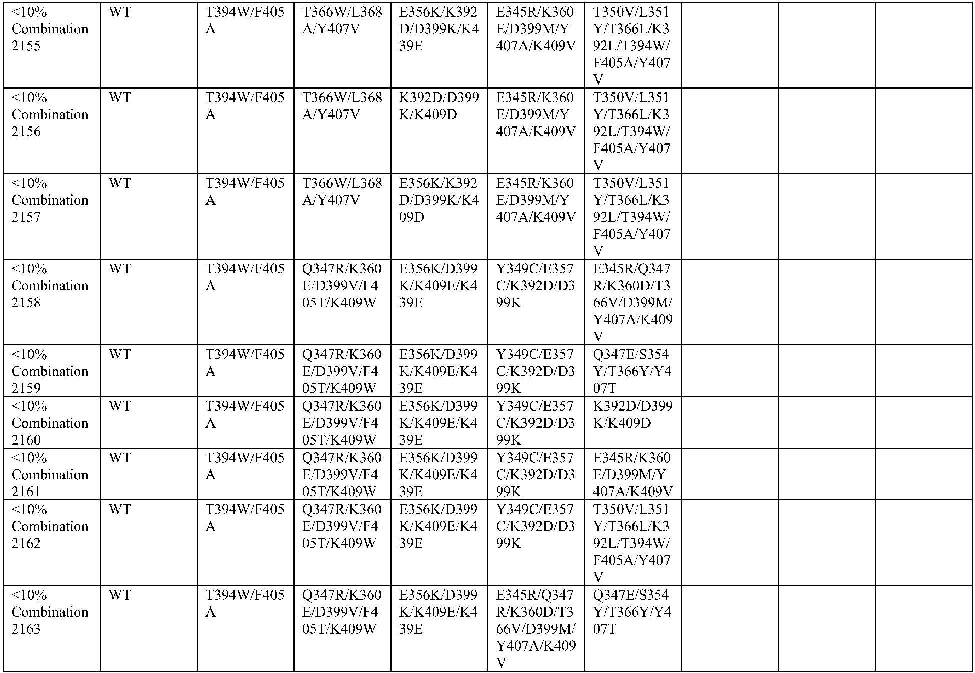

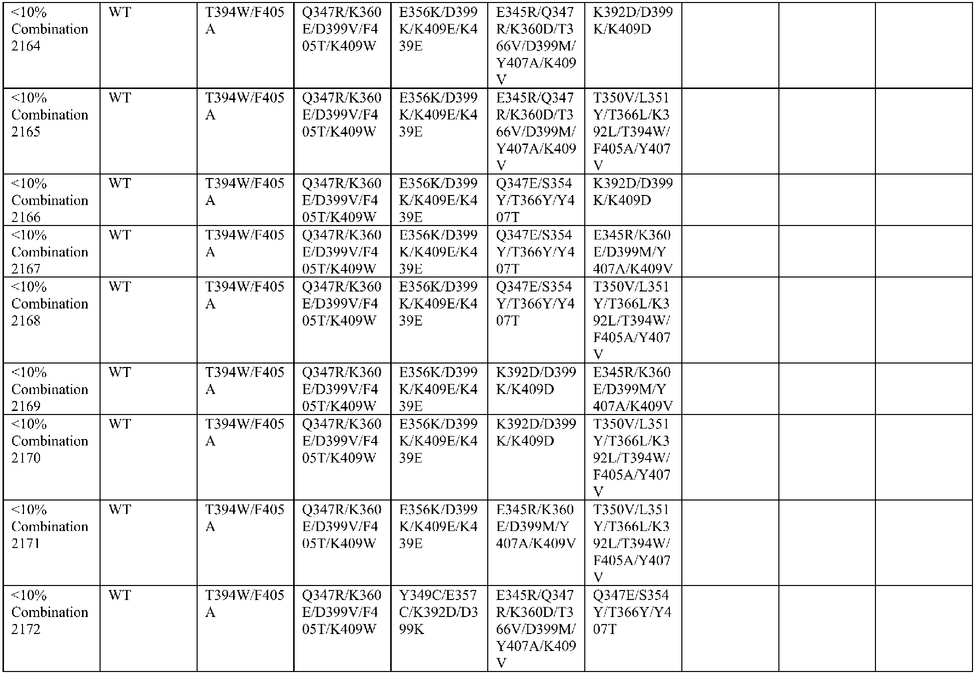

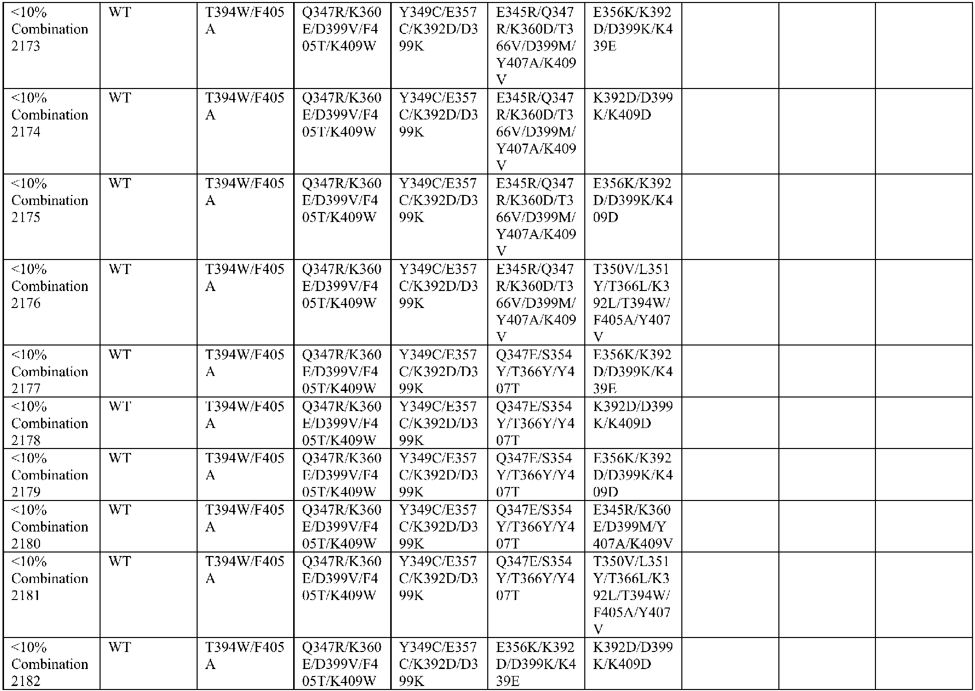

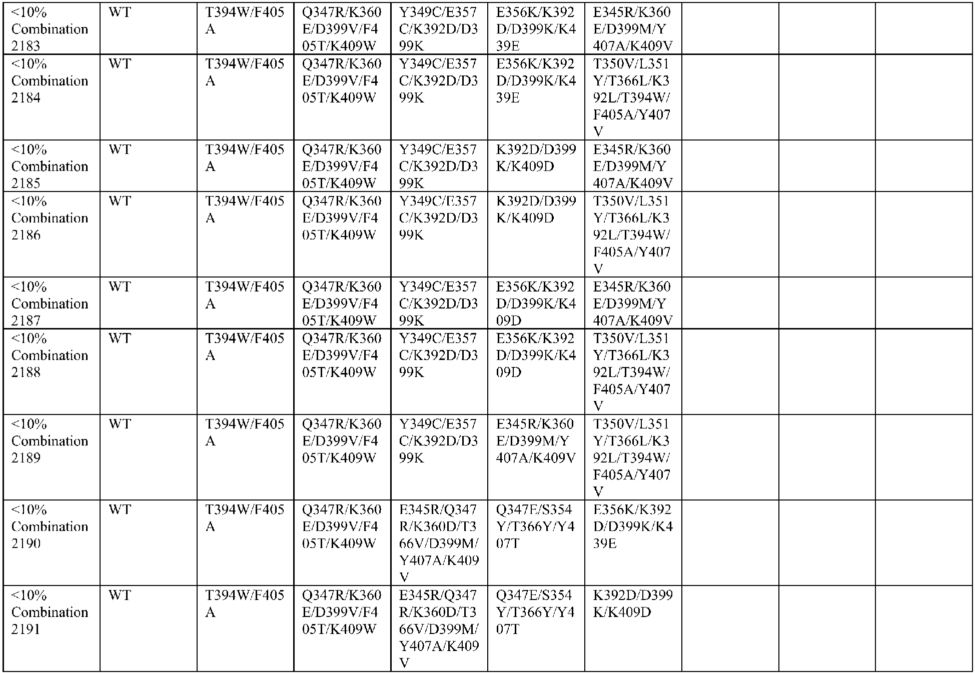

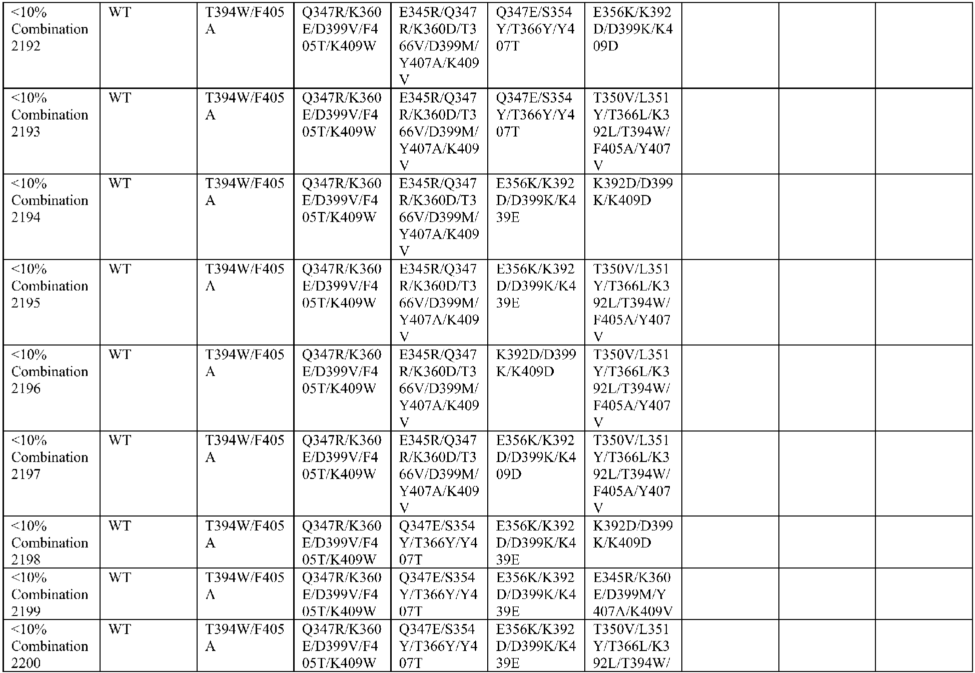

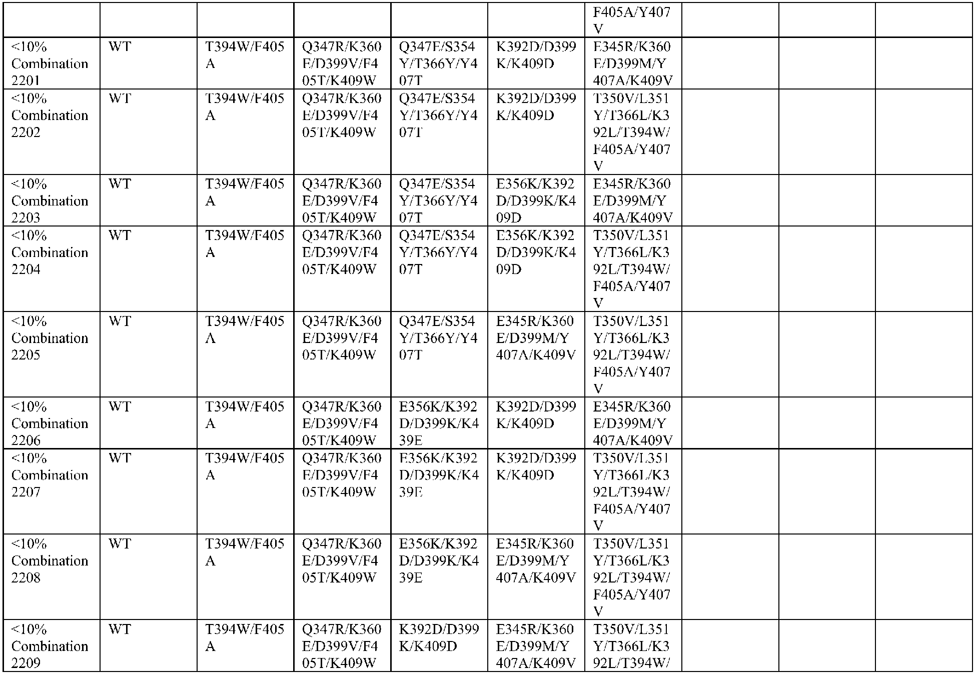

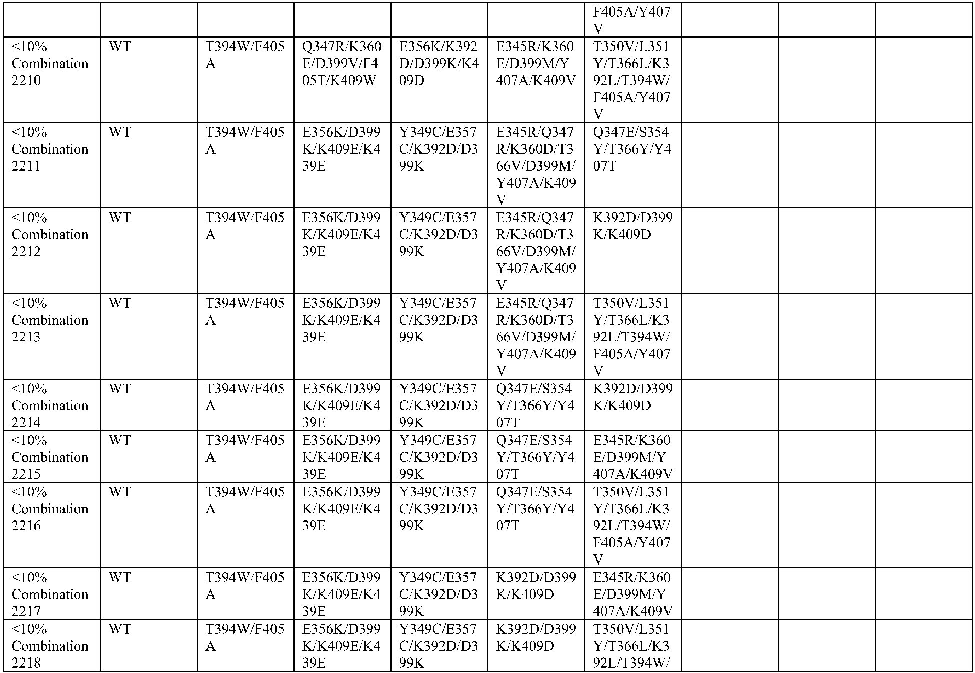

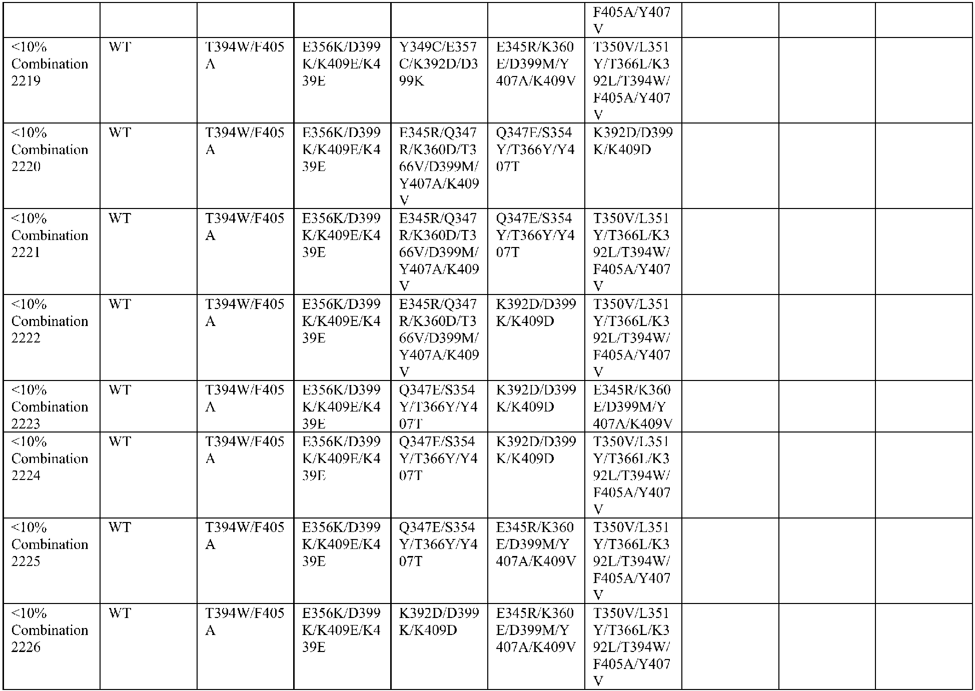

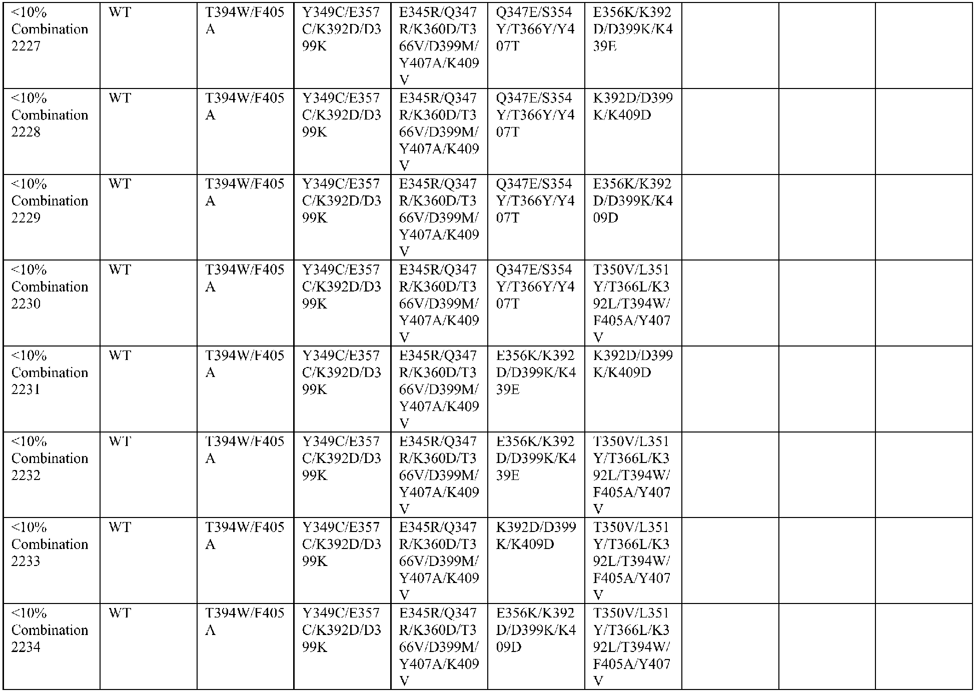

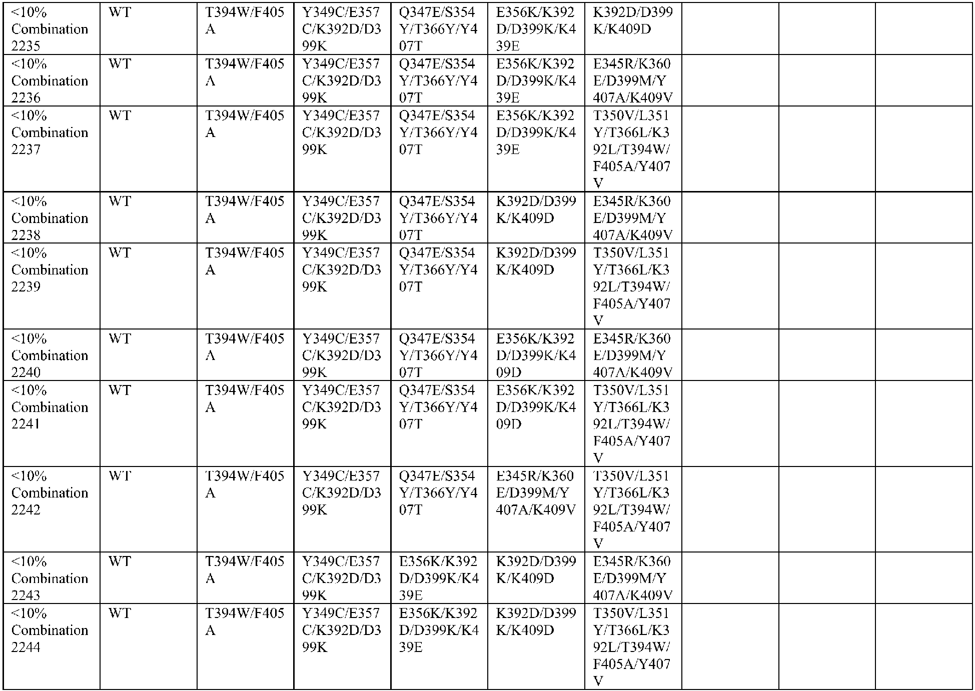

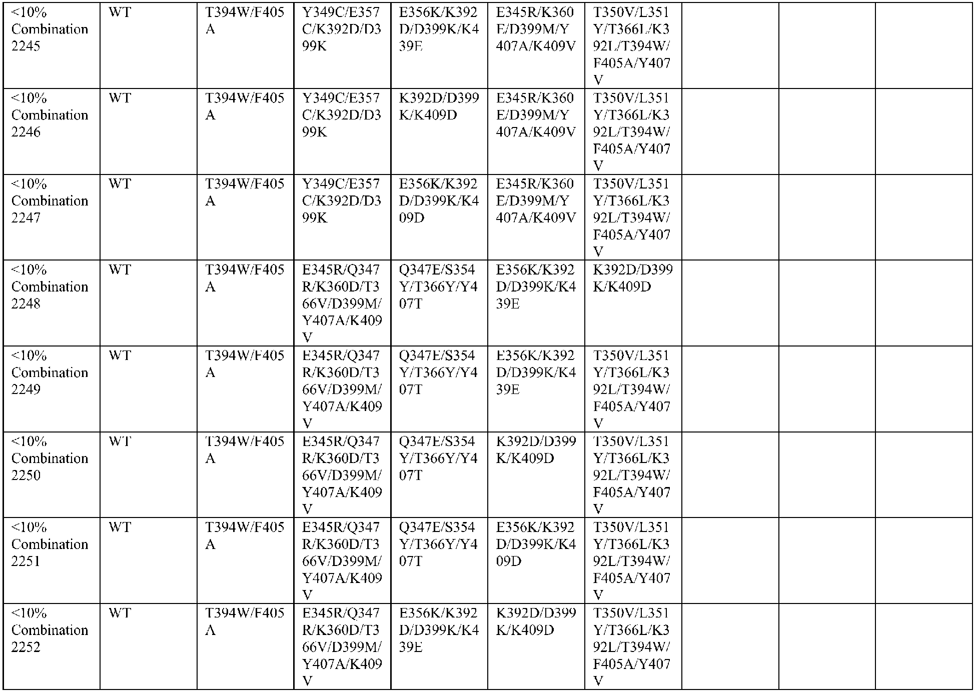

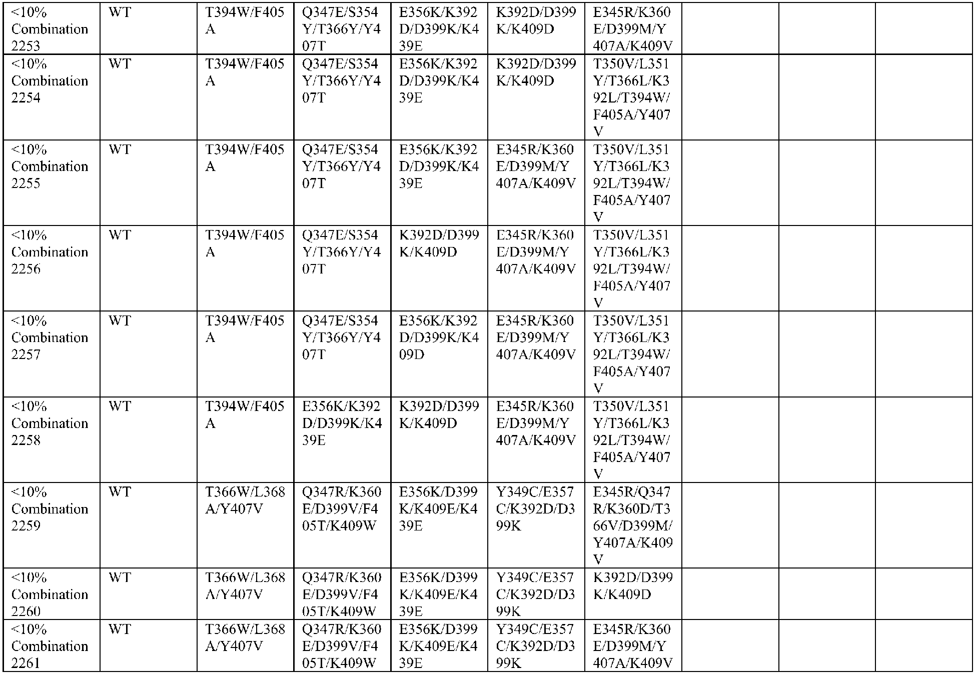

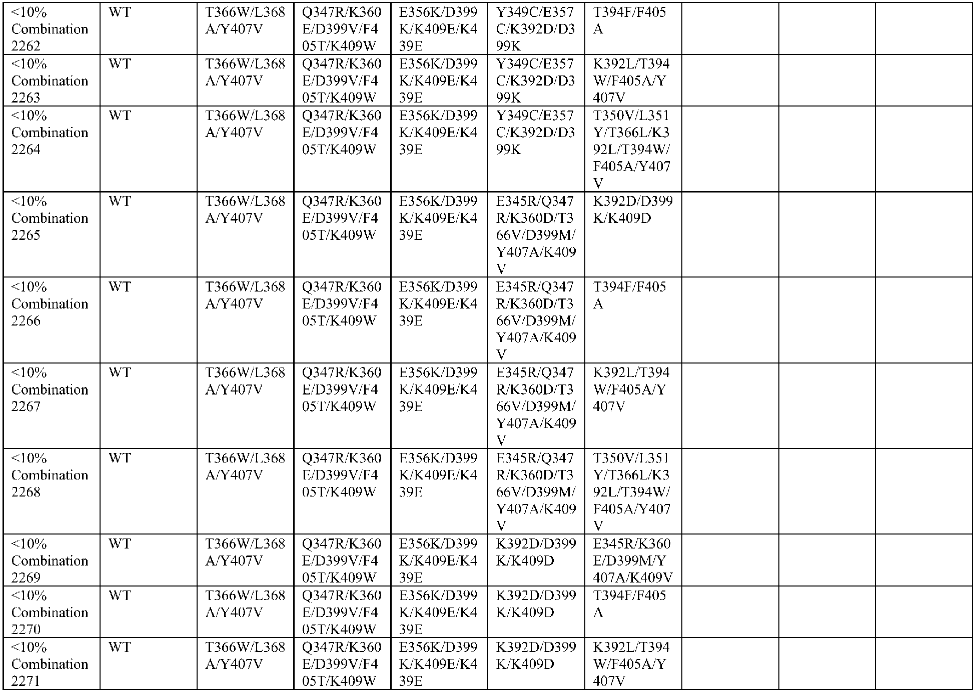

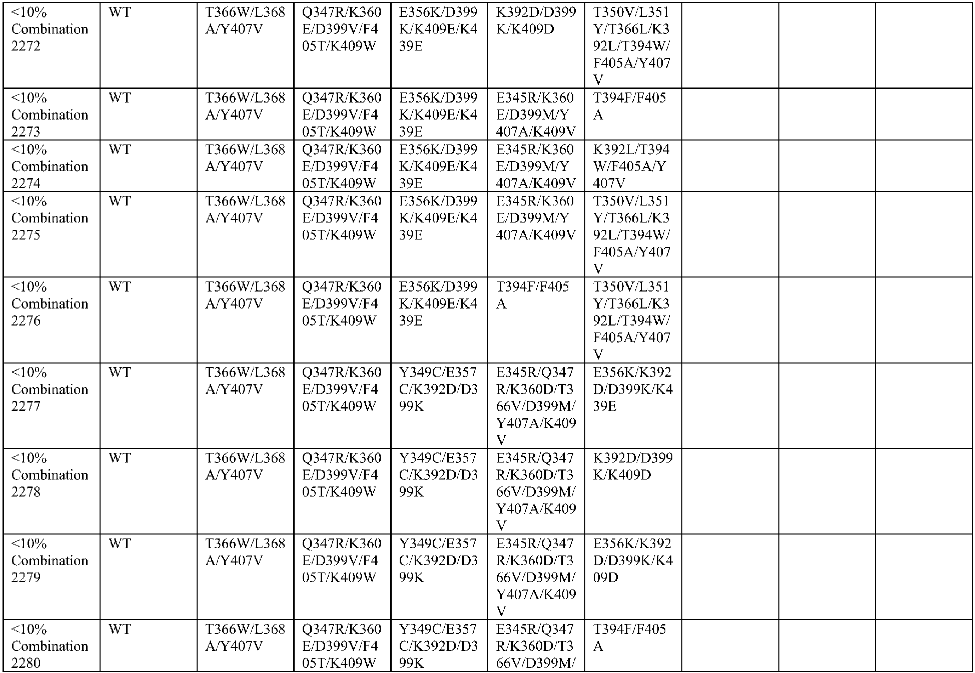

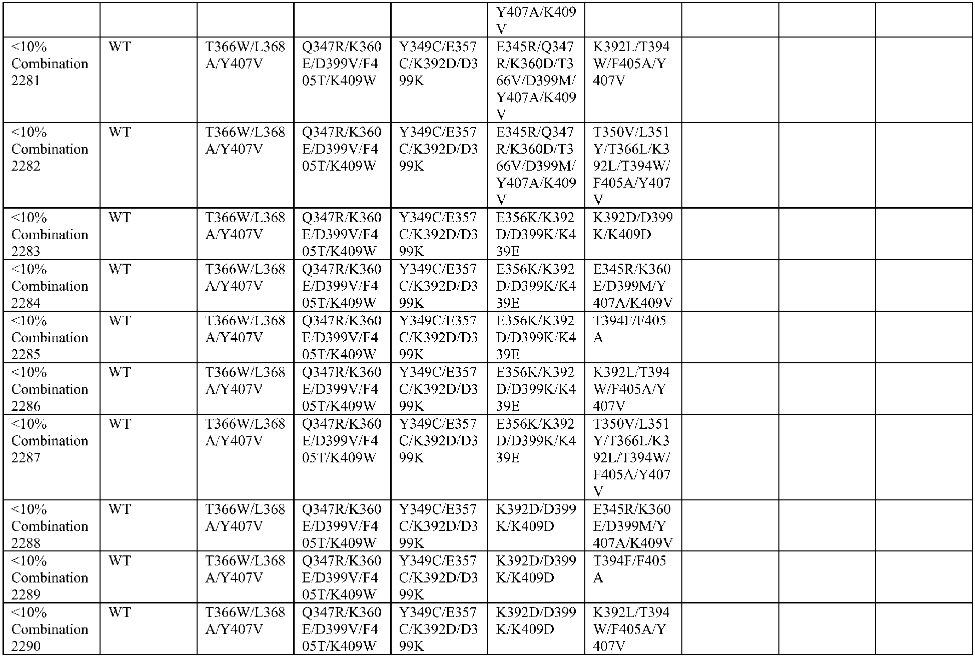

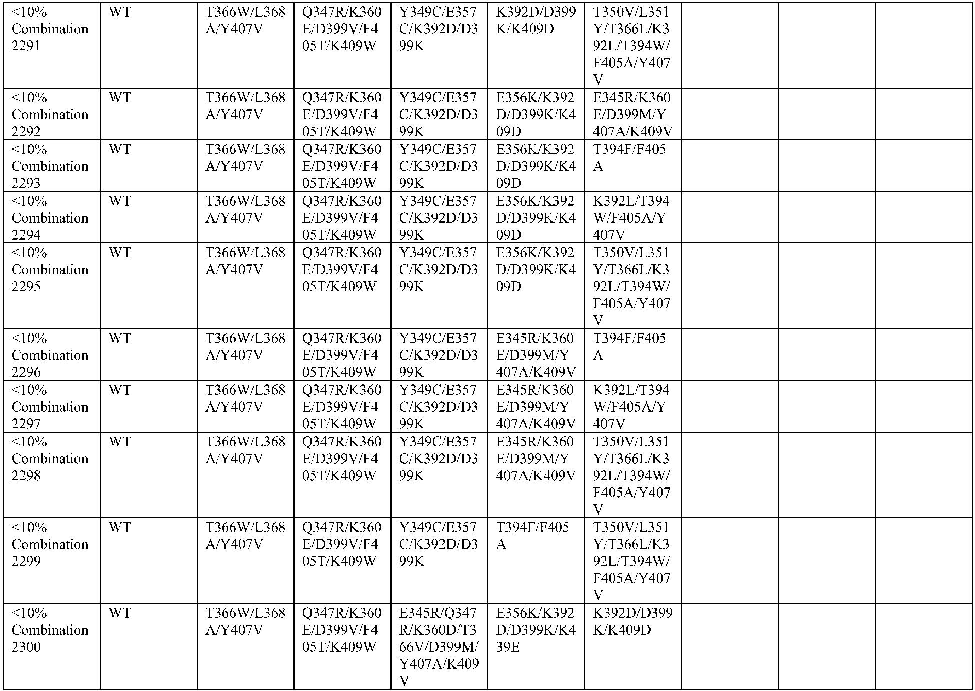

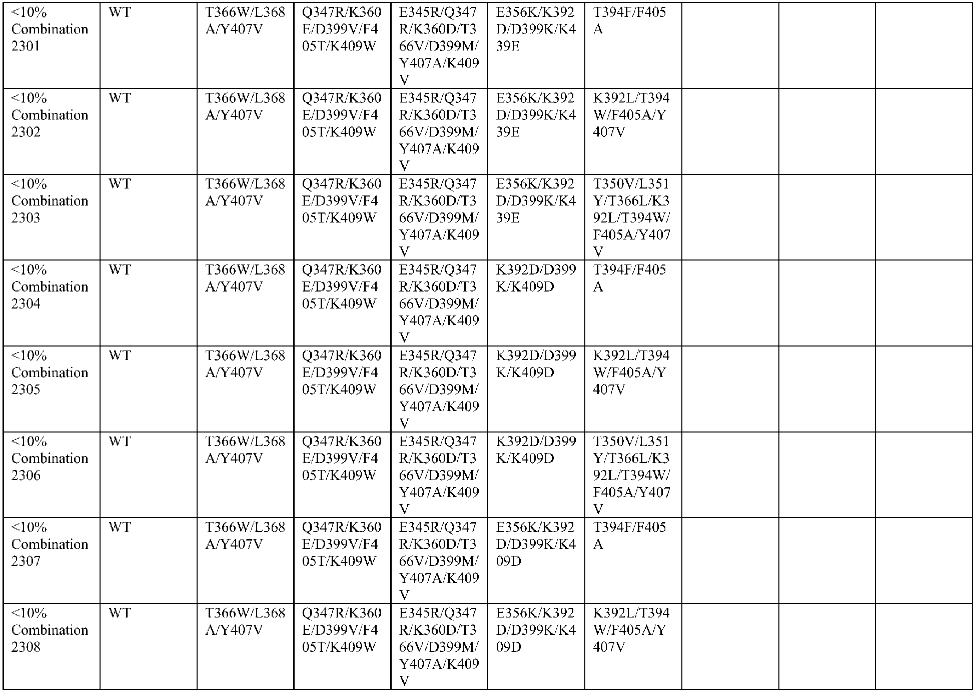

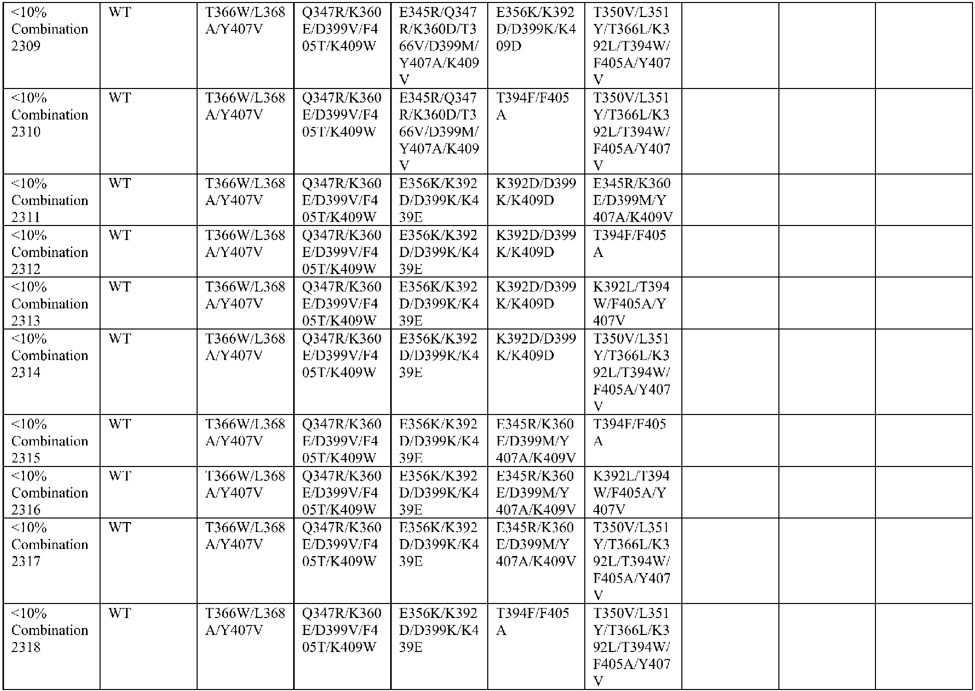

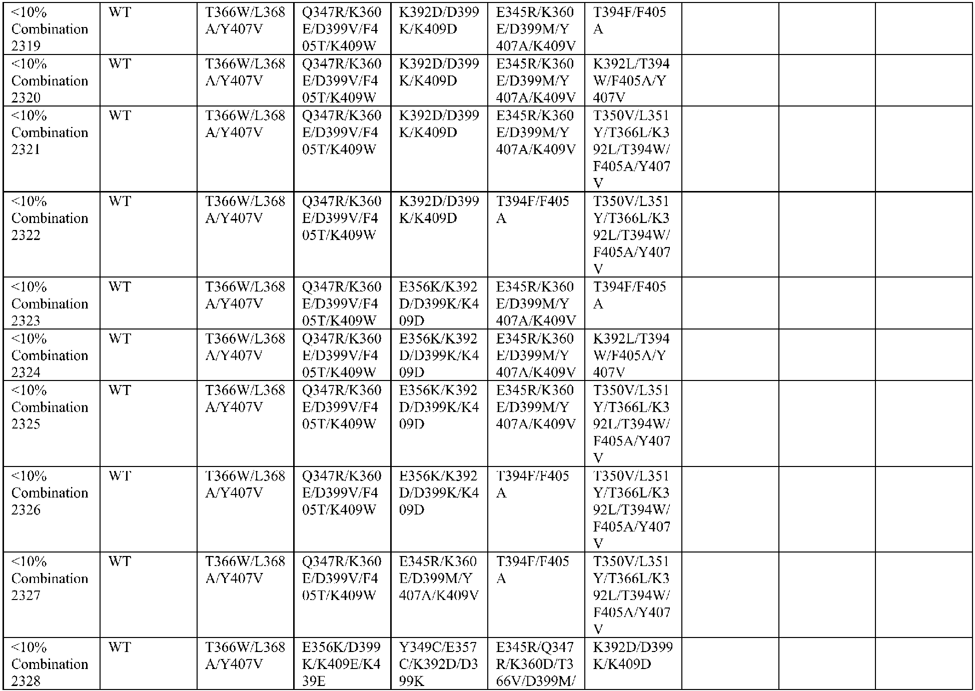

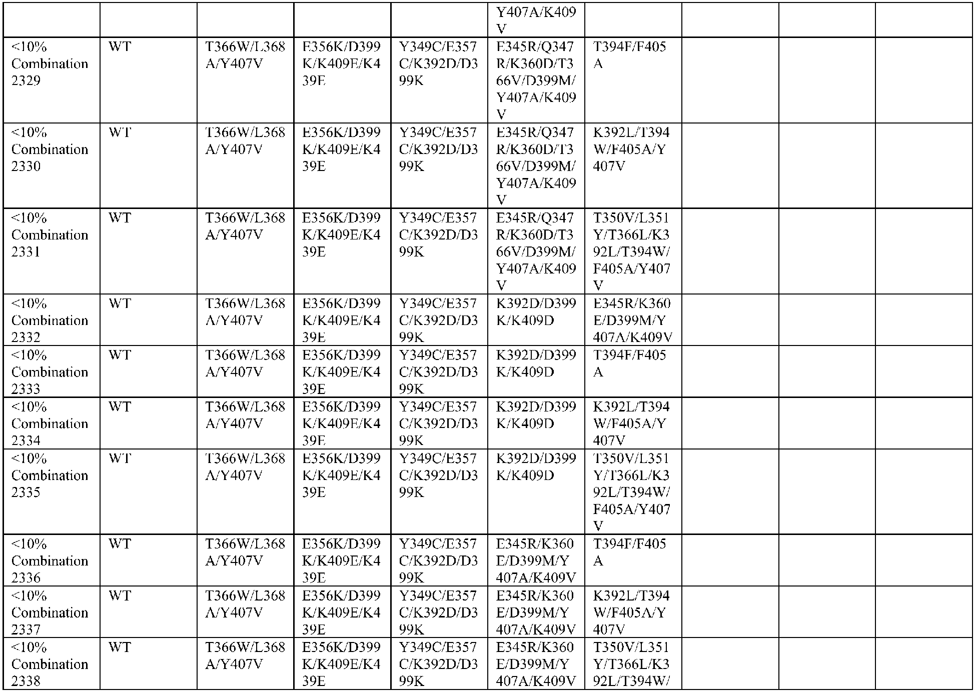

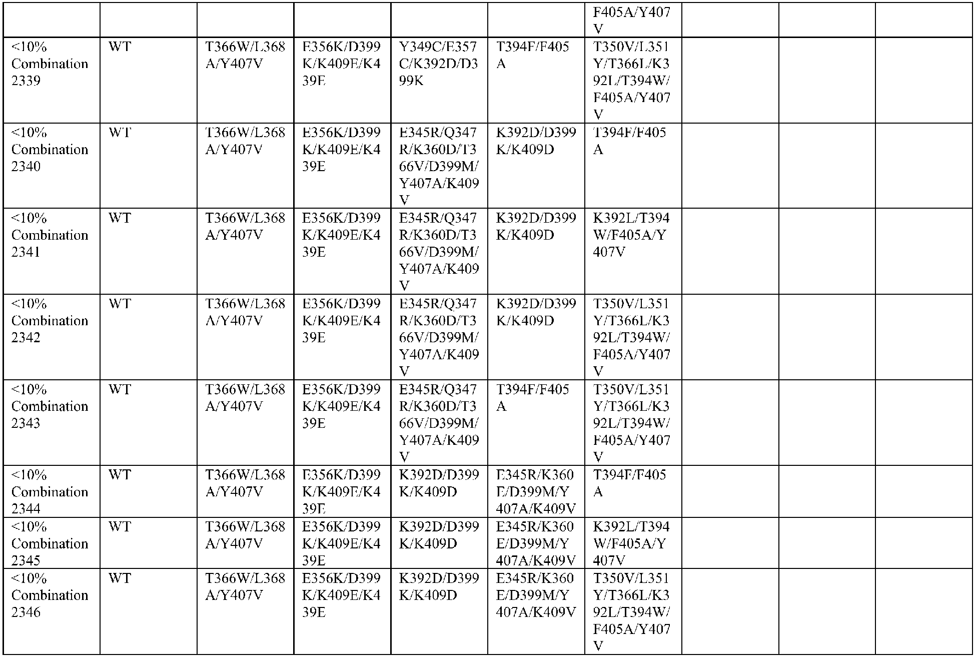

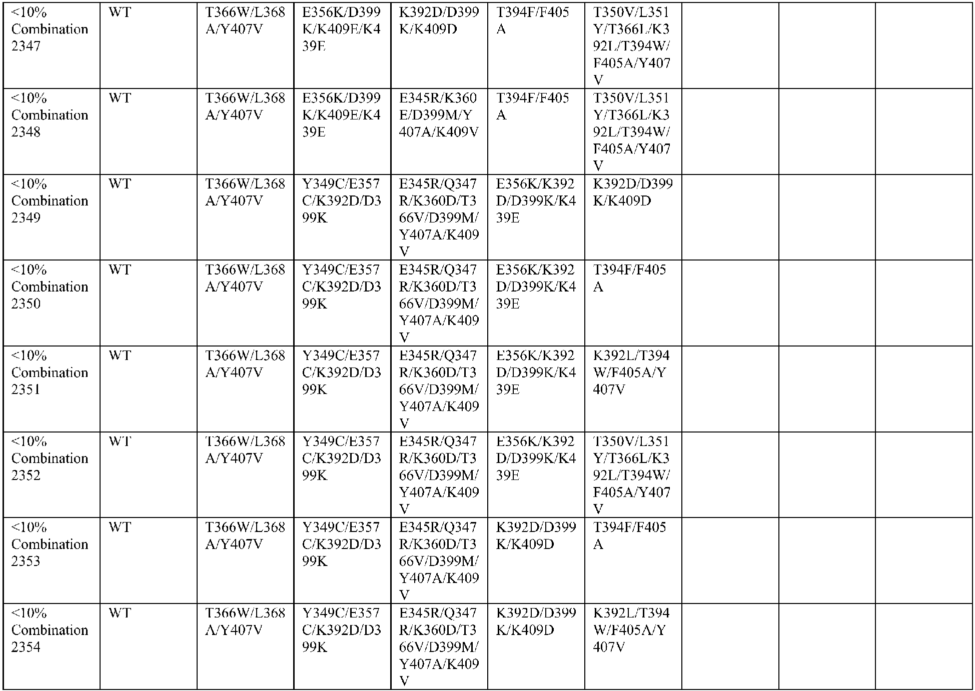

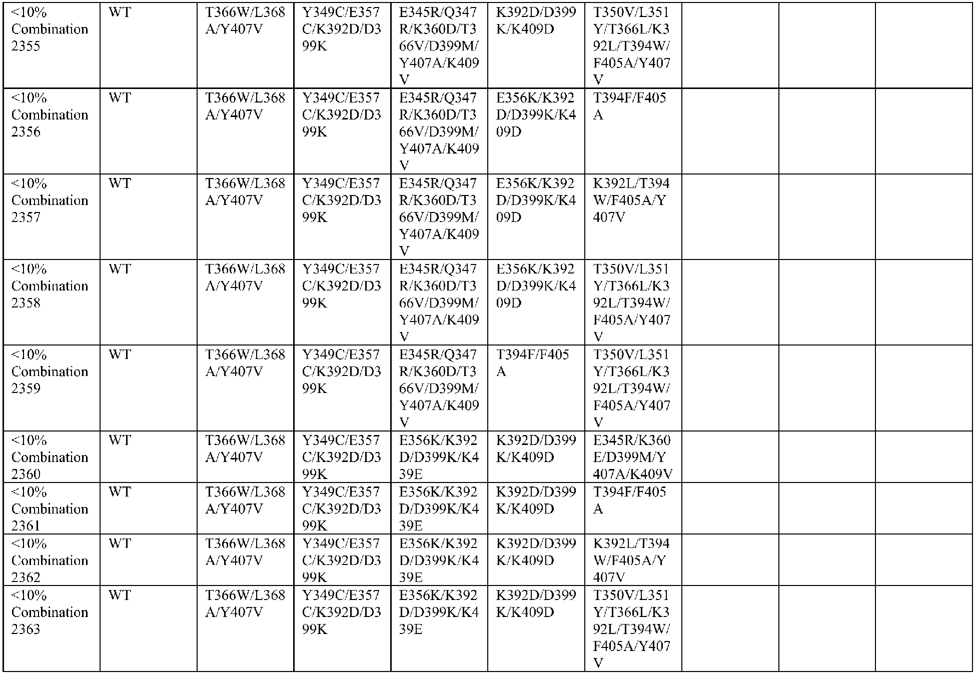

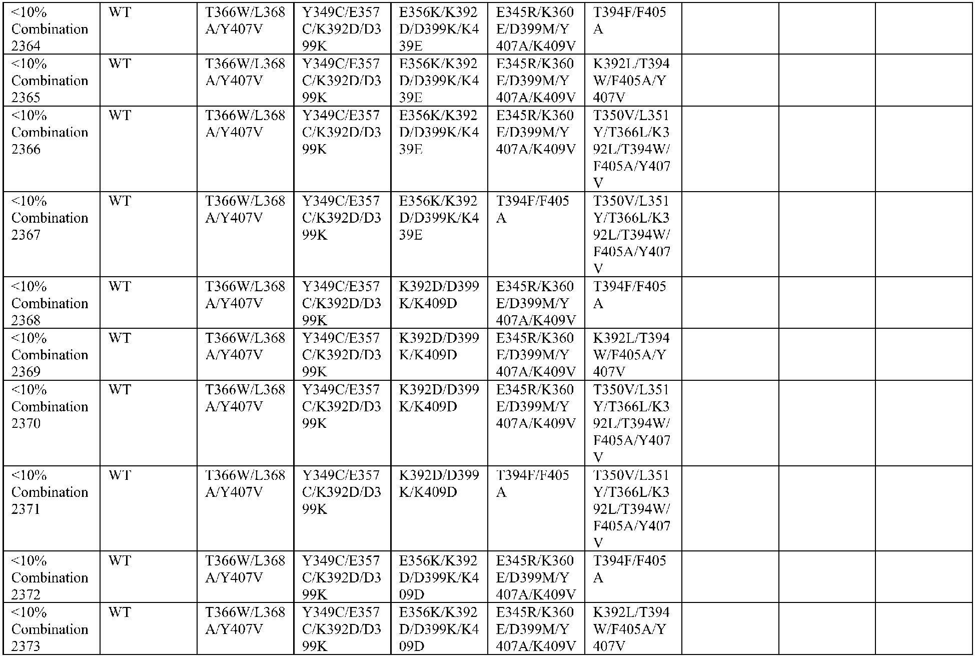

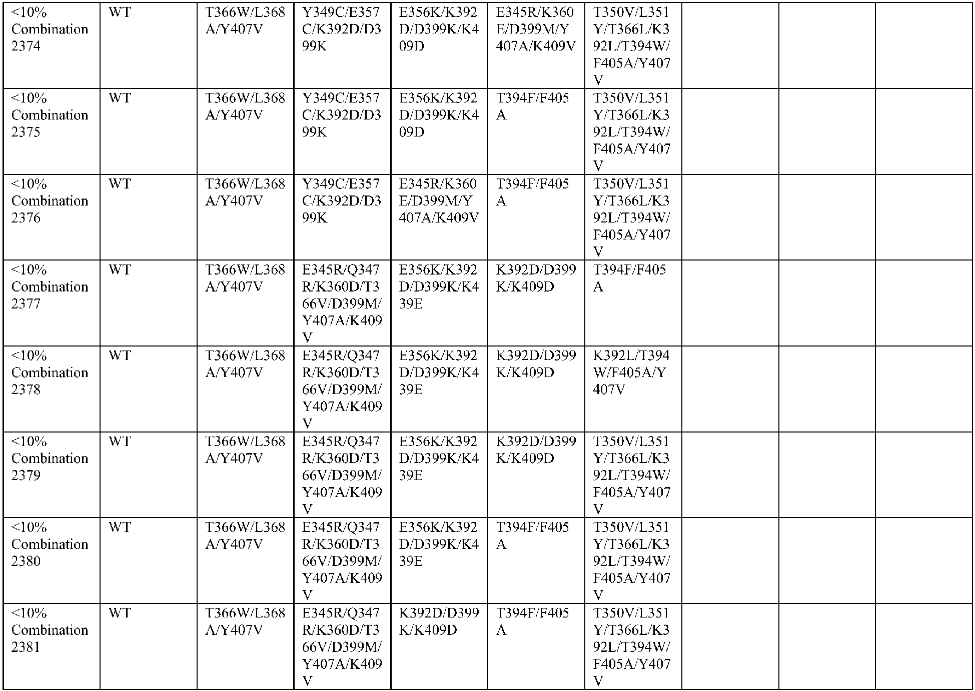

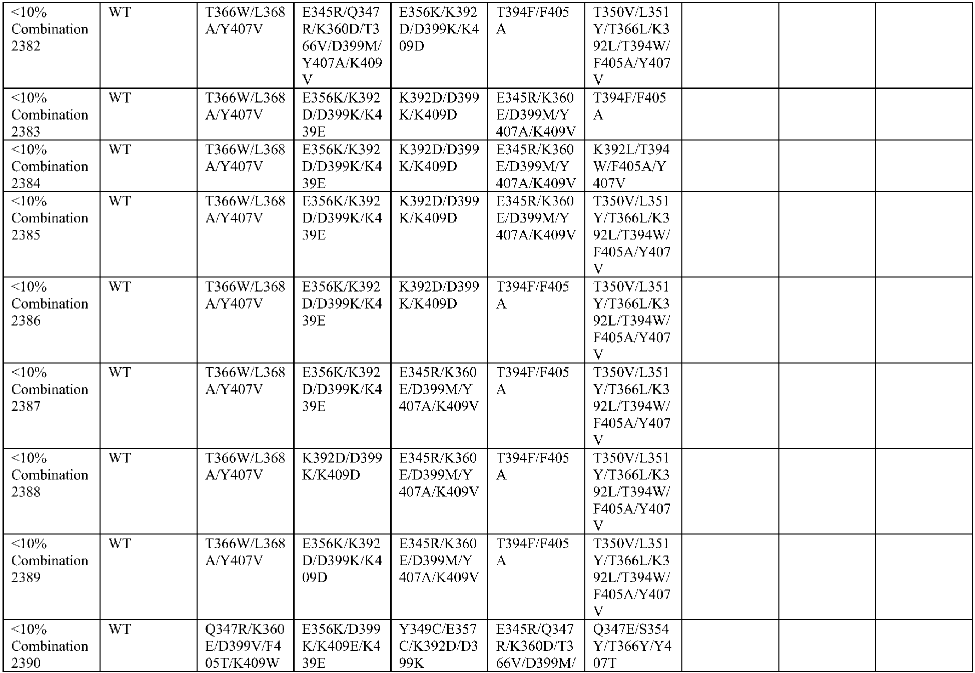

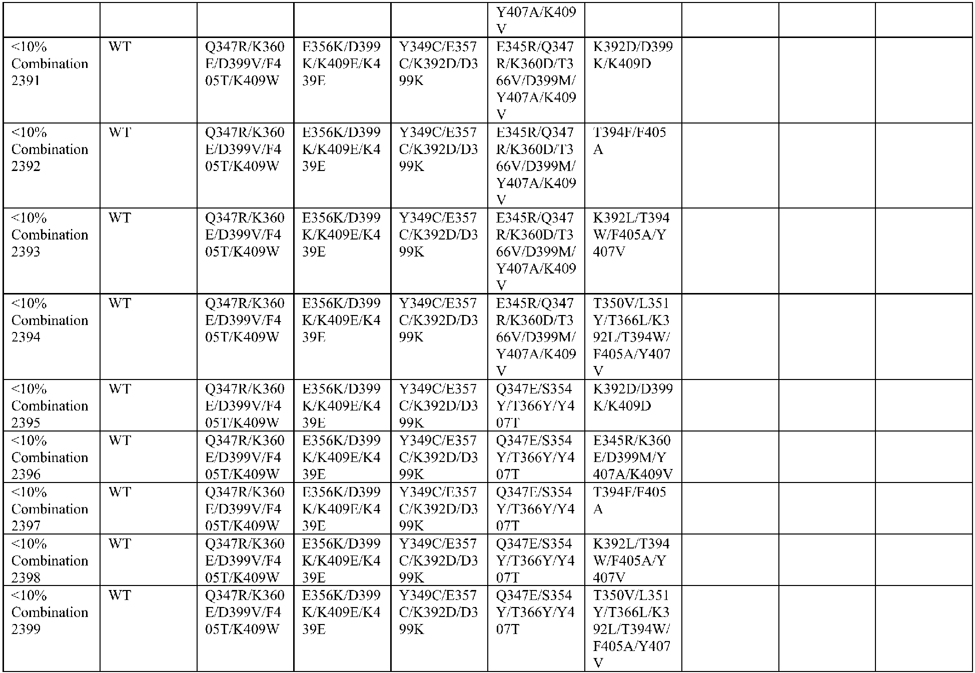

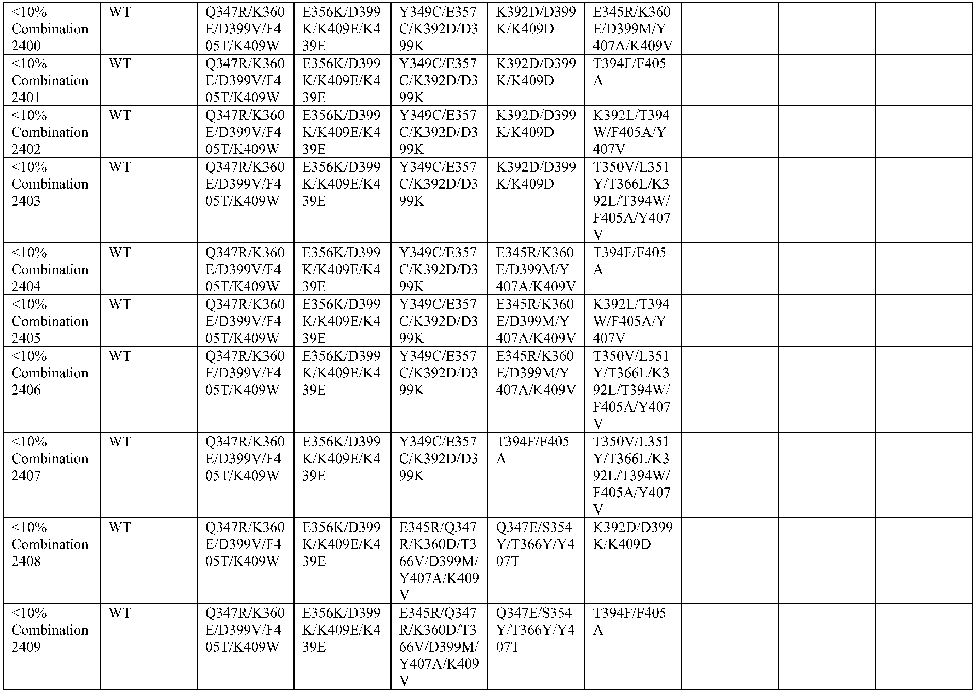

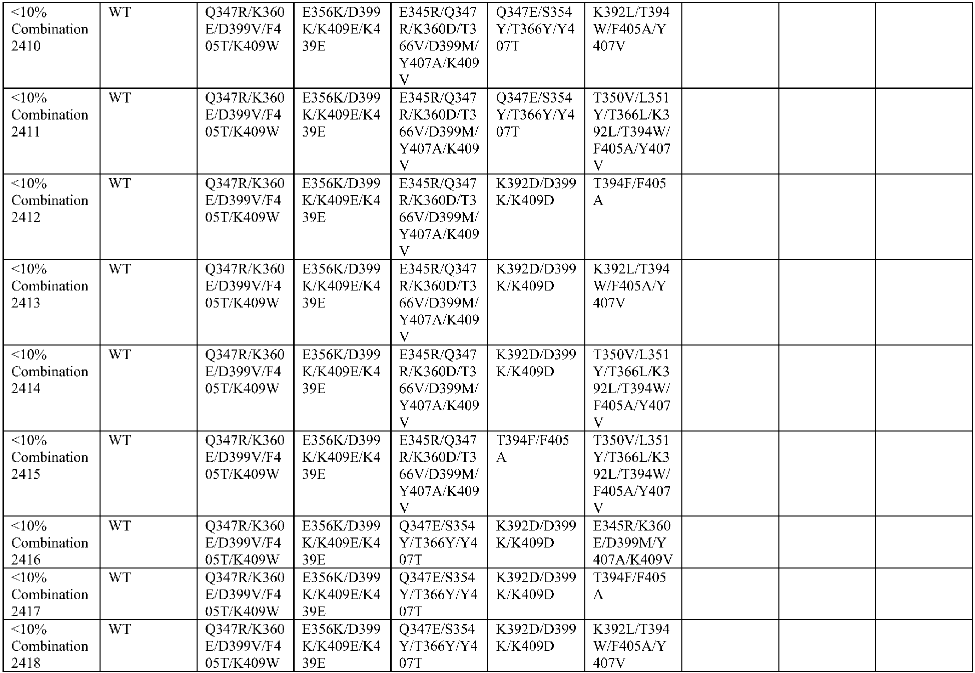

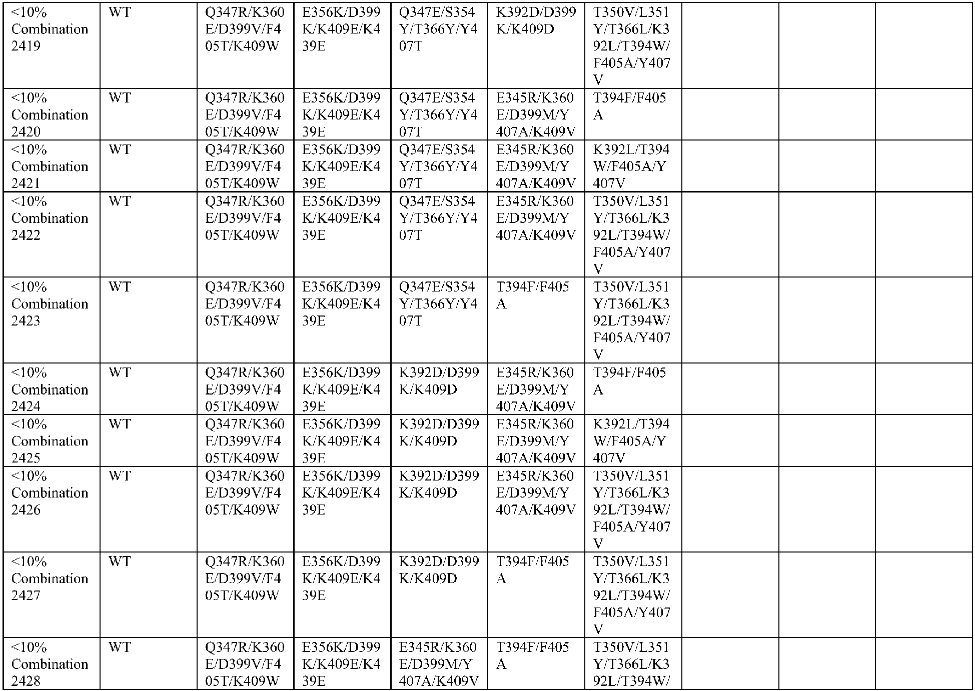

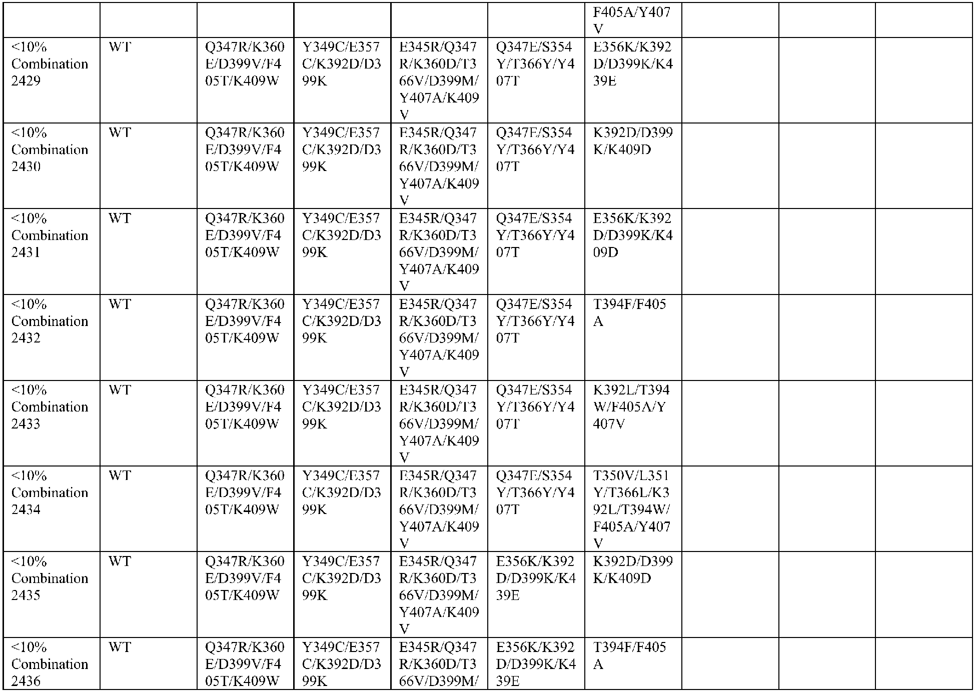

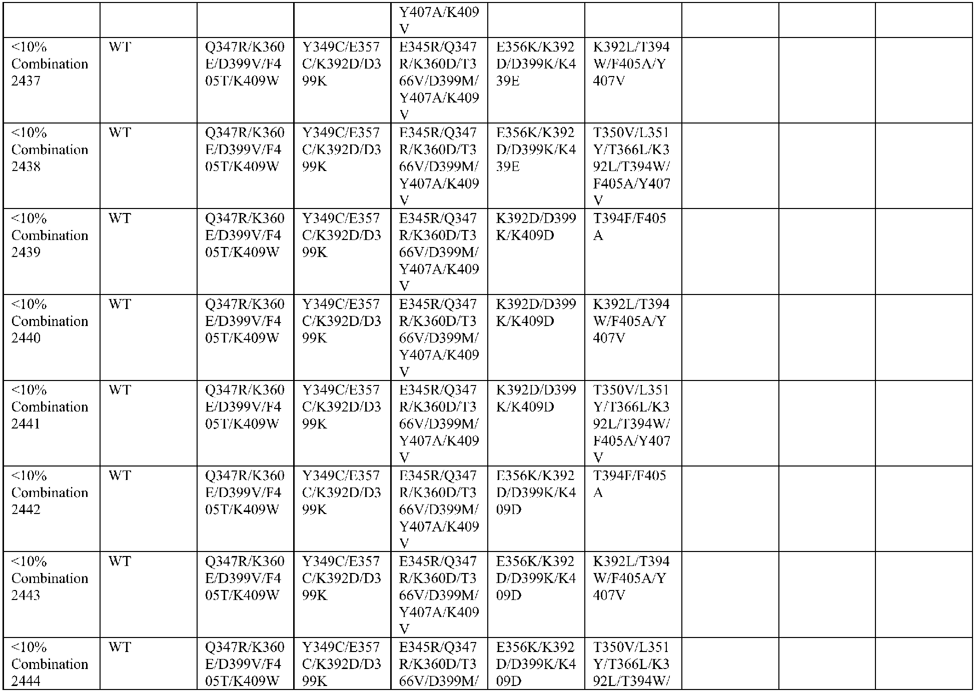

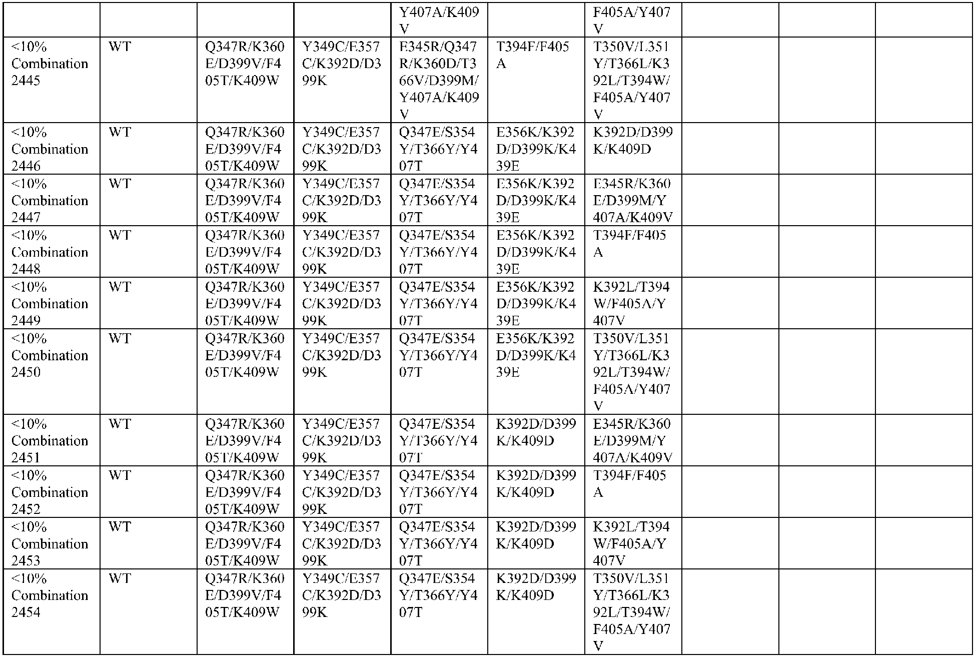

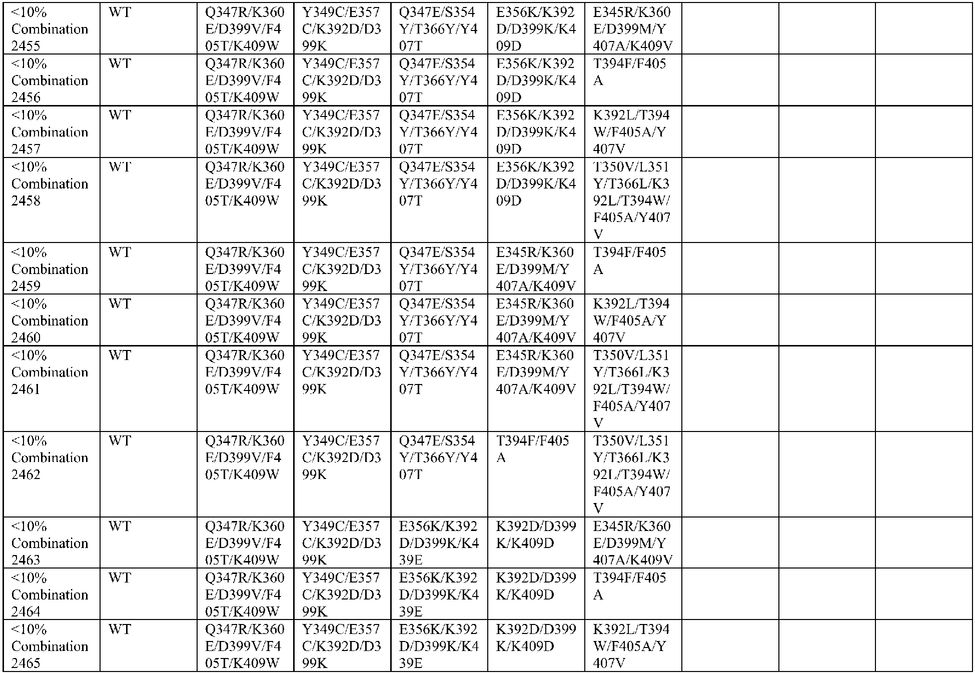

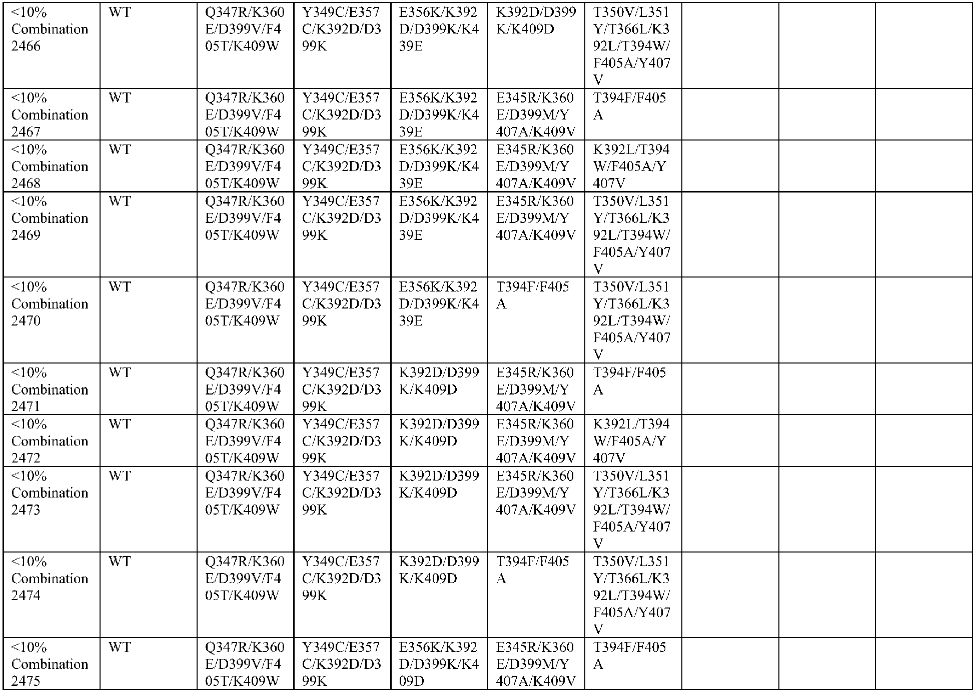

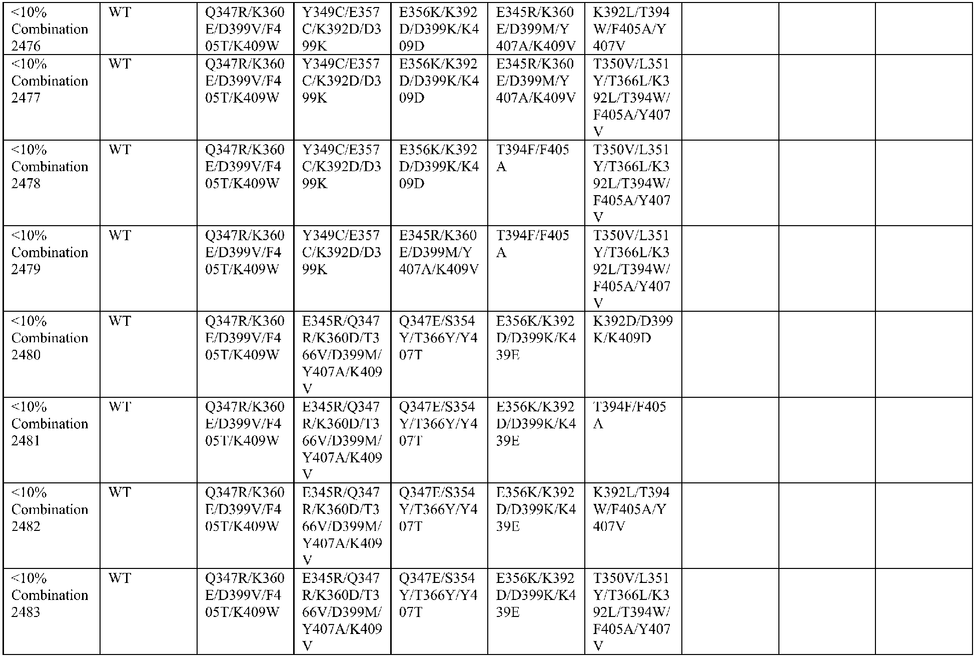

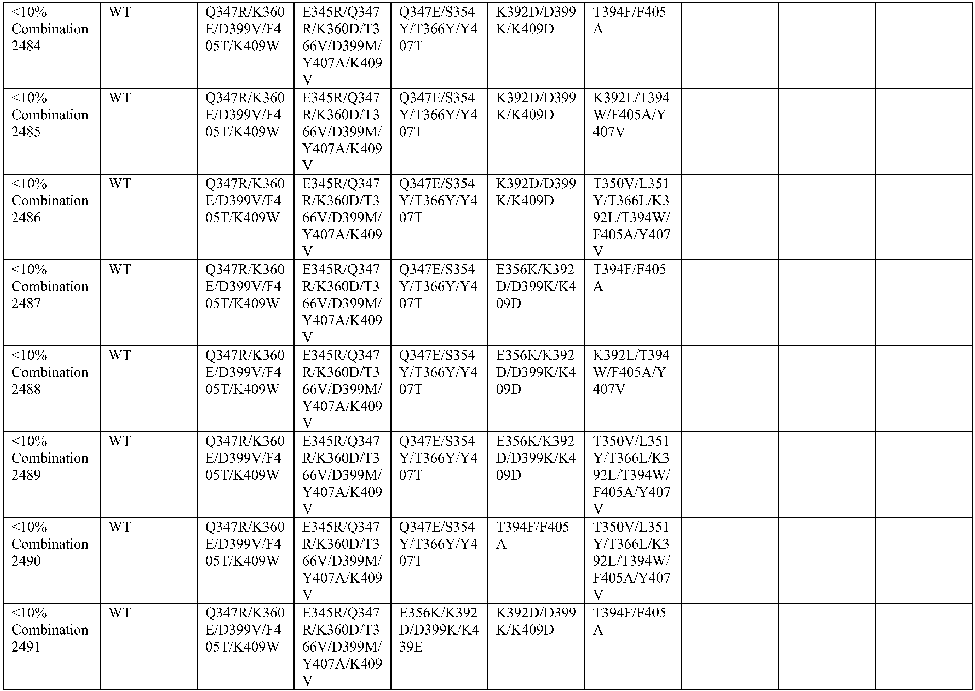

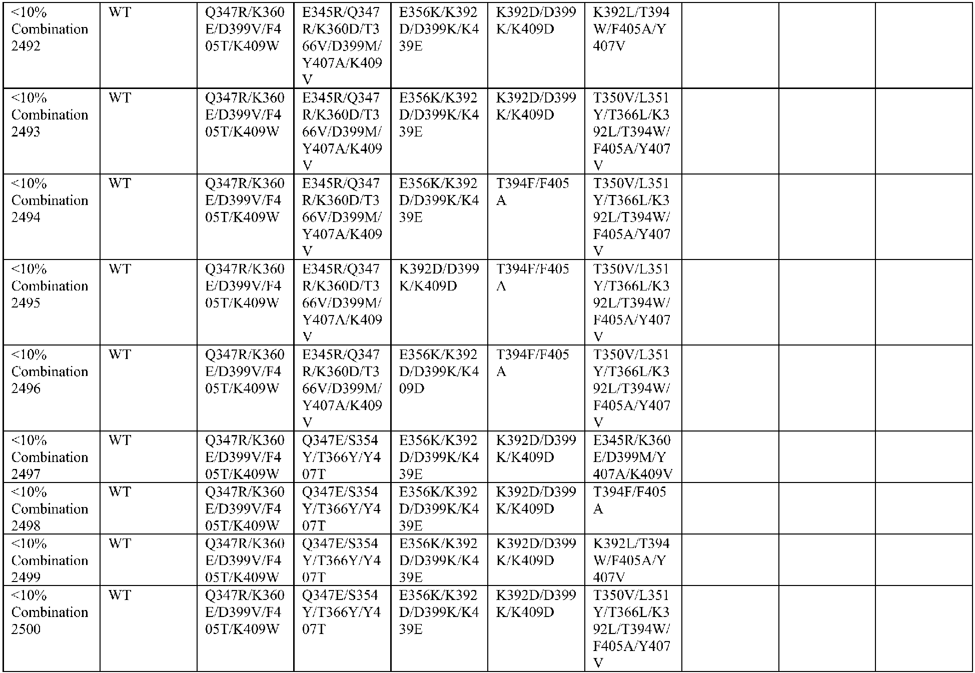

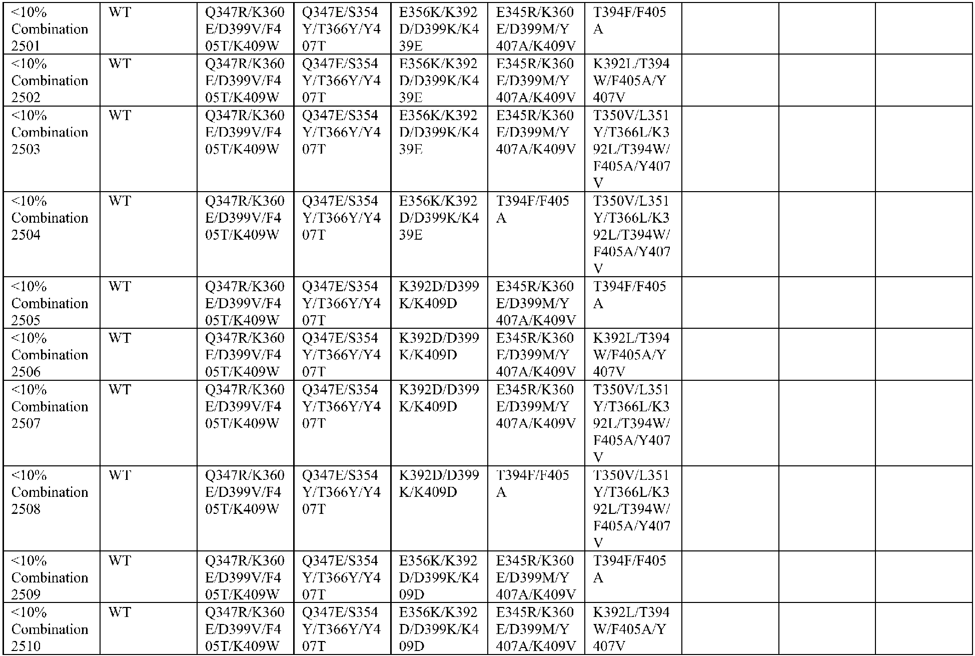

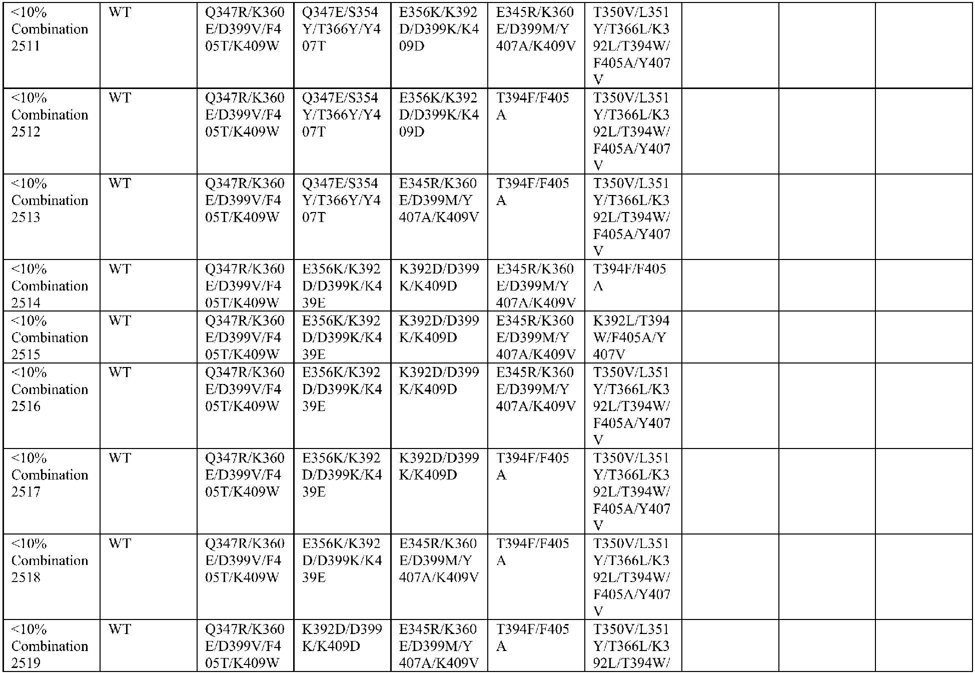

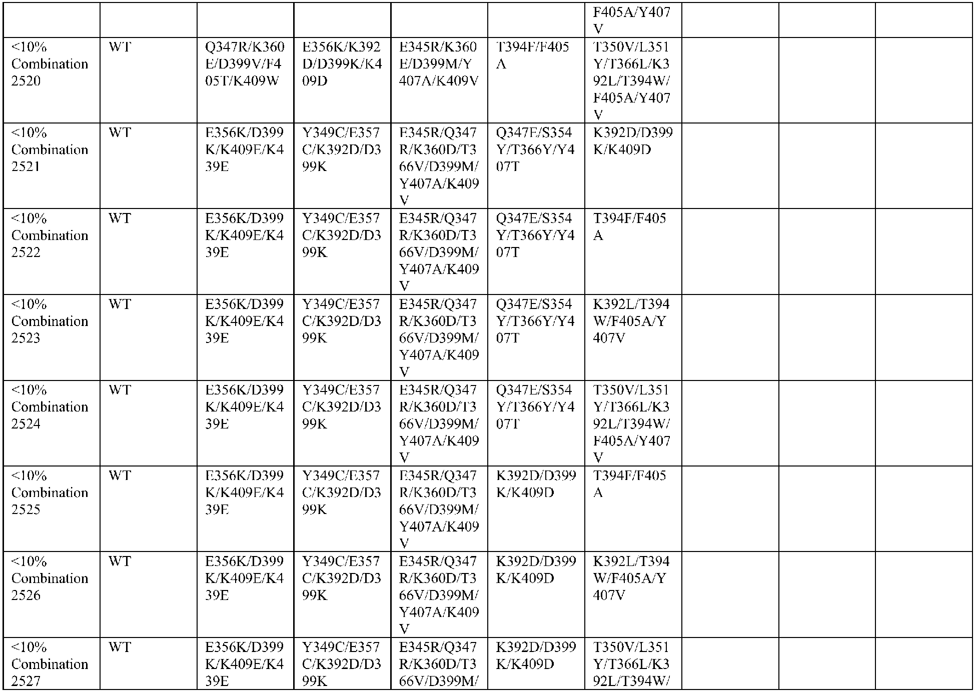

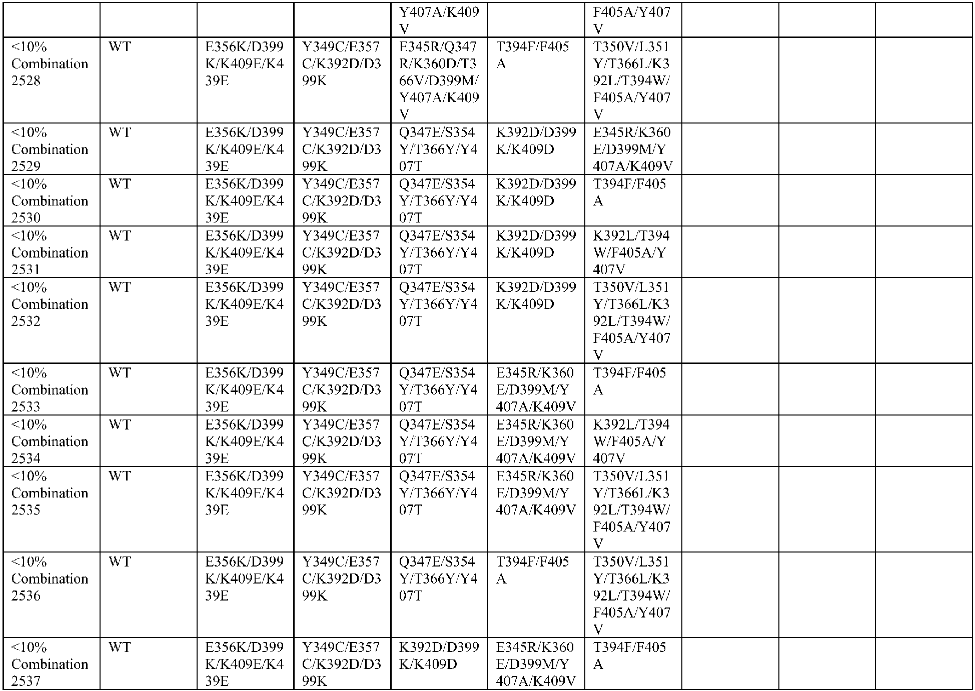

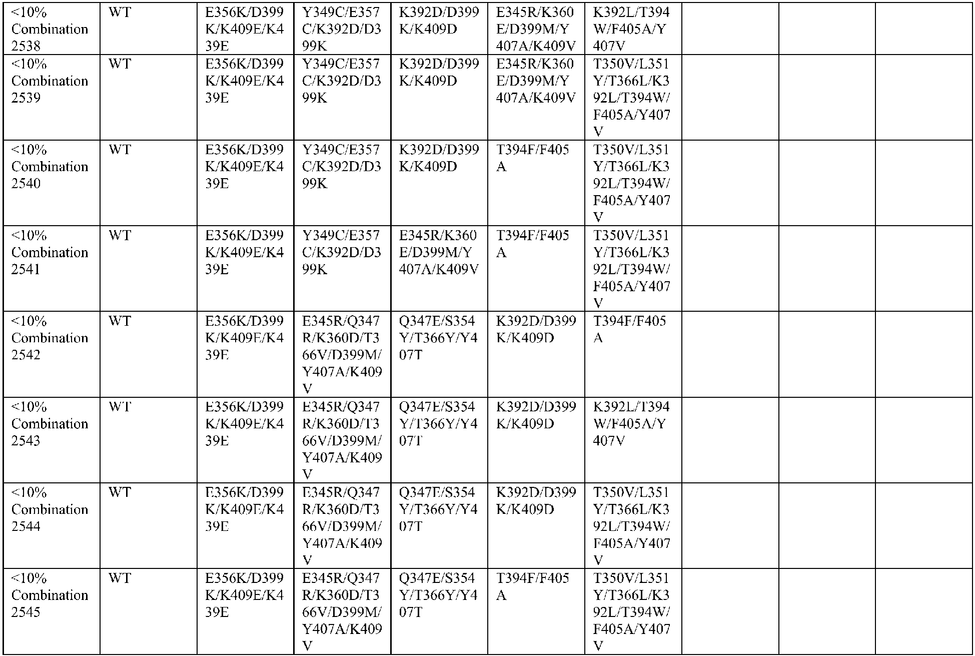

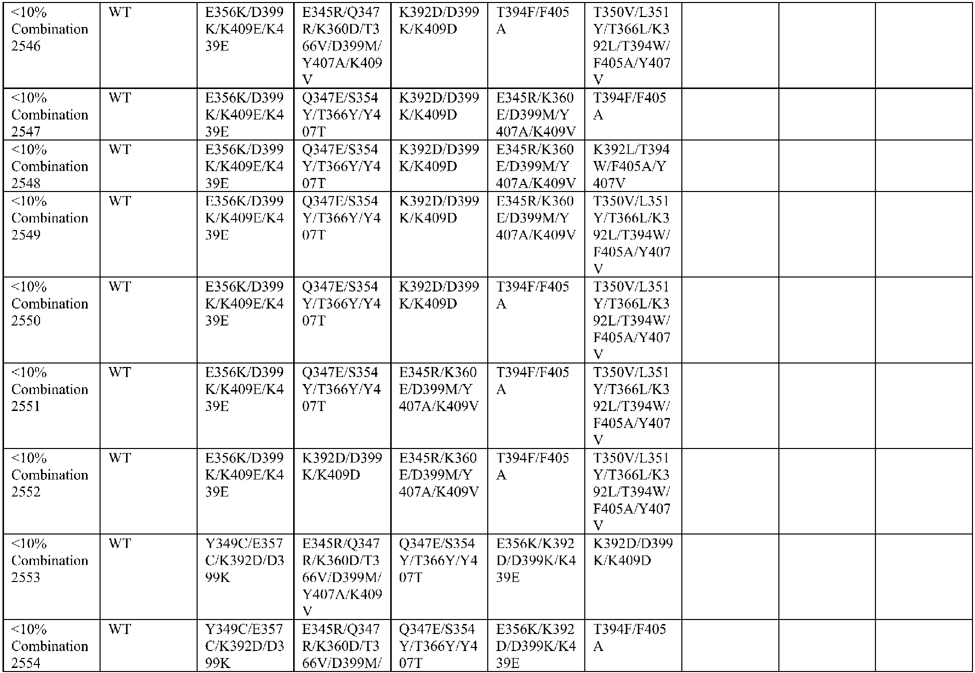

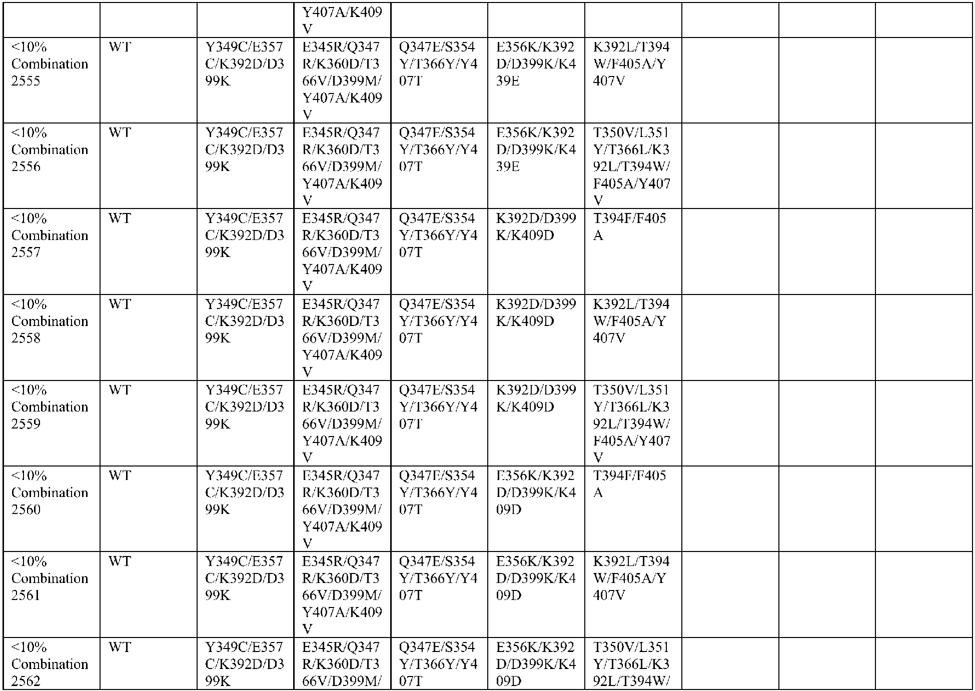

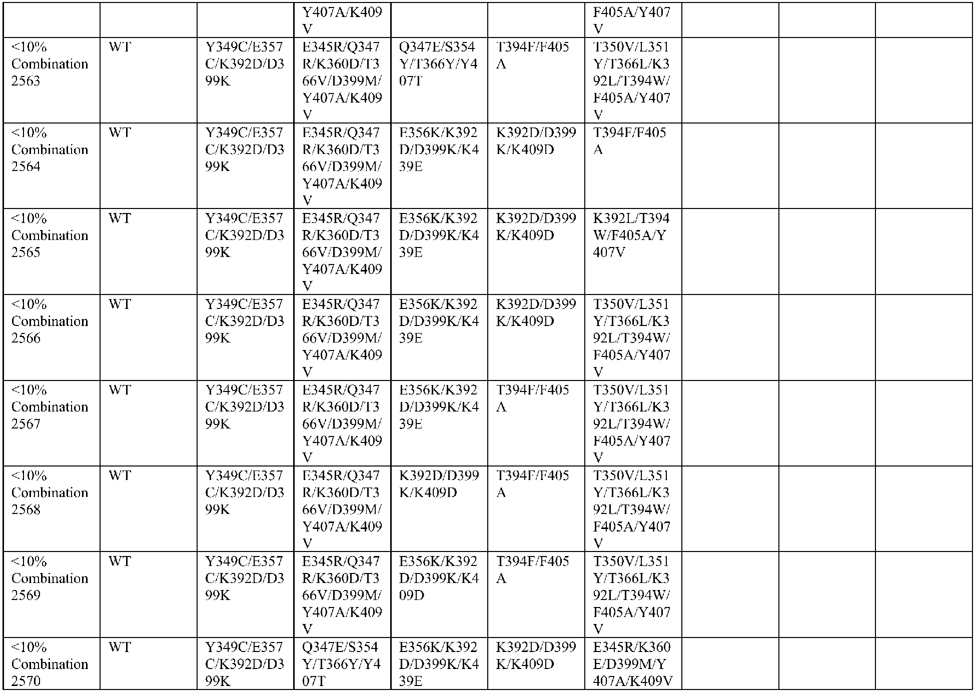

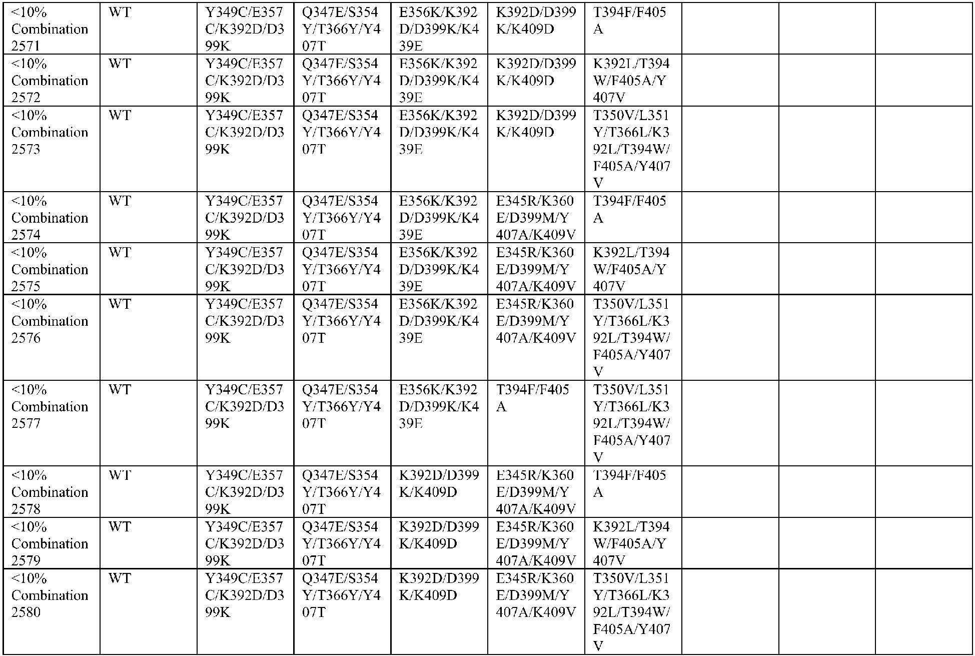

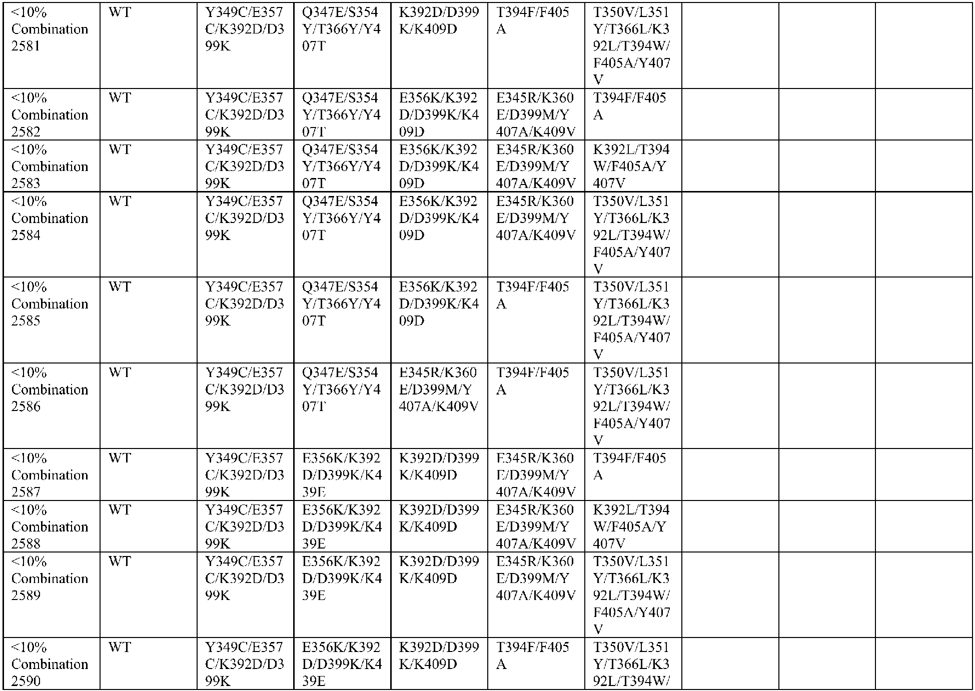

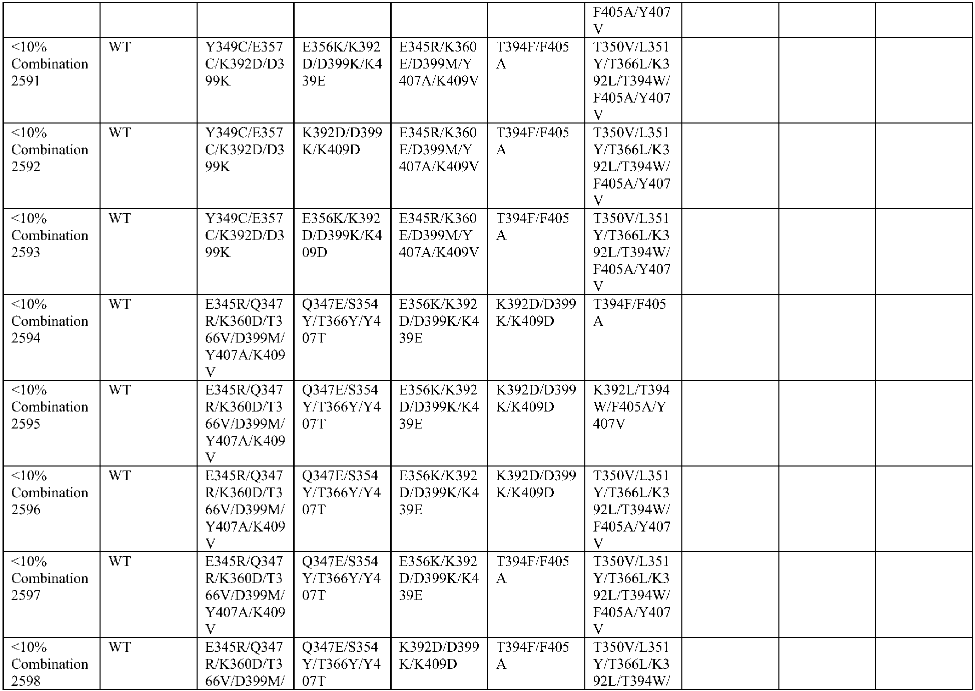

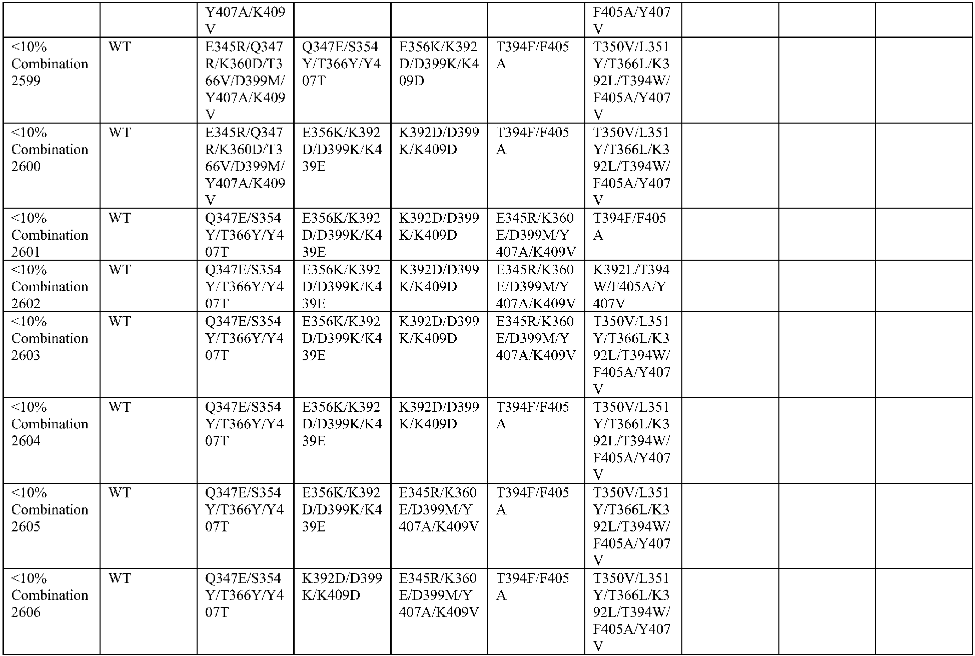

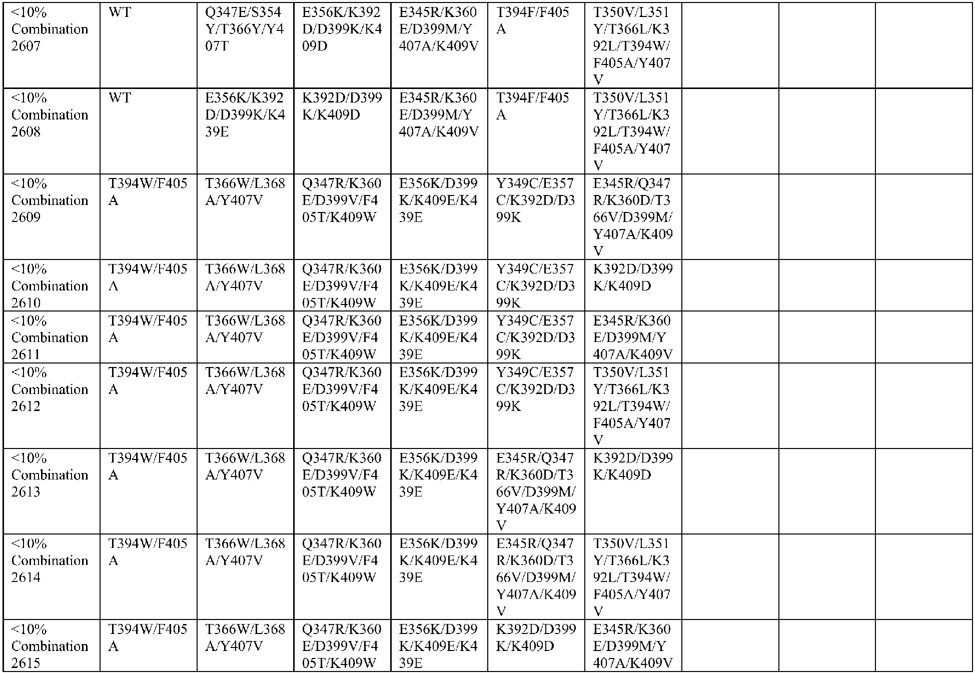

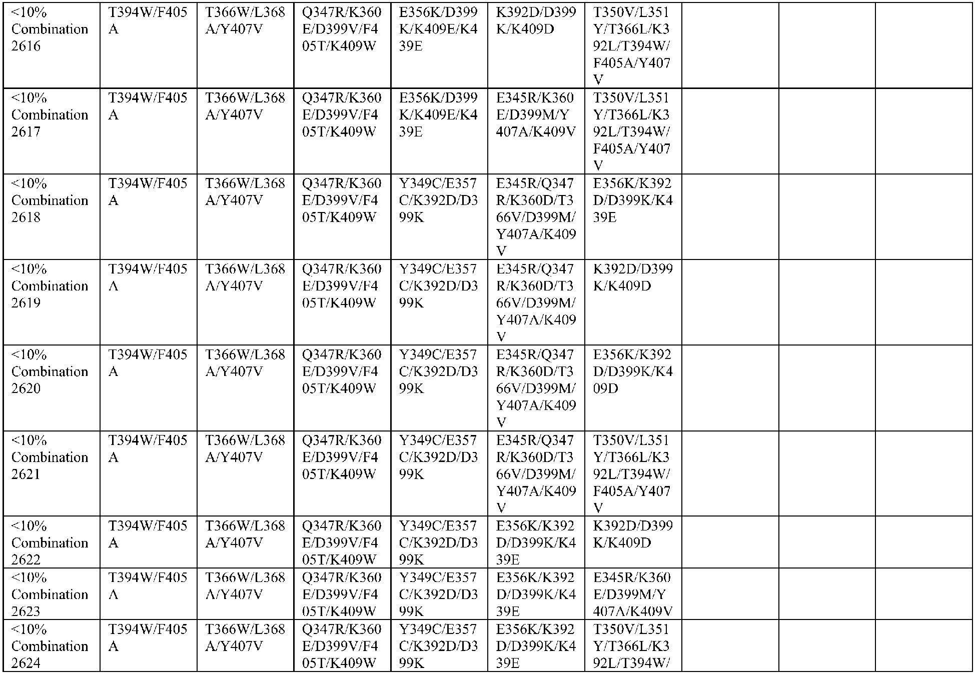

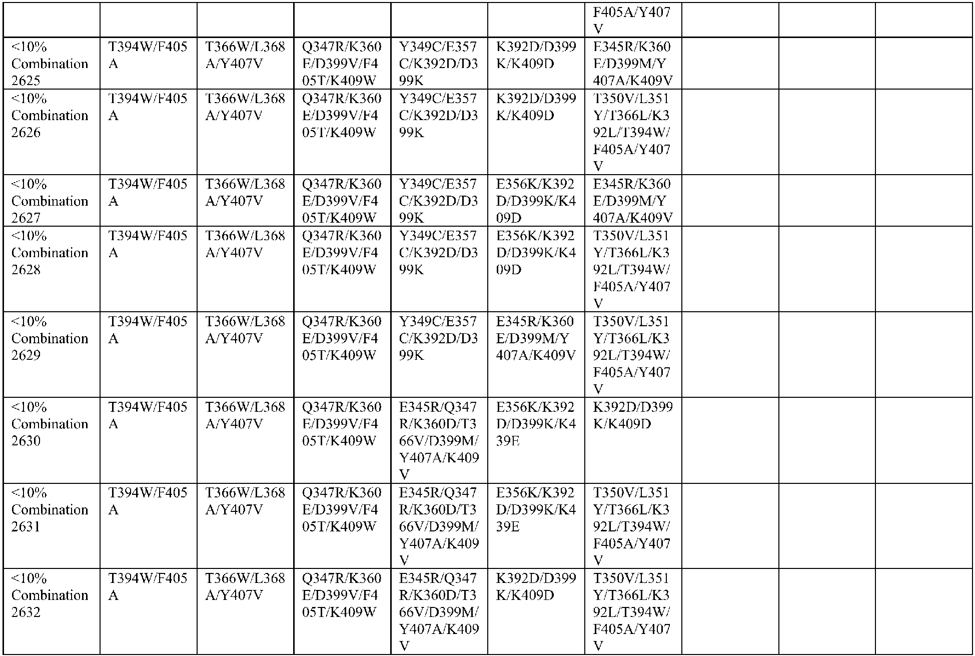

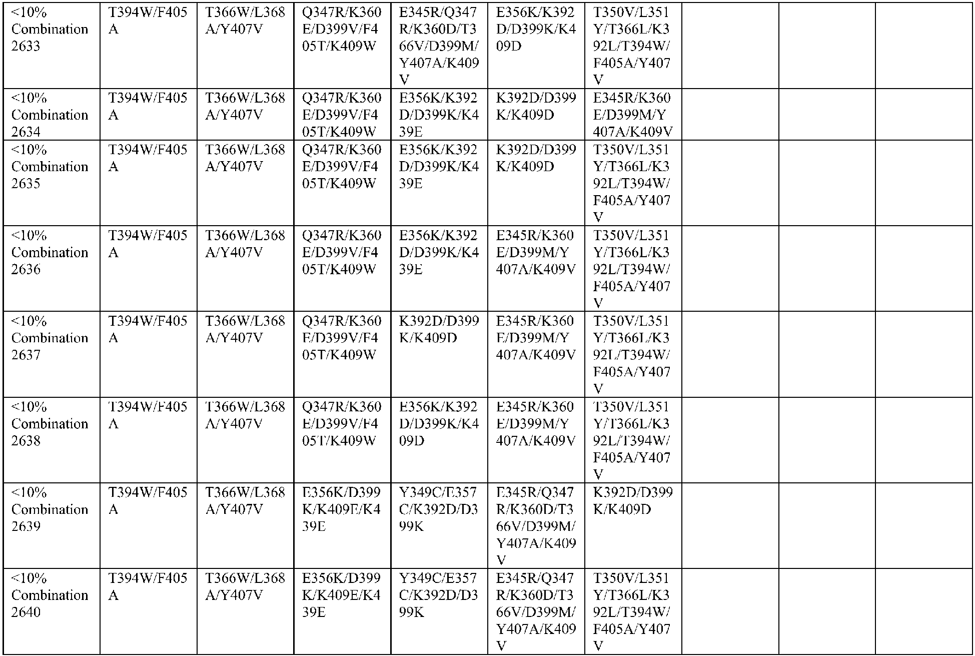

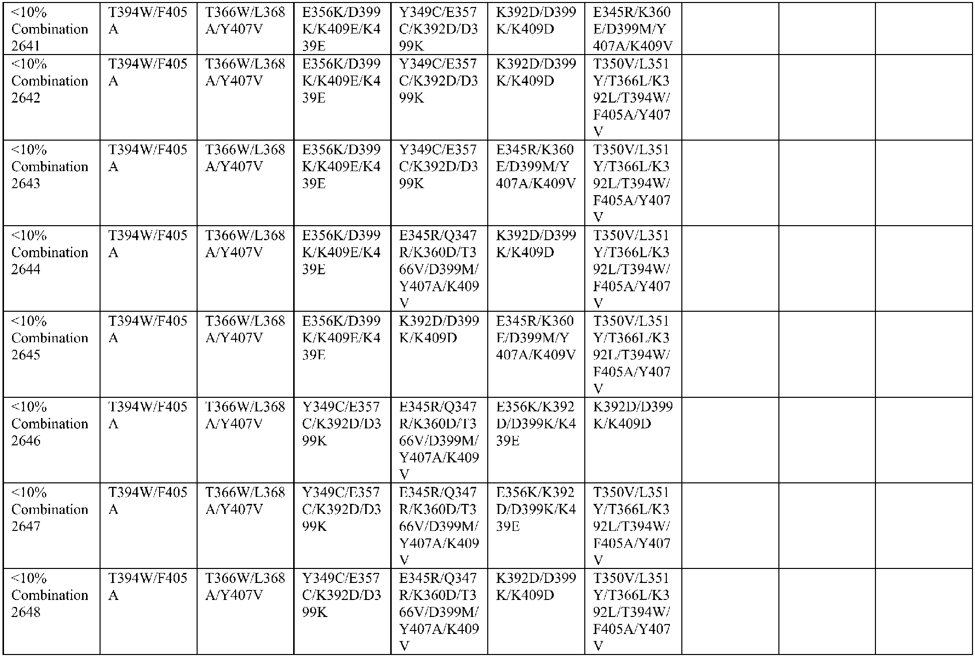

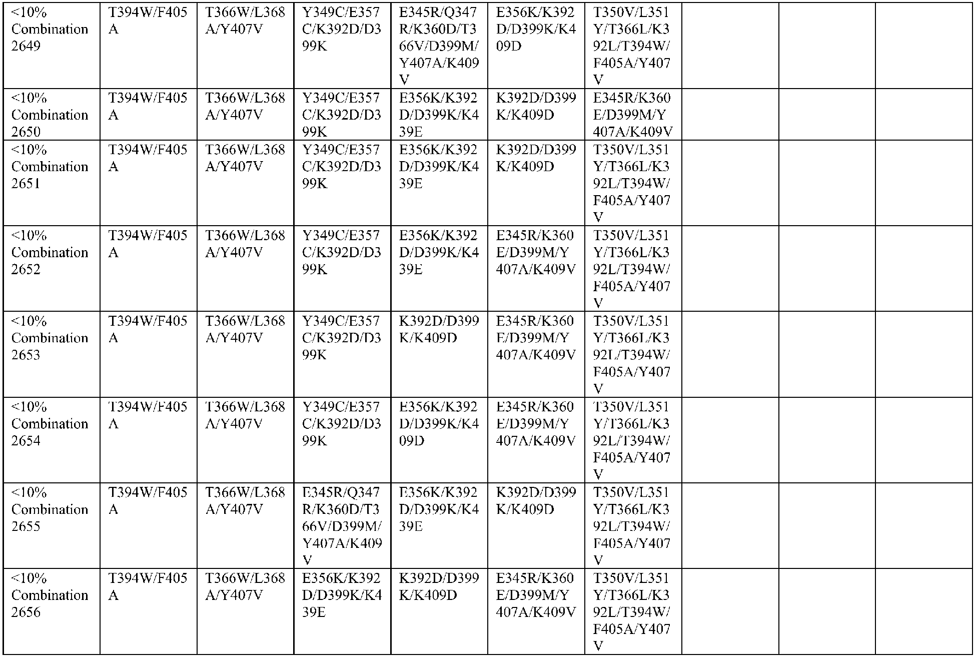

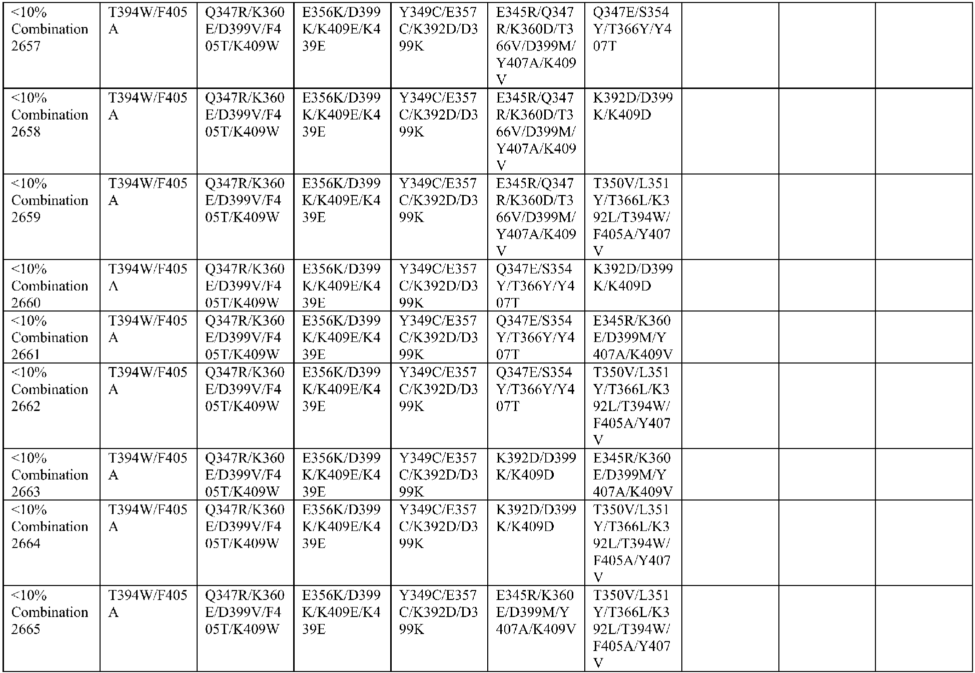

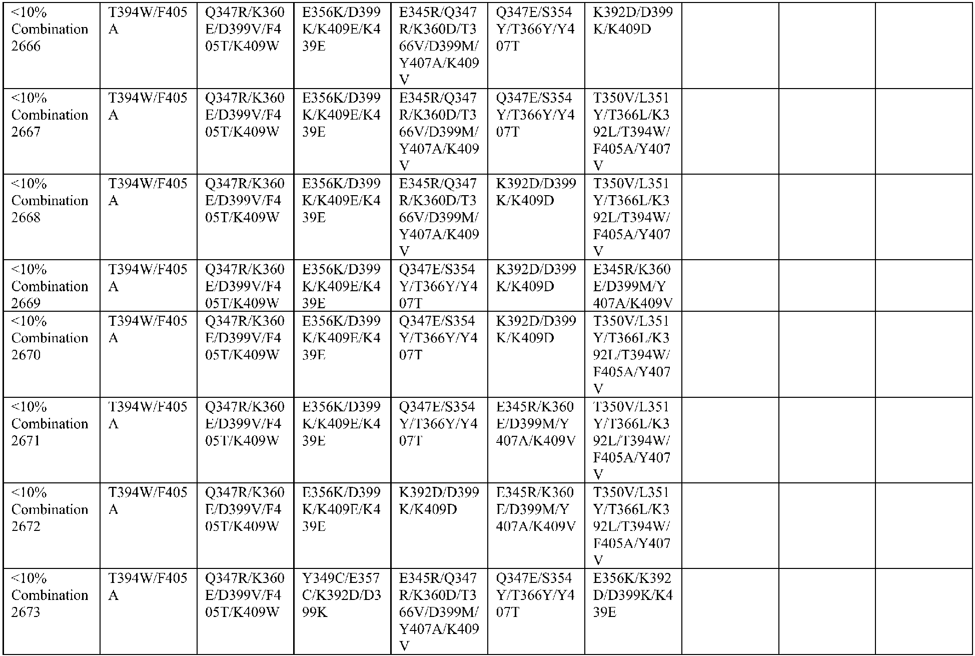

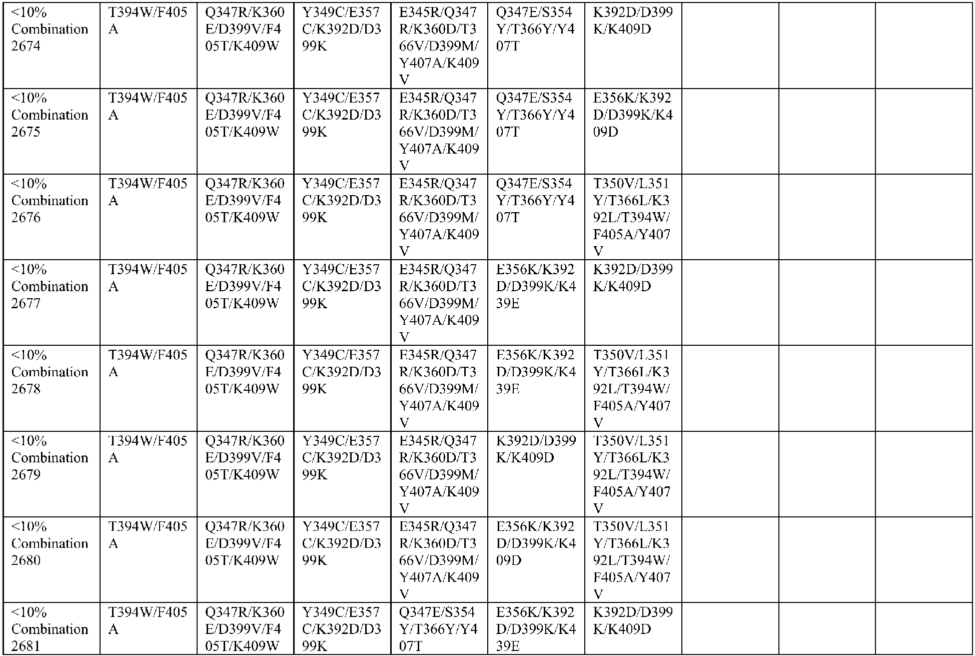

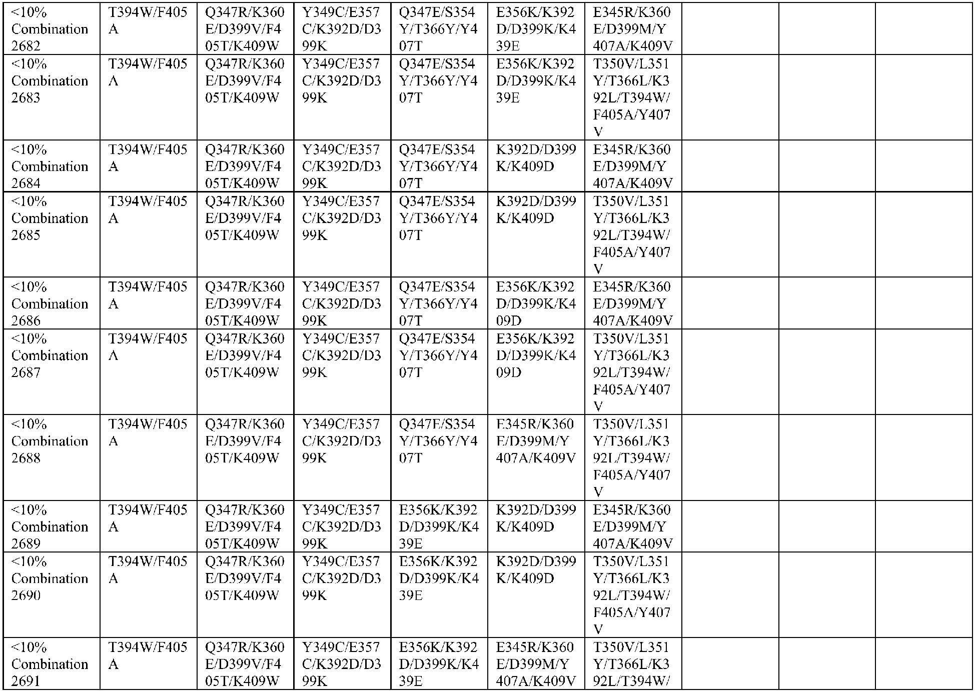

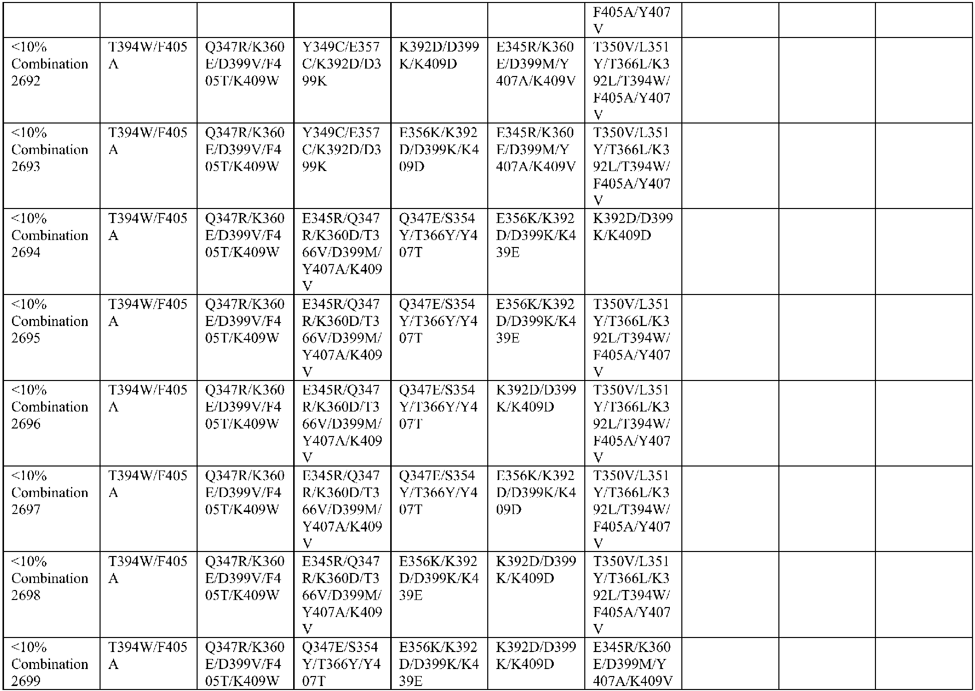

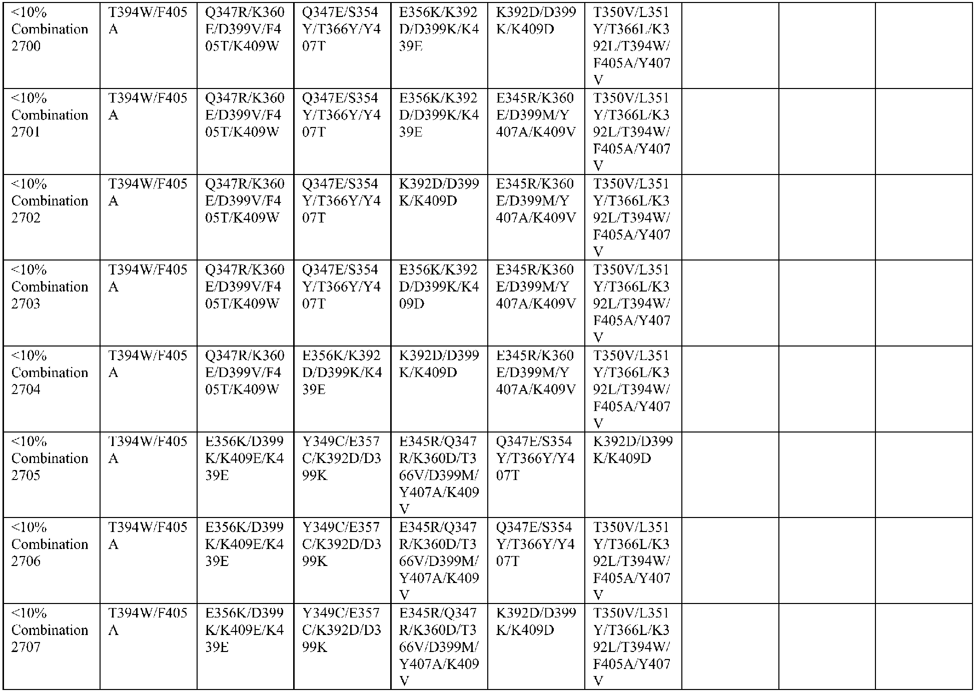

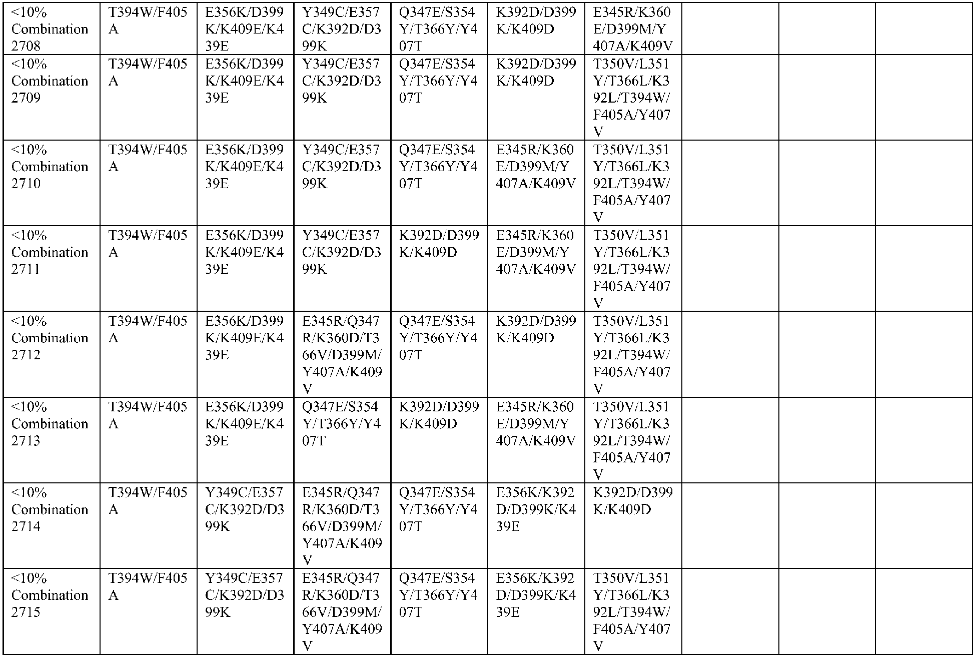

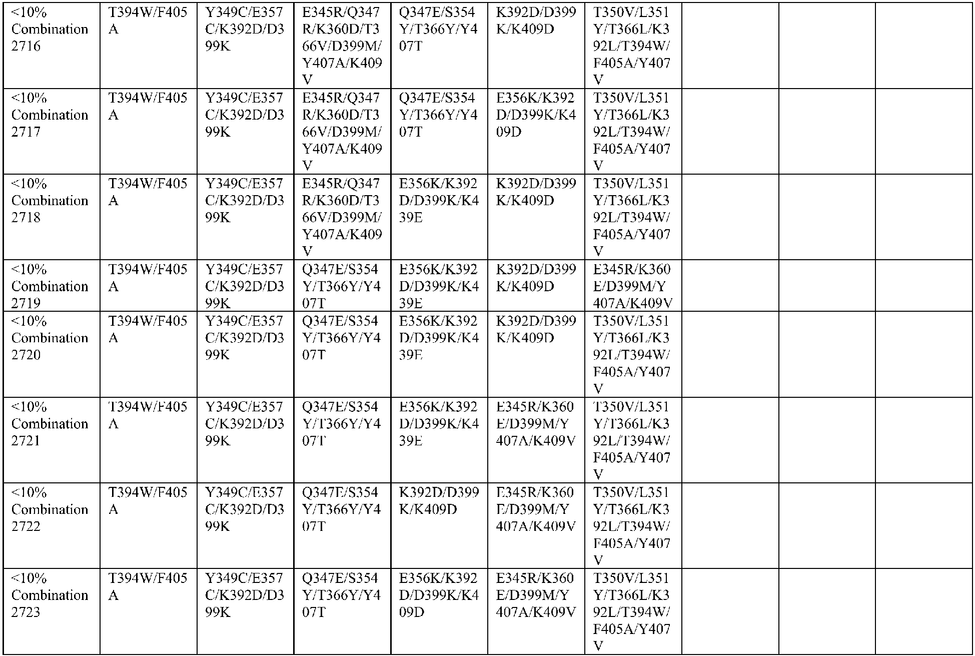

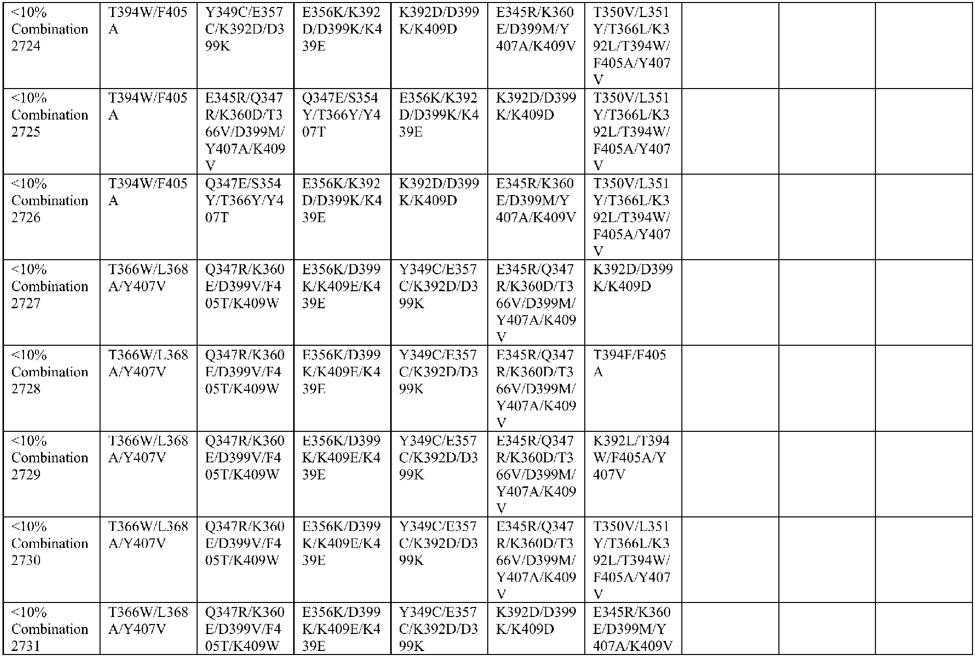

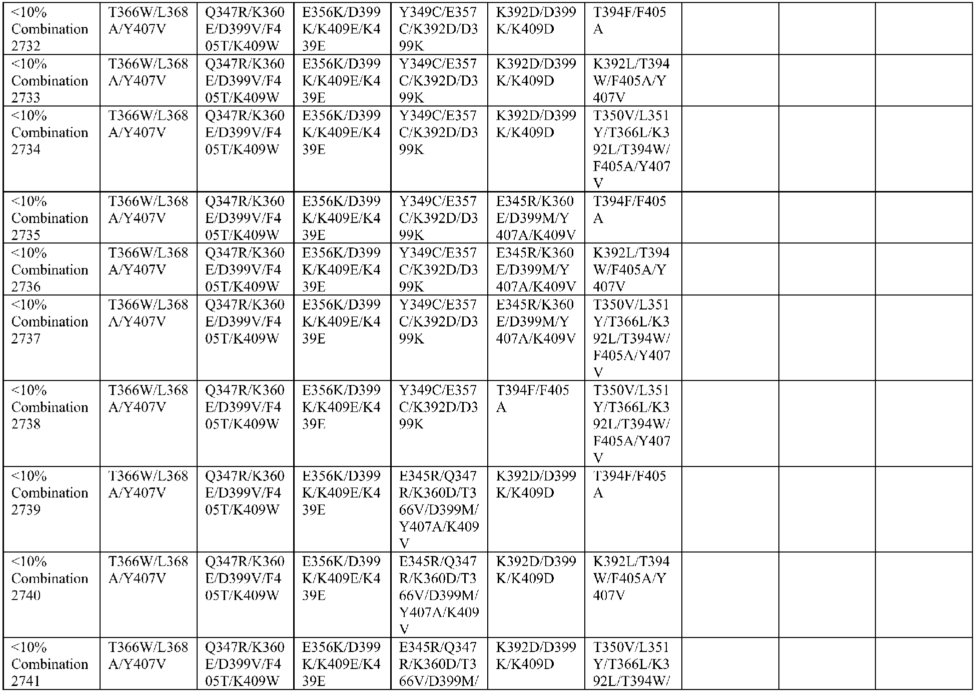

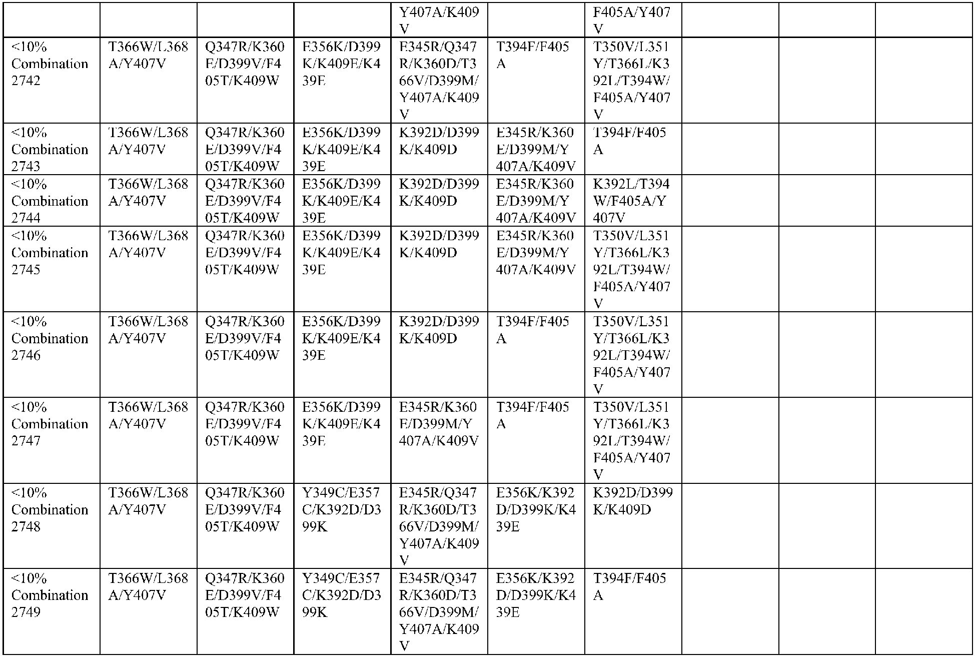

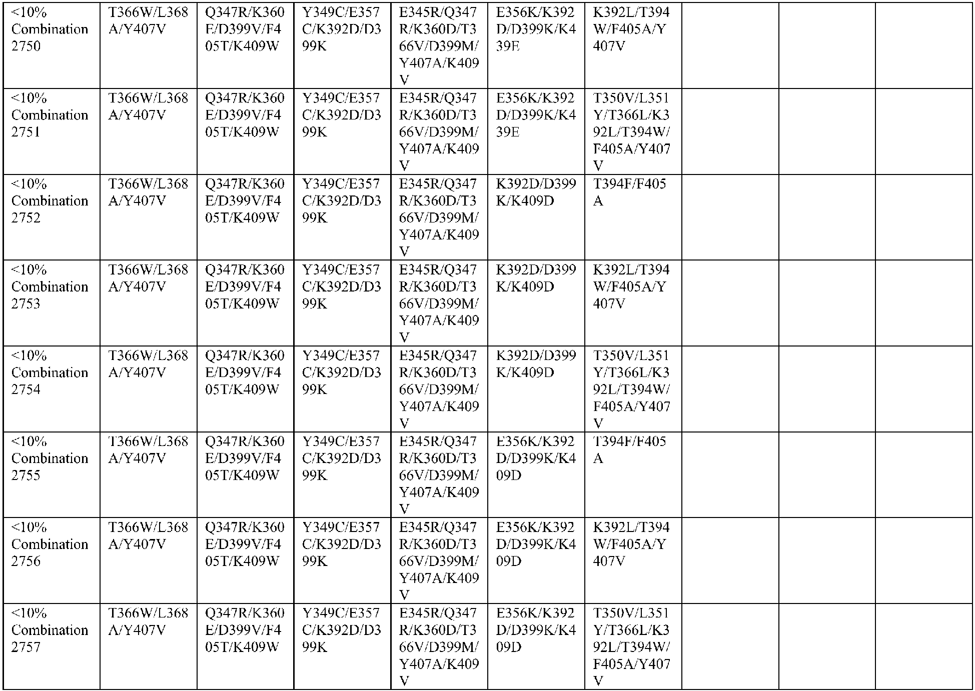

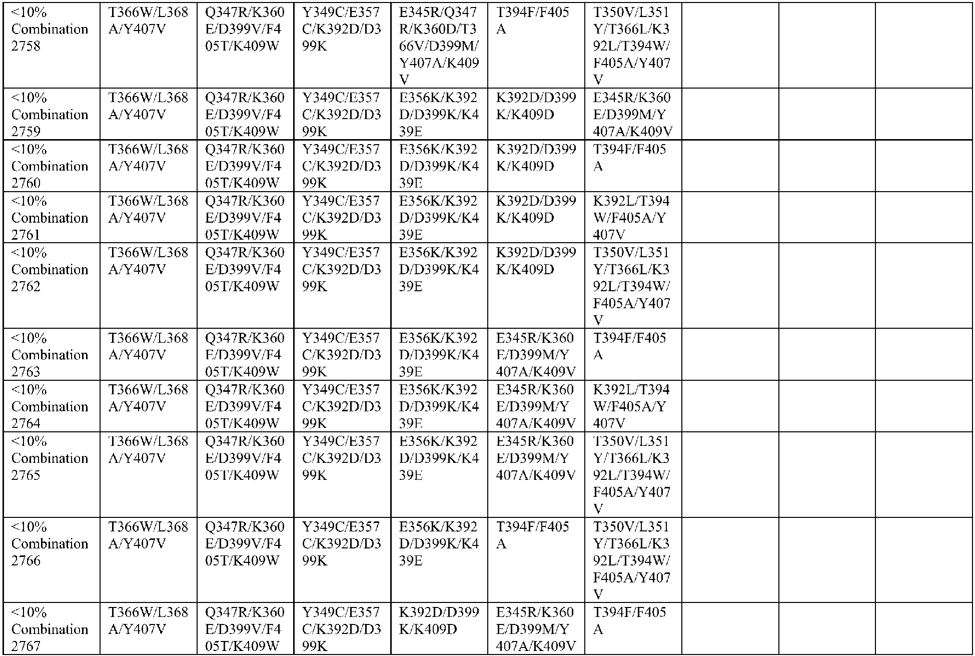

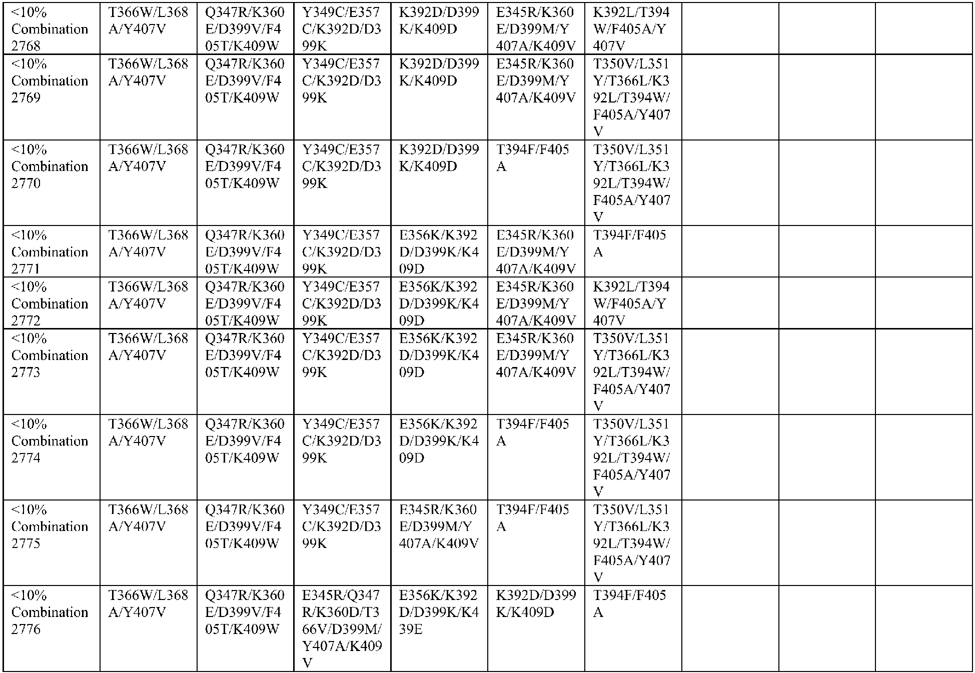

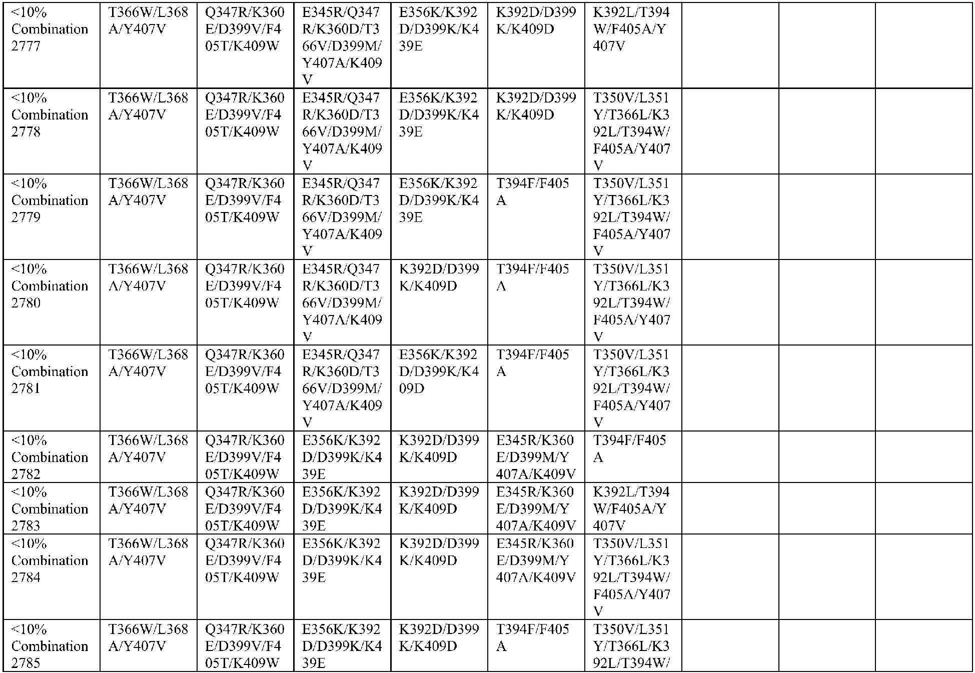

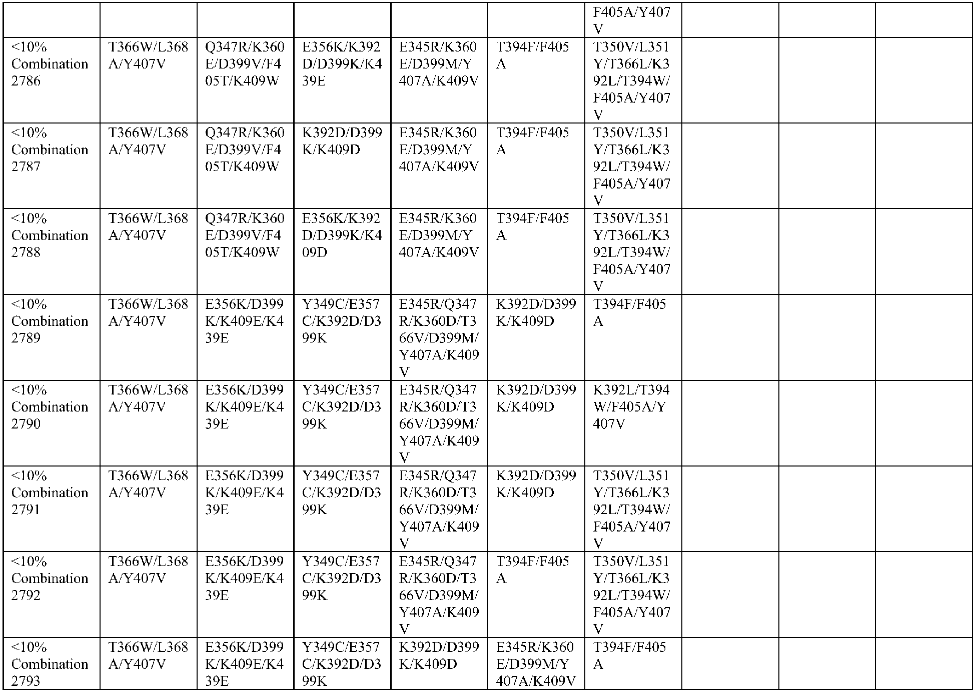

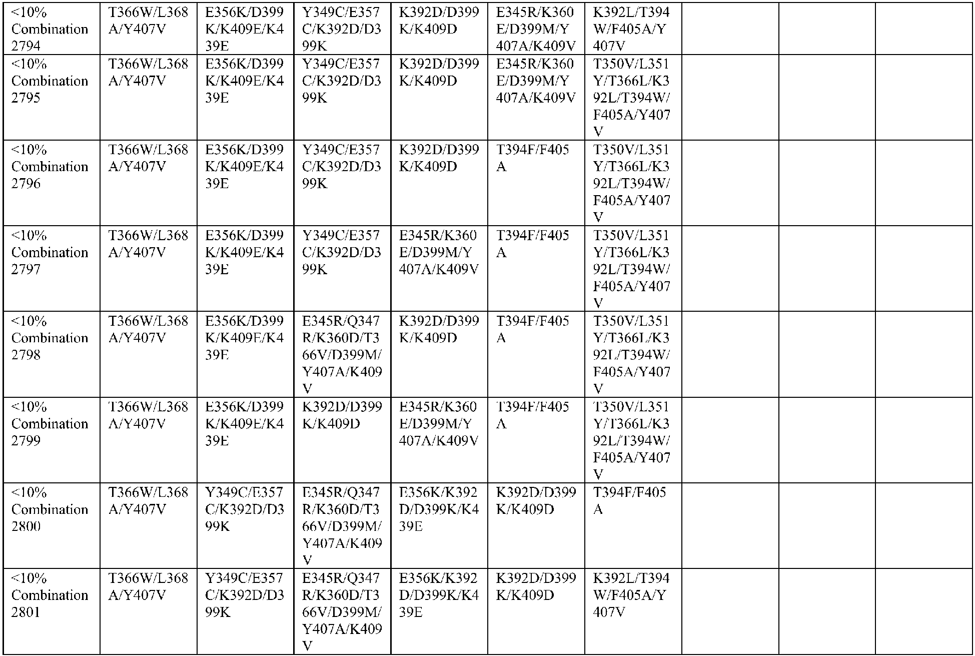

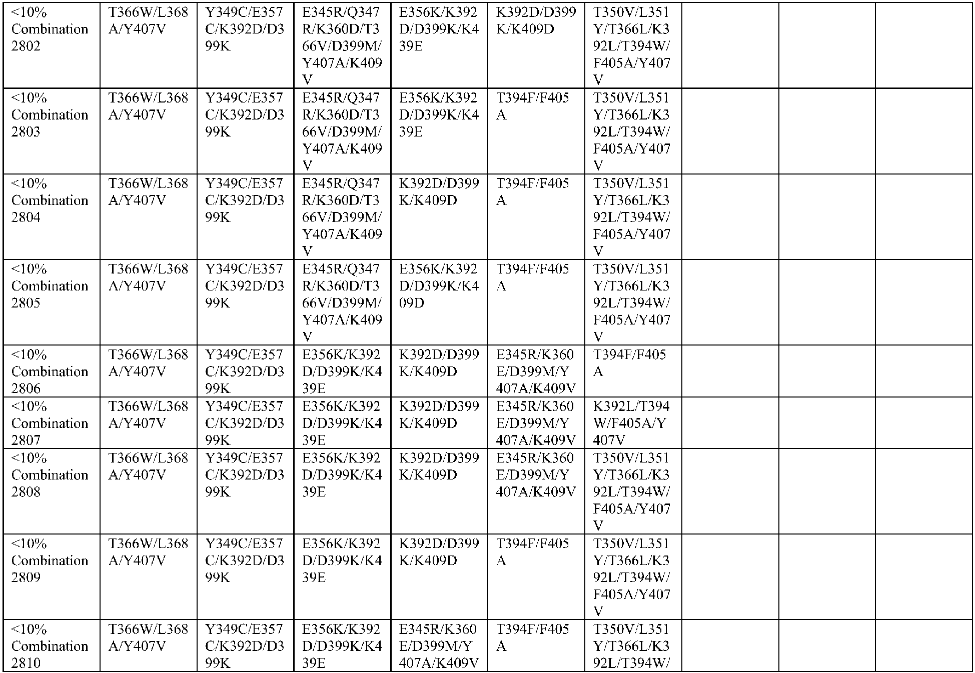

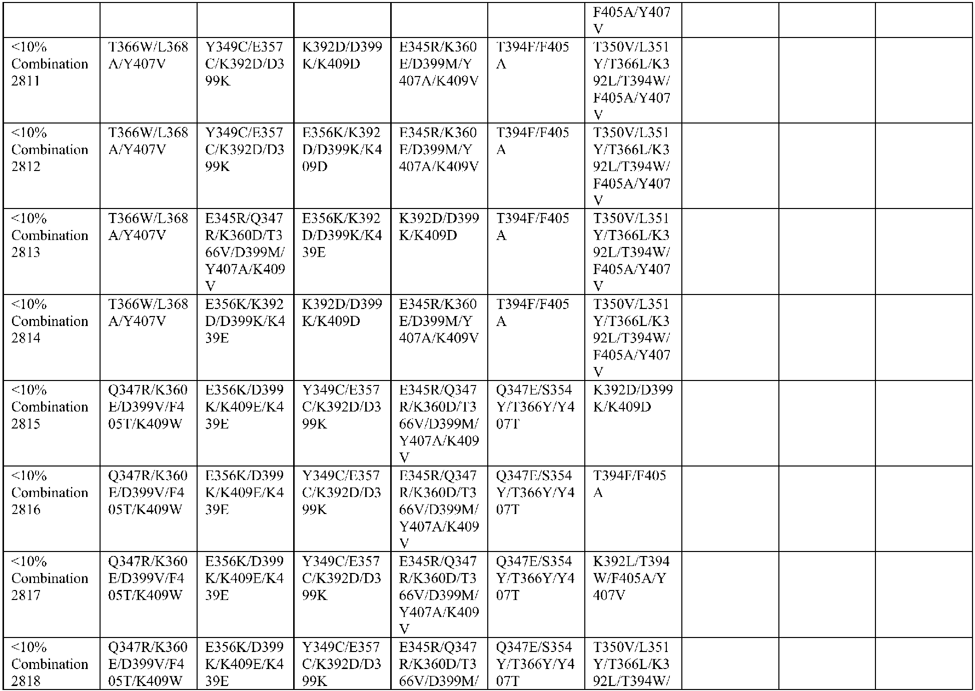

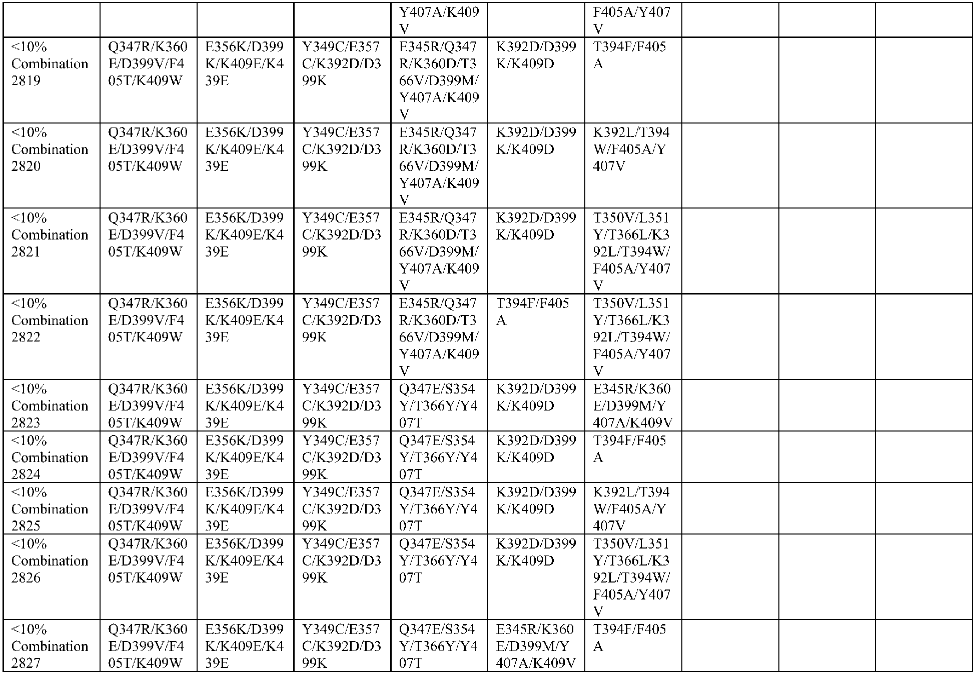

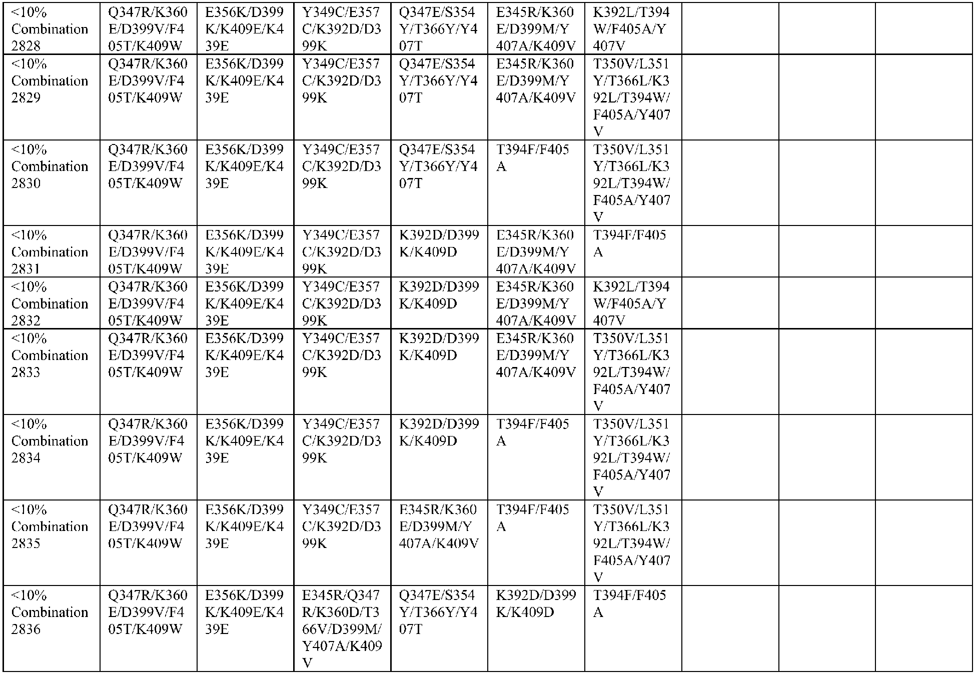

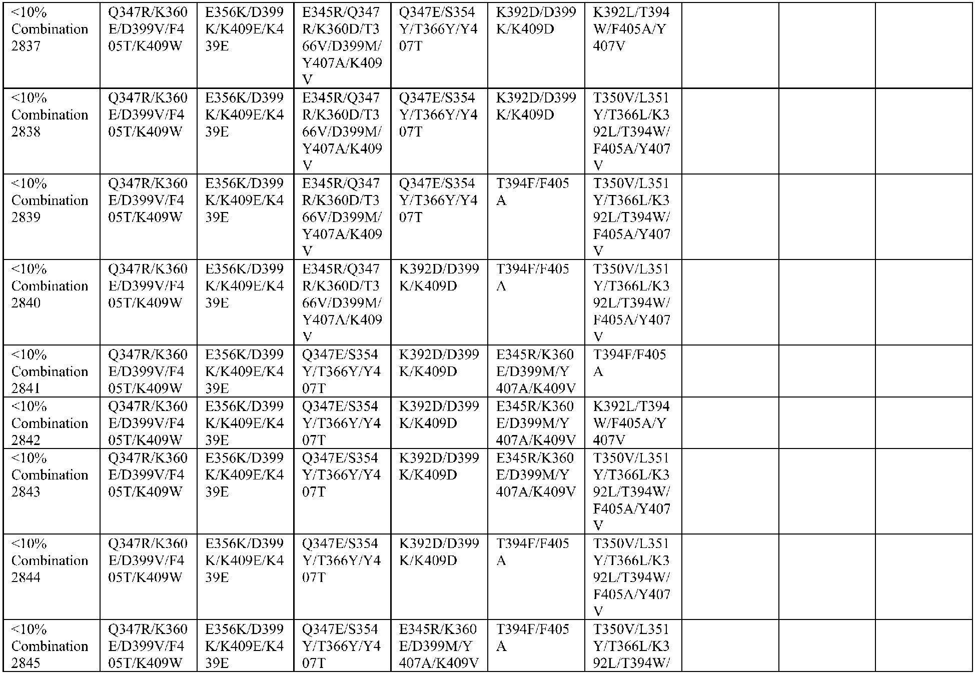

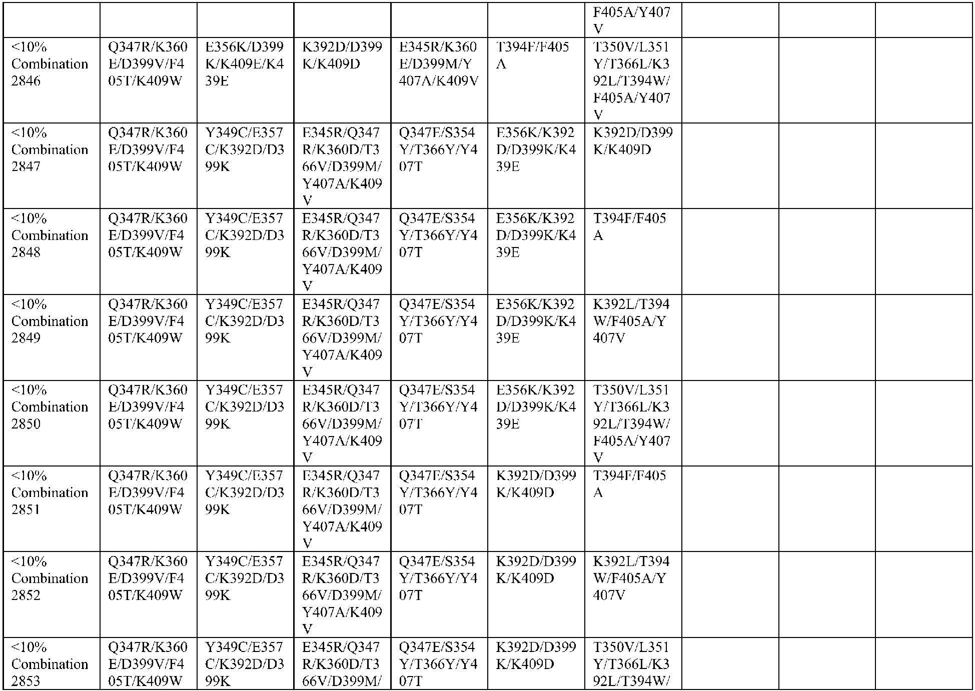

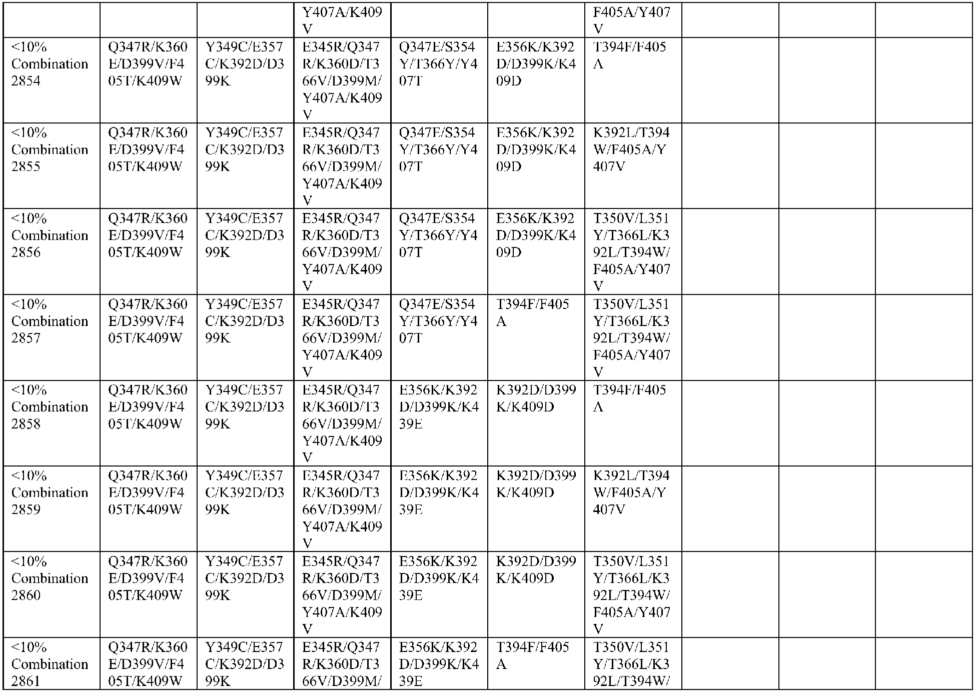

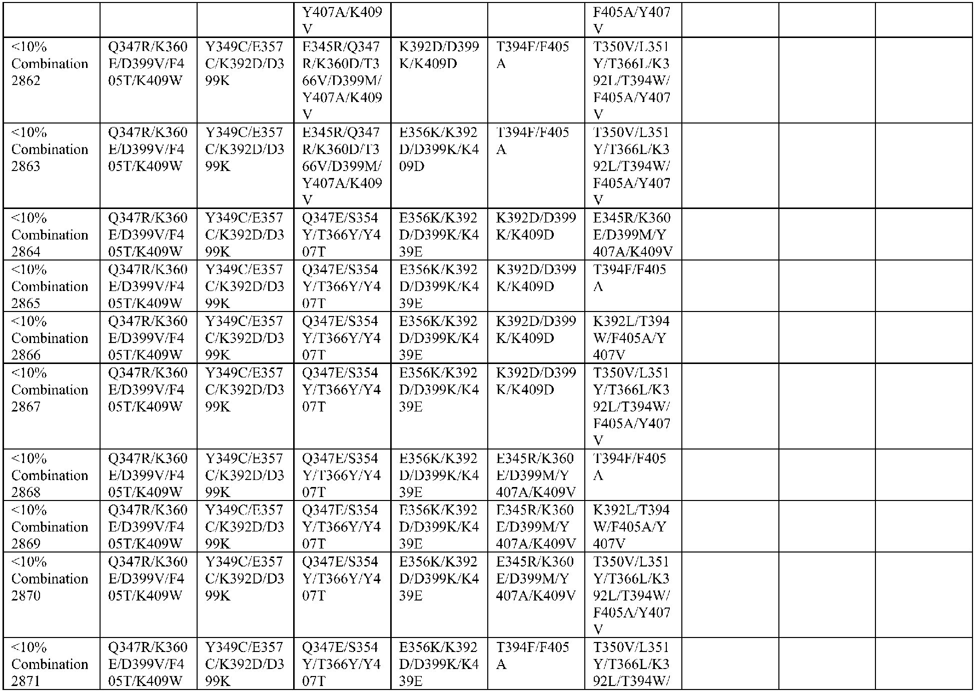

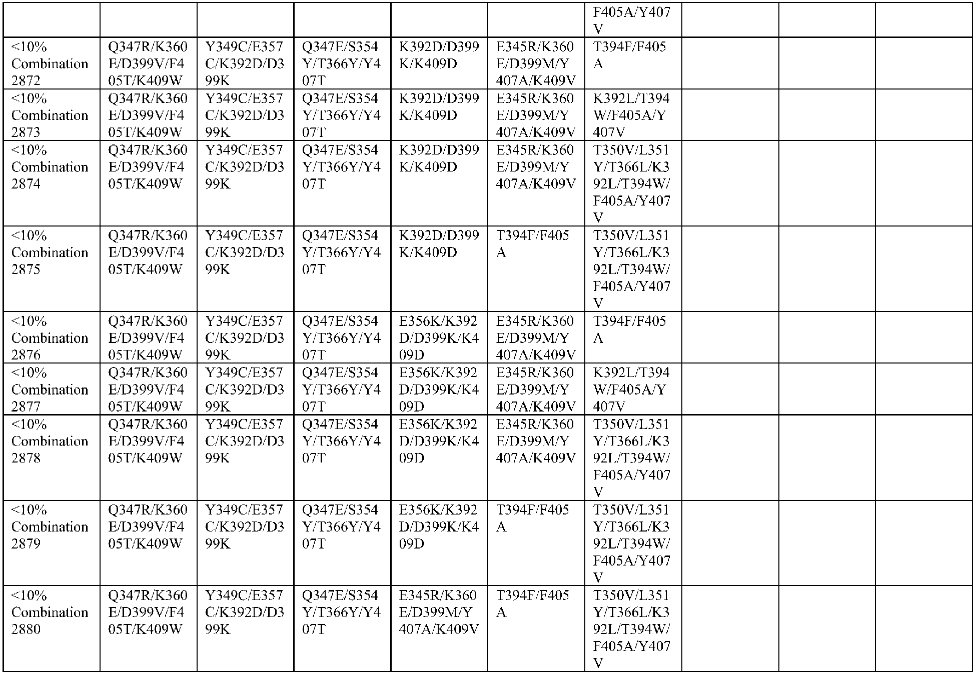

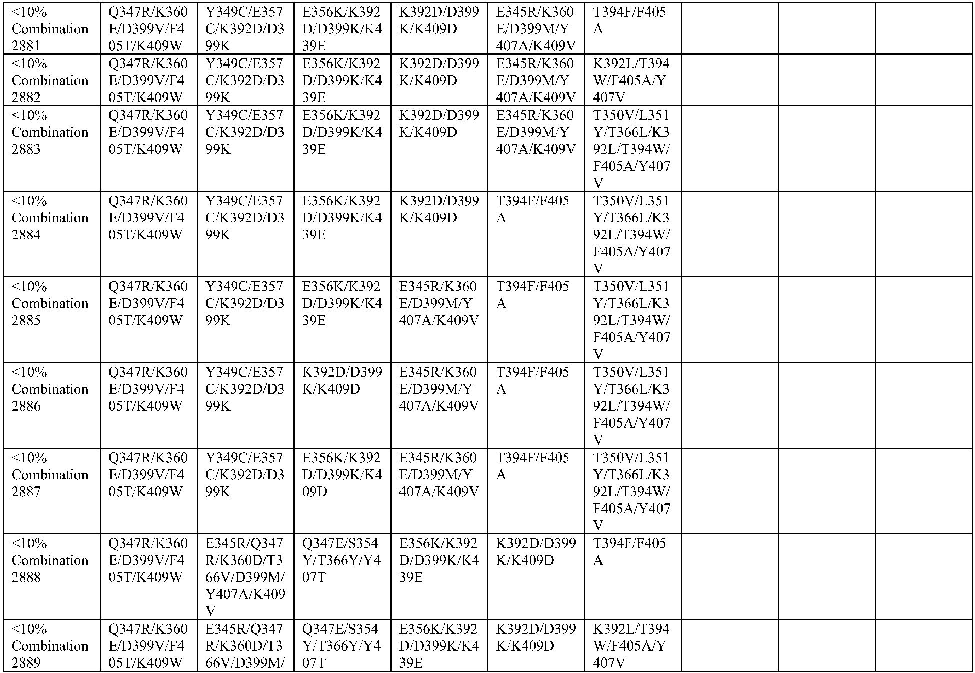

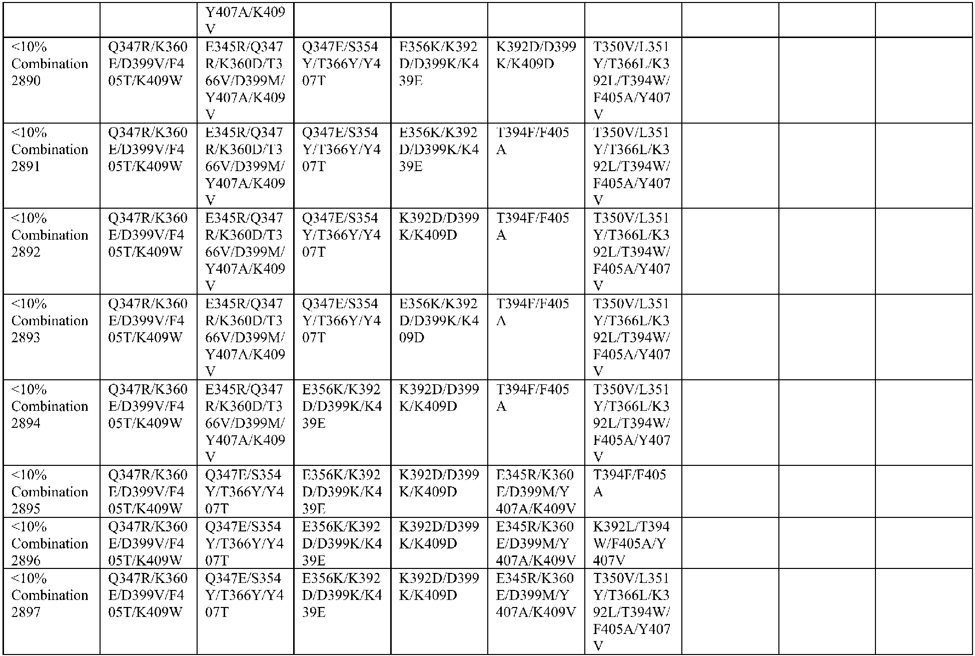

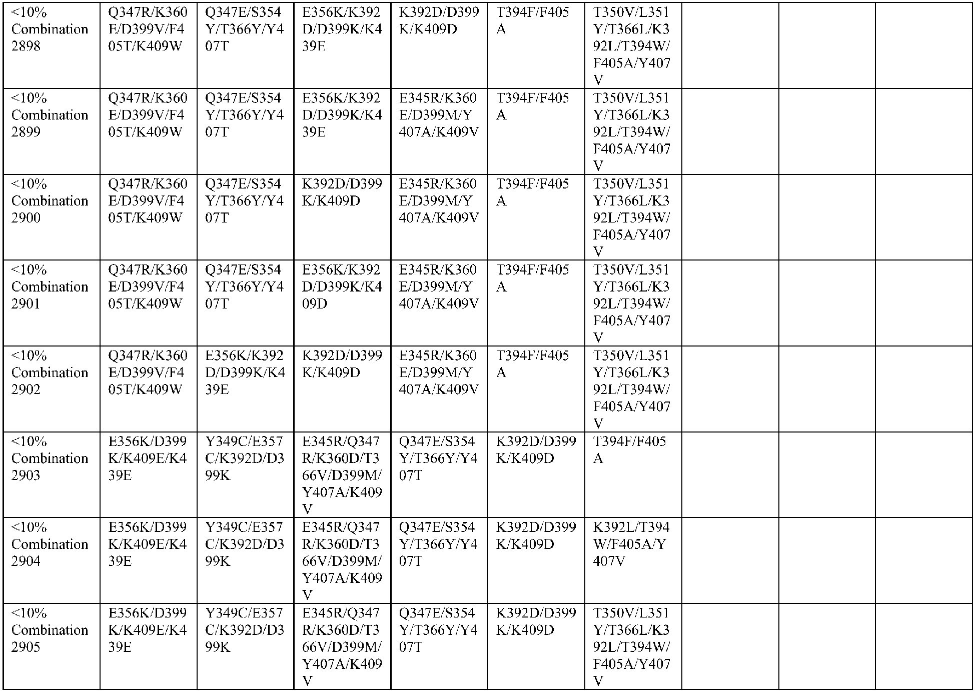

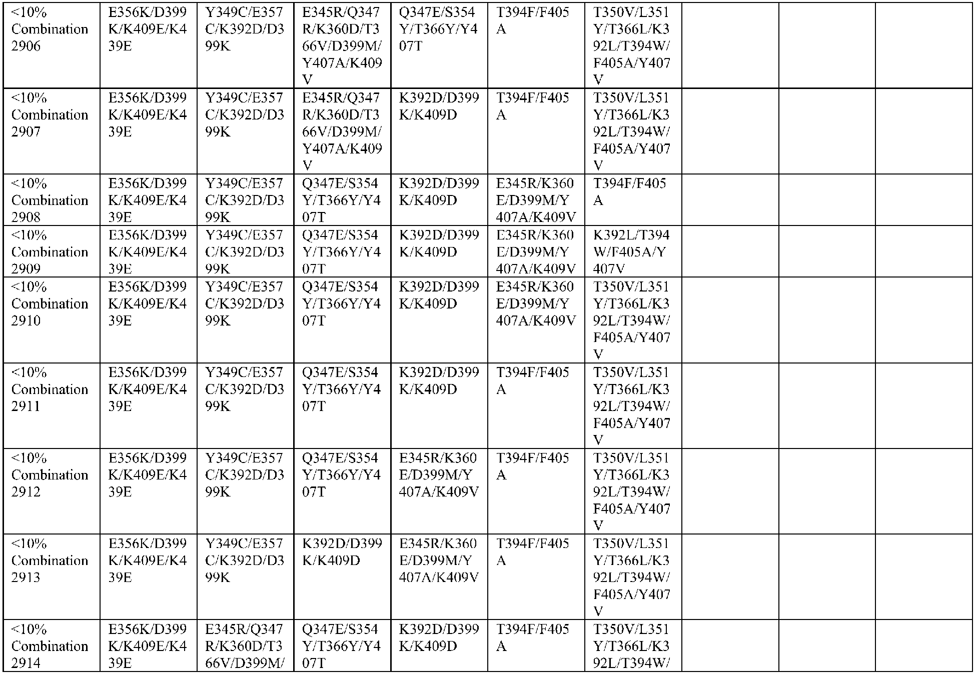

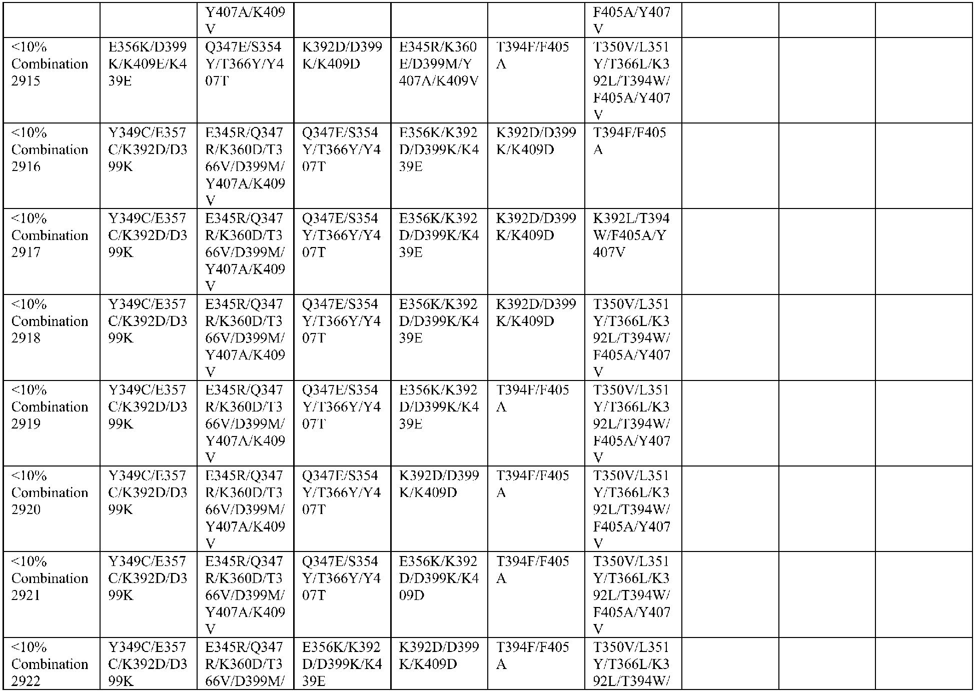

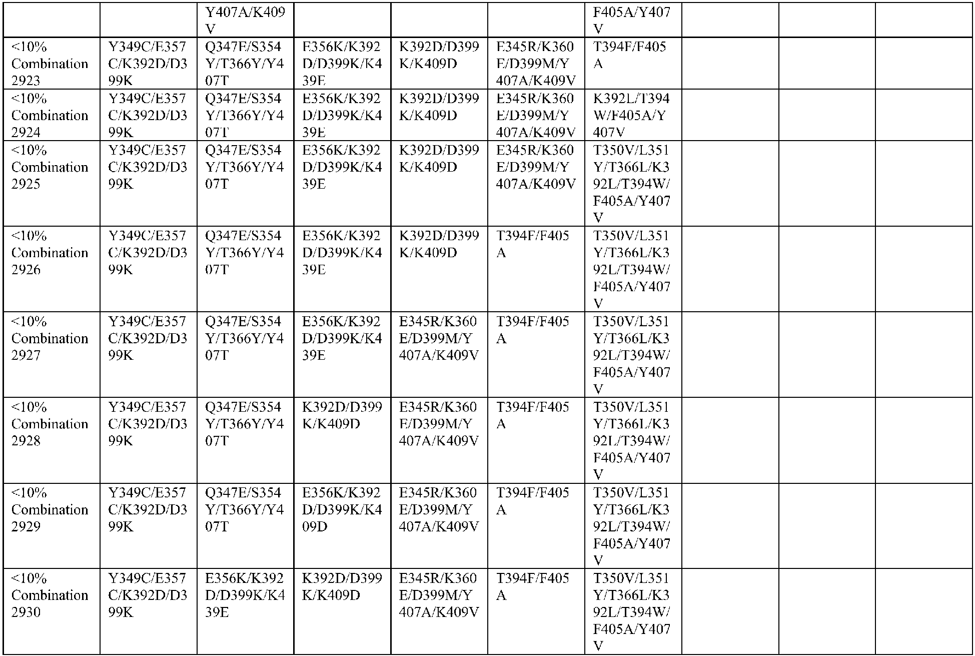

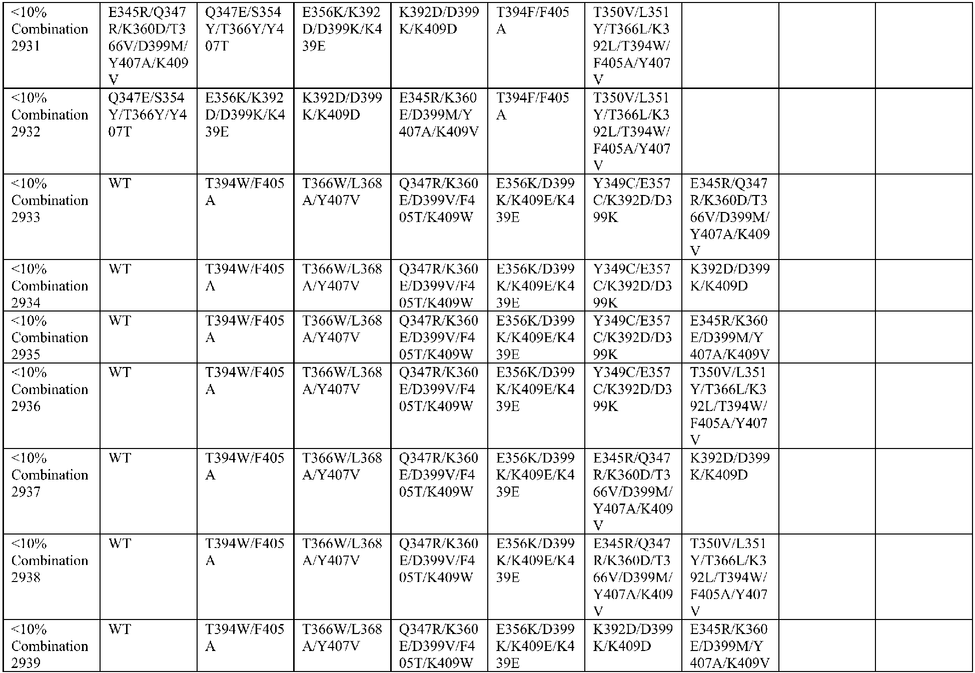

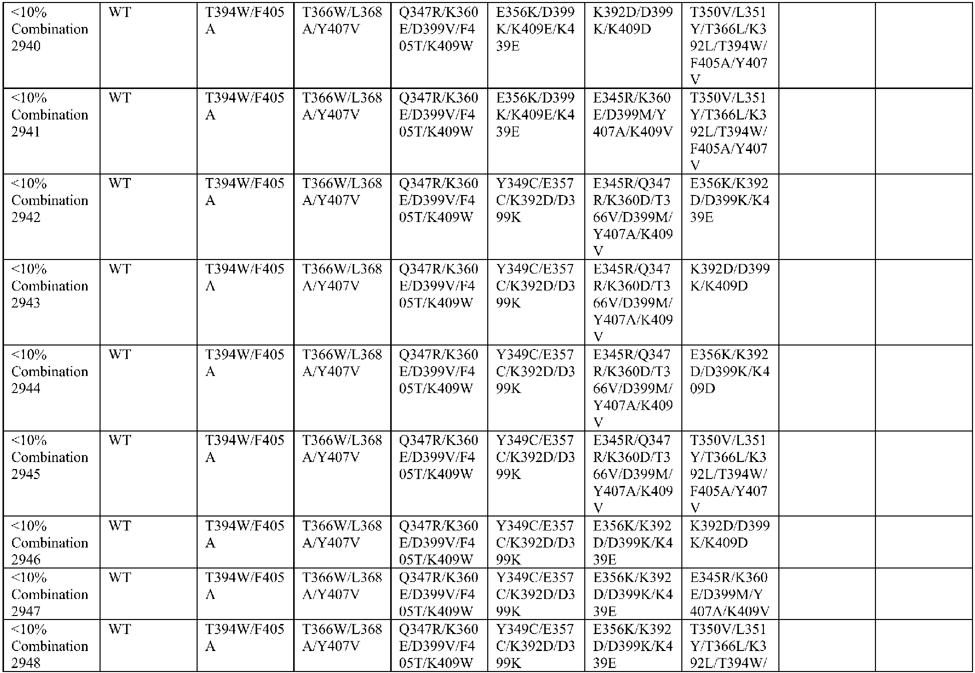

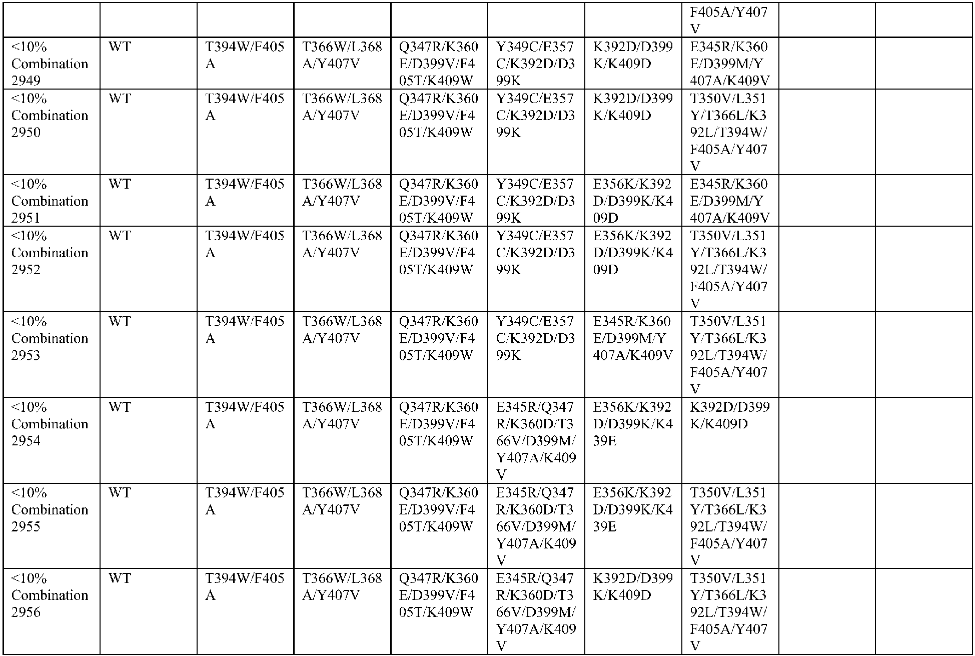

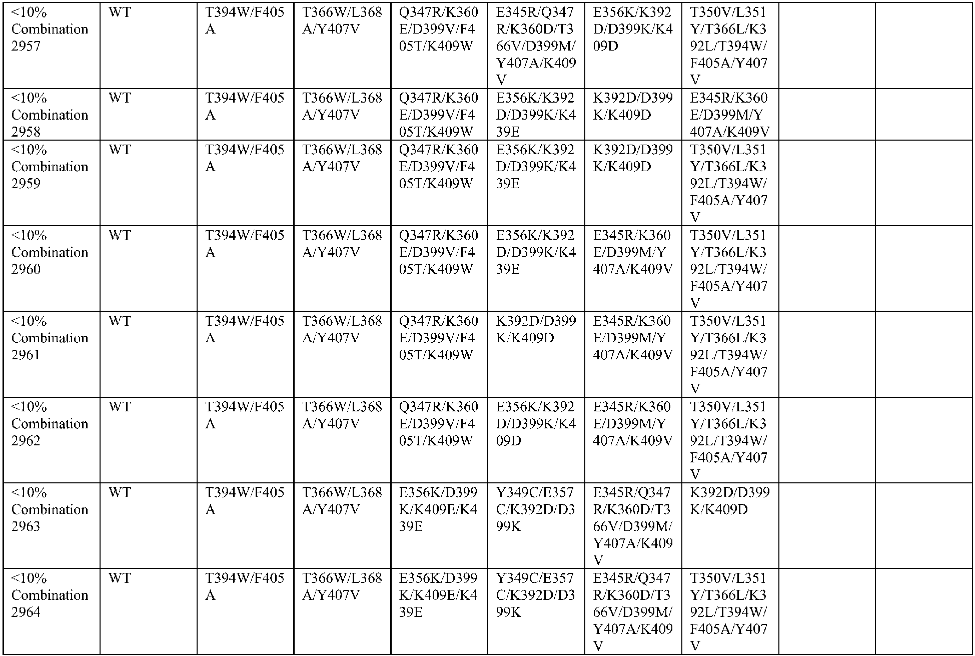

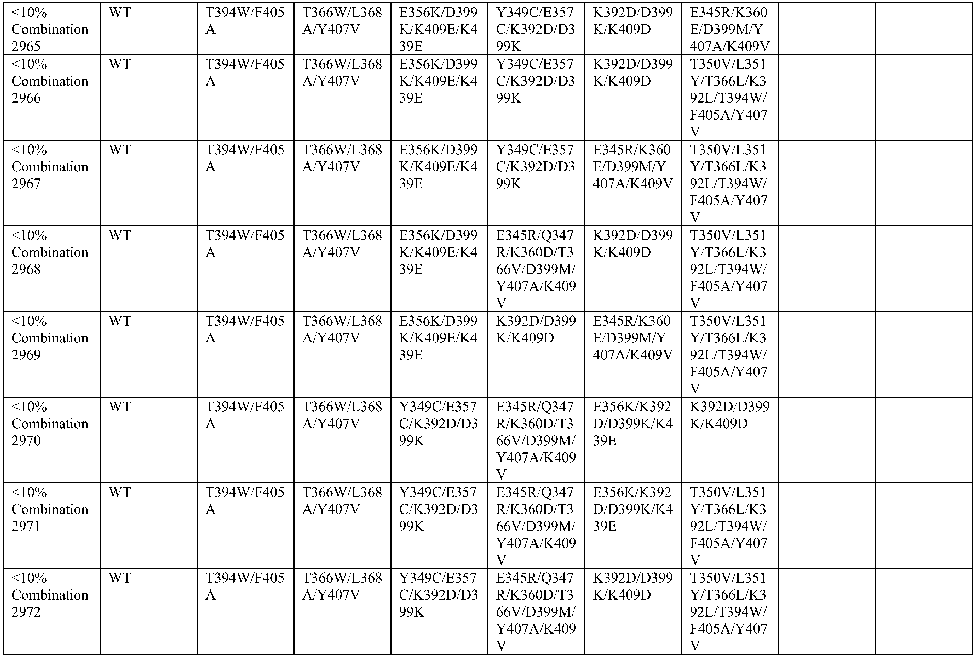

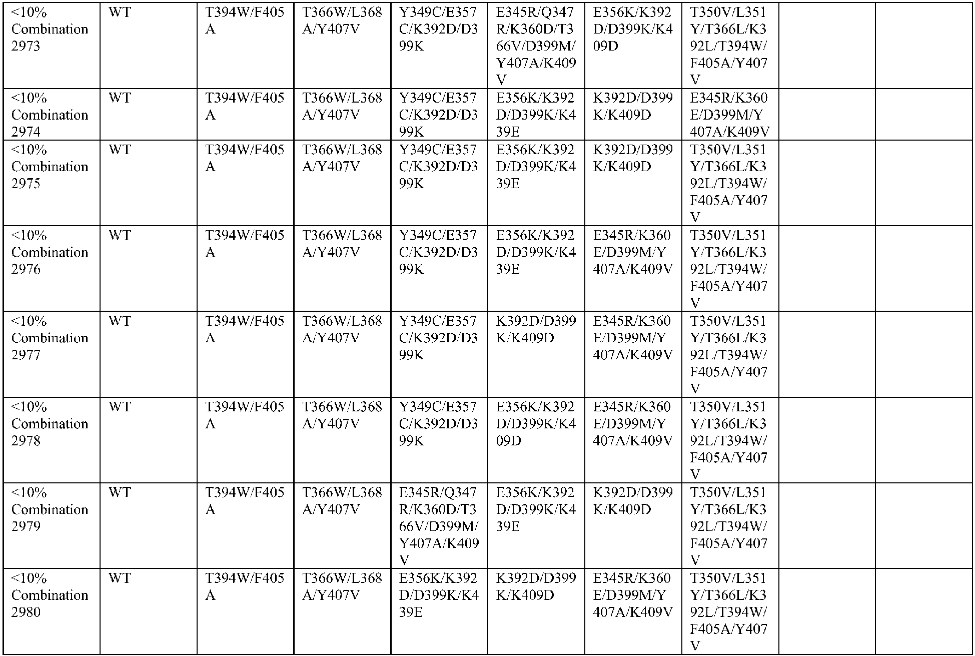

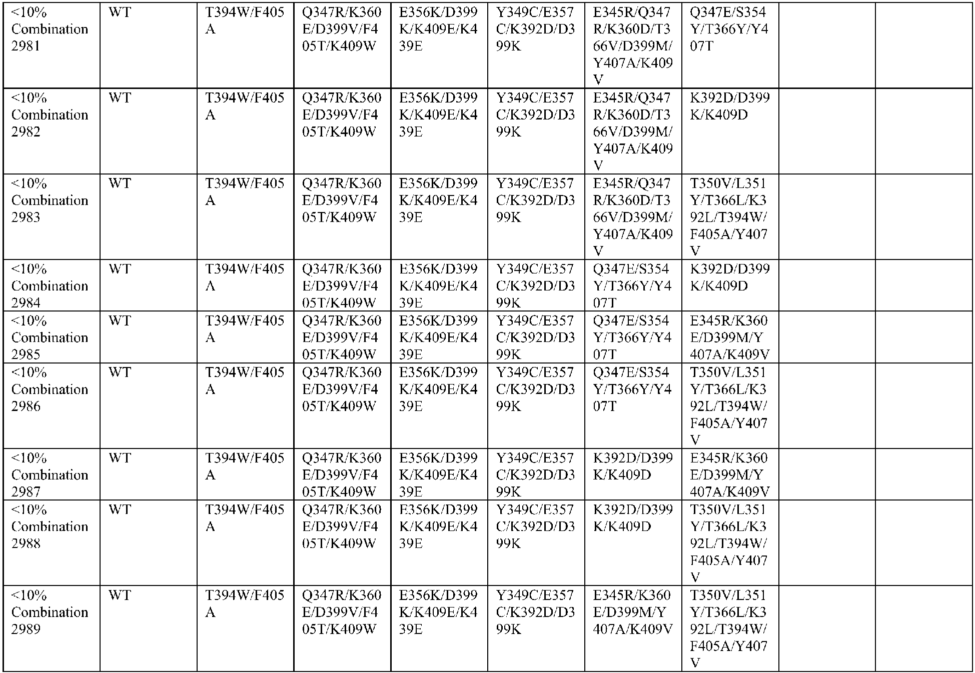

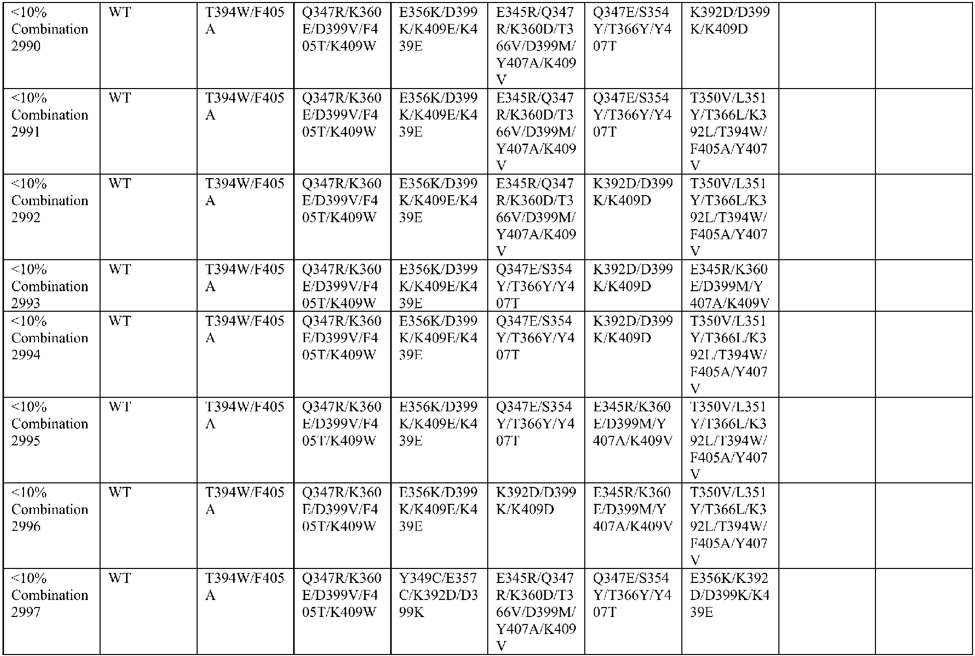

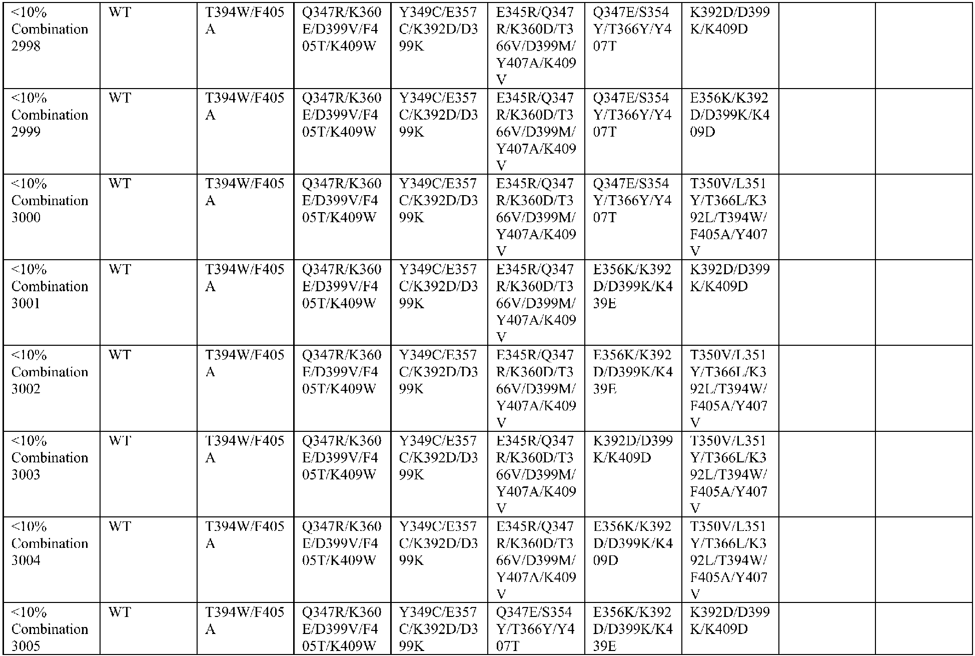

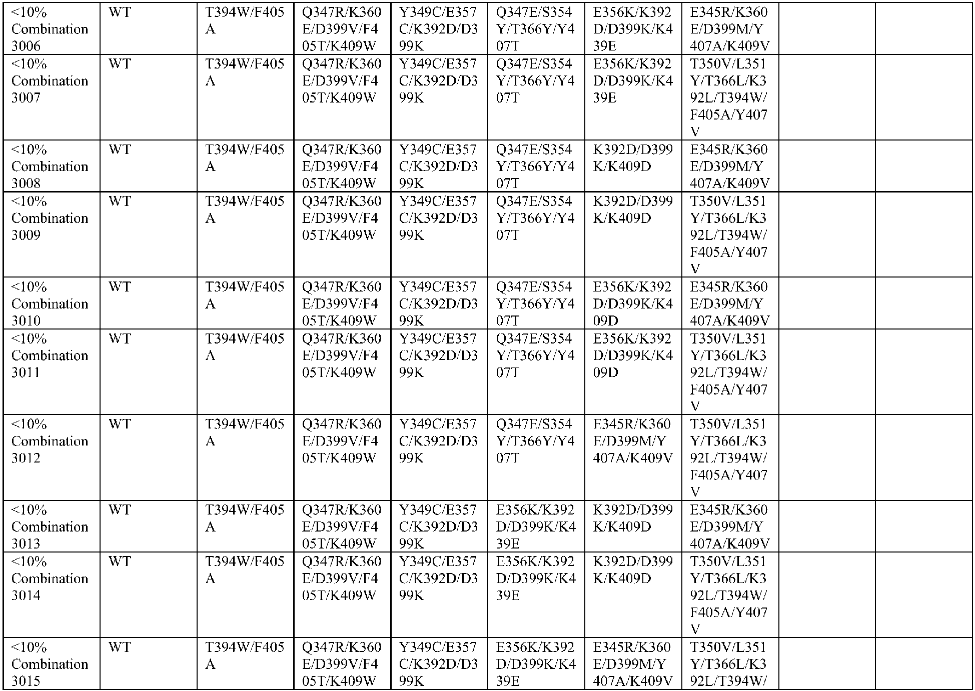

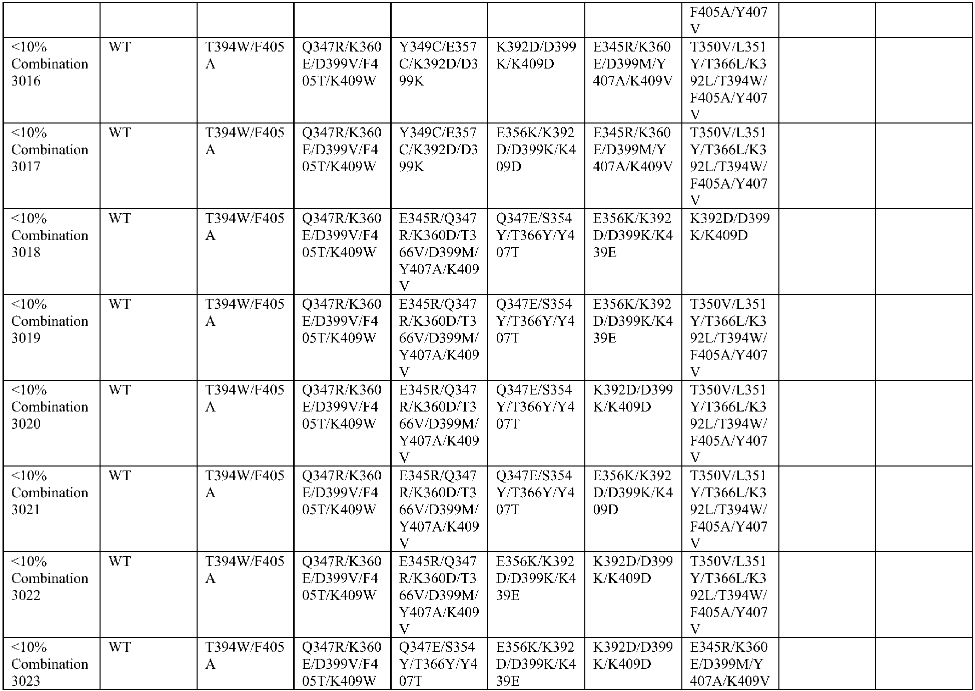

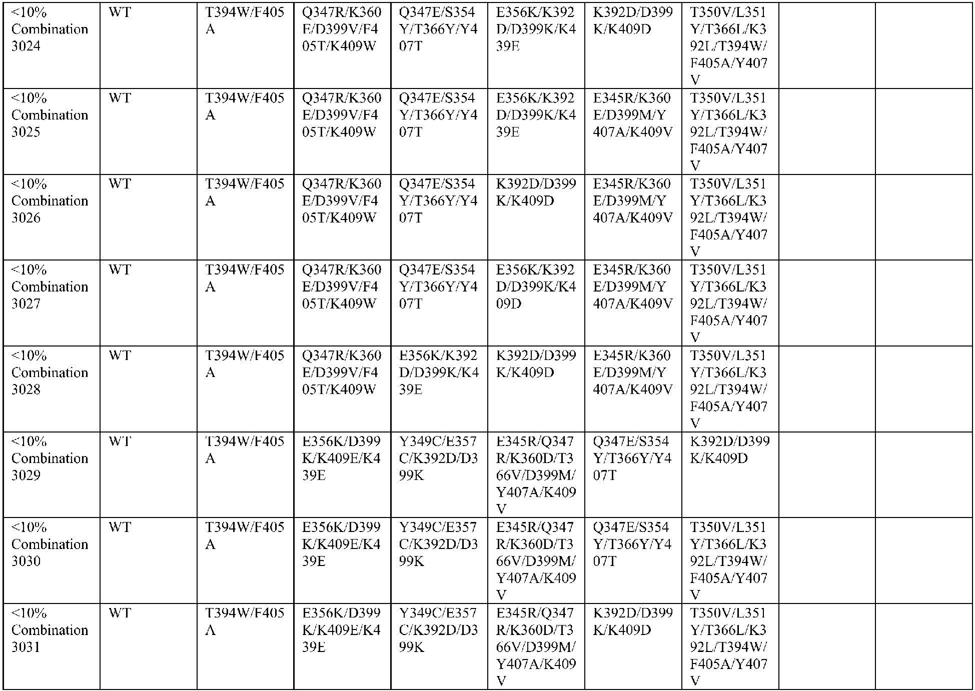

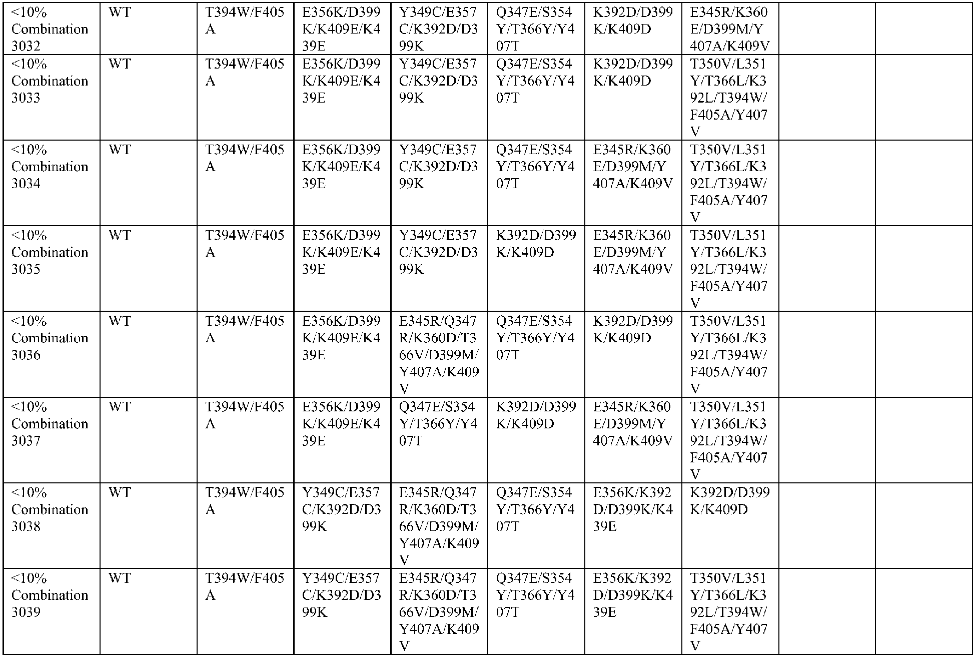

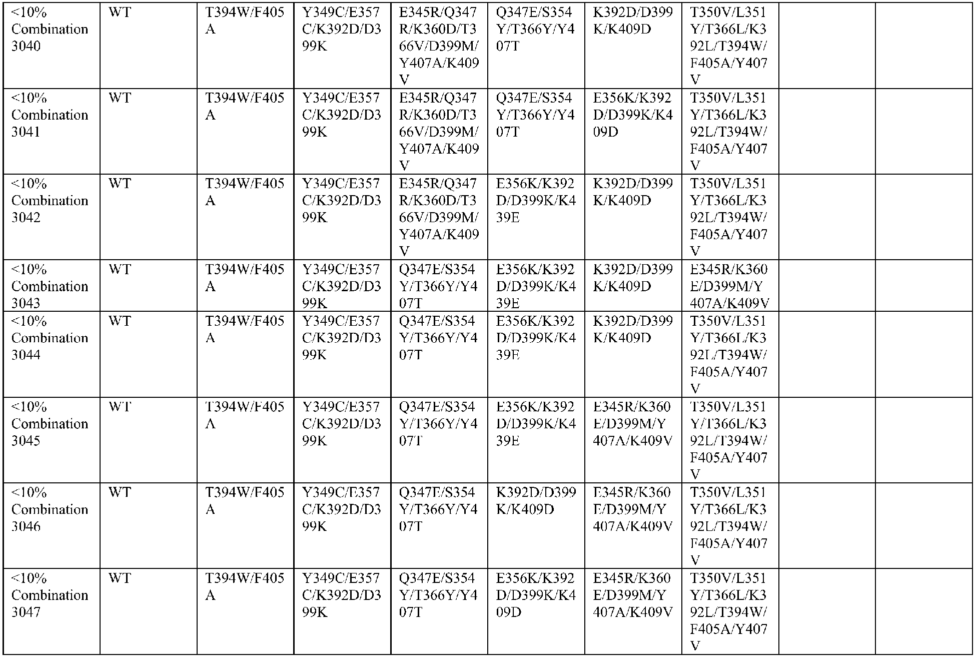

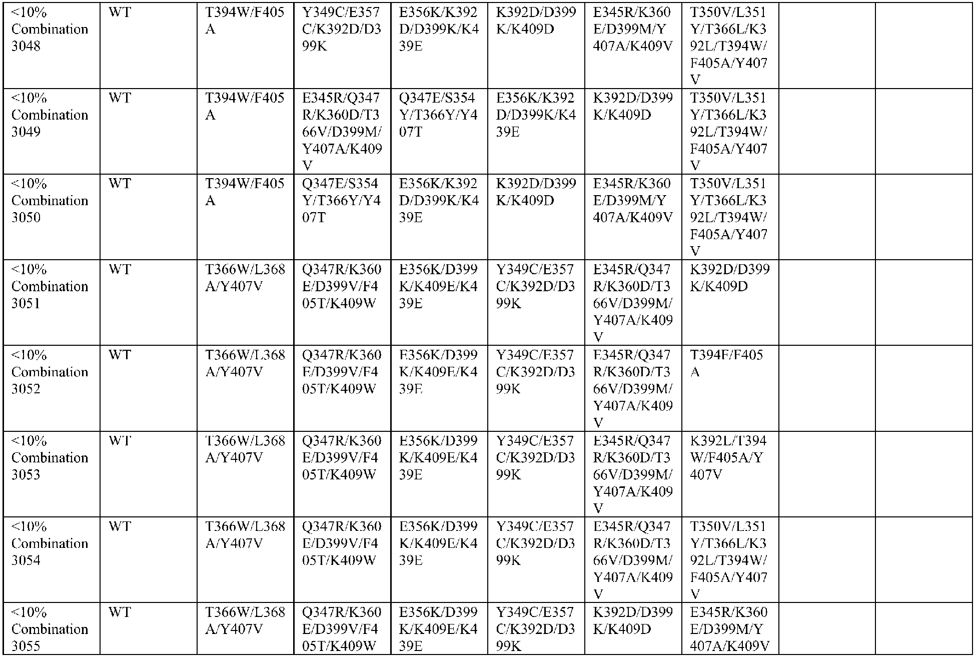

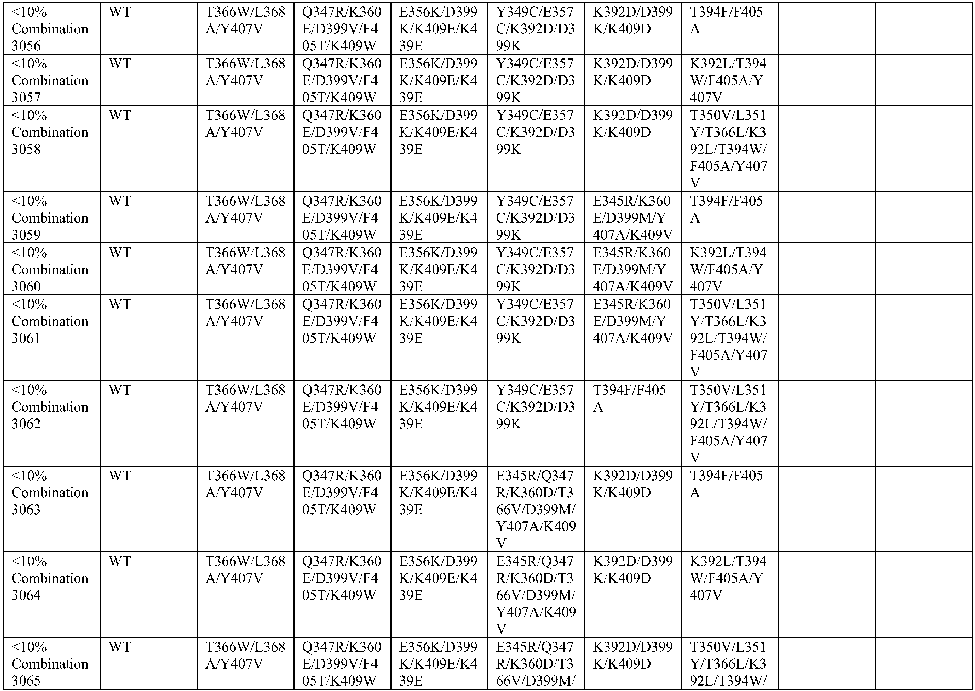

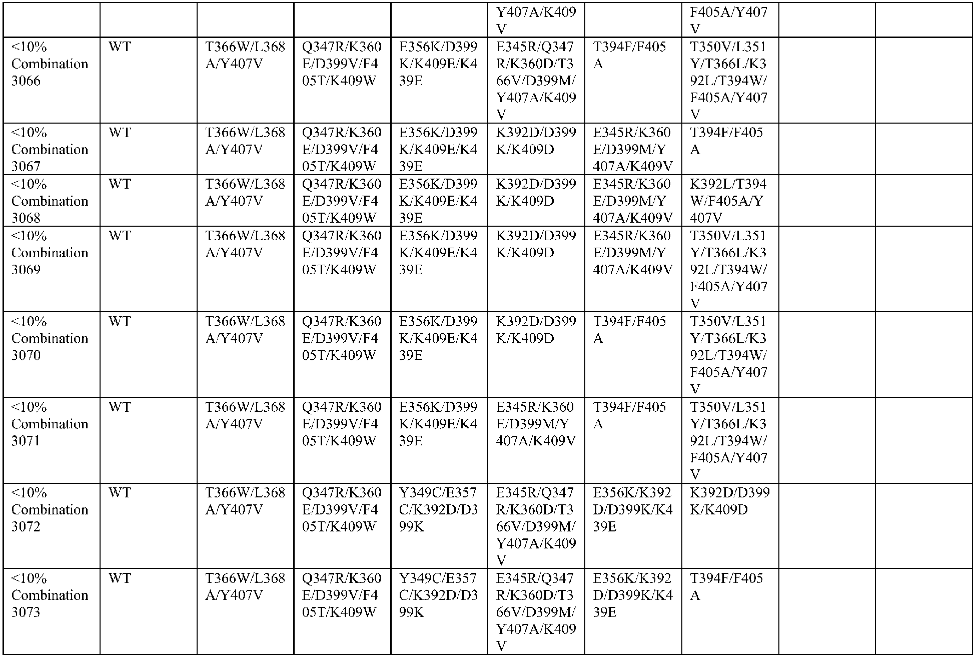

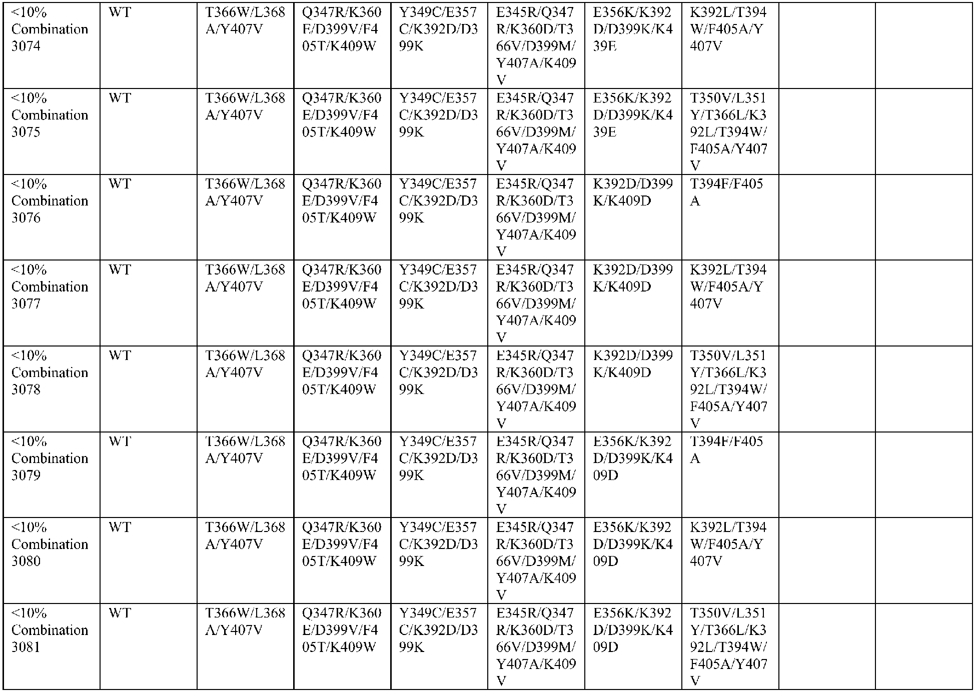

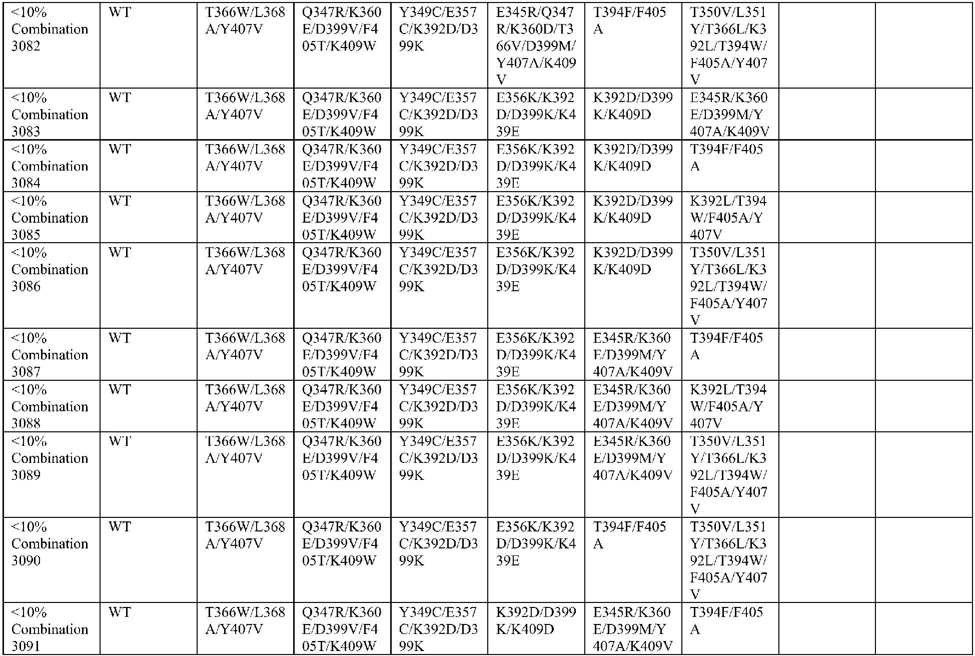

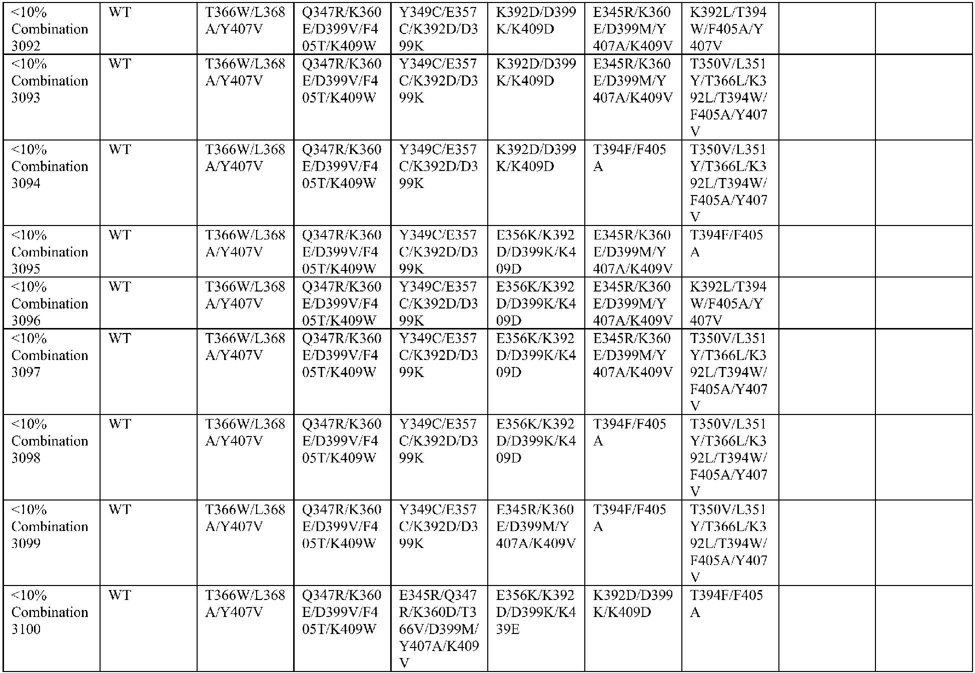

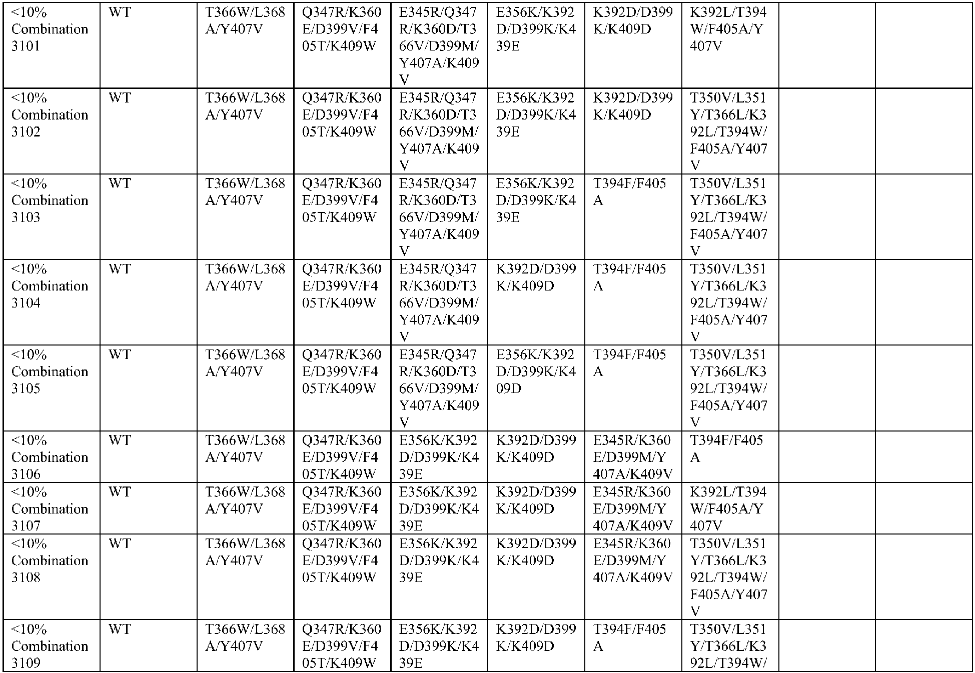

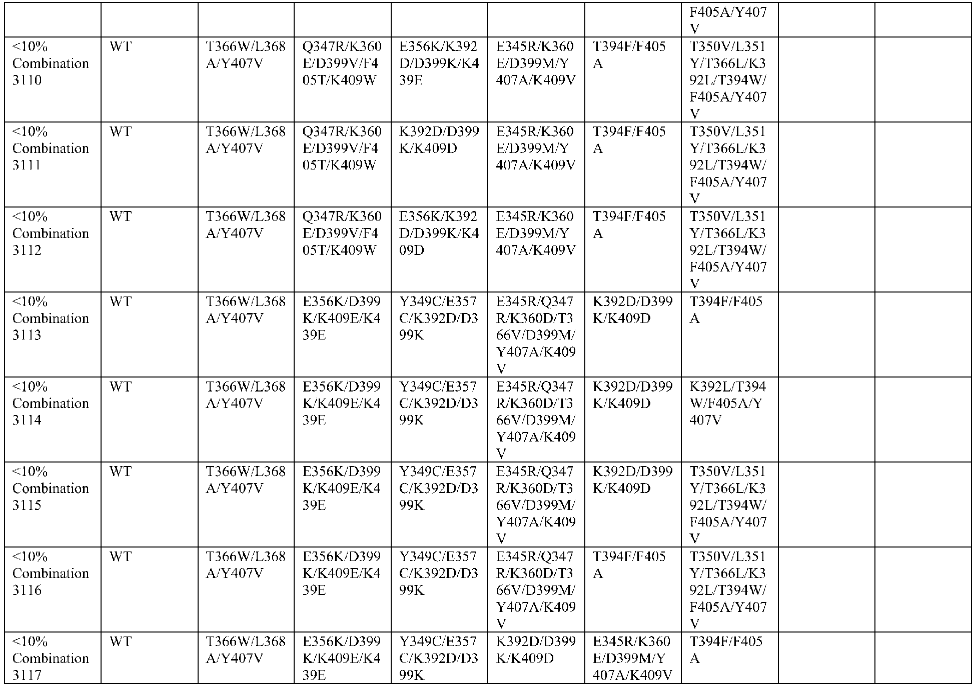

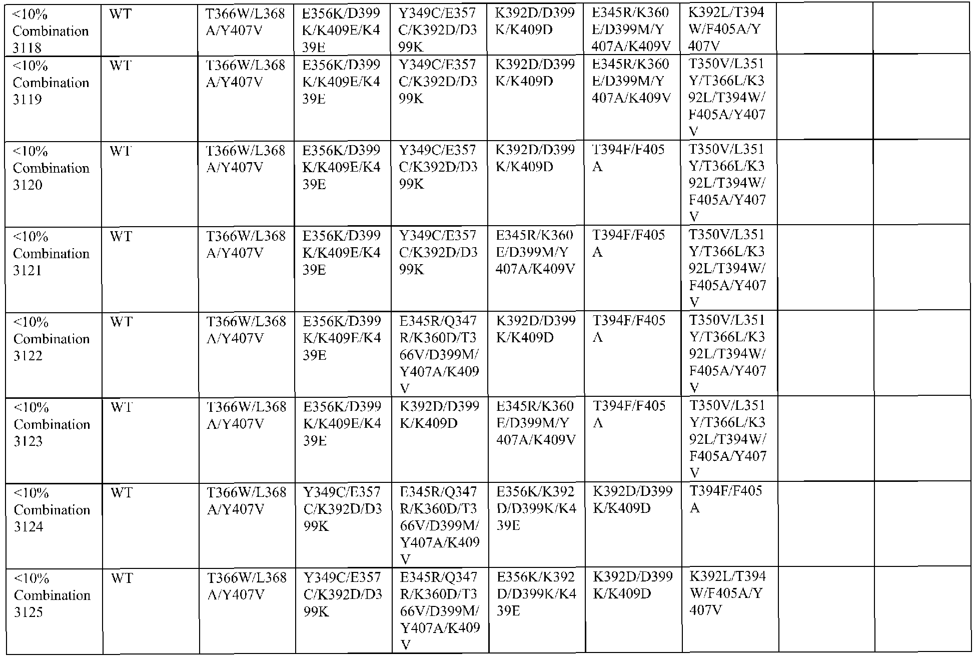

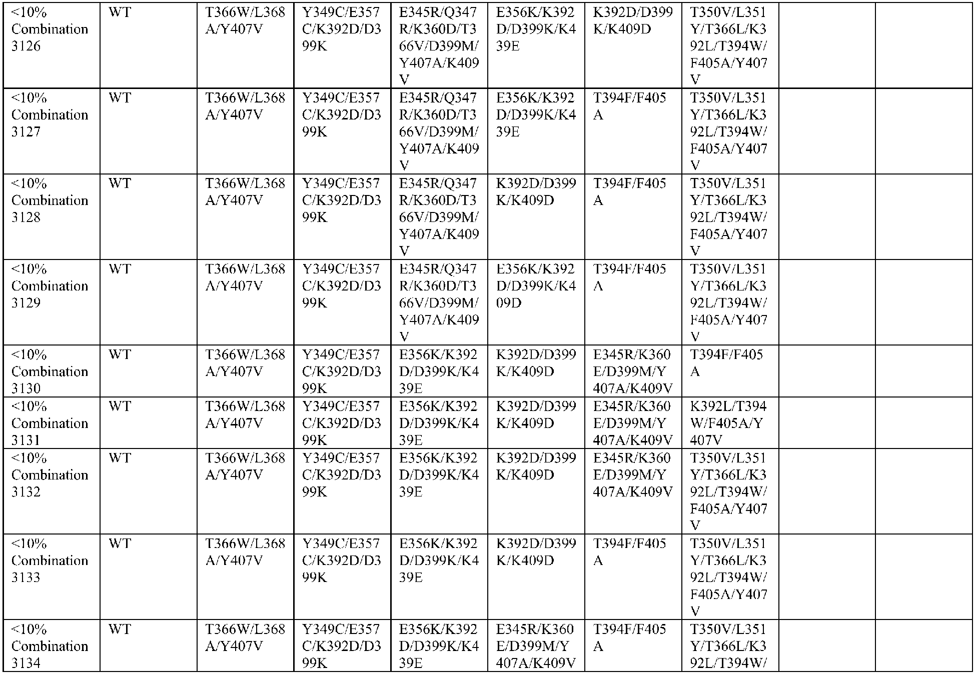

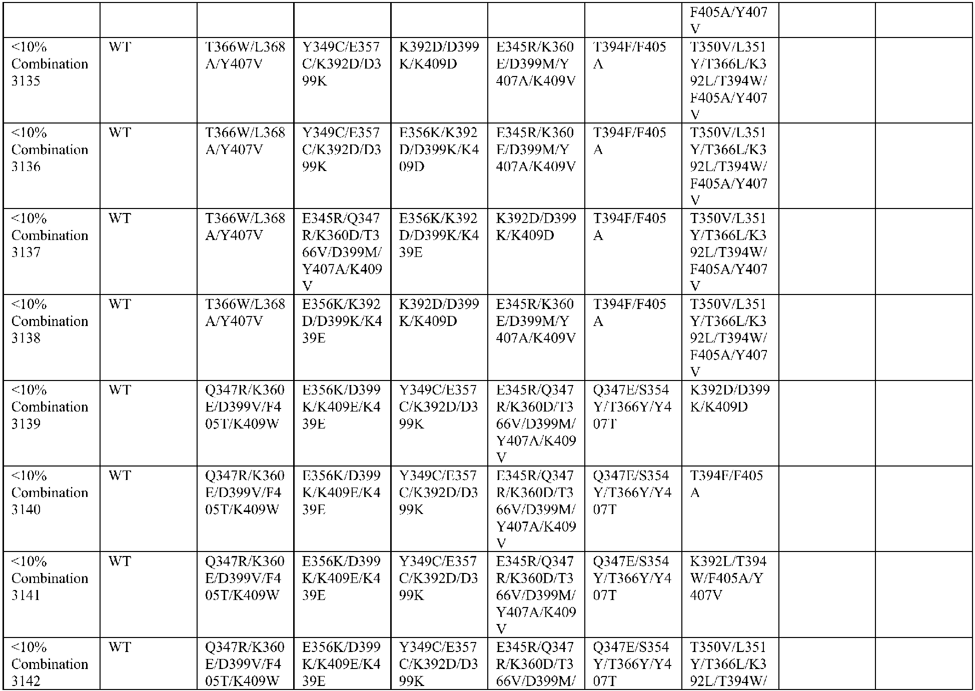

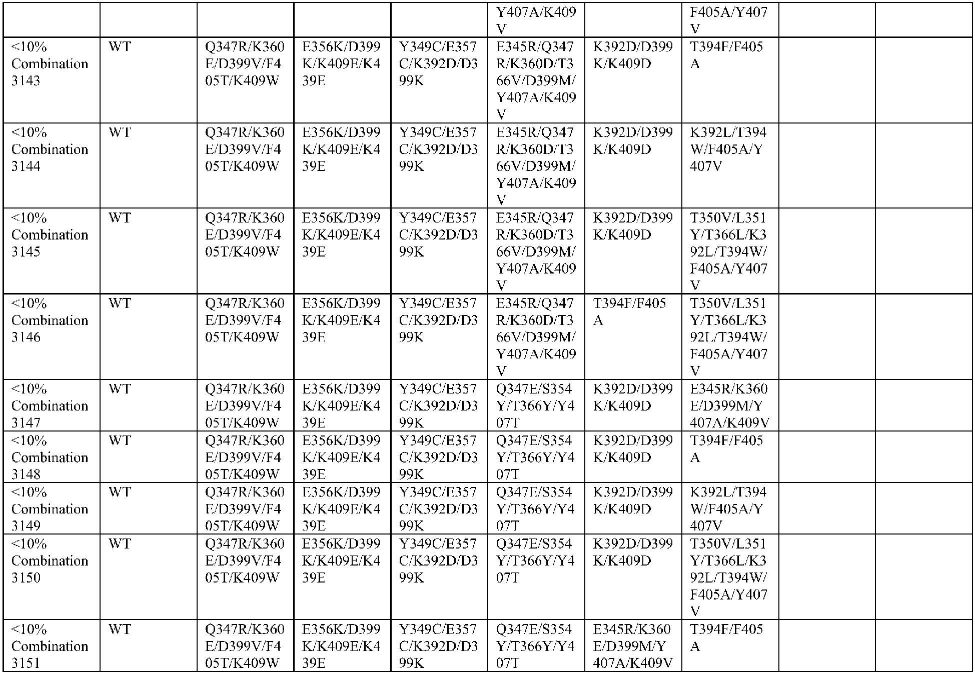

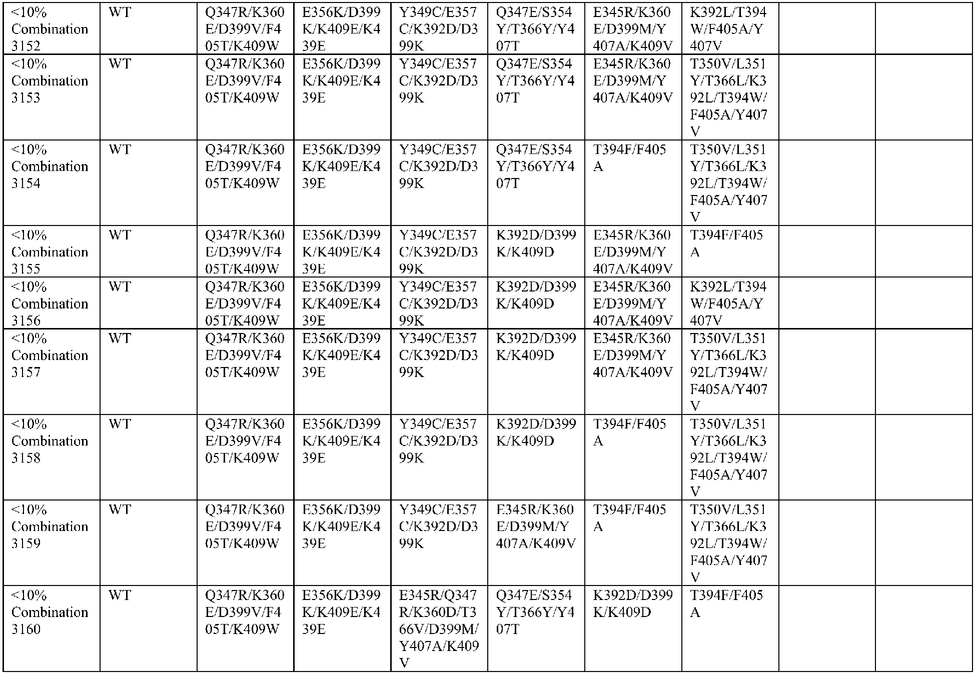

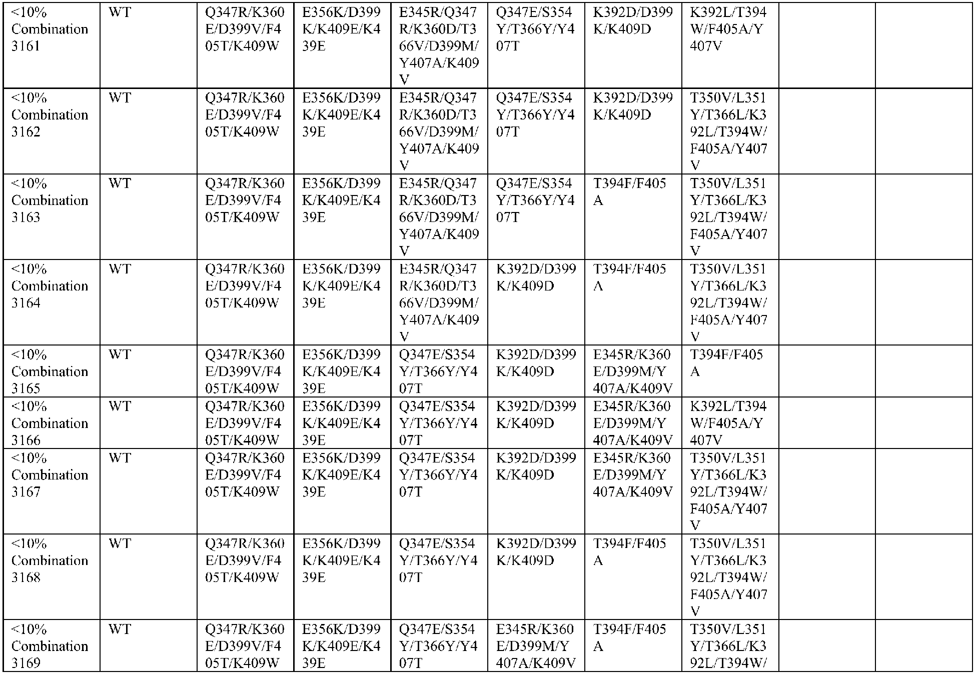

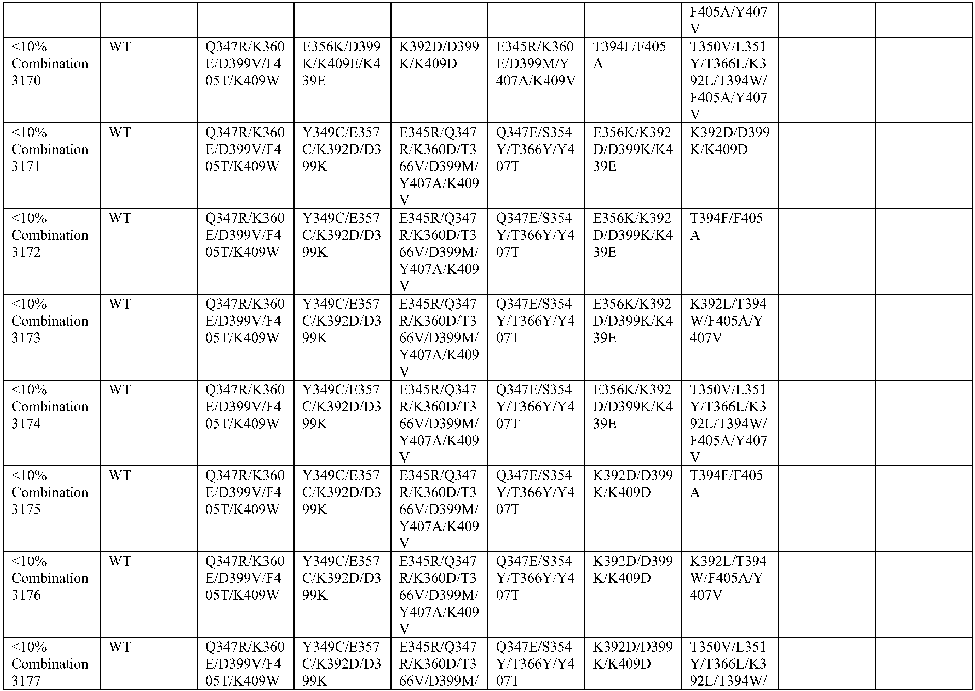

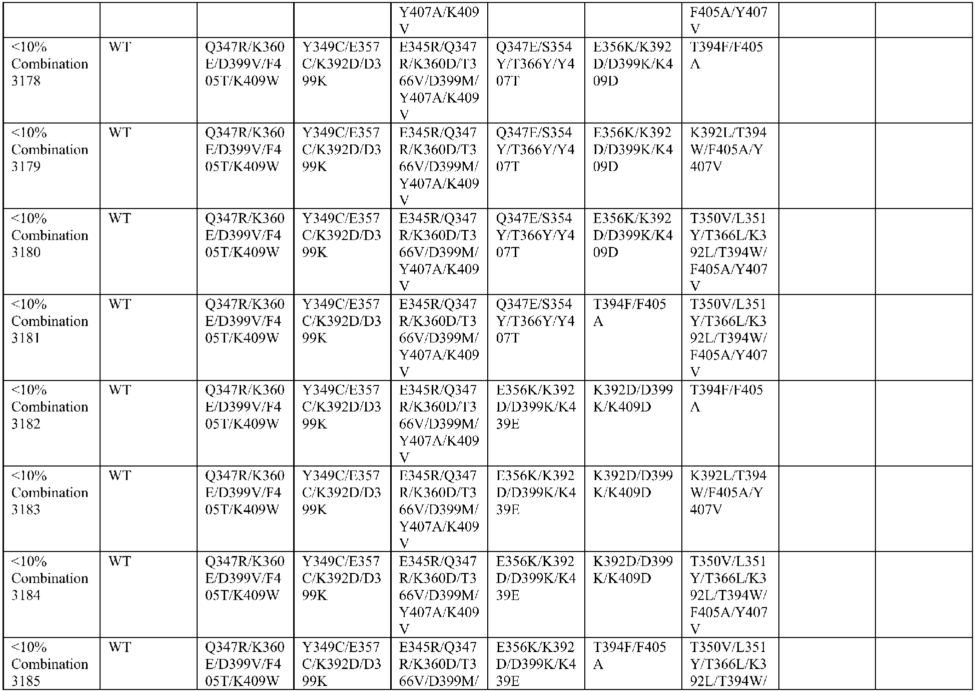

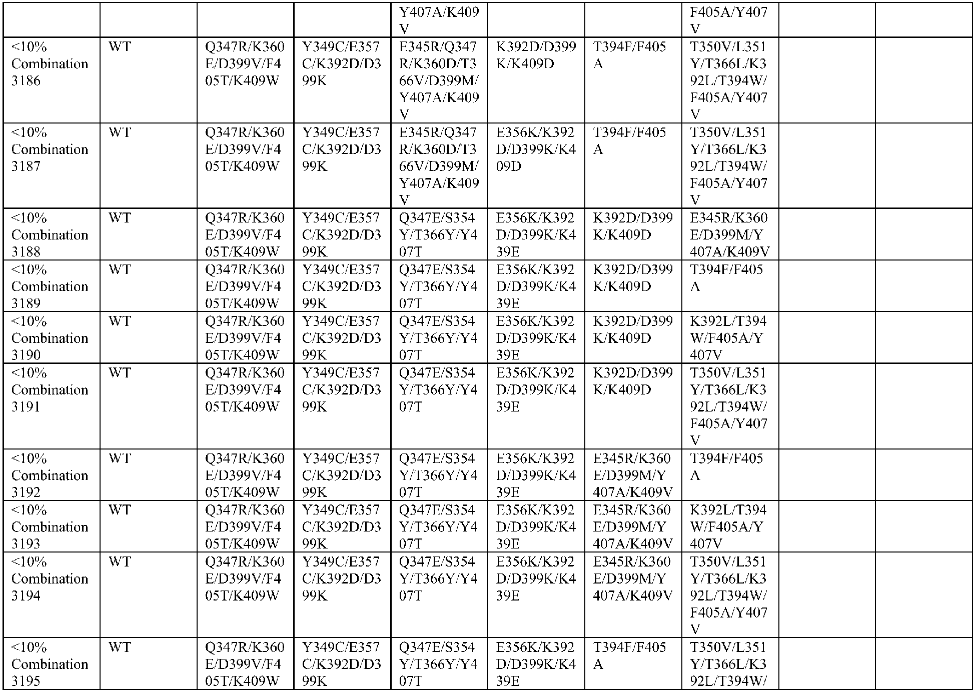

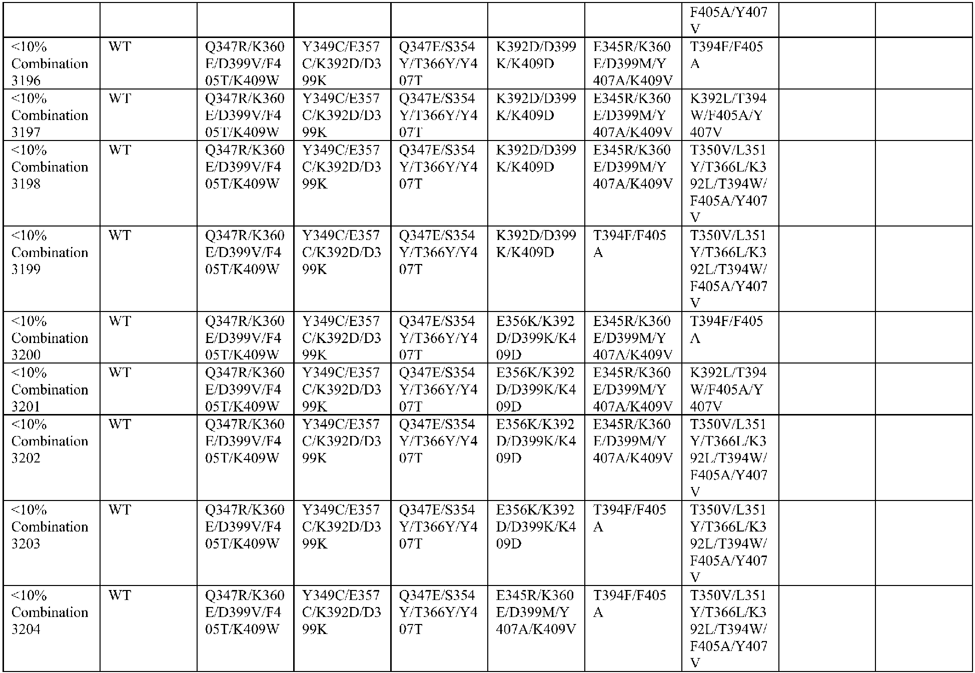

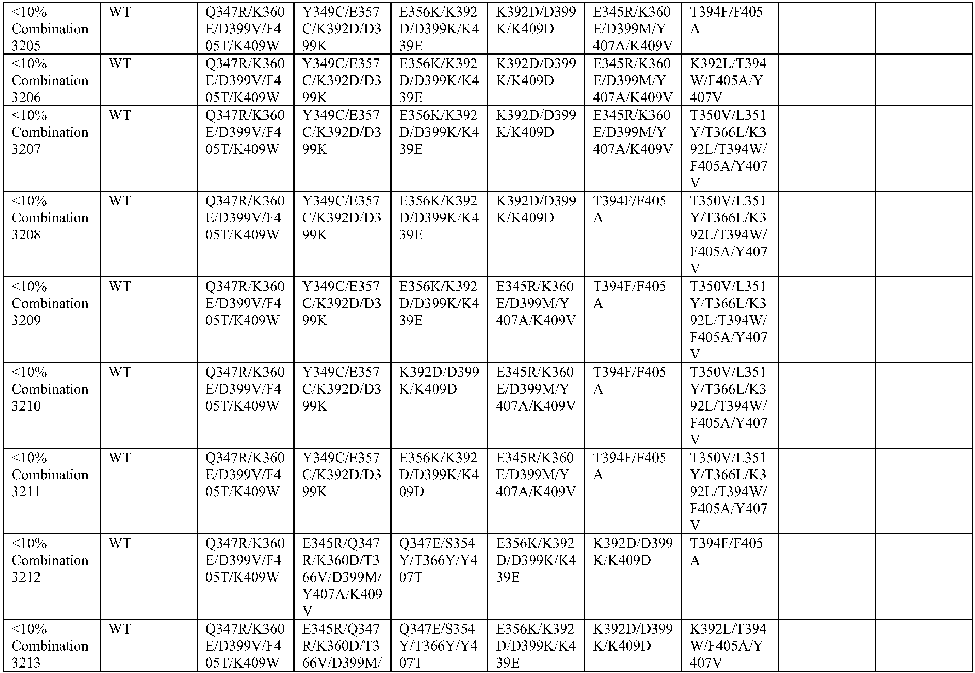

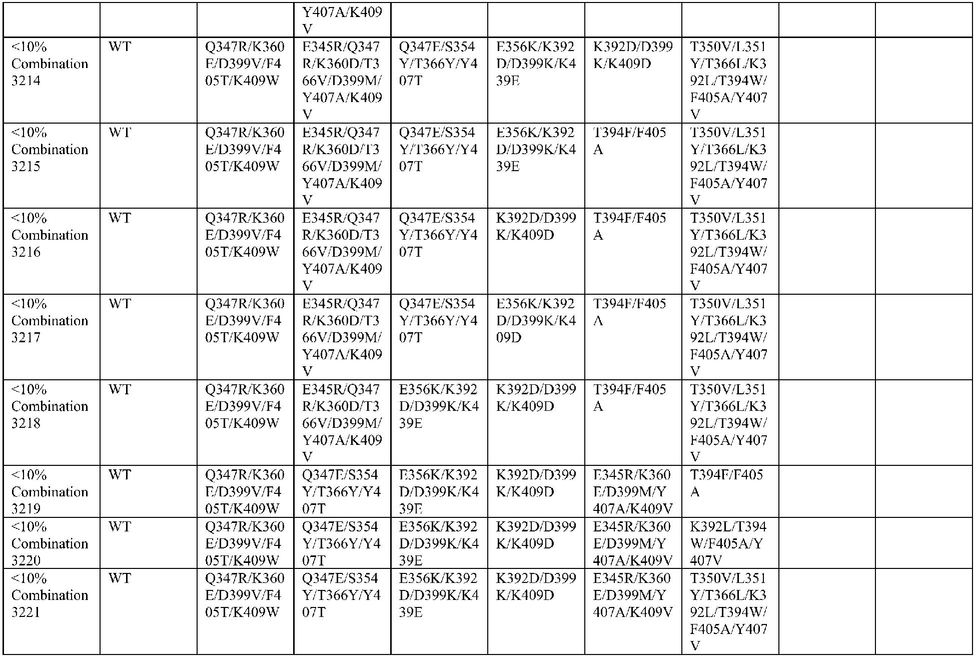

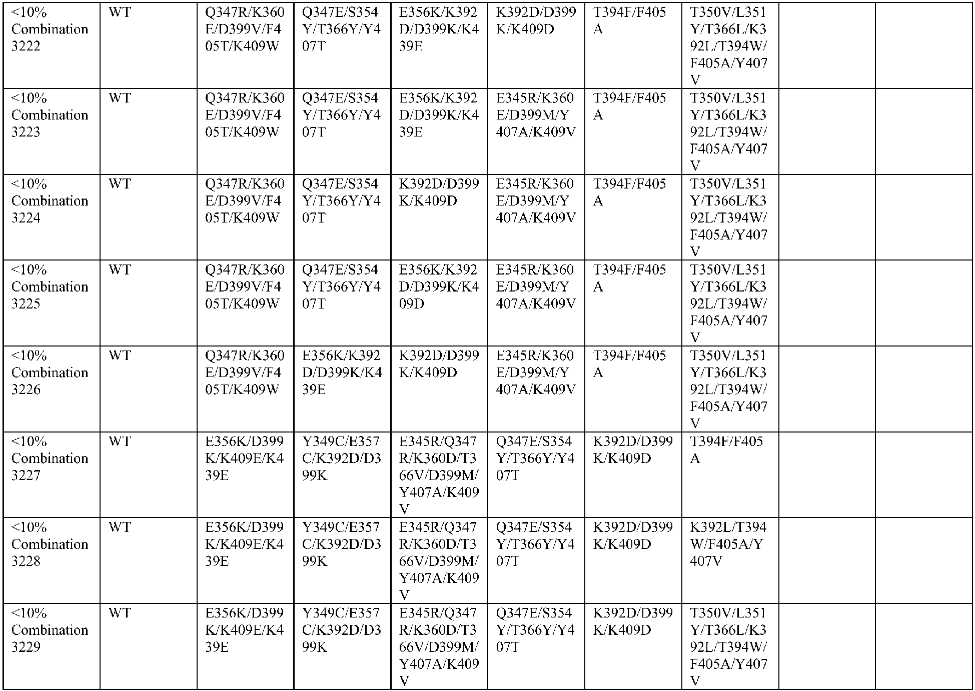

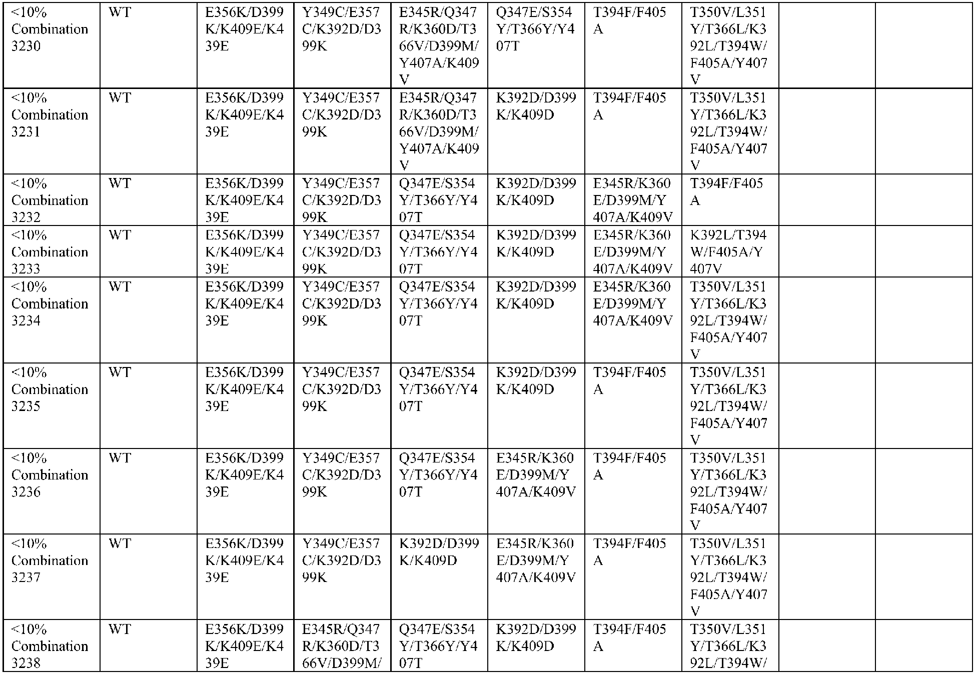

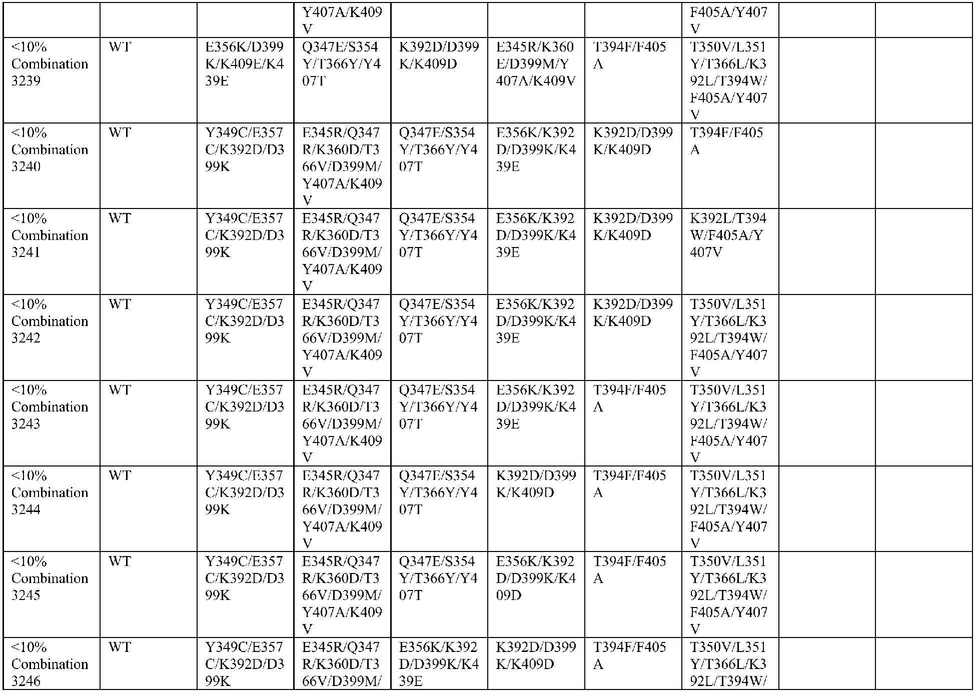

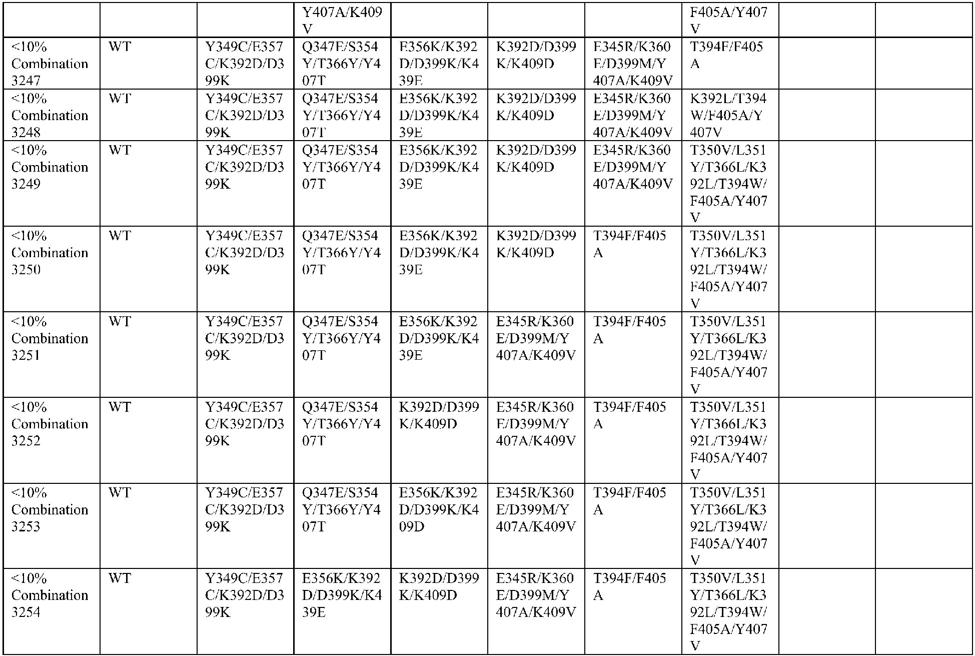

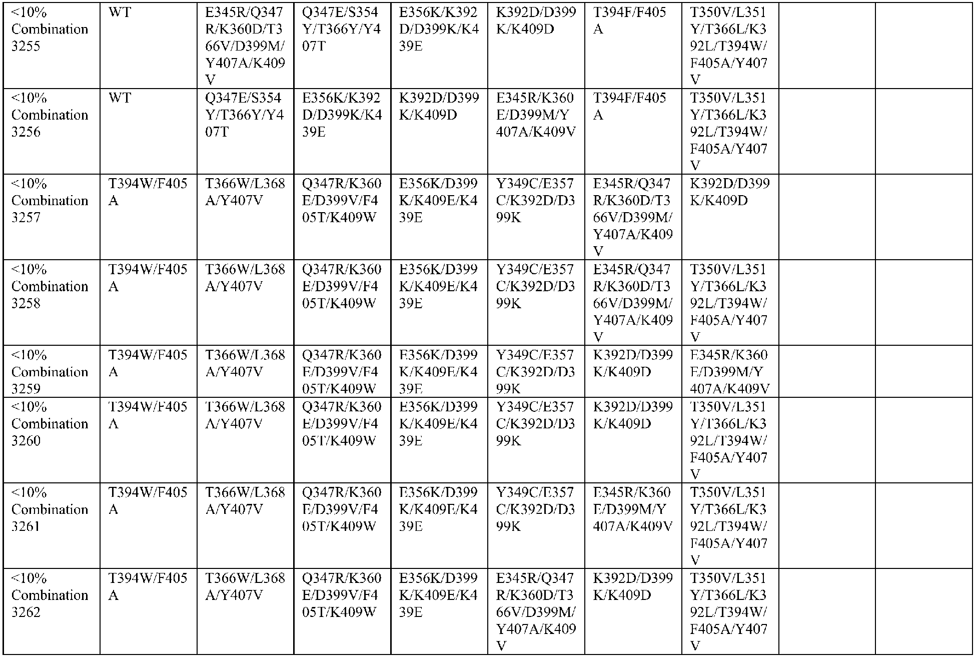

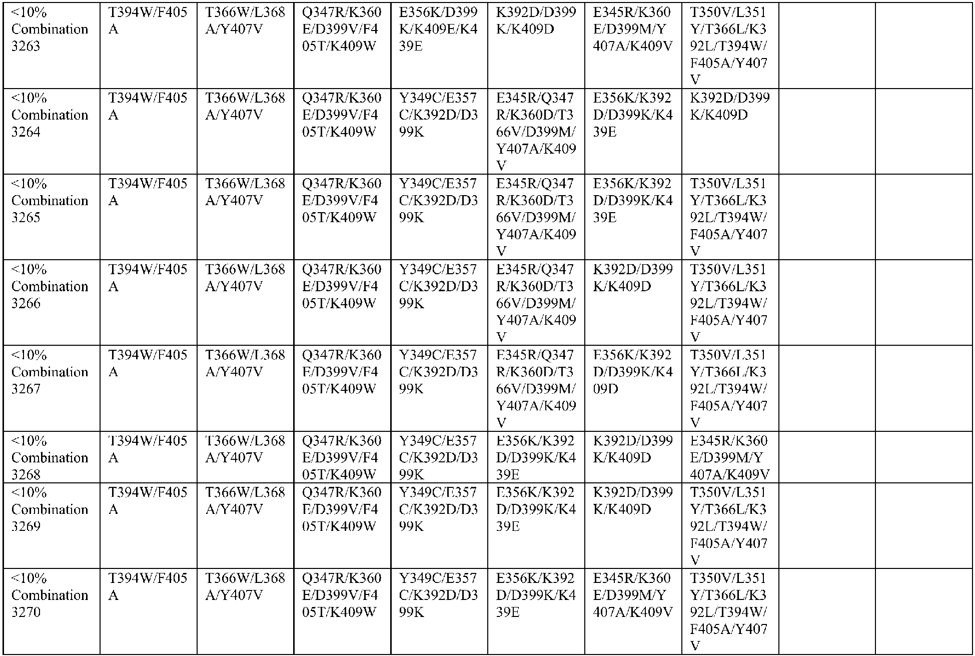

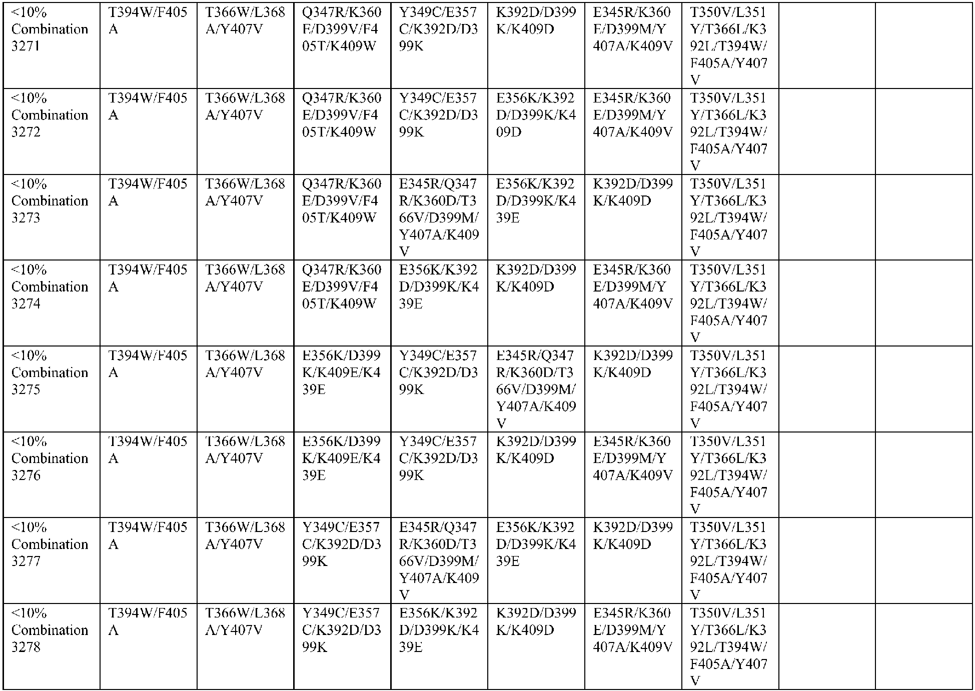

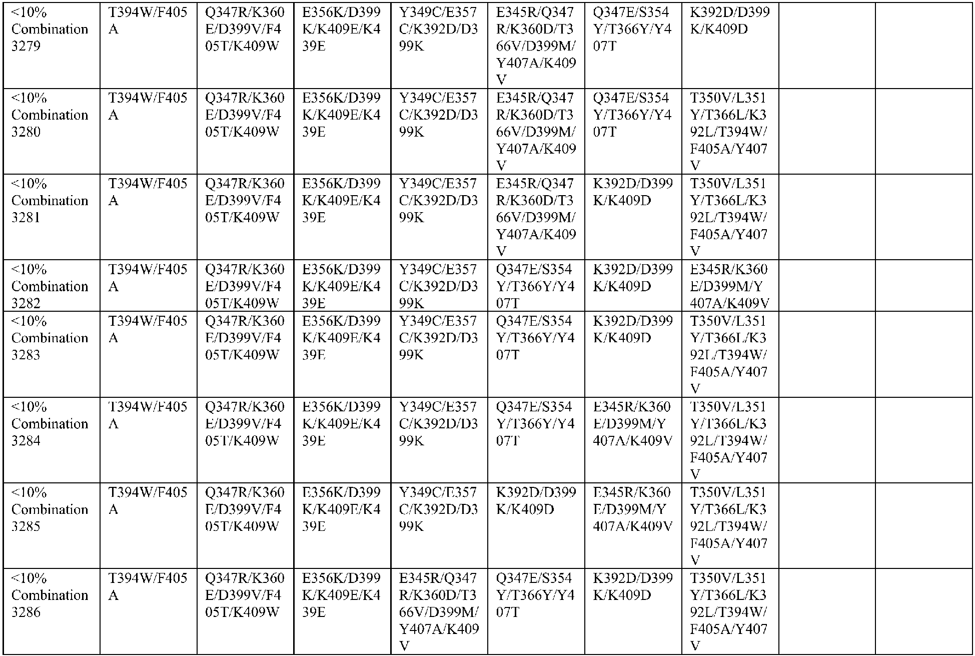

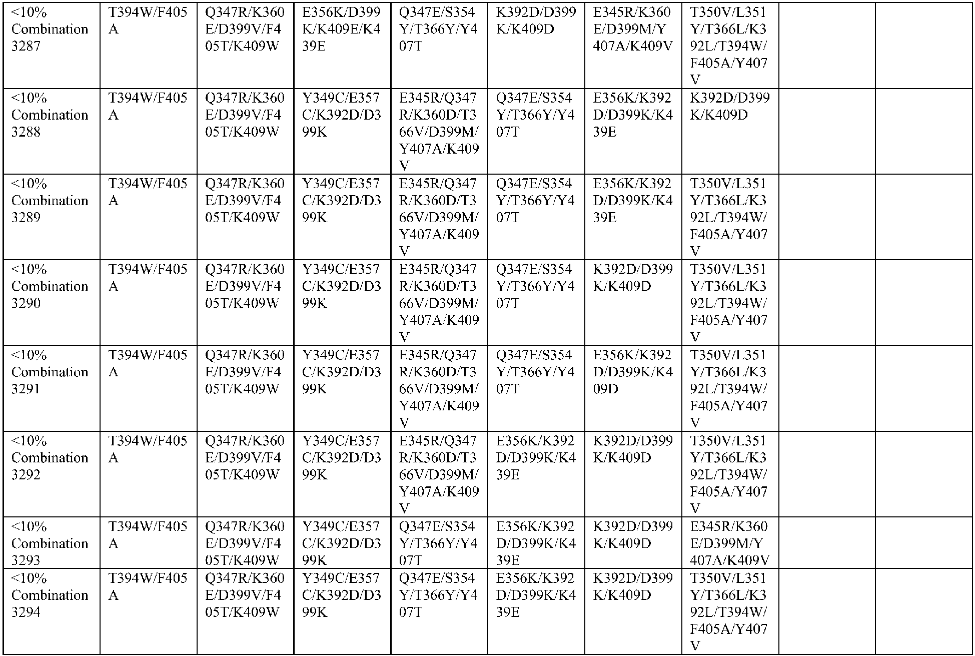

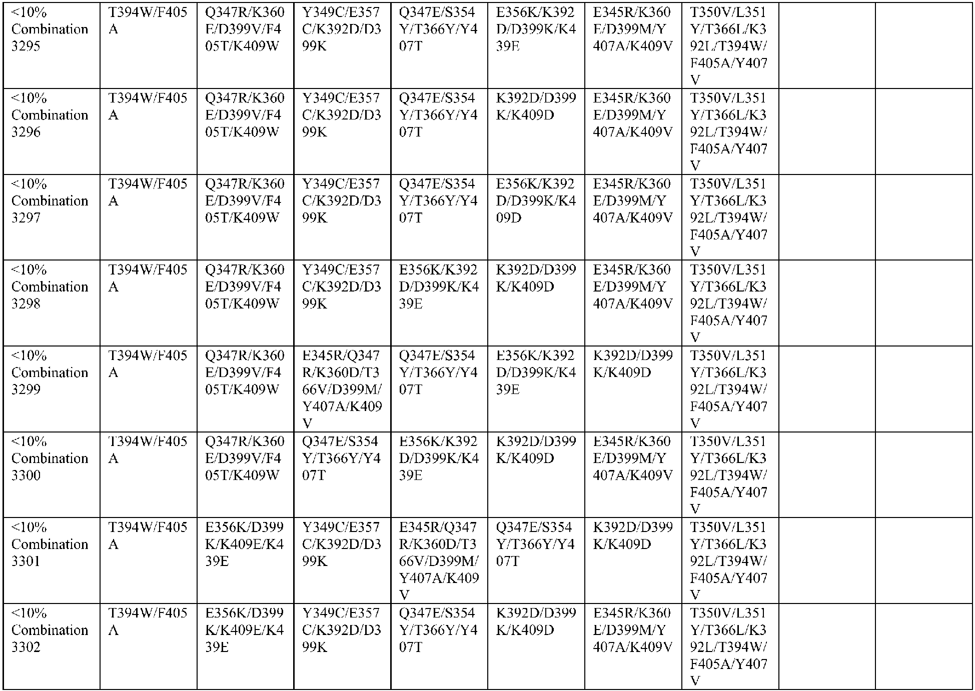

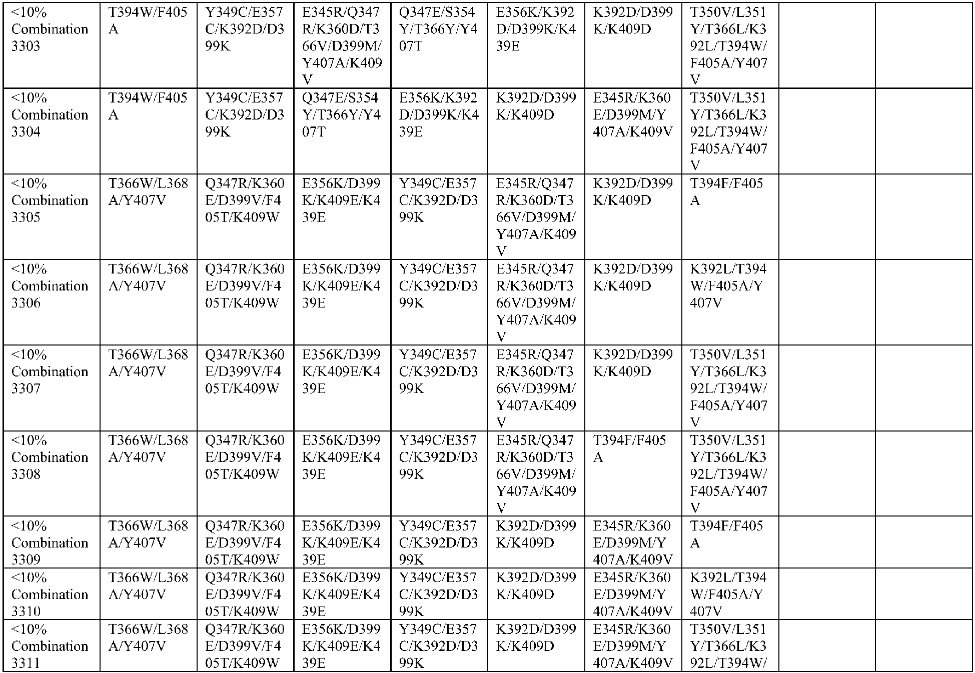

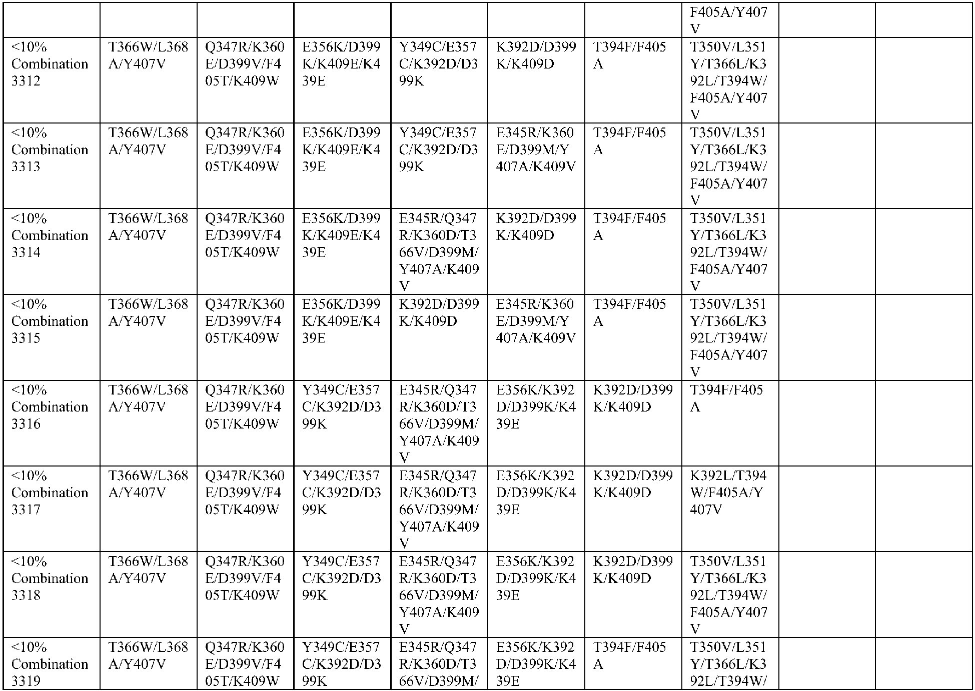

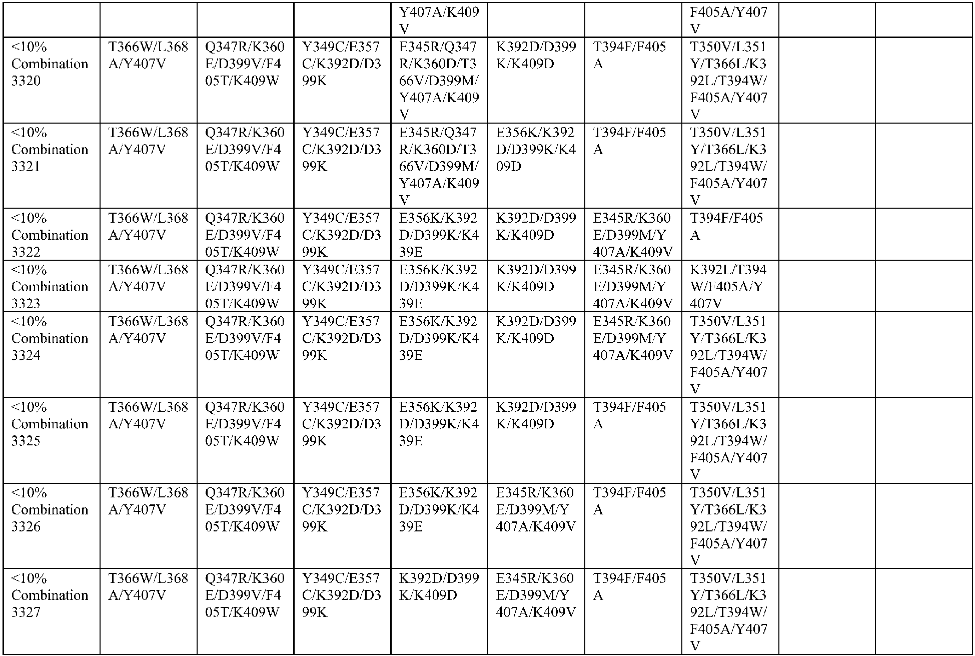

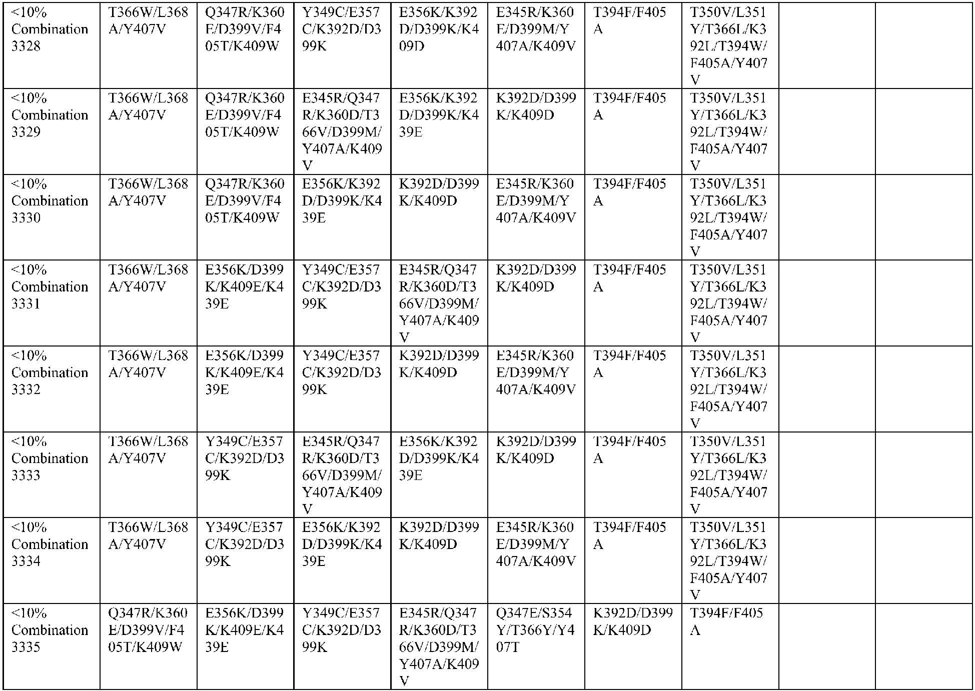

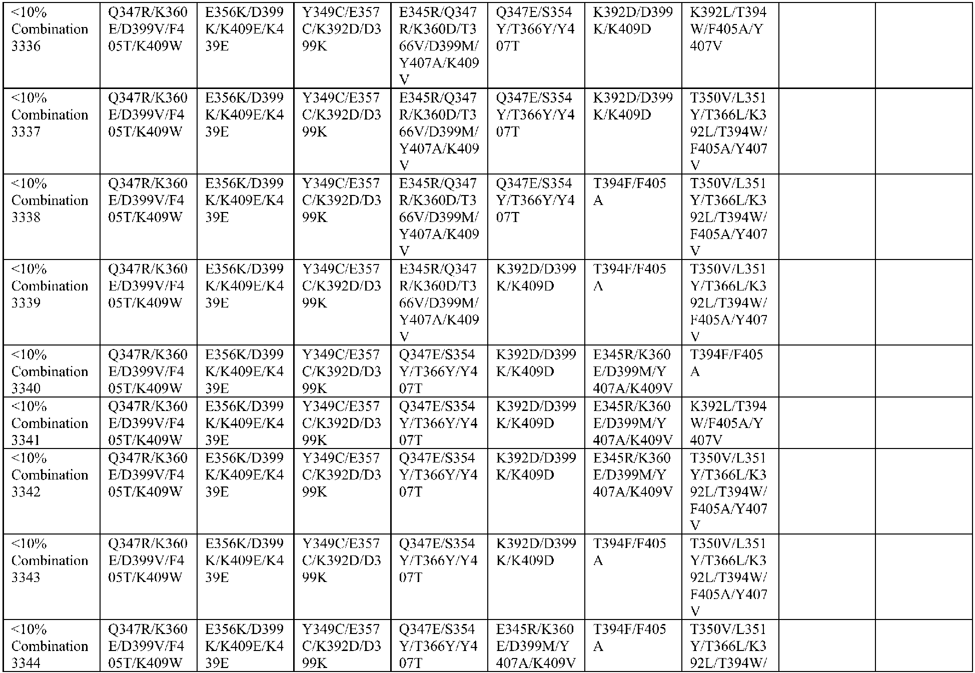

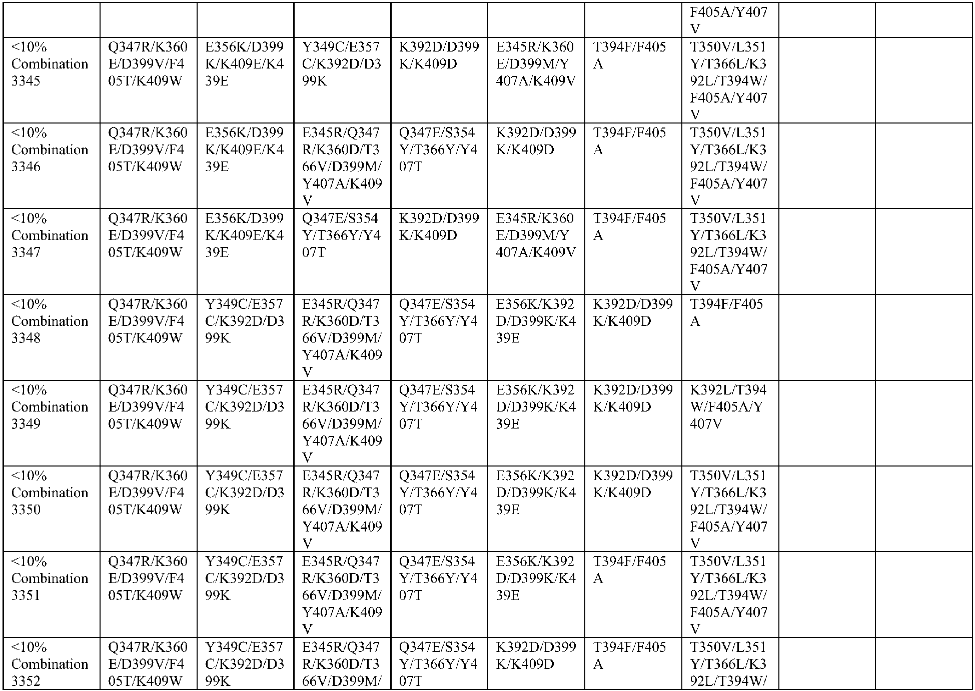

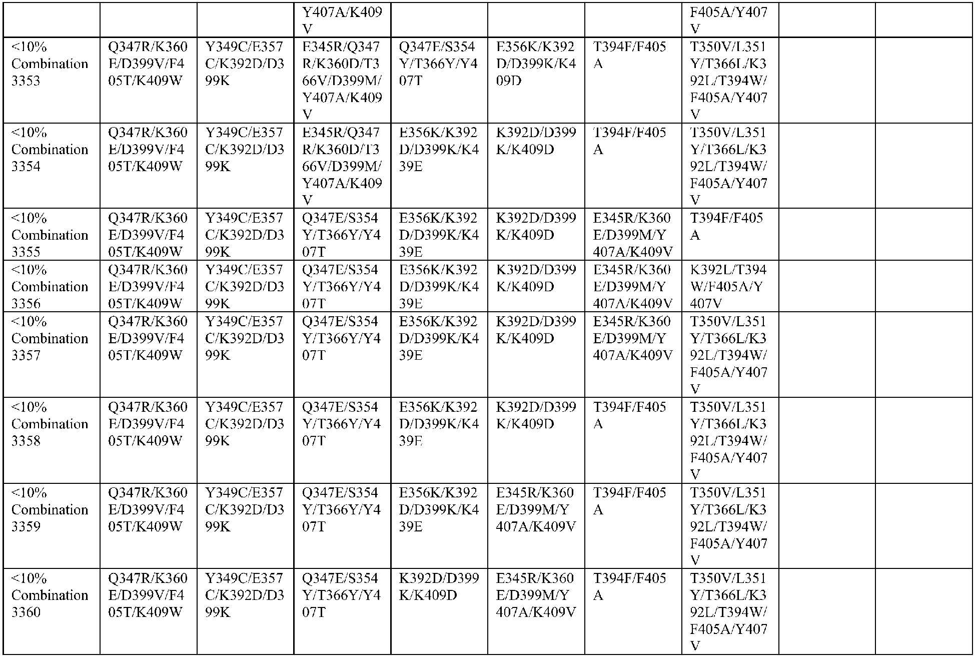

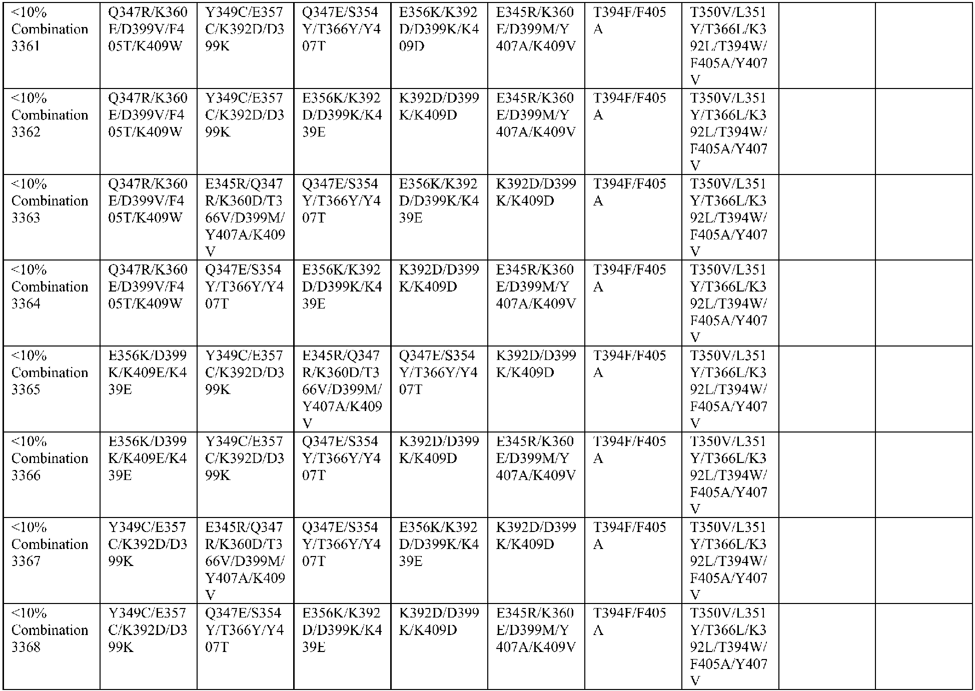

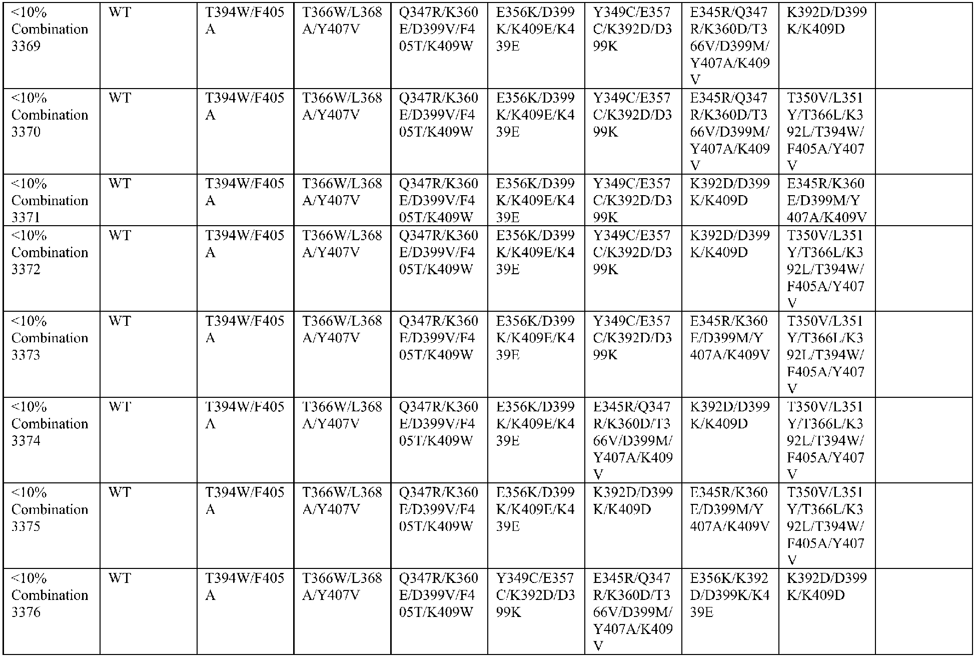

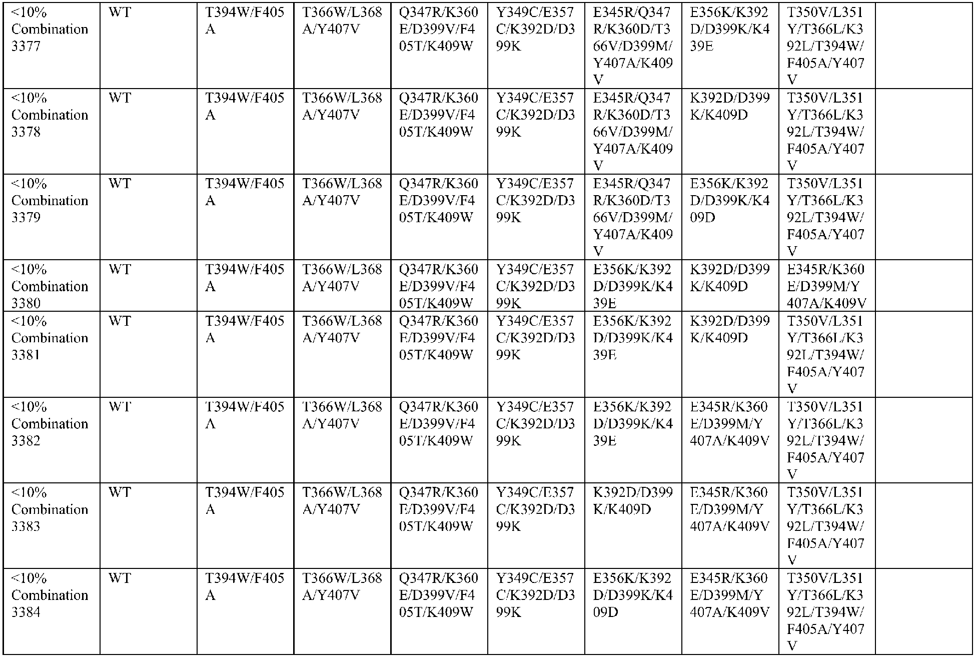

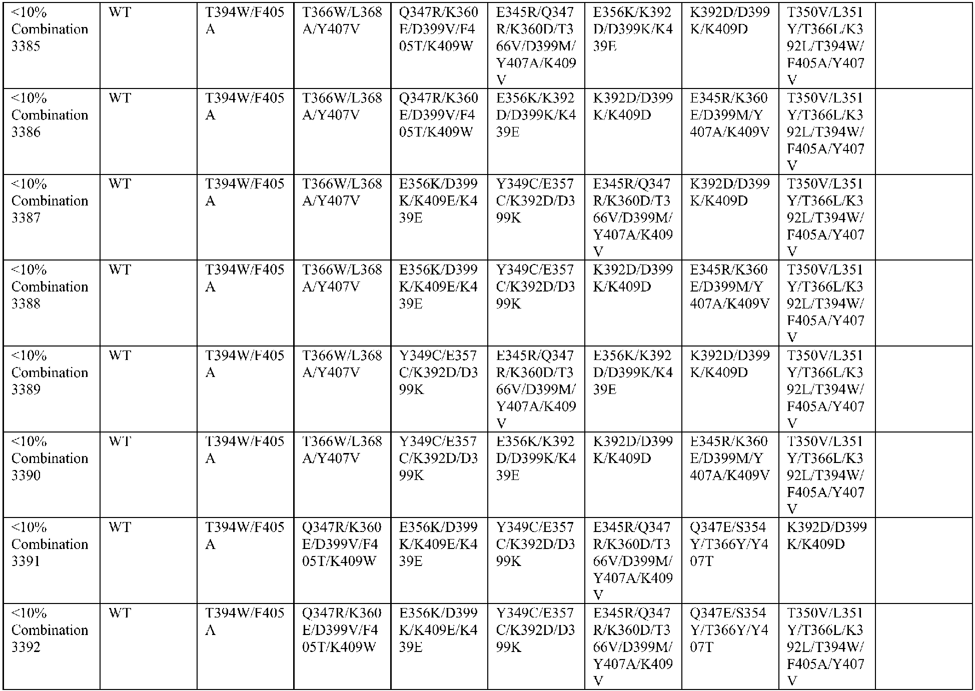

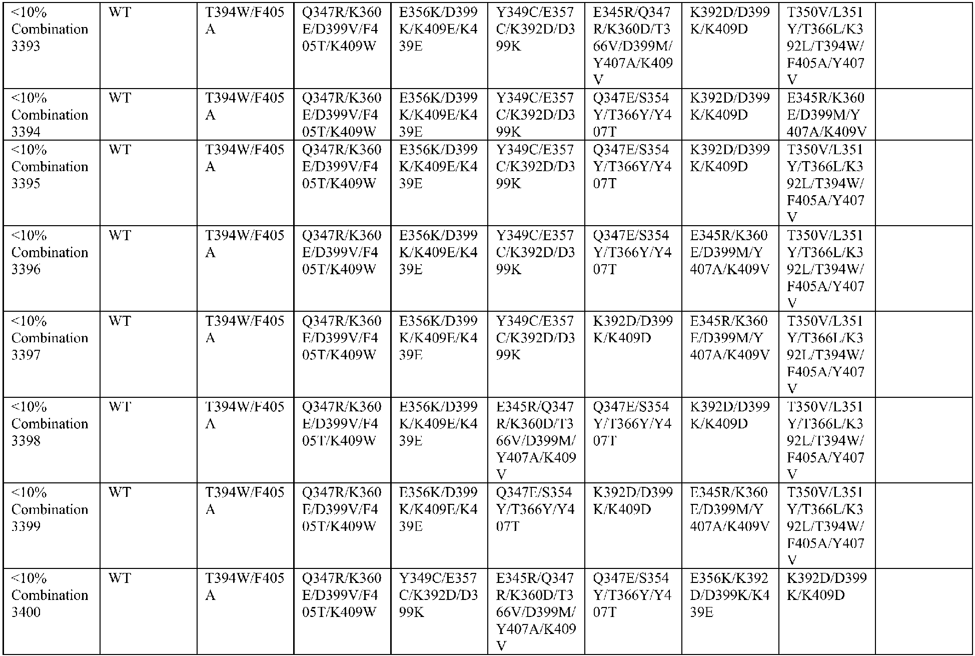

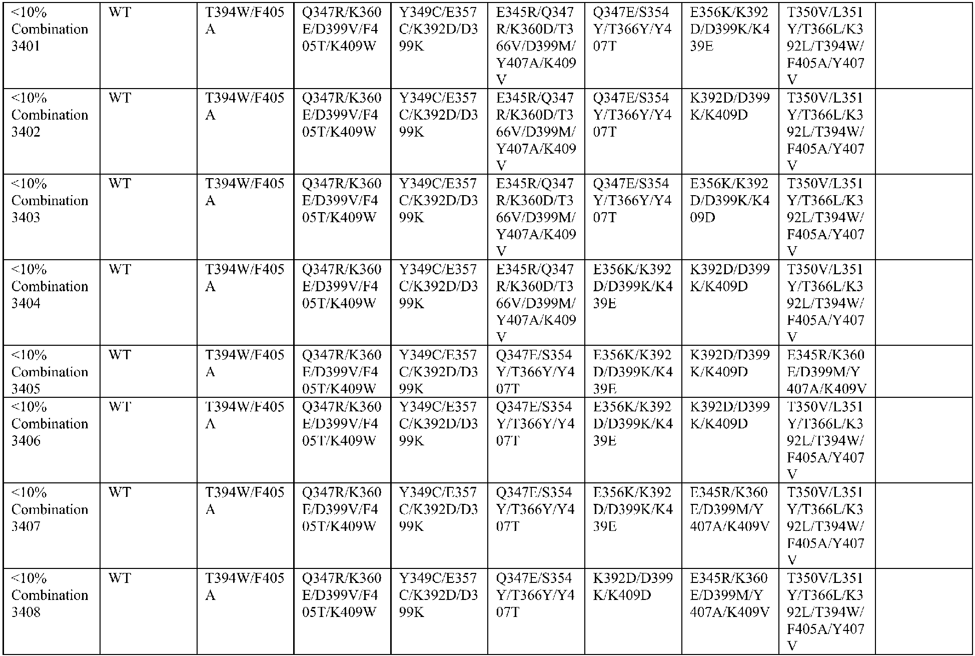

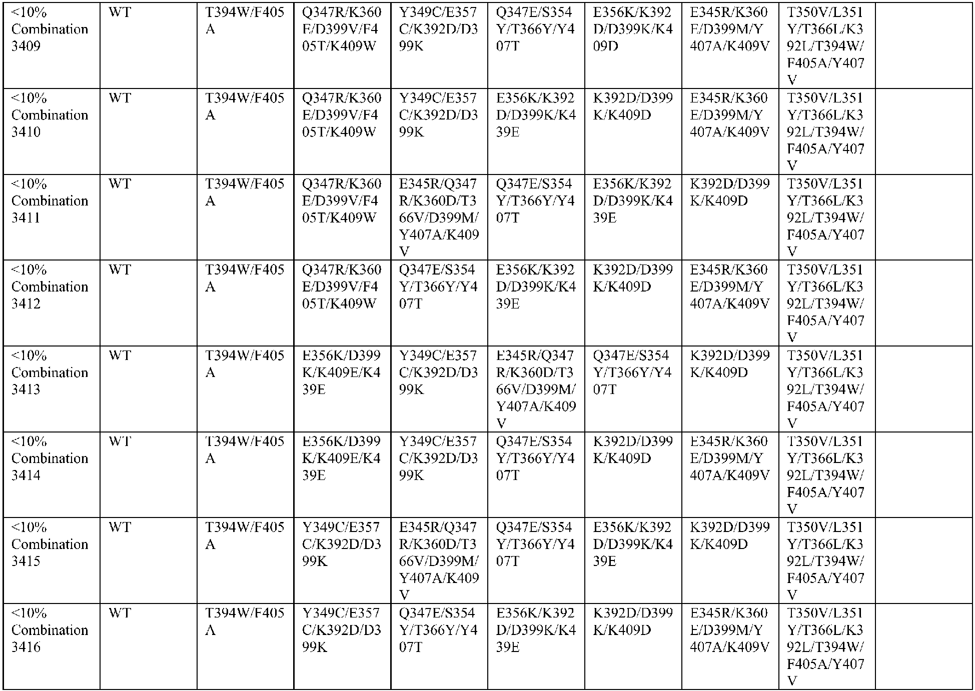

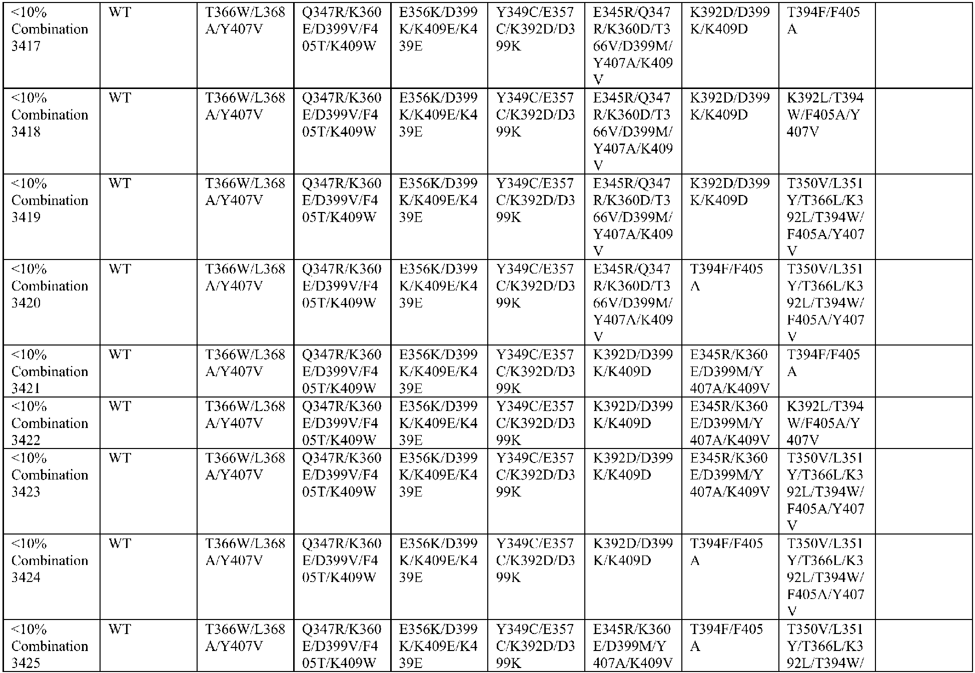

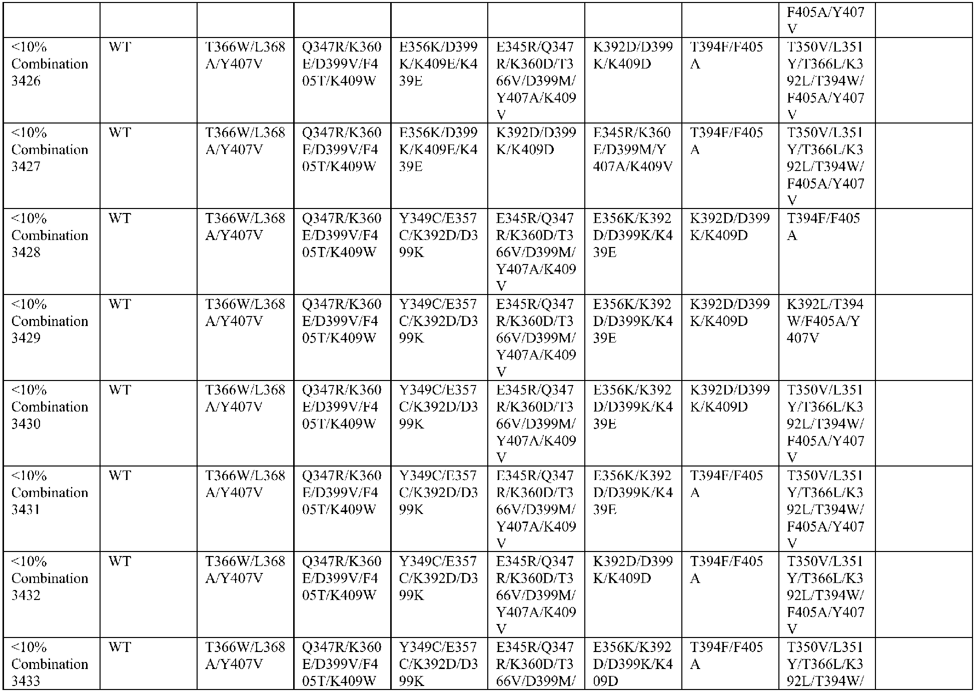

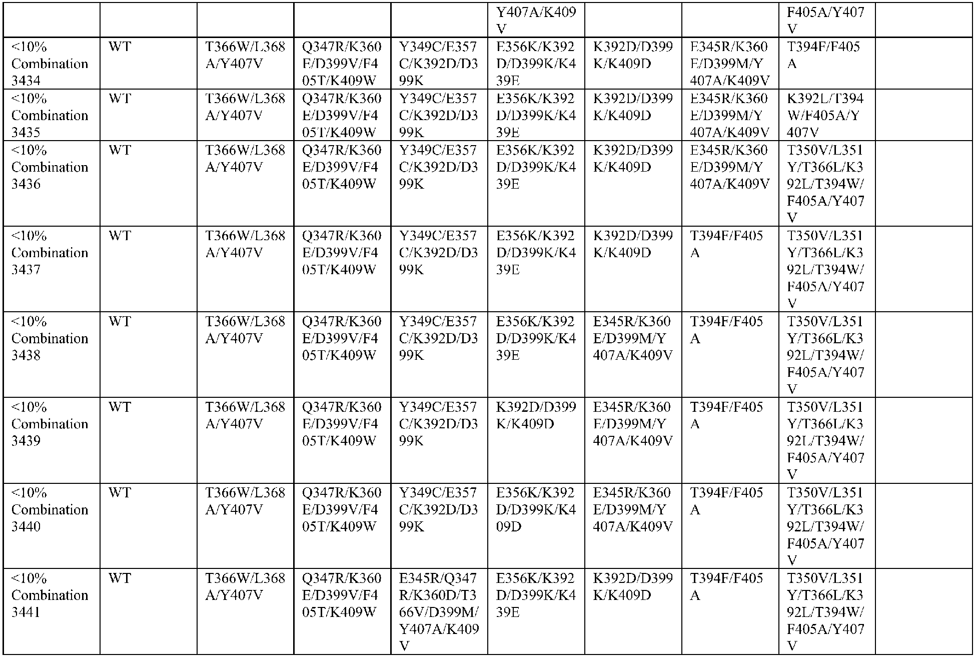

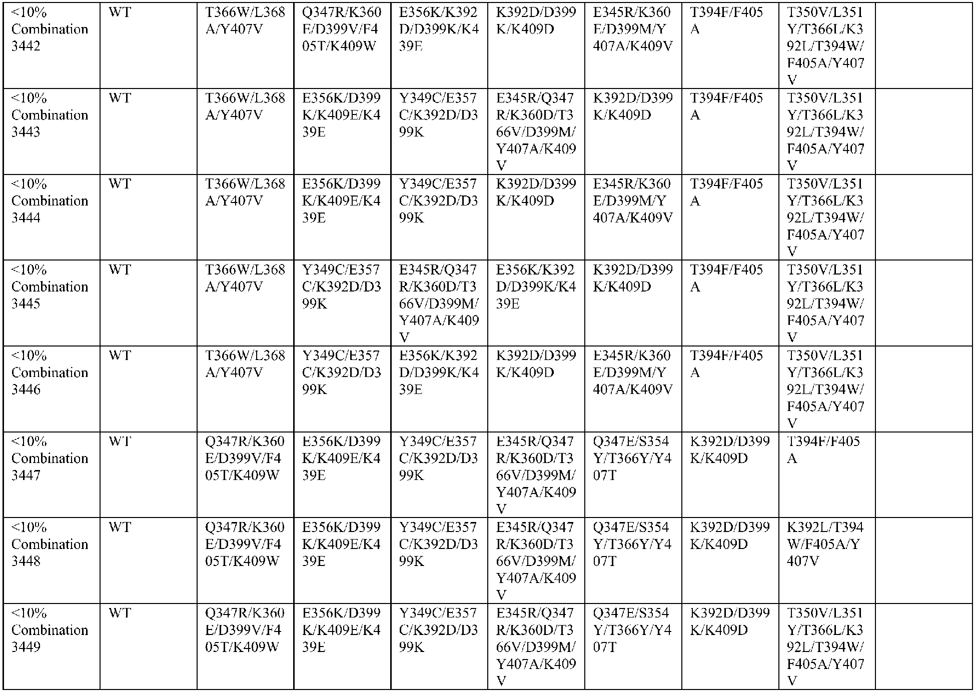

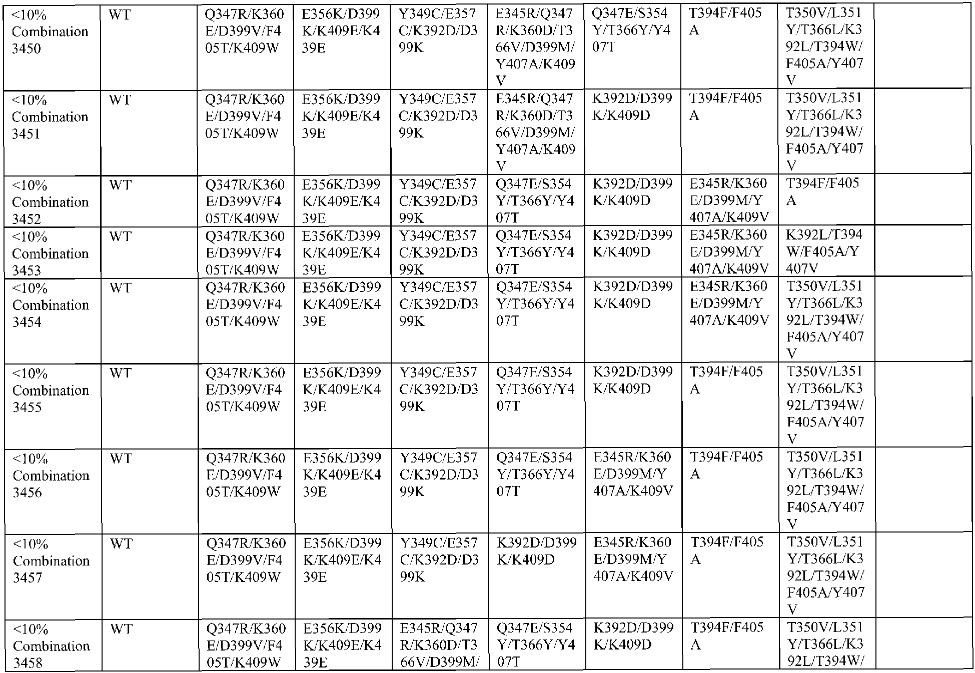

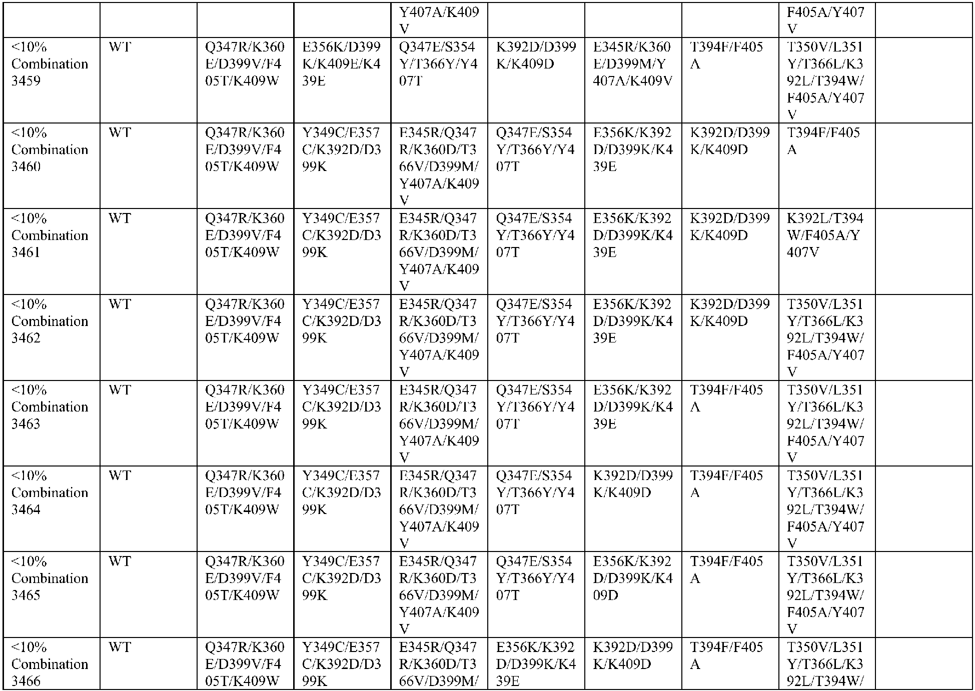

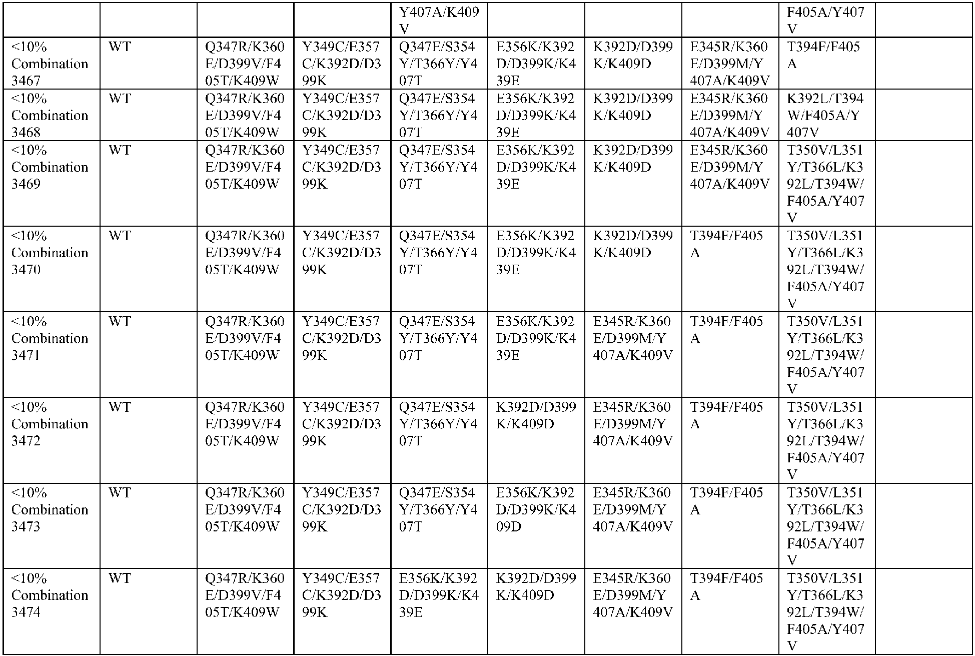

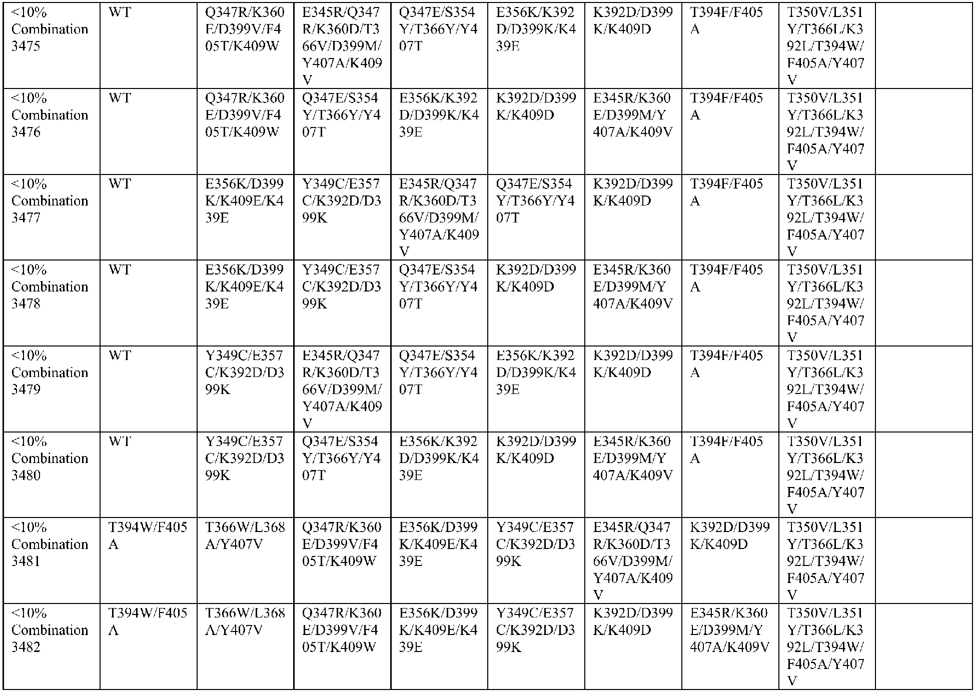

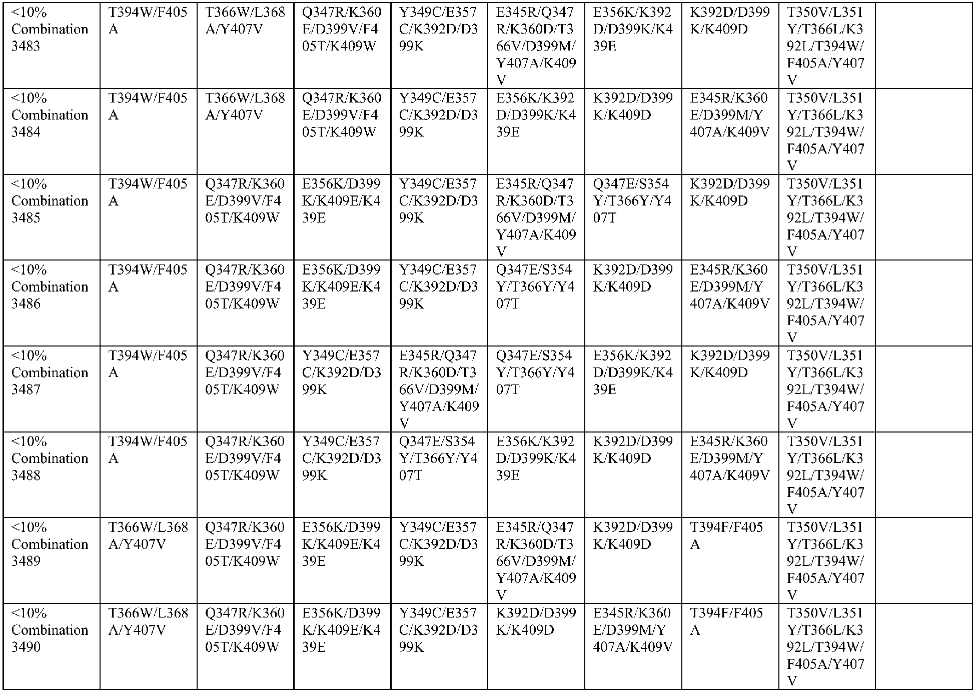

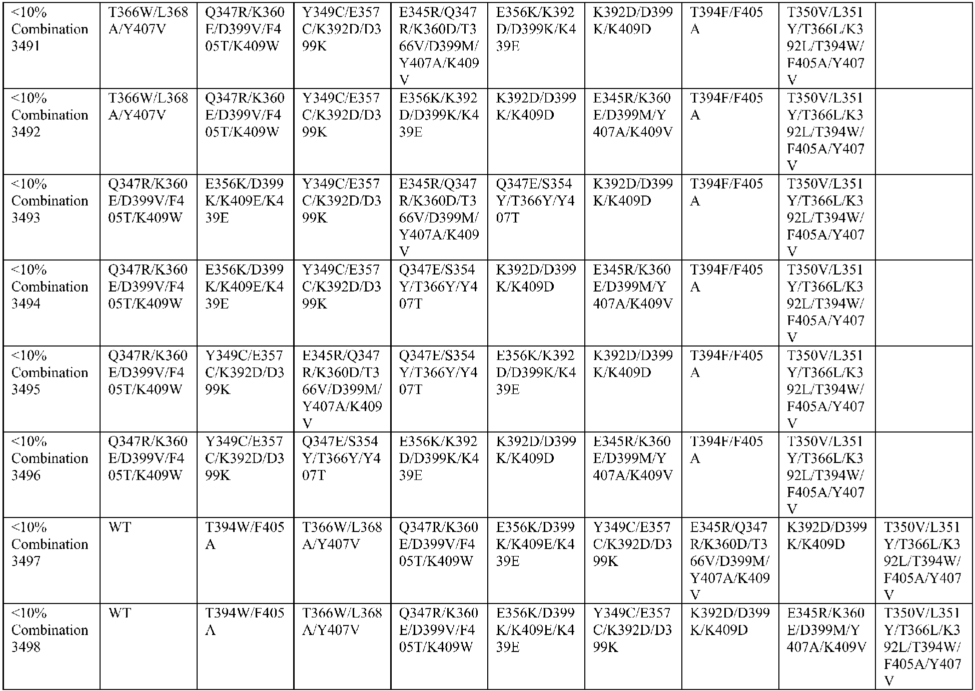

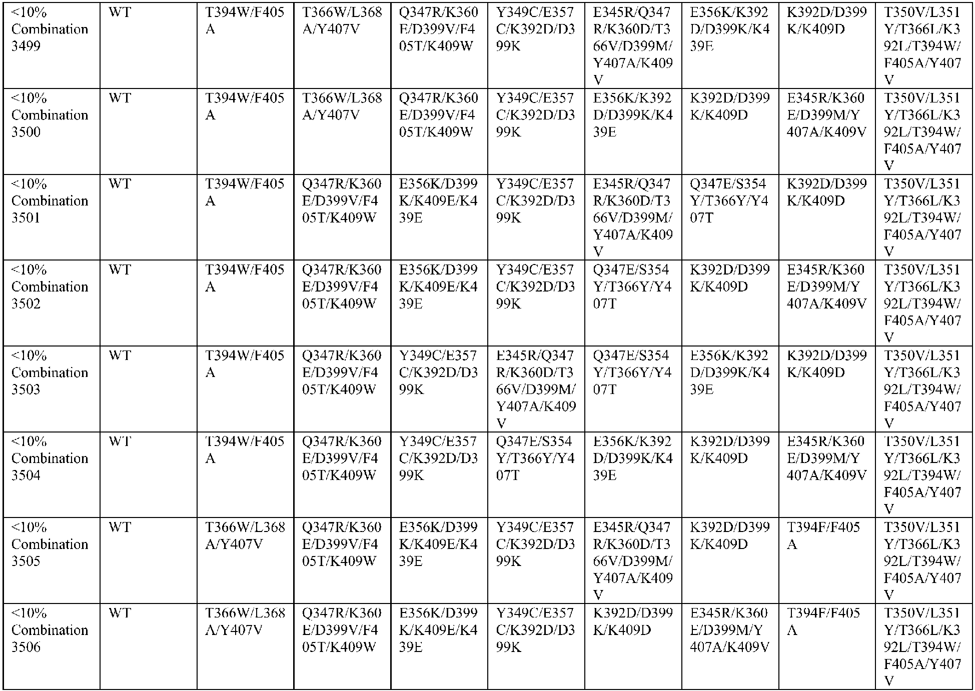

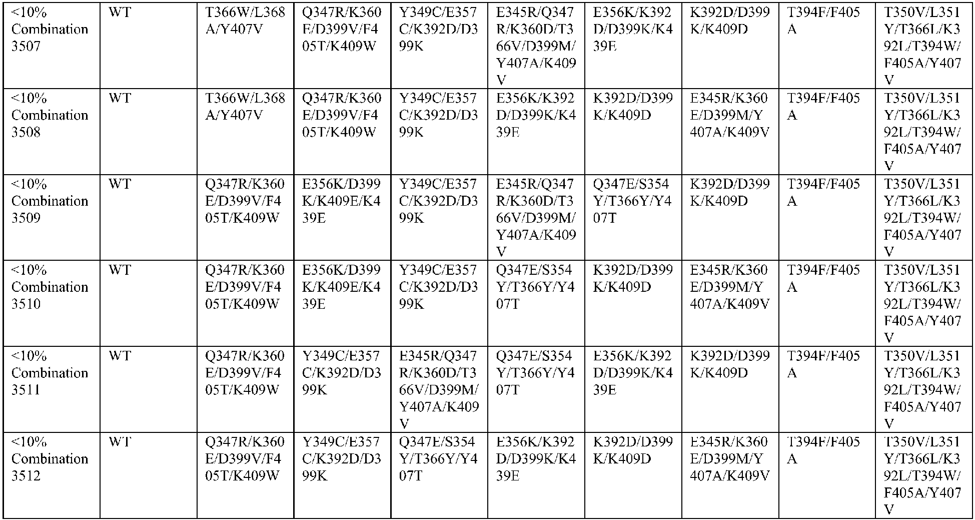

**(Table 7) Amino acid modification pairs that can be used for coexpression of two to six antibodies (composed of amino acid modification pairs that showed a heteromultimer level of 0% in Table 5)**

| Amino acid modification pairs | Antibody 1 | Antibody 2 | Antibody 3 | Antibody 4 | Antibody 5 | Antibody 6 |
|---|---|---|---|---|---|---|
| 0% Combination 1 | WT | T394W/F405A | | | | |
| 0% Combination 2 | WT | T366W/L368A/Y40 7V | | | | |
| 0% Combination 3 | WT | Q347R/K360E/D39 9V/F405T/K409W | | | | |
| 0% Combination 4 | WT | E356K/D399K/K40 9E/K439E | | | | |
| 0% Combination 5 | WT | Y349C/E357C/K39 2D/D399K | | | | |
| 0% Combination 6 | WT | E345R/Q347R/K36 0D/T366V/D399M/ Y407A/K409V | | | | |
| 0% Combination 7 | WT | Q347E/S354Y/T366 Y/Y407T | | | | |
| 0% Combination 8 | WT | E356K/K392D/D39 9K/K439E | | | | |
| 0% Combination 9 | WT | K392D/D399K/K40 9D | | | | |
| 0% Combination 10 | WT | E356K/K392D/D39 9K/K409D | | | | |
| 0% Combination 11 | WT | E345R/K360E/D39 9M/Y407A/K409V | | | | |
| 0% Combination 12 | WT | T394F/F405A | | | | |
| 0% Combination 13 | WT | K392L/T394W/F40 5A/Y407V | | | | |
| 0% Combination 14 | WT | T350V/L351Y/T36 6L/K392L/T394W/ F405A/Y407V | | | | |
| 0% Combination 15 | T394W/F405A | T366W/L368A/Y40 7V | | | | |
| 0% Combination 16 | T394W/F405A | Q347R/K360E/D39 9V/F405T/K409W | | | | |
| 0% Combination 17 | T394W/F405A | E356K/D399K/K40 9E/K439E | | | | |
| 0% Combination 18 | T394W/F405A | Y349C/E357C/K39 2D/D399K | | | | |
| 0% Combination 19 | T394W/F405A | E345R/Q347R/K36 0D/T366V/D399M/ Y407A/K409V | | | | |
| 0% Combination 20 | T394W/F405A | Q347E/S354Y/T366 Y/Y407T | | | | |
| 0% Combination 21 | T394W/F405A | E356K/K392D/D39 9K/K439E | | | | |
| 0% Combination 22 | T394W/F405A | K392D/D399K/K40 9D | | | | |
| 0% Combination 23 | T394W/F405A | E356K/K392D/D39 9K/K409D | | | | |
| 0% Combination 24 | T394W/F405A | E345R/K360E/D39 9M/Y407A/K409V | | | | |
| 0% Combination 25 | T366W/L368A/Y40 7V | Q347R/K360E/D39 9V/F405T/K409W | | | | |
| 0% Combination 26 | T366W/L368A/Y40 7V | E356K/D399K/K40 9E/K439E | | | | |
| 0% Combination 27 | T366W/L368A/Y40 7V | Y349C/E357C/K39 2D/D399K | | | | |
| 0% Combination 28 | T366W/L368A/Y40 7V | E345R/Q347R/K36 0D/T366V/D399M/ Y407A/K409V | | | | |
| 0% Combination 29 | T366W/L368A/Y40 7V | E356K/K392D/D39 9K/K439E | | | | |
| 0% Combination 30 | T366W/L368A/Y40 7V | K392D/D399K/K40 9D | | | | |
| 0% Combination 31 | T366W/L368A/Y40 7V | E356K/K392D/D39 9K/K409D | | | | |
| 0% Combination 32 | T366W/L368A/Y40 7V | E345R/K360E/D39 9M/Y407A/K409V | | | | |
| 0% Combination 33 | T366W/L368A/Y40 7V | T394F/F405A | | | | |
| 0% Combination 34 | T366W/L368A/Y40 7V | K392L/T394W/F40 5A/Y407V | | | | |
| 0% Combination 35 | T366W/L368A/Y40 7V | T350V/L351Y/T36 6L/K392L/T394W/ F405A/Y407V | | | | |
| 0% Combination 36 | Q347R/K360E/D39 9V/F405T/K409W | E356K/D399K/K40 9E/K439E | | | | |
| 0% Combination 37 | Q347R/K360E/D39 9V/F405T/K409W | Y349C/E357C/K39 2D/D399K | | | | |
| 0% Combination 38 | Q347R/K360E/D39 9V/F405T/K409W | Q347E/S354Y/T366 Y/Y407T | | | | |
| 0% Combination 39 | Q347R/K360E/D39 9V/F405T/K409W | E356K/K392D/D39 9K/K439E | | | | |
| 0% Combination 40 | Q347R/K360E/D39 9V/F405T/K409W | K392D/D399K/K40 9D | | | | |
| 0% Combination 41 | Q347R/K360E/D39 9V/F405T/K409W | E356K/K392D/D39 9K/K409D | | | | |
| 0% Combination 42 | Q347R/K360E/D39 9V/F405T/K409W | T394F/F405A | | | | |
| 0% Combination 43 | Q347R/K360E/D39 9V/F405T/K409W | K392L/T394W/F40 5A/Y407V | | | | |
| 0% Combination 44 | Q347R/K360E/D39 9V/F405T/K409W | T350V/L351Y/T36 6L/K392L/T394W/ F405A/Y407V | | | | |
| 0% Combination 45 | E356K/D399K/K40 9E/K439E | Y349C/E357C/K39 2D/D399K | | | | |
| 0% Combination 46 | E356K/D399K/K40 9E/K439E | E345R/Q347R/K36 0D/T366V/D399M/ Y407A/K409V | | | | |
| 0% Combination 47 | E356K/D399K/K40 9E/K439E | Q347E/S354Y/T366 Y/Y407T | | | | |
| 0% Combination 48 | E356K/D399K/K40 9E/K439E | E345R/K360E/D39 9M/Y407A/K409V | | | | |
| 0% Combination 49 | E356K/D399K/K40 9E/K439E | T394F/F405A | | | | |
| 0% Combination 50 | E356K/D399K/K40 9E/K439E | K392L/T394W/F40 5A/Y407V | | | | |
| 0% Combination 51 | E356K/D399K/K40 9E/K439E | T350V/L351Y/T36 6L/K392L/T394W/ F405A/Y407V | | | | |
| 0% Combination 52 | Y349C/E357C/K39 2D/D399K | E356K/K392D/D39 9K/K439E | | | | |
| 0% Combination 53 | Y349C/E357C/K39 2D/D399K | E356K/K392D/D39 9K/K409D | | | | |
| 0% Combination 54 | Y349C/E357C/K39 2D/D399K | E345R/K360E/D39 9M/Y407A/K409V | | | | |
| 0% Combination 55 | Y349C/E357C/K39 2D/D399K | T394F/F405A | | | | |
| 0% Combination 56 | Y349C/E357C/K39 2D/D399K | K392L/T394W/F40 5A/Y407V | | | | |
| 0% Combination 57 | Y349C/E357C/K39 2D/D399K | T350V/L351Y/T36 6L/K392L/T394W/ F405A/Y407V | | | | |
| 0% Combination 58 | E345R/Q347R/K36 0D/T366V/D399M/ Y407A/K409V | E356K/K392D/D39 9K/K439E | | | | |
| 0% Combination 59 | E345R/Q347R/K36 0D/T366V/D399M/ Y407A/K409V | K392D/D399K/K40 9D | | | | |
| 0% Combination 60 | E345R/Q347R/K36 0D/T366V/D399M/ Y407A/K409V | E356K/K392D/D39 9K/K409D | | | | |
| 0% Combination 61 | E345R/Q347R/K36 0D/T366V/D399M/ Y407A/K409V | T394F/F405A | | | | |
| 0% Combination 62 | E345R/Q347R/K36 0D/T366V/D399M/ Y407A/K409V | K392L/T394W/F40 5A/Y407V | | | | |
| 0% Combination 63 | E345R/Q347R/K36 0D/T366V/D399M/ Y407A/K409V | T350V/L351Y/T36 6L/K392L/T394W/ F405A/Y407V | | | | |
| 0% Combination 64 | Q347E/S354Y/T366 Y/Y407T | E356K/K392D/D39 9K/K439E | | | | |
| 0% Combination 65 | Q347E/S354Y/T366 Y/Y407T | K392D/D399K/K40 9D | | | | |
| 0% Combination 66 | Q347E/S354Y/T366 Y/Y407T | E356K/K392D/D39 9K/K409D | | | | |
| 0% Combination 67 | Q347E/S354Y/T366 Y/Y407T | E345R/K360E/D39 9M/Y407A/K409V | | | | |
| 0% Combination 68 | Q347E/S354Y/T366 Y/Y407T | T394F/F405A | | | | |
| 0% Combination 69 | Q347E/S354Y/T366 Y/Y407T | K392L/T394W/F40 5A/Y407V | | | | |
| 0% Combination 70 | Q347E/S354Y/T366 Y/Y407T | T350V/L351Y/T36 6L/K392L/T394W/ F405A/Y407V | | | | |
| 0% Combination 71 | E356K/K392D/D39 9K/K439E | E345R/K360E/D39 9M/Y407A/K409V | | | | |
| 0% Combination 72 | E356K/K392D/D39 9K/K439E | T394F/F405A | | | | |
| 0% Combination 73 | E356K/K392D/D39 9K/K439E | K392L/T394W/F40 5A/Y407V | | | | |
| 0% Combination 74 | E356K/K392D/D39 9K/K439E | T350V/L351Y/T36 6L/K392L/T394W/ F405A/Y407V | | | | |
| 0% Combination 75 | K392D/D399K/K40 9D | T394F/F405A | | | | |
| 0% Combination 76 | K392D/D399K/K40 9D | K392L/T394W/F40 5A/Y407V | | | | |
| 0% Combination 77 | K392D/D399K/K40 9D | T350V/L351Y/T36 6L/K392L/T394W/ F405A/Y407V | | | | |
| 0% Combination 78 | E356K/K392D/D39 9K/K409D | E345R/K360E/D39 9M/Y407A/K409V | | | | |
| 0% Combination 79 | E356K/K392D/D39 9K/K409D | T394F/F405A | | | | |
| 0% Combination 80 | E356K/K392D/D39 9K/K409D | K392L/T394W/F40 5A/Y407V | | | | |
| 0% Combination 81 | E356K/K392D/D39 9K/K409D | T350V/L351Y/T36 6L/K392L/T394W/ F405A/Y407V | | | | |
| 0% Combination 82 | E345R/K360E/D39 9M/Y407A/K409V | T394F/F405A | | | | |
| 0% Combination 83 | E345R/K360E/D39 9M/Y407A/K409V | K392L/T394W/F40 5A/Y407V | | | | |
| 0% Combination 84 | E345R/K360E/D39 9M/Y407A/K409V | T350V/L351Y/T36 6L/K392L/T394W/ F405A/Y407V | | | | |
| 0% Combination 85 | WT | T394W/F405A | T366W/L368A/Y40 7V | | | |
| 0% Combination 86 | WT | T394W/F405A | Q347R/K360E/D39 9V/F405T/K409W | | | |
| 0% Combination 87 | WT | T394W/F405A | E356K/D399K/K40 9E/K439E | | | |
| 0% Combination 88 | WT | T394W/F405A | Y349C/E357C/K39 2D/D399K | | | |
| 0% Combination 89 | WT | T394W/F405A | E345R/Q347R/K36 0D/T366V/D399M/ Y407A/K409V | | | |
| 0% Combination 90 | WT | T394W/F405A | Q347E/S354Y/T366 Y/Y407T | | | |
| 0% Combination 91 | WT | T394W/F405A | E356K/K392D/D39 9K/K439E | | | |
| 0% Combination 92 | WT | T394W/F405A | K392D/D399K/K40 9D | | | |
| 0% Combination 93 | WT | T394W/F405A | E356K/K392D/D39 9K/K409D | | | |
| 0% Combination 94 | WT | T394W/F405A | E345R/K360E/D39 9M/Y407A/K409V | | | |
| 0% Combination 95 | WT | T366W/L368A/Y40 7V | Q347R/K360E/D39 9V/F405T/K409W | | | |
| 0% Combination 96 | WT | T366W/L368A/Y40 7V | E356K/D399K/K40 9E/K439E | | | |
| 0% Combination 97 | WT | T366W/L368A/Y40 7V | Y349C/E357C/K39 2D/D399K | | | |
| 0% Combination 98 | WT | T366W/L368A/Y40 7V | E345R/Q347R/K36 0D/T366V/D399M/ Y407A/K409V | | | |
| 0% Combination 99 | WT | T366W/L368A/Y40 7V | E356K/K392D/D39 9K/K439E | | | |
| 0% Combination 100 | WT | T366W/L368A/Y40 7V | K392D/D399K/K40 9D | | | |
| 0% Combination 101 | WT | T366W/L368A/Y40 7V | E356K/K392D/D39 9K/K409D | | | |
| 0% Combination 102 | WT | T366W/L368A/Y40 7V | E345R/K360E/D39 9M/Y407A/K409V | | | |
| 0% Combination 103 | WT | T366W/L368A/Y40 7V | T394F/F405A | | | |
| 0% Combination 104 | WT | T366W/L368A/Y40 7V | K392L/T394W/F40 5A/Y407V | | | |
| 0% Combination 105 | WT | T366W/L368A/Y40 7V | T350V/L351Y/T36 6L/K392L/T394W/ F405A/Y407V | | | |
| 0% Combination 106 | WT | Q347R/K360E/D39 9V/F405T/K409W | E356K/D399K/K40 9E/K439E | | | |
| 0% Combination 107 | WT | Q347R/K360E/D39 9V/F405T/K409W | Y349C/E357C/K39 2D/D399K | | | |
| 0% Combination 108 | WT | Q347R/K360E/D39 9V/F405T/K409W | Q347E/S354Y/T366 Y/Y407T | | | |
| 0% Combination 109 | WT | Q347R/K360E/D39 9V/F405T/K409W | E356K/K392D/D39 9K/K439E | | | |
| 0% Combination 110 | WT | Q347R/K360E/D39 9V/F405T/K409W | K392D/D399K/K40 9D | | | |
| 0% Combination 111 | WT | Q347R/K360E/D39 9V/F405T/K409W | E356K/K392D/D39 9K/K409D | | | |
| 0% Combination 112 | WT | Q347R/K360E/D39 9V/F405T/K409W | T394F/F405A | | | |
| 0% Combination 113 | WT | Q347R/K360E/D39 9V/F405T/K409W | K392L/T394W/F40 5A/Y407V | | | |
| 0% Combination 114 | WT | Q347R/K360E/D39 9V/F405T/K409W | T350V/L351Y/T36 6L/K392L/T394W/ F405A/Y407V | | | |
| 0% Combination 115 | WT | E356K/D399K/K40 9E/K439E | Y349C/E357C/K39 2D/D399K | | | |
| 0% Combination 116 | WT | E356K/D399K/K40 9E/K439E | E345R/Q347R/K36 0D/T366V/D399M/ Y407A/K409V | | | |
| 0% Combination 117 | WT | E356K/D399K/K40 9E/K439E | Q347E/S354Y/T366 Y/Y407T | | | |
| 0% Combination 118 | WT | E356K/D399K/K40 9E/K439E | E345R/K360E/D39 9M/Y407A/K409V | | | |
| 0% Combination 119 | WT | E356K/D399K/K40 9E/K439E | T394F/F405A | | | |
| 0% Combination 120 | WT | E356K/D399K/K40 9E/K439E | K392L/T394W/F40 5A/Y407V | | | |
| 0% Combination 121 | WT | E356K/D399K/K40 9E/K439E | T350V/L351Y/T36 6L/K392L/T394W/ F405A/Y407V | | | |
| 0% Combination 122 | WT | Y349C/E357C/K39 2D/D399K | E356K/K392D/D39 9K/K439E | | | |
| 0% Combination 123 | WT | Y349C/E357C/K39 2D/D399K | E356K/K392D/D39 9K/K409D | | | |
| 0% Combination 124 | WT | Y349C/E357C/K39 2D/D399K | E345R/K360E/D39 9M/Y407A/K409V | | | |
| 0% Combination 125 | WT | Y349C/E357C/K39 2D/D399K | T394F/F405A | | | |
| 0% Combination 126 | WT | Y349C/E357C/K39 2D/D399K | K392L/T394W/F40 5A/Y407V | | | |
| 0% Combination 127 | WT | Y349C/E357C/K39 2D/D399K | T350V/L351Y/T36 6L/K392L/T394W/ F405A/Y407V | | | |
| 0% Combination 128 | WT | E345R/Q347R/K36 0D/T366V/D399M/ Y407A/K409V | E356K/K392D/D39 9K/K439E | | | |
| 0% Combination 129 | WT | E345R/Q347R/K36 0D/T366V/D399M/ Y407A/K409V | K392D/D399K/K40 9D | | | |
| 0% Combination 130 | WT | E345R/Q347R/K36 0D/T366V/D399M/ Y407A/K409V | E356K/K392D/D39 9K/K409D | | | |
| 0% Combination 131 | WT | E345R/Q347R/K36 0D/T366V/D399M/ Y407A/K409V | T394F/F405A | | | |
| 0% Combination 132 | WT | E345R/Q347R/K36 0D/T366V/D399M/ Y407A/K409V | K392L/T394W/F40 5A/Y407V | | | |
| 0% Combination 133 | WT | E345R/Q347R/K36 0D/T366V/D399M/ Y407A/K409V | T350V/L351Y/T36 6L/K392L/T394W/ F405A/Y407V | | | |
| 0% Combination 134 | WT | Q347E/S354Y/T366 Y/Y407T | E356K/K392D/D39 9K/K439E | | | |
| 0% Combination 135 | WT | Q347E/S354Y/T366 Y/Y407T | K392D/D399K/K40 9D | | | |
| 0% Combination 136 | WT | Q347E/S354Y/T366 Y/Y407T | E356K/K392D/D39 9K/K409D | | | |
| 0% Combination 137 | WT | Q347E/S354Y/T366 Y/Y407T | E345R/K360E/D39 9M/Y407A/K409V | | | |
| 0% Combination 138 | WT | Q347E/S354Y/T366 Y/Y407T | T394F/F405A | | | |
| 0% Combination 139 | WT | Q347E/S354Y/T366 Y/Y407T | K392L/T394W/F40 5A/Y407V | | | |
| 0% Combination 140 | WT | Q347E/S354Y/T366 Y/Y407T | T350V/L351Y/T36 6L/K392L/T394W/ F405A/Y407V | | | |
| 0% Combination 141 | WT | E356K/K392D/D39 9K/K439E | E345R/K360E/D39 9M/Y407A/K409V | | | |
| 0% Combination 142 | WT | E356K/K392D/D39 9K/K439E | T394F/F405A | | | |
| 0% Combination 143 | WT | E356K/K392D/D39 9K/K439E | K392L/T394W/F40 5A/Y407V | | | |
| 0% Combination 144 | WT | E356K/K392D/D39 9K/K439E | T350V/L351Y/T36 6L/K392L/T394W/ F405A/Y407V | | | |
| 0% Combination 145 | WT | K392D/D399K/K40 9D | T394F/F405A | | | |
| 0% Combination 146 | WT | K392D/D399K/K40 9D | K392L/T394W/F40 5A/Y407V | | | |
| 0% Combination 147 | WT | K392D/D399K/K40 9D | T350V/L351Y/T36 6L/K392L/T394W/ F405A/Y407V | | | |
| 0% Combination 148 | WT | E356K/K392D/D39 9K/K409D | E345R/K360E/D39 9M/Y407A/K409V | | | |
| 0% Combination 149 | WT | E356K/K392D/D39 9K/K409D | T394F/F405A | | | |
| 0% Combination 150 | WT | E356K/K392D/D39 9K/K409D | K392L/T394W/F40 5A/Y407V | | | |
| 0% Combination 151 | WT | E356K/K392D/D39 9K/K409D | T350V/L351Y/T36 6L/K392L/T394W/ F405A/Y407V | | | |
| 0% Combination 152 | WT | E345R/K360E/D39 9M/Y407A/K409V | T394F/F405A | | | |
| 0% Combination 153 | WT | E345R/K360E/D39 9M/Y407A/K409V | K392L/T394W/F40 5A/Y407V | | | |
| 0% Combination 154 | WT | E345R/K360E/D39 9M/Y407A/K409V | T350V/L351Y/T36 6L/K392L/T394W/ F405A/Y407V | | | |
| 0% Combination 155 | T394W/F405A | T366W/L368A/Y40 7V | Q347R/K360E/D39 9V/F405T/K409W | | | |
| 0% Combination 156 | T394W/F405A | T366W/L368A/Y40 7V | E356K/D399K/K40 9E/K439E | | | |
| 0% Combination 157 | T394W/F405A | T366W/L368A/Y40 7V | Y349C/E357C/K39 2D/D399K | | | |
| 0% Combination 158 | T394W/F405A | T366W/L368A/Y40 7V | E345R/Q347R/K36 0D/T366V/D399M/ Y407A/K409V | | | |
| 0% Combination 159 | T394W/F405A | T366W/L368A/Y40 7V | E356K/K392D/D39 9K/K439E | | | |
| 0% Combination 160 | T394W/F405A | T366W/L368A/Y40 7V | K392D/D399K/K40 9D | | | |
| 0% Combination 161 | T394W/F405A | T366W/L368A/Y40 7V | E356K/K392D/D39 9K/K409D | | | |
| 0% Combination 162 | T394W/F405A | T366W/L368A/Y40 7V | E345R/K360E/D39 9M/Y407A/K409V | | | |
| 0% Combination 163 | T394W/F405A | Q347R/K360E/D39 9V/F405T/K409W | E356K/D399K/K40 9E/K439E | | | |
| 0% Combination 164 | T394W/F405A | Q347R/K360E/D39 9V/F405T/K409W | Y349C/E357C/K39 2D/D399K | | | |
| 0% Combination 165 | T394W/F405A | Q347R/K360E/D39 9V/F405T/K409W | Q347E/S354Y/T366 Y/Y407T | | | |
| 0% Combination 166 | T394W/F405A | Q347R/K360E/D39 9V/F405T/K409W | E356K/K392D/D39 9K/K439E | | | |
| 0% Combination 167 | T394W/F405A | Q347R/K360E/D39 9V/F405T/K409W | K392D/D399K/K40 9D | | | |
| 0% Combination 168 | T394W/F405A | Q347R/K360E/D39 9V/F405T/K409W | E356K/K392D/D39 9K/K409D | | | |
| 0% Combination 169 | T394W/F405A | E356K/D399K/K40 9E/K439E | Y349C/E357C/K39 2D/D399K | | | |
| 0% Combination 170 | T394W/F405A | E356K/D399K/K40 9E/K439E | E345R/Q347R/K36 0D/T366V/D399M/ Y407A/K409V | | | |
| 0% Combination 171 | T394W/F405A | E356K/D399K/K40 9E/K439E | Q347E/S354Y/T366 Y/Y407T | | | |
| 0% Combination 172 | T394W/F405A | E356K/D399K/K40 9E/K439E | E345R/K360E/D39 9M/Y407A/K409V | | | |
| 0% Combination 173 | T394W/F405A | Y349C/E357C/K39 2D/D399K | E356K/K392D/D39 9K/K439E | | | |
| 0% Combination 174 | T394W/F405A | Y349C/E357C/K39 2D/D399K | E356K/K392D/D39 9K/K409D | | | |
| 0% Combination 175 | T394W/F405A | Y349C/E357C/K39 2D/D399K | E345R/K360E/D39 9M/Y407A/K409V | | | |
| 0% Combination 176 | T394W/F405A | E345R/Q347R/K36 0D/T366V/D399M/ Y407A/K409V | E356K/K392D/D39 9K/K439E | | | |
| 0% Combination 177 | T394W/F405A | E345R/Q347R/K36 0D/T366V/D399M/ Y407A/K409V | K392D/D399K/K40 9D | | | |
| 0% Combination 178 | T394W/F405A | E345R/Q347R/K36 0D/T366V/D399M/ Y407A/K409V | E356K/K392D/D39 9K/K409D | | | |
| 0% Combination 179 | T394W/F405A | Q347E/S354Y/T366 Y/Y407T | E356K/K392D/D39 9K/K439E | | | |
| 0% Combination 180 | T394W/F405A | Q347E/S354Y/T366 Y/Y407T | K392D/D399K/K40 9D | | | |
| 0% Combination 181 | T394W/F405A | Q347E/S354Y/T366 Y/Y407T | E356K/K392D/D39 9K/K409D | | | |
| 0% Combination 182 | T394W/F405A | Q347E/S354Y/T366 Y/Y407T | E345R/K360E/D39 9M/Y407A/K409V | | | |
| 0% Combination 183 | T394W/F405A | E356K/K392D/D39 9K/K439E | E345R/K360E/D39 9M/Y407A/K409V | | | |
| 0% Combination 184 | T394W/F405A | E356K/K392D/D39 9K/K409D | E345R/K360E/D39 9M/Y407A/K409V | | | |
| 0% Combination 185 | T366W/L368A/Y40 7V | Q347R/K360E/D39 9V/F405T/K409W | E356K/D399K/K40 9E/K439E | | | |
| 0% Combination 186 | T366W/L368A/Y40 7V | Q347R/K360E/D39 9V/F405T/K409W | Y349C/E357C/K39 2D/D399K | | | |
| 0% Combination 187 | T366W/L368A/Y40 7V | Q347R/K360E/D39 9V/F405T/K409W | E356K/K392D/D39 9K/K439E | | | |
| 0% Combination 188 | T366W/L368A/Y40 7V | Q347R/K360E/D39 9V/F405T/K409W | K392D/D399K/K40 9D | | | |
| 0% Combination 189 | T366W/L368A/Y40 7V | Q347R/K360E/D39 9V/F405T/K409W | E356K/K392D/D39 9K/K409D | | | |
| 0% Combination 190 | T366W/L368A/Y40 7V | Q347R/K360E/D39 9V/F405T/K409W | T394F/F405A | | | |
| 0% Combination 191 | T366W/L368A/Y40 7V | Q347R/K360E/D39 9V/F405T/K409W | K392L/T394W/F40 5A/Y407V | | | |
| 0% Combination 192 | T366W/L368A/Y40 7V | Q347R/K360E/D39 9V/F405T/K409W | T350V/L351Y/T36 6L/K392L/T394W/ F405A/Y407V | | | |
| 0% Combination 193 | T366W/L368A/Y40 7V | E356K/D399K/K40 9E/K439E | Y349C/E357C/K39 2D/D399K | | | |
| 0% Combination 194 | T366W/L368A/Y40 7V | E356K/D399K/K40 9E/K439E | E345R/Q347R/K36 0D/T366V/D399M/ Y407A/K409V | | | |
| 0% Combination 195 | T366W/L368A/Y40 7V | E356K/D399K/K40 9E/K439E | E345R/K360E/D39 9M/Y407A/K409V | | | |
| 0% Combination 196 | T366W/L368A/Y40 7V | E356K/D399K/K40 9E/K439E | T394F/F405A | | | |
| 0% Combination 197 | T366W/L368A/Y40 7V | E356K/D399K/K40 9E/K439E | K392L/T394W/F40 5A/Y407V | | | |
| 0% Combination 198 | T366W/L368A/Y40 7V | E356K/D399K/K40 9E/K439E | T350V/L351Y/T36 6L/K392L/T394W/ F405A/Y407V | | | |
| 0% Combination 199 | T366W/L368A/Y40 7V | Y349C/E357C/K39 2D/D399K | E356K/K392D/D39 9K/K439E | | | |
| 0% Combination 200 | T366W/L368A/Y40 7V | Y349C/E357C/K39 2D/D399K | E356K/K392D/D39 9K/K409D | | | |
| 0% Combination 201 | T366W/L368A/Y40 7V | Y349C/E357C/K39 2D/D399K | E345R/K360E/D39 9M/Y407A/K409V | | | |
| 0% Combination 202 | T366W/L368A/Y40 7V | Y349C/E357C/K39 2D/D399K | T394F/F405A | | | |
| 0% Combination 203 | T366W/L368A/Y40 7V | Y349C/E357C/K39 2D/D399K | K392L/T394W/F40 5A/Y407V | | | |
| 0% Combination 204 | T366W/L368A/Y40 7V | Y349C/E357C/K39 2D/D399K | T350V/L351Y/T36 6L/K392L/T394W/ F405A/Y407V | | | |
| 0% Combination 205 | T366W/L368A/Y40 7V | E345R/Q347R/K36 0D/T366V/D399M/ Y407A/K409V | E356K/K392D/D39 9K/K439E | | | |
| 0% Combination 206 | T366W/L368A/Y40 7V | E345R/Q347R/K36 0D/T366V/D399M/ Y407A/K409V | K392D/D399K/K40 9D | | | |
| 0% Combination 207 | T366W/L368A/Y40 7V | E345R/Q347R/K36 0D/T366V/D399M/ Y407A/K409V | E356K/K392D/D39 9K/K409D | | | |
| 0% Combination 208 | T366W/L368A/Y40 7V | E345R/Q347R/K36 0D/T366V/D399M/ Y407A/K409V | T394F/F405A | | | |
| 0% Combination 209 | T366W/L368A/Y40 7V | E345R/Q347R/K36 0D/T366V/D399M/ Y407A/K409V | K392L/T394W/F40 5A/Y407V | | | |
| 0% Combination 210 | T366W/L368A/Y40 7V | E345R/Q347R/K36 0D/T366V/D399M/ Y407A/K409V | T350V/L351Y/T36 6L/K392L/T394W/ F405A/Y407V | | | |
| 0% Combination 211 | T366W/L368A/Y40 7V | E356K/K392D/D39 9K/K439E | E345R/K360E/D39 9M/Y407A/K409V | | | |
| 0% Combination 212 | T366W/L368A/Y40 7V | E356K/K392D/D39 9K/K439E | T394F/F405A | | | |
| 0% Combination 213 | T366W/L368A/Y40 7V | E356K/K392D/D39 9K/K439E | K392L/T394W/F40 5A/Y407V | | | |
| 0% Combination 214 | T366W/L368A/Y40 7V | E356K/K392D/D39 9K/K439E | T350V/L351Y/T36 6L/K392L/T394W/ F405A/Y407V | | | |
| 0% Combination 215 | T366W/L368A/Y40 7V | K392D/D399K/K40 9D | T394F/F405A | | | |
| 0% Combination 216 | T366W/L368A/Y40 7V | K392D/D399K/K40 9D | K392L/T394W/F40 5A/Y407V | | | |
| 0% Combination 217 | T366W/L368A/Y40 7V | K392D/D399K/K40 9D | T350V/L351Y/T36 6L/K392L/T394W/ F405A/Y407V | | | |
| 0% Combination 218 | T366W/L368A/Y40 7V | E356K/K392D/D39 9K/K409D | E345R/K360E/D39 9M/Y407A/K409V | | | |
| 0% Combination 219 | T366W/L368A/Y40 7V | E356K/K392D/D39 9K/K409D | T394F/F405A | | | |
| 0% Combination 220 | T366W/L368A/Y40 7V | E356K/K392D/D39 9K/K409D | K392L/T394W/F40 5A/Y407V | | | |
| 0% Combination 221 | T366W/L368A/Y40 7V | E356K/K392D/D39 9K/K409D | T350V/L351Y/T36 6L/K392L/T394W/ F405A/Y407V | | | |
| 0% Combination 222 | T366W/L368A/Y40 7V | E345R/K360E/D39 9M/Y407A/K409V | T394F/F405A | | | |
| 0% Combination 223 | T366W/L368A/Y40 7V | E345R/K360E/D39 9M/Y407A/K409V | K392L/T394W/F40 5A/Y407V | | | |
| 0% Combination 224 | T366W/L368A/Y40 7V | E345R/K360E/D39 9M/Y407A/K409V | T350V/L351Y/T36 6L/K392L/T394W/ F405A/Y407V | | | |
| 0% Combination 225 | Q347R/K360E/D39 9V/F405T/K409W | E356K/D399K/K40 9E/K439E | Y349C/E357C/K39 2D/D399K | | | |
| 0% Combination 226 | Q347R/K360E/D39 9V/F405T/K409W | E356K/D399K/K40 9E/K439E | Q347E/S354Y/T366 Y/Y407T | | | |
| 0% Combination 227 | Q347R/K360E/D39 9V/F405T/K409W | E356K/D399K/K40 9E/K439E | T394F/F405A | | | |
| 0% Combination 228 | Q347R/K360E/D39 9V/F405T/K409W | E356K/D399K/K40 9E/K439E | K392L/T394W/F40 5A/Y407V | | | |
| 0% Combination 229 | Q347R/K360E/D39 9V/F405T/K409W | E356K/D399K/K40 9E/K439E | T350V/L351Y/T36 6L/K392L/T394W/ F405A/Y407V | | | |
| 0% Combination 230 | Q347R/K360E/D39 9V/F405T/K409W | Y349C/E357C/K39 2D/D399K | E356K/K392D/D39 9K/K439E | | | |
| 0% Combination 231 | Q347R/K360E/D39 9V/F405T/K409W | Y349C/E357C/K39 2D/D399K | E356K/K392D/D39 9K/K409D | | | |
| 0% Combination 232 | Q347R/K360E/D39 9V/F405T/K409W | Y349C/E357C/K39 2D/D399K | T394F/F405A | | | |
| 0% Combination 233 | Q347R/K360E/D39 9V/F405T/K409W | Y349C/E357C/K39 2D/D399K | K392L/T394W/F40 5A/Y407V | | | |
| 0% Combination 234 | Q347R/K360E/D39 9V/F405T/K409W | Y349C/E357C/K39 2D/D399K | T350V/L351Y/T36 6L/K392L/T394W/ F405A/Y407V | | | |
| 0% Combination 235 | Q347R/K360E/D39 9V/F405T/K409W | Q347E/S354Y/T366 Y/Y407T | E356K/K392D/D39 9K/K439E | | | |
| 0% Combination 236 | Q347R/K360E/D39 9V/F405T/K409W | Q347E/S354Y/T366 Y/Y407T | K392D/D399K/K40 9D | | | |
| 0% Combination 237 | Q347R/K360E/D39 9V/F405T/K409W | Q347E/S354Y/T366 Y/Y407T | E356K/K392D/D39 9K/K409D | | | |
| 0% Combination 238 | Q347R/K360E/D39 9V/F405T/K409W | Q347E/S354Y/T366 Y/Y407T | T394F/F405A | | | |
| 0% Combination 239 | Q347R/K360E/D39 9V/F405T/K409W | Q347E/S354Y/T366 Y/Y407T | K392L/T394W/F40 5A/Y407V | | | |
| 0% Combination 240 | Q347R/K360E/D39 9V/F405T/K409W | Q347E/S354Y/T366 Y/Y407T | T350V/L351Y/T36 6L/K392L/T394W/ F405A/Y407V | | | |
| 0% Combination 241 | Q347R/K360E/D39 9V/F405T/K409W | E356K/K392D/D39 9K/K439E | T394F/F405A | | | |
| 0% Combination 242 | Q347R/K360E/D39 9V/F405T/K409W | E356K/K392D/D39 9K/K439E | K392L/T394W/F40 5A/Y407V | | | |
| 0% Combination 243 | Q347R/K360E/D39 9V/F405T/K409W | E356K/K392D/D39 9K/K439E | T350V/L351Y/T36 6L/K392L/T394W/ F405A/Y407V | | | |
| 0% Combination 244 | Q347R/K360E/D39 9V/F405T/K409W | K392D/D399K/K40 9D | T394F/F405A | | | |
| 0% Combination 245 | Q347R/K360E/D39 9V/F405T/K409W | K392D/D399K/K40 9D | K392L/T394W/F40 5A/Y407V | | | |
| 0% Combination 246 | Q347R/K360E/D39 9V/F405T/K409W | K392D/D399K/K40 9D | T350V/L351Y/T36 6L/K392L/T394W/ F405A/Y407V | | | |
| 0% Combination 247 | Q347R/K360E/D39 9V/F405T/K409W | E356K/K392D/D39 9K/K409D | T394F/F405A | | | |
| 0% Combination 248 | Q347R/K360E/D39 9V/F405T/K409W | E356K/K392D/D39 9K/K409D | K392L/T394W/F40 5A/Y407V | | | |
| 0% Combination 249 | Q347R/K360E/D39 9V/F405T/K409W | E356K/K392D/D39 9K/K409D | T350V/L351Y/T36 6L/K392L/T394W/ F405A/Y407V | | | |
| 0% Combination 250 | E356K/D399K/K40 9E/K439E | Y349C/E357C/K39 2D/D399K | E345R/K360E/D39 9M/Y407A/K409V | | | |
| 0% Combination 251 | E356K/D399K/K40 9E/K439E | Y349C/E357C/K39 2D/D399K | T394F/F405A | | | |
| 0% Combination 252 | E356K/D399K/K40 9E/K439E | Y349C/E357C/K39 2D/D399K | K392L/T394W/F40 5A/Y407V | | | |
| 0% Combination 253 | E356K/D399K/K40 9E/K439E | Y349C/E357C/K39 2D/D399K | T350V/L351Y/T36 6L/K392L/T394W/ F405A/Y407V | | | |
| 0% Combination 254 | E356K/D399K/K40 9E/K439E | E345R/Q347R/K36 0D/T366V/D399M/ Y407A/K409V | T394F/F405A | | | |
| 0% Combination 255 | E356K/D399K/K40 9E/K439E | E345R/Q347R/K36 0D/T366V/D399M/ Y407A/K409V | K392L/T394W/F40 5A/Y407V | | | |
| 0% Combination 256 | E356K/D399K/K40 9E/K439E | E345R/Q347R/K36 0D/T366V/D399M/ Y407A/K409V | T350V/L351Y/T36 6L/K392L/T394W/ F405A/Y407V | | | |
| 0% Combination 257 | E356K/D399K/K40 9E/K439E | Q347E/S354Y/T366 Y/Y407T | E345R/K360E/D39 9M/Y407A/K409V | | | |
| 0% Combination 258 | E356K/D399K/K40 9E/K439E | Q347E/S354Y/T366 Y/Y407T | T394F/F405A | | | |
| 0% Combination 259 | E356K/D399K/K40 9E/K439E | Q347E/S354Y/T366 Y/Y407T | K392L/T394W/F40 5A/Y407V | | | |
| 0% Combination 260 | E356K/D399K/K40 9E/K439E | Q347E/S354Y/T366 Y/Y407T | T350V/L351Y/T36 6L/K392L/T394W/ F405A/Y407V | | | |
| 0% Combination 261 | E356K/D399K/K40 9E/K439E | E345R/K360E/D39 9M/Y407A/K409V | T394F/F405A | | | |
| 0% Combination 262 | E356K/D399K/K40 9E/K439E | E345R/K360E/D39 9M/Y407A/K409V | K392L/T394W/F40 5A/Y407V | | | |
| 0% Combination 263 | E356K/D399K/K40 9E/K439E | E345R/K360E/D39 9M/Y407A/K409V | T350V/L351Y/T36 6L/K392L/T394W/ F405A/Y407V | | | |
| 0% Combination 264 | Y349C/E357C/K39 2D/D399K | E356K/K392D/D39 9K/K439E | E345R/K360E/D39 9M/Y407A/K409V | | | |
| 0% Combination 265 | Y349C/E357C/K39 2D/D399K | E356K/K392D/D39 9K/K439E | T394F/F405A | | | |
| 0% Combination 266 | Y349C/E357C/K39 2D/D399K | E356K/K392D/D39 9K/K439E | K392L/T394W/F40 5A/Y407V | | | |
| 0% Combination 267 | Y349C/E357C/K39 2D/D399K | E356K/K392D/D39 9K/K439E | T350V/L351Y/T36 6L/K392L/T394W/ F405A/Y407V | | | |
| 0% Combination 268 | Y349C/E357C/K39 2D/D399K | E356K/K392D/D39 9K/K409D | E345R/K360E/D39 9M/Y407A/K409V | | | |
| 0% Combination 269 | Y349C/E357C/K39 2D/D399K | E356K/K392D/D39 9K/K409D | T394F/F405A | | | |
| 0% Combination 270 | Y349C/E357C/K39 2D/D399K | E356K/K392D/D39 9K/K409D | K392L/T394W/F40 5A/Y407V | | | |
| 0% Combination 271 | Y349C/E357C/K39 2D/D399K | E356K/K392D/D39 9K/K409D | T350V/L351Y/T36 6L/K392L/T394W/ F405A/Y407V | | | |
| 0% Combination 272 | Y349C/E357C/K39 2D/D399K | E345R/K360E/D39 9M/Y407A/K409V | T394F/F405A | | | |
| 0% Combination 273 | Y349C/E357C/K39 2D/D399K | E345R/K360E/D39 9M/Y407A/K409V | K392L/T394W/F40 5A/Y407V | | | |
| 0% Combination 274 | Y349C/E357C/K39 2D/D399K | E345R/K360E/D39 9M/Y407A/K409V | T350V/L351Y/T36 6L/K392L/T394W/ F405A/Y407V | | | |
| 0% Combination 275 | E345R/Q347R/K36 0D/T366V/D399M/ Y407A/K409V | E356K/K392D/D39 9K/K439E | T394F/F405A | | | |
| 0% Combination 276 | E345R/Q347R/K36 0D/T366V/D399M/ Y407A/K409V | E356K/K392D/D39 9K/K439E | K392L/T394W/F40 5A/Y407V | | | |
| 0% Combination 277 | E345R/Q347R/K36 0D/T366V/D399M/ Y407A/K409V | E356K/K392D/D39 9K/K439E | T350V/L351Y/T36 6L/K392L/T394W/ F405A/Y407V | | | |
| 0% Combination 278 | E345R/Q347R/K36 0D/T366V/D399M/ Y407A/K409V | K392D/D399K/K40 9D | T394F/F405A | | | |
| 0% Combination 279 | E345R/Q347R/K36 0D/T366V/D399M/ Y407A/K409V | K392D/D399K/K40 9D | K392L/T394W/F40 5A/Y407V | | | |
| 0% Combination 280 | E345R/Q347R/K36 0D/T366V/D399M/ Y407A/K409V | K392D/D399K/K40 9D | T350V/L351Y/T36 6L/K392L/T394W/ F405A/Y407V | | | |
| 0% Combination 281 | E345R/Q347R/K36 0D/T366V/D399M/ Y407A/K409V | E356K/K392D/D39 9K/K409D | T394F/F405A | | | |
| 0% Combination 282 | E345R/Q347R/K36 0D/T366V/D399M/ Y407A/K409V | E356K/K392D/D39 9K/K409D | K392L/T394W/F40 5A/Y407V | | | |
| 0% Combination 283 | E345R/Q347R/K36 0D/T366V/D399M/ Y407A/K409V | E356K/K392D/D39 9K/K409D | T350V/L351Y/T36 6L/K392L/T394W/ F405A/Y407V | | | |
| 0% Combination 284 | Q347E/S354Y/T366 Y/Y407T | E356K/K392D/D39 9K/K439E | E345R/K360E/D39 9M/Y407A/K409V | | | |
| 0% Combination 285 | Q347E/S354Y/T366 Y/Y407T | E356K/K392D/D39 9K/K439E | T394F/F405A | | | |
| 0% Combination 286 | Q347E/S354Y/T366 Y/Y407T | E356K/K392D/D39 9K/K439E | K392L/T394W/F40 5A/Y407V | | | |
| 0% Combination 287 | Q347E/S354Y/T366 Y/Y407T | E356K/K392D/D39 9K/K439E | T350V/L351Y/T36 6L/K392L/T394W/ F405A/Y407V | | | |
| 0% Combination 288 | Q347E/S354Y/T366 Y/Y407T | K392D/D399K/K40 9D | T394F/F405A | | | |
| 0% Combination 289 | Q347E/S354Y/T366 Y/Y407T | K392D/D399K/K40 9D | K392L/T394W/F40 5A/Y407V | | | |
| 0% Combination 290 | Q347E/S354Y/T366 Y/Y407T | K392D/D399K/K40 9D | T350V/L351Y/T36 6L/K392L/T394W/ F405A/Y407V | | | |
| 0% Combination 291 | Q347E/S354Y/T366 Y/Y407T | E356K/K392D/D39 9K/K409D | E345R/K360E/D39 9M/Y407A/K409V | | | |
| 0% Combination 292 | Q347E/S354Y/T366 Y/Y407T | E356K/K392D/D39 9K/K409D | T394F/F405A | | | |
| 0% Combination 293 | Q347E/S354Y/T366 Y/Y407T | E356K/K392D/D39 9K/K409D | K392L/T394W/F40 5A/Y407V | | | |
| 0% Combination 294 | Q347E/S354Y/T366 Y/Y407T | E356K/K392D/D39 9K/K409D | T350V/L351Y/T36 6L/K392L/T394W/ F405A/Y407V | | | |
| 0% Combination 295 | Q347E/S354Y/T366 Y/Y407T | E345R/K360E/D39 9M/Y407A/K409V | T394F/F405A | | | |
| 0% Combination 296 | Q347E/S354Y/T366 Y/Y407T | E345R/K360E/D39 9M/Y407A/K409V | K392L/T394W/F40 5A/Y407V | | | |
| 0% Combination 297 | Q347E/S354Y/T366 Y/Y407T | E345R/K360E/D39 9M/Y407A/K409V | T350V/L351Y/T36 6L/K392L/T394W/ F405A/Y407V | | | |
| 0% Combination 298 | E356K/K392D/D39 9K/K439E | E345R/K360E/D39 9M/Y407A/K409V | T394F/F405A | | | |
| 0% Combination 299 | E356K/K392D/D39 9K/K439E | E345R/K360E/D39 9M/Y407A/K409V | K392L/T394W/F40 5A/Y407V | | | |
| 0% Combination 300 | E356K/K392D/D39 9K/K439E | E345R/K360E/D39 9M/Y407A/K409V | T350V/L351Y/T36 6L/K392L/T394W/ F405A/Y407V | | | |
| 0% Combination 301 | E356K/K392D/D39 9K/K409D | E345R/K360E/D39 9M/Y407A/K409V | T394F/F405A | | | |
| 0% Combination 302 | E356K/K392D/D39 9K/K409D | E345R/K360E/D39 9M/Y407A/K409V | K392L/T394W/F40 5A/Y407V | | | |
| 0% Combination 303 | E356K/K392D/D39 9K/K409D | E345R/K360E/D39 9M/Y407A/K409V | T350V/L351Y/T36 6L/K392L/T394W/ F405A/Y407V | | | |
| 0% Combination 304 | WT | T394W/F405A | T366W/L368A/Y40 7V | Q347R/K360E/D39 9V/F405T/K409W | | |
| 0% Combination 305 | WT | T394W/F405A | T366W/L368A/Y40 7V | E356K/D399K/K40 9E/K439E | | |
| 0% Combination 306 | WT | T394W/F405A | T366W/L368A/Y40 7V | Y349C/E357C/K39 2D/D399K | | |
| 0% Combination 307 | WT | T394W/F405A | T366W/L368A/Y40 7V | E345R/Q347R/K36 0D/T366V/D399M/ Y407A/K409V | | |
| 0% Combination 308 | WT | T394W/F405A | T366W/L368A/Y40 7V | E356K/K392D/D39 9K/K439E | | |
| 0% Combination 309 | WT | T394W/F405A | T366W/L368A/Y40 7V | K392D/D399K/K40 9D | | |
| 0% Combination 310 | WT | T394W/F405A | T366W/L368A/Y40 7V | E356K/K392D/D39 9K/K409D | | |
| 0% Combination 311 | WT | T394W/F405A | T366W/L368A/Y40 7V | E345R/K360E/D39 9M/Y407A/K409V | | |
| 0% Combination 312 | WT | T394W/F405A | Q347R/K360E/D39 9V/F405T/K409W | E356K/D399K/K40 9E/K439E | | |
| 0% Combination 313 | WT | T394W/F405A | Q347R/K360E/D39 9V/F405T/K409W | Y349C/E357C/K39 2D/D399K | | |
| 0% Combination 314 | WT | T394W/F405A | Q347R/K360E/D39 9V/F405T/K409W | Q347E/S354Y/T366 Y/Y407T | | |
| 0% Combination 315 | WT | T394W/F405A | Q347R/K360E/D39 9V/F405T/K409W | E356K/K392D/D39 9K/K439E | | |
| 0% Combination 316 | WT | T394W/F405A | Q347R/K360E/D39 9V/F405T/K409W | K392D/D399K/K40 9D | | |
| 0% Combination 317 | WT | T394W/F405A | Q347R/K360E/D39 9V/F405T/K409W | E356K/K392D/D39 9K/K409D | | |
| 0% Combination 318 | WT | T394W/F405A | E356K/D399K/K40 9E/K439E | Y349C/E357C/K39 2D/D399K | | |
| 0% Combination 319 | WT | T394W/F405A | E356K/D399K/K40 9E/K439E | E345R/Q347R/K36 0D/T366V/D399M/ Y407A/K409V | | |
| 0% Combination 320 | WT | T394W/F405A | E356K/D399K/K40 9E/K439E | Q347E/S354Y/T366 Y/Y407T | | |
| 0% Combination 321 | WT | T394W/F405A | E356K/D399K/K40 9E/K439E | E345R/K360E/D39 9M/Y407A/K409V | | |
| 0% Combination 322 | WT | T394W/F405A | Y349C/E357C/K39 2D/D399K | E356K/K392D/D39 9K/K439E | | |
| 0% Combination 323 | WT | T394W/F405A | Y349C/E357C/K39 2D/D399K | E356K/K392D/D39 9K/K409D | | |
| 0% Combination 324 | WT | T394W/F405A | Y349C/E357C/K39 2D/D399K | E345R/K360E/D39 9M/Y407A/K409V | | |
| 0% Combination 325 | WT | T394W/F405A | E345R/Q347R/K36 0D/T366V/D399M/ Y407A/K409V | E356K/K392D/D39 9K/K439E | | |
| 0% Combination 326 | WT | T394W/F405A | E345R/Q347R/K36 0D/T366V/D399M/ Y407A/K409V | K392D/D399K/K40 9D | | |
| 0% Combination 327 | WT | T394W/F405A | E345R/Q347R/K36 0D/T366V/D399M/ Y407A/K409V | E356K/K392D/D39 9K/K409D | | |
| 0% Combination 328 | WT | T394W/F405A | Q347E/S354Y/T366 Y/Y407T | E356K/K392D/D39 9K/K439E | | |
| 0% Combination 329 | WT | T394W/F405A | Q347E/S354Y/T366 Y/Y407T | K392D/D399K/K40 9D | | |
| 0% Combination 330 | WT | T394W/F405A | Q347E/S354Y/T366 Y/Y407T | E356K/K392D/D39 9K/K409D | | |
| 0% Combination 331 | WT | T394W/F405A | Q347E/S354Y/T366 Y/Y407T | E345R/K360E/D39 9M/Y407A/K409V | | |
| 0% Combination 332 | WT | T394W/F405A | E356K/K392D/D39 9K/K439E | E345R/K360E/D39 9M/Y407A/K409V | | |
| 0% Combination 333 | WT | T394W/F405A | E356K/K392D/D39 9K/K409D | E345R/K360E/D39 9M/Y407A/K409V | | |
| 0% Combination 334 | WT | T366W/L368A/Y40 7V | Q347R/K360E/D39 9V/F405T/K409W | E356K/D399K/K40 9E/K439E | | |
| 0% Combination 335 | WT | T366W/L368A/Y40 7V | Q347R/K360E/D39 9V/F405T/K409W | Y349C/E357C/K39 2D/D399K | | |
| 0% Combination 336 | WT | T366W/L368A/Y40 7V | Q347R/K360E/D39 9V/F405T/K409W | E356K/K392D/D39 9K/K439E | | |
| 0% Combination 337 | WT | T366W/L368A/Y40 7V | Q347R/K360E/D39 9V/F405T/K409W | K392D/D399K/K40 9D | | |
| 0% Combination 338 | WT | T366W/L368A/Y40 7V | Q347R/K360E/D39 9V/F405T/K409W | E356K/K392D/D39 9K/K409D | | |
| 0% Combination 339 | WT | T366W/L368A/Y40 7V | Q347R/K360E/D39 9V/F405T/K409W | T394F/F405A | | |
| 0% Combination 340 | WT | T366W/L368A/Y40 7V | Q347R/K360E/D39 9V/F405T/K409W | K392L/T394W/F40 5A/Y407V | | |
| 0% Combination 341 | WT | T366W/L368A/Y40 7V | Q347R/K360E/D39 9V/F405T/K409W | T350V/L351Y/T36 6L/K392L/T394W/ F405A/Y407V | | |
| 0% Combination 342 | WT | T366W/L368A/Y40 7V | E356K/D399K/K40 9E/K439E | Y349C/E357C/K39 2D/D399K | | |
| 0% Combination 343 | WT | T366W/L368A/Y40 7V | E356K/D399K/K40 9E/K439E | E345R/Q347R/K36 0D/T366V/D399M/ Y407A/K409V | | |
| 0% Combination 344 | WT | T366W/L368A/Y40 7V | E356K/D399K/K40 9E/K439E | E345R/K360E/D39 9M/Y407A/K409V | | |
| 0% Combination 345 | WT | T366W/L368A/Y40 7V | E356K/D399K/K40 9E/K439E | T394F/F405A | | |
| 0% Combination 346 | WT | T366W/L368A/Y40 7V | E356K/D399K/K40 9E/K439E | K392L/T394W/F40 5A/Y407V | | |
| 0% Combination 347 | WT | T366W/L368A/Y40 7V | E356K/D399K/K40 9E/K439E | T350V/L351Y/T36 6L/K392L/T394W/ F405A/Y407V | | |
| 0% Combination 348 | WT | T366W/L368A/Y40 7V | Y349C/E357C/K39 2D/D399K | E356K/K392D/D39 9K/K439E | | |
| 0% Combination 349 | WT | T366W/L368A/Y40 7V | Y349C/E357C/K39 2D/D399K | E356K/K392D/D39 9K/K409D | | |
| 0% Combination 350 | WT | T366W/L368A/Y40 7V | Y349C/E357C/K39 2D/D399K | E345R/K360E/D39 9M/Y407A/K409V | | |
| 0% Combination 351 | WT | T366W/L368A/Y40 7V | Y349C/E357C/K39 2D/D399K | T394F/F405A | | |
| 0% Combination 352 | WT | T366W/L368A/Y40 7V | Y349C/E357C/K39 2D/D399K | K392L/T394W/F40 5A/Y407V | | |
| 0% Combination 353 | WT | T366W/L368A/Y40 7V | Y349C/E357C/K39 2D/D399K | T350V/L351Y/T36 6L/K392L/T394W/ F405A/Y407V | | |
| 0% Combination 354 | WT | T366W/L368A/Y40 7V | E345R/Q347R/K36 0D/T366V/D399M/ Y407A/K409V | E356K/K392D/D39 9K/K439E | | |
| 0% Combination 355 | WT | T366W/L368A/Y40 7V | E345R/Q347R/K36 0D/T366V/D399M/ Y407A/K409V | K392D/D399K/K40 9D | | |
| 0% Combination 356 | WT | T366W/L368A/Y40 7V | E345R/Q347R/K36 0D/T366V/D399M/ Y407A/K409V | E356K/K392D/D39 9K/K409D | | |
| 0% Combination 357 | WT | T366W/L368A/Y40 7V | E345R/Q347R/K36 0D/T366V/D399M/ Y407A/K409V | T394F/F405A | | |
| 0% Combination 358 | WT | T366W/L368A/Y40 7V | E345R/Q347R/K36 0D/T366V/D399M/ Y407A/K409V | K392L/T394W/F40 5A/Y407V | | |
| 0% Combination 359 | WT | T366W/L368A/Y40 7V | E345R/Q347R/K36 0D/T366V/D399M/ Y407A/K409V | T350V/L351Y/T36 6L/K392L/T394W/ F405A/Y407V | | |
| 0% Combination 360 | WT | T366W/L368A/Y40 7V | E356K/K392D/D39 9K/K439E | E345R/K360E/D39 9M/Y407A/K409V | | |
| 0% Combination 361 | WT | T366W/L368A/Y40 7V | E356K/K392D/D39 9K/K439E | T394F/F405A | | |
| 0% Combination 362 | WT | T366W/L368A/Y40 7V | E356K/K392D/D39 9K/K439E | K392L/T394W/F40 5A/Y407V | | |
| 0% Combination 363 | WT | T366W/L368A/Y40 7V | E356K/K392D/D39 9K/K439E | T350V/L351Y/T36 6L/K392L/T394W/ F405A/Y407V | | |
| 0% Combination 364 | WT | T366W/L368A/Y40 7V | K392D/D399K/K40 9D | T394F/F405A | | |
| 0% Combination 365 | WT | T366W/L368A/Y40 7V | K392D/D399K/K40 9D | K392L/T394W/F40 5A/Y407V | | |
| 0% Combination 366 | WT | T366W/L368A/Y40 7V | K392D/D399K/K40 9D | T350V/L351Y/T36 6L/K392L/T394W/ F405A/Y407V | | |
| 0% Combination 367 | WT | T366W/L368A/Y40 7V | E356K/K392D/D39 9K/K409D | E345R/K360E/D39 9M/Y407A/K409V | | |
| 0% Combination 368 | WT | T366W/L368A/Y40 7V | E356K/K392D/D39 9K/K409D | T394F/F405A | | |
| 0% Combination 369 | WT | T366W/L368A/Y40 7V | E356K/K392D/D39 9K/K409D | K392L/T394W/F40 5A/Y407V | | |
| 0% Combination 370 | WT | T366W/L368A/Y40 7V | E356K/K392D/D39 9K/K409D | T350V/L351Y/T36 6L/K392L/T394W/ F405A/Y407V | | |
| 0% Combination 371 | WT | T366W/L368A/Y40 7V | E345R/K360E/D39 9M/Y407A/K409V | T394F/F405A | | |
| 0% Combination 372 | WT | T366W/L368A/Y40 7V | E345R/K360E/D39 9M/Y407A/K409V | K392L/T394W/F40 5A/Y407V | | |
| 0% Combination 373 | WT | T366W/L368A/Y40 7V | E345R/K360E/D39 9M/Y407A/K409V | T350V/L351Y/T36 6L/K392L/T394W/ F405A/Y407V | | |
| 0% Combination 374 | WT | Q347R/K360E/D39 9V/F405T/K409W | E356K/D399K/K40 9E/K439E | Y349C/E357C/K39 2D/D399K | | |
| 0% Combination 375 | WT | Q347R/K360E/D39 9V/F405T/K409W | E356K/D399K/K40 9E/K439E | Q347E/S354Y/T366 Y/Y407T | | |
| 0% Combination 376 | WT | Q347R/K360E/D39 9V/F405T/K409W | E356K/D399K/K40 9E/K439E | T394F/F405A | | |
| 0% Combination 377 | WT | Q347R/K360E/D39 9V/F405T/K409W | E356K/D399K/K40 9E/K439E | K392L/T394W/F40 5A/Y407V | | |
| 0% Combination 378 | WT | Q347R/K360E/D39 9V/F405T/K409W | E356K/D399K/K40 9E/K439E | T350V/L351Y/T36 6L/K392L/T394W/ F405A/Y407V | | |
| 0% Combination 379 | WT | Q347R/K360E/D39 9V/F405T/K409W | Y349C/E357C/K39 2D/D399K | E356K/K392D/D39 9K/K439E | | |
| 0% Combination 380 | WT | Q347R/K360E/D39 9V/F405T/K409W | Y349C/E357C/K39 2D/D399K | E356K/K392D/D39 9K/K409D | | |
| 0% Combination 381 | WT | Q347R/K360E/D39 9V/F405T/K409W | Y349C/E357C/K39 2D/D399K | T394F/F405A | | |
| 0% Combination 382 | WT | Q347R/K360E/D39 9V/F405T/K409W | Y349C/E357C/K39 2D/D399K | K392L/T394W/F40 5A/Y407V | | |
| 0% Combination 383 | WT | Q347R/K360E/D39 9V/F405T/K409W | Y349C/E357C/K39 2D/D399K | T350V/L351Y/T36 6L/K392L/T394W/ F405A/Y407V | | |
| 0% Combination 384 | WT | Q347R/K360E/D39 9V/F405T/K409W | Q347E/S354Y/T366 Y/Y407T | E356K/K392D/D39 9K/K439E | | |
| 0% Combination 385 | WT | Q347R/K360E/D39 9V/F405T/K409W | Q347E/S354Y/T366 Y/Y407T | K392D/D399K/K40 9D | | |
| 0% Combination 386 | WT | Q347R/K360E/D39 9V/F405T/K409W | Q347E/S354Y/T366 Y/Y407T | E356K/K392D/D39 9K/K409D | | |
| 0% Combination 387 | WT | Q347R/K360E/D39 9V/F405T/K409W | Q347E/S354Y/T366 Y/Y407T | T394F/F405A | | |
| 0% Combination 388 | WT | Q347R/K360E/D39 9V/F405T/K409W | Q347E/S354Y/T366 Y/Y407T | K392L/T394W/F40 5A/Y407V | | |
| 0% Combination 389 | WT | Q347R/K360E/D39 9V/F405T/K409W | Q347E/S354Y/T366 Y/Y407T | T350V/L351Y/T36 6L/K392L/T394W/ F405A/Y407V | | |
| 0% Combination 390 | WT | Q347R/K360E/D39 9V/F405T/K409W | E356K/K392D/D39 9K/K439E | T394F/F405A | | |
| 0% Combination 391 | WT | Q347R/K360E/D39 9V/F405T/K409W | E356K/K392D/D39 9K/K439E | K392L/T394W/F40 5A/Y407V | | |
| 0% Combination 392 | WT | Q347R/K360E/D39 9V/F405T/K409W | E356K/K392D/D39 9K/K439E | T350V/L351Y/T36 6L/K392L/T394W/ F405A/Y407V | | |
| 0% Combination 393 | WT | Q347R/K360E/D39 9V/F405T/K409W | K392D/D399K/K40 9D | T394F/F405A | | |
| 0% Combination 394 | WT | Q347R/K360E/D39 9V/F405T/K409W | K392D/D399K/K40 9D | K392L/T394W/F40 5A/Y407V | | |
| 0% Combination 395 | WT | Q347R/K360E/D39 9V/F405T/K409W | K392D/D399K/K40 9D | T350V/L351Y/T36 6L/K392L/T394W/ F405A/Y407V | | |
| 0% Combination 396 | WT | Q347R/K360E/D39 9V/F405T/K409W | E356K/K392D/D39 9K/K409D | T394F/F405A | | |
| 0% Combination 397 | WT | Q347R/K360E/D39 9V/F405T/K409W | E356K/K392D/D39 9K/K409D | K392L/T394W/F40 5A/Y407V | | |
| 0% Combination 398 | WT | Q347R/K360E/D39 9V/F405T/K409W | E356K/K392D/D39 9K/K409D | T350V/L351Y/T36 6L/K392L/T394W/ F405A/Y407V | | |
| 0% Combination 399 | WT | E356K/D399K/K40 9E/K439E | Y349C/E357C/K39 2D/D399K | E345R/K360E/D39 9M/Y407A/K409V | | |
| 0% Combination 400 | WT | E356K/D399K/K40 9E/K439E | Y349C/E357C/K39 2D/D399K | T394F/F405A | | |
| 0% Combination 401 | WT | E356K/D399K/K40 9E/K439E | Y349C/E357C/K39 2D/D399K | K392L/T394W/F40 5A/Y407V | | |
| 0% Combination 402 | WT | E356K/D399K/K40 9E/K439E | Y349C/E357C/K39 2D/D399K | T350V/L351Y/T36 6L/K392L/T394W/ F405A/Y407V | | |
| 0% Combination 403 | WT | E356K/D399K/K40 9E/K439E | E345R/Q347R/K36 0D/T366V/D399M/ Y407A/K409V | T394F/F405A | | |
| 0% Combination 404 | WT | E356K/D399K/K40 9E/K439E | E345R/Q347R/K36 0D/T366V/D399M/ Y407A/K409V | K392L/T394W/F40 5A/Y407V | | |
| 0% Combination 405 | WT | E356K/D399K/K40 9E/K439E | E345R/Q347R/K36 0D/T366V/D399M/ Y407A/K409V | T350V/L351Y/T36 6L/K392L/T394W/ F405A/Y407V | | |
| 0% Combination 406 | WT | E356K/D399K/K40 9E/K439E | Q347E/S354Y/T366 Y/Y407T | E345R/K360E/D39 9M/Y407A/K409V | | |
| 0% Combination 407 | WT | E356K/D399K/K40 9E/K439E | Q347E/S354Y/T366 Y/Y407T | T394F/F405A | | |
| 0% Combination 408 | WT | E356K/D399K/K40 9E/K439E | Q347E/S354Y/T366 Y/Y407T | K392L/T394W/F40 5A/Y407V | | |
| 0% Combination 409 | WT | E356K/D399K/K40 9E/K439E | Q347E/S354Y/T366 Y/Y407T | T350V/L351Y/T36 6L/K392L/T394W/ F405A/Y407V | | |
| 0% Combination 410 | WT | E356K/D399K/K40 9E/K439E | E345R/K360E/D39 9M/Y407A/K409V | T394F/F405A | | |
| 0% Combination 411 | WT | E356K/D399K/K40 9E/K439E | E345R/K360E/D39 9M/Y407A/K409V | K392L/T394W/F40 5A/Y407V | | |
| 0% Combination 412 | WT | E356K/D399K/K40 9E/K439E | E345R/K360E/D39 9M/Y407A/K409V | T350V/L351Y/T36 6L/K392L/T394W/ F405A/Y407V | | |
| 0% Combination 413 | WT | Y349C/E357C/K39 2D/D399K | E356K/K392D/D39 9K/K439E | E345R/K360E/D39 9M/Y407A/K409V | | |
| 0% Combination 414 | WT | Y349C/E357C/K39 2D/D399K | E356K/K392D/D39 9K/K439E | T394F/F405A | | |
| 0% Combination 415 | WT | Y349C/E357C/K39 2D/D399K | E356K/K392D/D39 9K/K439E | K392L/T394W/F40 5A/Y407V | | |
| 0% Combination 416 | WT | Y349C/E357C/K39 2D/D399K | E356K/K392D/D39 9K/K439E | T350V/L351Y/T36 6L/K392L/T394W/ F405A/Y407V | | |
| 0% Combination 417 | WT | Y349C/E357C/K39 2D/D399K | E356K/K392D/D39 9K/K409D | E345R/K360E/D39 9M/Y407A/K409V | | |
| 0% Combination 418 | WT | Y349C/E357C/K39 2D/D399K | E356K/K392D/D39 9K/K409D | T394F/F405A | | |
| 0% Combination 419 | WT | Y349C/E357C/K39 2D/D399K | E356K/K392D/D39 9K/K409D | K392L/T394W/F40 5A/Y407V | | |
| 0% Combination 420 | WT | Y349C/E357C/K39 2D/D399K | E356K/K392D/D39 9K/K409D | T350V/L351Y/T36 6L/K392L/T394W/ F405A/Y407V | | |
| 0% Combination 421 | WT | Y349C/E357C/K39 2D/D399K | E345R/K360E/D39 9M/Y407A/K409V | T394F/F405A | | |
| 0% Combination 422 | WT | Y349C/E357C/K39 2D/D399K | E345R/K360E/D39 9M/Y407A/K409V | K392L/T394W/F40 5A/Y407V | | |
| 0% Combination 423 | WT | Y349C/E357C/K39 2D/D399K | E345R/K360E/D39 9M/Y407A/K409V | T350V/L351Y/T36 6L/K392L/T394W/ F405A/Y407V | | |
| 0% Combination 424 | WT | E345R/Q347R/K36 0D/T366V/D399M/ Y407A/K409V | E356K/K392D/D39 9K/K439E | T394F/F405A | | |
| 0% Combination 425 | WT | E345R/Q347R/K36 0D/T366V/D399M/ Y407A/K409V | E356K/K392D/D39 9K/K439E | K392L/T394W/F40 5A/Y407V | | |
| 0% Combination 426 | WT | E345R/Q347R/K36 0D/T366V/D399M/ Y407A/K409V | E356K/K392D/D39 9K/K439E | T350V/L351Y/T36 6L/K392L/T394W/ F405A/Y407V | | |
| 0% Combination 427 | WT | E345R/Q347R/K36 0D/T366V/D399M/ Y407A/K409V | K392D/D399K/K40 9D | T394F/F405A | | |
| 0% Combination 428 | WT | E345R/Q347R/K36 0D/T366V/D399M/ Y407A/K409V | K392D/D399K/K40 9D | K392L/T394W/F40 5A/Y407V | | |
| 0% Combination 429 | WT | E345R/Q347R/K36 0D/T366V/D399M/ Y407A/K409V | K392D/D399K/K40 9D | T350V/L351Y/T36 6L/K392L/T394W/ F405A/Y407V | | |
| 0% Combination 430 | WT | E345R/Q347R/K36 0D/T366V/D399M/ Y407A/K409V | E356K/K392D/D39 9K/K409D | T394F/F405A | | |
| 0% Combination 431 | WT | E345R/Q347R/K36 0D/T366V/D399M/ Y407A/K409V | E356K/K392D/D39 9K/K409D | K392L/T394W/F40 5A/Y407V | | |
| 0% Combination 432 | WT | E345R/Q347R/K36 0D/T366V/D399M/ Y407A/K409V | E356K/K392D/D39 9K/K409D | T350V/L351Y/T36 6L/K392L/T394W/ F405A/Y407V | | |
| 0% Combination 433 | WT | Q347E/S354Y/T366 Y/Y407T | E356K/K392D/D39 9K/K439E | E345R/K360E/D39 9M/Y407A/K409V | | |
| 0% Combination 434 | WT | Q347E/S354Y/T366 Y/Y407T | E356K/K392D/D39 9K/K439E | T394F/F405A | | |
| 0% Combination 435 | WT | Q347E/S354Y/T366 Y/Y407T | E356K/K392D/D39 9K/K439E | K392L/T394W/F40 5A/Y407V | | |
| 0% Combination 436 | WT | Q347E/S354Y/T366 Y/Y407T | E356K/K392D/D39 9K/K439E | T350V/L351Y/T36 6L/K392L/T394W/ F405A/Y407V | | |
| 0% Combination 437 | WT | Q347E/S354Y/T366 Y/Y407T | K392D/D399K/K40 9D | T394F/F405A | | |
| 0% Combination 438 | WT | Q347E/S354Y/T366 Y/Y407T | K392D/D399K/K40 9D | K392L/T394W/F40 5A/Y407V | | |
| 0% Combination 439 | WT | Q347E/S354Y/T366 Y/Y407T | K392D/D399K/K40 9D | T350V/L351Y/T36 6L/K392L/T394W/ F405A/Y407V | | |
| 0% Combination 440 | WT | Q347E/S354Y/T366 Y/Y407T | E356K/K392D/D39 9K/K409D | E345R/K360E/D39 9M/Y407A/K409V | | |
| 0% Combination 441 | WT | Q347E/S354Y/T366 Y/Y407T | E356K/K392D/D39 9K/K409D | T394F/F405A | | |
| 0% Combination 442 | WT | Q347E/S354Y/T366 Y/Y407T | E356K/K392D/D39 9K/K409D | K392L/T394W/F40 5A/Y407V | | |
| 0% Combination 443 | WT | Q347E/S354Y/T366 Y/Y407T | E356K/K392D/D39 9K/K409D | T350V/L351Y/T36 6L/K392L/T394W/ F405A/Y407V | | |
| 0% Combination 444 | WT | Q347E/S354Y/T366 Y/Y407T | E345R/K360E/D39 9M/Y407A/K409V | T394F/F405A | | |
| 0% Combination 445 | WT | Q347E/S354Y/T366 Y/Y407T | E345R/K360E/D39 9M/Y407A/K409V | K392L/T394W/F40 5A/Y407V | | |
| 0% Combination 446 | WT | Q347E/S354Y/T366 Y/Y407T | E345R/K360E/D39 9M/Y407A/K409V | T350V/L351Y/T36 6L/K392L/T394W/ F405A/Y407V | | |
| 0% Combination 447 | WT | E356K/K392D/D39 9K/K439E | E345R/K360E/D39 9M/Y407A/K409V | T394F/F405A | | |
| 0% Combination 448 | WT | E356K/K392D/D39 9K/K439E | E345R/K360E/D39 9M/Y407A/K409V | K392L/T394W/F40 5A/Y407V | | |
| 0% Combination 449 | WT | E356K/K392D/D39 9K/K439E | E345R/K360E/D39 9M/Y407A/K409V | T350V/L351Y/T36 6L/K392L/T394W/ F405A/Y407V | | |
| 0% Combination 450 | WT | E356K/K392D/D39 9K/K409D | E345R/K360E/D39 9M/Y407A/K409V | T394F/F405A | | |
| 0% Combination 451 | WT | E356K/K392D/D39 9K/K409D | E345R/K360E/D39 9M/Y407A/K409V | K392L/T394W/F40 5A/Y407V | | |
| 0% Combination 452 | WT | E356K/K392D/D39 9K/K409D | E345R/K360E/D39 9M/Y407A/K409V | T350V/L351Y/T36 6L/K392L/T394W/ F405A/Y407V | | |
| 0% Combination 453 | T394W/F405A | T366W/L368A/Y40 7V | Q347R/K360E/D39 9V/F405T/K409W | E356K/D399K/K40 9E/K439E | | |
| 0% Combination 454 | T394W/F405A | T366W/L368A/Y40 7V | Q347R/K360E/D39 9V/F405T/K409W | Y349C/E357C/K39 2D/D399K | | |
| 0% Combination 455 | T394W/F405A | T366W/L368A/Y40 7V | Q347R/K360E/D39 9V/F405T/K409W | E356K/K392D/D39 9K/K439E | | |
| 0% Combination 456 | T394W/F405A | T366W/L368A/Y40 7V | Q347R/K360E/D39 9V/F405T/K409W | K392D/D399K/K40 9D | | |
| 0% Combination 457 | T394W/F405A | T366W/L368A/Y40 7V | Q347R/K360E/D39 9V/F405T/K409W | E356K/K392D/D39 9K/K409D | | |
| 0% Combination 458 | T394W/F405A | T366W/L368A/Y40 7V | E356K/D399K/K40 9E/K439E | Y349C/E357C/K39 2D/D399K | | |
| 0% Combination 459 | T394W/F405A | T366W/L368A/Y40 7V | E356K/D399K/K40 9E/K439E | E345R/Q347R/K36 0D/T366V/D399M/ Y407A/K409V | | |
| 0% Combination 460 | T394W/F405A | T366W/L368A/Y40 7V | E356K/D399K/K40 9E/K439E | E345R/K360E/D39 9M/Y407A/K409V | | |
| 0% Combination 461 | T394W/F405A | T366W/L368A/Y40 7V | Y349C/E357C/K39 2D/D399K | E356K/K392D/D39 9K/K439E | | |
| 0% Combination 462 | T394W/F405A | T366W/L368A/Y40 7V | Y349C/E357C/K39 2D/D399K | E356K/K392D/D39 9K/K409D | | |
| 0% Combination 463 | T394W/F405A | T366W/L368A/Y40 7V | Y349C/E357C/K39 2D/D399K | E345R/K360E/D39 9M/Y407A/K409V | | |
| 0% Combination 464 | T394W/F405A | T366W/L368A/Y40 7V | E345R/Q347R/K36 0D/T366V/D399M/ Y407A/K409V | E356K/K392D/D39 9K/K439E | | |
| 0% Combination 465 | T394W/F405A | T366W/L368A/Y40 7V | E345R/Q347R/K36 0D/T366V/D399M/ Y407A/K409V | K392D/D399K/K40 9D | | |
| 0% Combination 466 | T394W/F405A | T366W/L368A/Y40 7V | E345R/Q347R/K36 0D/T366V/D399M/ Y407A/K409V | E356K/K392D/D39 9K/K409D | | |
| 0% Combination 467 | T394W/F405A | T366W/L368A/Y40 7V | E356K/K392D/D39 9K/K439E | E345R/K360E/D39 9M/Y407A/K409V | | |
| 0% Combination 468 | T394W/F405A | T366W/L368A/Y40 7V | E356K/K392D/D39 9K/K409D | E345R/K360E/D39 9M/Y407A/K409V | | |
| 0% Combination 469 | T394W/F405A | Q347R/K360E/D39 9V/F405T/K409W | E356K/D399K/K40 9E/K439E | Y349C/E357C/K39 2D/D399K | | |
| 0% Combination 470 | T394W/F405A | Q347R/K360E/D39 9V/F405T/K409W | E356K/D399K/K40 9E/K439E | Q347E/S354Y/T366 Y/Y407T | | |
| 0% Combination 471 | T394W/F405A | Q347R/K360E/D39 9V/F405T/K409W | Y349C/E357C/K39 2D/D399K | E356K/K392D/D39 9K/K439E | | |
| 0% Combination 472 | T394W/F405A | Q347R/K360E/D39 9V/F405T/K409W | Y349C/E357C/K39 2D/D399K | E356K/K392D/D39 9K/K409D | | |
| 0% Combination 473 | T394W/F405A | Q347R/K360E/D39 9V/F405T/K409W | Q347E/S354Y/T366 Y/Y407T | E356K/K392D/D39 9K/K439E | | |
| 0% Combination 474 | T394W/F405A | Q347R/K360E/D39 9V/F405T/K409W | Q347E/S354Y/T366 Y/Y407T | K392D/D399K/K40 9D | | |
| 0% Combination 475 | T394W/F405A | Q347R/K360E/D39 9V/F405T/K409W | Q347E/S354Y/T366 Y/Y407T | E356K/K392D/D39 9K/K409D | | |
| 0% Combination 476 | T394W/F405A | E356K/D399K/K40 9E/K439E | Y349C/E357C/K39 2D/D399K | E345R/K360E/D39 9M/Y407A/K409V | | |
| 0% Combination 477 | T394W/F405A | E356K/D399K/K40 9E/K439E | Q347E/S354Y/T366 Y/Y407T | E345R/K360E/D39 9M/Y407A/K409V | | |
| 0% Combination 478 | T394W/F405A | Y349C/E357C/K39 2D/D399K | E356K/K392D/D39 9K/K439E | E345R/K360E/D39 9M/Y407A/K409V | | |
| 0% Combination 479 | T394W/F405A | Y349C/E357C/K39 2D/D399K | E356K/K392D/D39 9K/K409D | E345R/K360E/D39 9M/Y407A/K409V | | |
| 0% Combination 480 | T394W/F405A | Q347E/S354Y/T366 Y/Y407T | E356K/K392D/D39 9K/K439E | E345R/K360E/D39 9M/Y407A/K409V | | |
| 0% Combination 481 | T394W/F405A | Q347E/S354Y/T366 Y/Y407T | E356K/K392D/D39 9K/K409D | E345R/K360E/D39 9M/Y407A/K409V | | |
| 0% Combination 482 | T366W/L368A/Y40 7V | Q347R/K360E/D39 9V/F405T/K409W | E356K/D399K/K40 9E/K439E | Y349C/E357C/K39 2D/D399K | | |
| 0% Combination 483 | T366W/L368A/Y40 7V | Q347R/K360E/D39 9V/F405T/K409W | E356K/D399K/K40 9E/K439E | T394F/F405A | | |
| 0% Combination 484 | T366W/L368A/Y40 7V | Q347R/K360E/D39 9V/F405T/K409W | E356K/D399K/K40 9E/K439E | K392L/T394W/F40 5A/Y407V | | |
| 0% Combination 485 | T366W/L368A/Y40 7V | Q347R/K360E/D39 9V/F405T/K409W | E356K/D399K/K40 9E/K439E | T350V/L351Y/T36 6L/K392L/T394W/ F405A/Y407V | | |
| 0% Combination 486 | T366W/L368A/Y40 7V | Q347R/K360E/D39 9V/F405T/K409W | Y349C/E357C/K39 2D/D399K | E356K/K392D/D39 9K/K439E | | |
| 0% Combination 487 | T366W/L368A/Y40 7V | Q347R/K360E/D39 9V/F405T/K409W | Y349C/E357C/K39 2D/D399K | E356K/K392D/D39 9K/K409D | | |
| 0% Combination 488 | T366W/L368A/Y40 7V | Q347R/K360E/D39 9V/F405T/K409W | Y349C/E357C/K39 2D/D399K | T394F/F405A | | |
| 0% Combination 489 | T366W/L368A/Y40 7V | Q347R/K360E/D39 9V/F405T/K409W | Y349C/E357C/K39 2D/D399K | K392L/T394W/F40 5A/Y407V | | |
| 0% Combination 490 | T366W/L368A/Y40 7V | Q347R/K360E/D39 9V/F405T/K409W | Y349C/E357C/K39 2D/D399K | T350V/L351Y/T36 6L/K392L/T394W/ F405A/Y407V | | |
| 0% Combination 491 | T366W/L368A/Y40 7V | Q347R/K360E/D39 9V/F405T/K409W | E356K/K392D/D39 9K/K439E | T394F/F405A | | |
| 0% Combination 492 | T366W/L368A/Y40 7V | Q347R/K360E/D39 9V/F405T/K409W | E356K/K392D/D39 9K/K439E | K392L/T394W/F40 5A/Y407V | | |
| 0% Combination 493 | T366W/L368A/Y40 7V | Q347R/K360E/D39 9V/F405T/K409W | E356K/K392D/D39 9K/K439E | T350V/L351Y/T36 6L/K392L/T394W/ F405A/Y407V | | |
| 0% Combination 494 | T366W/L368A/Y40 7V | Q347R/K360E/D39 9V/F405T/K409W | K392D/D399K/K40 9D | T394F/F405A | | |
| 0% Combination 495 | T366W/L368A/Y40 7V | Q347R/K360E/D39 9V/F405T/K409W | K392D/D399K/K40 9D | K392L/T394W/F40 5A/Y407V | | |
| 0% Combination 496 | T366W/L368A/Y40 7V | Q347R/K360E/D39 9V/F405T/K409W | K392D/D399K/K40 9D | T350V/L351Y/T36 6L/K392L/T394W/ F405A/Y407V | | |
| 0% Combination 497 | T366W/L368A/Y40 7V | Q347R/K360E/D39 9V/F405T/K409W | E356K/K392D/D39 9K/K409D | T394F/F405A | | |
| 0% Combination 498 | T366W/L368A/Y40 7V | Q347R/K360E/D39 9V/F405T/K409W | E356K/K392D/D39 9K/K409D | K392L/T394W/F40 5A/Y407V | | |
| 0% Combination 499 | T366W/L368A/Y40 7V | Q347R/K360E/D39 9V/F405T/K409W | E356K/K392D/D39 9K/K409D | T350V/L351Y/T36 6L/K392L/T394W/ F405A/Y407V | | |
| 0% Combination 500 | T366W/L368A/Y40 7V | E356K/D399K/K40 9E/K439E | Y349C/E357C/K39 2D/D399K | E345R/K360E/D39 9M/Y407A/K409V | | |
| 0% Combination 501 | T366W/L368A/Y40 7V | E356K/D399K/K40 9E/K439E | Y349C/E357C/K39 2D/D399K | T394F/F405A | | |
| 0% Combination 502 | T366W/L368A/Y40 7V | E356K/D399K/K40 9E/K439E | Y349C/E357C/K39 2D/D399K | K392L/T394W/F40 5A/Y407V | | |
| 0% Combination 503 | T366W/L368A/Y40 7V | E356K/D399K/K40 9E/K439E | Y349C/E357C/K39 2D/D399K | T350V/L351Y/T36 6L/K392L/T394W/ F405A/Y407V | | |
| 0% Combination 504 | T366W/L368A/Y40 7V | E356K/D399K/K40 9E/K439E | E345R/Q347R/K36 0D/T366V/D399M/ Y407A/K409V | T394F/F405A | | |
| 0% Combination 505 | T366W/L368A/Y40 7V | E356K/D399K/K40 9E/K439E | E345R/Q347R/K36 0D/T366V/D399M/ Y407A/K409V | K392L/T394W/F40 5A/Y407V | | |
| 0% Combination 506 | T366W/L368A/Y40 7V | E356K/D399K/K40 9E/K439E | E345R/Q347R/K36 0D/T366V/D399M/ Y407A/K409V | T350V/L351Y/T36 6L/K392L/T394W/ F405A/Y407V | | |
| 0% Combination 507 | T366W/L368A/Y40 7V | E356K/D399K/K40 9E/K439E | E345R/K360E/D39 9M/Y407A/K409V | T394F/F405A | | |
| 0% Combination 508 | T366W/L368A/Y40 7V | E356K/D399K/K40 9E/K439E | E345R/K360E/D39 9M/Y407A/K409V | K392L/T394W/F40 5A/Y407V | | |
| 0% Combination 509 | T366W/L368A/Y40 7V | E356K/D399K/K40 9E/K439E | E345R/K360E/D39 9M/Y407A/K409V | T350V/L351Y/T36 6L/K392L/T394W/ F405A/Y407V | | |
| 0% Combination 510 | T366W/L368A/Y40 7V | Y349C/E357C/K39 2D/D399K | E356K/K392D/D39 9K/K439E | E345R/K360E/D39 9M/Y407A/K409V | | |
| 0% Combination 511 | T366W/L368A/Y40 7V | Y349C/E357C/K39 2D/D399K | E356K/K392D/D39 9K/K439E | T394F/F405A | | |
| 0% Combination 512 | T366W/L368A/Y40 7V | Y349C/E357C/K39 2D/D399K | E356K/K392D/D39 9K/K439E | K392L/T394W/F40 5A/Y407V | | |
| 0% Combination 513 | T366W/L368A/Y40 7V | Y349C/E357C/K39 2D/D399K | E356K/K392D/D39 9K/K439E | T350V/L351Y/T36 6L/K392L/T394W/ F405A/Y407V | | |
| 0% Combination 514 | T366W/L368A/Y40 7V | Y349C/E357C/K39 2D/D399K | E356K/K392D/D39 9K/K409D | E345R/K360E/D39 9M/Y407A/K409V | | |
| 0% Combination 515 | T366W/L368A/Y40 7V | Y349C/E357C/K39 2D/D399K | E356K/K392D/D39 9K/K409D | T394F/F405A | | |
| 0% Combination 516 | T366W/L368A/Y40 7V | Y349C/E357C/K39 2D/D399K | E356K/K392D/D39 9K/K409D | K392L/T394W/F40 5A/Y407V | | |
| 0% Combination 517 | T366W/L368A/Y40 7V | Y349C/E357C/K39 2D/D399K | E356K/K392D/D39 9K/K409D | T350V/L351Y/T36 6L/K392L/T394W/ F405A/Y407V | | |
| 0% Combination 518 | T366W/L368A/Y40 7V | Y349C/E357C/K39 2D/D399K | E345R/K360E/D39 9M/Y407A/K409V | T394F/F405A | | |
| 0% Combination 519 | T366W/L368A/Y40 7V | Y349C/E357C/K39 2D/D399K | E345R/K360E/D39 9M/Y407A/K409V | K392L/T394W/F40 5A/Y407V | | |
| 0% Combination 520 | T366W/L368A/Y40 7V | Y349C/E357C/K39 2D/D399K | E345R/K360E/D39 9M/Y407A/K409V | T350V/L351Y/T36 6L/K392L/T394W/ F405A/Y407V | | |
| 0% Combination 521 | T366W/L368A/Y40 7V | E345R/Q347R/K36 0D/T366V/D399M/ Y407A/K409V | E356K/K392D/D39 9K/K439E | T394F/F405A | | |
| 0% Combination 522 | T366W/L368A/Y40 7V | E345R/Q347R/K36 0D/T366V/D399M/ Y407A/K409V | E356K/K392D/D39 9K/K439E | K392L/T394W/F40 5A/Y407V | | |
| 0% Combination 523 | T366W/L368A/Y40 7V | E345R/Q347R/K36 0D/T366V/D399M/ Y407A/K409V | E356K/K392D/D39 9K/K439E | T350V/L351Y/T36 6L/K392L/T394W/ F405A/Y407V | | |
| 0% Combination 524 | T366W/L368A/Y40 7V | E345R/Q347R/K36 0D/T366V/D399M/ Y407A/K409V | K392D/D399K/K40 9D | T394F/F405A | | |
| 0% Combination 525 | T366W/L368A/Y40 7V | E345R/Q347R/K36 0D/T366V/D399M/ Y407A/K409V | K392D/D399K/K40 9D | K392L/T394W/F40 5A/Y407V | | |
| 0% Combination 526 | T366W/L368A/Y40 7V | E345R/Q347R/K36 0D/T366V/D399M/ Y407A/K409V | K392D/D399K/K40 9D | T350V/L351Y/T36 6L/K392L/T394W/ F405A/Y407V | | |
| 0% Combination 527 | T366W/L368A/Y40 7V | E345R/Q347R/K36 0D/T366V/D399M/ Y407A/K409V | E356K/K392D/D39 9K/K409D | T394F/F405A | | |
| 0% Combination 528 | T366W/L368A/Y40 7V | E345R/Q347R/K36 0D/T366V/D399M/ Y407A/K409V | E356K/K392D/D39 9K/K409D | K392L/T394W/F40 5A/Y407V | | |
| 0% Combination 529 | T366W/L368A/Y40 7V | E345R/Q347R/K36 0D/T366V/D399M/ Y407A/K409V | E356K/K392D/D39 9K/K409D | T350V/L351Y/T36 6L/K392L/T394W/ F405A/Y407V | | |
| 0% Combination 530 | T366W/L368A/Y40 7V | E356K/K392D/D39 9K/K439E | E345R/K360E/D39 9M/Y407A/K409V | T394F/F405A | | |
| 0% Combination 531 | T366W/L368A/Y40 7V | E356K/K392D/D39 9K/K439E | E345R/K360E/D39 9M/Y407A/K409V | K392L/T394W/F40 5A/Y407V | | |
| 0% Combination 532 | T366W/L368A/Y40 7V | E356K/K392D/D39 9K/K439E | E345R/K360E/D39 9M/Y407A/K409V | T350V/L351Y/T36 6L/K392L/T394W/ F405A/Y407V | | |
| 0% Combination 533 | T366W/L368A/Y40 7V | E356K/K392D/D39 9K/K409D | E345R/K360E/D39 9M/Y407A/K409V | T394F/F405A | | |
| 0% Combination 534 | T366W/L368A/Y40 7V | E356K/K392D/D39 9K/K409D | E345R/K360E/D39 9M/Y407A/K409V | K392L/T394W/F40 5A/Y407V | | |
| 0% Combination 535 | T366W/L368A/Y40 7V | E356K/K392D/D39 9K/K409D | E345R/K360E/D39 9M/Y407A/K409V | T350V/L351Y/T36 6L/K392L/T394W/ F405A/Y407V | | |
| 0% Combination 536 | Q347R/K360E/D39 9V/F405T/K409W | E356K/D399K/K40 9E/K439E | Y349C/E357C/K39 2D/D399K | T394F/F405A | | |
| 0% Combination 537 | Q347R/K360E/D39 9V/F405T/K409W | E356K/D399K/K40 9E/K439E | Y349C/E357C/K39 2D/D399K | K392L/T394W/F40 5A/Y407V | | |
| 0% Combination 538 | Q347R/K360E/D39 9V/F405T/K409W | E356K/D399K/K40 9E/K439E | Y349C/E357C/K39 2D/D399K | T350V/L351Y/T36 6L/K392L/T394W/ F405A/Y407V | | |
| 0% Combination 539 | Q347R/K360E/D39 9V/F405T/K409W | E356K/D399K/K40 9E/K439E | Q347E/S354Y/T366 Y/Y407T | T394F/F405A | | |
| 0% Combination 540 | Q347R/K360E/D39 9V/F405T/K409W | E356K/D399K/K40 9E/K439E | Q347E/S354Y/T366 Y/Y407T | K392L/T394W/F40 5A/Y407V | | |
| 0% Combination 541 | Q347R/K360E/D39 9V/F405T/K409W | E356K/D399K/K40 9E/K439E | Q347E/S354Y/T366 Y/Y407T | T350V/L351Y/T36 6L/K392L/T394W/ F405A/Y407V | | |
| 0% Combination 542 | Q347R/K360E/D39 9V/F405T/K409W | Y349C/E357C/K39 2D/D399K | E356K/K392D/D39 9K/K439E | T394F/F405A | | |
| 0% Combination 543 | Q347R/K360E/D39 9V/F405T/K409W | Y349C/E357C/K39 2D/D399K | E356K/K392D/D39 9K/K439E | K392L/T394W/F40 5A/Y407V | | |
| 0% Combination 544 | Q347R/K360E/D39 9V/F405T/K409W | Y349C/E357C/K39 2D/D399K | E356K/K392D/D39 9K/K439E | T350V/L351Y/T36 6L/K392L/T394W/ F405A/Y407V | | |
| 0% Combination 545 | Q347R/K360E/D39 9V/F405T/K409W | Y349C/E357C/K39 2D/D399K | E356K/K392D/D39 9K/K409D | T394F/F405A | | |
| 0% Combination 546 | Q347R/K360E/D39 9V/F405T/K409W | Y349C/E357C/K39 2D/D399K | E356K/K392D/D39 9K/K409D | K392L/T394W/F40 5A/Y407V | | |
| 0% Combination 547 | Q347R/K360E/D39 9V/F405T/K409W | Y349C/E357C/K39 2D/D399K | E356K/K392D/D39 9K/K409D | T350V/L351Y/T36 6L/K392L/T394W/ F405A/Y407V | | |
| 0% Combination 548 | Q347R/K360E/D39 9V/F405T/K409W | Q347E/S354Y/T366 Y/Y407T | E356K/K392D/D39 9K/K439E | T394F/F405A | | |
| 0% Combination 549 | Q347R/K360E/D39 9V/F405T/K409W | Q347E/S354Y/T366 Y/Y407T | E356K/K392D/D39 9K/K439E | K392L/T394W/F40 5A/Y407V | | |
| 0% Combination 550 | Q347R/K360E/D39 9V/F405T/K409W | Q347E/S354Y/T366 Y/Y407T | E356K/K392D/D39 9K/K439E | T350V/L351Y/T36 6L/K392L/T394W/ F405A/Y407V | | |
| 0% Combination 551 | Q347R/K360E/D39 9V/F405T/K409W | Q347E/S354Y/T366 Y/Y407T | K392D/D399K/K40 9D | T394F/F405A | | |
| 0% Combination 552 | Q347R/K360E/D39 9V/F405T/K409W | Q347E/S354Y/T366 Y/Y407T | K392D/D399K/K40 9D | K392L/T394W/F40 5A/Y407V | | |
| 0% Combination 553 | Q347R/K360E/D39 9V/F405T/K409W | Q347E/S354Y/T366 Y/Y407T | K392D/D399K/K40 9D | T350V/L351Y/T36 6L/K392L/T394W/ F405A/Y407V | | |
| 0% Combination 554 | Q347R/K360E/D39 9V/F405T/K409W | Q347E/S354Y/T366 Y/Y407T | E356K/K392D/D39 9K/K409D | T394F/F405A | | |
| 0% Combination 555 | Q347R/K360E/D39 9V/F405T/K409W | Q347E/S354Y/T366 Y/Y407T | E356K/K392D/D39 9K/K409D | K392L/T394W/F40 5A/Y407V | | |
| 0% Combination 556 | Q347R/K360E/D39 9V/F405T/K409W | Q347E/S354Y/T366 Y/Y407T | E356K/K392D/D39 9K/K409D | T350V/L351Y/T36 6L/K392L/T394W/ F405A/Y407V | | |
| 0% Combination 557 | E356K/D399K/K40 9E/K439E | Y349C/E357C/K39 2D/D399K | E345R/K360E/D39 9M/Y407A/K409V | T394F/F405A | | |
| 0% Combination 558 | E356K/D399K/K40 9E/K439E | Y349C/E357C/K39 2D/D399K | E345R/K360E/D39 9M/Y407A/K409V | K392L/T394W/F40 5A/Y407V | | |
| 0% Combination 559 | E356K/D399K/K40 9E/K439E | Y349C/E357C/K39 2D/D399K | E345R/K360E/D39 9M/Y407A/K409V | T350V/L351Y/T36 6L/K392L/T394W/ F405A/Y407V | | |
| 0% Combination 560 | E356K/D399K/K40 9E/K439E | Q347E/S354Y/T366 Y/Y407T | E345R/K360E/D39 9M/Y407A/K409V | T394F/F405A | | |
| 0% Combination 561 | E356K/D399K/K40 9E/K439E | Q347E/S354Y/T366 Y/Y407T | E345R/K360E/D39 9M/Y407A/K409V | K392L/T394W/F40 5A/Y407V | | |
| 0% Combination 562 | E356K/D399K/K40 9E/K439E | Q347E/S354Y/T366 Y/Y407T | E345R/K360E/D39 9M/Y407A/K409V | T350V/L351Y/T36 6L/K392L/T394W/ F405A/Y407V | | |
| 0% Combination 563 | Y349C/E357C/K39 2D/D399K | E356K/K392D/D39 9K/K439E | E345R/K360E/D39 9M/Y407A/K409V | T394F/F405A | | |
| 0% Combination 564 | Y349C/E357C/K39 2D/D399K | E356K/K392D/D39 9K/K439E | E345R/K360E/D39 9M/Y407A/K409V | K392L/T394W/F40 5A/Y407V | | |
| 0% Combination 565 | Y349C/E357C/K39 2D/D399K | E356K/K392D/D39 9K/K439E | E345R/K360E/D39 9M/Y407A/K409V | T350V/L351Y/T36 6L/K392L/T394W/ F405A/Y407V | | |
| 0% Combination 566 | Y349C/E357C/K39 2D/D399K | E356K/K392D/D39 9K/K409D | E345R/K360E/D39 9M/Y407A/K409V | T394F/F405A | | |
| 0% Combination 567 | Y349C/E357C/K39 2D/D399K | E356K/K392D/D39 9K/K409D | E345R/K360E/D39 9M/Y407A/K409V | K392L/T394W/F40 5A/Y407V | | |
| 0% Combination 568 | Y349C/E357C/K39 2D/D399K | E356K/K392D/D39 9K/K409D | E345R/K360E/D39 9M/Y407A/K409V | T350V/L351Y/T36 6L/K392L/T394W/ F405A/Y407V | | |
| 0% Combination 569 | Q347E/S354Y/T366 Y/Y407T | E356K/K392D/D39 9K/K439E | E345R/K360E/D39 9M/Y407A/K409V | T394F/F405A | | |
| 0% Combination 570 | Q347E/S354Y/T366 Y/Y407T | E356K/K392D/D39 9K/K439E | E345R/K360E/D39 9M/Y407A/K409V | K392L/T394W/F40 5A/Y407V | | |
| 0% Combination 571 | Q347E/S354Y/T366 Y/Y407T | E356K/K392D/D39 9K/K439E | E345R/K360E/D39 9M/Y407A/K409V | T350V/L351Y/T36 6L/K392L/T394W/ F405A/Y407V | | |
| 0% Combination 572 | Q347E/S354Y/T366 Y/Y407T | E356K/K392D/D39 9K/K409D | E345R/K360E/D39 9M/Y407A/K409V | T394F/F405A | | |
| 0% Combination 573 | Q347E/S354Y/T366 Y/Y407T | E356K/K392D/D39 9K/K409D | E345R/K360E/D39 9M/Y407A/K409V | K392L/T394W/F40 5A/Y407V | | |
| 0% Combination 574 | Q347E/S354Y/T366 Y/Y407T | E356K/K392D/D39 9K/K409D | E345R/K360E/D39 9M/Y407A/K409V | T350V/L351Y/T36 6L/K392L/T394W/ F405A/Y407V | | |
| 0% Combination 575 | WT | T394W/F405A | T366W/L368A/Y40 7V | Q347R/K360E/D39 9V/F405T/K409W | E356K/D399K/K40 9E/K439E | |
| 0% Combination 576 | WT | T394W/F405A | T366W/L368A/Y40 7V | Q347R/K360E/D39 9V/F405T/K409W | Y349C/E357C/K39 2D/D399K | |
| 0% Combination 577 | WT | T394W/F405A | T366W/L368A/Y40 7V | Q347R/K360E/D39 9V/F405T/K409W | E356K/K392D/D39 9K/K439E | |
| 0% Combination 578 | WT | T394W/F405A | T366W/L368A/Y40 7V | Q347R/K360E/D39 9V/F405T/K409W | K392D/D399K/K40 9D | |
| 0% Combination 579 | WT | T394W/F405A | T366W/L368A/Y40 7V | Q347R/K360E/D39 9V/F405T/K409W | E356K/K392D/D39 9K/K409D | |
| 0% Combination 580 | WT | T394W/F405A | T366W/L368A/Y40 7V | E356K/D399K/K40 9E/K439E | Y349C/E357C/K39 2D/D399K | |
| 0% Combination 581 | WT | T394W/F405A | T366W/L368A/Y40 7V | E356K/D399K/K40 9E/K439E | E345R/Q347R/K36 0D/T366V/D399M/ Y407A/K409V | |
| 0% Combination 582 | WT | T394W/F405A | T366W/L368A/Y40 7V | E356K/D399K/K40 9E/K439E | E345R/K360E/D39 9M/Y407A/K409V | |
| 0% Combination 583 | WT | T394W/F405A | T366W/L368A/Y40 7V | Y349C/E357C/K39 2D/D399K | E356K/K392D/D39 9K/K439E | |
| 0% Combination 584 | WT | T394W/F405A | T366W/L368A/Y40 7V | Y349C/E357C/K39 2D/D399K | E356K/K392D/D39 9K/K409D | |
| 0% Combination 585 | WT | T394W/F405A | T366W/L368A/Y40 7V | Y349C/E357C/K39 2D/D399K | E345R/K360E/D39 9M/Y407A/K409V | |
| 0% Combination 586 | WT | T394W/F405A | T366W/L368A/Y40 7V | E345R/Q347R/K36 0D/T366V/D399M/ Y407A/K409V | E356K/K392D/D39 9K/K439E | |
| 0% Combination 587 | WT | T394W/F405A | T366W/L368A/Y40 7V | E345R/Q347R/K36 0D/T366V/D399M/ Y407A/K409V | K392D/D399K/K40 9D | |
| 0% Combination 588 | WT | T394W/F405A | T366W/L368A/Y40 7V | E345R/Q347R/K36 0D/T366V/D399M/ Y407A/K409V | E356K/K392D/D39 9K/K409D | |
| 0% Combination 589 | WT | T394W/F405A | T366W/L368A/Y40 7V | E356K/K392D/D39 9K/K439E | E345R/K360E/D39 9M/Y407A/K409V | |
| 0% Combination 590 | WT | T394W/F405A | T366W/L368A/Y40 7V | E356K/K392D/D39 9K/K409D | E345R/K360E/D39 9M/Y407A/K409V | |
| 0% Combination 591 | WT | T394W/F405A | Q347R/K360E/D39 9V/F405T/K409W | E356K/D399K/K40 9E/K439E | Y349C/E357C/K39 2D/D399K | |
| 0% Combination 592 | WT | T394W/F405A | Q347R/K360E/D39 9V/F405T/K409W | E356K/D399K/K40 9E/K439E | Q347E/S354Y/T366 Y/Y407T | |
| 0% Combination 593 | WT | T394W/F405A | Q347R/K360E/D39 9V/F405T/K409W | Y349C/E357C/K39 2D/D399K | E356K/K392D/D39 9K/K439E | |
| 0% Combination 594 | WT | T394W/F405A | Q347R/K360E/D39 9V/F405T/K409W | Y349C/E357C/K39 2D/D399K | E356K/K392D/D39 9K/K409D | |
| 0% Combination 595 | WT | T394W/F405A | Q347R/K360E/D39 9V/F405T/K409W | Q347E/S354Y/T366 Y/Y407T | E356K/K392D/D39 9K/K439E | |
| 0% Combination 596 | WT | T394W/F405A | Q347R/K360E/D39 9V/F405T/K409W | Q347E/S354Y/T366 Y/Y407T | K392D/D399K/K40 9D | |
| 0% Combination 597 | WT | T394W/F405A | Q347R/K360E/D39 9V/F405T/K409W | Q347E/S354Y/T366 Y/Y407T | E356K/K392D/D39 9K/K409D | |
| 0% Combination 598 | WT | T394W/F405A | E356K/D399K/K40 9E/K439E | Y349C/E357C/K39 2D/D399K | E345R/K360E/D39 9M/Y407A/K409V | |
| 0% Combination 599 | WT | T394W/F405A | E356K/D399K/K40 9E/K439E | Q347E/S354Y/T366 Y/Y407T | E345R/K360E/D39 9M/Y407A/K409V | |
| 0% Combination 600 | WT | T394W/F405A | Y349C/E357C/K39 2D/D399K | E356K/K392D/D39 9K/K439E | E345R/K360E/D39 9M/Y407A/K409V | |
| 0% Combination 601 | WT | T394W/F405A | Y349C/E357C/K39 2D/D399K | E356K/K392D/D39 9K/K409D | E345R/K360E/D39 9M/Y407A/K409V | |
| 0% Combination 602 | WT | T394W/F405A | Q347E/S354Y/T366 Y/Y407T | E356K/K392D/D39 9K/K439E | E345R/K360E/D39 9M/Y407A/K409V | |
| 0% Combination 603 | WT | T394W/F405A | Q347E/S354Y/T366 Y/Y407T | E356K/K392D/D39 9K/K409D | E345R/K360E/D39 9M/Y407A/K409V | |
| 0% Combination 604 | WT | T366W/L368A/Y40 7V | Q347R/K360E/D39 9V/F405T/K409W | E356K/D399K/K40 9E/K439E | Y349C/E357C/K39 2D/D399K | |
| 0% Combination 605 | WT | T366W/L368A/Y40 7V | Q347R/K360E/D39 9V/F405T/K409W | E356K/D399K/K40 9E/K439E | T394F/F405A | |
| 0% Combination 606 | WT | T366W/L368A/Y40 7V | Q347R/K360E/D39 9V/F405T/K409W | E356K/D399K/K40 9E/K439E | K392L/T394W/F40 5A/Y407V | |
| 0% Combination 607 | WT | T366W/L368A/Y40 7V | Q347R/K360E/D39 9V/F405T/K409W | E356K/D399K/K40 9E/K439E | T350V/L351Y/T36 6L/K392L/T394W/ F405A/Y407V | |
| 0% Combination 608 | WT | T366W/L368A/Y40 7V | Q347R/K360E/D39 9V/F405T/K409W | Y349C/E357C/K39 2D/D399K | E356K/K392D/D39 9K/K439E | |
| 0% Combination 609 | WT | T366W/L368A/Y40 7V | Q347R/K360E/D39 9V/F405T/K409W | Y349C/E357C/K39 2D/D399K | E356K/K392D/D39 9K/K409D | |
| 0% Combination 610 | WT | T366W/L368A/Y40 7V | Q347R/K360E/D39 9V/F405T/K409W | Y349C/E357C/K39 2D/D399K | T394F/F405A | |
| 0% Combination 611 | WT | T366W/L368A/Y40 7V | Q347R/K360E/D39 9V/F405T/K409W | Y349C/E357C/K39 2D/D399K | K392L/T394W/F40 5A/Y407V | |
| 0% Combination 612 | WT | T366W/L368A/Y40 7V | Q347R/K360E/D39 9V/F405T/K409W | Y349C/E357C/K39 2D/D399K | T350V/L351Y/T36 6L/K392L/T394W/ F405A/Y407V | |
| 0% Combination 613 | WT | T366W/L368A/Y40 7V | Q347R/K360E/D39 9V/F405T/K409W | E356K/K392D/D39 9K/K439E | T394F/F405A | |
| 0% Combination 614 | WT | T366W/L368A/Y40 7V | Q347R/K360E/D39 9V/F405T/K409W | E356K/K392D/D39 9K/K439E | K392L/T394W/F40 5A/Y407V | |
| 0% Combination 615 | WT | T366W/L368A/Y40 7V | Q347R/K360E/D39 9V/F405T/K409W | E356K/K392D/D39 9K/K439E | T350V/L351Y/T36 6L/K392L/T394W/ F405A/Y407V | |
| 0% Combination 616 | WT | T366W/L368A/Y40 7V | Q347R/K360E/D39 9V/F405T/K409W | K392D/D399K/K40 9D | T394F/F405A | |
| 0% Combination 617 | WT | T366W/L368A/Y40 7V | Q347R/K360E/D39 9V/F405T/K409W | K392D/D399K/K40 9D | K392L/T394W/F40 5A/Y407V | |
| 0% Combination 618 | WT | T366W/L368A/Y40 7V | Q347R/K360E/D39 9V/F405T/K409W | K392D/D399K/K40 9D | T350V/L351Y/T36 6L/K392L/T394W/ F405A/Y407V | |
| 0% Combination 619 | WT | T366W/L368A/Y40 7V | Q347R/K360E/D39 9V/F405T/K409W | E356K/K392D/D39 9K/K409D | T394F/F405A | |
| 0% Combination 620 | WT | T366W/L368A/Y40 7V | Q347R/K360E/D39 9V/F405T/K409W | E356K/K392D/D39 9K/K409D | K392L/T394W/F40 5A/Y407V | |
| 0% Combination 621 | WT | T366W/L368A/Y40 7V | Q347R/K360E/D39 9V/F405T/K409W | E356K/K392D/D39 9K/K409D | T350V/L351Y/T36 6L/K392L/T394W/ F405A/Y407V | |
| 0% Combination 622 | WT | T366W/L368A/Y40 7V | E356K/D399K/K40 9E/K439E | Y349C/E357C/K39 2D/D399K | E345R/K360E/D39 9M/Y407A/K409V | |
| 0% Combination 623 | WT | T366W/L368A/Y40 7V | E356K/D399K/K40 9E/K439E | Y349C/E357C/K39 2D/D399K | T394F/F405A | |
| 0% Combination 624 | WT | T366W/L368A/Y40 7V | E356K/D399K/K40 9E/K439E | Y349C/E357C/K39 2D/D399K | K392L/T394W/F40 5A/Y407V | |
| 0% Combination 625 | WT | T366W/L368A/Y40 7V | E356K/D399K/K40 9E/K439E | Y349C/E357C/K39 2D/D399K | T350V/L351Y/T36 6L/K392L/T394W/ F405A/Y407V | |
| 0% Combination 626 | WT | T366W/L368A/Y40 7V | E356K/D399K/K40 9E/K439E | E345R/Q347R/K36 0D/T366V/D399M/ Y407A/K409V | T394F/F405A | |
| 0% Combination 627 | WT | T366W/L368A/Y40 7V | E356K/D399K/K40 9E/K439E | E345R/Q347R/K36 0D/T366V/D399M/ Y407A/K409V | K392L/T394W/F40 5A/Y407V | |
| 0% Combination 628 | WT | T366W/L368A/Y40 7V | E356K/D399K/K40 9E/K439E | E345R/Q347R/K36 0D/T366V/D399M/ Y407A/K409V | T350V/L351Y/T36 6L/K392L/T394W/ F405A/Y407V | |
| 0% Combination 629 | WT | T366W/L368A/Y40 7V | E356K/D399K/K40 9E/K439E | E345R/K360E/D39 9M/Y407A/K409V | T394F/F405A | |
| 0% Combination 630 | WT | T366W/L368A/Y40 7V | E356K/D399K/K40 9E/K439E | E345R/K360E/D39 9M/Y407A/K409V | K392L/T394W/F40 5A/Y407V | |
| 0% Combination 631 | WT | T366W/L368A/Y40 7V | E356K/D399K/K40 9E/K439E | E345R/K360E/D39 9M/Y407A/K409V | T350V/L351Y/T36 6L/K392L/T394W/ F405A/Y407V | |
| 0% Combination 632 | WT | T366W/L368A/Y40 7V | Y349C/E357C/K39 2D/D399K | E356K/K392D/D39 9K/K439E | E345R/K360E/D39 9M/Y407A/K409V | |
| 0% Combination 633 | WT | T366W/L368A/Y40 7V | Y349C/E357C/K39 2D/D399K | E356K/K392D/D39 9K/K439E | T394F/F405A | |
| 0% Combination 634 | WT | T366W/L368A/Y40 7V | Y349C/E357C/K39 2D/D399K | E356K/K392D/D39 9K/K439E | K392L/T394W/F40 5A/Y407V | |
| 0% Combination 635 | WT | T366W/L368A/Y40 7V | Y349C/E357C/K39 2D/D399K | E356K/K392D/D39 9K/K439E | T350V/L351Y/T36 6L/K392L/T394W/ F405A/Y407V | |
| 0% Combination 636 | WT | T366W/L368A/Y40 7V | Y349C/E357C/K39 2D/D399K | E356K/K392D/D39 9K/K409D | E345R/K360E/D39 9M/Y407A/K409V | |
| 0% Combination 637 | WT | T366W/L368A/Y40 7V | Y349C/E357C/K39 2D/D399K | E356K/K392D/D39 9K/K409D | T394F/F405A | |
| 0% Combination 638 | WT | T366W/L368A/Y40 7V | Y349C/E357C/K39 2D/D399K | E356K/K392D/D39 9K/K409D | K392L/T394W/F40 5A/Y407V | |
| 0% Combination 639 | WT | T366W/L368A/Y40 7V | Y349C/E357C/K39 2D/D399K | E356K/K392D/D39 9K/K409D | T350V/L351Y/T36 6L/K392L/T394W/ F405A/Y407V | |
| 0% Combination 640 | WT | T366W/L368A/Y40 7V | Y349C/E357C/K39 2D/D399K | E345R/K360E/D39 9M/Y407A/K409V | T394F/F405A | |
| 0% Combination 641 | WT | T366W/L368A/Y40 7V | Y349C/E357C/K39 2D/D399K | E345R/K360E/D39 9M/Y407A/K409V | K392L/T394W/F40 5A/Y407V | |
| 0% Combination 642 | WT | T366W/L368A/Y40 7V | Y349C/E357C/K39 2D/D399K | E345R/K360E/D39 9M/Y407A/K409V | T350V/L351Y/T36 6L/K392L/T394W/ F405A/Y407V | |
| 0% Combination 643 | WT | T366W/L368A/Y40 7V | E345R/Q347R/K36 0D/T366V/D399M/ Y407A/K409V | E356K/K392D/D39 9K/K439E | T394F/F405A | |
| 0% Combination 644 | WT | T366W/L368A/Y40 7V | E345R/Q347R/K36 0D/T366V/D399M/ Y407A/K409V | E356K/K392D/D39 9K/K439E | K392L/T394W/F40 5A/Y407V | |
| 0% Combination 645 | WT | T366W/L368A/Y40 7V | E345R/Q347R/K36 0D/T366V/D399M/ Y407A/K409V | E356K/K392D/D39 9K/K439E | T350V/L351Y/T36 6L/K392L/T394W/ F405A/Y407V | |
| 0% Combination 646 | WT | T366W/L368A/Y40 7V | E345R/Q347R/K36 0D/T366V/D399M/ Y407A/K409V | K392D/D399K/K40 9D | T394F/F405A | |
| 0% Combination 647 | WT | T366W/L368A/Y40 7V | E345R/Q347R/K36 0D/T366V/D399M/ Y407A/K409V | K392D/D399K/K40 9D | K392L/T394W/F40 5A/Y407V | |
| 0% Combination 648 | WT | T366W/L368A/Y40 7V | E345R/Q347R/K36 0D/T366V/D399M/ Y407A/K409V | K392D/D399K/K40 9D | T350V/L351Y/T36 6L/K392L/T394W/ F405A/Y407V | |
| 0% Combination 649 | WT | T366W/L368A/Y40 7V | E345R/Q347R/K36 0D/T366V/D399M/ Y407A/K409V | E356K/K392D/D39 9K/K409D | T394F/F405A | |
| 0% Combination 650 | WT | T366W/L368A/Y40 7V | E345R/Q347R/K36 0D/T366V/D399M/ Y407A/K409V | E356K/K392D/D39 9K/K409D | K392L/T394W/F40 5A/Y407V | |
| 0% Combination 651 | WT | T366W/L368A/Y40 7V | E345R/Q347R/K36 0D/T366V/D399M/ Y407A/K409V | E356K/K392D/D39 9K/K409D | T350V/L351Y/T36 6L/K392L/T394W/ F405A/Y407V | |
| 0% Combination 652 | WT | T366W/L368A/Y40 7V | E356K/K392D/D39 9K/K439E | E345R/K360E/D39 9M/Y407A/K409V | T394F/F405A | |
| 0% Combination 653 | WT | T366W/L368A/Y40 7V | E356K/K392D/D39 9K/K439E | E345R/K360E/D39 9M/Y407A/K409V | K392L/T394W/F40 5A/Y407V | |
| 0% Combination 654 | WT | T366W/L368A/Y40 7V | E356K/K392D/D39 9K/K439E | E345R/K360E/D39 9M/Y407A/K409V | T350V/L351Y/T36 6L/K392L/T394W/ F405A/Y407V | |
| 0% Combination 655 | WT | T366W/L368A/Y40 7V | E356K/K392D/D39 9K/K409D | E345R/K360E/D39 9M/Y407A/K409V | T394F/F405A | |
| 0% Combination 656 | WT | T366W/L368A/Y40 7V | E356K/K392D/D39 9K/K409D | E345R/K360E/D39 9M/Y407A/K409V | K392L/T394W/F40 5A/Y407V | |
| 0% Combination 657 | WT | T366W/L368A/Y40 7V | E356K/K392D/D39 9K/K409D | E345R/K360E/D39 9M/Y407A/K409V | T350V/L351Y/T36 6L/K392L/T394W/ F405A/Y407V | |
| 0% Combination 658 | WT | Q347R/K360E/D39 9V/F405T/K409W | E356K/D399K/K40 9E/K439E | Y349C/E357C/K39 2D/D399K | T394F/F405A | |
| 0% Combination 659 | WT | Q347R/K360E/D39 9V/F405T/K409W | E356K/D399K/K40 9E/K439E | Y349C/E357C/K39 2D/D399K | K392L/T394W/F40 5A/Y407V | |
| 0% Combination 660 | WT | Q347R/K360E/D39 9V/F405T/K409W | E356K/D399K/K40 9E/K439E | Y349C/E357C/K39 2D/D399K | T350V/L351Y/T36 6L/K392L/T394W/ F405A/Y407V | |
| 0% Combination 661 | WT | Q347R/K360E/D39 9V/F405T/K409W | E356K/D399K/K40 9E/K439E | Q347E/S354Y/T366 Y/Y407T | T394F/F405A | |
| 0% Combination 662 | WT | Q347R/K360E/D39 9V/F405T/K409W | E356K/D399K/K40 9E/K439E | Q347E/S354Y/T366 Y/Y407T | K392L/T394W/F40 5A/Y407V | |
| 0% Combination 663 | WT | Q347R/K360E/D39 9V/F405T/K409W | E356K/D399K/K40 9E/K439E | Q347E/S354Y/T366 Y/Y407T | T350V/L351Y/T36 6L/K392L/T394W/ F405A/Y407V | |
| 0% Combination 664 | WT | Q347R/K360E/D39 9V/F405T/K409W | Y349C/E357C/K39 2D/D399K | E356K/K392D/D39 9K/K439E | T394F/F405A | |
| 0% Combination 665 | WT | Q347R/K360E/D39 9V/F405T/K409W | Y349C/E357C/K39 2D/D399K | E356K/K392D/D39 9K/K439E | K392L/T394W/F40 5A/Y407V | |
| 0% Combination 666 | WT | Q347R/K360E/D39 9V/F405T/K409W | Y349C/E357C/K39 2D/D399K | E356K/K392D/D39 9K/K439E | T350V/L351Y/T36 6L/K392L/T394W/ F405A/Y407V | |
| 0% Combination 667 | WT | Q347R/K360E/D39 9V/F405T/K409W | Y349C/E357C/K39 2D/D399K | E356K/K392D/D39 9K/K409D | T394F/F405A | |
| 0% Combination 668 | WT | Q347R/K360E/D39 9V/F405T/K409W | Y349C/E357C/K39 2D/D399K | E356K/K392D/D39 9K/K409D | K392L/T394W/F40 5A/Y407V | |
| 0% Combination 669 | WT | Q347R/K360E/D39 9V/F405T/K409W | Y349C/E357C/K39 2D/D399K | E356K/K392D/D39 9K/K409D | T350V/L351Y/T36 6L/K392L/T394W/ F405A/Y407V | |
| 0% Combination 670 | WT | Q347R/K360E/D39 9V/F405T/K409W | Q347E/S354Y/T366 Y/Y407T | E356K/K392D/D39 9K/K439E | T394F/F405A | |
| 0% Combination 671 | WT | Q347R/K360E/D39 9V/F405T/K409W | Q347E/S354Y/T366 Y/Y407T | E356K/K392D/D39 9K/K439E | K392L/T394W/F40 5A/Y407V | |
| 0% Combination 672 | WT | Q347R/K360E/D39 9V/F405T/K409W | Q347E/S354Y/T366 Y/Y407T | E356K/K392D/D39 9K/K439E | T350V/L351Y/T36 6L/K392L/T394W/ F405A/Y407V | |
| 0% Combination 673 | WT | Q347R/K360E/D39 9V/F405T/K409W | Q347E/S354Y/T366 Y/Y407T | K392D/D399K/K40 9D | T394F/F405A | |
| 0% Combination 674 | WT | Q347R/K360E/D39 9V/F405T/K409W | Q347E/S354Y/T366 Y/Y407T | K392D/D399K/K40 9D | K392L/T394W/F40 5A/Y407V | |
| 0% Combination 675 | WT | Q347R/K360E/D39 9V/F405T/K409W | Q347E/S354Y/T366 Y/Y407T | K392D/D399K/K40 9D | T350V/L351Y/T36 6L/K392L/T394W/ F405A/Y407V | |
| 0% Combination 676 | WT | Q347R/K360E/D39 9V/F405T/K409W | Q347E/S354Y/T366 Y/Y407T | E356K/K392D/D39 9K/K409D | T394F/F405A | |
| 0% Combination 677 | WT | Q347R/K360E/D39 9V/F405T/K409W | Q347E/S354Y/T366 Y/Y407T | E356K/K392D/D39 9K/K409D | K392L/T394W/F40 5A/Y407V | |
| 0% Combination 678 | WT | Q347R/K360E/D39 9V/F405T/K409W | Q347E/S354Y/T366 Y/Y407T | E356K/K392D/D39 9K/K409D | T350V/L351Y/T36 6L/K392L/T394W/ F405A/Y407V | |
| 0% Combination 679 | WT | E356K/D399K/K40 9E/K439E | Y349C/E357C/K39 2D/D399K | E345R/K360E/D39 9M/Y407A/K409V | T394F/F405A | |
| 0% Combination 680 | WT | E356K/D399K/K40 9E/K439E | Y349C/E357C/K39 2D/D399K | E345R/K360E/D39 9M/Y407A/K409V | K392L/T394W/F40 5A/Y407V | |
| 0% Combination 681 | WT | E356K/D399K/K40 9E/K439E | Y349C/E357C/K39 2D/D399K | E345R/K360E/D39 9M/Y407A/K409V | T350V/L351Y/T36 6L/K392L/T394W/ F405A/Y407V | |
| 0% Combination 682 | WT | E356K/D399K/K40 9E/K439E | Q347E/S354Y/T366 Y/Y407T | E345R/K360E/D39 9M/Y407A/K409V | T394F/F405A | |
| 0% Combination 683 | WT | E356K/D399K/K40 9E/K439E | Q347E/S354Y/T366 Y/Y407T | E345R/K360E/D39 9M/Y407A/K409V | K392L/T394W/F40 5A/Y407V | |
| 0% Combination 684 | WT | E356K/D399K/K40 9E/K439E | Q347E/S354Y/T366 Y/Y407T | E345R/K360E/D39 9M/Y407A/K409V | T350V/L351Y/T36 6L/K392L/T394W/ F405A/Y407V | |
| 0% Combination 685 | WT | Y349C/E357C/K39 2D/D399K | E356K/K392D/D39 9K/K439E | E345R/K360E/D39 9M/Y407A/K409V | T394F/F405A | |
| 0% Combination 686 | WT | Y349C/E357C/K39 2D/D399K | E356K/K392D/D39 9K/K439E | E345R/K360E/D39 9M/Y407A/K409V | K392L/T394W/F40 5A/Y407V | |
| 0% Combination 687 | WT | Y349C/E357C/K39 2D/D399K | E356K/K392D/D39 9K/K439E | E345R/K360E/D39 9M/Y407A/K409V | T350V/L351Y/T36 6L/K392L/T394W/ F405A/Y407V | |
| 0% Combination 688 | WT | Y349C/E357C/K39 2D/D399K | E356K/K392D/D39 9K/K409D | E345R/K360E/D39 9M/Y407A/K409V | T394F/F405A | |
| 0% Combination 689 | WT | Y349C/E357C/K39 2D/D399K | E356K/K392D/D39 9K/K409D | E345R/K360E/D39 9M/Y407A/K409V | K392L/T394W/F40 5A/Y407V | |
| 0% Combination 690 | WT | Y349C/E357C/K39 2D/D399K | E356K/K392D/D39 9K/K409D | E345R/K360E/D39 9M/Y407A/K409V | T350V/L351Y/T36 6L/K392L/T394W/ F405A/Y407V | |
| 0% Combination 691 | WT | Q347E/S354Y/T366 Y/Y407T | E356K/K392D/D39 9K/K439E | E345R/K360E/D39 9M/Y407A/K409V | T394F/F405A | |
| 0% Combination 692 | WT | Q347E/S354Y/T366 Y/Y407T | E356K/K392D/D39 9K/K439E | E345R/K360E/D39 9M/Y407A/K409V | K392L/T394W/F40 5A/Y407V | |
| 0% Combination 693 | WT | Q347E/S354Y/T366 Y/Y407T | E356K/K392D/D39 9K/K439E | E345R/K360E/D39 9M/Y407A/K409V | T350V/L351Y/T36 6L/K392L/T394W/ F405A/Y407V | |
| 0% Combination 694 | WT | Q347E/S354Y/T366 Y/Y407T | E356K/K392D/D39 9K/K409D | E345R/K360E/D39 9M/Y407A/K409V | T394F/F405A | |
| 0% Combination 695 | WT | Q347E/S354Y/T366 Y/Y407T | E356K/K392D/D39 9K/K409D | E345R/K360E/D39 9M/Y407A/K409V | K392L/T394W/F40 5A/Y407V | |
| 0% Combination 696 | WT | Q347E/S354Y/T366 Y/Y407T | E356K/K392D/D39 9K/K409D | E345R/K360E/D39 9M/Y407A/K409V | T350V/L351Y/T36 6L/K392L/T394W/ F405A/Y407V | |
| 0% Combination 697 | T394W/F405A | T366W/L368A/Y40 7V | Q347R/K360E/D39 9V/F405T/K409W | E356K/D399K/K40 9E/K439E | Y349C/E357C/K39 2D/D399K | |
| 0% Combination 698 | T394W/F405A | T366W/L368A/Y40 7V | Q347R/K360E/D39 9V/F405T/K409W | Y349C/E357C/K39 2D/D399K | E356K/K392D/D39 9K/K439E | |
| 0% Combination 699 | T394W/F405A | T366W/L368A/Y40 7V | Q347R/K360E/D39 9V/F405T/K409W | Y349C/E357C/K39 2D/D399K | E356K/K392D/D39 9K/K409D | |
| 0% Combination 700 | T394W/F405A | T366W/L368A/Y40 7V | E356K/D399K/K40 9E/K439E | Y349C/E357C/K39 2D/D399K | E345R/K360E/D39 9M/Y407A/K409V | |
| 0% Combination 701 | T394W/F405A | T366W/L368A/Y40 7V | Y349C/E357C/K39 2D/D399K | E356K/K392D/D39 9K/K439E | E345R/K360E/D39 9M/Y407A/K409V | |
| 0% Combination 702 | T394W/F405A | T366W/L368A/Y40 7V | Y349C/E357C/K39 2D/D399K | E356K/K392D/D39 9K/K409D | E345R/K360E/D39 9M/Y407A/K409V | |
| 0% Combination 703 | T366W/L368A/Y40 7V | Q347R/K360E/D39 9V/F405T/K409W | E356K/D399K/K40 9E/K439E | Y349C/E357C/K39 2D/D399K | T394F/F405A | |
| 0% Combination 704 | T366W/L368A/Y40 7V | Q347R/K360E/D39 9V/F405T/K409W | E356K/D399K/K40 9E/K439E | Y349C/E357C/K39 2D/D399K | K392L/T394W/F40 5A/Y407V | |
| 0% Combination 705 | T366W/L368A/Y40 7V | Q347R/K360E/D39 9V/F405T/K409W | E356K/D399K/K40 9E/K439E | Y349C/E357C/K39 2D/D399K | T350V/L351Y/T36 6L/K392L/T394W/ F405A/Y407V | |
| 0% Combination 706 | T366W/L368A/Y40 7V | Q347R/K360E/D39 9V/F405T/K409W | Y349C/E357C/K39 2D/D399K | E356K/K392D/D39 9K/K439E | T394F/F405A | |
| 0% Combination 707 | T366W/L368A/Y40 7V | Q347R/K360E/D39 9V/F405T/K409W | Y349C/E357C/K39 2D/D399K | E356K/K392D/D39 9K/K439E | K392L/T394W/F40 5A/Y407V | |
| 0% Combination 708 | T366W/L368A/Y40 7V | Q347R/K360E/D39 9V/F405T/K409W | Y349C/E357C/K39 2D/D399K | E356K/K392D/D39 9K/K439E | T350V/L351Y/T36 6L/K392L/T394W/ F405A/Y407V | |
| 0% Combination 709 | T366W/L368A/Y40 7V | Q347R/K360E/D39 9V/F405T/K409W | Y349C/E357C/K39 2D/D399K | E356K/K392D/D39 9K/K409D | T394F/F405A | |
| 0% Combination 710 | T366W/L368A/Y40 7V | Q347R/K360E/D39 9V/F405T/K409W | Y349C/E357C/K39 2D/D399K | E356K/K392D/D39 9K/K409D | K392L/T394W/F40 5A/Y407V | |
| 0% Combination 711 | T366W/L368A/Y40 7V | Q347R/K360E/D39 9V/F405T/K409W | Y349C/E357C/K39 2D/D399K | E356K/K392D/D39 9K/K409D | T350V/L351Y/T36 6L/K392L/T394W/ F405A/Y407V | |
| 0% Combination 712 | T366W/L368A/Y40 7V | E356K/D399K/K40 9E/K439E | Y349C/E357C/K39 2D/D399K | E345R/K360E/D39 9M/Y407A/K409V | T394F/F405A | |
| 0% Combination 713 | T366W/L368A/Y40 7V | E356K/D399K/K40 9E/K439E | Y349C/E357C/K39 2D/D399K | E345R/K360E/D39 9M/Y407A/K409V | K392L/T394W/F40 5A/Y407V | |
| 0% Combination 714 | T366W/L368A/Y40 7V | E356K/D399K/K40 9E/K439E | Y349C/E357C/K39 2D/D399K | E345R/K360E/D39 9M/Y407A/K409V | T350V/L351Y/T36 6L/K392L/T394W/ F405A/Y407V | |
| 0% Combination 715 | T366W/L368A/Y40 7V | Y349C/E357C/K39 2D/D399K | E356K/K392D/D39 9K/K439E | E345R/K360E/D39 9M/Y407A/K409V | T394F/F405A | |
| 0% Combination 716 | T366W/L368A/Y40 7V | Y349C/E357C/K39 2D/D399K | E356K/K392D/D39 9K/K439E | E345R/K360E/D39 9M/Y407A/K409V | K392L/T394W/F40 5A/Y407V | |
| 0% Combination 717 | T366W/L368A/Y40 7V | Y349C/E357C/K39 2D/D399K | E356K/K392D/D39 9K/K439E | E345R/K360E/D39 9M/Y407A/K409V | T350V/L351Y/T36 6L/K392L/T394W/ F405A/Y407V | |
| 0% Combination 718 | T366W/L368A/Y40 7V | Y349C/E357C/K39 2D/D399K | E356K/K392D/D39 9K/K409D | E345R/K360E/D39 9M/Y407A/K409V | T394F/F405A | |
| 0% Combination 719 | T366W/L368A/Y40 7V | Y349C/E357C/K39 2D/D399K | E356K/K392D/D39 9K/K409D | E345R/K360E/D39 9M/Y407A/K409V | K392L/T394W/F40 5A/Y407V | |
| 0% Combination 720 | T366W/L368A/Y40 7V | Y349C/E357C/K39 2D/D399K | E356K/K392D/D39 9K/K409D | E345R/K360E/D39 9M/Y407A/K409V | T350V/L351Y/T36 6L/K392L/T394W/ F405A/Y407V | |
| 0% Combination 721 | WT | T394W/F405A | T366W/L368A/Y40 7V | Q347R/K360E/D39 9V/F405T/K409W | E356K/D399K/K40 9E/K439E | Y349C/E357C/K39 2D/D399K |
| 0% Combination 722 | WT | T394W/F405A | T366W/L368A/Y40 7V | Q347R/K360E/D39 9V/F405T/K409W | Y349C/E357C/K39 2D/D399K | E356K/K392D/D39 9K/K439E |
| 0% Combination 723 | WT | T394W/F405A | T366W/L368A/Y40 7V | Q347R/K360E/D39 9V/F405T/K409W | Y349C/E357C/K39 2D/D399K | E356K/K392D/D39 9K/K409D |
| 0% Combination 724 | WT | T394W/F405A | T366W/L368A/Y40 7V | E356K/D399K/K40 9E/K439E | Y349C/E357C/K39 2D/D399K | E345R/K360E/D39 9M/Y407A/K409V |
| 0% Combination 725 | WT | T394W/F405A | T366W/L368A/Y40 7V | Y349C/E357C/K39 2D/D399K | E356K/K392D/D39 9K/K439E | E345R/K360E/D39 9M/Y407A/K409V |
| 0% Combination 726 | WT | T394W/F405A | T366W/L368A/Y40 7V | Y349C/E357C/K39 2D/D399K | E356K/K392D/D39 9K/K409D | E345R/K360E/D39 9M/Y407A/K409V |
| 0% Combination 727 | WT | T366W/L368A/Y40 7V | Q347R/K360E/D39 9V/F405T/K409W | E356K/D399K/K40 9E/K439E | Y349C/E357C/K39 2D/D399K | T394F/F405A |
| 0% Combination 728 | WT | T366W/L368A/Y40 7V | Q347R/K360E/D39 9V/F405T/K409W | E356K/D399K/K40 9E/K439E | Y349C/E357C/K39 2D/D399K | K392L/T394W/F40 5A/Y407V |
| 0% Combination 729 | WT | T366W/L368A/Y40 7V | Q347R/K360E/D39 9V/F405T/K409W | E356K/D399K/K40 9E/K439E | Y349C/E357C/K39 2D/D399K | T350V/L351Y/T36 6L/K392L/T394W/ F405A/Y407V |
| 0% Combination 730 | WT | T366W/L368A/Y40 7V | Q347R/K360E/D39 9V/F405T/K409W | Y349C/E357C/K39 2D/D399K | E356K/K392D/D39 9K/K439E | T394F/F405A |
| 0% Combination 731 | WT | T366W/L368A/Y40 7V | Q347R/K360E/D39 9V/F405T/K409W | Y349C/E357C/K39 2D/D399K | E356K/K392D/D39 9K/K439E | K392L/T394W/F40 5A/Y407V |
| 0% Combination 732 | WT | T366W/L368A/Y40 7V | Q347R/K360E/D39 9V/F405T/K409W | Y349C/E357C/K39 2D/D399K | E356K/K392D/D39 9K/K439E | T350V/L351Y/T36 6L/K392L/T394W/ F405A/Y407V |
| 0% Combination 733 | WT | T366W/L368A/Y40 7V | Q347R/K360E/D39 9V/F405T/K409W | Y349C/E357C/K39 2D/D399K | E356K/K392D/D39 9K/K409D | T394F/F405A |
| 0% Combination 734 | WT | T366W/L368A/Y40 7V | Q347R/K360E/D39 9V/F405T/K409W | Y349C/E357C/K39 2D/D399K | E356K/K392D/D39 9K/K409D | K392L/T394W/F40 5A/Y407V |
| 0% Combination 735 | WT | T366W/L368A/Y40 7V | Q347R/K360E/D39 9V/F405T/K409W | Y349C/E357C/K39 2D/D399K | E356K/K392D/D39 9K/K409D | T350V/L351Y/T36 6L/K392L/T394W/ F405A/Y407V |
| 0% Combination 736 | WT | T366W/L368A/Y40 7V | E356K/D399K/K40 9E/K439E | Y349C/E357C/K39 2D/D399K | E345R/K360E/D39 9M/Y407A/K409V | T394F/F405A |
| 0% Combination 737 | WT | T366W/L368A/Y40 7V | E356K/D399K/K40 9E/K439E | Y349C/E357C/K39 2D/D399K | E345R/K360E/D39 9M/Y407A/K409V | K392L/T394W/F40 5A/Y407V |
| 0% Combination 738 | WT | T366W/L368A/Y40 7V | E356K/D399K/K40 9E/K439E | Y349C/E357C/K39 2D/D399K | E345R/K360E/D39 9M/Y407A/K409V | T350V/L351Y/T36 6L/K392L/T394W/ F405A/Y407V |
| 0% Combination 739 | WT | T366W/L368A/Y40 7V | Y349C/E357C/K39 2D/D399K | E356K/K392D/D39 9K/K439E | E345R/K360E/D39 9M/Y407A/K409V | T394F/F405A |
| 0% Combination 740 | WT | T366W/L368A/Y40 7V | Y349C/E357C/K39 2D/D399K | E356K/K392D/D39 9K/K439E | E345R/K360E/D39 9M/Y407A/K409V | K392L/T394W/F40 5A/Y407V |
| 0% Combination 741 | WT | T366W/L368A/Y40 7V | Y349C/E357C/K39 2D/D399K | E356K/K392D/D39 9K/K439E | E345R/K360E/D39 9M/Y407A/K409V | T350V/L351Y/T36 6L/K392L/T394W/ F405A/Y407V |
| 0% Combination 742 | WT | T366W/L368A/Y40 7V | Y349C/E357C/K39 2D/D399K | E356K/K392D/D39 9K/K409D | E345R/K360E/D39 9M/Y407A/K409V | T394F/F405A |
| 0% Combination 743 | WT | T366W/L368A/Y40 7V | Y349C/E357C/K39 2D/D399K | E356K/K392D/D39 9K/K409D | E345R/K360E/D39 9M/Y407A/K409V | K392L/T394W/F40 5A/Y407V |
| 0% Combination 744 | WT | T366W/L368A/Y40 7V | Y349C/E357C/K39 2D/D399K | E356K/K392D/D39 9K/K409D | E345R/K360E/D39 9M/Y407A/K409V | T350V/L351Y/T36 6L/K392L/T394W/ F405A/Y407V |

### [Example 6] Confirmation of coexpression of multiple antibodies by ion exchange chromatography (IEC)

Example 5 identified pairs that allow coexpression of two or more antibodies. In order to confirm whether they can actually achieve promotion of homodimer formation between multiple IgG antibodies, Expi293F was allowed to express multiple modified IgG antibodies. For expression, a plasmid encoding the heavy chain and a plasmid encoding the light chain were used at a mass ratio of 1:1 for transfection. When multiple heavy chains were used for expression, they were used such that the mass ratio of each heavy chain was equal. Each antibody after Protein A purification was analyzed by ion exchange chromatography (IEC). Specifically, the produced antibody was analyzed by cation exchange chromatography (CIEX) using Alliance system (Waters). A two-solvent gradient method was performed using YMC-BioPro SP-F 5 µm 4.6x100 (YMC) as an analysis column, CX-1 pH Gradient Buffer A, pH 5.6 (Thermo) as mobile phase A, and CX-1 pH Gradient Buffer B, pH 10.2 (Thermo) as mobile phase B. Samples were detected by absorption at a wavelength of 280 nm (denoted as AU on the vertical axes in figures). Data was analyzed using Empower3 (Waters) to calculate the ratio of each detected peak.

The modifications used in antibody expression and the corresponding SEQ ID NOs are shown in Table 8 (the non-introduction of modifications is denoted as "-"). In expression, the heavy chains of four different antibodies with different isoelectric points (pI) were used and, to simplify analysis, a common light chain (SEQ ID NO: 61) was used. The antibodies prior to modification and the individual modified antibodies were also analyzed for assignment of the peaks detected when the multiple heavy chains were expressed. Analysis results are shown in Figs. 3-1 to 3-47 along with the assignment and area ratio of each peak. In these figures, peak assignments are indicated using abbreviations that feature heavy chain association. The abbreviations and the correspondences between the template heavy chain sequences and the modifications are shown in Table 9 (the non-introduction of modifications is denoted as "-"). For example, the antibody with homodimerized identical heavy chains of SEQ ID NO: 1 and the common light chain is denoted as "aa". All combinations implemented were found to have a strong heavy-chain homomeric association-promoting ability. Similarly, the combinations shown in Tables 6 and 7 are also considered to exhibit a heavy-chain homomeric association ability.

**(Table 8) Correspondences between SEQ ID NOs and amino acid modifications in the samples used for confirming controlled heavy-chain association of multiple antibodies**

| Sample No. | Heavy chain 1: SEQ ID NO | Modifications that promote formation of heavy chain homodimers | Heavy chain 2: SEQ ID NO | Modifications that promote formation of heavy chain homodimers | Heavy chain 3: SEQ ID NO | Modifications that promote formation of heavy chain homodimers | Heavy chain 4: SEQ ID NO | Modifications that promote formation of heavy chain homodimers | Light chain: SEQ ID NO |
|---|---|---|---|---|---|---|---|---|---|
| 122 | 1 | - | | | | | | | 61 |
| 123 | 78 | - | | | | | | | 61 |
| 124 | 79 | - | | | | | | | 61 |
| 125 | 80 | - | | | | | | | 61 |
| 126 | 1 | - | 78 | - | | | | | 61 |
| 127 | 1 | - | 79 | - | | | | | 61 |
| 128 | 1 | - | 80 | - | | | | | 61 |
| 129 | 78 | - | 79 | - | | | | | 61 |
| 130 | 78 | - | 80 | - | | | | | 61 |
| 131 | 79 | - | 80 | - | | | | | 61 |
| 132 | 1 | - | 78 | - | 79 | - | | | 61 |
| 133 | 78 | - | 79 | - | 80 | - | | | 61 |
| 134 | 79 | - | 80 | - | 1 | - | | | 61 |
| 135 | 1 | - | 78 | - | 79 | - | 80 | - | 61 |
| 136 | 19 | T394W/F405A | | | | | | | 61 |
| 137 | 81 | T366W/L368A/Y4 07V | | | | | | | 61 |
| 138 | 82 | Q347R/K360E/D3 99V/F405T/K409 W | | | | | | | 61 |
| 139 | 83 | E356K/D399K/K4 09E/K439E | | | | | | | 61 |
| 140 | 19 | T394W/F405A | 81 | T366W/L368A/Y4 07V | | | | | 61 |
| 141 | 19 | T394W/F405A | 82 | Q347R/K360E/D39 9V/F405T/K409W | | | | | 61 |
| 142 | 19 | T394W/F405A | 83 | E356K/D399K/K40 9E/K439E | | | | | 61 |
| 143 | 81 | T366W/L368A/Y4 07V | 82 | Q347R/K360E/D39 9V/F405T/K409W | | | | | 61 |
| 144 | 81 | T366W/L368A/Y4 07V | 83 | E356K/D399K/K40 9E/K439E | | | | | 61 |
| 145 | 82 | Q347R/K360E/D3 99V/F405T/K409 W | 83 | E356K/D399K/K40 9E/K439E | | | | | 61 |
| 146 | 19 | T394W/F405A | 81 | T366W/L368A/Y4 07V | 82 | Q347R/K360E/D399 V/F405T/K409W | | | 61 |
| 147 | 81 | T366W/L368A/Y4 07V | 82 | Q347R/K360E/D39 9V/F405T/K409W | 83 | E356K/D399K/K40 9E/K439E | | | 61 |
| 148 | 82 | Q347R/K360E/D3 99V/F405T/K409 W | 83 | E356K/D399K/K40 9E/K439E | 19 | T394W/F405A | | | 61 |
| 149 | 19 | T394W/F405A | 81 | T366W/L368A/Y4 07V | 82 | Q347R/K360E/D399 V/F405T/K409W | 83 | E356K/D399K/K 409E/K439E | 61 |
| 150 | 84 | E356K/K392D/D3 99K/K439E | | | | | | | 61 |
| 151 | 19 | T394W/F405A | 84 | E356K/K392D/D39 9K/K439E | | | | | 61 |
| 152 | 81 | T366W/L368A/Y4 07V | 84 | E356K/K392D/D39 9K/K439E | | | | | 61 |
| 153 | 82 | Q347R/K360E/D3 99V/F405T/K409 W | 84 | E356K/K392D/D39 9K/K439E | | | | | 61 |
| 154 | 81 | T366W/L368A/Y4 07V | 82 | Q347R/K360E/D39 9V/F405T/K409W | 84 | E356K/K392D/D39 9K/K439E | | | 61 |
| 155 | 82 | Q347R/K360E/D3 99V/F405T/K409 W | 84 | E356K/K392D/D39 9K/K439E | 19 | T394W/F405A | | | 61 |
| 156 | 19 | T394W/F405A | 81 | T366W/L368A/Y4 07V | 82 | Q347R/K360E/D399 V/F405T/K409W | 84 | E356K/K392D/D 399K/K439E | 61 |
| 157 | 85 | E345R/K360E/D39 9M/Y407A/K409V | | | | | | | 61 |
| 158 | 19 | T394W/F405A | 85 | E345R/K360E/D39 9M/Y407A/K409V | | | | | 61 |
| 159 | 81 | T366W/L368A/Y4 07V | 85 | E345R/K360E/D39 9M/Y407A/K409V | | | | | 61 |
| 160 | 85 | E345R/K360E/D39 9M/Y407A/K409V | 83 | E356K/D399K/K40 9E/K439E | | | | | 61 |
| 161 | 19 | T394W/F405A | 81 | T366W/L368A/Y4 07V | 85 | E345R/K360E/D399 M/Y407A/K409V | | | 61 |
| 162 | 81 | T366W/L368A/Y4 07V | 85 | E345R/K360E/D39 9M/Y407A/K409V | 83 | E356K/D399K/K40 9E/K439E | | | 61 |
| 163 | 85 | E345R/K360E/D39 9M/Y407A/K409V | 83 | E356K/D399K/K40 9E/K439E | 19 | T394W/F405A | | | 61 |
| 164 | 19 | T394W/F405A | 81 | T366W/L368A/Y4 07V | 85 | E345R/K360E/D399 M/Y407A/K409V | 83 | E356K/D399K/K 409E/K439E | 61 |
| 165 | 85 | E345R/K360E/D39 9M/Y407A/K409V | 84 | E356K/K392D/D39 9K/K439E | | | | | 61 |
| 166 | 81 | T366W/L368A/Y4 07V | 85 | E345R/K360E/D39 9M/Y407A/K409V | 84 | E356K/K392D/D39 9K/K439E | | | 61 |
| 167 | 85 | E345R/K360E/D39 9M/Y407A/K409V | 84 | E356K/K392D/D39 9K/K439E | 19 | T394W/F405A | | | 61 |
| 168 | 19 | T394W/F405A | 81 | T366W/L368A/Y4 07V | 85 | E345R/K360E/D399 M/Y407A/K409V | 84 | E356K/K392D/D 399K/K439E | 61 |

**(Table 9) Correspondences between template heavy chains, introduced modifications, and heavy chain abbreviations in figures and tables**

| Template heavy chain: SEQ ID NO | Heavy chain abbreviation | Modification | Heavy chain SEQ ID NO | Heavy chain abbreviation | Modifications introduced into template heavy chain | Heavy chain SEQ ID NO | Heavy chain abbreviation | Modifications introduced into template heavy chain |
|---|---|---|---|---|---|---|---|---|
| 1 | a | - | 19 | A | T394W/F405A | | | |
| 78 | b | - | 81 | B | T366W/L368A/Y407V | | | |
| 79 | c | - | 82 | C | Q347R/K360E/D399V/F40 5T/K409W | 85 | C' | E345R/K360E/D399M/Y40 7A/K409V |
| 80 | d | - | 83 | D | E356K/D399K/K409E/K43 9E | 84 | D' | E356K/K392D/D399K/K43 9E |

### [Example 7] Evaluation of human FcRn binding of antibodies with modifications introduced into CH3 region

Next, the effect of the modifications for promoting heavy-chain homomeric association on Fc functions was examined.

Binding to human neonatal Fc receptor (FcRn) was evaluated using Biacore T200 (Cytiva). Evaluation was performed at 25°C using 50 mM phosphate buffer, 150 mM NaCl, 0.05 w/v%-P20, pH6.0, as a running buffer. rProtein L (BioVision) was immobilized onto Series S CM4 (Cytiva) as a ligand-capturing molecule. An antibody solution prepared with the running buffer was allowed to interact with this CM4 sensor chip to capture about 400 RU of the antibody. The human FcRn protein used in this measurement was prepared by the method described in WO02010107110. Human FcRn was diluted to 0, 250, 500, 1000, 2000, and 4000 nM with the running buffer and allowed to bind to the captured antibody. The chip was regenerated using 10 mM Glycine-HCl (pH 1.5) and repeatedly used to capture antibodies for measurement. The FcRn-binding activity of each antibody was evaluated by calculating KD (M) using Biacore T200 Evaluation Software 3.2.1 with a steady-state model. KD values were similar in the presence and absence of the modifications in the CH3 interface. Table 10 shows the KD (M) between human FcRn and each antibody (the non-introduction of modifications is denoted as "-").

**(Table 10) Evaluation of the human FcRn-binding activity of antibodies with modifications introduced into the CH3 region**

| Sample No. | Modifications that promote formation of heavy chain homodimers | KD value (M) for human FcRn |
|---|---|---|
| 61 | - | 1.83E-06 |
| 80 | T394W/F405A | 1.83E-06 |
| 87 | T366W/L368A/Y407V | 1.73E-06 |
| 92 | Q347R/K360E/D399V/F405T/K409W | 1.86E-06 |
| 95 | T394F/F405A | 1.77E-06 |
| 97 | E356K/D399K/K409E/K439E | 1.74E-06 |
| 100 | Q347E/S354Y/T366Y/Y407T | 1.76E-06 |
| 102 | K392L/T394W/F405A/Y407V | 1.69E-06 |
| 109 | E356K/K392D/D399K/K439E | 1.69E-06 |
| 111 | K392D/D399K/K409D | 1.53E-06 |
| 112 | Y349C/E357C/K392D/D399K | 1.57E-06 |
| 116 | E345R/Q347R/K360D/T366V/D399M/Y407A/K409V | 1.78E-06 |
| 117 | E356K/K392D/D399K/K409D | 1.59E-06 |
| 119 | E345R/K360E/D399M/Y407A/K409V | 1.61E-06 |
| 121 | T350V/L351Y/T366L/K392L/T394W/F405A/Y407V | 1.73E-06 |

### [Example 8] Evaluation of human Fcy receptor binding of antibodies with modifications introduced into the CH3 region

The binding activity of the produced modified antibodies for each human Fcγ receptor (hereinafter denoted as FcγR) was evaluated using Biacore T200 (Cytiva). Evaluation was performed at 25°C using 50 mM phosphate buffer, 150 mM NaCl, 0.05 w/v%-P20, pH7.4, as a running buffer. rProtein L (BioVision) was immobilized onto Series S CM4 (Cytiva) as a ligand-capturing molecule. An antibody solution prepared with the running buffer was allowed to interact with this CM4 sensor chip to capture about 500 RU of the antibody in the case of measurement for human FcγRIa, and 2000 RU in the case of measurement for the other human FcyRs. The human FcγR proteins used in this measurement were prepared by the method described in WO2022220275. The human FcγR was diluted with the running buffer to 8 nM in the case of FcγRIa or 1000 nM in the case of the other FcγRs, and allowed to bind to the captured antibody. The chip was regenerated using 10 mM Glycine-HCl (pH 1.5) and repeatedly used to capture antibodies for measurement. The binding activity of each antibody to each FcγR was evaluated by calculating the level of FcyR-binding (RU) per unit amount of antibody using Biacore T200 Evaluation Software version 3.2.1.

Table 11 shows binding levels per unit amount of antibody, and Table 12 shows relative levels (%) to the binding level of the Fc control antibody (Sample No. 61) (the non-introduction of modifications is denoted as "-"). The antibodies with CH3 modifications that promote heavy chain homodimer formation generally showed comparable or slightly higher binding activity to each human FcγR as compared to the control. However, Sample No. 117 showed a binding level of half that of the control or lower for some FcyRs. Therefore, if Fc functions comparable to the control antibody are desired for the purpose of use, the CH3 modifications other than those of Sample No. 117 should be used.

**(Table 11) Response values of CH3-modified antibodies to human FcyRs per RU of captured antibody**

| Sample No. | Modifications that promote formation of heavy chain homodimers | Level of binding to FcγR/amount of captured antibody | | | | | | | |
|---|---|---|---|---|---|---|---|---|---|
| | | hFcgRIa | hFcgRIIa_1 67H | hFcgRIIa_1 67R | hFcgRIIb | hFcgRIIa_ 176F | hFcgRIIIa_ 176V | hFcgRIIIb_ NA1 | hFcgRIIIb_ NA2 |
| 61 | - | 0.2221 | 0.0637 | 0.0512 | 0.0124 | 0.0281 | 0.0879 | 0.0058 | 0.0097 |
| 80 | T394W/F405A | 0.2143 | 0.0745 | 0.0641 | 0.0191 | 0.0345 | 0.0977 | 0.0078 | 0.0123 |
| 87 | T366W/L368A/Y407V | 0.2087 | 0.0784 | 0.0673 | 0.0201 | 0.0427 | 0.1104 | 0.0097 | 0.0153 |
| 92 | Q347R/K360E/D399V/F405T/K409W | 0.2235 | 0.0815 | 0.0690 | 0.0204 | 0.0379 | 0.1036 | 0.0097 | 0.0148 |
| 95 | T394F/F405A | 0.2160 | 0.0815 | 0.0711 | 0.0226 | 0.0382 | 0.1053 | 0.0095 | 0.0144 |
| 97 | E356K/D399K/K409E/K439E | 0.1818 | 0.0664 | 0.0537 | 0.0135 | 0.0276 | 0.0874 | 0.0065 | 0.0100 |
| 100 | Q347E/S354Y/T366Y/Y407T | 0.2242 | 0.0633 | 0.0583 | 0.0177 | 0.0406 | 0.1104 | 0.0078 | 0.0125 |
| 102 | K392L/T394W/F405A/Y407V | 0.2195 | 0.0857 | 0.0751 | 0.0255 | 0.0422 | 0.1084 | 0.0108 | 0.0163 |
| 109 | E356K/K392D/D399K/K439E | 0.2011 | 0.0831 | 0.0676 | 0.0191 | 0.0368 | 0.0995 | 0.0094 | 0.0148 |
| 111 | K392D/D399K/K409D | 0.2237 | 0.0839 | 0.0698 | 0.0197 | 0.0405 | 0.1042 | 0.0111 | 0.0167 |
| 112 | Y349C/E357C/K392D/D399K | 0.2392 | 0.0889 | 0.0747 | 0.0227 | 0.0405 | 0.1044 | 0.0113 | 0.0176 |
| 116 | E345R/Q347R/K360D/T366V/D399M/Y407A /K409V | 0.1823 | 0.0475 | 0.0424 | 0.0111 | 0.0299 | 0.0804 | 0.0054 | 0.0087 |
| 117 | E356K/K392D/D399K/K409D | 0.2067 | 0.0343 | 0.0245 | 0.0049 | 0.0171 | 0.0545 | 0.0019 | 0.0043 |
| 119 | E345R/K360E/D399M/Y407A/K409V | 0.1732 | 0.0561 | 0.0480 | 0.0120 | 0.0311 | 0.0876 | 0.0063 | 0.0103 |
| 121 | T350V/L351Y/T366L/K392L/T394W/F405A/ Y407V | 0.1957 | 0.0927 | 0.0839 | 0.0316 | 0.0518 | 0.1183 | 0.0143 | 0.0214 |

**(Table 12) Relative binding ratios (%) of CH3-modified antibodies for human FcyRs**

| Sample No. | Modifications that promote formation of heavy chain homodimers | Ratio of (level of binding to FcγR/amount of captured antibody) to WT-IgG1 (%) | | | | | | | |
|---|---|---|---|---|---|---|---|---|---|
| | | hFcgRIa | hFcgRIIa_1 67H | hFcgRIIa_1 67R | hFcgRIIb | bFcgRIIIa_ 176F | hFcgRIIIa_ 176V | hFcgRIIIb_ NA1 | hFcgRIIIb_ NA2 |
| 61 | - | 100 | 100 | 100 | 100 | 100 | 100 | 100 | 100 |
| 80 | T394W/F405A | 96 | 117 | 125 | 155 | 123 | 111 | 135 | 127 |
| 87 | T366W/L368A/Y407V | 94 | 123 | 131 | 163 | 152 | 126 | 168 | 159 |
| 92 | Q347R/K360E/D399V/F405T/K409W | 101 | 128 | 135 | 165 | 135 | 118 | 168 | 153 |
| 95 | T394F/F405A | 97 | 128 | 139 | 183 | 136 | 120 | 164 | 149 |
| 97 | E356K/D399K/K409E/K439E | 82 | 104 | 105 | 109 | 98 | 99 | 111 | 104 |
| 100 | Q347E/S354Y/T366Y/Y407T | 101 | 99 | 114 | 143 | 144 | 126 | 134 | 129 |
| 102 | K392L/T394W/F405A/Y407V | 99 | 135 | 147 | 206 | 150 | 123 | 186 | 169 |
| 109 | E356K/K392D/D399K/K439E | 91 | 131 | 132 | 155 | 131 | 113 | 162 | 154 |
| 111 | K392D/D399K/K409D | 101 | 132 | 136 | 160 | 144 | 119 | 191 | 172 |
| 112 | Y349C/E357C/K392D/D399K | 108 | 140 | 146 | 184 | 144 | 119 | 196 | 183 |
| 116 | E345R/Q347R/K360D/T366V/D399M/Y407A /K409V | 82 | 75 | 83 | 90 | 107 | 92 | 93 | 90 |
| 117 | E356K/K392D/D399K/K409D | 93 | 54 | 48 | 40 | 61 | 62 | 33 | 44 |
| 119 | E345R/K360E/D399M/Y407A/K409V | 78 | 88 | 94 | 97 | 110 | 100 | 108 | 107 |
| 121 | T350V/L351Y/T366L/K392L/T394W/F405A/ Y407V | 88 | 146 | 164 | 256 | 184 | 135 | 247 | 221 |

### [Example 9] Evaluation of ECM binding of antibodies with modifications introduced into CH3 region

Next, the effect of heavy-chain homomeric association-promoting modifications on the non-specific binding of antibody was examined.

A system of evaluating binding to extracellular matrix (ECM) is known as an *in vitro* evaluation system for non-specific binding. This assay system was used for evaluation (US Patent 2014/0080153). Measurement results are shown in Figure 4. The presence and absence of the modifications in the CH3 interface resulted in similar levels of ECM binding.

### [Example 10] Evaluation of heavy-chain homodimer formation ability between different IgG subclasses

In Examples 1-9, antibodies of IgG1 subclass were used for evaluation. However, other subclasses (IgG2 and IgG4) are also important for therapeutic antibodies. Moreover, for prevention of association with endogenous antibodies in living organisms, it is important that no heavy chain heteromers are formed between different subclasses. Here, some of the modifications that exhibited strong heavy-chain homodimer formation ability in IgG1 were applied to different IgG subclasses, and their heavy-chain homodimer formation ability was examined by SEC analysis. The correspondences between the template subclasses used for antibody expression, amino acid modifications, SEQ ID NOs, and area ratios are shown in Table 13 (the non-introduction of modifications is denoted as "-"). Antibody expression and analysis were performed according to the methods described in Example 1. However, pairs containing IgG3 were purified using MonoSpin ProG (GL science) as their affinity purification with Protein A is difficult. The results of Table 13 showed that all subclass pairs of IgG1, IgG2, and IgG4 have heavy-chain homodimer formation ability.

In addition, the analysis of WT-IgG1, IgG2, and IgG4 paired with WT-IgG3 revealed that WT-IgG1, IgG2, and IgG4 intrinsically less readily form a pair with IgG3. This suggested that these amino acid-modified antibodies which do not form heavy-chain heterodimers between IgG1, IgG2, and IgG4 even less readily form heavy-chain heteromultimers with WT-IgG3.

**(Table 13) Evaluation of heavy-chain homodimer forming ability of pairs of different IgG subclasses**

| Sample No. | Full-length heavy chain | IgG subclass | Modifications that promote formation of heavy chain homodimers | Fc fragment | IgG subclass | Modifications that promote formation of heavy chain homodimers | Light chain | Homo full-length heavy chain (%) | Heteromultimer (%) | Homo Fc fragment (%) |
|---|---|---|---|---|---|---|---|---|---|---|
| | SEQ ID NO | | | SEQ ID NO | | | SEQ ID NO | | | |
| 169 | 1 | IgG1 | - | 62 | IgG1 | - | 61 | 28.7 | 52.1 | 19.2 |
| 170 | 86 | IgG2 | - | 62 | IgG1 | - | 61 | 21.6 | 55 | 23.5 |
| 171 | 87 | IgG2 | T394W/F405A | 62 | IgG1 | - | 61 | 58.9 | 0 | 41.1 |
| 172 | 88 | IgG2 | T366W/L368A/Y407V | 62 | IgG1 | - | 61 | 48.2 | 0 | 51.8 |
| 173 | 89 | IgG2 | Q347R/K360E/D399V/F405T/K409W | 62 | IgG1 | - | 61 | 55.9 | 0 | 44.1 |
| 174 | 90 | IgG2 | K392L/T394W/F405A/Y407V | 62 | IgG1 | - | 61 | 48.8 | 0 | 51.2 |
| 175 | 91 | IgG2 | K392D/D399K/K409D | 62 | IgG1 | - | 61 | 52.7 | 0 | 47.3 |
| 176 | 86 | IgG2 | - | 102 | IgG4 | - | 61 | 18.2 | 60.4 | 21.4 |
| 177 | 87 | IgG2 | T394W/F405A | 102 | IgG4 | - | 61 | 66.7 | 0 | 33.3 |
| 178 | 88 | IgG2 | T366W/L368A/Y407V | 102 | IgG4 | - | 61 | 66.6 | 0 | 33.4 |
| 179 | 89 | IgG2 | Q347R/K360E/D399V/F405T/K409W | 102 | IgG4 | - | 61 | 66.8 | 0 | 33.2 |
| 180 | 90 | IgG2 | K392L/T394W/F405A/Y407V | 102 | IgG4 | - | 61 | 66.3 | 0 | 33.7 |
| 181 | 91 | IgG2 | K392D/D399K/K409D | 102 | IgG4 | - | 61 | 60.5 | 0 | 39.5 |
| 182 | 92 | IgG4 | - | 62 | IgG1 | - | 61 | 46.4 | 47.7 | 5.8 |
| 183 | 93 | IgG4 | T394W/F405A | 62 | IgG1 | - | 61 | 56.3 | 0 | 43.7 |
| 184 | 94 | IgG4 | T366W/L368A/Y407V | 62 | IgG1 | - | 61 | 87.3 | 0 | 12.7 |
| 185 | 95 | IgG4 | Q347R/K360E/D399V/F405T/R409W | 62 | IgG1 | - | 61 | 58.6 | 0 | 41.4 |
| 186 | 96 | IgG4 | K392L/T394W/F405A/Y407V | 62 | IgG1 | - | 61 | 78.7 | 0.2 | 21 |
| 187 | 97 | IgG4 | K392D/D399K/R409D | 62 | IgG1 | - | 61 | 56.5 | 0 | 43.5 |
| 188 | 92 | IgG4 | - | 102 | IgG4 | - | 61 | 19.4 | 54.3 | 26.2 |
| 189 | 93 | IgG4 | T394W/F405A | 102 | IgG4 | - | 61 | 60.3 | 0 | 39.7 |
| 190 | 94 | IgG4 | T366W/L368A/Y407V | 102 | IgG4 | - | 61 | 65.3 | 0 | 34.7 |
| 191 | 95 | IgG4 | Q347R/K360E/D399V/F405T/R409W | 102 | IgG4 | - | 61 | 66.8 | 0 | 33.2 |
| 192 | 96 | IgG4 | K392L/T394W/F405A/Y407V | 102 | IgG4 | - | 61 | 66.2 | 0 | 33.8 |
| 193 | 97 | IgG4 | K392D/D399K/R409D | 102 | IgG4 | - | 61 | 64.5 | 0 | 35.5 |
| 194 | 1 | IgG1 | - | 102 | IgG4 | - | 61 | 33.8 | 55.3 | 10.9 |
| 195 | 19 | IgG1 | T394W/F405A | 102 | IgG4 | - | 61 | 75.3 | 0 | 24.7 |
| 196 | 26 | IgG1 | T366W/L368A/Y407V | 102 | IgG4 | - | 61 | 73.8 | 0 | 26.2 |
| 197 | 31 | IgG1 | Q347R/K360E/D399V/F405T/K409W | 102 | IgG4 | - | 61 | 72.1 | 0 | 27.9 |
| 198 | 36 | IgG1 | E356K/D399K/K409E/K439E | 102 | IgG4 | - | 61 | 73.3 | 0 | 26.7 |
| 199 | 39 | IgG1 | Q347E/S354Y/T366Y/Y407T | 102 | IgG4 | - | 61 | 70.4 | 0 | 29.6 |
| 200 | 41 | IgG1 | K392L/T394W/F405A/Y407V | 102 | IgG4 | - | 61 | 72.7 | 0 | 27.3 |
| 201 | 48 | IgG1 | E356K/K392D/D399K/K439E | 102 | IgG4 | - | 61 | 69.6 | 0 | 30.4 |
| 202 | 50 | IgG1 | K392D/D399K/K409D | 102 | IgG4 | - | 61 | 76.8 | 0 | 23.2 |
| 203 | 55 | IgG1 | E345R/Q347R/K360D/T366V/D399M/Y407 A/K409V | 102 | IgG4 | - | 61 | 70.7 | 0 | 29.3 |
| 204 | 56 | IgG1 | E356K/K392D/D399K/K409D | 102 | IgG4 | - | 61 | 70.7 | 0 | 29.3 |
| 205 | 58 | IgG1 | E345R/K360E/D399M/Y407A/K409V | 102 | IgG4 | - | 61 | 70.3 | 0 | 29.7 |
| 206 | 60 | IgG1 | T350V/L351Y/T366L/K392L/T394W/F405A /Y407V | 102 | IgG4 | - | 61 | 71.9 | 0 | 28.1 |
| 207 | 86 | IgG2 | - | 103 | IgG2 | - | 61 | 37.8 | 48.8 | 13.4 |
| 208 | 87 | IgG2 | T394W/F405A | 103 | IgG2 | - | 61 | 80.8 | 0 | 19.2 |
| 209 | 88 | IgG2 | T366W/L368A/Y407V | 103 | IgG2 | - | 61 | 80 | 0 | 20 |
| 210 | 89 | IgG2 | Q347R/K360E/D399V/F405T/K409W | 103 | IgG2 | - | 61 | 80.4 | 0 | 19.6 |
| 211 | 90 | IgG2 | K392L/T394W/F405A/Y407V | 103 | IgG2 | - | 61 | 78.5 | 0 | 21.5 |
| 212 | 91 | IgG2 | K392D/D399K/K409D | 103 | IgG2 | - | 61 | 79.6 | 0 | 20.4 |
| 213 | 92 | IgG4 | - | 103 | IgG2 | - | 61 | 31.8 | 56.1 | 12.1 |
| 214 | 93 | IgG4 | T394W/F405A | 103 | IgG2 | - | 61 | 82 | 0 | 18 |
| 215 | 94 | IgG4 | T366W/L368A/Y407V | 103 | IgG2 | - | 61 | 83 | 0 | 17 |
| 216 | 95 | IgG4 | Q347R/K360E/D399V/F405T/R409W | 103 | IgG2 | - | 61 | 83.5 | 0 | 16.5 |
| 217 | 96 | IgG4 | K392L/T394W/F405A/Y407V | 103 | IgG2 | - | 61 | 81.5 | 0 | 18.5 |
| 218 | 97 | IgG4 | K392D/D399K/R409D | 103 | IgG2 | - | 61 | 82.5 | 0 | 17.5 |
| 219 | 1 | IgG1 | - | 103 | IgG2 | - | 61 | 38.7 | 50 | 11.3 |
| 220 | 19 | IgG1 | T394W/F405A | 103 | IgG2 | - | 61 | 84.3 | 0 | 15.7 |
| 221 | 26 | IgG1 | T366W/L368A/Y407V | 103 | IgG2 | - | 61 | 85.3 | 0 | 14.7 |
| 222 | 31 | IgG1 | Q347R/K360E/D399V/F405T/K409W | 103 | IgG2 | - | 61 | 82.7 | 0 | 17.3 |
| 223 | 36 | IgG1 | E356K/D399K/K409E/K439E | 103 | IgG2 | - | 61 | 84.3 | 0 | 15.7 |
| 224 | 39 | IgG1 | Q347E/S354Y/T366Y/Y407T | 103 | IgG2 | - | 61 | 82.9 | 0 | 17.1 |
| 225 | 41 | IgG1 | K392L/T394W/F405A/Y407V | 103 | IgG2 | - | 61 | 83.8 | 0 | 16.2 |
| 226 | 48 | IgG1 | E356K/K392D/D399K/K439E | 103 | IgG2 | - | 61 | 85.1 | 0 | 14.9 |
| 227 | 50 | IgG1 | K392D/D399K/K409D | 103 | IgG2 | - | 61 | 81.4 | 0 | 18.6 |
| 228 | 55 | IgG1 | E345R/Q347R/K360D/T366V/D399M/Y407 A/K409V | 103 | IgG2 | - | 61 | 83.5 | 0 | 16.5 |
| 229 | 56 | IgG1 | E356K/K392D/D399K/K409D | 103 | IgG2 | - | 61 | 81.4 | 0 | 18.6 |
| 230 | 58 | IgG1 | E345R/K360E/D399M/Y407A/K409V | 103 | IgG2 | - | 61 | 84.3 | 0 | 15.7 |
| 231 | 60 | IgG1 | T350V/L351Y/T366L/K392L/T394W/F405A /Y407V | 103 | IgG2 | - | 61 | 85.8 | 0 | 14.2 |
| 232 | 98 | IgG2 | E356K/D399K/K409E | 103 | IgG2 | - | 61 | 51.1 | 8.2 | 40.7 |
| 233 | 99 | IgG2 | Q347E/S354Y/T366Y/Y407T | 103 | IgG2 | - | 61 | 76.3 | 0 | 23.7 |
| 234 | 100 | IgG2 | E345R/Q347R/K360D/T366V/D399M/Y407 A/K409V | 103 | IgG2 | - | 61 | 76.1 | 0 | 23.9 |
| 235 | 101 | IgG3 | - | 62 | IgG1 | - | 61 | 49.3 | 20 | 30.6 |
| 236 | 101 | IgG3 | - | 103 | IgG2 | - | 61 | 37.2 | 37.4 | 25.4 |
| 237 | 101 | IgG3 | - | 102 | IgG4 | - | 61 | 62.2 | 10.4 | 27.3 |
| 238 | 104 | IgG2 | E356K/K392D/D399K/K439E | 62 | IgG1 | - | 61 | 50.9 | 0 | 49.1 |
| 239 | 98 | IgG2 | E356K/D399K/K409E | 62 | IgG1 | - | 61 | 20.7 | 5.4 | 73.9 |
| 240 | 99 | IgG2 | Q347E/S354Y/T366Y/Y407T | 62 | IgG1 | - | 61 | 46.9 | 0 | 53.1 |
| 241 | 100 | IgG2 | E345R/Q347R/K360D/T366V/D399M/Y407 A/K409V | 62 | IgG1 | - | 61 | 49.3 | 0 | 50.7 |
| 242 | 105 | IgG2 | E356K/K392D/D399K/K409D | 62 | IgG1 | - | 61 | 44.1 | 0 | 55.9 |
| 243 | 106 | IgG2 | E345R/K360E/D399M/Y407A/K409V | 62 | IgG1 | - | 61 | 49.8 | 0 | 50.2 |
| 244 | 107 | IgG2 | T350V/L351Y/T366L/K392L/T394W/F405A /Y407V | 62 | IgG1 | - | 61 | 47.6 | 0 | 52.4 |
| 245 | 108 | IgG4 | E356K/K392D/D399K/K439E | 62 | IgG1 | - | 61 | 48.6 | 0 | 51.4 |
| 246 | 109 | IgG4 | E356K/D399K/R409E/K439E | 62 | IgG1 | - | 61 | 56.4 | 0 | 43.6 |
| 247 | 110 | IgG4 | Q347E/S354Y/T366Y/Y407T | 62 | IgG1 | - | 61 | 39 | 10.6 | 50.5 |
| 248 | 111 | IgG4 | E345R/Q347R/K360D/T366V/D399M/Y407 A/R409V | 62 | IgG1 | - | 61 | 54 | 0 | 46 |
| 249 | 112 | IgG4 | E356K/K392D/D399K/R409D | 62 | IgG1 | - | 61 | 55.8 | 0 | 44.2 |
| 250 | 113 | IgG4 | E345R/K360E/D399M/Y407A/R409V | 62 | IgG1 | - | 61 | 60.2 | 0 | 39.8 |
| 251 | 114 | IgG4 | T350V/L351Y/T366L/K392L/T394W/F405A /Y407V | 62 | IgG1 | - | 61 | 55.1 | 0 | 44.9 |
| 252 | 104 | IgG2 | E356K/K392D/D399K/K439E | 103 | IgG2 | - | 61 | 82.7 | 0 | 17.3 |
| 253 | 98 | IgG2 | E356K/D399K/K409E | 103 | IgG2 | - | 61 | 63.7 | 8.7 | 27.6 |
| 254 | 99 | IgG2 | Q347E/S354Y/T366Y/Y407T | 103 | IgG2 | - | 61 | 81.5 | 0 | 18.5 |
| 255 | 100 | IgG2 | E345R/Q347R/K360D/T366V/D399M/Y407 A/K409V | 103 | IgG2 | - | 61 | 80.8 | 0 | 19.2 |
| 256 | 105 | IgG2 | E356K/K392D/D399K/K409D | 103 | IgG2 | - | 61 | 78.2 | 0 | 21.8 |
| 257 | 106 | IgG2 | E345R/K360E/D399M/Y407A/K409V | 103 | IgG2 | - | 61 | 83.2 | 0 | 16.8 |
| 258 | 108 | IgG4 | E356K/K392D/D399K/K439E | 103 | IgG2 | - | 61 | 81.9 | 0 | 18.1 |
| 259 | 109 | IgG4 | E356K/D399K/R409E/K439E | 103 | IgG2 | - | 61 | 86.2 | 0 | 13.8 |
| 260 | 110 | IgG4 | Q347E/S354Y/T366Y/Y407T | 103 | IgG2 | - | 61 | 73.4 | 5.3 | 21.3 |
| 261 | 111 | IgG4 | E345R/Q347R/K360D/T366V/D399M/Y407 A/R409V | 103 | IgG2 | - | 61 | 82 | 0 | 18 |
| 262 | 112 | IgG4 | E356K/K392D/D399K/R409D | 103 | IgG2 | - | 61 | 82.8 | 0 | 17.2 |
| 263 | 113 | IgG4 | E345R/K360E/D399M/Y407A/R409V | 103 | IgG2 | - | 61 | 84.5 | 0 | 15.5 |
| 264 | 114 | IgG4 | T350V/L351Y/T366L/K392L/T394W/F405A /Y407V | 103 | IgG2 | - | 61 | 85.6 | 0 | 14.4 |
| 265 | 104 | IgG2 | E356K/K392D/D399K/K439E | 102 | IgG4 | - | 61 | 61.3 | 0 | 38.7 |
| 266 | 98 | IgG2 | E356K/D399K/K409E | 102 | IgG4 | - | 61 | 24.9 | 0 | 75.1 |
| 267 | 99 | IgG2 | Q347E/S354Y/T366Y/Y407T | 102 | IgG4 | - | 61 | 56.5 | 0 | 43.5 |
| 268 | 100 | IgG2 | E345R/Q347R/K360D/T366V/D399M/Y407 A/K409V | 102 | IgG4 | - | 61 | 57.7 | 0 | 42.3 |
| 269 | 105 | IgG2 | E356K/K392D/D399K/K409D | 102 | IgG4 | - | 61 | 50.8 | 0 | 49.2 |
| 270 | 106 | IgG2 | E345R/K360E/D399M/Y407A/K409V | 102 | IgG4 | - | 61 | 62.8 | 0 | 37.2 |
| 271 | 107 | IgG2 | T350V/L351Y/T366L/K392L/T394W/F405A /Y407V | 102 | IgG4 | - | 61 | 59.1 | 0 | 40.9 |
| 272 | 108 | IgG4 | E356K/K392D/D399K/K439E | 102 | IgG4 | - | 61 | 58 | 0 | 42 |
| 273 | 109 | IgG4 | E356K/D399K/R409E/K439E | 102 | IgG4 | - | 61 | 64.9 | 0 | 35.1 |
| 274 | 110 | IgG4 | Q347E/S354Y/T366Y/Y407T | 102 | IgG4 | - | 61 | 51.3 | 4.6 | 44.1 |
| 275 | 111 | IgG4 | E345R/Q347R/K360D/T366V/D399M/Y407 A/R409V | 102 | IgG4 | - | 61 | 60.8 | 0 | 39.2 |
| 276 | 112 | IgG4 | E356K/K392D/D399K/R409D | 102 | IgG4 | - | 61 | 62.6 | 0 | 37.4 |
| 277 | 113 | IgG4 | E345R/K360E/D399M/Y407A/R409V | 102 | IgG4 | - | 61 | 67 | 0 | 33 |
| 278 | 114 | IgG4 | T350V/L351Y/T366L/K392L/T394W/F405A /Y407V | 102 | IgG4 | - | 61 | 64 | 0 | 36 |
| 279 | 107 | IgG2 | T350V/L351Y/T366L/K392L/T394W/F405A /Y407V | 103 | IgG2 | - | 61 | 85.5 | 0 | 14.5 |

### [Example 11] Evaluation of heavy chain homodimer-forming ability in antibodies containing different IgG formats and Fc modifications

The evaluation of Examples 1-10 was performed using the basic IgG format and the Fc of wild-type IgG containing no other modification than CH3 interface control. However, to prepare and express more effective therapeutic antibodies, applicability to various antibody formats, such as formats in which a VHH or scFv is linked with an Fc, and antibodies into which amino acid modifications for altering Fc functions are introduced, is ideal. Here, some of the modifications that exhibited strong heavy-chain homodimer formation ability in IgG were applied to various antibody formats and Fc-modified antibodies, and their heavy chain homodimer formation ability was examined by SEC analysis. The correspondences between the heavy chain backbone or amino acid modifications (and their functions) contained in the template antibodies used for antibody expression, the amino acid modifications for promoting heavy chain homodimer formation, the SEQ ID NOs, and the area ratios are shown in Table 14 (the non-introduction of modifications is denoted as "-"). Antibody expression and analysis were performed according to the methods described in Example 1. However, for antibodies containing the E345R/E430G/S440Y modification, which promotes hexamer formation of IgG, a multimeric component was detected in SEC analysis. In addition, for pairs containing the format of scFv.LH-Fc or scFv.HL-Fc, complete separation of each homomer and heteromeric multimer was difficult. Thus, for these samples, the effect of suppressing heteromultimer formation was qualitatively evaluated focusing on the peak of the heteromultimeric component (Figs. 5-1 to 5-14).

Based on the results of Table 14 and Figs. 5-1 to 5-14, it was revealed that the heavy-chain homodimer formation ability (or the effect of suppressing heteromultimer formation) is maintained even when the binding region of the antibody is VHH or scFv, and even when the Fc region is deglycosylated or contains modifications for enhancing FcRn binding, suppressing FcγR binding, or promoting hexamer formation.

Furthermore, since the heavy-chain homodimer formation ability (or the effect of suppressing heterodimer formation) is maintained in all formats, it can expected to be also maintained even when Fc is linked to a polypeptide other than Fab, VHH, and scFv, and widely maintained even in the presence of amino acid modifications that do not affect the Fc interface.

**(Table 14) Evaluation of heavy-chain homodimer formation ability in antibodies including VHH-Fc, scFv-Fc, and modifications**

| Sample No. | Heavy chain backbone or amino acid modifications (functions) contained | Modifications that promote formation of heavy chain homodimers | Full-length heavy chain | Light chain | Fc fragment or scFv, VHH-Fc | Homo full-length heavy chain (%) | Heteromultimer (%) | Homo Fc fragment (%) |
|---|---|---|---|---|---|---|---|---|
| | | | SEQ ID NO | SEQ ID NO | SEQ ID NO | | | |
| 280 | N297A (deglycosylation) | - | 115 | 61 | 62 | 26.0 | 50.2 | 23.9 |
| 281 | N297A (deglycosylation) | T394W/F405A | 116 | 61 | 62 | 63.3 | 0.0 | 36.7 |
| 282 | N297A (deglycosylation) | T366W/L368A/Y407V | 117 | 61 | 62 | 57.7 | 2.3 | 40.0 |
| 283 | N297A (deglycosylation) | Q347R/K360E/D399V/F405T/K409W | 118 | 61 | 62 | 61.4 | 0.0 | 38.6 |
| 284 | N297A (deglycosylation) | E356K/D399K/K409E/K439E | 119 | 61 | 62 | 62.6 | 0.0 | 37.4 |
| 285 | N297A (deglycosylation) | E356K/K392D/D399K/K439E | 120 | 61 | 62 | 59.8 | 2.6 | 37.6 |
| 286 | N297A (deglycosylation) | K392D/D399K/K409D | 121 | 61 | 62 | 57.1 | 1.5 | 41.3 |
| 287 | N297A (deglycosylation) | E345R/K360E/D399M/Y407A/K409V | 122 | 61 | 62 | 66.3 | 0.0 | 33.7 |
| 288 | N297A (deglycosylation) | T350V/L351Y/T366L/K392L/T394W/F405A/Y407V | 123 | 61 | 62 | 60.2 | 0.0 | 39.8 |
| 289 | M428L/N434A/Y436T/Q438R/S440E (enhance FcRn binding) | - | 124 | 61 | 62 | 18.6 | 54.5 | 26.8 |
| 290 | M428L/N434A/Y436T/Q438R/S440E (enhance FcRn binding) | T394W/F405A | 125 | 61 | 62 | 43.4 | 1.6 | 55.1 |
| 291 | M428L/N434A/Y436T/Q438R/S440E (enhance FcRn binding) | T366W/L368A/Y407V | 126 | 61 | 62 | 51.9 | 2.4 | 45.7 |
| 292 | M428L/N434A/Y436T/Q438R/S440E (enhance FcRn binding) | Q347R/K360E/D399V/F405T/K409W | 127 | 61 | 62 | 49.9 | 1.5 | 48.6 |
| 293 | M428L/N434A/Y436T/Q438R/S440E (enhance FcRn binding) | E356K/D399K/K409E/K439E | 128 | 61 | 62 | 51.7 | 1.5 | 46.8 |
| 294 | M428L/N434A/Y436T/Q438R/S440E (enhance FcRn binding) | E356K/K392D/D399K/K439E | 129 | 61 | 62 | 58.8 | 2.2 | 39.1 |
| 295 | M428L/N434A/Y436T/Q438R/S440E (enhance FcRn binding) | K392D/D399K/K409D | 130 | 61 | 62 | 43.2 | 1.6 | 55.2 |
| 296 | M428L/N434A/Y436T/Q438R/S440E (enhance FcRn binding) | E345R/K360E/D399M/Y407A/K409V | 131 | 61 | 62 | 45.4 | 1.7 | 52.9 |
| 297 | M428L/N434A/Y436T/Q438R/S440E (enhance FcRn binding) | T350V/L351Y/T366L/K392L/T394W/F405A/Y407V | 132 | 61 | 62 | 52.0 | 0.0 | 48.0 |
| 298 | VHH-Fc | - | 1 | 61 | 579 | 52.2 | 41.8 | 5.9 |
| 299 | VHH-Fc | T394W/F405A | 1 | 61 | 580 | 68.9 | 0.0 | 31.1 |
| 300 | VHH-Fc | T366W/L368A/Y407V | 1 | 61 | 581 | 69.5 | 0.0 | 30.5 |
| 301 | VHH-Fc | Q347R/K360E/D399V/F405T/K409W | 1 | 61 | 582 | 72.8 | 0.0 | 27.2 |
| 302 | VHH-Fc | T394F/F405A | 1 | 61 | 583 | 73.6 | 0.0 | 26.4 |
| 303 | VHH-Fc | E356K/D399K/K409E/K439E | 1 | 61 | 584 | 73.4 | 0.0 | 26.6 |
| 304 | VHH-Fc | Q347E/S354Y/T366Y/Y407T | 1 | 61 | 585 | 68.8 | 0.0 | 31.2 |
| 305 | VHH-Fc | K392L/T394W/F405A/Y407V | 1 | 61 | 586 | 66.1 | 0.0 | 33.9 |
| 306 | VHH-Fc | E356K/K392D/D399K/K439E | 1 | 61 | 587 | 68.5 | 0.0 | 31.5 |
| 307 | VHH-Fc | K392D/D399K/K409D | 1 | 61 | 588 | 75.7 | 0.0 | 24.3 |
| 308 | VHH-Fc | Y349C/E357C/K392D/D399K | 1 | 61 | 589 | 91.4 | 0.0 | 8.6 |
| 309 | VHH-Fc | E345R/Q347R/K360D/T366V/D399M/Y407A/K409V | 1 | 61 | 590 | 67.7 | 0.0 | 32.3 |
| 310 | VHH-Fc | E356K/K392D/D399K/K409D | 1 | 61 | 591 | 71.1 | 0.0 | 28.9 |
| 311 | VHH-Fc | E345R/K360E/D399M/Y407A/K409V | 1 | 61 | 592 | 67.3 | 0.0 | 32.7 |
| 312 | VHH-Fc | T350V/L351Y/T366L/K392L/T394W/F405A/Y407V | 1 | 61 | 593 | 68.2 | 0.0 | 31.8 |
| 313 | L235R/G236R (suppress FcγR binding) | - | 133 | 61 | 594 | 27.9 | 52.9 | 19.2 |
| 314 | L235R/G236R (suppress FcγR binding) | T394W/F405A | 134 | 61 | 594 | 64.1 | 0.0 | 35.9 |
| 315 | L235R/G236R (suppress FcγR binding) | T366W/L368A/Y407V | 135 | 61 | 594 | 58.1 | 3.4 | 38.5 |
| 316 | L235R/G236R (suppress FcγR binding) | Q347R/K360E/D399V/F405T/K409W | 136 | 61 | 594 | 60.2 | 0.0 | 39.8 |
| 317 | L235R/G236R (suppress FcγR binding) | E356K/K392D/D399K/K439E | 137 | 61 | 594 | 62.2 | 0.0 | 37.8 |
| 318 | L234A/L235A (suppress FcγR binding) | - | 138 | 61 | 595 | 36.0 | 50.3 | 13.7 |
| 319 | L234A/L235A (suppress FcγR binding) | T394W/F405A | 139 | 61 | 595 | 66.9 | 0.0 | 33.1 |
| 320 | L234A/L235A (suppress FcγR binding) | T366W/L368A/Y407V | 140 | 61 | 595 | 67.5 | 0.0 | 32.5 |
| 321 | L234A/L235A (suppress FcγR binding) | Q347R/K360E/D399V/F405T/K409W | 141 | 61 | 595 | 65.3 | 0.0 | 34.7 |
| 322 | L234A/L235A (suppress FcγR binding) | E356K/K392D/D399K/K439E | 142 | 61 | 595 | 67.9 | 0.0 | 32.1 |
| 323 | M252Y/S254T/T256E (enhance FcRn binding) | - | 143 | 61 | 596 | 16.3 | 50.1 | 33.7 |
| 324 | M252Y/S254T/T256E (enhance FcRn binding) | T394W/F405A | 144 | 61 | 596 | 69.8 | 0.0 | 30.2 |
| 325 | M252Y/S254T/T256E (enhance FcRn binding) | T366W/L368A/Y407V | 145 | 61 | 596 | 62.3 | 0.0 | 37.7 |
| 326 | M252Y/S254T/T256E (enhance FcRn binding) | Q347R/K360E/D399V/F405T/K409W | 146 | 61 | 596 | 63.3 | 0.0 | 36.7 |
| 327 | M252Y/S254T/T256E (enhance FcRn binding) | E356K/K392D/D399K/K439E | 147 | 61 | 596 | 61.6 | 0.0 | 38.4 |
| 328 | M428L/N434S (enhance FcRn binding) | - | 148 | 61 | 597 | 32.6 | 51.7 | 15.8 |
| 329 | M428L/N434S (enhance FcRn binding) | T394W/F405A | 149 | 61 | 597 | 61.0 | 0.0 | 39.0 |
| 330 | M428L/N434S (enhance FcRn binding) | T366W/L368A/Y407V | 150 | 61 | 597 | 54.8 | 0.0 | 45.2 |
| 331 | M428L/N434S (enhance FcRn binding) | Q347R/K360E/D399V/F405T/K409W | 151 | 61 | 597 | 66.9 | 0.0 | 33.1 |
| 332 | M428L/N434S (enhance FcRn binding) | E356K/K392D/D399K/K439E | 152 | 61 | 597 | 67.9 | 0.0 | 32.1 |
| 333 | L234A/L235A/P329G (suppress FcγR binding) | - | 153 | 61 | 598 | 22.6 | 51.4 | 26.0 |
| 334 | L234A/L235A/P329G (suppress FcγR binding) | T394W/F405A | 154 | 61 | 598 | 62.8 | 0.0 | 37.2 |
| 335 | L234A/L235A/P329G (suppress FcγR binding) | T366W/L368A/Y407V | 155 | 61 | 598 | 63.9 | 0.0 | 36.1 |
| 336 | L234A/L235A/P329G (suppress FcγR binding) | Q347R/K360E/D399V/F405T/K409W | 156 | 61 | 598 | 61.5 | 0.0 | 38.5 |
| 337 | L234A/L235A/P329G (suppress FcγR binding) | E356K/K392D/D399K/K439E | 157 | 61 | 598 | 57.4 | 0.0 | 42.6 |
| 338 | L234F/L235E/P331S (suppress FcγR binding) | T394W/F405A | 158 | 61 | 599 | 61.9 | 0.0 | 38.1 |
| 339 | L234F/L235E/P331S (suppress FcγR binding) | Q347R/K360E/D399V/F405T/K409W | 159 | 61 | 599 | 64.6 | 0.0 | 35.4 |
| 340 | L234F/L235E/P331S (suppress FcγR binding) | E356K/K392D/D399K/K439E | 160 | 61 | 599 | 61.6 | 0.0 | 38.4 |
| 341 | E345R/E430G/S440Y (promote hexamer formation) | - | 161 | 61 | 165 | Modified Fc control | | |
| 342 | E345R/E430G/S440Y (promote hexamer formation) | T394W/F405A | 162 | 61 | 165 | Qualitative evaluation of decrease in heteromultimers | | |
| 343 | E345R/E430G/S440Y (promote hexamer formation) | T366W/L368A/Y407V | 163 | 61 | 165 | Qualitative evaluation of decrease in heteromultimers | | |
| 344 | E345R/E430G/S440Y (promote hexamer formation) | Q347R/K360E/D399V/F405T/K409W | 164 | 61 | 165 | Qualitative evaluation of decrease in heteromultimers | | |
| 345 | scFv.LH-Fc | - | 1 | 61 | 166 | scFv.LH-Fc control | | |
| 346 | scFv.LH-Fc | Q347R/K360E/D399V/F405T/K409W | 1 | 61 | 167 | Qualitative evaluation of decrease in heteromultimers | | |
| 347 | scFv.LH-Fc | E356K/K392D/D399K/K439E | 1 | 61 | 168 | Qualitative evaluation of decrease in heteromultimers | | |
| 348 | scFv.LH-Fc | T394W/F405A | 1 | 61 | 169 | Qualitative evaluation of decrease in heteromultimers | | |
| 349 | scFv.LH-Fc | T366W/L368A/Y407V | 1 | 61 | 170 | Qualitative evaluation of decrease in heteromultimers | | |
| 350 | scFv.HL-Fc | - | 1 | 61 | 171 | scFv.HL-Fc control | | |
| 351 | scFv.HL-Fc | Q347R/K360E/D399V/F405T/K409W | 1 | 61 | 172 | Qualitative evaluation of decrease in heteromultimers | | |
| 352 | scFv.HL-Fc | E356K/K392D/D399K/K439E | 1 | 61 | 173 | Qualitative evaluation of decrease in heteromultimers | | |
| 353 | scFv.HL-Fc | T394W/F405A | 1 | 61 | 174 | Qualitative evaluation of decrease in heteromultimers | | |
| 354 | scFv.HL-Fc | T366W/L368A/Y407V | 1 | 61 | 175 | Qualitative evaluation of decrease in heteromultimers | | |
| 355 | L234F/L235E/P331S (suppress FcγR binding) Fc fragment | - | 1 | 61 | 599 | 25.5 | 51.4 | 23.1 |
| 356 | L234F/L235E/P331S (suppress FcγR binding) Fc fragment | T394W/F405A | 1 | 61 | 600 | 62.1 | 0.0 | 37.9 |
| 357 | L234F/L235E/P331S (suppress FcγR binding) Fc fragment | T366W/L368A/Y407V | 1 | 61 | 601 | 60.7 | 0.0 | 39.3 |
| 358 | L234F/L235E/P331S (suppress FcγR binding) Fc fragment | Q347R/K360E/D399V/F405T/K409W | 1 | 61 | 602 | 61.2 | 0.0 | 38.8 |
| 359 | L234F/L235E/P331S (suppress FcγR binding) Fc fragment | E356K/K392D/D399K/K439E | 1 | 61 | 603 | 62.3 | 0.0 | 37.7 |
| 360 | E345R/E430G/S440Y (promote hexamer formation) Fc fragment | - | 1 | 61 | 165 | 30.1 | 61.1 | 8.8 |
| 361 | E345R/E430G/S440Y (promote hexamer formation) Fc fragment | T394W/F405A | 1 | 61 | 604 | 86.1 | 0.0 | 13.9 |
| 362 | E345R/E430G/S440Y (promote hexamer formation) Fc fragment | T366W/L368A/Y407V | 1 | 61 | 605 | 87.2 | 0.0 | 12.8 |
| 363 | E345R/E430G/S440Y (promote hexamer formation) Fc fragment | Q347R/K360E/D399V/F405T/K409W | 1 | 61 | 606 | 92.0 | 0.0 | 8.0 |
| 364 | E345R/E430G/S440Y (promote hexamer formation) | - | 161 | 61 | - | Reference sample | | |
| 365 | E345R/E430G/S440Y (promote hexamer formation) | T394W/F405A | 162 | 61 | - | Reference sample | | |
| 366 | E345R/E430G/S440Y (promote hexamer formation) | T366W/L368A/Y407V | 163 | 61 | - | Reference sample | | |
| 367 | E345R/E430G/S440Y (promote hexamer formation) | Q347R/K360E/D399V/F405T/K409W | 164 | 61 | - | Reference sample | | |
| 368 | E345R/E430G/S440Y (promote hexamer formation) | - | - | - | 165 | Reference sample | | |
| 369 | WT-IgG1 | - | 1 | 61 | - | Reference sample | | |
| 370 | scFv.LH-Fc | - | - | - | 166 | Reference sample | | |
| 371 | scFv.LH-Fc | Q347R/K360E/D399V/F405T/K409W | - | - | 167 | Reference sample | | |
| 372 | scFv.LH-Fc | E356K/K392D/D399K/K439E | - | - | 168 | Reference sample | | |
| 373 | scFv.LH-Fc | T394W/F405A | - | - | 169 | Reference sample | | |
| 374 | scFv.LH-Fc | T366W/L368A/Y407V | - | - | 170 | Reference sample | | |
| 375 | scFv.HL-Fc | - | - | - | 171 | Reference sample | | |
| 376 | scFv.HL-Fc | Q347R/K360E/D399V/F405T/K409W | - | - | 172 | Reference sample | | |
| 377 | scFv.HL-Fc | E356K/K392D/D399K/K439E | - | - | 173 | Reference sample | | |
| 378 | scFv.HL-Fc | T394W/F405A | - | - | 174 | Reference sample | | |
| 379 | scFv.HL-Fc | T366W/L368A/Y407V | - | - | 175 | Reference sample | | |

### [Example 12] Further search for modifications promoting heavy-chain homodimer formation

The above Examples revealed that the amino acid modifications in Table 4 have particularly strong heavy-chain homodimerization-promoting ability, and have wide applicability regardless of antibody subclasses, formats, and Fc modifications in template antibodies. However, amino acid modifications surrounding these effective modification sets have not been fully searched yet, and there may be other effective modifications than the modification sets of Table 4. Thus, for the modifications of T394W/F405A (T394F/F405A), T366W/L368A/Y407V, Q347R/K360E/D399V/F405T/K409W, E345R/Q347R/K360D/T366V/D399M/Y407A/K409V, E356K/D399K/K409E/K439E, E356K/K392D/D399K/K409D, E356K/K392D/D399K/K409D, K392D/D399K/K409D, and E356K/K392D/D399K/K439E in Table 4, large-scale amino acid modification was performed on each modification position. Furthermore, their heavy-chain homodimer formation ability was evaluated by SEC analysis. The correspondences between the categories of effect of the modifications used for antibody expression, amino acid modifications, SEQ ID NOs, and area ratios are shown in Table 15 (the non-introduction of modifications is denoted as "-"). Antibody expression and analysis were performed according to the methods described in Example 1. Homodimers containing each Fc modification were prepared according to the method described in Example 3, and the monomer ratio, Tm, and yield (mg) in SEC analysis were evaluated by similar methods and are shown together in Table 15. As a result, modifications for which the heteromultimer (%) in Table 15 was lower than that of the control, 51.7%, were evaluated as effective modifications. These effective modifications at each position may also be able to be used in combination.

In this test, for samples with a low monomer ratio, a half antibody derived from the homodimer (a monomer composed of one heavy chain and one light chain) was contained in the peak of heteromultimer in the homodimer analysis. Therefore, the apparent ratio of heteromultimer in these samples may be higher than the true value. Nevertheless, since this was considered not to affect the determination of amino acid modifications with strong homodimerization ability and good physical properties, the single criterion of 51.7% or lower was used to evaluate the presence or absence of the ability to suppress heteromultimer formation.

Modifications that showed a heteromultimer ratio (%) of 10% or lower in Table 15 and were therefore viewed as more effective are shown in Table 16. Further, modifications that showed a heteromultimer ratio (%) of 0% in Table 15 and were therefore viewed as more effective are shown in Table 17. Furthermore, modifications that showed a heteromultimer ratio (%) of 0% in Table 15 and for which the monomer ratio, Tm, and yield (mg) upon preparation of the homodimer were 95% or higher, 65°C or higher, and 0.15 mg or higher in SEC analysis, respectively, and which are therefore particularly advantageous in terms of the heavy-chain homodimer formation ability and the physicochemical properties of the homodimer, are shown in Table 18. Meanwhile, the samples in Table 4 whose heteromultimer ratio (%) is 0% and for which the monomer ratio, Tm, and yield (mg) of the homodimer are 95% or higher, 65°C or higher, and 0.15 mg or higher, respectively, are Samples Nos. 80, 87, 92, 95, 97, 100, 102, 109, 111, 119, and 121. The modifications contained in these also have a superior heavy-chain homodimer formation ability and physicochemical properties.

The amino acid modifications and their combinations at the respective positions that have been shown to be effective in Tables 15-18 are expected to have similar properties, and therefore can be used as alternatives for the modification sets of T394W/F405A (T394F/F405A), T366W/L368A/Y407V, Q347R/K360E/D399V/F405T/K409W, E345R/Q347R/K360D/T366V/D399M/Y407A/K409V, E356K/D399K/K409E/K439E, E356K/K392D/D399K/K409D, K392D/D399K/K409D, and E356K/K392D/D399K/K439E shown in Tables 6 and 7, and may be applicable as Fc modifications for expressing multiple homodimers.

In addition, in each combination of modifications, amino acids that have shown to be effective at the same modified positions may also be able to be used in combination. Combinations of modifications that showed strong suppression of heteromeric association in Table 1 and amino acid modifications in Table 15 that suppressed the heteromultimer ratio below that of WT, 51.7%, are shown in Table 19. For example, for Modification Set No. 1, the effect was present with W, F, Y, and H at EU numbering position 394 and A, S, T, C, V, and G at position 405. Thus, 4 x 6 = 24 combinations of the modifications at the respective positions are also presumed to exert a certain effect. For the other Modification Sets, similarly, the combinations of the modifications shown in the table may also exert a certain heteromultimer-suppressing ability.

Similarly, amino acid modifications that suppressed the heteromultimer ratio to 10% or lower in Table 15 are shown in Table 20. Combinations of these respective amino acids may be more preferable heteromultimer-suppressing modifications.

Furthermore, amino acid modifications that suppressed the heteromultimer ratio to 0% in Table 15 are shown in Table 21. Combinations of these respective amino acids may be even more preferable heteromultimer-suppressing modifications.

**(Table 15) Results of analysis of the homodimer formation ability and physicochemical properties of antibodies with introduced amino acid modifications**

| Sample No. | Category of modification effect | Modifications that promote formation of heavy chain homodimers | Full-length heavy chain | Light chain | Fc fragment | Homo full-length heavy chain (%) | Hetero-multime r (%) | Homo Fc fragment (%) | Sample No. | Full-length heavy chain | Light chain | SEC monomer ratio (%) | Tm (°C) | Yield (mg) |
|---|---|---|---|---|---|---|---|---|---|---|---|---|---|---|
| | | | SEQ ID NO | SEQ ID NO | SEQ ID NO | | | | | SEQ ID NO | SEQ ID NO | | | |
| 1 | WT-IgG1 | - | 1 | 61 | 62 | 31.5 | 51.7 | 16.8 | 765 | 1 | 61 | 98.19 | 68.4 | 0.23 |
| 380 | Charge | E356R/D399K/K409E/ K439E | 176 | 61 | 62 | 61.1 | 0.0 | 38.9 | 766 | 176 | 61 | 98.02 | 63.6 | 0.22 |
| 381 | Charge | E356H/D399K/K409E/ K439E | 177 | 61 | 62 | 56.3 | 3.3 | 40.4 | 767 | 177 | 61 | 97.16 | 61.8 | 0.22 |
| 382 | Charge | E356K/D399R/K409E/ K439E | 178 | 61 | 62 | 61.8 | 0.0 | 38.2 | 768 | 178 | 61 | 98.83 | 51 | 0.23 |
| 383 | Charge | E356K/D399H/K409E/ K439E | 179 | 61 | 62 | 63.8 | 0.0 | 36.2 | 769 | 179 | 61 | 97.25 | 66 | 0.15 |
| 384 | Charge | E356K/D399K/K409D/ K439E | 180 | 61 | 62 | 64.3 | 0.0 | 35.7 | 770 | 180 | 61 | 97.73 | 63.6 | 0.15 |
| 385 | Charge | E356K/D399K/K409E/ K439D | 181 | 61 | 62 | 57.8 | 0.0 | 42.2 | 771 | 181 | 61 | 98.88 | 64.8 | 0.23 |
| 386 | Charge | E356K/D399R/K409D/ K439E | 182 | 61 | 62 | 58.7 | 0.0 | 41.3 | 772 | 182 | 61 | 99.1 | 66 | 0.24 |
| 387 | Charge | E356K/D399H/K409D/ K439E | 183 | 61 | 62 | 56.4 | 1.9 | 41.7 | 773 | 183 | 61 | 99.02 | 66 | 0.24 |
| 388 | Charge | E356R/K392D/D399K/ K439E | 184 | 61 | 62 | 59.7 | 2.6 | 37.7 | 774 | 184 | 61 | 98.74 | 67.8 | 0.2 |
| 389 | Charge | E356K/K392D/D399K/ K439D | 185 | 61 | 62 | 56.2 | 2.4 | 41.4 | 775 | 185 | 61 | 98.66 | 67.2 | 0.24 |
| 390 | Charge | E356K/K392D/D399R/ K439E | 186 | 61 | 62 | 57.6 | 2.5 | 39.8 | 776 | 186 | 61 | 98.79 | 69 | 0.22 |
| 391 | Charge | E356K/K392E/D399K/ K439E | 187 | 61 | 62 | 56.5 | 2.4 | 41.0 | 777 | 187 | 61 | 98.92 | 68.4 | 0.24 |
| 392 | Charge | K392D/D399R/K409D | 188 | 61 | 62 | 56.5 | 1.8 | 41.6 | 778 | 188 | 61 | 98.97 | 65.4 | 0.22 |
| 393 | Charge | K392D/D399K/K409E | 189 | 61 | 62 | 60.0 | 0.0 | 40.0 | 779 | 189 | 61 | 98.94 | 66.6 | 0.21 |
| 394 | Charge | K392E/D399K/K409D | 190 | 61 | 62 | 54.5 | 1.9 | 43.6 | 780 | 190 | 61 | 99.27 | 64.2 | 0.24 |
| 395 | Charge | E356K/K392D/D399K/ K409E | 191 | 61 | 62 | 57.7 | 0.0 | 42.3 | 781 | 191 | 61 | 99.17 | 64.2 | 0.24 |
| 396 | Charge | E356K/K392D/D399R/ K409D | 192 | 61 | 62 | 59.6 | 0.0 | 40.4 | 782 | 192 | 61 | 99.32 | 62.4 | 0.21 |
| 397 | Charge | E356R/K392D/D399K/ K409D | 193 | 61 | 62 | 61.1 | 0.0 | 38.9 | 783 | 193 | 61 | 99.4 | 61.2 | 0.2 |
| 398 | Charge | E356H/K392D/D399K/ K409D | 194 | 61 | 62 | 59.4 | 0.0 | 40.6 | 784 | 194 | 61 | 99.16 | 61.8 | 0.23 |
| 399 | Charge | E356K/K392E/D399K/ K409D | 195 | 61 | 62 | 59.5 | 0.0 | 40.5 | 785 | 195 | 61 | 97.87 | 61.8 | 0.21 |
| 400 | Charge | E356R/K392D/D399K/ K439D | 196 | 61 | 62 | 57.0 | 2.7 | 40.3 | 786 | 196 | 61 | 98.69 | 66.6 | 0.21 |
| 401 | Charge | E356K/K392D/D399H/ K409D | 197 | 61 | 62 | 61.2 | 0.0 | 38.8 | 787 | 197 | 61 | 98.75 | 62.4 | 0.18 |
| 402 | Charge | E356R/D399K/K409E/ K439D | 198 | 61 | 62 | 56.7 | 0.0 | 43.3 | 788 | 198 | 61 | 98.84 | 63.6 | 0.21 |
| 403 | Charge | E356H/D399K/K409E/ K439D | 199 | 61 | 62 | 54.9 | 2.3 | 42.7 | 789 | 199 | 61 | 98.33 | 61.2 | 0.25 |
| 404 | Steric hindrance + Charge | Q347R1K360D/T366V I D399M/Y407A/K409V | 200 | 61 | 62 | 70.6 | 0.0 | 29.4 | 790 | 200 | 61 | 94.56 | 68.4 | 0.09 |
| 405 | Steric hindrance + Charge | E345R/Q347K/K360D/ T366V/D399M/Y407A/ K409V | 201 | 61 | 62 | 68.5 | 0.0 | 31.5 | 791 | 201 | 61 | 97.44 | 68.4 | 0.15 |
| 406 | Steric hindrance + Charge | E345R/Q347R/K360E/ T366V/D399M/Y407A/ K409V | 202 | 61 | 62 | 65.2 | 0.0 | 34.8 | 792 | 202 | 61 | 97.72 | 68.4 | 0.19 |
| 407 | Steric hindrance + Charge | Q347K/K360D/T366V/ D399M/Y407A/K409V | 203 | 61 | 62 | 63.8 | 0.0 | 36.2 | 793 | 203 | 61 | 97.64 | 68.4 | 0.19 |
| 408 | Steric hindrance + Charge | Q347R/K360E/T366V/ D399M/Y407A/K409V | 204 | 61 | 62 | 64.0 | 0.0 | 36.0 | 794 | 204 | 61 | 97.39 | 68.4 | 0.18 |
| 409 | Steric hindrance + Charge | Q347K/K360E/T366V/ D399M/Y407A/K409V | 205 | 61 | 62 | 66.6 | 0.0 | 33.4 | 795 | 205 | 61 | 97.43 | 68.4 | 0.18 |
| 410 | Steric hindrance + Charge | E345R/Q347R/K360D/ T366V/D399Q/Y407A/ K409Q | 206 | 61 | 62 | 64.4 | 0.3 | 35.3 | 796 | 206 | 61 | 97.83 | 66 | 0.21 |
| 411 | Steric hindrance + Charge | E345R/Q347R/K360D/ T366V/D399N/Y407A/ K409Q | 207 | 61 | 62 | 65.7 | 0.0 | 34.3 | 797 | 207 | 61 | 96.8 | 67.2 | 0.1 |
| 412 | Steric hindrance + Charge | E345R/Q347R/K360D/ T366V/D399H/Y407A/ K409Q | 208 | 61 | 62 | 63.7 | 0.0 | 36.3 | 798 | 208 | 61 | 98.57 | 68.4 | 0.21 |
| 413 | Steric hindrance + Charge | E345R/Q347R/K360D/ T366V/D399I/Y407A/ K409Q | 209 | 61 | 62 | 64.1 | 0.0 | 35.9 | 799 | 209 | 61 | 98.96 | 65.4 | 0.21 |
| 414 | Steric hindrance + Charge | E345R/Q347R/K360D/ T366V/D399F/Y407A/ K409Q | 210 | 61 | 62 | 64.1 | 0.0 | 35.9 | 800 | 210 | 61 | 98.2 | 63 | 0.19 |
| 415 | Steric hindrance + Charge | E345R1Q347R1K360D/ T366V/D399Y/Y407A/ K409Q | 211 | 61 | 62 | 65.9 | 0.0 | 34.1 | 801 | 211 | 61 | 98.36 | 63.6 | 0.19 |
| 416 | Steric hindrance + Charge | E345R/Q347R/K360D/ T366V/D399T/Y407A/ K409Q | 212 | 61 | 62 | 67.8 | 0.0 | 32.2 | 802 | 212 | 61 | 98.38 | 68.4 | 0.19 |
| 417 | Steric hindrance + Charge | E345R1Q347R1K360D/ T366V/D399S/Y407A/ K409Q | 213 | 61 | 62 | 65.2 | 0.0 | 34.8 | 803 | 213 | 61 | 98.76 | 68.4 | 0.22 |
| 418 | Steric hindrance + Charge | E345R/Q347R/K360D/ T366V/D399Q/Y407A/ K409N | 214 | 61 | 62 | 36.0 | 24.9 | 39.1 | 804 | 214 | 61 | 56.71 | 54 | 0.22 |
| 419 | Steric hindrance + Charge | E345R/Q347R/K360D/ T366V/D399H/Y407A/ K409N | 215 | 61 | 62 | 22.0 | 27.9 | 50.0 | 805 | 215 | 61 | 55.72 | 50.4 | 0.2 |
| 420 | Steric hindrance + Charge | E345R/Q347R/K360D/ T366V/D399V/Y407A/ K409N | 216 | 61 | 62 | 46.6 | 12.7 | 40.7 | 806 | 216 | 61 | 80.31 | 55.2 | 0.2 |
| 421 | Steric hindrance + Charge | E345R/Q347R/K360D/ T366V/D399L/Y407A/ K409N | 217 | 61 | 62 | 50.6 | 11.1 | 38.3 | 807 | 217 | 61 | 82.56 | 51.6 | 0.18 |
| 422 | Steric hindrance + Charge | E345R/Q347R/K360D/ T366V/D399F/Y407A/ K409N | 218 | 61 | 62 | 51.5 | 4.2 | 44.2 | 808 | 218 | 61 | 93.41 | 50.4 | 0.2 |
| 423 | Steric hindrance + Charge | E345R/Q347R/K360D/ T366V/D399Y/Y407A/ K409N | 219 | 61 | 62 | 31.9 | 16.3 | 51.9 | 809 | 219 | 61 | 74.87 | 62.4 | 0.18 |
| 424 | Steric hindrance + Charge | E345R/Q347R/K360D/ T366V/D399T/Y407A/ K409N | 220 | 61 | 62 | 48.6 | 13.7 | 37.6 | 810 | 220 | 61 | 79.22 | 53.4 | 0.23 |
| 425 | Steric hindrance + Charge | E345R1Q347R1K360D/ T366V/D399S/Y407A/ K409N | 221 | 61 | 62 | 28.4 | 27.6 | 44.0 | 811 | 221 | 61 | 50.49 | 52.2 | 0.23 |
| 426 | Steric hindrance + Charge | E345R/Q347R/K360D/ T366V/D399Q/Y407A/ K409H | 222 | 61 | 62 | 57.9 | 1.7 | 40.4 | 812 | 222 | 61 | 96.33 | 65.4 | 0.22 |
| 427 | Steric hindrance + Charge | E345R/Q347R/K360D/ T366V/D399N/Y407A/ K409H | 223 | 61 | 62 | 59.2 | 1.1 | 39.7 | 813 | 223 | 61 | 97.13 | 64.8 | 0.21 |
| 428 | Steric hindrance + Charge | E345R/Q347R/K360D/ T366V/D399H/Y407A/ K409H | 224 | 61 | 62 | 59.3 | 1.5 | 39.2 | 814 | 224 | 61 | 96.11 | 64.2 | 0.21 |
| 429 | Steric hindrance + Charge | E345R1Q347R1K360D/ T366V/D399V/Y407A/ K409H | 225 | 61 | 62 | 65.7 | 0.0 | 34.3 | 815 | 225 | 61 | 98.81 | 67.2 | 0.2 |
| 430 | Steric hindrance + Charge | E345R/Q347R/K360D/ T366V/D399L/Y407A/ K409H | 226 | 61 | 62 | 60.7 | 0.0 | 39.3 | 816 | 226 | 61 | 98.22 | 64.8 | 0.21 |
| 431 | Steric hindrance + Charge | E345R1Q347R1K360D/ T366V/D399F/Y407A/ K409H | 227 | 61 | 62 | 59.3 | 1.4 | 39.3 | 817 | 227 | 61 | 95.94 | 59.4 | 0.22 |
| 432 | Steric hindrance + Charge | E345R/Q347R/K360D/ T366V/D399Y/Y407A/ K409H | 228 | 61 | 62 | 59.9 | 1.6 | 38.5 | 818 | 228 | 61 | 96.08 | 60.6 | 0.18 |
| 433 | Steric hindrance + Charge | E345R/Q347R/K360D/ T366V/D399T/Y407A/ K409H | 229 | 61 | 62 | 65.1 | 0.0 | 34.9 | 819 | 229 | 61 | 98.64 | 67.8 | 0.2 |
| 434 | Steric hindrance + Charge | E345R/Q347R/K360D/ T366V/D399S/Y407A/ K409H | 230 | 61 | 62 | 62.2 | 0.0 | 37.8 | 820 | 230 | 61 | 98.28 | 67.8 | 0.2 |
| 435 | Steric hindrance + Charge | E345R1Q347R1K360D/ T366V/D399Q/Y407A/ K409V | 231 | 61 | 62 | 64.5 | 0.0 | 35.5 | 821 | 231 | 61 | 98.23 | 69 | 0.2 |
| 436 | Steric hindrance + Charge | E345R/Q347R/K360D/ T366V/D399N/Y407A/ K409V | 232 | 61 | 62 | 65.4 | 0.0 | 34.6 | 822 | 232 | 61 | 98.41 | 68.4 | 0.2 |
| 437 | Steric hindrance + Charge | E345R/Q347R/K360D/ T366V/D399H/Y407A/ K409V | 233 | 61 | 62 | 62.6 | 0.0 | 37.4 | 823 | 233 | 61 | 98.66 | 67.8 | 0.2 |
| 438 | Steric hindrance + Charge | E345R/Q347R/K360D/ T366V/D399V/Y407A/ K409V | 234 | 61 | 62 | 64.3 | 0.0 | 35.7 | 824 | 234 | 61 | 98.42 | 68.4 | 0.22 |
| 439 | Steric hindrance + Charge | E345R/Q347R/K360D/ T366V/D399L/Y407A/ K409V | 235 | 61 | 62 | 61.7 | 0.0 | 38.3 | 825 | 235 | 61 | 97.67 | 67.2 | 0.2 |
| 440 | Steric hindrance + Charge | E345R/Q347R/K360D/ T366V/D3991/Y407A/ K409V | 236 | 61 | 62 | 62.9 | 0.0 | 37.1 | 826 | 236 | 61 | 98.16 | 68.4 | 0.21 |
| 441 | Steric hindrance + Charge | E345R/Q347R/K360D/ T366V/D399F/Y407A/ K409V | 237 | 61 | 62 | 62.6 | 0.0 | 37.4 | 827 | 237 | 61 | 98.26 | 65.4 | 0.2 |
| 442 | Steric hindrance + Charge | E345R/Q347R/K360D/ T366V/D399Y/Y407A/ K409V | 238 | 61 | 62 | 63.7 | 0.0 | 36.3 | 828 | 238 | 61 | 98.46 | 67.2 | 0.21 |
| 443 | Steric hindrance + Charge | E345R/Q347R/K360D/ T366V/D399T/Y407A/ K409V | 239 | 61 | 62 | 65.2 | 0.0 | 34.8 | 829 | 239 | 61 | 98.22 | 69 | 0.19 |
| 444 | Steric hindrance + Charge | E345R/Q347R/K360D/ T366V/D399S/Y407A/ K409V | 240 | 61 | 62 | 66.1 | 0.0 | 33.9 | 830 | 240 | 61 | 98.19 | 69 | 0.18 |
| 445 | Steric hindrance + Charge | E345R1Q347R1K360D/ T366V/D399Q/Y407A/ K409L | 241 | 61 | 62 | 66.9 | 0.0 | 33.1 | 831 | 241 | 61 | 98.01 | 69 | 0.19 |
| 446 | Steric hindrance + Charge | E345R/Q347R/K360D/ T366V/D399N/Y407A/ K409L | 242 | 61 | 62 | 61.5 | 0.0 | 38.5 | 832 | 242 | 61 | 98.01 | 68.4 | 0.18 |
| 447 | Steric hindrance + Charge | E345R/Q347R/K360D/ T366V/D399H/Y407A/ K409L | 243 | 61 | 62 | 62.2 | 0.0 | 37.8 | 833 | 243 | 61 | 98.41 | 67.8 | 0.2 |
| 448 | Steric hindrance + Charge | E345R/Q347R/K360D/ T366V/D3991/Y407A/ K409L | 244 | 61 | 62 | 61.5 | 0.0 | 38.5 | 834 | 244 | 61 | 97.25 | 67.8 | 0.19 |
| 449 | Steric hindrance + Charge | E345R1Q347R1K360D/ T366V/D399F/Y407A/ K409L | 245 | 61 | 62 | 60.9 | 0.0 | 39.1 | 835 | 245 | 61 | 97.83 | 66 | 0.19 |
| 450 | Steric hindrance + Charge | E345R/Q347R/K360D/ T366V/D399T/Y407A/ K409L | 246 | 61 | 62 | 61.2 | 0.0 | 38.8 | 836 | 246 | 61 | 97.48 | 69 | 0.21 |
| 451 | Steric hindrance + Charge | E345R/Q347R/K360D/ T366V/D399S/Y407A/ K409L | 247 | 61 | 62 | 62.2 | 0.0 | 37.8 | 837 | 247 | 61 | 98.08 | 69.6 | 0.21 |
| 452 | Steric hindrance + Charge | E345R/Q347R/K360D/ T366V/D399Q/Y407A/ K409I | 248 | 61 | 62 | 66.2 | 0.0 | 33.8 | 838 | 248 | 61 | 97.82 | 68.4 | 0.17 |
| 453 | Steric hindrance + Charge | E345R1Q347R1K360D/ T366V/D399N/Y407A/ K409I | 249 | 61 | 62 | 63.0 | 0.0 | 37.0 | 839 | 249 | 61 | 98.15 | 68.4 | 0.17 |
| 454 | Steric hindrance + Charge | E345R/Q347R/K360D/ T366V/D399H/Y407A/ K4091 | 250 | 61 | 62 | 62.1 | 0.0 | 37.9 | 840 | 250 | 61 | 98.95 | 67.2 | 0.21 |
| 455 | Steric hindrance + Charge | E345R/Q347R/K360D/ T366V/D399V/Y407A/ K409I | 251 | 61 | 62 | 65.1 | 0.0 | 34.9 | 841 | 251 | 61 | 97.95 | 68.4 | 0.21 |
| 456 | Steric hindrance + Charge | E345R/Q347R/K360D/ T366V/D399L/Y407A/ K4091 | 252 | 61 | 62 | 59.0 | 0.0 | 41.0 | 842 | 252 | 61 | 97.02 | 65.4 | 0.17 |
| 457 | Steric hindrance + Charge | E345R1Q347R1K360D/ T366V/D399F/Y407A/ K409I | 253 | 61 | 62 | 61.0 | 0.0 | 39.0 | 843 | 253 | 61 | 98.53 | 64.8 | 0.21 |
| 458 | Steric hindrance + Charge | E345R/Q347R/K360D/ T366V/D399Y/Y407A/ K409I | 254 | 61 | 62 | 62.4 | 0.0 | 37.6 | 844 | 254 | 61 | 98.49 | 67.2 | 0.21 |
| 459 | Steric hindrance + Charge | E345R1Q347R1K360D/ T366V/D399T/Y407A/ K409I | 255 | 61 | 62 | 60.5 | 0.0 | 39.5 | 845 | 255 | 61 | 98.24 | 69 | 0.21 |
| 460 | Steric hindrance + Charge | E345R/Q347R/K360D/ T366V/D399S/Y407A/ K4091 | 256 | 61 | 62 | 65.6 | 0.0 | 34.4 | 846 | 256 | 61 | 97.66 | 69 | 0.16 |
| 461 | Steric hindrance + Charge | E345R/Q347R/K360D/ T366V/D399Q/Y407A/ K409F | 257 | 61 | 62 | 63.4 | 0.0 | 36.6 | 847 | 257 | 61 | 98.6 | 67.2 | 0.21 |
| 462 | Steric hindrance + Charge | E345R/Q347R/K360D/ T366V/D399N/Y407A/ K409F | 258 | 61 | 62 | 65.9 | 0.0 | 34.1 | 848 | 258 | 61 | 98.68 | 66.6 | 0.2 |
| 463 | Steric hindrance + Charge | E345R1Q347R1K360D/ T366V/D399H/Y407A/ K409F | 259 | 61 | 62 | 60.5 | 0.0 | 39.5 | 849 | 259 | 61 | 98.77 | 67.2 | 0.2 |
| 464 | Steric hindrance + Charge | E345R/Q347R/K360D/ T366V/D399V/Y407A/ K409F | 260 | 61 | 62 | 62.3 | 0.0 | 37.7 | 850 | 260 | 61 | 98.54 | 67.2 | 0.21 |
| 465 | Steric hindrance + Charge | E345R/Q347R/K360D/ T366V/D399L/Y407A/ K409F | 261 | 61 | 62 | 59.8 | 0.0 | 40.2 | 851 | 261 | 61 | 98.63 | 66 | 0.18 |
| 466 | Steric hindrance + Charge | E345R/Q347R/K360D/ T366V/D3991/Y407A/ K409F | 262 | 61 | 62 | 61.5 | 0.0 | 38.5 | 852 | 262 | 61 | 98.5 | 66 | 0.22 |
| 467 | Steric hindrance + Charge | E345R1Q347R1K360D/ T366V/D399F/Y407A/ K409F | 263 | 61 | 62 | 60.3 | 0.0 | 39.7 | 853 | 263 | 61 | 98.73 | 63 | 0.19 |
| 468 | Steric hindrance + Charge | E345R/Q347R/K360D/ T366V/D399Y/Y407A/ K409F | 264 | 61 | 62 | 66.0 | 0.0 | 34.0 | 854 | 264 | 61 | 98.86 | 64.8 | 0.19 |
| 469 | Steric hindrance + Charge | E345R/Q347R/K360D/ T366V/D399T/Y407A/ K409F | 265 | 61 | 62 | 64.2 | 0.0 | 35.8 | 855 | 265 | 61 | 98.56 | 67.2 | 0.22 |
| 470 | Steric hindrance + Charge | E345R/Q347R/K360D/ T366V/D399Q/Y407A/ K409Y | 266 | 61 | 62 | 59.8 | 1.8 | 38.5 | 856 | 266 | 61 | 95.94 | 64.8 | 0.24 |
| 471 | Steric hindrance + Charge | E345R1Q347R1K360D/ T366V/D399H/Y407A/ K409Y | 267 | 61 | 62 | 59.3 | 1.4 | 39.2 | 857 | 267 | 61 | 96 | 64.2 | 0.13 |
| 472 | Steric hindrance + Charge | E345R/Q347R/K360D/ T366V/D399V/Y407A/ K409Y | 268 | 61 | 62 | 58.8 | 0.0 | 41.2 | 858 | 268 | 61 | 98.16 | 66.6 | 0.23 |
| 473 | Steric hindrance + Charge | E345R1Q347R1K360D/ T366V/D399L/Y407A/ K409Y | 269 | 61 | 62 | 57.7 | 0.0 | 42.3 | 859 | 269 | 61 | 96.91 | 64.2 | 0.21 |
| 474 | Steric hindrance + Charge | E345R/Q347R/K360D/ T366V/D3991/Y407A/ K409Y | 270 | 61 | 62 | 65.9 | 0.0 | 34.1 | 860 | 270 | 61 | 97.86 | 64.8 | 0.19 |
| 475 | Steric hindrance + Charge | E345R/Q347R/K360D/ T366V/D399F/Y407A/ K409Y | 271 | 61 | 62 | 59.8 | 0.0 | 40.2 | 861 | 271 | 61 | 97.35 | 60.6 | 0.18 |
| 476 | Steric hindrance + Charge | E345R/Q347R/K360D/ T366V/D399Y/Y407A/ K409Y | 272 | 61 | 62 | 66.7 | 1.3 | 32.1 | 862 | 272 | 61 | 96.17 | 61.8 | 0.15 |
| 477 | Steric hindrance + Charge | E345R1Q347R1K360D/ T366V/D399S/Y407A/ K409Y | 273 | 61 | 62 | 64.6 | 0.0 | 35.4 | 863 | 273 | 61 | 97.16 | 66 | 0.21 |
| 478 | Steric hindrance + Charge | E345R/Q347R/K360D/ T366V/D399N/Y407A/ K409T | 274 | 61 | 62 | 66.7 | 0.0 | 33.3 | 864 | 274 | 61 | 97.13 | 67.2 | 0.19 |
| 479 | Steric hindrance + Charge | E345R/Q347R/K360D/ T366V/D399H/Y407A/ K409T | 275 | 61 | 62 | 61.2 | 2.1 | 36.7 | 865 | 275 | 61 | 94.75 | 65.4 | 0.19 |
| 480 | Steric hindrance + Charge | E345R/Q347R/K360D/ T366V/D399V/Y407A/ K409T | 276 | 61 | 62 | 63.2 | 0.0 | 36.8 | 866 | 276 | 61 | 97.89 | 67.2 | 0.23 |
| 481 | Steric hindrance + Charge | E345R1Q347R1K360D/ T366V/D399L/Y407A/ K409T | 277 | 61 | 62 | 68.0 | 0.0 | 32.0 | 867 | 277 | 61 | 96.8 | 51 | 0.19 |
| 482 | Steric hindrance + Charge | E345R/Q347R/K360D/ T366V/D3991/Y407A/ K409T | 278 | 61 | 62 | 72.4 | 0.0 | 27.6 | 868 | 278 | 61 | 95.62 | 67.2 | 0.09 |
| 483 | Steric hindrance + Charge | E345R/Q347R/K360D/ T366V/D399F/Y407A/ K409T | 279 | 61 | 62 | 61.6 | 1.1 | 37.3 | 869 | 279 | 61 | 96.53 | 63 | 0.22 |
| 484 | Steric hindrance + Charge | E345R/Q347R/K360D/ T366V/D399Y/Y407A/ K409T | 280 | 61 | 62 | 62.2 | 1.1 | 36.7 | 870 | 280 | 61 | 96.78 | 64.2 | 0.22 |
| 485 | Steric hindrance + Charge | E345R1Q347R1K360D/ T366V/D399T/Y407A/ K409T | 281 | 61 | 62 | 66.8 | 0.0 | 33.2 | 871 | 281 | 61 | 97.6 | 67.8 | 0.22 |
| 486 | Steric hindrance + Charge | E345R/Q347R/K360D/ T366V/D399S/Y407A/ K409T | 282 | 61 | 62 | 64.9 | 0.0 | 35.1 | 872 | 282 | 61 | 97.27 | 68.4 | 0.16 |
| 487 | Steric hindrance + Charge | E345R1Q347R1K360D/ T366V/D399Q/Y407A/ K409S | 283 | 61 | 62 | 62.9 | 0.0 | 37.1 | 873 | 283 | 61 | 98.65 | 69 | 0.23 |
| 488 | Steric hindrance + Charge | E345R/Q347R/K360D/ T366V/D399N/Y407A/ K409S | 284 | 61 | 62 | 61.1 | 0.0 | 38.9 | 874 | 284 | 61 | 98.77 | 67.8 | 0.24 |
| 489 | Steric hindrance + Charge | E345R/Q347R/K360D/ T366V/D399H/Y407A/ K409S | 285 | 61 | 62 | 60.8 | 0.0 | 39.2 | 875 | 285 | 61 | 97.45 | 65.4 | 0.24 |
| 490 | Steric hindrance + Charge | E345R/Q347R/K360D/ T366V/D399V/Y407A/ K409S | 286 | 61 | 62 | 64.5 | 0.0 | 35.5 | 876 | 286 | 61 | 98.58 | 67.2 | 0.21 |
| 491 | Steric hindrance + Charge | E345R1Q347R1K360D/ T366V/D399L/Y407A/ K409S | 287 | 61 | 62 | 60.9 | 0.0 | 39.1 | 877 | 287 | 61 | 98.74 | 67.2 | 0.22 |
| 492 | Steric hindrance + Charge | E345R/Q347R/K360D/ T366V/D3991/Y407A/ K409S | 288 | 61 | 62 | 60.8 | 0.0 | 39.2 | 878 | 288 | 61 | 98.89 | 66.6 | 0.23 |
| 493 | Steric hindrance + Charge | E345R/Q347R/K360D/ T366V/D399F/Y407A/ K409S | 289 | 61 | 62 | 59.9 | 0.0 | 40.1 | 879 | 289 | 61 | 98.26 | 61.8 | 0.22 |
| 494 | Steric hindrance + Charge | E345R/Q347R/K360D/ T366V/D399Y/Y407A/ K409S | 290 | 61 | 62 | 65.7 | 0.0 | 34.3 | 880 | 290 | 61 | 97.06 | 62.4 | 0.15 |
| 495 | Steric hindrance + Charge | E345R1Q347R1K360D/ T366V/D399T/Y407A/ K409S | 291 | 61 | 62 | 67.0 | 0.0 | 33.0 | 881 | 291 | 61 | 98.57 | 68.4 | 0.23 |
| 496 | Steric hindrance + Charge | E345R/Q347R/K360D/ T366V/D399S/Y407A/ K409S | 292 | 61 | 62 | 61.5 | 0.0 | 38.5 | 882 | 292 | 61 | 98.73 | 53.4 | 0.22 |
| 497 | Steric hindrance + Charge | E345R/Q347R/K360D/ T366V/D399M/Y407A/ K409Q | 293 | 61 | 62 | 65.0 | 0.0 | 35.0 | 883 | 293 | 61 | 97.5 | 66.6 | 0.16 |
| 498 | Steric hindrance + Charge | E345R/Q347R/K360D/ T366V/D399M/Y407A/ K409N | 294 | 61 | 62 | 47.6 | 14.3 | 38.1 | 884 | 294 | 61 | 75.67 | 55.8 | 0.22 |
| 499 | Steric hindrance + Charge | E345R1Q347R1K360D/ T366V/D399M/Y407A/ K409H | 295 | 61 | 62 | 54.4 | 0.7 | 44.9 | 885 | 295 | 61 | 95.32 | 65.4 | 0.22 |
| 500 | Steric hindrance + Charge | E345R/Q347R/K360D/ T366V/D399M/Y407A/ K409L | 296 | 61 | 62 | 59.0 | 0.0 | 41.0 | 886 | 296 | 61 | 97.5 | 68.4 | 0.22 |
| 501 | Steric hindrance + Charge | E345R1Q347R1K360D/ T366V/D399M/Y407A/ K409I | 297 | 61 | 62 | 59.0 | 0.0 | 41.0 | 887 | 297 | 61 | 97.7 | 67.2 | 0.23 |
| 502 | Steric hindrance + Charge | E345R/Q347R/K360D/ T366V/D399M/Y407A/ K409F | 298 | 61 | 62 | 64.9 | 0.0 | 35.1 | 888 | 298 | 61 | 98.14 | 66.6 | 0.22 |
| 503 | Steric hindrance + Charge | E345R/Q347R/K360D/ T366V/D399M/Y407A/ K409Y | 299 | 61 | 62 | 60.5 | 0.0 | 39.5 | 889 | 299 | 61 | 97.03 | 65.4 | 0.22 |
| 504 | Steric hindrance + Charge | E345R/Q347R/K360D/ T366V/D399M/Y407A/ K409T | 300 | 61 | 62 | 62.1 | 0.0 | 37.9 | 890 | 300 | 61 | 98.04 | 68.4 | 0.25 |
| 505 | Steric hindrance + Charge | E345R1Q347R1K360D/ T366V/D399M/Y407A/ K409S | 301 | 61 | 62 | 61.3 | 0.0 | 38.7 | 891 | 301 | 61 | 98.79 | 68.4 | 0.25 |
| 506 | Steric hindrance + Charge | E345R/Q347R/K360D/ T366V/D399V/Y407A/ K409Q | 302 | 61 | 62 | 65.0 | 0.0 | 35.0 | 892 | 302 | 61 | 98.63 | 67.2 | 0.22 |
| 507 | Steric hindrance + Charge | E345R/Q347R/K360D/ T366V/D399L/Y407A/ K409Q | 303 | 61 | 62 | 67.2 | 0.0 | 32.8 | 893 | 303 | 61 | 97.55 | 64.2 | 0.19 |
| 508 | Steric hindrance + Charge | E345R/Q347R/K360D/ T366V/D399I/Y407A/ K409N | 304 | 61 | 62 | 47.3 | 9.0 | 43.7 | 894 | 304 | 61 | 86.62 | 52.2 | 0.19 |
| 509 | Steric hindrance + Charge | E345R1Q347R1K360D/ T366V/D3991/Y407A/ K409H | 305 | 61 | 62 | 60.9 | 0.0 | 39.1 | 895 | 305 | 61 | 98.46 | 64.8 | 0.24 |
| 510 | Steric hindrance + Charge | E345R/Q347R/K360D/ T366V/D399V/Y407A/ K409L | 306 | 61 | 62 | 61.1 | 0.0 | 38.9 | 896 | 306 | 61 | 97.51 | 69 | 0.22 |
| 511 | Steric hindrance + Charge | E345R/Q347R/K360D/ T366V/D399Y/Y407A/ K409L | 307 | 61 | 62 | 66.4 | 0.0 | 33.6 | 897 | 307 | 61 | 97.98 | 67.2 | 0.22 |
| 512 | Steric hindrance + Charge | E345R/Q347R/K360D/ T366V/D399S/Y407A/ K409F | 308 | 61 | 62 | 63.9 | 0.0 | 36.1 | 898 | 308 | 61 | 98.3 | 67.8 | 0.23 |
| 513 | Steric hindrance + Charge | E345R1Q347R1K360D/ T366V/D399N/Y407A/ K409Y | 309 | 61 | 62 | 63.4 | 0.6 | 35.9 | 899 | 309 | 61 | 96.94 | 64.8 | 0.21 |
| 514 | Steric hindrance + Charge | E345R/Q347R/K360D/ T366V/D399T/Y407A/ K409Y | 310 | 61 | 62 | 64.0 | 0.0 | 36.0 | 900 | 310 | 61 | 97.62 | 66.6 | 0.21 |
| 515 | Steric hindrance + Charge | E345R1Q347R1K360D/ T366V/D399Q/Y407A/ K409T | 311 | 61 | 62 | 64.7 | 0.0 | 35.3 | 901 | 311 | 61 | 96.89 | 67.8 | 0.23 |
| 516 | Steric hindrance + Charge | E345R/Q347R/K360D/ T366V/D399N/Y407A/ K409N | 312 | 61 | 62 | 26.0 | 16.6 | 57.4 | 902 | 312 | 61 | 61.5 | 51.6 | 0.23 |
| 517 | Steric hindrance + Charge | Q347R/K360E/D399V/ F405T/K409F | 313 | 61 | 62 | 64.0 | 0.0 | 36.0 | 903 | 313 | 61 | 98.54 | 68.4 | 0.2 |
| 518 | Steric hindrance + Charge | Q347K/K360E/D399V/ F405T/K409W | 314 | 61 | 62 | 62.0 | 0.0 | 38.0 | 904 | 314 | 61 | 98.56 | 69.6 | 0.24 |
| 519 | Steric hindrance + Charge | Q347Y/K360E/D399V/ F405T/K409W | 315 | 61 | 62 | 61.7 | 0.0 | 38.3 | 905 | 315 | 61 | 98.39 | 68.4 | 0.25 |
| 520 | Steric hindrance + Charge | Q347R/K360D/D399V/ F405T/K409W | 316 | 61 | 62 | 58.5 | 0.0 | 41.5 | 906 | 316 | 61 | 98.43 | 68.4 | 0.25 |
| 521 | Steric hindrance + Charge | Q347K/K360D/D399V/ F405T/K409W | 317 | 61 | 62 | 63.2 | 0.0 | 36.8 | 907 | 317 | 61 | 97.86 | 69 | 0.22 |
| 522 | Steric hindrance + Charge | Q347H/K360D/D399V/ F405T/K409W | 318 | 61 | 62 | 64.0 | 0.0 | 36.0 | 908 | 318 | 61 | 98.1 | 67.8 | 0.22 |
| 523 | Steric hindrance + Charge | Q347Y/K360D/D399V/ F405T/K409W | 319 | 61 | 62 | 59.1 | 0.9 | 40.0 | 909 | 319 | 61 | 97.64 | 66.6 | 0.24 |
| 524 | Steric hindrance + Charge | Q347R/K360E/D399L/ F405T/K409W | 320 | 61 | 62 | 58.1 | 0.0 | 41.9 | 910 | 320 | 61 | 98.62 | 69 | 0.25 |
| 525 | Steric hindrance + Charge | Q347R/K360E/D3991/F 405T/K409W | 321 | 61 | 62 | 60.9 | 0.0 | 39.1 | 911 | 321 | 61 | 98.75 | 69 | 0.22 |
| 526 | Steric hindrance + Charge | Q347R/K360E/D399M/ F405T/K409W | 322 | 61 | 62 | 61.9 | 0.0 | 38.1 | 912 | 322 | 61 | 98.69 | 69 | 0.23 |
| 527 | Steric hindrance + Charge | Q347R/K360E/D399S/ F405T/K409W | 323 | 61 | 62 | 62.4 | 0.0 | 37.6 | 913 | 323 | 61 | 98.64 | 69 | 0.24 |
| 528 | Steric hindrance + Charge | Q347R/K360E/D399T/ F405T/K409W | 324 | 61 | 62 | 61.8 | 0.0 | 38.2 | 914 | 324 | 61 | 98.56 | 69 | 0.24 |
| 529 | Steric hindrance + Charge + Disulfide bond | Q347R/K360E/D399C/ F405T/K409W | 325 | 61 | 62 | 61.3 | 0.0 | 38.7 | 915 | 325 | 61 | 98.55 | 69.6 | 0.23 |
| 530 | Steric hindrance + Charge | Q347R/K360E/D399H/ F405T/K409W | 326 | 61 | 62 | 59.2 | 0.0 | 40.8 | 916 | 326 | 61 | 96.9 | 67.8 | 0.2 |
| 531 | Steric hindrance + Charge | Q347R/K360E/D399V/ F405A/K409W | 327 | 61 | 62 | 62.5 | 0.0 | 37.5 | 917 | 327 | 61 | 98.07 | 67.8 | 0.21 |
| 532 | Steric hindrance + Charge | Q347R/K360E/D399V/ F405V/K409W | 328 | 61 | 62 | 62.8 | 0.0 | 37.2 | 918 | 328 | 61 | 98.66 | 69 | 0.23 |
| 533 | Steric hindrance + Charge | Q347R/K360E/D399V/ F405S/K409W | 329 | 61 | 62 | 62.3 | 0.2 | 37.4 | 919 | 329 | 61 | 97.95 | 67.8 | 0.25 |
| 534 | Steric hindrance + Charge + Disulfide bond | Q347R/K360E/D399V/ F405C/K409W | 330 | 61 | 62 | 61.3 | 1.4 | 37.3 | 920 | 330 | 61 | 95.73 | 67.8 | 0.16 |
| 535 | Steric hindrance + Charge | Q347R/K360E/D399V/ F405N/K409W | 331 | 61 | 62 | 65.8 | 1.4 | 32.8 | 921 | 331 | 61 | 97.02 | 67.2 | 0.19 |
| 536 | Steric hindrance + Charge | Q347R/K360E/D399V/ F405D/K409W | 332 | 61 | 62 | 61.2 | 2.2 | 36.5 | 922 | 332 | 61 | 96.39 | 65.4 | 0.2 |
| 537 | Steric hindrance + Charge | Q347R/K360E/D399V/ F405T/K409Y | 333 | 61 | 62 | 59.5 | 3.2 | 37.3 | 923 | 333 | 61 | 94.8 | 66 | 0.2 |
| 538 | Steric hindrance + Charge | Q347R/K360E/D399V/ F405T/K409H | 334 | 61 | 62 | 59.6 | 1.8 | 38.6 | 924 | 334 | 61 | 96.49 | 66 | 0.19 |
| 539 | Steric hindrance + Charge | Q347R/K360E/D399A/ F405T/K409F | 335 | 61 | 62 | 62.8 | 0.5 | 36.7 | 925 | 335 | 61 | 97.81 | 67.8 | 0.21 |
| 540 | Steric hindrance + Charge | Q347R/K360E/D399L/ F405T/K409F | 336 | 61 | 62 | 64.1 | 0.0 | 35.9 | 926 | 336 | 61 | 96.89 | 68.4 | 0.14 |
| 541 | Steric hindrance + Charge | Q347R/K360E/D3991/F 405T/K409F | 337 | 61 | 62 | 64.4 | 0.0 | 35.6 | 927 | 337 | 61 | 97.37 | 68.4 | 0.15 |
| 542 | Steric hindrance + Charge | Q347R/K360E/D399M/ F405T/K409F | 338 | 61 | 62 | 64.4 | 0.0 | 35.6 | 928 | 338 | 61 | 98.41 | 69 | 0.21 |
| 543 | Steric hindrance + Charge | Q347R/K360E/D399T/ F405T/K409F | 339 | 61 | 62 | 64.2 | 0.0 | 35.8 | 929 | 339 | 61 | 98.03 | 69 | 0.19 |
| 544 | Steric hindrance + Charge + Disulfide bond | Q347R/K360E/D399C/ F405T/K409F | 340 | 61 | 62 | 62.8 | 0.4 | 36.8 | 930 | 340 | 61 | 97.49 | 69 | 0.17 |
| 545 | Steric hindrance + Charge | Q347R/K360E/D399N/ F405T/K409F | 341 | 61 | 62 | 62.9 | 0.0 | 37.1 | 931 | 341 | 61 | 98.21 | 68.4 | 0.19 |
| 546 | Steric hindrance + Charge | Q347R/K360E/D399Q/ F405T/K409F | 342 | 61 | 62 | 63.4 | 0.9 | 35.7 | 932 | 342 | 61 | 97.4 | 67.8 | 0.2 |
| 547 | Steric hindrance + Charge | Q347R/K360E/D399H/ F405T/K409F | 343 | 61 | 62 | 67.3 | 0.4 | 32.3 | 933 | 343 | 61 | 96.36 | 67.2 | 0.16 |
| 548 | Steric hindrance + Charge | Q347R/K360E/D399A/ F405T/K409Y | 344 | 61 | 62 | 63.8 | 1.3 | 34.9 | 934 | 344 | 61 | 95.83 | 66.6 | 0.17 |
| 549 | Steric hindrance + Charge | Q347R/K360E/D399L/ F405T/K409Y | 345 | 61 | 62 | 67.7 | 1.3 | 31.1 | 935 | 345 | 61 | 95.94 | 66 | 0.14 |
| 550 | Steric hindrance + Charge | Q347R/K360E/D399M/ F405T/K409Y | 346 | 61 | 62 | 63.8 | 1.2 | 35.0 | 936 | 346 | 61 | 96.74 | 67.2 | 0.21 |
| 551 | Steric hindrance + Charge | Q347R/K360E/D399S/ F405T/K409Y | 347 | 61 | 62 | 66.2 | 1.2 | 32.6 | 937 | 347 | 61 | 95.37 | 63.6 | 0.15 |
| 552 | Steric hindrance + Charge | Q347R/K360E/D399T/ F405T/K409Y | 348 | 61 | 62 | 62.2 | 2.1 | 35.7 | 938 | 348 | 61 | 95.85 | 66.6 | 0.2 |
| 553 | Steric hindrance + Charge + Disulfide bond | Q347R/K360E/D399C/ F405T/K409Y | 349 | 61 | 62 | 59.3 | 2.3 | 38.4 | 939 | 349 | 61 | 95.53 | 66.6 | 0.23 |
| 554 | Steric hindrance + Charge | Q347R/K360E/D399N/ F405T/K409Y | 350 | 61 | 62 | 64.3 | 0.3 | 35.4 | 940 | 350 | 61 | 97.11 | 68.4 | 0.16 |
| 555 | Steric hindrance + Charge | Q347R/K360E/D399Q/ F405T/K409Y | 351 | 61 | 62 | 58.9 | 2.8 | 38.3 | 941 | 351 | 61 | 94.93 | 66.6 | 0.19 |
| 556 | Steric hindrance + Charge | Q347R/K360E/D399H/ F405T/K409Y | 352 | 61 | 62 | 62.1 | 0.7 | 37.1 | 942 | 352 | 61 | 97.51 | 67.2 | 0.22 |
| 557 | Steric hindrance + Charge | Q347R/K360E/D399V/ F405A/K409F | 353 | 61 | 62 | 65.1 | 0.0 | 349 | 943 | 353 | 61 | 98.15 | 67.8 | 0.18 |
| 558 | Steric hindrance + Charge | Q347R/K360E/D399V/ F405V/K409F | 354 | 61 | 62 | 66.7 | 0.0 | 33.3 | 944 | 354 | 61 | 97.13 | 68.4 | 0.15 |
| 559 | Steric hindrance + Charge | Q347R/K360E/D399V/ F405S/K409F | 355 | 61 | 62 | 60.0 | 1.1 | 39.0 | 945 | 355 | 61 | 96.76 | 67.2 | 0.17 |
| 560 | Steric hindrance + Charge + Disulfide bond | Q347R/K360E/D399V/ F405C/K409F | 356 | 61 | 62 | 62.4 | 2.0 | 35.6 | 946 | 356 | 61 | 95.59 | 67.8 | 0.16 |
| 561 | Steric hindrance + Charge | Q347R/K360E/D399V/ F405D/K409F | 357 | 61 | 62 | 64.9 | 2.7 | 32.4 | 947 | 357 | 61 | 94.5 | 62.4 | 0.15 |
| 562 | Steric hindrance + Charge | Q347R/K360E/D399V/ F405A/K409Y | 358 | 61 | 62 | 63.8 | 2.9 | 33.3 | 948 | 358 | 61 | 93.45 | 64.2 | 0.15 |
| 563 | Steric hindrance + Charge + Disulfide bond | Q347R/K360E/D399V/ F405C/K409Y | 359 | 61 | 62 | 63.9 | 5.7 | 30.4 | 949 | 359 | 61 | 88.5 | 64.2 | 0.11 |
| 564 | Steric hindrance + Charge | Q347R/K360E/D399V/ F405N/K409Y | 360 | 61 | 62 | 53.0 | 8.8 | 38.3 | 950 | 360 | 61 | 84.57 | 60 | 0.2 |
| 565 | Steric hindrance + Charge | Q347R/K360E/D399V/ F405D/K409Y | 361 | 61 | 62 | 57.0 | 3.2 | 39.8 | 951 | 361 | 61 | 94.89 | 63 | 0.23 |
| 566 | Steric hindrance + Charge | Q347R/K360E/D399L/ F405A/K409W | 362 | 61 | 62 | 65.6 | 0.0 | 34.4 | 952 | 362 | 61 | 98.49 | 68.4 | 0.21 |
| 567 | Steric hindrance + Charge | Q347R/K360E/D399L/ F405S/K409W | 363 | 61 | 62 | 66.0 | 0.3 | 33.7 | 953 | 363 | 61 | 97.73 | 67.8 | 0.21 |
| 568 | Steric hindrance + Charge + Disulfide bond | Q347R/K360E/D399L/ F405C/K409W | 364 | 61 | 62 | 64.3 | 1.7 | 34.0 | 954 | 364 | 61 | 95.13 | 67.8 | 0.19 |
| 569 | Steric hindrance + Charge | Q347R/K360E/D399I/F 405S/K409W | 365 | 61 | 62 | 63.8 | 0.3 | 35.9 | 955 | 365 | 61 | 98.21 | 52.8 | 0.21 |
| 570 | Steric hindrance + Charge + Disulfide bond | Q347R/K360E/D399I/F 405C/K409W | 366 | 61 | 62 | 64.1 | 1.3 | 34.6 | 956 | 366 | 61 | 96.18 | 67.8 | 0.19 |
| 571 | Steric hindrance + Charge | Q347R/K360E/D399M/ F405A/K409W | 367 | 61 | 62 | 67.0 | 0.0 | 33.0 | 957 | 367 | 61 | 98.03 | 67.8 | 0.2 |
| 572 | Steric hindrance + Charge | Q347R/K360E/D399M/ F405S/K409W | 368 | 61 | 62 | 63.6 | 0.4 | 36.1 | 958 | 368 | 61 | 97.92 | 67.8 | 0.2 |
| 573 | Steric hindrance + Charge + Disulfide bond | Q347R/K360E/D399M/ F405C/K409W | 369 | 61 | 62 | 60.0 | 2.3 | 37.7 | 959 | 369 | 61 | 93.7 | 68.4 | 0.19 |
| 574 | Steric hindrance + Charge | Q347R/K360E/D399G/ F405T/K409W | 370 | 61 | 62 | 69.3 | 0.0 | 30.7 | 960 | 370 | 61 | 97.05 | 69 | 0.16 |
| 575 | Steric hindrance + Charge | Q347H/K360E/D399V/ F405T/K409W | 371 | 61 | 62 | 66.1 | 0.0 | 33.9 | 961 | 371 | 61 | 98.13 | 68.4 | 0.2 |
| 576 | Steric hindrance + Charge | Q347R/K360E/D399A/ F405T/K409W | 372 | 61 | 62 | 70.9 | 0.0 | 29.1 | 962 | 372 | 61 | 95.45 | 69 | 0.11 |
| 577 | Steric hindrance + Charge | Q347R/K360E/D399N/ F405T/K409W | 373 | 61 | 62 | 67.1 | 0.0 | 32.9 | 963 | 373 | 61 | 97.1 | 69 | 0.16 |
| 578 | Steric hindrance + Charge | Q347R/K360E/D399Q/ F405T/K409W | 374 | 61 | 62 | 73.5 | 0.7 | 25.8 | 964 | 374 | 61 | 93.84 | 68.4 | 0.07 |
| 579 | Steric hindrance + Charge | Q347R/K360E/D399S/ F405T/K409F | 375 | 61 | 62 | 69.2 | 0.7 | 30.2 | 965 | 375 | 61 | 95.51 | 67.8 | 0.13 |
| 580 | Steric hindrance + Charge | Q347R/K360E/D399V/ F405N/K409F | 376 | 61 | 62 | 72.0 | 1.3 | 26.7 | 966 | 376 | 61 | 94.02 | 66 | 0.09 |
| 581 | Steric hindrance + Charge | Q347R/K360E/D399V/ F405V/K409Y | 377 | 61 | 62 | 66.7 | 0.9 | 32.4 | 967 | 377 | 61 | 96.4 | 67.8 | 0.17 |
| 582 | Steric hindrance + Charge | Q347R/K360E/D399V/ F405S/K409Y | 378 | 61 | 62 | 60.7 | 5.3 | 34.0 | 968 | 378 | 61 | 90.67 | 63.6 | 0.2 |
| 583 | Steric hindrance + Charge | Q347R1K360E/D399I/F 405A/K409W | 379 | 61 | 62 | 64.9 | 0.0 | 35.1 | 969 | 379 | 61 | 97.8 | 67.8 | 0.18 |
| 584 | Steric hindrance + Charge | Q347R/K360E/D399V/ F405G/K409W | 380 | 61 | 62 | 63.2 | 0.9 | 35.9 | 970 | 380 | 61 | 97.48 | 66 | 0.18 |
| 585 | Steric hindrance | T394W/F405S | 381 | 61 | 62 | 63.3 | 0.0 | 36.7 | 971 | 381 | 61 | 99.05 | 68.4 | 0.28 |
| 586 | Steric hindrance | T394W/F405T | 382 | 61 | 62 | 67.2 | 0.0 | 32.8 | 972 | 382 | 61 | 98.67 | 67.2 | 0.26 |
| 587 | Steric hindrance | T394Y/F405A | 383 | 61 | 62 | 58.3 | 3.2 | 38.5 | 973 | 383 | 61 | 99.12 | 64.8 | 0.3 |
| 588 | Steric hindrance | T394H/F405A | 384 | 61 | 62 | 34.0 | 39.4 | 26.6 | 974 | 384 | 61 | 98.59 | 63 | 0.25 |
| 589 | Steric hindrance | T394Y/F405T | 385 | 61 | 62 | 52.4 | 5.2 | 42.4 | 975 | 385 | 61 | 96.59 | 63 | 0.34 |
| 590 | Steric hindrance + Disulfide bond | T394Y/F405C | 386 | 61 | 62 | 52.3 | 8.6 | 39.1 | 976 | 386 | 61 | 90.84 | 67.2 | 0.23 |
| 591 | Steric hindrance | T394Y/F405V | 387 | 61 | 62 | 42.8 | 16.3 | 40.9 | 977 | 387 | 61 | 73.47 | 61.2 | 0.3 |
| 592 | Steric hindrance | T394F/F405G | 388 | 61 | 62 | 57.9 | 5.6 | 36.5 | 978 | 388 | 61 | 99.01 | 63 | 0.26 |
| 593 | Steric hindrance + Disulfide bond | T394F/F405C | 389 | 61 | 62 | 60.9 | 3.8 | 35.3 | 979 | 389 | 61 | 98.17 | 69 | 0.28 |
| 594 | Steric hindrance | T394F/F405V | 390 | 61 | 62 | 60.8 | 3.7 | 35.5 | 980 | 390 | 61 | 98.58 | 67.2 | 0.25 |
| 595 | Steric hindrance | T394H/F405G | 391 | 61 | 62 | 48.9 | 21.9 | 29.2 | 981 | 391 | 61 | 96.41 | 58.8 | 0.27 |
| 596 | Steric hindrance | T394H/F405S | 392 | 61 | 62 | 45.0 | 26.5 | 28.6 | 982 | 392 | 61 | 97.1 | 64.8 | 0.28 |
| 597 | Steric hindrance | T394H/F405T | 393 | 61 | 62 | 37.4 | 35.7 | 26.9 | 983 | 393 | 61 | 97.36 | 64.2 | 0.28 |
| 598 | Steric hindrance + Disulfide bond | T394H/F405C | 394 | 61 | 62 | 20.2 | 59.9 | 19.8 | 984 | 394 | 61 | 94.34 | 67.2 | 0.23 |
| 599 | Disulfide bond | T394C/F405C | 395 | 61 | 62 | 9.0 | 67.1 | 23.9 | 985 | 395 | 61 | 92.35 | 67.8 | 0.27 |
| 600 | Steric hindrance + Disulfide bond | T394W/F405C | 396 | 61 | 62 | 57.7 | 4.3 | 38.0 | 986 | 396 | 61 | 91.43 | 67.8 | 0.25 |
| 601 | Steric hindrance | T394W/F405V | 397 | 61 | 62 | 61.9 | 1.3 | 36.9 | 987 | 397 | 61 | 97.09 | 67.2 | 0.21 |
| 602 | Steric hindrance | T394Y/F405G | 398 | 61 | 62 | 61.0 | 2.3 | 36.7 | 988 | 398 | 61 | 95.65 | 62.4 | 0.21 |
| 603 | Steric hindrance | T394Y/F405S | 399 | 61 | 62 | 63.7 | 1.5 | 34.8 | 989 | 399 | 61 | 95.84 | 65.4 | 0.24 |
| 604 | Steric hindrance | T394F/F405S | 400 | 61 | 62 | 60.5 | 3.9 | 35.6 | 990 | 400 | 61 | 97.33 | 67.2 | 0.25 |
| 605 | Steric hindrance | T394F/F405T | 401 | 61 | 62 | 65.6 | 4.0 | 30.4 | 991 | 401 | 61 | 95.95 | 66.6 | 0.17 |
| 606 | Steric hindrance | T394H/F405V | 402 | 61 | 62 | 16.6 | 77.1 | 6.2 | 992 | 402 | 61 | 80.86 | 61.8 | 0.08 |
| 607 | Steric hindrance | T394W/F405G | 403 | 61 | 62 | 63.7 | 0.0 | 36.3 | 993 | 403 | 61 | 96.78 | 66.6 | 0.22 |
| 608 | Steric hindrance | T366W/L368S/Y407V | 404 | 61 | 62 | 56.9 | 4.0 | 39.2 | 994 | 404 | 61 | 98.62 | 67.2 | 0.28 |
| 609 | Steric hindrance | T366W/L368T/Y407V | 405 | 61 | 62 | 58.0 | 3.4 | 38.7 | 995 | 405 | 61 | 98.22 | 69 | 0.27 |
| 610 | Steric hindrance + Disulfide bond | T366W/L368C/Y407V | 406 | 61 | 62 | 60.9 | 0.0 | 39.1 | 996 | 406 | 61 | 96.88 | 69 | 0.31 |
| 611 | Steric hindrance | T366W/L368V/Y407V | 407 | 61 | 62 | 59.3 | 4.5 | 36.2 | 997 | 407 | 61 | 98.55 | 69 | 0.28 |
| 612 | Steric hindrance | T366W/L368G/Y407V | 408 | 61 | 62 | 63.6 | 0.0 | 36.4 | 998 | 408 | 61 | 98.78 | 67.2 | 0.29 |
| 613 | Steric hindrance | T366Y/L368A/Y407V | 409 | 61 | 62 | 61.2 | 0.0 | 38.8 | 999 | 409 | 61 | 98.59 | 67.8 | 0.26 |
| 614 | Steric hindrance | T366F/L368A/Y407V | 410 | 61 | 62 | 61.4 | 0.0 | 38.6 | 1000 | 410 | 61 | 98.73 | 67.2 | 0.28 |
| 615 | Steric hindrance | T366H/L368A/Y407V | 411 | 61 | 62 | 20.5 | 60.9 | 18.6 | 1001 | 411 | 61 | 96.33 | 64.2 | 0.26 |
| 616 | Steric hindrance | T366W/L368A/Y407L | 412 | 61 | 62 | 65.5 | 0.0 | 34.5 | 1002 | 412 | 61 | 97.67 | 66.6 | 0.21 |
| 617 | Steric hindrance | T366W/L368A/Y407I | 413 | 61 | 62 | 62.4 | 3.1 | 34.5 | 1003 | 413 | 61 | 98.11 | 68.4 | 0.2 |
| 618 | Steric hindrance | T366W/L368A/Y407M | 414 | 61 | 62 | 61.3 | 1.9 | 36.8 | 1004 | 414 | 61 | 98.02 | 67.8 | 0.21 |
| 619 | Steric hindrance | T366W/L368A/Y407A | 415 | 61 | 62 | 60.6 | 2.6 | 36.7 | 1005 | 415 | 61 | 98.98 | 67.8 | 0.01 |
| 620 | Steric hindrance | T366W/L368A/Y407S | 416 | 61 | 62 | 62.6 | 1.2 | 36.2 | 1006 | 416 | 61 | 96.53 | 66 | 0.17 |
| 621 | Steric hindrance | T366W/L368A/Y407T | 417 | 61 | 62 | 65.9 | 0.4 | 33.8 | 1007 | 417 | 61 | 97.14 | 67.2 | 0.22 |
| 622 | Steric hindrance + Disulfide bond | T366W/L368A/Y407C | 418 | 61 | 62 | 60.5 | 4.4 | 35.1 | 1008 | 418 | 61 | 95.38 | 68.4 | 0.21 |
| 623 | Steric hindrance | T366W/L368A/Y407H | 419 | 61 | 62 | 28.0 | 35.5 | 36.5 | 1009 | 419 | 61 | 57.08 | 54.6 | 0.21 |
| 624 | Steric hindrance | T366W/L368A/Y407N | 420 | 61 | 62 | 58.5 | 3.4 | 38.1 | 1010 | 420 | 61 | 93.71 | 60 | 0.22 |
| 625 | Steric hindrance + Disulfide bond | T366W/L368C/Y407C | 421 | 61 | 62 | 61.8 | 0.0 | 38.2 | 1011 | 421 | 61 | 97.93 | 67.8 | 0.21 |
| 626 | Steric hindrance + Disulfide bond | T366C/L368A/Y407C | 422 | 61 | 62 | 5.1 | 63.8 | 31.0 | 1012 | 422 | 61 | 56.77 | 60.6 | 0.1 |
| 627 | Steric hindrance | T366W/L368S/Y407L | 423 | 61 | 62 | 56.7 | 5.2 | 38.1 | 1013 | 423 | 61 | 94.19 | 63 | 0.22 |
| 628 | Steric hindrance | T366W/L368T/Y407L | 424 | 61 | 62 | 63.1 | 2.8 | 34.1 | 1014 | 424 | 61 | 97.73 | 66 | 0.17 |
| 629 | Steric hindrance + Disulfide bond | T366W/L368C/Y407L | 425 | 61 | 62 | 62.4 | 2.1 | 35.5 | 1015 | 425 | 61 | 94.95 | 66 | 0.22 |
| 630 | Steric hindrance | T366W/L368V/Y407L | 426 | 61 | 62 | 62.1 | 2.5 | 35.4 | 1016 | 426 | 61 | 98.65 | 67.2 | 0.23 |
| 631 | Steric hindrance | T366W/L368G/Y407L | 427 | 61 | 62 | 60.3 | 2.1 | 37.6 | 1017 | 427 | 61 | 95.76 | 72 | 0.18 |
| 632 | Steric hindrance | T366W/L368S/Y407I | 428 | 61 | 62 | 53.5 | 10.9 | 35.6 | 1018 | 428 | 61 | 96.32 | 66 | 0.22 |
| 633 | Steric hindrance + Disulfide bond | T366W/L368C/Y407I | 429 | 61 | 62 | 59.1 | 3.4 | 37.5 | 1019 | 429 | 61 | 95.18 | 67.8 | 0.22 |
| 634 | Steric hindrance | T366W/L368V/Y407I | 430 | 61 | 62 | 59.7 | 4.5 | 35.8 | 1020 | 430 | 61 | 98.66 | 67.8 | 0.22 |
| 635 | Steric hindrance | T366W/L368G/Y407I | 431 | 61 | 62 | 58.2 | 6.1 | 35.7 | 1021 | 431 | 61 | 96.76 | 64.8 | 0.22 |
| 636 | Steric hindrance | T366W/L368S/Y407M | 432 | 61 | 62 | 54.6 | 5.6 | 39.8 | 1022 | 432 | 61 | 96.55 | 65.4 | 0.22 |
| 637 | Steric hindrance | T366W/L368T/Y407M | 433 | 61 | 62 | 60.6 | 3.0 | 36.4 | 1023 | 433 | 61 | 97.12 | 65.4 | 0.23 |
| 638 | Steric hindrance + Disulfide bond | T366W/L368C/Y407M | 434 | 61 | 62 | 60.5 | 4.4 | 35.2 | 1024 | 434 | 61 | 94.73 | 66 | 0.2 |
| 639 | Steric hindrance | T366W/L368V/Y407M | 435 | 61 | 62 | 56.7 | 3.5 | 39.8 | 1025 | 435 | 61 | 96.02 | 66.6 | 0.2 |
| 640 | Steric hindrance | T366W/L368G/Y407M | 436 | 61 | 62 | 55.7 | 5.6 | 38.7 | 1026 | 436 | 61 | 97.25 | 64.8 | 0.25 |
| 641 | Steric hindrance | T366W/L368S/Y407A | 437 | 61 | 62 | 57.6 | 3.5 | 38.9 | 1027 | 437 | 61 | 97.38 | 66 | 0.22 |
| 642 | Steric hindrance | T366W/L368T/Y407A | 438 | 61 | 62 | 57.3 | 4.3 | 38.4 | 1028 | 438 | 61 | 98.42 | 68.4 | 0.22 |
| 643 | Steric hindrance + Disulfide bond | T366W/L368C/Y407A | 439 | 61 | 62 | 49.4 | 4.1 | 46.5 | 1029 | 439 | 61 | 94.57 | 69 | 0.22 |
| 644 | Steric hindrance | T366W/L368V/Y407A | 440 | 61 | 62 | 56.9 | 5.1 | 37.9 | 1030 | 440 | 61 | 98.72 | 68.4 | 0.2 |
| 645 | Steric hindrance | T366W/L368G/Y407A | 441 | 61 | 62 | 61.6 | 0.0 | 38.4 | 1031 | 441 | 61 | 98.12 | 66 | 0.24 |
| 646 | Steric hindrance | T366W/L368S/Y407T | 442 | 61 | 62 | 63.6 | 1.2 | 35.2 | 1032 | 442 | 61 | 95.99 | 64.8 | 0.23 |
| 647 | Steric hindrance | T366W/L368T/Y407T | 443 | 61 | 62 | 63.7 | 0.6 | 35.7 | 1033 | 443 | 61 | 96.69 | 66.6 | 0.21 |
| 648 | Steric hindrance + Disulfide bond | T366W/L368C/Y407T | 444 | 61 | 62 | 55.0 | 1.3 | 43.7 | 1034 | 444 | 61 | 94.61 | 68.4 | 0.25 |
| 649 | Steric hindrance | T366W/L368V/Y407T | 445 | 61 | 62 | 62.7 | 3.7 | 33.6 | 1035 | 445 | 61 | 96.19 | 67.8 | 0.23 |
| 650 | Steric hindrance | T366W/L368G/Y407T | 446 | 61 | 62 | 58.9 | 1.5 | 39.6 | 1036 | 446 | 61 | 95.89 | 63.6 | 0.24 |
| 651 | Steric hindrance + Disulfide bond | T366W/L368S/Y407C | 447 | 61 | 62 | 46.8 | 4.7 | 48.5 | 1037 | 447 | 61 | 96.66 | 66.6 | 0.2 |
| 652 | Steric hindrance + Disulfide bond | T366W/L368T/Y407C | 448 | 61 | 62 | 56.8 | 6.6 | 36.6 | 1038 | 448 | 61 | 95.78 | 68.4 | 0.19 |
| 653 | Steric hindrance + Disulfide bond | T366W/L368V/Y407C | 449 | 61 | 62 | 55.0 | 8.9 | 36.1 | 1039 | 449 | 61 | 91.35 | 68.4 | 0.21 |
| 654 | Steric hindrance + Disulfide bond | T366W/L368G/Y407C | 450 | 61 | 62 | 60.1 | 0.6 | 39.3 | 1040 | 450 | 61 | 96.64 | 66 | 0.21 |
| 655 | Steric hindrance | T366W/L368S/Y407H | 451 | 61 | 62 | 25.3 | 28.4 | 46.3 | 1041 | 451 | 61 | 53.32 | 53.4 | 0.25 |
| 656 | Steric hindrance | T366W/L368T/Y407H | 452 | 61 | 62 | 39.0 | 20.6 | 40.4 | 1042 | 452 | 61 | 59.36 | 55.2 | 0.24 |
| 657 | Steric hindrance | T366W/L368V/Y407H | 453 | 61 | 62 | 29.4 | 38.4 | 32.2 | 1043 | 453 | 61 | 58.63 | 58.2 | 0.23 |
| 658 | Steric hindrance | T366W/L368G/Y407H | 454 | 61 | 62 | 26.5 | 11.9 | 61.6 | 1044 | 454 | 61 | 66.71 | 53.4 | 0.19 |
| 659 | Steric hindrance | T366W/L368S/Y407N | 455 | 61 | 62 | 49.0 | 6.9 | 44.1 | 1045 | 455 | 61 | 85.83 | 54.6 | 0.22 |
| 660 | Steric hindrance | T366W/L368T/Y407N | 456 | 61 | 62 | 62.7 | 3.7 | 33.6 | 1046 | 456 | 61 | 91.72 | 58.2 | 0.19 |
| 661 | Steric hindrance + Disulfide bond | T366W/L368C/Y407N | 457 | 61 | 62 | 49.2 | 11.0 | 39.8 | 1047 | 457 | 61 | 78.42 | 55.2 | 0.19 |
| 662 | Steric hindrance | T366W/L368G/Y407N | 458 | 61 | 62 | 31.3 | 10.1 | 58.6 | 1048 | 458 | 61 | 69.65 | 52.2 | 0.18 |
| 663 | Steric hindrance | T366W/L368S/Y407Q | 459 | 61 | 62 | 48.7 | 6.0 | 45.3 | 1049 | 459 | 61 | 89.65 | 56.4 | 0.22 |
| 664 | Steric hindrance | T366W/L368T/Y407Q | 460 | 61 | 62 | 49.3 | 8.6 | 42.2 | 1050 | 460 | 61 | 75.82 | 57 | 0.08 |
| 665 | Steric hindrance + Disulfide bond | T366W/L368C/Y407Q | 461 | 61 | 62 | 42.5 | 16.5 | 41.0 | 1051 | 461 | 61 | 62.48 | 56.4 | 0.16 |
| 666 | Steric hindrance | T366W/L368V/Y407Q | 462 | 61 | 62 | 28.6 | 35.9 | 35.5 | 1052 | 462 | 61 | 62.16 | 57.6 | 0.25 |
| 667 | Steric hindrance | T366W/L368G/Y407Q | 463 | 61 | 62 | 24.9 | 28.3 | 46.8 | 1053 | 463 | 61 | 62.44 | 53.4 | 0.09 |
| 668 | Steric hindrance | T366Y/L368S/Y407L | 464 | 61 | 62 | 57.4 | 9.0 | 33.6 | 1054 | 464 | 61 | 96.1 | 66.6 | 0.19 |
| 669 | Steric hindrance + Disulfide bond | T366Y/L368C/Y407L | 465 | 61 | 62 | 31.0 | 6.9 | 62.0 | 1055 | 465 | 61 | 95.43 | 67.2 | 0.23 |
| 670 | Steric hindrance | T366Y/L368V/Y407L | 466 | 61 | 62 | 57.3 | 3.1 | 39.6 | 1056 | 466 | 61 | 97.98 | 69 | 0.2 |
| 671 | Steric hindrance | T366Y/L368G/Y407L | 467 | 61 | 62 | 52.1 | 11.1 | 36.8 | 1057 | 467 | 61 | 93.15 | 75.6 | 0.16 |
| 672 | Steric hindrance | T366Y/L368S/Y407I | 468 | 61 | 62 | 58.4 | 3.4 | 38.2 | 1058 | 468 | 61 | 98.75 | 67.8 | 0.01 |
| 673 | Steric hindrance + Disulfide bond | T366Y/L368C/Y4071 | 469 | 61 | 62 | 65.2 | 1.5 | 33.3 | 1059 | 469 | 61 | 94.16 | 67.8 | 0.16 |
| 674 | Steric hindrance | T366Y/L368V/Y407I | 470 | 61 | 62 | 61.9 | 2.4 | 35.7 | 1060 | 470 | 61 | 97.68 | 68.4 | 0.19 |
| 675 | Steric hindrance | T366Y/L368G/Y407I | 471 | 61 | 62 | 51.1 | 7.3 | 41.6 | 1061 | 471 | 61 | 94.77 | 62.4 | 0.19 |
| 676 | Steric hindrance | T366Y/L368G/Y407M | 472 | 61 | 62 | 57.2 | 8.3 | 34.5 | 1062 | 472 | 61 | 90.25 | 61.2 | 0.04 |
| 677 | Steric hindrance | T366Y/L368V/Y407A | 473 | 61 | 62 | 66.5 | 0.0 | 33.5 | 1063 | 473 | 61 | 97.7 | 67.8 | 0.21 |
| 678 | Steric hindrance | T366Y/L368G/Y407A | 474 | 61 | 62 | 43.6 | 16.2 | 40.2 | 1064 | 474 | 61 | 92.18 | 66.6 | 0.2 |
| 679 | Steric hindrance | T366Y/L368S/Y407T | 475 | 61 | 62 | 58.3 | 3.2 | 38.5 | 1065 | 475 | 61 | 93.85 | 63 | 0.23 |
| 680 | Steric hindrance | T366Y/L368T/Y407T | 476 | 61 | 62 | 59.2 | 3.9 | 36.9 | 1066 | 476 | 61 | 92.9 | 63 | 0.2 |
| 681 | Steric hindrance + Disulfide bond | T366Y/L368C/Y407T | 477 | 61 | 62 | 54.6 | 3.9 | 41.5 | 1067 | 477 | 61 | 92.82 | 64.8 | 0.22 |
| 682 | Steric hindrance | T366Y/L368V/Y407T | 478 | 61 | 62 | 63.6 | 1.9 | 34.6 | 1068 | 478 | 61 | 95.64 | 67.2 | 0.23 |
| 683 | Steric hindrance | T366Y/L368G/Y407T | 479 | 61 | 62 | 45.2 | 11.1 | 43.7 | 1069 | 479 | 61 | 78.6 | 55.8 | 0.21 |
| 684 | Steric hindrance + Disulfide bond | T366Y/L368T/Y407C | 480 | 61 | 62 | 54.1 | 6.9 | 39.0 | 1070 | 480 | 61 | 96.32 | 66 | 0.22 |
| 685 | Steric hindrance + Disulfide bond | T366Y/L368V/Y407C | 481 | 61 | 62 | 58.3 | 2.5 | 39.2 | 1071 | 481 | 61 | 93.21 | 68.4 | 0.24 |
| 686 | Steric hindrance + Disulfide bond | T366Y/L368G/Y407C | 482 | 61 | 62 | 44.1 | 11.5 | 44.3 | 1072 | 482 | 61 | 90.88 | 63.6 | 0.19 |
| 687 | Steric hindrance | T366Y/L368S/Y407H | 483 | 61 | 62 | 45.6 | 11.6 | 42.9 | 1073 | 483 | 61 | 74.46 | 55.2 | 0.22 |
| 688 | Steric hindrance | T366Y/L368T/Y407H | 484 | 61 | 62 | 47.1 | 11.3 | 41.6 | 1074 | 484 | 61 | 79.63 | 57 | 0.23 |
| 689 | Steric hindrance + Disulfide bond | T366Y/L368C/Y407H | 485 | 61 | 62 | 42.6 | 13.5 | 44.0 | 1075 | 485 | 61 | 72 | 57 | 0.24 |
| 690 | Steric hindrance | T366Y/L368V/Y407H | 486 | 61 | 62 | 40.3 | 23.6 | 36.1 | 1076 | 486 | 61 | 57.82 | 60.6 | 0.27 |
| 691 | Steric hindrance | T366Y/L368G/Y407H | 487 | 61 | 62 | 19.8 | 28.6 | 51.7 | 1077 | 487 | 61 | 49.01 | 51.6 | 0.14 |
| 692 | Steric hindrance | T366Y/L368S/Y407N | 488 | 61 | 62 | 50.4 | 7.4 | 42.2 | 1078 | 488 | 61 | 86.8 | 54.6 | 0.22 |
| 693 | Steric hindrance | T366Y/L368T/Y407N | 489 | 61 | 62 | 45.3 | 11.0 | 43.7 | 1079 | 489 | 61 | 79.68 | 54 | 0.23 |
| 694 | Steric hindrance + Disulfide bond | T366Y/L368C/Y407N | 490 | 61 | 62 | 43.3 | 13.1 | 43.6 | 1080 | 490 | 61 | 74.89 | 54 | 0.24 |
| 695 | Steric hindrance | T366Y/L368V/Y407N | 491 | 61 | 62 | 51.9 | 12.3 | 35.8 | 1081 | 491 | 61 | 76.44 | 61.2 | 0.25 |
| 696 | Steric hindrance | T366Y/L368G/Y407N | 492 | 61 | 62 | 16.1 | 20.3 | 63.5 | 1082 | 492 | 61 | 56.22 | 94.2 | 0.14 |
| 697 | Steric hindrance | T366Y/L368S/Y407Q | 493 | 61 | 62 | 46.5 | 8.9 | 44.5 | 1083 | 493 | 61 | 80.63 | 55.8 | 0.2 |
| 698 | Steric hindrance | T366Y/L368T/Y407Q | 494 | 61 | 62 | 40.3 | 15.3 | 44.4 | 1084 | 494 | 61 | 65.43 | 55.2 | 0.2 |
| 699 | Steric hindrance + Disulfide bond | T366Y/L368C/Y407Q | 495 | 61 | 62 | 43.6 | 13.7 | 42.7 | 1085 | 495 | 61 | 78.31 | 56.4 | 0.19 |
| 700 | Steric hindrance | T366W/L368A/Y407Q | 496 | 61 | 62 | 55.3 | 3.4 | 41.2 | 1086 | 496 | 61 | 93.08 | 60 | 0.23 |
| 701 | Steric hindrance | T366W/L368T/Y4071 | 497 | 61 | 62 | 64.2 | 0.0 | 35.8 | 1087 | 497 | 61 | 98.13 | 67.2 | 0.24 |
| 702 | Steric hindrance + Disulfide bond | T366W/L368C/Y407H | 498 | 61 | 62 | 32.0 | 26.5 | 41.5 | 1088 | 498 | 61 | 52.44 | 57.6 | 0.25 |
| 703 | Steric hindrance | T366W/L368V/Y407N | 499 | 61 | 62 | 33.7 | 29.7 | 36.6 | 1089 | 499 | 61 | 51.46 | 55.2 | 0.26 |
| 704 | Steric hindrance | T366Y/L368T/Y407L | 500 | 61 | 62 | 50.0 | 15.4 | 34.6 | 1090 | 500 | 61 | 97.09 | 66 | 0.24 |
| 705 | Steric hindrance + Disulfide bond | T366Y/L368C/Y407C | 501 | 61 | 62 | 57.0 | 1.3 | 41.7 | 1091 | 501 | 61 | 96.7 | 64.8 | 0.17 |
| 706 | Steric hindrance | T366F/L368S/Y407L | 502 | 61 | 62 | 55.4 | 7.7 | 36.8 | 1092 | 502 | 61 | 96.07 | 64.2 | 0.23 |
| 707 | Steric hindrance | T366F/L368T/Y407L | 503 | 61 | 62 | 52.7 | 11.4 | 35.8 | 1093 | 503 | 61 | 97.05 | 65.4 | 0.22 |
| 708 | Steric hindrance + Disulfide bond | T366F/L368C/Y407L | 504 | 61 | 62 | 59.1 | 4.0 | 36.9 | 1094 | 504 | 61 | 95.62 | 66.6 | 0.22 |
| 709 | Steric hindrance | T366F/L368V/Y407L | 505 | 61 | 62 | 62.1 | 0.0 | 37.9 | 1095 | 505 | 61 | 98.14 | 68.4 | 0.23 |
| 710 | Steric hindrance | T366F/L368G/Y407L | 506 | 61 | 62 | 55.4 | 6.0 | 38.6 | 1096 | 506 | 61 | 93.82 | 78 | 0.19 |
| 711 | Steric hindrance | T366F/L368S/Y407I | 507 | 61 | 62 | 60.4 | 5.3 | 34.3 | 1097 | 507 | 61 | 96.31 | 66 | 0.19 |
| 712 | Steric hindrance | T366F/L368T/Y407I | 508 | 61 | 62 | 55.1 | 5.3 | 39.7 | 1098 | 508 | 61 | 96.55 | 65.4 | 0.23 |
| 713 | Steric hindrance + Disulfide bond | T366F/L368C/Y407I | 509 | 61 | 62 | 60.2 | 1.8 | 38.0 | 1099 | 509 | 61 | 95.42 | 67.2 | 0.24 |
| 714 | Steric hindrance | T366F/L368V/Y407I | 510 | 61 | 62 | 61.9 | 0.0 | 38.1 | 1100 | 510 | 61 | 93.8 | 67.8 | 0.22 |
| 715 | Steric hindrance | T366F/L368G/Y407I | 511 | 61 | 62 | 54.4 | 8.9 | 36.8 | 1101 | 511 | 61 | 88.22 | 60.6 | 0.19 |
| 716 | Steric hindrance | T366F/L368S/Y407M | 512 | 61 | 62 | 55.2 | 5.8 | 39.0 | 1102 | 512 | 61 | 96.03 | 64.2 | 0.23 |
| 717 | Steric hindrance | T366F/L368T/Y407M | 513 | 61 | 62 | 52.3 | 9.9 | 37.8 | 1103 | 513 | 61 | 96.16 | 64.8 | 0.21 |
| 718 | Steric hindrance + Disulfide bond | T366F/L368C/Y407M | 514 | 61 | 62 | 52.4 | 4.0 | 43.5 | 1104 | 514 | 61 | 94.83 | 66.6 | 0.23 |
| 719 | Steric hindrance | T366F/L368V/Y407M | 515 | 61 | 62 | 62.1 | 0.0 | 37.9 | 1105 | 515 | 61 | 98.04 | 68.4 | 0.24 |
| 720 | Steric hindrance | T366F/L368G/Y407M | 516 | 61 | 62 | 55.5 | 11.8 | 32.7 | 1106 | 516 | 61 | 91.42 | 59.4 | 0.16 |
| 721 | Steric hindrance | T366F/L368S/Y407A | 517 | 61 | 62 | 57.2 | 5.9 | 36.9 | 1107 | 517 | 61 | 96.82 | 64.2 | 0.22 |
| 722 | Steric hindrance | T366F/L368T/Y407A | 518 | 61 | 62 | 56.8 | 7.4 | 35.8 | 1108 | 518 | 61 | 97.21 | 64.8 | 0.2 |
| 723 | Steric hindrance + Disulfide bond | T366F/L368C/Y407A | 519 | 61 | 62 | 59.2 | 4.2 | 36.5 | 1109 | 519 | 61 | 94.78 | 66.6 | 0.17 |
| 724 | Steric hindrance | T366F/L368V/Y407A | 520 | 61 | 62 | 63.7 | 2.6 | 33.7 | 1110 | 520 | 61 | 95.93 | 67.8 | 0.14 |
| 725 | Steric hindrance | T366F/L368G/Y407A | 521 | 61 | 62 | 44.7 | 18.9 | 36.5 | 1111 | 521 | 61 | 93 | 57.6 | 0.2 |
| 726 | Steric hindrance | T366F/L368S/Y407T | 522 | 61 | 62 | 11.6 | 2.7 | 85.7 | 1112 | 522 | 61 | 91.24 | 60.6 | 0.16 |
| 727 | Steric hindrance | T366F/L368T/Y407T | 523 | 61 | 62 | 61.8 | 2.2 | 36.0 | 1113 | 523 | 61 | 94.78 | 64.2 | 0.17 |
| 728 | Steric hindrance + Disulfide bond | T366F/L368C/Y407T | 524 | 61 | 62 | 59.2 | 4.1 | 36.7 | 1114 | 524 | 61 | 91.42 | 64.2 | 0.12 |
| 729 | Steric hindrance | T366F/L368V/Y407T | 525 | 61 | 62 | 58.1 | 1.9 | 40.0 | 1115 | 525 | 61 | 96.13 | 67.2 | 0.2 |
| 730 | Steric hindrance | T366F/L368G/Y407T | 526 | 61 | 62 | 42.4 | 14.7 | 42.9 | 1116 | 526 | 61 | 68.82 | 54.6 | 0.16 |
| 731 | Steric hindrance + Disulfide bond | T366F/L368S/Y407C | 527 | 61 | 62 | 47.9 | 5.2 | 46.9 | 1117 | 527 | 61 | 95.97 | 64.2 | 0.17 |
| 732 | Steric hindrance + Disulfide bond | T366F/L368T/Y407C | 528 | 61 | 62 | 62.9 | 5.2 | 31.8 | 1118 | 528 | 61 | 95.49 | 65.4 | 0.12 |
| 733 | Steric hindrance + Disulfide bond | T366F/L368V/Y407C | 529 | 61 | 62 | 61.0 | 2.1 | 36.9 | 1119 | 529 | 61 | 93.2 | 68.4 | 0.18 |
| 734 | Steric hindrance + Disulfide bond | T366F/L368G/Y407C | 530 | 61 | 62 | 37.9 | 21.6 | 40.5 | 1120 | 530 | 61 | 90.66 | 64.8 | 0.16 |
| 735 | Steric hindrance | T366F/L368S/Y407H | 531 | 61 | 62 | 30.2 | 18.4 | 51.4 | 1121 | 531 | 61 | 59.38 | 52.8 | 0.19 |
| 736 | Steric hindrance | T366F/L368T/Y407H | 532 | 61 | 62 | 35.7 | 15.9 | 48.4 | 1122 | 532 | 61 | 70.92 | 54 | 0.22 |
| 737 | Steric hindrance + Disulfide bond | T366F/L368C/Y407H | 533 | 61 | 62 | 43.8 | 14.3 | 41.9 | 1123 | 533 | 61 | 73.11 | 65.4 | 0.19 |
| 738 | Steric hindrance | T366F/L368G/Y407H | 534 | 61 | 62 | 25.9 | 26.2 | 47.8 | 1124 | 534 | 61 | 50.76 | 52.2 | 0.19 |
| 739 | Steric hindrance | T366F/L368S/Y407N | 535 | 61 | 62 | 27.5 | 15.1 | 57.4 | 1125 | 535 | 61 | 61.65 | 51 | 0.2 |
| 740 | Steric hindrance + Disulfide bond | T366F/L368C/Y407N | 536 | 61 | 62 | 39.4 | 12.9 | 47.7 | 1126 | 536 | 61 | 72.42 | 53.4 | 0.22 |
| 741 | Steric hindrance | T366F/L368V/Y407N | 537 | 61 | 62 | 48.6 | 11.2 | 40.2 | 1127 | 537 | 61 | 83.11 | 60 | 0.22 |
| 742 | Steric hindrance | T366F/L368G/Y407N | 538 | 61 | 62 | 11.5 | 20.9 | 67.6 | 1128 | 538 | 61 | 61.64 | 50.4 | 0.16 |
| 743 | Steric hindrance | T366F/L368S/Y407Q | 539 | 61 | 62 | 29.0 | 11.8 | 59.2 | 1129 | 539 | 61 | 65 | 52.8 | 0.19 |
| 744 | Steric hindrance | T366F/L368T/Y407Q | 540 | 61 | 62 | 36.2 | 14.6 | 49.2 | 1130 | 540 | 61 | 68.88 | 55.2 | 0.2 |
| 745 | Steric hindrance + Disulfide bond | T366F/L368C/Y407Q | 541 | 61 | 62 | 36.4 | 21.5 | 42.1 | 1131 | 541 | 61 | 58.87 | 55.8 | 0.19 |
| 746 | Steric hindrance | T366F/L368V/Y407Q | 542 | 61 | 62 | 31.6 | 28.1 | 40.2 | 1132 | 542 | 61 | 53.09 | 57 | 0.23 |
| 747 | Steric hindrance | T366F/L368G/Y407Q | 543 | 61 | 62 | 16.4 | 20.9 | 62.6 | 1133 | 543 | 61 | 47.58 | 51.6 | 0.15 |
| 748 | Steric hindrance + Disulfide bond | T366F/L368C/Y407C | 544 | 61 | 62 | 58.0 | 0.0 | 42.0 | 1134 | 544 | 61 | 97.71 | 66 | 0.19 |
| 749 | Steric hindrance | T366Y/L368T/Y407I | 545 | 61 | 62 | 59.6 | 4.2 | 36.1 | 1135 | 545 | 61 | 96.94 | 66.6 | 0.2 |
| 750 | Steric hindrance | T366F/L368V/Y407H | 546 | 61 | 62 | 50.5 | 21.4 | 28.1 | 1136 | 546 | 61 | 63.31 | 58.2 | 0.13 |
| 751 | Steric hindrance | T366F/L368T/Y407N | 547 | 61 | 62 | 36.6 | 12.9 | 50.5 | 1137 | 547 | 61 | 68.95 | 51.6 | 0.16 |
| 752 | Steric hindrance + Disulfide bond | T366Y/L368S/Y407C | 548 | 61 | 62 | 59.9 | 1.3 | 38.8 | 1138 | 548 | 61 | 96.16 | 66 | 0.21 |
| 753 | Steric hindrance | T366Y/L368V/Y407Q | 549 | 61 | 62 | 40.2 | 21.7 | 38.2 | 1139 | 549 | 61 | 61.87 | 59.4 | 0.26 |
| 754 | Steric hindrance | T366Y/L368S/Y407M | 550 | 61 | 62 | 57.9 | 4.5 | 37.6 | 1140 | 550 | 61 | 96.63 | 67.2 | 0.2 |
| 755 | Steric hindrance | T366Y/L368T/Y407M | 551 | 61 | 62 | 55.6 | 8.8 | 35.6 | 1141 | 551 | 61 | 96.86 | 66.6 | 0.2 |
| 756 | Steric hindrance + Disulfide bond | T366Y/L368C/Y407M | 552 | 61 | 62 | 55.7 | 5.3 | 39.0 | 1142 | 552 | 61 | 95.11 | 67.2 | 0.19 |
| 757 | Steric hindrance | T366Y/L368V/Y407M | 553 | 61 | 62 | 66.1 | 0.0 | 33.9 | 1143 | 553 | 61 | 98.67 | 69 | 0.23 |
| 758 | Steric hindrance | T366Y/L368S/Y407A | 554 | 61 | 62 | 65.0 | 1.1 | 33.9 | 1144 | 554 | 61 | 96.86 | 64.8 | 0.21 |
| 759 | Steric hindrance | T366Y/L368T/Y407A | 555 | 61 | 62 | 61.3 | 3.6 | 35.1 | 1145 | 555 | 61 | 97.83 | 66 | 0.2 |
| 760 | Steric hindrance + Disulfide bond | T366Y/L368C/Y407A | 556 | 61 | 62 | 57.4 | 2.4 | 40.2 | 1146 | 556 | 61 | 94.46 | 65.4 | 0.2 |
| 761 | Steric hindrance | T366Y/L368G/Y407Q | 557 | 61 | 62 | 5.9 | 5.9 | 88.2 | 1147 | 557 | 61 | 51.19 | 51.6 | 0.07 |
| 762 | Steric hindrance + Charge | Q347R/K360E/D399I/F 405T/K409Y | 558 | 61 | 62 | 65.6 | 0.0 | 34.4 | 1148 | 558 | 61 | 96.74 | 65.4 | 0.2 |
| 763 | Steric hindrance + Charge | E345R/Q347R/K360D/ T366V/D399L/Y407A/ K409L | 559 | 61 | 62 | 61.2 | 0.0 | 38.8 | 1149 | 559 | 61 | 97.02 | 66.6 | 0.19 |
| 764 | Steric hindrance + Charge | E345R/Q347R/K360D/ T366V/D399I/Y407A/ K409I | 560 | 61 | 62 | 58.2 | 0.0 | 41.8 | 1150 | 560 | 61 | 97.69 | 68.4 | 0.2 |

**(Table 16) Amino acid modifications that showed strong heavy-chain homodimerization-promoting ability with a heteromultimer ratio (%) of 10% or lower in Table 15**

| Sample No. | Category of modification effect | Modifications that promote formation of heavy chain homodimers |
|---|---|---|
| 766 | Charge | E356R/D399K/K409E/K439E |
| 767 | Charge | E356H/D399K/K409E/K439E |
| 768 | Charge | E356K/D399R/K409E/K439E |
| 769 | Charge | E356K/D399H/K409E/K439E |
| 770 | Charge | E356K/D399K/K409D/K439E |
| 771 | Charge | E356K/D399K/K409E/K439D |
| 772 | Charge | E356K/D399R/K409D/K439E |
| 773 | Charge | E356K/D399H/K409D/K439E |
| 774 | Charge | E356R/K392D/D399K/K439E |
| 775 | Charge | E356K/K392D/D399K/K439D |
| 776 | Charge | E356K/K392D/D399R/K439E |
| 777 | Charge | E356K/K392E/D399K/K439E |
| 778 | Charge | K392D/D399R/K409D |
| 779 | Charge | K392D/D399K/K409E |
| 780 | Charge | K392E/D399K/K409D |
| 781 | Charge | E356K/K392D/D399K/K409E |
| 782 | Charge | E356K/K392D/D399R/K409D |
| 783 | Charge | E356R/K392D/D399K/K409D |
| 784 | Charge | E356H/K392D/D399K/K409D |
| 785 | Charge | E356K/K392E/D399K/K409D |
| 786 | Charge | E356R/K392D/D399K/K439D |
| 787 | Charge | E356K/K392D/D399H/K409D |
| 788 | Charge | E356R/D399K/K409E/K439D |
| 789 | Charge | E356H/D399K/K409E/K439D |
| 790 | Steric hindrance + Charge | Q347R/K360D/T366V/D399M/Y407A/K409V |
| 791 | Steric hindrance + Charge | E345R/Q347K/K360D/T366V/D399M/Y407A/K409V |
| 792 | Steric hindrance + Charge | E345R/Q347R/K360E/T366V/D399M/Y407A/K409V |
| 793 | Steric hindrance + Charge | Q347K/K360D/T366V/D399M/Y407A/K409V |
| 794 | Steric hindrance + Charge | Q347R/K360E/T366V/D399M/Y407A/K409V |
| 795 | Steric hindrance + Charge | Q347K/K360E/T366V/D399M/Y407A/K409V |
| 796 | Steric hindrance + Charge | E345R/Q347R/K360D/T366V/D399Q/Y407A/K409Q |
| 797 | Steric hindrance + Charge | E345R/Q347R/K360D/T366V/D399N/Y407A/K409Q |
| 798 | Steric hindrance + Charge | E345R/Q347R/K360D/T366V/D399H/Y407A/K409Q |
| 799 | Steric hindrance + Charge | E345R/Q347R/K360D/T366V/D399I/Y407A/K409Q |
| 800 | Steric hindrance + Charge | E345R/Q347R/K360D/T366V/D399F/Y407A/K409Q |
| 801 | Steric hindrance + Charge | E345R/Q347R/K360D/T366V/D399Y/Y407A/K409Q |
| 802 | Steric hindrance + Charge | E345R/Q347R/K360D/T366V/D399T/Y407A/K409Q |
| 803 | Steric hindrance + Charge | E345R/Q347R/K360D/T366V/D399S/Y407A/K409Q |
| 808 | Steric hindrance + Charge | E345R/Q347R/K360D/T366V/D399F/Y407A/K409N |
| 812 | Steric hindrance + Charge | E345R/Q347R/K360D/T366V/D399Q/Y407A/K409H |
| 813 | Steric hindrance + Charge | E345R/Q347R/K360D/T366V/D399N/Y407A/K409H |
| 814 | Steric hindrance + Charge | E345R/Q347R/K360D/T366V/D399H/Y407A/K409H |
| 815 | Steric hindrance + Charge | E345R/Q347R/K360D/T366V/D399V/Y407A/K409H |
| 816 | Steric hindrance + Charge | E345R/Q347R/K360D/T366V/D399L/Y407A/K409H |
| 817 | Steric hindrance + Charge | E345R/Q347R/K360D/T366V/D399F/Y407A/K409H |
| 818 | Steric hindrance + Charge | E345R/Q347R/K360D/T366V/D399Y/Y407A/K409H |
| 819 | Steric hindrance + Charge | E345R/Q347R/K360D/T366V/D399T/Y407A/K409H |
| 820 | Steric hindrance + Charge | E345R/Q347R/K360D/T366V/D399S/Y407A/K409H |
| 821 | Steric hindrance + Charge | E345R/Q347R/K360D/T366V/D399Q/Y407A/K409V |
| 822 | Steric hindrance + Charge | E345R/Q347R/K360D/T366V/D399N/Y407A/K409V |
| 823 | Steric hindrance + Charge | E345R/Q347R/K360D/T366V/D399H/Y407A/K409V |
| 824 | Steric hindrance + Charge | E345R/Q347R/K360D/T366V/D399V/Y407A/K409V |
| 825 | Steric hindrance + Charge | E345R/Q347R/K360D/T366V/D399L/Y407A/K409V |
| 826 | Steric hindrance + Charge | E345R/Q347R/K360D/T366V/D399I/Y407A/K409V |
| 827 | Steric hindrance + Charge | E345R/Q347R/K360D/T366V/D399F/Y407A/K409V |
| 828 | Steric hindrance + Charge | E345R/Q347R/K360D/T366V/D399Y/Y407A/K409V |
| 829 | Steric hindrance + Charge | E345R/Q347R/K360D/T366V/D399T/Y407A/K409V |
| 830 | Steric hindrance + Charge | E345R/Q347R/K360D/T366V/D399S/Y407A/K409V |
| 831 | Steric hindrance + Charge | E345R/Q347R/K360D/T366V/D399Q/Y407A/K409L |
| 832 | Steric hindrance + Charge | E345R/Q347R/K360D/T366V/D399N/Y407A/K409L |
| 833 | Steric hindrance + Charge | E345R/Q347R/K360D/T366V/D399H/Y407A/K409L |
| 834 | Steric hindrance + Charge | E345R/Q347R/K360D/T366V/D399I/Y407A/K409L |
| 835 | Steric hindrance + Charge | E345R/Q347R/K360D/T366V/D399F/Y407A/K409L |
| 836 | Steric hindrance + Charge | E345R/Q347R/K360D/T366V/D399T/Y407A/K409L |
| 837 | Steric hindrance + Charge | E345R/Q347R/K360D/T366V/D399S/Y407A/K409L |
| 838 | Steric hindrance + Charge | E345R/Q347R/K360D/T366V/D399Q/Y407A/K409I |
| 839 | Steric hindrance + Charge | E345R/Q347R/K360D/T366V/D399N/Y407A/K409I |
| 840 | Steric hindrance + Charge | E345R/Q347R/K360D/T366V/D399H/Y407A/K409I |
| 841 | Steric hindrance + Charge | E345R/Q347R/K360D/T366V/D399V/Y407A/K409I |
| 842 | Steric hindrance + Charge | E345R/Q347R/K360D/T366V/D399L/Y407A/K409I |
| 843 | Steric hindrance + Charge | E345R/Q347R/K360D/T366V/D399F/Y407A/K409I |
| 844 | Steric hindrance + Charge | E345R/Q347R/K360D/T366V/D399Y/Y407A/K409I |
| 845 | Steric hindrance + Charge | E345R/Q347R/K360D/T366V/D399T/Y407A/K409I |
| 846 | Steric hindrance + Charge | E345R/Q347R/K360D/T366V/D399S/Y407A/K409I |
| 847 | Steric hindrance + Charge | E345R/Q347R/K360D/T366V/D399Q/Y407A/K409F |
| 848 | Steric hindrance + Charge | E345R/Q347R/K360D/T366V/D399N/Y407A/K409F |
| 849 | Steric hindrance + Charge | E345R/Q347R/K360D/T366V/D399H/Y407A/K409F |
| 850 | Steric hindrance + Charge | E345R/Q347R/K360D/T366V/D399V/Y407A/K409F |
| 851 | Steric hindrance + Charge | E345R/Q347R/K360D/T366V/D399L/Y407A/K409F |
| 852 | Steric hindrance + Charge | E345R/Q347R/K360D/T366V/D399I/Y407A/K409F |
| 853 | Steric hindrance + Charge | E345R/Q347R/K360D/T366V/D399F/Y407A/K409F |
| 854 | Steric hindrance + Charge | E345R/Q347R/K360D/T366V/D399Y/Y407A/K409F |
| 855 | Steric hindrance + Charge | E345R/Q347R/K360D/T366V/D399T/Y407A/K409F |
| 856 | Steric hindrance + Charge | E345R/Q347R/K360D/T366V/D399Q/Y407A/K409Y |
| 857 | Steric hindrance + Charge | E345R/Q347R/K360D/T366V/D399H/Y407A/K409Y |
| 858 | Steric hindrance + Charge | E345R/Q347R/K360D/T366V/D399V/Y407A/K409Y |
| 859 | Steric hindrance + Charge | E345R/Q347R/K360D/T366V/D399L/Y407A/K409Y |
| 860 | Steric hindrance + Charge | E345R/Q347R/K360D/T366V/D399I/Y407A/K409Y |
| 861 | Steric hindrance + Charge | E345R/Q347R/K360D/T366V/D399F/Y407A/K409Y |
| 862 | Steric hindrance + Charge | E345R/Q347R/K360D/T366V/D399Y/Y407A/K409Y |
| 863 | Steric hindrance + Charge | E345R/Q347R/K360D/T366V/D399S/Y407A/K409Y |
| 864 | Steric hindrance + Charge | E345R/Q347R/K360D/T366V/D399N/Y407A/K409T |
| 865 | Steric hindrance + Charge | E345R/Q347R/K360D/T366V/D399H/Y407A/K409T |
| 866 | Steric hindrance + Charge | E345R/Q347R/K360D/T366V/D399V/Y407A/K409T |
| 867 | Steric hindrance + Charge | E345R/Q347R/K360D/T366V/D399L/Y407A/K409T |
| 868 | Steric hindrance + Charge | E345R/Q347R/K360D/T366V/D399I/Y407A/K409T |
| 869 | Steric hindrance + Charge | E345R/Q347R/K360D/T366V/D399F/Y407A/K409T |
| 870 | Steric hindrance + Charge | E345R/Q347R/K360D/T366V/D399Y/Y407A/K409T |
| 871 | Steric hindrance + Charge | E345R/Q347R/K360D/T366V/D399T/Y407A/K409T |
| 872 | Steric hindrance + Charge | E345R/Q347R/K360D/T366V/D399S/Y407A/K409T |
| 873 | Steric hindrance + Charge | E345R/Q347R/K360D/T366V/D399Q/Y407A/K409S |
| 874 | Steric hindrance + Charge | E345R/Q347R/K360D/T366V/D399N/Y407A/K409S |
| 875 | Steric hindrance + Charge | E345R/Q347R/K360D/T366V/D399H/Y407A/K409S |
| 876 | Steric hindrance + Charge | E345R/Q347R/K360D/T366V/D399V/Y407A/K409S |
| 877 | Steric hindrance + Charge | E345R/Q347R/K360D/T366V/D399L/Y407A/K409S |
| 878 | Steric hindrance + Charge | E345R/Q347R/K360D/T366V/D399I/Y407A/K409S |
| 879 | Steric hindrance + Charge | E345R/Q347R/K360D/T366V/D399F/Y407A/K409S |
| 880 | Steric hindrance + Charge | E345R/Q347R/K360D/T366V/D399Y/Y407A/K409S |
| 881 | Steric hindrance + Charge | E345R/Q347R/K360D/T366V/D399T/Y407A/K409S |
| 882 | Steric hindrance + Charge | E345R/Q347R/K360D/T366V/D399S/Y407A/K409S |
| 883 | Steric hindrance + Charge | E345R/Q347R/K360D/T366V/D399M/Y407A/K409Q |
| 885 | Steric hindrance + Charge | E345R/Q347R/K360D/T366V/D399M/Y407A/K409H |
| 886 | Steric hindrance + Charge | E345R/Q347R/K360D/T366V/D399M/Y407A/K409L |
| 887 | Steric hindrance + Charge | E345R/Q347R/K360D/T366V/D399M/Y407A/K409I |
| 888 | Steric hindrance + Charge | E345R/Q347R/K360D/T366V/D399M/Y407A/K409F |
| 889 | Steric hindrance + Charge | E345R/Q347R/K360D/T366V/D399M/Y407A/K409Y |
| 890 | Steric hindrance + Charge | E345R/Q347R/K360D/T366V/D399M/Y407A/K409T |
| 891 | Steric hindrance + Charge | E345R/Q347R/K360D/T366V/D399M/Y407A/K409S |
| 892 | Steric hindrance + Charge | E345R/Q347R/K360D/T366V/D399V/Y407A/K409Q |
| 893 | Steric hindrance + Charge | E345R/Q347R/K360D/T366V/D399L/Y407A/K409Q |
| 894 | Steric hindrance + Charge | E345R/Q347R/K360D/T366V/D399I/Y407A/K409N |
| 895 | Steric hindrance + Charge | E345R/Q347R/K360D/T366V/D399I/Y407A/K409H |
| 896 | Steric hindrance + Charge | E345R/Q347R/K360D/T366V/D399V/Y407A/K409L |
| 897 | Steric hindrance + Charge | E345R/Q347R/K360D/T366V/D399Y/Y407A/K409L |
| 898 | Steric hindrance + Charge | E345R/Q347R/K360D/T366V/D399S/Y407A/K409F |
| 899 | Steric hindrance + Charge | E345R/Q347R/K360D/T366V/D399N/Y407A/K409Y |
| 900 | Steric hindrance + Charge | E345R/Q347R/K360D/T366V/D399T/Y407A/K409Y |
| 901 | Steric hindrance + Charge | E345R/Q347R/K360D/T366V/D399Q/Y407A/K409T |
| 903 | Steric hindrance + Charge | Q347R/K360E/D399V/F405T/K409F |
| 904 | Steric hindrance + Charge | Q347K/K360E/D399V/F405T/K409W |
| 905 | Steric hindrance + Charge | Q347Y/K360E/D399V/F405T/K409W |
| 906 | Steric hindrance + Charge | Q347R/K360D/D399V/F405T/K409W |
| 907 | Steric hindrance + Charge | Q347K/K360D/D399V/F405T/K409W |
| 908 | Steric hindrance + Charge | Q347H/K360D/D399V/F405T/K409W |
| 909 | Steric hindrance + Charge | Q347Y/K360D/D399V/F405T/K409W |
| 910 | Steric hindrance + Charge | Q347R/K360E/D399L/F405T/K409W |
| 911 | Steric hindrance + Charge | Q347R/K360E/D399I/F405T/K409W |
| 912 | Steric hindrance + Charge | Q347R/K360E/D399M/F405T/K409W |
| 913 | Steric hindrance + Charge | Q347R/K360E/D399S/F405T/K409W |
| 914 | Steric hindrance + Charge | Q347R/K360E/D399T/F405T/K409W |
| 915 | Steric hindrance + Charge + Disulfide bond | Q347R/K360E/D399C/F405T/K409W |
| 916 | Steric hindrance + Charge | Q347R/K360E/D399H/F405T/K409W |
| 917 | Steric hindrance + Charge | Q347R/K360E/D399V/F405A/K409W |
| 918 | Steric hindrance + Charge | Q347R/K360E/D399V/F405V/K409W |
| 919 | Steric hindrance + Charge | Q347R/K360E/D399V/F405S/K409W |
| 920 | Steric hindrance + Charge + Disulfide bond | Q347R/K360E/D399V/F405C/K409W |
| 921 | Steric hindrance + Charge | Q347R/K360E/D399V/F405N/K409W |
| 922 | Steric hindrance + Charge | Q347R/K360E/D399V/F405D/K409W |
| 923 | Steric hindrance + Charge | Q347R/K360E/D399V/F405T/K409Y |
| 924 | Steric hindrance + Charge | Q347R/K360E/D399V/F405T/K409H |
| 925 | Steric hindrance + Charge | Q347R/K360E/D399A/F405T/K409F |
| 926 | Steric hindrance + Charge | Q347R/K360E/D399L/F405T/K409F |
| 927 | Steric hindrance + Charge | Q347R/K360E/D399I/F405T/K409F |
| 928 | Steric hindrance + Charge | Q347R/K360E/D399M/F405T/K409F |
| 929 | Steric hindrance + Charge | Q347R/K360E/D399T/F405T/K409F |
| 930 | Steric hindrance + Charge + Disulfide bond | Q347R/K360E/D399C/F405T/K409F |
| 931 | Steric hindrance + Charge | Q347R/K360E/D399N/F405T/K409F |
| 932 | Steric hindrance + Charge | Q347R/K360E/D399Q/F405T/K409F |
| 933 | Steric hindrance + Charge | Q347R/K360E/D399H/F405T/K409F |
| 934 | Steric hindrance + Charge | Q347R/K360E/D399A/F405T/K409Y |
| 935 | Steric hindrance + Charge | Q347R/K360E/D399L/F405T/K409Y |
| 936 | Steric hindrance + Charge | Q347R/K360E/D399M/F405T/K409Y |
| 937 | Steric hindrance + Charge | Q347R/K360E/D399S/F405T/K409Y |
| 938 | Steric hindrance + Charge | Q347R/K360E/D399T/F405T/K409Y |
| 939 | Steric hindrance + Charge + Disulfide bond | Q347R/K360E/D399C/F405T/K409Y |
| 940 | Steric hindrance + Charge | Q347R/K360E/D399N/F405T/K409Y |
| 941 | Steric hindrance + Charge | Q347R/K360E/D399Q/F405T/K409Y |
| 942 | Steric hindrance + Charge | Q347R/K360E/D399H/F405T/K409Y |
| 943 | Steric hindrance + Charge | Q347R/K360E/D399V/F405A/K409F |
| 944 | Steric hindrance + Charge | Q347R/K360E/D399V/F405V/K409F |
| 945 | Steric hindrance + Charge | Q347R/K360E/D399V/F405S/K409F |
| 946 | Steric hindrance + Charge + Disulfide bond | Q347R/K360E/D399V/F405C/K409F |
| 947 | Steric hindrance + Charge | Q347R/K360E/D399V/F405D/K409F |
| 948 | Steric hindrance + Charge | Q347R/K360E/D399V/F405A/K409Y |
| 949 | Steric hindrance + Charge + Disulfide bond | Q347R/K360E/D399V/F405C/K409Y |
| 950 | Steric hindrance + Charge | Q347R/K360E/D399V/F405N/K409Y |
| 951 | Steric hindrance + Charge | Q347R/K360E/D399V/F405D/K409Y |
| 952 | Steric hindrance + Charge | Q347R/K360E/D399L/F405A/K409W |
| 953 | Steric hindrance + Charge | Q347R/K360E/D399L/F405S/K409W |
| 954 | Steric hindrance + Charge + Disulfide bond | Q347R/K360E/D399L/F405C/K409W |
| 955 | Steric hindrance + Charge | Q347R/K360E/D399I/F405S/K409W |
| 956 | Steric hindrance + Charge + Disulfide bond | Q347R/K360E/D399I/F405C/K409W |
| 957 | Steric hindrance + Charge | Q347R/K360E/D399M/F405A/K409W |
| 958 | Steric hindrance + Charge | Q347R/K360E/D399M/F405S/K409W |
| 959 | Steric hindrance + Charge + Disulfide bond | Q347R/K360E/D399M/F405C/K409W |
| 960 | Steric hindrance + Charge | Q347R/K360E/D399G/F405T/K409W |
| 961 | Steric hindrance + Charge | Q347H/K360E/D399V/F405T/K409W |
| 962 | Steric hindrance + Charge | Q347R/K360E/D399A/F405T/K409W |
| 963 | Steric hindrance + Charge | Q347R/K360E/D399N/F405T/K409W |
| 964 | Steric hindrance + Charge | Q347R/K360E/D399Q/F405T/K409W |
| 965 | Steric hindrance + Charge | Q347R/K360E/D399S/F405T/K409F |
| 966 | Steric hindrance + Charge | Q347R/K360E/D399V/F405N/K409F |
| 967 | Steric hindrance + Charge | Q347R/K360E/D399V/F405V/K409Y |
| 968 | Steric hindrance + Charge | Q347R/K360E/D399V/F405S/K409Y |
| 969 | Steric hindrance + Charge | Q347R/K360E/D399I/F405A/K409W |
| 970 | Steric hindrance + Charge | Q347R/K360E/D399V/F405G/K409W |
| 971 | Steric hindrance | T394W/F405S |
| 972 | Steric hindrance | T394W/F405T |
| 973 | Steric hindrance | T394Y/F405A |
| 975 | Steric hindrance | T394Y/F405T |
| 976 | Steric hindrance + Disulfide bond | T394Y/F405C |
| 978 | Steric hindrance | T394F/F405G |
| 979 | Steric hindrance + Disulfide bond | T394F/F405C |
| 980 | Steric hindrance | T394F/F405V |
| 986 | Steric hindrance + Disulfide bond | T394W/F405C |
| 987 | Steric hindrance | T394W/F405V |
| 988 | Steric hindrance | T394Y/F405G |
| 989 | Steric hindrance | T394Y/F405S |
| 990 | Steric hindrance | T394F/F405S |
| 991 | Steric hindrance | T394F/F405T |
| 993 | Steric hindrance | T394W/F405G |
| 994 | Steric hindrance | T366W/L368S/Y407V |
| 995 | Steric hindrance | T366W/L368T/Y407V |
| 996 | Steric hindrance + Disulfide bond | T366W/L368C/Y407V |
| 997 | Steric hindrance | T366W/L368V/Y407V |
| 998 | Steric hindrance | T366W/L368G/Y407V |
| 999 | Steric hindrance | T366Y/L368A/Y407V |
| 1000 | Steric hindrance | T366F/L368A/Y407V |
| 1002 | Steric hindrance | T366W/L368A/Y407L |
| 1003 | Steric hindrance | T366W/L368A/Y407I |
| 1004 | Steric hindrance | T366W/L368A/Y407M |
| 1005 | Steric hindrance | T366W/L368A/Y407A |
| 1006 | Steric hindrance | T366W/L368A/Y407S |
| 1007 | Steric hindrance | T366W/L368A/Y407T |
| 1008 | Steric hindrance + Disulfide bond | T366W/L368A/Y407C |
| 1010 | Steric hindrance | T366W/L368A/Y407N |
| 1011 | Steric hindrance + Disulfide bond | T366W/L368C/Y407C |
| 1013 | Steric hindrance | T366W/L368S/Y407L |
| 1014 | Steric hindrance | T366W/L368T/Y407L |
| 1015 | Steric hindrance + Disulfide bond | T366W/L368C/Y407L |
| 1016 | Steric hindrance | T366W/L368V/Y407L |
| 1017 | Steric hindrance | T366W/L368G/Y407L |
| 1019 | Steric hindrance + Disulfide bond | T366W/L368C/Y4071 |
| 1020 | Steric hindrance | T366W/L368V/Y407I |
| 1021 | Steric hindrance | T366W/L368G/Y407I |
| 1022 | Steric hindrance | T366W/L368S/Y407M |
| 1023 | Steric hindrance | T366W/L368T/Y407M |
| 1024 | Steric hindrance + Disulfide bond | T366W/L368C/Y407M |
| 1025 | Steric hindrance | T366W/L368V/Y407M |
| 1026 | Steric hindrance | T366W/L368G/Y407M |
| 1027 | Steric hindrance | T366W/L368S/Y407A |
| 1028 | Steric hindrance | T366W/L368T/Y407A |
| 1029 | Steric hindrance + Disulfide bond | T366W/L368C/Y407A |
| 1030 | Steric hindrance | T366W/L368V/Y407A |
| 1031 | Steric hindrance | T366W/L368G/Y407A |
| 1032 | Steric hindrance | T366W/L368S/Y407T |
| 1033 | Steric hindrance | T366W/L368T/Y407T |
| 1034 | Steric hindrance + Disulfide bond | T366W/L368C/Y407T |
| 1035 | Steric hindrance | T366W/L368V/Y407T |
| 1036 | Steric hindrance | T366W/L368G/Y407T |
| 1037 | Steric hindrance + Disulfide bond | T366W/L368S/Y407C |
| 1038 | Steric hindrance + Disulfide bond | T366W/L368T/Y407C |
| 1039 | Steric hindrance + Disulfide bond | T366W/L368V/Y407C |
| 1040 | Steric hindrance + Disulfide bond | T366W/L368G/Y407C |
| 1045 | Steric hindrance | T366W/L368S/Y407N |
| 1046 | Steric hindrance | T366W/L368T/Y407N |
| 1049 | Steric hindrance | T366W/L368S/Y407Q |
| 1050 | Steric hindrance | T366W/L368T/Y407Q |
| 1054 | Steric hindrance | T366Y/L368S/Y407L |
| 1055 | Steric hindrance + Disulfide bond | T366Y/L368C/Y407L |
| 1056 | Steric hindrance | T366Y/L368V/Y407L |
| 1058 | Steric hindrance | T366Y/L368S/Y407I |
| 1059 | Steric hindrance + Disulfide bond | T366Y/L368C/Y407I |
| 1060 | Steric hindrance | T366Y/L368V/Y407I |
| 1061 | Steric hindrance | T366Y/L368G/Y407I |
| 1062 | Steric hindrance | T366Y/L368G/Y407M |
| 1063 | Steric hindrance | T366Y/L368V/Y407A |
| 1065 | Steric hindrance | T366Y/L368S/Y407T |
| 1066 | Steric hindrance | T366Y/L368T/Y407T |
| 1067 | Steric hindrance + Disulfide bond | T366Y/L368C/Y407T |
| 1068 | Steric hindrance | T366Y/L368V/Y407T |
| 1070 | Steric hindrance + Disulfide bond | T366Y/L368T/Y407C |
| 1071 | Steric hindrance + Disulfide bond | T366Y/L368V/Y407C |
| 1078 | Steric hindrance | T366Y/L368S/Y407N |
| 1083 | Steric hindrance | T366Y/L368S/Y407Q |
| 1086 | Steric hindrance | T366W/L368A/Y407Q |
| 1087 | Steric hindrance | T366W/L368T/Y407I |
| 1091 | Steric hindrance + Disulfide bond | T366Y/L368C/Y407C |
| 1092 | Steric hindrance | T366F/L368S/Y407L |
| 1094 | Steric hindrance + Disulfide bond | T366F/L368C/Y407L |
| 1095 | Steric hindrance | T366F/L368V/Y407L |
| 1096 | Steric hindrance | T366F/L368G/Y407L |
| 1097 | Steric hindrance | T366F/L368S/Y407I |
| 1098 | Steric hindrance | T366F/L368T/Y407I |
| 1099 | Steric hindrance + Disulfide bond | T366F/L368C/Y407I |
| 1100 | Steric hindrance | T366F/L368V/Y407I |
| 1101 | Steric hindrance | T366F/L368G/Y407I |
| 1102 | Steric hindrance | T366F/L368S/Y407M |
| 1103 | Steric hindrance | T366F/L368T/Y407M |
| 1104 | Steric hindrance + Disulfide bond | T366F/L368C/Y407M |
| 1105 | Steric hindrance | T366F/L368V/Y407M |
| 1107 | Steric hindrance | T366F/L368S/Y407A |
| 1108 | Steric hindrance | T366F/L368T/Y407A |
| 1109 | Steric hindrance + Disulfide bond | T366F/L368C/Y407A |
| 1110 | Steric hindrance | T366F/L368V/Y407A |
| 1112 | Steric hindrance | T366F/L368S/Y407T |
| 1113 | Steric hindrance | T366F/L368T/Y407T |
| 1114 | Steric hindrance + Disulfide bond | T366F/L368C/Y407T |
| 1115 | Steric hindrance | T366F/L368V/Y407T |
| 1117 | Steric hindrance + Disulfide bond | T366F/L368S/Y407C |
| 1118 | Steric hindrance + Disulfide bond | T366F/L368T/Y407C |
| 1119 | Steric hindrance + Disulfide bond | T366F/L368V/Y407C |
| 1134 | Steric hindrance + Disulfide bond | T366F/L368C/Y407C |
| 1135 | Steric hindrance | T366Y/L368T/Y407I |
| 1138 | Steric hindrance + Disulfide bond | T366Y/L368S/Y407C |
| 1140 | Steric hindrance | T366Y/L368S/Y407M |
| 1141 | Steric hindrance | T366Y/L368T/Y407M |
| 1142 | Steric hindrance + Disulfide bond | T366Y/L368C/Y407M |
| 1143 | Steric hindrance | T366Y/L368V/Y407M |
| 1144 | Steric hindrance | T366Y/L368S/Y407A |
| 1145 | Steric hindrance | T366Y/L368T/Y407A |
| 1146 | Steric hindrance + Disulfide bond | T366Y/L368C/Y407A |
| 1147 | Steric hindrance | T366Y/L368G/Y407Q |
| 1148 | Steric hindrance + Charge | Q347R/K360E/D399I/F405T/K409Y |
| 1149 | Steric hindrance + Charge | E345R/Q347R/K360D/T366V/D399L/Y407A/K409L |
| 1150 | Steric hindrance + Charge | E345R/Q347R/K360D/T366V/D399I/Y407A/K409I |

**(Table 17) Amino acid modifications that showed strong heavy-chain homodimerization-promoting ability with a heteromultimer ratio (%) of 0% in Table 15**

| Sample No. | Category of modification effect | Modifications that promote formation of heavy chain homodimers |
|---|---|---|
| 766 | Charge | E356R/D399K/K409E/K439E |
| 768 | Charge | E356K/D399R/K409E/K439E |
| 769 | Charge | E356K/D399H/K409E/K439E |
| 770 | Charge | E356K/D399K/K409D/K439E |
| 771 | Charge | E356K/D399K/K409E/K439D |
| 772 | Charge | E356K/D399R/K409D/K439E |
| 779 | Charge | K392D/D399K/K409E |
| 781 | Charge | E356K/K392D/D399K/K409E |
| 782 | Charge | E356K/K392D/D399R/K409D |
| 783 | Charge | E356R/K392D/D399K/K409D |
| 784 | Charge | E356H/K392D/D399K/K409D |
| 785 | Charge | E356K/K392E/D399K/K409D |
| 787 | Charge | E356K/K392D/D399H/K409D |
| 788 | Charge | E356R/D399K/K409E/K439D |
| 790 | Steric hindrance + Charge | Q347R/K360D/T366V/D399M/Y407A/K409V |
| 791 | Steric hindrance + Charge | E345R/Q347K/K360D/T366V/D399M/Y407A/K409V |
| 792 | Steric hindrance + Charge | E345R/Q347R/K360E/T366V/D399M/Y407A/K409V |
| 793 | Steric hindrance + Charge | Q347K/K360D/T366V/D399M/Y407A/K409V |
| 794 | Steric hindrance + Charge | Q347R/K360E/T366V/D399M/Y407A/K409V |
| 795 | Steric hindrance + Charge | Q347K/K360E/T366V/D399M/Y407A/K409V |
| 797 | Steric hindrance + Charge | E345R/Q347R/K360D/T366V/D399N/Y407A/K409Q |
| 798 | Steric hindrance + Charge | E345R/Q347R/K360D/T366V/D399H/Y407A/K409Q |
| 799 | Steric hindrance + Charge | E345R/Q347R/K360D/T366V/D399I/Y407A/K409Q |
| 800 | Steric hindrance + Charge | E345R/Q347R/K360D/T366V/D399F/Y407A/K409Q |
| 801 | Steric hindrance + Charge | E345R/Q347R/K360D/T366V/D399Y/Y407A/K409Q |
| 802 | Steric hindrance + Charge | E345R/Q347R/K360D/T366V/D399T/Y407A/K409Q |
| 803 | Steric hindrance + Charge | E345R/Q347R/K360D/T366V/D399S/Y407A/K409Q |
| 815 | Steric hindrance + Charge | E345R/Q347R/K360D/T366V/D399V/Y407A/K409H |
| 816 | Steric hindrance + Charge | E345R/Q347R/K360D/T366V/D399L/Y407A/K409H |
| 819 | Steric hindrance + Charge | E345R/Q347R/K360D/T366V/D399T/Y407A/K409H |
| 820 | Steric hindrance + Charge | E345R/Q347R/K360D/T366V/D399S/Y407A/K409H |
| 821 | Steric hindrance + Charge | E345R/Q347R/K360D/T366V/D399Q/Y407A/K409V |
| 822 | Steric hindrance + Charge | E345R/Q347R/K360D/T366V/D399N/Y407A/K409V |
| 823 | Steric hindrance + Charge | E345R/Q347R/K360D/T366V/D399H/Y407A/K409V |
| 824 | Steric hindrance + Charge | E345R/Q347R/K360D/T366V/D399V/Y407A/K409V |
| 825 | Steric hindrance + Charge | E345R/Q347R/K360D/T366V/D399L/Y407A/K409V |
| 826 | Steric hindrance + Charge | E345R/Q347R/K360D/T366V/D399I/Y407A/K409V |
| 827 | Steric hindrance + Charge | E345R/Q347R/K360D/T366V/D399F/Y407A/K409V |
| 828 | Steric hindrance + Charge | E345R/Q347R/K360D/T366V/D399Y/Y407A/K409V |
| 829 | Steric hindrance + Charge | E345R/Q347R/K360D/T366V/D399T/Y407A/K409V |
| 830 | Steric hindrance + Charge | E345R/Q347R/K360D/T366V/D399S/Y407A/K409V |
| 831 | Steric hindrance + Charge | E345R/Q347R/K360D/T366V/D399Q/Y407A/K409L |
| 832 | Steric hindrance + Charge | E345R/Q347R/K360D/T366V/D399N/Y407A/K409L |
| 833 | Steric hindrance + Charge | E345R/Q347R/K360D/T366V/D399H/Y407A/K409L |
| 834 | Steric hindrance + Charge | E345R/Q347R/K360D/T366V/D399I/Y407A/K409L |
| 835 | Steric hindrance + Charge | E345R/Q347R/K360D/T366V/D399F/Y407A/K409L |
| 836 | Steric hindrance + Charge | E345R/Q347R/K360D/T366V/D399T/Y407A/K409L |
| 837 | Steric hindrance + Charge | E345R/Q347R/K360D/T366V/D399S/Y407A/K409L |
| 838 | Steric hindrance + Charge | E345R/Q347R/K360D/T366V/D399Q/Y407A/K409I |
| 839 | Steric hindrance + Charge | E345R/Q347R/K360D/T366V/D399N/Y407A/K409I |
| 840 | Steric hindrance + Charge | E345R/Q347R/K360D/T366V/D399H/Y407A/K409I |
| 841 | Steric hindrance + Charge | E345R/Q347R/K360D/T366V/D399V/Y407A/K409I |
| 842 | Steric hindrance + Charge | E345R/Q347R/K360D/T366V/D399L/Y407A/K409I |
| 843 | Steric hindrance + Charge | E345R/Q347R/K360D/T366V/D399F/Y407A/K409I |
| 844 | Steric hindrance + Charge | E345R/Q347R/K360D/T366V/D399Y/Y407A/K409I |
| 845 | Steric hindrance + Charge | E345R/Q347R/K360D/T366V/D399T/Y407A/K409I |
| 846 | Steric hindrance + Charge | E345R/Q347R/K360D/T366V/D399S/Y407A/K409I |
| 847 | Steric hindrance + Charge | E345R/Q347R/K360D/T366V/D399Q/Y407A/K409F |
| 848 | Steric hindrance + Charge | E345R/Q347R/K360D/T366V/D399N/Y407A/K409F |
| 849 | Steric hindrance + Charge | E345R/Q347R/K360D/T366V/D399H/Y407A/K409F |
| 850 | Steric hindrance + Charge | E345R/Q347R/K360D/T366V/D399V/Y407A/K409F |
| 851 | Steric hindrance + Charge | E345R/Q347R/K360D/T366V/D399L/Y407A/K409F |
| 852 | Steric hindrance + Charge | E345R/Q347R/K360D/T366V/D399I/Y407A/K409F |
| 853 | Steric hindrance + Charge | E345R/Q347R/K360D/T366V/D399F/Y407A/K409F |
| 854 | Steric hindrance + Charge | E345R/Q347R/K360D/T366V/D399Y/Y407A/K409F |
| 855 | Steric hindrance + Charge | E345R/Q347R/K360D/T366V/D399T/Y407A/K409F |
| 858 | Steric hindrance + Charge | E345R/Q347R/K360D/T366V/D399V/Y407A/K409Y |
| 859 | Steric hindrance + Charge | E345R/Q347R/K360D/T366V/D399L/Y407A/K409Y |
| 860 | Steric hindrance + Charge | E345R/Q347R/K360D/T366V/D399I/Y407A/K409Y |
| 861 | Steric hindrance + Charge | E345R/Q347R/K360D/T366V/D399F/Y407A/K409Y |
| 863 | Steric hindrance + Charge | E345R/Q347R/K360D/T366V/D399S/Y407A/K409Y |
| 864 | Steric hindrance + Charge | E345R/Q347R/K360D/T366V/D399N/Y407A/K409T |
| 866 | Steric hindrance + Charge | E345R/Q347R/K360D/T366V/D399V/Y407A/K409T |
| 867 | Steric hindrance + Charge | E345R/Q347R/K360D/T366V/D399L/Y407A/K409T |
| 868 | Steric hindrance + Charge | E345R/Q347R/K360D/T366V/D399I/Y407A/K409T |
| 871 | Steric hindrance + Charge | E345R/Q347R/K360D/T366V/D399T/Y407A/K409T |
| 872 | Steric hindrance + Charge | E345R/Q347R/K360D/T366V/D399S/Y407A/K409T |
| 873 | Steric hindrance + Charge | E345R/Q347R/K360D/T366V/D399Q/Y407A/K409S |
| 874 | Steric hindrance + Charge | E345R/Q347R/K360D/T366V/D399N/Y407A/K409S |
| 875 | Steric hindrance + Charge | E345R/Q347R/K360D/T366V/D399H/Y407A/K409S |
| 876 | Steric hindrance + Charge | E345R/Q347R/K360D/T366V/D399V/Y407A/K409S |
| 877 | Steric hindrance + Charge | E345R/Q347R/K360D/T366V/D399L/Y407A/K409S |
| 878 | Steric hindrance + Charge | E345R/Q347R/K360D/T366V/D399I/Y407A/K409S |
| 879 | Steric hindrance + Charge | E345R/Q347R/K360D/T366V/D399F/Y407A/K409S |
| 880 | Steric hindrance + Charge | E345R/Q347R/K360D/T366V/D399Y/Y407A/K409S |
| 881 | Steric hindrance + Charge | E345R/Q347R/K360D/T366V/D399T/Y407A/K409S |
| 882 | Steric hindrance + Charge | E345R/Q347R/K360D/T366V/D399S/Y407A/K409S |
| 883 | Steric hindrance + Charge | E345R/Q347R/K360D/T366V/D399M/Y407A/K409Q |
| 886 | Steric hindrance + Charge | E345R/Q347R/K360D/T366V/D399M/Y407A/K409L |
| 887 | Steric hindrance + Charge | E345R/Q347R/K360D/T366V/D399M/Y407A/K409I |
| 888 | Steric hindrance + Charge | E345R/Q347R/K360D/T366V/D399M/Y407A/K409F |
| 889 | Steric hindrance + Charge | E345R/Q347R/K360D/T366V/D399M/Y407A/K409Y |
| 890 | Steric hindrance + Charge | E345R/Q347R/K360D/T366V/D399M/Y407A/K409T |
| 891 | Steric hindrance + Charge | E345R/Q347R/K360D/T366V/D399M/Y407A/K409S |
| 892 | Steric hindrance + Charge | E345R/Q347R/K360D/T366V/D399V/Y407A/K409Q |
| 893 | Steric hindrance + Charge | E345R/Q347R/K360D/T366V/D399L/Y407A/K409Q |
| 895 | Steric hindrance + Charge | E345R/Q347R/K360D/T366V/D399I/Y407A/K409H |
| 896 | Steric hindrance + Charge | E345R/Q347R/K360D/T366V/D399V/Y407A/K409L |
| 897 | Steric hindrance + Charge | E345R/Q347R/K360D/T366V/D399Y/Y407A/K409L |
| 898 | Steric hindrance + Charge | E345R/Q347R/K360D/T366V/D399S/Y407A/K409F |
| 900 | Steric hindrance + Charge | E345R/Q347R/K360D/T366V/D399T/Y407A/K409Y |
| 901 | Steric hindrance + Charge | E345R/Q347R/K360D/T366V/D399Q/Y407A/K409T |
| 903 | Steric hindrance + Charge | Q347R/K360E/D399V/F405T/K409F |
| 904 | Steric hindrance + Charge | Q347K/K360E/D399V/F405T/K409W |
| 905 | Steric hindrance + Charge | Q347Y/K360E/D399V/F405T/K409W |
| 906 | Steric hindrance + Charge | Q347R/K360D/D399V/F405T/K409W |
| 907 | Steric hindrance + Charge | Q347K/K360D/D399V/F405T/K409W |
| 908 | Steric hindrance + Charge | Q347H/K360D/D399V/F405T/K409W |
| 910 | Steric hindrance + Charge | Q347R/K360E/D399L/F405T/K409W |
| 911 | Steric hindrance + Charge | Q347R/K360E/D3991/F405T/K409W |
| 912 | Steric hindrance + Charge | Q347R/K360E/D399M/F405T/K409W |
| 913 | Steric hindrance + Charge | Q347R/K360E/D399S/F405T/K409W |
| 914 | Steric hindrance + Charge | Q347R/K360E/D399T/F405T/K409W |
| 915 | Steric hindrance + Charge + Disulfide bond | Q347R/K360E/D399C/F405T/K409W |
| 916 | Steric hindrance + Charge | Q347R/K360E/D399H/F405T/K409W |
| 917 | Steric hindrance + Charge | Q347R/K360E/D399V/F405A/K409W |
| 918 | Steric hindrance + Charge | Q347R/K360E/D399V/F405V/K409W |
| 926 | Steric hindrance + Charge | Q347R/K360E/D399L/F405T/K409F |
| 927 | Steric hindrance + Charge | Q347R/K360E/D399I/F405T/K409F |
| 928 | Steric hindrance + Charge | Q347R/K360E/D399M/F405T/K409F |
| 929 | Steric hindrance + Charge | Q347R/K360E/D399T/F405T/K409F |
| 931 | Steric hindrance + Charge | Q347R/K360E/D399N/F405T/K409F |
| 943 | Steric hindrance + Charge | Q347R/K360E/D399V/F405A/K409F |
| 944 | Steric hindrance + Charge | Q347R/K360E/D399V/F405V/K409F |
| 952 | Steric hindrance + Charge | Q347R/K360E/D399L/F405A/K409W |
| 957 | Steric hindrance + Charge | Q347R/K360E/D399M/F405A/K409W |
| 960 | Steric hindrance + Charge | Q347R/K360E/D399G/F405T/K409W |
| 961 | Steric hindrance + Charge | Q347H/K360E/D399V/F405T/K409W |
| 962 | Steric hindrance + Charge | Q347R/K360E/D399A/F405T/K409W |
| 963 | Steric hindrance + Charge | Q347R/K360E/D399N/F405T/K409W |
| 969 | Steric hindrance + Charge | Q347R/K360E/D399I/F405A/K409W |
| 971 | Steric hindrance | T394W/F405S |
| 972 | Steric hindrance | T394W/F405T |
| 993 | Steric hindrance | T394W/F405G |
| 996 | Steric hindrance + Disulfide bond | T366W/L368C/Y407V |
| 998 | Steric hindrance | T366W/L368G/Y407V |
| 999 | Steric hindrance | T366Y/L368A/Y407V |
| 1000 | Steric hindrance | T366F/L368A/Y407V |
| 1002 | Steric hindrance | T366W/L368A/Y407L |
| 1011 | Steric hindrance + Disulfide bond | T366W/L368C/Y407C |
| 1031 | Steric hindrance | T366W/L368G/Y407A |
| 1063 | Steric hindrance | T366Y/L368V/Y407A |
| 1087 | Steric hindrance | T366W/L368T/Y4071 |
| 1095 | Steric hindrance | T366F/L368V/Y407L |
| 1100 | Steric hindrance | T366F/L368V/Y407I |
| 1105 | Steric hindrance | T366F/L368V/Y407M |
| 1134 | Steric hindrance + Disulfide bond | T366F/L368C/Y407C |
| 1143 | Steric hindrance | T366Y/L368V/Y407M |
| 1148 | Steric hindrance + Charge | Q347R/K360E/D399I/F405T/K409Y |
| 1149 | Steric hindrance + Charge | E345R/Q347R/K360D/T366V/D399L/Y407A/K409L |
| 1150 | Steric hindrance + Charge | E345R/Q347R/K360D/T366V/D399I/Y407A/K409I |

**(Table 18) Amino acid modifications that showed a heteromultimer ratio (%) of 0%, a homodimer monomer ratio of 95% or higher, a Tm of 65°C or higher, and a yield (mg) of 0.15 mg or more in Table 15**

| Sample No. | Category of modification effect | Modifications that promote formation of heavy chain homodimers |
|---|---|---|
| 769 | Charge | E356K/D399H/K409E/K439E |
| 772 | Charge | E356K/D399R/K409D/K439E |
| 779 | Charge | K392D/D399K/K409E |
| 791 | Steric hindrance + Charge | E345R/Q347K/K360D/T366V/D399M/Y407A/K409V |
| 792 | Steric hindrance + Charge | E345R/Q347R/K360E/T366V/D399M/Y407A/K409V |
| 793 | Steric hindrance + Charge | Q347K/K360D/T366V/D399M/Y407A/K409V |
| 794 | Steric hindrance + Charge | Q347R/K360E/T366V/D399M/Y407A/K409V |
| 795 | Steric hindrance + Charge | Q347K/K360E/T366V/D399M/Y407A/K409V |
| 798 | Steric hindrance + Charge | E345R/Q347R/K360D/T366V/D399H/Y407A/K409Q |
| 799 | Steric hindrance + Charge | E345R/Q347R/K360D/T366V/D399I/Y407A/K409Q |
| 802 | Steric hindrance + Charge | E345R/Q347R/K360D/T366V/D399T/Y407A/K409Q |
| 803 | Steric hindrance + Charge | E345R/Q347R/K360D/T366V/D399S/Y407A/K409Q |
| 815 | Steric hindrance + Charge | E345R/Q347R/K360D/T366V/D399V/Y407A/K409H |
| 819 | Steric hindrance + Charge | E345R/Q347R/K360D/T366V/D399T/Y407A/K409H |
| 820 | Steric hindrance + Charge | E345R/Q347R/K360D/T366V/D399S/Y407A/K409H |
| 821 | Steric hindrance + Charge | E345R/Q347R/K360D/T366V/D399Q/Y407A/K409V |
| 822 | Steric hindrance + Charge | E345R/Q347R/K360D/T366V/D399N/Y407A/K409V |
| 823 | Steric hindrance + Charge | E345R/Q347R/K360D/T366V/D399H/Y407A/K409V |
| 824 | Steric hindrance + Charge | E345R/Q347R/K360D/T366V/D399V/Y407A/K409V |
| 825 | Steric hindrance + Charge | E345R/Q347R/K360D/T366V/D399L/Y407A/K409V |
| 826 | Steric hindrance + Charge | E345R/Q347R/K360D/T366V/D399I/Y407A/K409V |
| 827 | Steric hindrance + Charge | E345R/Q347R/K360D/T366V/D399F/Y407A/K409V |
| 828 | Steric hindrance + Charge | E345R/Q347R/K360D/T366V/D399Y/Y407A/K409V |
| 829 | Steric hindrance + Charge | E345R/Q347R/K360D/T366V/D399T/Y407A/K409V |
| 830 | Steric hindrance + Charge | E345R/Q347R/K360D/T366V/D399S/Y407A/K409V |
| 831 | Steric hindrance + Charge | E345R/Q347R/K360D/T366V/D399Q/Y407A/K409L |
| 832 | Steric hindrance + Charge | E345R/Q347R/K360D/T366V/D399N/Y407A/K409L |
| 833 | Steric hindrance + Charge | E345R/Q347R/K360D/T366V/D399H/Y407A/K409L |
| 834 | Steric hindrance + Charge | E345R/Q347R/K360D/T366V/D399I/Y407A/K409L |
| 835 | Steric hindrance + Charge | E345R/Q347R/K360D/T366V/D399F/Y407A/K409L |
| 836 | Steric hindrance + Charge | E345R/Q347R/K360D/T366V/D399T/Y407A/K409L |
| 837 | Steric hindrance + Charge | E345R/Q347R/K360D/T366V/D399S/Y407A/K409L |
| 838 | Steric hindrance + Charge | E345R/Q347R/K360D/T366V/D399Q/Y407A/K409I |
| 839 | Steric hindrance + Charge | E345R/Q347R/K360D/T366V/D399N/Y407A/K409I |
| 840 | Steric hindrance + Charge | E345R/Q347R/K360D/T366V/D399H/Y407A/K409I |
| 841 | Steric hindrance + Charge | E345R/Q347R/K360D/T366V/D399V/Y407A/K409I |
| 842 | Steric hindrance + Charge | E345R/Q347R/K360D/T366V/D399L/Y407A/K409I |
| 844 | Steric hindrance + Charge | E345R/Q347R/K360D/T366V/D399Y/Y407A/K409I |
| 845 | Steric hindrance + Charge | E345R/Q347R/K360D/T366V/D399T/Y407A/K409I |
| 846 | Steric hindrance + Charge | E345R/Q347R/K360D/T366V/D399S/Y407A/K409I |
| 847 | Steric hindrance + Charge | E345R/Q347R/K360D/T366V/D399Q/Y407A/K409F |
| 848 | Steric hindrance + Charge | E345R/Q347R/K360D/T366V/D399N/Y407A/K409F |
| 849 | Steric hindrance + Charge | E345R/Q347R/K360D/T366V/D399H/Y407A/K409F |
| 850 | Steric hindrance + Charge | E345R/Q347R/K360D/T366V/D399V/Y407A/K409F |
| 851 | Steric hindrance + Charge | E345R/Q347R/K360D/T366V/D399L/Y407A/K409F |
| 852 | Steric hindrance + Charge | E345R/Q347R/K360D/T366V/D3991/Y407A/K409F |
| 855 | Steric hindrance + Charge | E345R/Q347R/K360D/T366V/D399T/Y407A/K409F |
| 858 | Steric hindrance + Charge | E345R/Q347R/K360D/T366V/D399V/Y407A/K409Y |
| 863 | Steric hindrance + Charge | E345R/Q347R/K360D/T366V/D399S/Y407A/K409Y |
| 864 | Steric hindrance + Charge | E345R/Q347R/K360D/T366V/D399N/Y407A/K409T |
| 866 | Steric hindrance + Charge | E345R/Q347R/K360D/T366V/D399V/Y407A/K409T |
| 871 | Steric hindrance + Charge | E345R/Q347R/K360D/T366V/D399T/Y407A/K409T |
| 872 | Steric hindrance + Charge | E345R/Q347R/K360D/T366V/D399S/Y407A/K409T |
| 873 | Steric hindrance + Charge | E345R/Q347R/K360D/T366V/D399Q/Y407A/K409S |
| 874 | Steric hindrance + Charge | E345R/Q347R/K360D/T366V/D399N/Y407A/K409S |
| 875 | Steric hindrance + Charge | E345R/Q347R/K360D/T366V/D399H/Y407A/K409S |
| 876 | Steric hindrance + Charge | E345R/Q347R/K360D/T366V/D399V/Y407A/K409S |
| 877 | Steric hindrance + Charge | E345R/Q347R/K360D/T366V/D399L/Y407A/K409S |
| 878 | Steric hindrance + Charge | E345R/Q347R/K360D/T366V/D3991/Y407A/K409S |
| 881 | Steric hindrance + Charge | E345R/Q347R/K360D/T366V/D399T/Y407A/K409S |
| 883 | Steric hindrance + Charge | E345R/Q347R/K360D/T366V/D399M/Y407A/K409Q |
| 886 | Steric hindrance + Charge | E345R/Q347R/K360D/T366V/D399M/Y407A/K409L |
| 887 | Steric hindrance + Charge | E345R/Q347R/K360D/T366V/D399M/Y407A/K409I |
| 888 | Steric hindrance + Charge | E345R/Q347R/K360D/T366V/D399M/Y407A/K409F |
| 889 | Steric hindrance + Charge | E345R/Q347R/K360D/T366V/D399M/Y407A/K409Y |
| 890 | Steric hindrance + Charge | E345R/Q347R/K360D/T366V/D399M/Y407A/K409T |
| 891 | Steric hindrance + Charge | E345R/Q347R/K360D/T366V/D399M/Y407A/K409S |
| 892 | Steric hindrance + Charge | E345R/Q347R/K360D/T366V/D399V/Y407A/K409Q |
| 896 | Steric hindrance + Charge | E345R/Q347R/K360D/T366V/D399V/Y407A/K409L |
| 897 | Steric hindrance + Charge | E345R/Q347R/K360D/T366V/D399Y/Y407A/K409L |
| 898 | Steric hindrance + Charge | E345R/Q347R/K360D/T366V/D399S/Y407A/K409F |
| 900 | Steric hindrance + Charge | E345R/Q347R/K360D/T366V/D399T/Y407A/K409Y |
| 901 | Steric hindrance + Charge | E345R/Q347R/K360D/T366V/D399Q/Y407A/K409T |
| 903 | Steric hindrance + Charge | Q347R/K360E/D399V/F405T/K409F |
| 904 | Steric hindrance + Charge | Q347K/K360E/D399V/F405T/K409W |
| 905 | Steric hindrance + Charge | Q347Y/K360E/D399V/F405T/K409W |
| 906 | Steric hindrance + Charge | Q347R/K360D/D399V/F405T/K409W |
| 907 | Steric hindrance + Charge | Q347K/K360D/D399V/F405T/K409W |
| 908 | Steric hindrance + Charge | Q347H/K360D/D399V/F405T/K409W |
| 910 | Steric hindrance + Charge | Q347R/K360E/D399L/F405T/K409W |
| 911 | Steric hindrance + Charge | Q347R/K360E/D399I/F405T/K409W |
| 912 | Steric hindrance + Charge | Q347R/K360E/D399M/F405T/K409W |
| 913 | Steric hindrance + Charge | Q347R/K360E/D399S/F405T/K409W |
| 914 | Steric hindrance + Charge | Q347R/K360E/D399T/F405T/K409W |
| 915 | Steric hindrance + Charge + Disulfide bond | Q347R/K360E/D399C/F405T/K409W |
| 916 | Steric hindrance + Charge | Q347R/K360E/D399H/F405T/K409W |
| 917 | Steric hindrance + Charge | Q347R/K360E/D399V/F405A/K409W |
| 918 | Steric hindrance + Charge | Q347R/K360E/D399V/F405V/K409W |
| 927 | Steric hindrance + Charge | Q347R/K360E/D399I/F405T/K409F |
| 928 | Steric hindrance + Charge | Q347R/K360E/D399M/F405T/K409F |
| 929 | Steric hindrance + Charge | Q347R/K360E/D399T/F405T/K409F |
| 931 | Steric hindrance + Charge | Q347R/K360E/D399N/F405T/K409F |
| 943 | Steric hindrance + Charge | Q347R/K360E/D399V/F405A/K409F |
| 944 | Steric hindrance + Charge | Q347R/K360E/D399V/F405V/K409F |
| 952 | Steric hindrance + Charge | Q347R/K360E/D399L/F405A/K409W |
| 957 | Steric hindrance + Charge | Q347R/K360E/D399M/F405A/K409W |
| 960 | Steric hindrance + Charge | Q347R/K360E/D399G/F405T/K409W |
| 961 | Steric hindrance + Charge | Q347H/K360E/D399V/F405T/K409W |
| 963 | Steric hindrance + Charge | Q347R/K360E/D399N/F405T/K409W |
| 969 | Steric hindrance + Charge | Q347R/K360E/D399I/F405A/K409W |
| 971 | Steric hindrance | T394W/F405S |
| 972 | Steric hindrance | T394W/F405T |
| 993 | Steric hindrance | T394W/F405G |
| 996 | Steric hindrance + Disulfide bond | T366W/L368C/Y407V |
| 998 | Steric hindrance | T366W/L368G/Y407V |
| 999 | Steric hindrance | T366Y/L368A/Y407V |
| 1000 | Steric hindrance | T366F/L368A/Y407V |
| 1002 | Steric hindrance | T366W/L368A/Y407L |
| 1011 | Steric hindrance + Disulfide bond | T366W/L368C/Y407C |
| 1031 | Steric hindrance | T366W/L368G/Y407A |
| 1063 | Steric hindrance | T366Y/L368V/Y407A |
| 1087 | Steric hindrance | T366W/L368T/Y407I |
| 1095 | Steric hindrance | T366F/L368V/Y407L |
| 1105 | Steric hindrance | T366F/L368V/Y407M |
| 1134 | Steric hindrance + Disulfide bond | T366F/L368C/Y407C |
| 1143 | Steric hindrance | T366Y/L368V/Y407M |
| 1148 | Steric hindrance + Charge | Q347R/K360E/D399I/F405T/K409Y |
| 1149 | Steric hindrance + Charge | E345R/Q347R/K360D/T366V/D399L/Y407A/K409L |
| 1150 | Steric hindrance + Charge | E345R/Q347R/K360D/T366V/D399I/Y407A/K409I |

**(Table 19) Combinations of modifications that showed strong suppression of heteromeric association in Table 1, and amino acid modifications at the respective residue positions that suppressed the heteromultimer ratio below 51.7% in Table 15**

| Modification set No. | 1 | | 2 | | | 3 | | | | | 4 | | | | | | |
|---|---|---|---|---|---|---|---|---|---|---|---|---|---|---|---|---|---|
| EU numbering | 394 | 405 | 366 | 368 | 407 | 347 | 360 | 399 | 405 | 409 | 345 | 347 | 360 | 366 | 399 | 407 | 409 |
| Combination s of modifications that showed strong suppression of heteromeric association in Table 1 | W | A | W | A | V | R | E | V | T | W | R | R | D | V | M | A | V |
| Amino acid modifications in Table 15 that suppressed the heteromultimer ratio below that of WT, 51.7% | F | S | Y | S | L | K | D | L | A | F | E | K | E | | Q | | Q |
| | Y | T | F | T | I | Y | | I | V | Y | | | | | N | | N |
| | H | C | | C | M | H | | M | S | H | | | | | H | | H |
| | | V | | V | A | | | S | C | | | | | | I | | L |
| | | G | | G | S | | | T | N | | | | | | F | | I |
| | | | | | T | | | C | D | | | | | | Y | | F |
| | | | | | C | | | H | G | | | | | | T | | Y |
| | | | | | H | | | A | | | | | | | S | | T |
| | | | | | N | | | N | | | | | | | V | | S |
| | | | | | Q | | | Q | | | | | | | L | | |
| | | | | | | | | G | | | | | | | | | |

| Modification set No. | 5 | | | | 6 | | | | 7 | | | 8 | | | |
|---|---|---|---|---|---|---|---|---|---|---|---|---|---|---|---|
| EU numbering | 356 | 399 | 409 | 439 | 356 | 392 | 399 | 409 | 392 | 399 | 409 | 356 | 392 | 399 | 439 |
| Combinations of modifications that showed strong suppression of heteromeric association in Table 1 | K | K | E | E | K | D | K | D | D | K | D | K | D | K | E |
| Amino acid modifications in Table 15 that suppressed the heteromultimer ratio below that of WT, 51.7% | R | R | D | D | R | E | R | E | E | R | E | R | E | R | D |
| | H | H | | | H | | H | | | | | | | | |
| | | | | | | | | | | | | | | | |
| | | | | | | | | | | | | | | | |
| | | | | | | | | | | | | | | | |
| | | | | | | | | | | | | | | | |
| | | | | | | | | | | | | | | | |
| | | | | | | | | | | | | | | | |
| | | | | | | | | | | | | | | | |
| | | | | | | | | | | | | | | | |
| | | | | | | | | | | | | | | | |

**(Table 20) Combinations of modifications that showed strong suppression of heteromeric association in Table 1, and amino acid modifications at the respective residue positions that suppressed the heteromultimer ratio to 10% or lower in Table 15**

| Modification set No. | 1 | | 2 | | | 3 | | | | | 4 | | | | | | |
|---|---|---|---|---|---|---|---|---|---|---|---|---|---|---|---|---|---|
| EU numbering | 394 | 405 | 366 | 368 | 407 | 347 | 360 | 399 | 405 | 409 | 345 | 347 | 360 | 366 | 399 | 407 | 409 |
| Combinations of modifications that showed strong suppression of heteromeric association in Table 1 | W | A | W | A | V | R | E | V | T | W | R | R | D | V | M | A | V |
| Amino acid modifications in Table 15 that suppressed the heteromultimer ratio to 10% or lower | F | S | Y | S | L | K | D | L | A | F | E | K | E | | Q | | Q |
| | Y | T | F | T | I | Y | | I | V | Y | | | | | N | | N |
| | | C | | C | M | H | | M | S | H | | | | | H | | H |
| | | G | | V | A | | | S | C | | | | | | I | | L |
| | | V | | G | S | | | T | N | | | | | | F | | I |
| | | | | | T | | | C | D | | | | | | Y | | F |
| | | | | | C | | | H | G | | | | | | T | | Y |
| | | | | | N | | | A | | | | | | | S | | T |
| | | | | | Q | | | N | | | | | | | V | | S |
| | | | | | | | | Q | | | | | | | L | | |
| | | | | | | | | G | | | | | | | | | |

| Modification set No. | 5 | | | | 6 | | | | 7 | | | 8 | | | |
|---|---|---|---|---|---|---|---|---|---|---|---|---|---|---|---|
| EU numbering | 356 | 399 | 409 | 439 | 356 | 392 | 399 | 409 | 392 | 399 | 409 | 356 | 392 | 399 | 439 |
| Combinations of modifications that showed strong suppression of heteromeric association in Table 1 | K | K | E | E | K | D | K | D | D | K | D | K | D | K | E |
| Amino acid modifications in Table 15 that suppressed the hetero- | R | R | D | D | R | E | R | E | E | R | E | R | E | R | D |
| | H | H | | | H | | H | | | | | | | | |
| | | | | | | | | | | | | | | | |
| multimer ratio to 10% or lower | | | | | | | | | | | | | | | |
| | | | | | | | | | | | | | | | |
| | | | | | | | | | | | | | | | |
| | | | | | | | | | | | | | | | |
| | | | | | | | | | | | | | | | |
| | | | | | | | | | | | | | | | |
| | | | | | | | | | | | | | | | |
| | | | | | | | | | | | | | | | |

**(Table 21) Combinations of modifications that showed strong suppression of heteromeric association in Table 1, and amino acid modifications at the respective residue positions that suppressed the heteromultimer ratio to 0% in Table 15**

| Modification set No. | 1 | | 2 | | | 3 | | | | | 4 | | | | | | |
|---|---|---|---|---|---|---|---|---|---|---|---|---|---|---|---|---|---|
| EU numbering | 394 | 405 | 366 | 368 | 407 | 347 | 360 | 399 | 405 | 409 | 345 | 347 | 360 | 366 | 399 | 407 | 409 |
| Combinations of modifications that showed strong suppression of heteromeric association in Table 1 | W | A | W | A | V | R | E | V | T | W | R | R | D | V | M | A | V |
| Amino acid modifications in Table 15 that suppressed the heteromultimer ratio to 0% | F | S | Y | C | L | K | D | L | A | F | E | K | E | | N | | Q |
| | | T | F | G | C | Y | | I | V | Y | | | | | H | | H |
| | | G | | T | A | H | | M | | | | | | | I | | L |
| | | | | V | M | | | S | | | | | | | F | | I |
| | | | | | I | | | T | | | | | | | Y | | F |
| | | | | | | | | C | | | | | | | T | | Y |
| | | | | | | | | H | | | | | | | S | | T |
| | | | | | | | | N | | | | | | | V | | S |
| | | | | | | | | G | | | | | | | L | | |
| | | | | | | | | A | | | | | | | Q | | |
| | | | | | | | | | | | | | | | | | |

| Modification set No. | 5 | | | | 6 | | | | 7 | | | 8 | | | |
|---|---|---|---|---|---|---|---|---|---|---|---|---|---|---|---|
| EU numbering | 356 | 399 | 409 | 439 | 356 | 392 | 399 | 409 | 392 | 399 | 409 | 356 | 392 | 399 | 439 |
| Combinations of modifications that showed strong suppression of heteromeric association in Table 1 | K | K | E | E | K | D | K | D | D | K | D | K | D | K | E |
| Amino acid modifications in Table 15 that suppressed the heteromultimer ratio to 0% | R | R | D | D | R | E | R | E | | | E | | | | |
| | | H | | | H | | H | | | | | | | | |
| | | | | | | | | | | | | | | | |
| | | | | | | | | | | | | | | | |
| | | | | | | | | | | | | | | | |
| | | | | | | | | | | | | | | | |
| | | | | | | | | | | | | | | | |
| | | | | | | | | | | | | | | | |
| | | | | | | | | | | | | | | | |
| | | | | | | | | | | | | | | | |
| | | | | | | | | | | | | | | | |

### [Example 13] Evaluation of coexpression of homodimer and heteromultimer

The amino acid modifications discovered in the present invention which promote homodimer formation may be able to be used concurrently with amino acid modifications promoting heterodimer formation (e.g., knob-into-hole modifications) To verify this possibility, antibodies containing some of the modifications that showed strong homodimer formation ability in IgG and knob-into-hole modifications were coexpressed, and the efficiencies (%) of formation of the homodimer and heterodimer of interest when coexpressed with WT-IgG1 were compared by IEC analysis. Antibody preparation and IEC analysis were performed according to the methods described in Example 6. Samples were detected at an excitation wavelength of 280 nm and a fluorescence wavelength of 330 nm (denoted as EU on the vertical axes in figures).

The correspondences between the amino acid modifications promoting heavy chain homodimer formation and heterodimer formation, SEQ ID NOs, and area ratios are shown in Table 22 (the non-introduction of modifications is denoted as "-"). This test was performed using the template antibody pairs of two homodimers and two heteromultimers (In Table 22, the template antibody group "hetero1+homo1" refers to the pair of a heterodimer containing SEQ ID NOs: 561 and 562 and a homodimer containing SEQ ID NO: 563, and the template antibody group "hetero2+homo2" refers to the pair of a heterodimer containing SEQ ID NOs: 567 and 568 and a homodimer containing SEQ ID NO: 78. The respective homodimer formation-promoting modifications were introduced into the homodimers.) The chromatograms of each antibody analysis are shown in Figs 6-1 to 6-10. The elution positions of the intended heavy chain heterodimer and heavy chain homodimer, and the area ratio of unintended multimers were calculated by assignment of peaks based on the elution positions of the respective reference samples.

This result showed that the amino acid modifications discovered in the present invention which promote homodimer formation can be used concurrently with amino acid modifications promoting heteromultimers (e.g., heterodimer formation). It was also shown that this effect allows more efficient expression of the homodimer and heteromultimer of interest than expression of the homodimer of WT-IgG.

**(Table 22) Combinations of modifications promoting heavy chain heterodimer formation and modifications promoting heavy chain homodimer formation, and the reference samples of unintended multimers**

| Template Antibody Group | Sample No. | Heavy chain 1: SEQ ID NO | Heavy chain abbreviation | Modifications that promote formation of heavy chain heterodimers | Heavy chain 2: SEQ ID NO | Heavy chain abbreviation | Modifications that promote formation of heavy chain heterodimers | Heavy chain 3: SEQ ID NO | Heavy chain abbreviation | Modifications that promote formation of heavy chain homodimers | Light chain: SEQ ID NO |
|---|---|---|---|---|---|---|---|---|---|---|---|
| Hetero 1 +Homo1 | 1151 | 561 | K | T366W | | | | | | | 61 |
| | 1152 | 562 | H | T366S/L368A/Y407V | | | | | | | 61 |
| | 1153 | 561 | K | T366W | 562 | H | T366S/L368A/Y407V | | | | 61 |
| | 1154 | | | | | | | 563 | e | - | 61 |
| | 1155 | 561 | K | T366W | | | | 563 | e | - | 61 |
| | 1156 | 562 | H | T366S/L368A/Y407V | | | | 563 | e | - | 61 |
| | 1157 | 561 | K | T366W | 562 | H | T366S/L368A/Y407V | 563 | e | - | 61 |
| | 1158 | | | | | | | 564 | E | T394W/F405A | 61 |
| | 1159 | 561 | K | T366W | | | | 564 | E | T394W/F405A | 61 |
| | 1160 | 562 | H | T366S/L368A/Y407V | | | | 564 | E | T394W/F405A | 61 |
| | 1161 | 561 | K | T366W | 562 | H | T366S/L368A/Y407V | 564 | E | T394W/F405A | 61 |
| | 1162 | | | | | | | 565 | E' | E356K/K392D/D399 K/K439E | 61 |
| | 1163 | 561 | K | T366W | | | | 565 | E' | E356K/K392D/D399 K/K439E | 61 |
| | 1164 | 562 | H | T366S/L368A/Y407V | | | | 565 | E' | E356K/K392D/D399 K/K439E | 61 |
| | 1165 | 561 | K | T366W | 562 | H | T366S/L368A/Y407V | 565 | E' | E356K/K392D/D399 K/K439E | 61 |
| | 1166 | | | | | | | 82 | E" | Q347R/K360E/D399 V/F405T/K409W | 61 |
| | 1167 | 561 | K | T366W | | | | 82 | E" | Q347R/K360E/D399 V/F405T/K409W | 61 |
| | 1168 | 562 | H | T366S/L368A/Y407V | | | | 82 | E" | Q347R/K360E/D399 V/F405T/K409W | 61 |
| | 1169 | 561 | K | T366W | 562 | H | T366S/L368A/Y407V | 82 | E" | Q347R/K360E/D399 V/F405T/K409W | 61 |
| Hetero2 +Homo2 | 1170 | 567 | K' | Y349C/T366W | | | | | | | 61 |
| | 1171 | 568 | H' | E356C/T366S/L368A /Y407V | | | | | | | 61 |
| | 1172 | 567 | K' | Y349C/T366W | 568 | H' | E356C/T366S/L368A/ Y407V | | | | 61 |
| | 1173 | | | | | | | 78 | f | - | 61 |
| | 1174 | 567 | K' | Y349C/T366W | | | | 78 | f | - | 61 |
| | 1175 | 568 | H' | E356C/T366S/L368A /Y407V | | | | 78 | f | - | 61 |
| | 1176 | 567 | K' | Y349C/T366W | 568 | H' | E356C/T366S/L368A/ Y407V | 78 | f | - | 61 |
| | 1177 | | | | | | | 570 | F | T394W/F405A | 61 |
| | 1178 | 567 | K' | Y349C/T366W | | | | 570 | F | T394W/F405A | 61 |
| | 1179 | 568 | H' | E356C/T366S/L368A /Y407V | | | | 570 | F | T394W/F405A | 61 |
| | 1180 | 567 | K' | Y349C/T366W | 568 | H' | E356C/T366S/L368A/ Y407V | 570 | F | T394W/F405A | 61 |
| | 1181 | | | | | | | 571 | F' | E356K/K392D/D399 K/K439E | 61 |
| | 1182 | 567 | K' | Y349C/T366W | | | | 571 | F' | E356K/K392D/D399 K/K439E | 61 |
| | 1183 | 568 | H' | E356C/T366S/L368A /Y407V | | | | 571 | F' | E356K/K392D/D399 K/K439E | 61 |
| | 1184 | 567 | K' | Y349C/T366W | 568 | H' | E356C/T366S/L368A/ Y407V | 571 | F' | E356K/K392D/D399 K/K439E | 61 |
| | 1185 | | | | | | | 572 | F" | Q347R/K360E/D399 V/F405T/K409W | 61 |
| | 1186 | 567 | K' | Y349C/T366W | | | | 572 | F" | Q347R/K360E/D399 V/F405T/K409W | 61 |
| | 1187 | 568 | H' | E356C/T366S/L368A /Y407V | | | | 572 | F" | Q347R/K360E/D399 V/F405T/K409W | 61 |
| | 1188 | 567 | K' | Y349C/T366W | 568 | H' | E356C/T366S/L368A/ Y407V | 572 | F" | Q347R/K360E/D399 V/F405T/K409W | 61 |

The amino acid modifications contained in the heavy chain, which are denoted as heavy chain abbreviations in the table, are as follows:

| | |
|---|---|
| K | T366W |
| H | T366S/L368A/Y407V |
| K' | Y349C/T366W |
| H' | E356C/T366S/L368A/Y407V |
| e | - |
| E | T394W/F405A |
| E' | E356K/K392D/D399K/K439E |
| E" | Q347R/K360E/D399V/F405T/K409W |
| f | - |
| F | T394W/F405A |
| F' | E356K/K392D/D399K/K439E |
| F" | Q347R/K360E/D399V/F405T/K409W |

In this test, two types of knob-into-hole modifications were used as heteromultimer-promoting modifications; however, other reported modifications may also be used. For example, modifications that may be used include strand-exchange engineered domain (SEED) body, which utilizes the differences in association preference between Ig classes, heterodimeric T-cell receptor (TCR) α and β chains (BEAT Fc), T366W/S354C-T366S/L368A/Y407V/Y349C, K409D/K392D-D399K/E356K, S364H/F405A-Y349T/T394F, D221E/P228E/L368E-D221R/P228R/K409R, F405L-K409R, T350V/T366L/K392L/T394W-T350V/L351Y/F405A/Y407V, K360E/K409W-Q347R/D399V/F405T, K360E/K409W/Y349C-Q347R/D399V/F405T/S354C, K370E/K409W-E357N/D399V/F405T, K360D/D399M/Y407A-E345R/Q347R/T366V/K409V, Y349S/K370Y/T366M/K409V-E356G/E357D/S364Q/Y407A, L351D/L368E-L351K/T366K, and L368D/K370S-E357Q/S364K (separation between two different heavy chains is denoted as "-"). In this case, as modifications for promoting homodimer formation, it is more preferable to use, e.g., a set of modifications that do not have modifications at the same residue numbers as, or that are largely different in the properties of amino acid (in terms of size or the presence/absence of charge) from, the amino acid modifications for promoting heteromeric association because this is expected to be less likely to result in promotion of unintended association due to both types of modifications.

In this test, one heteromultimer and one homomultimer were expressed; however, the number of samples to be coexpressed is not limited thereto. For example, when multiple heteromultimers are expressed, modifications at residue numbers different from each other are preferentially combined. It is further possible to express multiple homomultimers having amino acid modifications for promoting homomeric association that are different in amino acid residue number from those modifications for promoting association of the multiple heteromultimers, or that contain modifications at the same residue numbers but composed of amino acids with different properties. This will be able to allow multiple heteromultimers and multiple homomultimers to be expressed simultaneously with high purity.

### [Example 14] Evaluation of heavy-chain homodimer formation-promoting ability in mammalian expression system using mRNA

Next, it was examined whether the transfection of mammalian cells with messenger RNAs (mRNAs) rather then plasmids would also yield the effect of promoting homodimer formation. The antibodies set forth in Table 25 were transiently expressed in mammalian cells by a method known to those skilled in the art using mRNAs containing sequences encoding the antibody heavy and light chains set forth in Tables 23 and 24, and purified by a method known to those skilled in the art. Specifically, plasmid DNAs containing sequences encoding the antibody heavy and light chains set forth in Tables 23 and 24 were used as templates and subjected to PCR to add a T7 promoter sequence and a poly A sequence. The constructed PCR fragments were used as templates to prepare mRNAs by *in vitro* transcription (IVT) method using MEGAscript T7 Transcription Kit (Thermo Fisher Scientific, AM1333). In transcription, ARCA (TriLink BioTechnologies, N-7003-1) was added as a cap structure, and 1-methylpseudouridine-5'-triphosphate (Trilink BioTechnologies, N-1081-1) as a modified nucleic acid. The prepared mRNAs were transfected into 1 mL of 2.5E+6 cells/mL Expi293 cells at the mass ratios set forth in Table 25 to express the antibodies transiently. After 4 days of transfection, the culture supernatant was collected and the antibodies were purified by a method known to those skilled in the art.

The prepared antibodies were analyzed by the IEC method described in Example 6. The respective area ratios resulting from the three-chain transfection are shown in Table 25. The constructs containing amino acid modifications for promoting homodimer formation showed a marked decrease in the ratio of heteromultimers.

The above results confirm that the amino acid modifications for promoting homodimer formation are effective even when mRNAs are used to express antibodies in mammalian cells.

**(Table 23) mRNAs containing sequences encoding antibody heavy chains**

| Antibody heavy chain name | Antigen | Heavy chain abbreviation | IgG subclass | Modifications that promote formation of heavy chain homodimers | mRNA ID (Example 14) | mRNA ID (Examples 15, 16) | Amino acid sequence: SEQ ID NO | ORF nucleotide sequence: SEQ ID NO |
|---|---|---|---|---|---|---|---|---|
| TR01H113-G1T6 | CD3 | T | IgG1 | - | TW_EBL_A1 | WOTL93145 | 573 | 611 |
| GCH065-G1T6 | GPC3 | G | IgG1 | - | TW_EBL_B1 | WOTL93146 | 607 | 612 |
| TR01H113-G1T6v1 | CD3 | T1 | IgG1 | Q347R/K360E/D399V/F405T/K409W | TW_EBL_C1 | WOTL93147 | 574 | 613 |
| GCH065-G1T6v2 | GPC3 | G2 | IgG1 | E356K/K392D/D399K/K439E | TW_EBL_D1 | WOTL93148 | 608 | 614 |
| Q499-G4d | FIXa | Q | IgG4 | - | TW_EBL_E1 | WOTL93149 | 575 | 615 |
| J327-G4d | FX | J | IgG4 | - | TW_EBL_F1 | WOTL93150 | 609 | 616 |
| Q499-G4dv3 | FIXa | Q3 | IgG4 | T394W/F405A | TW_EBL_G1 | WOTL93151 | 576 | 617 |
| J327-G4dv4 | FX | J4 | IgG4 | T366W/L368A/Y407V | TW_EBL_H1 | WOTL93152 | 610 | 618 |

**(Table 24) mRNAs containing sequences encoding antibody light chains**

| Antibody light chain name | Antigen | mRNA ID (Example 14) | mRNA ID (Examples 15, 16) | Amino acid sequence: SEQ ID NO | ORF nucleotide sequence: SEQ ID NO |
|---|---|---|---|---|---|
| L0011-kT0 | CD3, GPC3 | TW_EBL_A2 | WOTL93153 | 577 | 619 |
| L404-kT0 | FIXa, FX | TW_EBL_B2 | WOTL93154 | 578 | 620 |

**(Table 25) Correspondences of mRNAs used in in vitro test and CIEX analysis results**

| Antibody name | mRNA1 (Heavy chain 1) | mRNA2 (Light chain) | mRNA3 (Heavy chain 2) | mRNA mass ratio (mRNA1/ mRNA2/ mRNA3) | CIEX analysis of samples expressed in vitro [Example 14] | | |
|---|---|---|---|---|---|---|---|
| | | | | | Heavy chain homomultimer (GG, G2G2, JJ, J4J4) (%) | Heavy chain heteromultimer (TG, T1G2, QJ, Q3J4) (%) | Heavy chain homomultimer (TT, T1T1, QQ, Q3Q3) (%) |
| TR01H113-G1T6/L0011-kT0//GCH065-G1T6/L0011-kT0 | TW_EBL_A1 | TW_EBL_A2 | TW_EBL_B1 | 1/4/1 | 24.59 | 53.68 | 21.73 |
| TR01H113-G1T6/L0011-kT0//GCH065-G1T6v2/L0011-kT0 | TW_EBL_A1 | TW_EBL_A2 | TW_EBL_D1 | 1/4/1 | 47.1 | 2.07 | 50.84 |
| TR01_H113-G1T6v1/L0011-kT0//GCH065-G1T6/L0011-kT0 | TW_EBL_C1 | TW_EBL_A2 | TW_EBL_B1 | 1/4/1 | 55.03 | 0 | 44.97 |
| TR01H113-G1T6v1/L0011-kT0//GCH065-G1T6v2/L0011-kT0 | TW_EBL_C1 | TW_EBL_A2 | TW_EBL_D1 | 1/4/1 | 46.13 | 0 | 53.87 |
| Q499-G4d/L404-kT0//J327-G4d/L404-kT0 | TW_EBL_E1 | TW_EBL_B2 | TW_EBL_F1 | 1/4/1 | 13.64 | 48.9 | 37.46 |
| Q499-G4d/L404-kT0//J327-G4dv4/L404-kTO | TW_EBL_E1 | TW_EBL_B2 | TW_EBL_H1 | 1/4/1 | 27.71 | 0 | 72.29 |
| Q499-G4dv3/L404-kT0//1327-G4d/L404-kT0 | TW_EBL_G1 | TW_EBL_B2 | TW_EBL_F1 | 1/4/1 | 39.96 | 0 | 60.04 |
| Q499-G4dv3/L404-kT0//J327-G4dv4/L404-kT0 | TW_EBL_G1 | TW_EBL_B2 | TW_EBL_H1 | 1/4/1 | 27.47 | 0 | 72.53 |

### [Example 15] Preparation of mRNA-encapsulated lipid nanoparticles (mRNA-LNPs)

Lipid nanoparticles encapsulating the mRNAs shown in Tables 23 and 24 (mRNA-LNPs) were prepared using the general lipid composition and preparation method described in references etc. (Hou X, et al., Nat Rev Mater. 2021;6(12):1078-1094, Weng Y, et al., Biotechnol Adv. 2020 May-Jun;40:107534, Webb C, et al., Mol Pharm. 2022 Apr 4;19(4):1047-1058). Specifically, cationic lipid, DOPE, cholesterol, and PEG lipid were dissolved in EtOH at a molar ratio of 35:16:46.5:1.5 and at a total lipid concentration of about 1.3 mg/mL. The untranslated region (UTR) of mRNA was designed and the full-length mRNA was synthesized by Trilink, using 5-methoxyuridine as a modified nucleic acid and CleanCap AG as a cap structure. The prepared mRNAs were diluted to about 0.02 mg/mL in 25 mM sodium acetate buffer (pH 4.0). mRNA-LNPs were prepared by mixing the lipid ethanol solution and the aqueous mRNA solution at a ratio of 1:3 (vol/vol) using NanoAssemblr Ignite (Precision NanoSystems). The prepared mRNA-LNPs were diluted with about three times their volume of PBS. Then, the external buffer components were replaced with PBS using Amicon 100kDa centrifugal filter (Millipore), and the mRNA-LNPs were concentrated to a desired concentration. The resulting mRNA-LNPs were filtered using a 0.22 µm sterile filter. The final mRNA-LNPs were stored at - 80°C until further use.

The particle diameter, polydispersity index (PdI), and zeta potential of mRNA-LNPs were measured using Zetasizer NanoZS (Malvern Panalytical). The mRNA-LNP samples were diluted 20 times in PBS before measuring the particle diameter and PdI, or diluted 170 times in 10 mM phosphate buffer (pH7.2) before measuring the zeta potential.

The total RNA concentration and free RNA in the mRNA-LNP samples were determined by a fluorescence-based assay (Ribogreen^{®} reagent, Thermo Fisher Scientific). Encapsulation efficiency is calculated as (total RNA - free RNA)/total RNA. The mRNA-LNP samples were appropriately diluted with 1x TBE buffer containing 1% Triton-X100 and Ribogreen^{®} reagent for determination of total RNA, or diluted with 1x TBE buffer containing only Ribogreen^{®} reagent for determination of free RNA. A calibration curve was produced using the starting RNA solution used for preparing mRNA-LNP and diluted with 1x TBE buffer containing Ribogreen^{®} reagent +/- 1.0% Triton-X100. Samples containing a mixture of Triton-X100, Ribogreen^{®} reagent, buffer, and mRNA-LNP sample were incubated at room temperature for about 5 minutes in the absence of light, and measured using SpectraMax M3 Microplate Reader (Molecular Devices) with the excitation, auto cutoff, and emission wavelengths set to 488 nm, 515 nm, and 525 nm, respectively. Total RNA and free RNA were determined from an appropriate calibration curve.

When LNPs were prepared in a typical manner, the encapsulation, particle size, and PdI were >90%, <100 nm, and <0.1, respectively.

LNPs encapsulating the corresponding mRNAs were mixed to give the combinations of mRNAs 1-3 shown in Table 26, and subjected to the *in vivo* test of Example 16.

**(Table 26) Names of mRNA-LNPs used in the in vivo test and mRNAs contained, and abbreviations of in vivo expression samples**

| Antibody name | mRNA1 (Heavy chain 1) | mRNA2 (Light chain) | mRNA3 (Heavy chain 2) | mRNA mass ratio (mRNA1/ mRNA2/ mRNA3) | Abbreviation of samples expressed in in vivo test [Example 16] |
|---|---|---|---|---|---|
| TR01H113-G1T6/L0011-kT0//GCH065-G1T6/L0011-kT0 | WOTL93145 | WOTL93153 | WOTL93146 | 1/4/1 | CD3-WT x GPC3-WT |
| TR01H113-G1T6/L0011-kT0//GCH065-G1T6v2/L0011-kT0 | WOTL93145 | WOTL93153 | WOTL93148 | 1/4/1 | CD3-WT x GPC3-Design2 |
| TR01H113-G1T6v1/L0011-kT0//GCH065-G1T6/L0011-kT0 | WOTL93147 | WOTL93153 | WOTL93146 | 1/4/1 | CD3-Design1 x GPC3-WT |
| TR01H113-G1T6v1/L0011-kT0//GCH065-G1T6v2/L0011-kT0 | WOTL93147 | WOTL93153 | WOTL93148 | 1/4/1 | CD3-Design1 x GPC3-Design2 |
| Q499-G4d/L404-kT0//J327-G4d/L404-kT0 | WOTL93149 | WOTL93154 | WOTL93150 | 1/4/1 | FIXa-WT x FX-WT |
| Q499-G4d/L404-kT0//J327-G4dv4/L404-kT0 | WOTL93149 | WOTL93154 | WOTL93152 | 1/4/1 | FIXa-WT x FX-Design4 |
| Q499-G4dv3/L404-kT0//J327-G4d/L404-kT0 | WOTL93151 | WOTL93154 | WOTL93150 | 1/4/1 | FIXa-Design3 x FX-WT |
| Q499-G4dv3/L404-kT0//J327-G4dv4/L404-kT0 | WOTL93151 | WOTL93154 | WOTL93152 | 1/4/1 | FIXa-Design3 x FX-Design4 |

### [Example 16] Evaluation of heavy-chain homodimer formation-promoting ability in mouse in vivo expression using mRNA-LNPs

To test the *in vivo* effect of amino acid modifications for promoting heavy-chain homodimer formation, the mRNA-LNPs produced in Example 15 were intravenously administered to C57BL6/J mice (male, 6 weeks old) at 1.0 mg RNA/kg. The correspondences between the names of mRNA-LNPs used in the *in vivo* test, the mRNAs contained and their mass ratios, and the abbreviations of expression samples, are shown in Table 26.

Whole blood of mice was collected from the jugular vein after 3 and 7 days of administration. After centrifugation at 4°C, 12,000 rpm for 5 minutes, plasma was collected.

The homodimers and heterodimers in the collected plasma were measured. This measurement was carried out using Gyrolab xP workstation or Gyrolab xPand (Gyros Protein Technologies).

First, Human GPC3 Core Protein, TIA0124-rabbitFc (anti-idiotype (ID) antibody for anti-CD3 antibody (TR01H113-G1T6/L0011-kT0 and TR01H113-G1T6v1/L0011-kT0)), rAQ8-mIgG2b described in WO2016047652 (anti-ID antibody for anti-FIXa antibody (Q499-G4d/L404-kT0 and Q499-G4dv3/L404-kT0)), and rAJ540-rbtIgG (anti-ID antibody for anti-FX antibody (J327-G4d/L404-kT0 and J327-G4dv4/L404-kT0)) were labeled with biotin and Alexa Fluor 647 using EZ-Link Sulfo-NHS-Biotin (21217, Thermo Fisher Scientific) and Alexa Fluor 647 Antibody Labeling Kit (A20186, Life Technologies) according to the manufacturers' protocols.

In the measurement of homodimer, the collected plasma or plasma diluted 2 to 200 times with pooled plasma of C57BL/6J (male, 8 weeks old, Jackson Laboratory Japan) was mixed with an equal volume of Rexxip A-max (P0004821, Gyros Protein Technologies). The plasma mixed with an equal volume of Rexxip A-max was mixed with the biotin-labeled antibody and Alexa Fluor 647-labeled antibody diluted with Rexxip A (P0004820, Gyros Protein Technologies) at 1:1:1 (the final concentration of the biotin-labeled antibody and the Alexa Fluor 647-labeled antibody was 1 µg/mL), and incubated at room temperature for 2 hours.

In the measurement of the anti-CD3 homodimer, a plasma sample was mixed with biotin-labeled TIA0124-rabbitFc and Alexa Fluor 647-labeled TIA0124-rabbitFc at 1:1:1.

In the measurement of the anti-GPC3 homodimer, a plasma sample was mixed with biotin-labeled Human GPC3 Core Protein and Alexa Fluor 647-labeled Human GPC3 Core Protein at 1:1:1.

In the measurement of the anti-FIXa homodimer, a plasma sample was mixed with biotin-labeled rAQ8-mIgG2b and Alexa Fluor 647-labeled rAQ8-mIgG2b at 1:1:1.

In the measurement of the anti-FX homodimer, a plasma sample was mixed with biotin-labeled rAJ540-rbtIgG and Alexa Fluor 647-labeled rAJ540-rbtIgG at 1:1:1.

The incubated samples were measured using Gyrolab xP workstation or Gyrolab xPand. In this measurement, Gyrolab Bioaffy 1000 (P0004253, Gyros Protein Technologies) was used as a measurement disk. The above incubated mixture of the biotin-labeled antibody, Alexa Fluor 647-labeled antibody, and plasma sample was added onto the measurement disk, and the biotin-labeled antibody-homodimer-Alexa Fluor 647-labeled antibody complex was captured on the reaction layer in the disk. The amount of the captured complex was detected by the fluorescent signal of Alexa647 (Detect PMT 1%). The fluorescent signal of Alexa647 was analyzed with Gyro Evaluator (version 3.7.2.5976, Gyros Protein Technologies).

In the measurement of heterodimer, the collected plasma was diluted 3 to 5 times with pooled plasma of C57BL/6J (male, 8 weeks old, Jackson Laboratory Japan) and mixed with an equal volume of Rexxip A-max (P0004821, Gyros Protein Technologies). The mixed plasma was measured using Gyrolab xP workstation or Gyrolab xPand. In this measurement, Gyrolab Bioaffy 1000 (P0004253, Gyros Protein Technologies) was used as a measurement disk. The biotin-labeled antibody diluted to 25 µg/mL with PBS 0.05% Tween 20 (P3563, SIGMA-ALDRICH) was added to the measurement disk and immobilized to the reaction layer in the disk. The above prepared plasma sample was added thereto, and the Alexa Flour 647-labeled antibody diluted to 5 µg/mL with Rexxip F (P0004825, Gyros Protein Technologies) was added. The captured heterodimer was detected with the fluorescent signal of Alexa647 (Detect PMT 1%). The fluorescent signal of Alexa647 was analyzed with Gyro Evaluator (version 3.7.2.5976, Gyros Protein Technologies).

In the measurement of the anti-CD3/GPC3 heterodimer, biotin-labeled Human GPC3 Core Protein was used as an immobilization antigen, and Alexa Fluor 647-labeled TIA0124-rabbitFc was used as a detection antibody.

In the measurement of the anti-FIXa/FX heterodimer, biotin-labeled rAQ8-mIgG2b was used as an immobilization antibody, and Alexa Fluor 647-labeled rAJ540-rbtIgG was used as a detection antibody.

As a result of this analysis, the plasma concentration of each antibody is shown in Figs 7-1 and 7-2 and Figs. 7-3 and 7-4.

On day 3 after administration, for the WT antibodies which did not contain homomeric association-promoting modifications (CD3-WT x GPC3-WT and FIXa-WT x FX-WT), the expression of two types of homodimers and heterodimers was detected. For the Design antibodies which contained homomeric association-promoting modifications in either or both antibodies (CD3-WT x GPC3-Design2, CD3-Design1 x GPC3-WT, CD3-Design1 x GPC3-Design2, FIXa-WT x FX-Design4, FIXa-Design3 x FX-WT, and FIXa-Design3 x FX-Design4), the expression of homodimers was detected at a similar level as the WT antibodies or slightly increased. On the other hand, the heterodimer expression of the Design antibodies was significantly suppressed in all samples. Compared to the heterodimer expression in the WT antibodies, the expression of the anti-CD3/GPC3 heterodimer was suppressed by 72 to 878 times or more, and the expression of the anti-FIXa/FX heterodimer was suppressed by 241 to 446 times or more.

The suppression effect on the heterodimer expression continued even on day 7 after administration, showing 84- to 717-fold or more suppression of the expression of the anti-CD3/GPC3 heterodimer, and 184- to 375-fold or more suppression of the expression of the anti-FIXa/FX heterodimer.

This demonstrated that the Design antibodies can suppress the expression of heterodimers *in vivo.*

This test demonstrated the *in vivo* expression of two antibodies containing the Q347R/K360E/D399V/F405T/K409W or E356K/K392D/D399K/K439E modification in one or two heavy chains, and two antibodies containing the T394W/F405A or T366W/L368A/Y407V modification in one or two heavy chains. Similar suppression of heterodimer expression is also expected with the other amino acid modifications for which the promotion of homodimer formation was confirmed in the *in vitro* test using plasmids, in the other molecular formats and functional Fc, and in combinations of three or more antibodies.

Examples 1-16 above found a number of modifications that have a strong ability to promote heavy-chain homodimerization and also have superior physicochemical properties and Fc functions for therapeutic antibodies. Furthermore, an exhaustive study of combinations of modified Fc regions found a number of combinations that allow heavy-chain homodimerization to be maintained between modified Fc regions. Since these are antibodies that do not associate with WT-IgG, they make it possible to provide therapeutic drugs based on multiple antibodies that are homogeneous and safe when *in vivo* antibody expression is intended.

### [Industrial Applicability]

The present disclosure provides polypeptides for which association between polypeptides is controlled, methods for producing polypeptides for which association is controlled, methods for controlling the association of polypeptides, nucleic acids encoding polypeptides for which association is controlled, compositions containing such a nucleic acid, and such. Because of the controlled association between polypeptides, the polypeptides of the present disclosure can particularly be used for production of antibodies and expression of therapeutic drugs in living organisms.

## Claims

1. A nucleic acid encoding a first polypeptide,
wherein the first polypeptide comprises an Fc region into which a modification has been introduced,
wherein, because of the modification introduced into the Fc region, the first polypeptide associates more readily with a first polypeptide having the modification via the Fc region than with a first polypeptide comprising an Fc region into which the modification has not been introduced.

2. The nucleic acid of claim 1, wherein the first polypeptide associates more readily with a first polypeptide having the modification via the Fc region than with a first polypeptide comprising an Fc region into which the modification has not been introduced due to at least one of the following actions resulting from the introduced modification: (1) steric complementarity, (2) disulfide linkage, and (3) electrostatic charge.

3. A composition comprising:
the nucleic acid of claim 1; and
a nucleic acid encoding a second polypeptide,
wherein the second polypeptide comprises an Fc region into which a modification has been introduced,
wherein, because of the modification introduced into the Fc region, the second polypeptide associates more readily with a second polypeptide having the modification via the Fc region than with a second polypeptide comprising an Fc region into which the modification has not been introduced.

4. A composition comprising:
the nucleic acid of claim 1;
a nucleic acid encoding a second polypeptide; and
a nucleic acid encoding a third polypeptide,
wherein the second polypeptide associates more readily with the third polypeptide than with the second polypeptide.

5. A composition comprising:
the nucleic acid of claim 2; and
a nucleic acid encoding a second polypeptide,
wherein the second polypeptide does not have the modification the first polypeptide has.

6. The composition of claim 3, wherein the modification introduced into the first polypeptide is different from the modification introduced into the second polypeptide.

7. The composition of claim 3, wherein the second polypeptide associates more readily with a second polypeptide having the modification via the Fc region than with a second polypeptide comprising an Fc region into which the modification has not been introduced due to at least one of the following actions resulting from the introduced modification: (1) steric complementarity, (2) disulfide linkage, and (3) electrostatic charge.

8. The nucleic acid of claim 1, wherein the modification introduced into the Fc region is at least one of the modifications set forth in Table 2.

9. The nucleic acid of claim 1, wherein the modification introduced into the Fc region is at least one of the modifications set forth in Table 4.

10. The nucleic acid of claim 6, wherein the modification introduced into the first polypeptide and the modification introduced into the second polypeptide are at least one of the combinations of modifications set forth in Table 6.

11. The nucleic acid of claim 1, wherein the polypeptide is an antibody.

12. The nucleic acid of claim 1, wherein the Fc region is an Fc region of IgG.

13. The nucleic acid of claim 1, wherein the polypeptide into which the modification has been introduced retains a function of the polypeptide prior to modification.

14. The nucleic acid of claim 12, wherein the Fc region into which the modification has been introduced retains a function of an Fc of IgG.

15. The nucleic acid of claim 1, wherein the Fc region is derived from any one of IgG1, 2, 3, and 4.

16. A host cell into which the nucleic acid of any one of claims 1, 2, and 8-14 or the composition of any one of claims 3-7 has been introduced.

17. A polypeptide expressed from the nucleic acid of claim 1.

18. A composition comprising a nucleic acid encoding a polypeptide,
wherein the polypeptide comprises an Fc region into which a modification has been introduced, wherein the modification is at least one of the modifications set forth in Table 2.

19. A polypeptide which comprises an Fc region into which a modification has been introduced, wherein the polypeptide associates more readily with a polypeptide having the modification via the Fc region than with a polypeptide comprising an Fc region into which the modification has not been introduced due to at least one of the following actions resulting from the introduced modification: (1) steric complementarity, (2) disulfide linkage, and (3) electrostatic charge.

20. A method for obtaining a polypeptide for which association is controlled,
comprising:
obtaining a nucleic acid encoding the polypeptide; and
expressing the nucleic acid,
wherein the polypeptide comprises an Fc region, and
wherein, because of the modification introduced into the Fc region, the polypeptide associates more readily with a polypeptide having the modification via the Fc region than with a polypeptide comprising an Fc region into which the modification has not been introduced.

21. A method for controlling association of a homomer of a polypeptide, comprising:
obtaining a nucleic acid encoding the polypeptide; and
expressing the nucleic acid,
wherein the polypeptide comprises an Fc region, and
wherein, because of the modification introduced into the Fc region, the polypeptide associates more readily with a polypeptide having the modification via the Fc region than with a polypeptide comprising an Fc region into which the modification has not been introduced.

22. A method for promoting expression of a homomer of a polypeptide, comprising:
obtaining a nucleic acid encoding the polypeptide; and
expressing the nucleic acid,
wherein the polypeptide comprises an Fc region, and
wherein, because of the modification introduced into the Fc region, the polypeptide associates more readily with a polypeptide having the modification via the Fc region than with a polypeptide comprising an Fc region into which the modification has not been introduced.

23. The composition of claim 4, wherein the second polypeptide comprises an Fc region into which a modification has been introduced,
wherein, because of the modification introduced into the Fc region, the second polypeptide associates more readily with the third polypeptide via the Fc region than with the second polypeptide.

24. The composition of claim 1, wherein the Fc region into which the modification has been introduced comprises an amino acid modification at one combination or two or more combinations of positions selected from the combinations of positions shown in (a) to (d) below according to EU numbering:
(a) positions 394 and 405;
(b) positions 366, 368, and 407;
(c) positions 347, 360, 399, 405 and 409; and
(d) positions 356, 392, 399, and 439.

25. The composition of claim 24, wherein the Fc region into which the modification has been introduced comprises at least one amino acid selected from the group consisting of:
(a) W, F, or Y at position 394, and
A, S, T, C, G, or V at position 405;
(b) W, Y, or F at position 366,
A, S, T, C, V, or G at position 368, and
V, L, I, M, A, S, T, C, N, or Q at position 407;
(c) R, K, Y, or H at position 347,
E or D at position 360,
V, L, I, M, S, T, C, H, A, N, Q, or G at position 399,
T, A, V, S, C, N, D, or G at position 405, and
W, F, Y, or H at position 409; and
(d) K or R at position 356,
D or E at position 392,
K or R at position 399, and
E or D at position 439,
according to EU numbering.

26. The composition of claim 24, wherein the Fc region into which the modification has been introduced comprises at least one amino acid selected from the group consisting of:
(a) W or F at position 394, and
A, S, T, or G at position 405;
(b) W, Y, or F at position 366,
A, T, C, V, or G at position 368, and
V, L, I, M, A, or C at position 407;
(c) R, K, Y, or H at position 347,
E or D at position 360,
V, L, I, M, S, T, C, H, A, N, or G at position 399,
T, A, or V at position 405, and
W, F, or Y at position 409; and
(d) K at position 356,
D at position 392,
K at position 399, and
E at position 439,
according to EU numbering.

27. The nucleic acid of claim 1, wherein the modification introduced into the Fc region is at least one of the modifications set forth in Table 15.

28. The nucleic acid of claim 1, wherein the modification introduced into the Fc region is at least one of the modifications set forth in Table 17.
